# EUROPEAN PATENT APPLICATION

(11) **EP 1 657 238 A1**
(43) Date of publication of application: **17.05.2006**
(21) Application number: 04772278.0
(22) Date of filing: 20.08.2004
(51) Int. Cl.: C07D 239/95, C07D 239/96, C07D 401/04, C07D 401/10, C07D 401/12, C07D 403/12, C07D 405/12, C07D 405/14, C07D 409/04, C07D 409/14, C07D 413/12, C07D 417/12, C07D 471/04, A61K 31/517, A61K 31/498, A61P 1/00, A61P 5/02, A61P 13/08, A61P 15/08, A61P 15/18, A61P 17/10

(54) **FUSED PYRIMIDINE DERIVATIVE AND USE THEREOF**

(30) Priority: 22.08.2003 JP 2003298637
(71) Applicant: Takeda Pharmaceutical Company Limited, Osaka-shi, Osaka 541-0045 (JP)
(72) Inventor: HAMAMURA, Kazumasa, Kawanishi-shi, Hyogo 6660005 (JP); ODA, Tsuneo, 5670895 (JP); KUSAKA, Masami, Kobe-shi, Hyogo 6512102 (JP); KANZAKI, Naoyuki, 5670867 (JP)
(74) Representative: Rickard, Timothy Mark Adrian
(86) International application number: PCT/JP2004/012322
(87) International publication number: WO 2005/019188

(57) **Abstract**

There are provided a fused pyrimidine compound having antagonistic activity against luteinizing hormone releasing hormone, and a medicine containing the compound. A luteinizing hormone releasing hormone antagonist containing a compound represented by the formula: 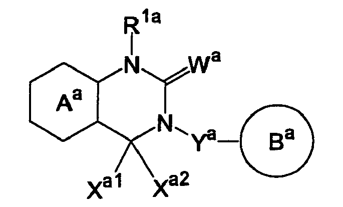 wherein R^{1a} is a hydrocarbon group which may be substituted or a hydrogen atom,
ring A^{a} is a 6-membered aromatic ring which may be further substituted,
ring B^{a} is a homocyclic or heterocyclic ring which may be further substituted,
W^{a} is an oxygen atom or a sulfur atom,
X^{a1} and X^{a2}, which may be identical or different, are each a hydrogen atom, a hydrocarbon group which may be substituted, or a heterocyclic group which may be substituted, or X^{a1} and X^{a2} together may form an oxygen atom, a sulfur atom or NR^{3a} (wherein R^{3a} is a hydrocarbon group which may be substituted or a hydrogen atom), and
Y^{a} is C₁₋₆ alkylene which may be substituted or a bond, or a salt or prodrug thereof.

## Description

### Technical Field

The present invention relates to a fused pyrimidine derivative having excellent antagonistic activity against gonadotropin releasing hormone.

### Background Art

Secretion of anterior pituitary hormones is regulated by peripheral hormones secreted from target organs of the respective hormones and by secretion-promoting or secretion-inhibiting hormones secreted from the hypothalamus, which is the upper central organ of the anterior lobe of the pituitary (hereinafter, these hormones are collectively called "hypothalamic hormones" in the present specification). So far, the existence of nine species of hypothalamic hormones has been confirmed, including, for example, thyrotropin releasing hormone (TRH), gonadotropin releasing hormone [GnRH, sometimes called LH-RH (luteinizing hormone releasing hormone)] and the like. These hypothalamic hormones are believed to show their actions via the receptors that are considered to exist in the anterior lobe of the pituitary, and analysis of the receptor gene which is specific to these hormones., including for humans, is being made. Accordingly, antagonists or agonists specifically and selectively acting on these receptors should control the action of the hypothalamic hormones and the secretion of anterior pituitary hormones. As a result, such antagonists or agonists are expected to be useful in preventing or treating anterior pituitary hormone-dependent diseases.

As the compounds having GnRH-antagonizing activity, there are known GnRH-derived linear peptides (Patent Document 1 and Patent Document 2), a cyclic hexapeptide derivative (Patent Document 3), a bicyclic peptide derivative (Non-Patent Document 1) and the like. Non-peptidic compounds having GnRH-antagonizing activity include the compounds described in Patent Documents 4 through 11.

Further, as the compounds having an aromatic group having amide or sulfonamide bound via a nitrogen atom of a fused pyrimidine ring, the following compounds may be mentioned.

Patent Document 12 describes compounds represented by the formula: 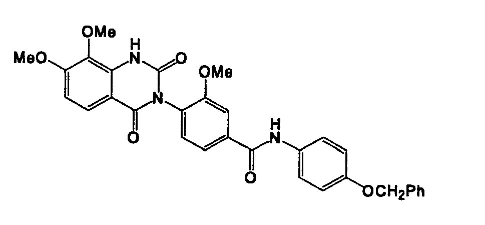 as a neurovascularization inhibitor.

Patent Document 13 describes compounds represented by the formula: 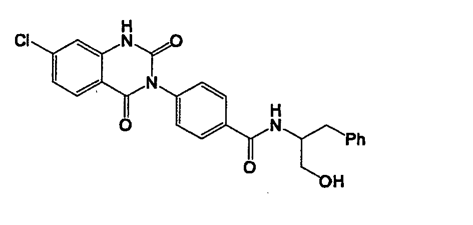 as a cysteine protease inhibitor.

Patent Document 14 describes a compound represented by the formula: 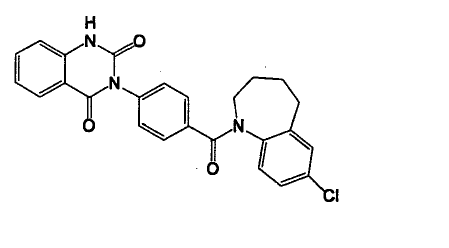 as a vasopressin antagonist as well as a vasopressin agonist.

Non-Patent Document 2 describes synthesis of compounds represented by the formula:

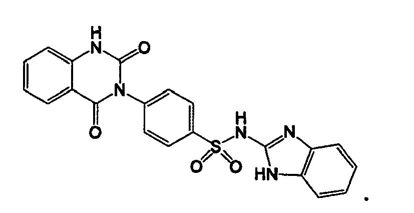

Patent Document 15 describes a compound represented by the formula: 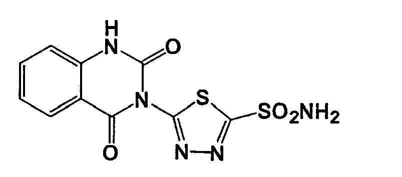 as a carbonic anhydrase inhibitor.

Non-Patent Document 3 describes synthesis of a compound represented by the formula:

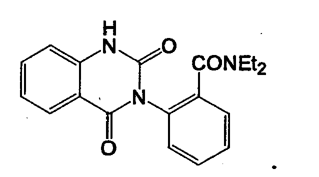

Non-Patent Document 4 describes synthesis of compounds represented by the formula:

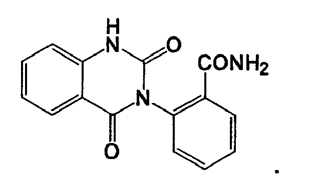

Non-Patent Document 5 describes synthesis of a compound represented by the formula:

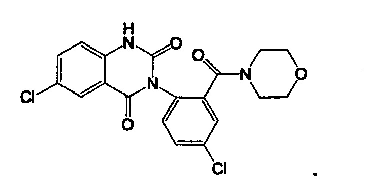

Non-Patent Document 6 describes compounds represented by the formula: 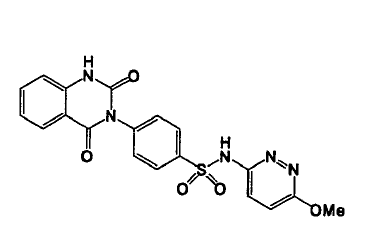 as compounds having antibacterial activity.

Non-Patent Document 7 describes synthesis of compounds represented by the formula:

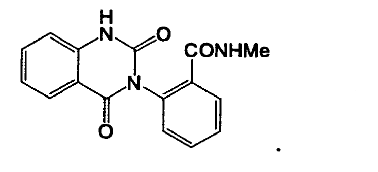

Non-Patent Document 8 describes synthesis of a compound represented by the formula:

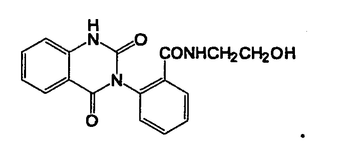

Patent Document 16 describes a compound represented by the formula: 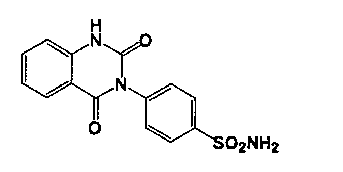 as a starting material for the synthesis of medicine, dye and insecticide.

Non-Patent Document 9 describes the following compounds as LT00082529 and LT00082554:

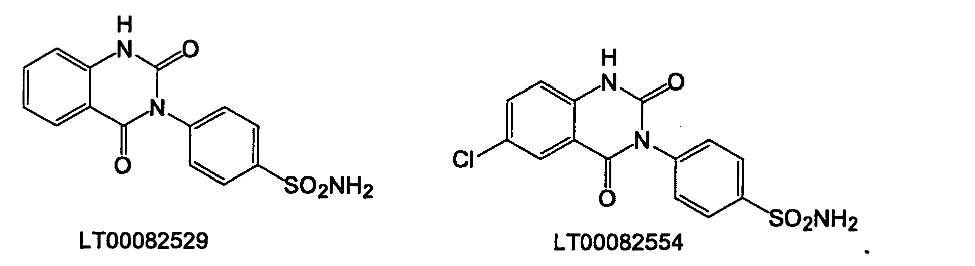

Patent Document 17 describes a method for synthesis of the following indole derivatives as chymase inhibitors:

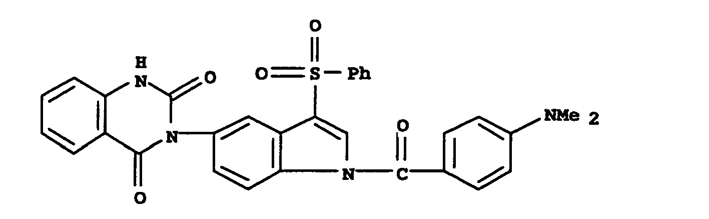

Patent Document 18 describes compounds represented by the formula: 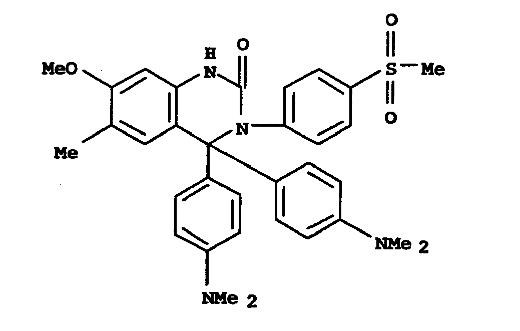 as colorants or color-forming agents.

Patent Document 19 describes compounds represented by the formula: 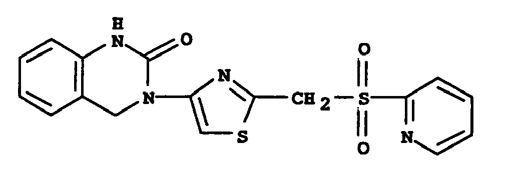 as Cdk2 and Cdk5 kinase inhibitors for the prevention of cell growth-related diseases.

Non-Patent Document 10 describes a method for solid phase synthesis of compounds represented by the formula:

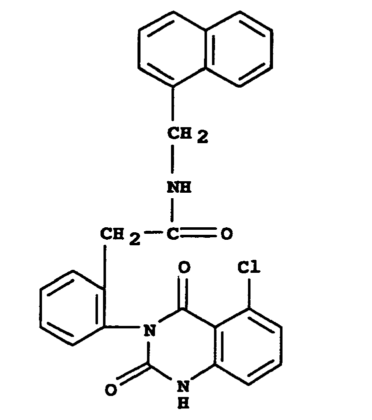

Non-Patent Document 11 describes a compound represented by the formula: 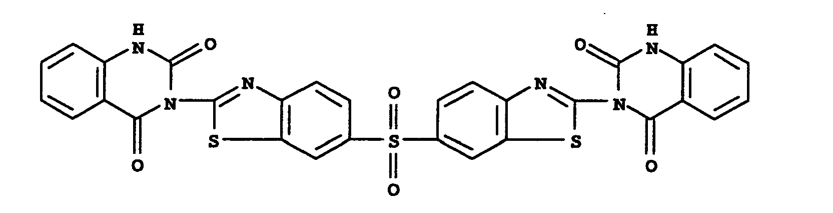
[Patent Document 1] USP 5,140,009
[Patent Document 2] USP 5,171,835
[Patent Document 3] JP-A No. 61-191698
[Patent Document 4] JP-A No. 8-295693 (WO 95/28405)
[Patent Document 5] JP-A No. 9-169768 (WO 96/24597)
[Patent Document 6] JP-A No. 9-169735 (WO 97/14682)
[Patent Document 7] JP-A No. 9-169767 (WO 97/14697)
[Patent Document 8] JP-A No. 11-315079 (WO 99/33831)
[Patent Document 9] JP-A No. 2000-219691 (WO 00/00493)
[Patent Document 10] JP-A No. 2001-278884 (WO 00/56739)
[Patent Document 11] JP-A No. 2002-30087
[Patent Document 12] JP-A No. 10-259176
[Patent Document 13] DE 19650975 A
[Patent Document 14] JP-A No. 9-221476 (WO 95/34540)
[Patent Document 15] WO 91/14430
[Patent Document 16] GB 753171 A
[Patent Document 17] WO 2001/032621
[Patent Document 18] DE 3420799
[Patent Document 19] WO 2003/101985
[Non-Patent Document 1] Journal of Medicinal Chemistry, Vol. 36, pp. 3265-3273 (1993)
[Non-Patent Document 2] Journal of Heterocyclic Chemistry, Vol. 32(4), pp. 1181-1183 (1995)
[Non-Patent Document 3] Journal of the Chemical Society, Perkin Transaction 1, Vol. 11, pp. 2884-2889 (1981)
[Non-Patent Document 4] Acta Chimica Academiae Scientiarum Hungaricae, Vol. 107(1), pp. 57-66 (1981)
[Non-Patent Document 5] Journal of Organic Chemistry, Vol. 41 (16), pp. 2728-2731 (1976)
[Non-Patent Document 6] Pharmazie, Vol. 30(4), pp. 254-255 (1975)
[Non-Patent Document 7] Acta Chimica Academiae Scientiarum Hungaricae, Vol. 48(1), pp. 77-87 (1966)
[Non-Patent Document 8] Chemische Berichte, Vol. 99(5), pp. 1532-1540 (1966)
[Non-Patent Document 9] LaboTest Stock catalogue, published on Jan. 2, 2002
[Non-Patent Document 10] Tetrahedron, Vol. 59(29), pp. 5603-5608 (2003)
-[Non-Patent Document 11] Journal of Heterocyclic Chemistry, Vol. 27(5), pp. 1341-1344 (1990)

### Disclosure of the Invention

Peptidic compounds have many problems to be resolved in the aspects of oral absorption, administration mode, dosage, drug stability, sustained action, metabolic stability and the like. There is a strong demand for a GnRH antagonist having excellent oral absorption, especially a non-peptidic antagonist, which has excellent therapeutic effect on hormone-dependent cancers such as prostate cancer, endometriosis, precocious puberty and the like, and which does not show transient pituitary-gonadotropic action (acute action).

The inventors of the present invention have diligently conducted research and, as a result, have found that a compound represented by the formula: 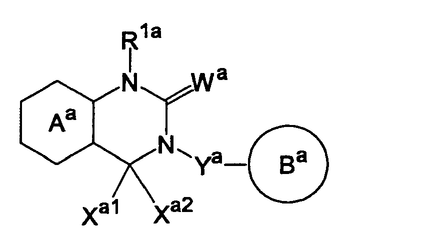 wherein R^{1a} is a hydrocarbon group which may be substituted or a hydrogen atom,
ring A^{a} is a 6-membered aromatic ring which may be further substituted,
ring B^{a} is a homocyclic or heterocyclic ring which may be further substituted,
W^{a} is an oxygen atom or a sulfur atom,
X^{a1} and X^{a2}, which may be identical or different, are each a hydrogen atom, a hydrocarbon group which may be substituted, or a heterocyclic group which may be substituted, or X^{a1} and X^{a2} together may form an oxygen atom, a sulfur atom, or NR^{3a} (wherein R^{3a} is a hydrocarbon group which may be substituted or a hydrogen atom), and
Y^{a} is C₁₋₆ alkylene which may be substituted or a bond, (hereinafter, may be briefly referred to as Compound (II)) or a salt thereof has unexpectedly excellent GnRH antagonizing activity, especially strong antagonist activity. Furthermore, they have found that the precursor of Compound (II) also is converted to Compound (II) in a mixture to be tested during testing (during the evaluation of compound activity) or in vivo, and thus, exhibits unexpectedly excellent GnRH antagonizing activity, especially strong antagonist activity. Moreover, they have also found for the first time that these compounds have low toxicity and are sufficiently satisfactory as medicines having GnRH antagonizing activity.

The inventors have also synthesized for the first time a fused pyrimidine derivative [hereinafter, may be briefly referred to as Compound (I). In addition, Compound (I) includes the compound represented by Formula (I-a) (hereinafter, may be briefly referred to as Compound (I-a)), and the compound represented by Formula (I-b) (hereinafter, may be briefly referred to as Compound (I-b)), and in the present specification, Compound (I) refers to both or either of Compound (I-a) and Compound (I-b)] represented by Formula (I-a) : 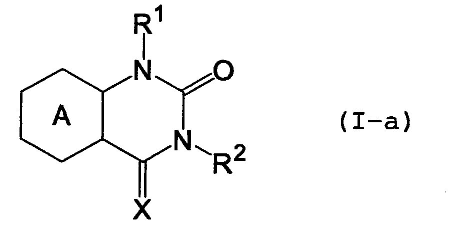 wherein ring A is a 6-membered aromatic ring which may be further substituted,
X is an oxygen atom, a sulfur atom or NR³ (wherein R³ is a hydrocarbon group which may be substituted or a hydrogen atom),
R¹ is a hydrocarbon group which may be substituted or a hydrogen atom, and
R² is
(1) a group represented by the formula: 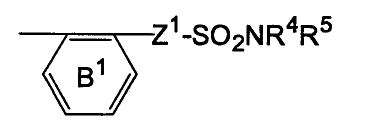 [wherein ring B¹ is a benzene ring which may be further substituted; Z¹ is C₁₋₆ alkylene which may be substituted or a bond; R⁴ is (a) a hydrogen atom, (b) a hydrocarbon group which may be substituted, or (c) a heterocyclic group which may be substituted; and R⁵ is (a) a hydrocarbon group which may be substituted, or (b) a heterocyclic group which may be substituted; or R⁴ and R⁵ may form a ring together with the adjacent nitrogen atom],
(2) a group represented by the formula: 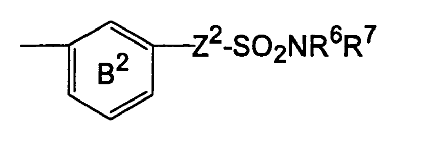 [wherein ring B² is a benzene ring which may be further substituted; Z² is C₁₋₆ alkylene which may be substituted or a bond; R⁶ is (a) a hydrogen atom, (b) a hydrocarbon group which may be substituted, or (c) a heterocyclic group which may be substituted; and R⁷ is (a) a hydrocarbon group which may be substituted, or (b) a heterocyclic group which may be substituted; or R⁶ and R⁷ may form a ring together with the adjacent nitrogen atom],
(3) a group represented by the formula: 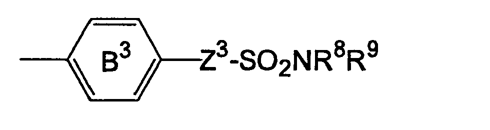 [wherein ring B³ is a benzene ring which may be further substituted; Z³ is C₁₋₆ alkylene which may be substituted or a bond; R⁸ is (a) a hydrogen atom, (b) a hydrocarbon group which may be substituted, or (c) a heterocyclic group which may be substituted; and R⁹ is (a) a hydrocarbon group which may be substituted, or (b) a heterocyclic group which may be substituted; or R⁸ and R⁹ may form a ring together with the adjacent nitrogen atom, provided that when R⁸ is a hydrogen atom, R⁹ is (a) an aryl group which may be substituted, (b) an aralkyl group which may be substituted, or (c) an alkyl group substituted with a heterocyclic group which may be substituted],
(4) a group represented by the formula: 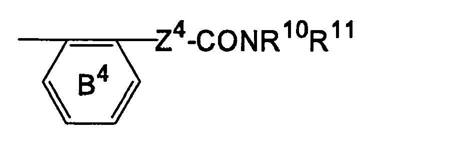 [wherein ring B⁴ is a benzene ring which may be further substituted; Z⁴ is C₁₋₆ alkylene which may be substituted or a bond; R¹⁰ is (a) a hydrogen atom, (b) a hydrocarbon group which may be substituted, or (c) a heterocyclic group which may be substituted; and R¹¹ is (a) an aromatic group which may be substituted, (b) an aralkyl group which may be substituted, or (c) an alkyl group substituted with a heterocyclic group which may be substituted; or R¹⁰ and R¹¹ may form, together with the adjacent nitrogen atom, a heterocyclic ring comprising carbon and nitrogen atoms as ring-constituting moieties],
(5) a group represented by the formula: 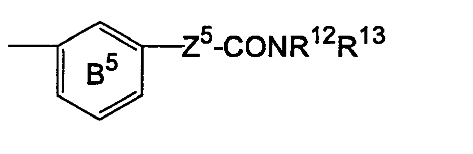 [wherein ring B⁵ is a benzene ring which may be further substituted; Z⁵ is C₁₋₆ alkylene which may be substituted or a bond; and R¹² and R¹³, which may be identical or different, are each (a) a hydrogen atom, (b) a hydrocarbon group which may be substituted, or (c) a heterocyclic group which may be substituted; or R¹² and R¹³ may form a ring together with the adjacent nitrogen atom],
(6) a group represented by the formula: 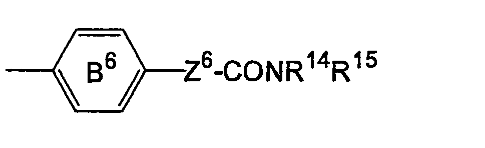 [wherein ring B⁶ is a benzene ring which may be further substituted; Z⁶ is C₁₋₆ alkylene which may be substituted or a bond; and R¹⁴ and R¹⁵, which may be identical or different, are each (a) a hydrocarbon group which may be substituted, or (b) a heterocyclic group which may be substituted; or R¹⁴ and R¹⁵ may form a ring together with the adjacent nitrogen atom],
(7) a group represented by the formula: 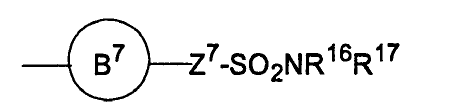 [wherein ring B⁷ is a C₁₀₋₁₄ aryl ring which may be further substituted, or an aromatic heterocycle which may be further substituted; Z⁷ is C₁₋₆ alkylene which may be substituted or a bond; R¹⁶ is (a) a hydrogen atom, (b) a hydrocarbon group which may be substituted, or (c) a heterocyclic group which may be substituted; and R¹⁷ is (a) a hydrocarbon group which may be substituted, or (b) a heterocyclic group which may be substituted; or R¹⁶ and R¹⁷ may form a ring together with the adjacent nitrogen atom],
(8) a group represented by the formula: 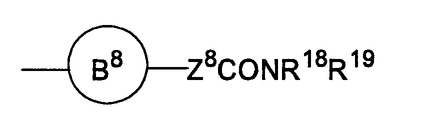 [wherein ring B⁸ is a C₁₀₋₁₄ aryl ring which may be further substituted, or an aromatic heterocycle which may be further substituted; Z⁸ is C₁₋₆ alkylene which may be substituted or a bond; R¹⁸ is (a) a hydrogen atom, (b) a hydrocarbon group which may be substituted, or (c) a heterocyclic group which may be substituted; and R¹⁹ is (a) a hydrocarbon group which may be substituted, or (b) a heterocyclic group which may be substituted; or R¹⁸ and R¹⁹ may form a ring together with the adjacent nitrogen atom], or
(9) a group represented by the formula: 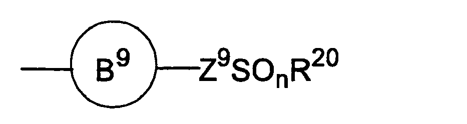 [wherein ring B⁹ is a C₆₋₁₄ aryl ring which may be further substituted, or an aromatic heterocycle which may be further substituted, provided that when X is an oxygen atom, ring B⁹ is not an indole ring; Z⁹ is C₁₋₆ alkylene which may be substituted or a bond; R²⁰ is a hydrocarbon group which may be substituted, or a heterocyclic group which may be substituted; and n is an integer from 0 to 2]; or
   Formula (1-b) : 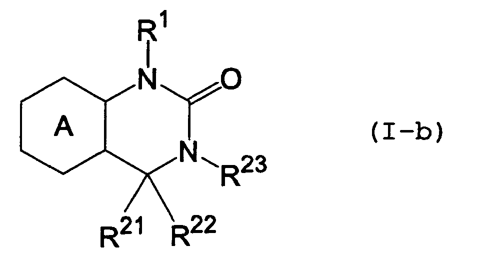
wherein ring A is a 6-membered aromatic ring which may be further substituted,
R¹ is a hydrocarbon group which may be substituted or a hydrogen atom,
R²¹ and R²², which may be identical or different, are each a hydrocarbon group which may be substituted or a hydrogen atom, and
R²³ is
(1) a group represented by the formula: 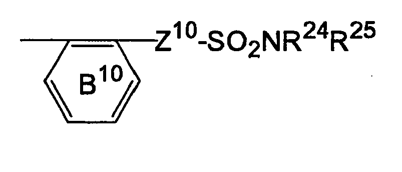 [wherein ring B¹⁰ is a benzene ring which may be further substituted; Z¹⁰ is C₁₋₆ alkylene which may be substituted or a bond; and R²⁴ and R²⁵, which may be identical or different, are each (a) a hydrogen atom, (b) a hydrocarbon group which may be substituted, or (c) a heterocyclic group which may be substituted; or R²⁴ and R²⁵ may form a ring together with the adjacent nitrogen atom],
(2) a group represented by the formula: 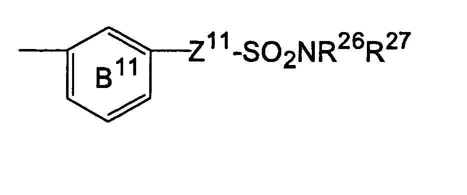 [wherein ring B¹¹ is a benzene ring which may be further substituted; Z¹¹ is C₁₋₆ alkylene which may be substituted or a bond; and R²⁶ and R²⁷, which may be identical or different, are each (a) a hydrogen atom, (b) a hydrocarbon group which may be substituted, or (c) a heterocyclic group which may be substituted; or R²⁶ and R²⁷ may form a ring together with the adjacent nitrogen atom],
(3) a group represented by the formula: 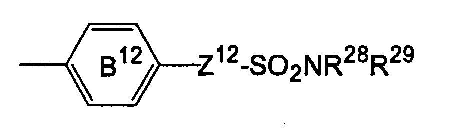 [wherein ring B¹² is a benzene ring which may be further substituted; Z¹² is C₁₋₆ alkylene which may be substituted or a bond; and R²⁸ and R²⁹, which may be identical or different, are each (a) a hydrogen atom, (b) a hydrocarbon group which may be substituted, or (c) a heterocyclic group which may be substituted; or R²⁸ and R²⁹ may form a ring together with the adjacent nitrogen atom],
(4) a group represented by the formula: 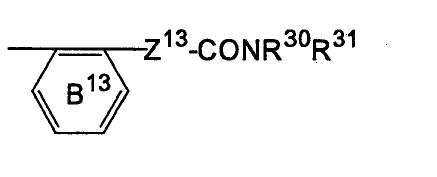 [wherein ring B¹³ is a benzene ring which may be further substituted; Z¹³ is C₁₋₆ alkylene which may be substituted or a bond; and R³⁰ and R³¹, which may be identical or different, are each (a) a hydrogen atom, (b) a hydrocarbon group which may be substituted, or (c) a heterocyclic group which may be substituted; or R³⁰ and R³¹ may form a ring together with the adjacent nitrogen atom],
(5) a group represented by the formula: 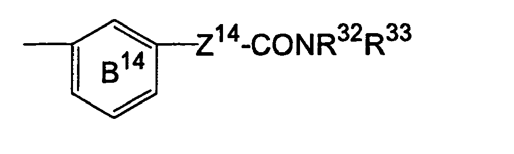 [wherein ring B¹⁴ is a benzene ring which may be further substituted; Z¹⁴ is C₁₋₆ alkylene which may be substituted or a bond; and R³² and R³³, which may be identical or different, are each (a) a hydrogen atom, (b) a hydrocarbon group which may be substituted, or (c) a heterocyclic group which may be substituted; or R³² and R³³ may form a ring together with the adjacent nitrogen atom],
(6) a group represented by the formula: 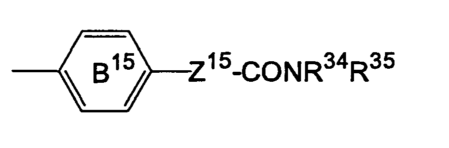 [wherein ring B¹⁵ is a benzene ring which may be further substituted; Z¹⁵ is C₁₋₆ alkylene which may be substituted or a bond; and R³⁴ and R³⁵, which may be identical or different, are each (a) a hydrogen atom, (b) a hydrocarbon group which may be substituted, or (c) a heterocyclic group which may be substituted; or R³⁴ and R³⁵ may form a ring together with the adjacent nitrogen atom],
(7) a group represented by the formula: 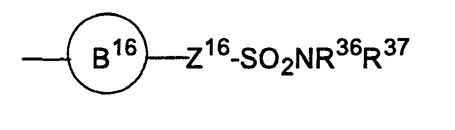 [wherein ring B¹⁶ is a C₁₀₋₁₄ aryl ring which may be further substituted, or an aromatic heterocycle which may be further substituted; Z¹⁶ is C₁₋₆ alkylene which may be substituted or a bond; and R³⁶ and R³⁷, which may be identical or different, are each (a) a hydrogen atom, (b) a hydrocarbon group which may be substituted, or (c) a heterocyclic group which may be substituted; or R³⁶ and R³⁷ may form a ring together with the adjacent nitrogen atom],
(8) a group represented by the formula: 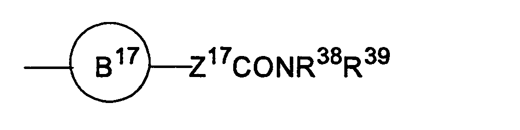 [wherein ring B¹⁷ is a C₁₀₋₁₄ aryl ring which may be further substituted, or an aromatic heterocycle which may be further substituted; Z¹⁷ is C₁₋₆ alkylene which may be substituted or a bond; and R³⁸ and R³⁹, which may be identical or different, are each (a) a hydrogen atom, (b) a hydrocarbon group which may be substituted, or (c) a heterocyclic group which may be substituted; or R³⁸ and R³⁹ may form a ring together with the adjacent nitrogen atom], or
(9) a group represented by the formula: 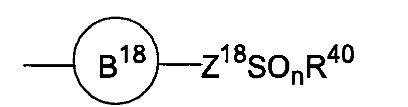 [wherein ring B¹⁸ is a C₆₋₁₄ aryl ring which may be further substituted, or an aromatic heterocycle which may be further substituted; Z¹⁸ is C₁₋₆ alkylene which may be substituted or a bond; R⁴⁰ is a hydrocarbon group which may be substituted, or a heterocyclic group which may be substituted; and n is an integer from 0 to 2, provided that the case where R²¹ and R²² are each a hydrogen atom, ring B¹⁸ is a thiazole ring, Z¹⁸ is methylene, and n is 2, and the case where R²¹ and R²² are each a dimethylaminophenyl, are excluded],
   which is characterized in having a sulfonamide or amide, or thioether, sulfinyl or sulfonyl structure at the end of side chains, or a salt thereof. The inventors have also found for the first time that these compounds have unexpectedly excellent GnRH antagonizing activity, especially strong antagonist activity, based on their specific chemical structures, and that these compounds have very low toxicity and are sufficiently satisfactory as medicines having GnRH antagonizing activity, thus completing the invention based on these findings.

Thus, the invention relates to the following [1] through [31]:
[1] A gonadotropin releasing hormone antagonist which comprises a compound represented by the formula: 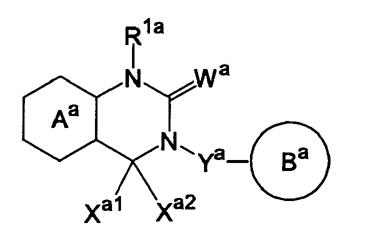 wherein R^{1a} is a hydrocarbon group which may be substituted or a hydrogen atom,
   ring A^{a} is a 6-membered aromatic ring which may be further substituted,
   ring B^{a} is a homocyclic or heterocyclic ring which may be further substituted,
   W^{a} is an oxygen atom or a sulfur atom,
   X^{a1} and X^{a2}, which may be identical or different, are each a hydrogen atom, a hydrocarbon group which may be substituted or a heterocyclic group which may be substituted, or X^{a1} and X^{a2} together may form an oxygen atom, a sulfur atom or NR^{3a} (wherein R^{3a} is a hydrocarbon group which may be substituted or a hydrogen atom), and
   Y^{a} is C₁₋₆ alkylene which may be substituted or a bond, or a salt or prodrug thereof;
[2] The gonadotropin releasing hormone antagonist according to [1] above, wherein X^{a1} and X^{a2} together form an oxygen atom, a sulfur atom, or NR^{3a} (wherein R^{3a} is a hydrocarbon group which may be substituted or a hydrogen atom);
[3] The gonadotropin releasing hormone antagonist according to [1] or [2] above, which is a prophylactic and/or therapeutic agent for prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease, infertility, irritable bowel syndrome, prostate cancer, uterine cancer, breast cancer or pituitary tumor, a reproduction regulator, a contraceptive, an ovulation inducing agent, or a preventive agent for post-operative recurrence of sex hormone-dependent cancers;
[4] A method for prevention and/or treatment of prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease, infertility or irritable bowel syndrome, a method for reproduction regulation, a method for contraception, or a method for ovulation induction, which comprises administering an effective amount of a compound represented by the formula: 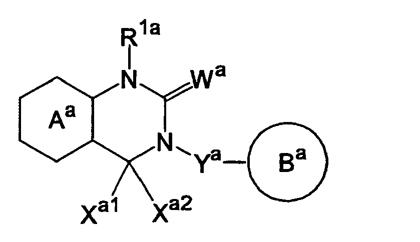 wherein R^{1a} is a hydrocarbon group which may be substituted or a hydrogen atom,
   ring A^{a} is a 6-membered aromatic ring which may be further substituted,
   ring B^{a} is a homocyclic or heterocyclic ring which may be further substituted,
   W^{a} is an oxygen atom or a sulfur atom,
   X^{a1} and X^{a2}, which may be identical or different, are each a hydrogen atom, a hydrocarbon group which may be substituted or a heterocyclic group which may be substituted, or X^{a1} and X^{a2} together may form an oxygen atom, a sulfur atom or NR^{3a} (wherein R^{3a} is a hydrocarbon group which may be substituted or a hydrogen atom), and
   Y^{a} is C₁₋₆ alkylene which may be substituted or a bond, or a salt or prodrug thereof to a mammal;
[5] The method according to [4] above, wherein X^{a1} and X^{a2} together form an oxygen atom, a sulfur atom, or NR^{3a} (wherein R^{3a} is a hydrocarbon group which may be substituted or a hydrogen atom);
[6] Use of a compound represented by the formula: 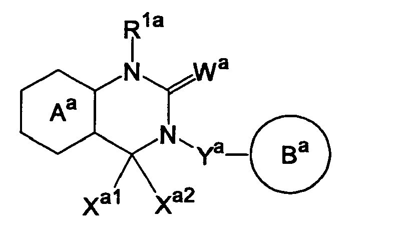 wherein R^{1a} is a hydrocarbon group which may be substituted or a hydrogen atom,
   ring A^{a} is a 6-membered aromatic ring which may be further substituted,
   ring B^{a} is a homocyclic or heterocyclic ring which may be further substituted,
   W^{a} is an oxygen atom or a sulfur atom,
   X^{a1}, and X^{a2}, which may be identical or different, are each a hydrogen atom, a hydrocarbon group which may be substituted or a heterocyclic group which may be substituted, or X^{a1} and X^{a2} together may form an oxygen atom, a sulfur atom or NR^{3a} (wherein R^{3a} is a hydrocarbon group which may be substituted or a hydrogen atom), and
   Y^{a} is C₁₋₆ alkylene which may be substituted or a bond, or a salt or prodrug thereof for the manufacture of a prophylactic and/or therapeutic agent for prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease, infertility or irritable bowel syndrome, a reproduction regulating agent, a contraceptive, or an ovulation inducing agent;
[7] The use according to [6] above, wherein X^{a1} and X^{a2} together form an oxygen atom, a sulfur atom or NR^{3a} (wherein R^{3a} is a hydrocarbon group which may be substituted or a hydrogen atom) ;
[8] A compound represented by
   Formula (I-a) : 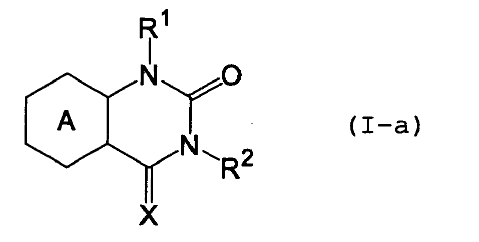
wherein ring A is a 6-membered aromatic ring which may be further substituted,
X is an oxygen atom, a sulfur atom or NR³ (wherein R³ is a hydrocarbon group which may be substituted or a hydrogen atom),
R¹ is a hydrocarbon group which may be substituted or a hydrogen atom, and
R² is
(1) a group represented by the formula: 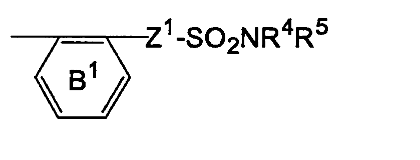 [wherein ring B¹ is a benzene ring which may be further substituted; Z¹ is C₁₋₆ alkylene which may be substituted or a bond; R⁴ is (a) a hydrogen atom, (b) a hydrocarbon group which may be substituted, or (c) a heterocyclic group which may be substituted; and R⁵ is (a) a hydrocarbon group which may be substituted, or (b) a heterocyclic group which may be substituted; or R⁴ and R⁵ may form a ring together with the adjacent nitrogen atom],
(2) a group represented by the formula: 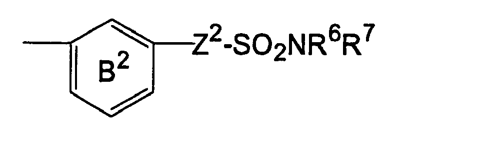 [wherein ring B² is a benzene ring which may be further substituted; Z² is C₁₋₆ alkylene which may be substituted or a bond; R⁶ is (a) a hydrogen atom, (b) a hydrocarbon group which may be substituted, or (c) a heterocyclic group which may be substituted; and R⁷ is (a) a hydrocarbon group which may be substituted, or (b) a heterocyclic group which may be substituted; or R⁶ and R⁷ may form a ring together with the adjacent nitrogen atom],
(3) a group represented by the formula: 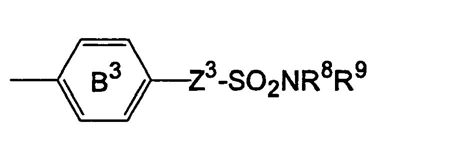 [wherein ring B³ is a benzene ring which may be further substituted; Z³ is C₁₋₆ alkylene which may be substituted or a bond; R⁸ is (a) a hydrogen atom, (b) a hydrocarbon group which may be substituted, or (c) a heterocyclic group which may be substituted; and R⁹ is (a) a hydrocarbon group which may be substituted, or (b) a heterocyclic group which may be substituted; or R⁸ and R⁹ may form a ring together with the adjacent nitrogen atom, provided that when R⁸ is a hydrogen atom, R⁹ is (a) an aryl group which may be substituted, (b) an aralkyl group which may be substituted, or (c) an alkyl group substituted with a heterocyclic group which may be substituted],
(4) a group represented by the formula: 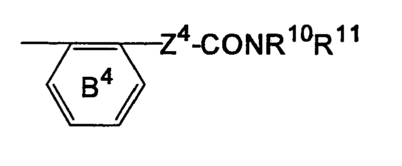 [wherein ring B⁴ is a benzene ring which may be further substituted; Z⁴ is C₁₋₆ alkylene which may be substituted or a bond; R¹⁰ is (a) a hydrogen atom, (b) a hydrocarbon group which may be substituted, or (c) a heterocyclic group which may be substituted; and R¹¹ is (a) an aromatic group which may be substituted, (b) an aralkyl group which may be substituted, or (c) an alkyl group substituted with a heterocyclic group which may be substituted; or R¹⁰ and R¹¹ together with the adjacent nitrogen atom may form a heterocyclic ring comprising carbon and nitrogen atoms as ring-constituting moieties],
(5) a group represented by the formula: 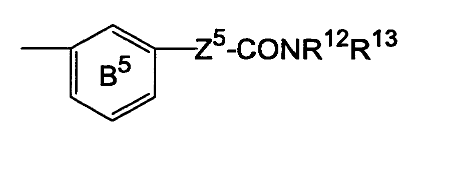 [wherein ring B⁵ is a benzene ring which may be further substituted; Z⁵ is C₁₋₆ alkylene which may be substituted or a bond; and R¹² and R¹³, which may be identical or different, are each (a) a hydrogen atom, (b) a hydrocarbon group which may be substituted, or (c) a heterocyclic group which may be substituted; or R¹² and R¹³ may form a ring together with the adjacent nitrogen atom],
(6) a group represented by the formula: 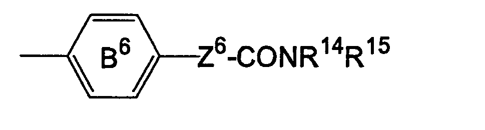 [wherein ring B⁶ is a benzene ring which may be further substituted; Z⁶ is C₁₋₆ alkylene which may be substituted or a bond; and R¹⁴ and R¹⁵, which may be identical or different, are each (a) a hydrocarbon group which may be substituted, or (b) a heterocyclic group which may be substituted; or R¹⁴ and R¹⁵ may form a ring together with the adjacent nitrogen atom],
(7) a group represented by the formula: 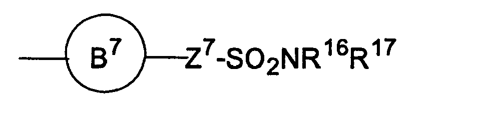 [wherein ring B⁷ is a C₁₀₋₁₄ aryl ring which may be further substituted, or an aromatic heterocycle which may be further substituted; Z⁷ is C₁₋₆ alkylene which may be substituted or a bond; R¹⁶ is (a) a hydrogen atom, (b) a hydrocarbon group which may be substituted, or (c) a heterocyclic group which may be substituted; and R¹⁷ is (a) a hydrocarbon group which may be substituted, or (b) a heterocyclic group which may be substituted; or R¹⁶ and R¹⁷ may form a ring together with the adjacent nitrogen atom],
(8) a group represented by the formula: 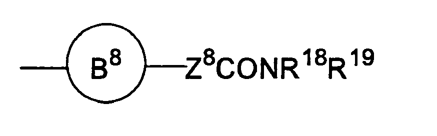 [wherein ring B⁸ is a C₁₀₋₁₄ aryl ring which may be further substituted, or an aromatic heterocycle which may be further substituted; Z⁸ is C₁₋₆ alkylene which may be substituted or a bond; R¹⁸ is (a) a hydrogen atom, (b) a hydrocarbon group which may be substituted, or (c) a heterocyclic group which may be substituted; and R¹⁹ is (a) a hydrocarbon group which may be substituted, or (b) a heterocyclic group which may be substituted; or R¹⁸ and R¹⁹ may form a ring together with the adjacent nitrogen atom], or
(9) a group represented by the formula: 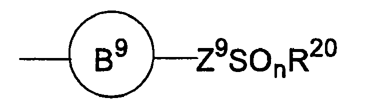 [wherein ring B⁹ is a C₆₋₁₄ aryl ring which may be further substituted, or an aromatic heterocycle which may be further substituted, provided that when X is an oxygen atom, ring B⁹ is not an indole ring; Z⁹ is C₁₋₆ alkylene which may be substituted or a bond; R²⁰ is a hydrocarbon group which may be substituted, or a heterocyclic group which may be substituted; and n is an integer from 0 to 2]; or
   Formula (I-b) : 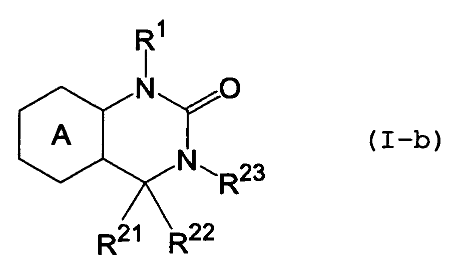
wherein ring A is a 6-membered aromatic ring which may be further substituted,
R¹ is a hydrocarbon group which may be substituted or a hydrogen atom,
R²¹ and R²², which may be identical or different, are each a hydrocarbon group which may be substituted or a hydrogen atom, and
R²³ is
(1) a group represented by the formula: 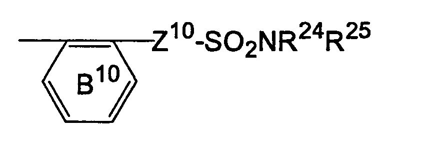 [wherein ring B¹⁰ is a benzene ring which may be further substituted; Z¹⁰ is C₁₋₆ alkylene which may be substituted or a bond; and R²⁴ and R²⁵, which may be identical or different, are each (a) a hydrogen atom, (b) a hydrocarbon group which may be substituted, or (c) a heterocyclic group which may be substituted; or R²⁴ and R²⁵ may form a ring together with the adjacent nitrogen atom],
(2) a group represented by the formula: 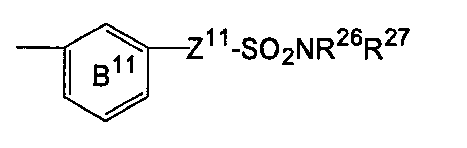 [wherein ring B¹¹ is a benzene ring which may be further substituted; Z¹¹ is C₁₋₆ alkylene which may be substituted or a bond; and R²⁶ and R²⁷, which may be identical or different, are each (a) a hydrogen atom, (b) a hydrocarbon group which may be substituted, or (c) a heterocyclic group which may be substituted; or R²⁶ and R²⁷ may form a ring together with the adjacent nitrogen atom],
(3) a group represented by the formula: 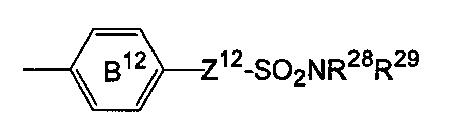 [wherein ring B¹² is a benzene ring which may be further substituted; Z¹² is C₁₋₆ alkylene which may be substituted or a bond; and _{R}2B and R²⁹, which may be identical or different, are each (a) a hydrogen atom, (b) a hydrocarbon group which may be substituted, or (c) a heterocyclic group which may be substituted; or R²⁸ and R²⁹ may form a ring together with the adjacent nitrogen atom],
(4) a group represented by the formula: 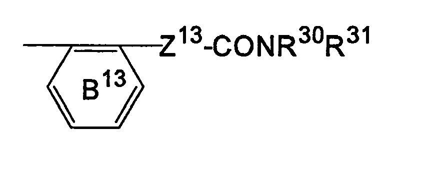 [wherein ring B¹³ is a benzene ring which may be further substituted; Z¹³ is C₁₋₆ alkylene which may be substituted or a bond; and R³⁰ and R³¹, which may be identical or different, are each (a) a hydrogen atom, (b) a hydrocarbon group which may be substituted, or (c) a heterocyclic group which may be substituted; or R³⁰ and R³¹ may form a ring together with the adjacent nitrogen atom],
(5) a group represented by the formula: 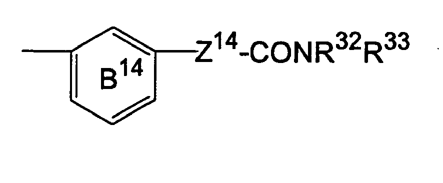 [wherein ring B¹⁴ is a benzene ring which may be further substituted; Z¹⁴ is C₁₋₆ alkylene which may be substituted or a bond; and R³² and R³³, which may be identical or different, are each (a) a hydrogen atom, (b) a hydrocarbon group which may be substituted, or (c) a heterocyclic group which may be substituted; or R³² and R³³ may form a ring together with the adjacent nitrogen atom],
(6) a group represented by the formula: 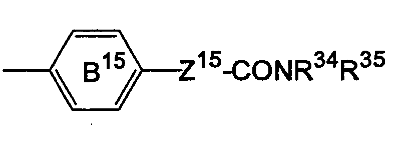 [wherein ring B¹⁵ is a benzene ring which may be further substituted; Z¹⁵ is C₁₋₆ alkylene which may be substituted or a bond; and R³⁴ and R³⁵, which may be identical or different, are each (a) a hydrogen atom, (b) a hydrocarbon group which may be substituted, or (c) a heterocyclic group which may be substituted; or R³⁴ and R³⁵ may form a ring together with the adjacent nitrogen atom],
(7) a group represented by the formula: 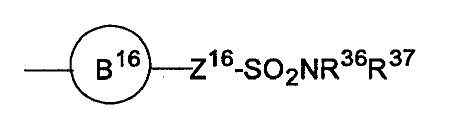 [wherein ring B¹⁶ is a C₁₀₋₁₄ aryl ring which may be further substituted, or an aromatic heterocycle which may be further substituted; Z¹⁶ is C₁₋₆ alkylene which may be substituted or a bond; and R³⁶ and R³⁷, which may be identical or different, are each (a) a hydrogen atom, (b) a hydrocarbon group which may be substituted, or (c) a heterocyclic group which may be substituted; or R³⁶ and R³⁷ may form a ring together with the adjacent nitrogen atom],
(8) a group represented by the formula: 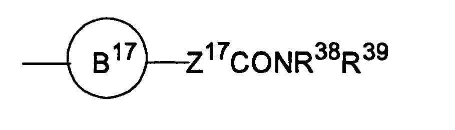 [wherein ring B¹⁷ is a C₁₀₋₁₄ aryl ring which may be further substituted, or an aromatic heterocycle which may be further substituted; Z¹⁷ is C₁₋₆ alkylene which may be substituted or a bond; and R³⁸ and R³⁹, which may be identical or different, are each (a) a hydrogen atom, (b) a hydrocarbon group which may be substituted, or (c) a heterocyclic group which may be substituted; or R³⁸ and R³⁹ may form a ring together with the adjacent nitrogen atom], or
(9) a group represented by the formula: 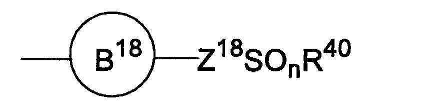 [wherein ring B¹⁸ is a C₆₋₁₄ aryl ring which may be further substituted, or an aromatic heterocycle which may be further substituted; Z¹⁸ is C₁₋₆ alkylene which may be substituted or a bond; R⁴⁰ is a hydrocarbon group which may be substituted, or a heterocyclic group which may be substituted; and n is an integer from 0 to 2, provided that the case where R²¹ and R²² are each a hydrogen atom, ring B¹⁸ is a thiazole ring, Z¹⁸ is methylene, and n is 2, and the case where R²¹ and R²² are each dimethylaminophenyl, are excluded],
   or a salt thereof;
   provided that the following are excluded:
   2-[2-(5-chloro-2,4-dioxo-1,2,3,4-tetrahydro-3-quinazolinyl)phenyl]-N-(1-naphthylmethyl)acetamide,
   2-[3-(5-chloro-2,4-dioxo-1,2,3,4-tetrahydro-3-quinazolinyl)phenyl]-N-(1-naphthylmethyl)acetamide,
   3-(5-chloro-2,4-dioxo-1,2,3,4-tetrahydro-3-quinazolinyl)-N-(1-naphthylmethyl)benzamide,
   7-chloro-1-[4-(1,4-dihydro-2,4-dioxo-3(2H)-quinazolinyl)benzoyl]-2,3,4,5-tetrahydro-1H-1-benzazepine,
   N,N-dibutyl-4-(1,4-dihydro-2,4-dioxo-3(2H)-quinazolinyl)benzenesulfonamide,
   3,3'-(6,6'-sulfonyldibenzothiazole-2,2'-diyl)bis(2,4-dioxo-1,2,3,4-tetrahydroquinazoline),
   N-[4-(benzyloxy)phenyl]-3-methoxy-4-(2-oxo-1,4-dihydroquinazolin-3(2H)-yl)benzamide, and
   N-[4-(benzyloxy)phenyl]-4-(6,7-dimethoxy-2-oxo-1,4-dihydroquinazolin-3(2H)-yl)-3-methoxybenzamide;
[9] The compound according to [8] above, represented by the formula: 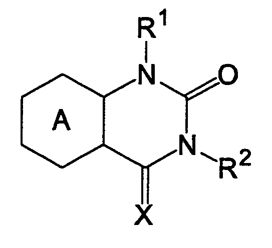
wherein ring A is a 6-membered aromatic ring which may be further substituted,
X is an oxygen atom, a sulfur atom or NR³ (wherein R³ is a hydrocarbon group which may be substituted or a hydrogen atom),
R¹ is a hydrocarbon group which may be substituted or a hydrogen atom, and
R² is
(1) a group represented by the formula: 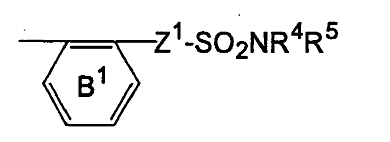 [wherein ring B¹ is a benzene ring which may be further substituted; Z¹ is C₁₋₆ alkylene which may be substituted or a bond; R⁴ is (a) a hydrogen atom, (b) a hydrocarbon group which may be substituted, or (c) a heterocyclic group which may be substituted; and R⁵ is (a) a hydrocarbon group which may be substituted, or (b) a heterocyclic group which may be substituted; or R⁴ and R⁵ may form a ring together with the adjacent nitrogen atom],
(2) a group represented by the formula: 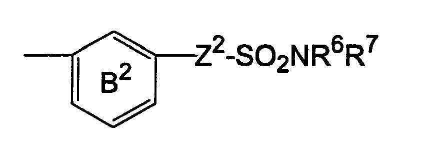 [wherein ring B² is a benzene ring which may be further substituted; Z² is C₁₋₆ alkylene which may be substituted or a bond; R⁶ is (a) a hydrogen atom, (b) a hydrocarbon group which may be substituted, or (c) a heterocyclic group which may be substituted; and R⁷ is (a) a hydrocarbon group which may be substituted, or (b) a heterocyclic group which may be substituted; or R⁶ and R⁷ may form a ring together with the adjacent nitrogen atom],
(3) a group represented by the formula: 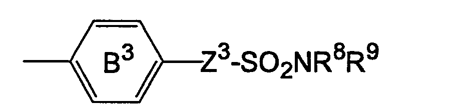 [wherein ring B³ is a benzene ring which may be further substituted; Z³ is C₁₋₆ alkylene which may be substituted or a bond; R⁸ is (a) a hydrogen atom, (b) a hydrocarbon group which may be substituted, or (c) a heterocyclic group which may be substituted; and R⁹ is (a) a hydrocarbon group which may be substituted, or (b) a heterocyclic group which may be substituted; or R⁸ and R⁹ may form a ring together with the adjacent nitrogen atom, provided that when R⁸ is a hydrogen atom, R⁹ is (a) an aryl group which may be substituted, (b) an aralkyl group which may be substituted, or (c) an alkyl group substituted with a heterocyclic group which may be substituted],
(4) a group represented by the formula: 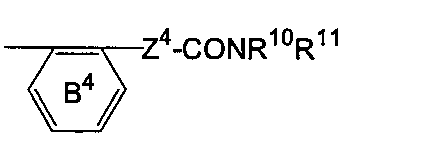 [wherein ring B⁴ is a benzene ring which may be further substituted; Z⁴ is C₁₋₆ alkylene which may be substituted or a bond; R¹⁰ is (a) a hydrogen atom, (b) a hydrocarbon group which may be substituted, or (c) a heterocyclic group which may be substituted; and R¹¹ is (a) an aromatic group which may be substituted, (b) an aralkyl group which may be substituted, or (c) an alkyl group substituted with a heterocyclic group which may be substituted; or R¹⁰ and R¹¹ may form, together with the adjacent nitrogen atom, a heterocyclic ring comprising carbon and nitrogen atoms as ring-constituting moieties],
(5) a group represented by the formula: 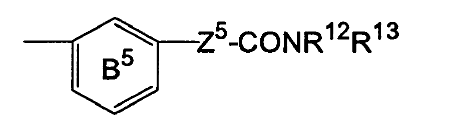 [wherein ring B⁵ is a benzene ring which may be further substituted; Z⁵ is C₁₋₆ alkylene which may be substituted or a bond; and R¹² and R¹³, which may be identical or different, are each (a) a hydrogen atom, (b) a hydrocarbon group which may be substituted, or (c) a heterocyclic group which may be substituted; or R¹² and R¹³ may form a ring together with the adjacent nitrogen atom],
(6) a group represented by the formula: 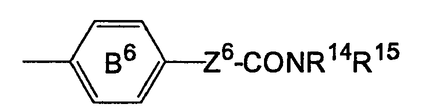 [wherein ring B⁶ is a benzene ring which may be further substituted; Z⁶ is C₁₋₆ alkylene which may be substituted or a bond; and R¹⁴ and R¹⁵, which may be identical or different, are each (a) a hydrocarbon group which may be substituted, or (b) a heterocyclic group which may be substituted; or R¹⁴ and R¹⁵ may form a ring together with the adjacent nitrogen atom],
(7) a group represented by the formula: 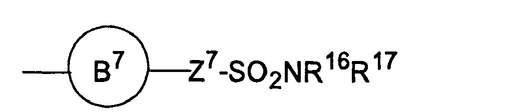 [wherein ring B⁷ is a C₁₀₋₁₄ aryl ring which may be further substituted, or an aromatic heterocycle which may be further substituted; Z⁷ is C₁₋₆ alkylene which may be substituted or a bond; R¹⁶ is (a) a hydrogen atom, (b) a hydrocarbon group which may be substituted, or (c) a heterocyclic group which may be substituted; and R¹⁷ is (a) a hydrocarbon group which may be substituted, or (b) a heterocyclic group which may be substituted; or R¹⁶ and R¹⁷ may form a ring together with the adjacent nitrogen atom], or
(8) a group represented by the formula: 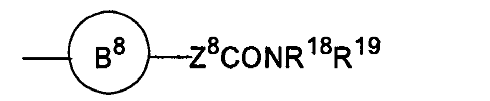 [wherein ring B⁸ is a C₁₀₋₁₄ aryl ring which may be further substituted, or an aromatic heterocycle which may be further substituted; Z⁸ is C₁₋₆ alkylene which may be substituted or a bond; R¹⁸ is (a) a hydrogen atom, (b) a hydrocarbon group which may be substituted, or (c) a heterocyclic group which may be substituted; and R¹⁹ is (a) a hydrocarbon group which may be substituted, or (b) a heterocyclic group which may be substituted; or R¹⁸ and R¹⁹ may form a ring together with the adjacent nitrogen atom], provided that the following are excluded:
   7-chloro-1-[4-(1,4-dihydro-2,4-dioxo-3(2H)-quinazolinyl)benzoyl]-2,3,4,5-tetrahydro-1H-1-benzazepine, and
   N,N-dibutyl-4-(1,4-dihydro-2,4-dioxo-3(2H)-quinazolinyl)benzenesulfonamide;
[10] The compound according to [8] or [9] above, wherein
   R¹ is (1) a hydrogen atom, or (2) C₁₋₆ alkyl which may be substituted with 1 to 3 substituents selected from (a) C₆₋₁₄ aryl which may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen, (b) cyano, (c) hydroxy, (d) carboxy, (e) nitro, (f) C₁₋₆ alkyl which may be substituted with di-C₁₋₆ alkylamino, (g) C₁₋₆ alkoxy, (h) C₁₋₆ alkyl-carbonyloxy, (i) C₁₋₆ alkylthio, (j) C₁₋₆ alkylsulfinyl, (k) C₁₋₆ alkylsulfonyl, (1) halogen, (m) amino, (n) mono-C₁₋₆ alkylamino, (o) di-C₁₋₆ alkylamino, (p) a 5-membered ring group containing 1 to 4 heteroatoms selected from oxygen, sulfur and nitrogen atoms and the like in addition to carbon atoms (wherein this 5-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (q) a 6-membered ring group containing 1 to 4 heteroatoms selected from oxygen, sulfur and nitrogen atoms and the like in addition to carbon atoms (wherein this 6-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (r) a bicyclic or tricyclic fused ring group containing 1 to 4 heteroatoms selected from oxygen, sulfur and nitrogen atoms and the like in addition to carbon atoms (wherein this bicyclic or tricyclic fused ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (s) C₁₋₆ alkoxycarbonyl, (t) carbamoyl, (u) N-mono-C₁₋₆ alkylcarbamoyl, (v) N,N-di-C₁₋₆ alkylcarbamoyl, and (w) C₁₋₂ alkylenedioxy;
[11] The compound according to [8] or [9] above, wherein X is an oxygen atom or NR³ (wherein R³ is (1) a hydrogen atom, (2) C₁₋₆ alkyl which may be substituted with 1 to 3 substituents selected from (a) C₆₋₁₄ aryl which may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen, (b) cyano, (c) hydroxy, (d) carboxy, (e) nitro, (f) C₁₋₆ alkyl which may be substituted with di-C₁₋₆ alkylamino, (g) C₁₋₆ alkoxy, (h) C₁₋₆ alkyl-carbonyloxy, (i) C₁₋₆ alkylthio, (j) C₁₋₆ alkylsulfinyl, (k) C₁₋₆ alkylsulfonyl, (1) halogen, (m) amino, (n) mono-C₁₋₆ alkylamino, (o) di-C₁₋₆ alkylamino, (p) a 5-membered ring group containing 1 to 4 heteroatoms selected from oxygen, sulfur and nitrogen atoms and the like in addition to carbon atoms (wherein this 5-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (q) a 6-membered ring group containing 1 to 4 heteroatoms selected from oxygen, sulfur and nitrogen atoms and the like in addition to carbon atoms (wherein this 6-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (r) a bicyclic or tricyclic fused ring group containing 1 to 4 heteroatoms selected from oxygen, sulfur and nitrogen atoms and the like in addition to carbon atoms (wherein this bicyclic or tricyclic fused ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (s) C₁₋₆ alkoxycarbonyl, (t) carbamoyl, (u) N-mono-C₁₋₆ alkylcarbamoyl, (v) N,N-di-C₁₋₆ alkylcarbamoyl, and (w) C₁₋₂ alkylenedioxy (hereinafter (a) - (w) are briefly referred to as Substituent Group A), (3) C₂₋₆ alkenyl which may be substituted with 1 to 3 substituents selected from the Substituent Group A, (4) C₂₋₆ alkynyl which may be substituted with 1 to 3 substituents selected from the Substituent Group A, (5) C₃₋₈ cycloalkyl which may be substituted with 1 to 3 substituents selected from the Substituent Group A, (6) C₆₋₁₄ aryl which may be substituted with 1 to 3 substituents selected from the Substituent Group A, or (7) C₇₋₁₄ aralkyl which may be substituted with 1 to 3 substituents selected from the Substituent Group A);
[12] The compound according to [8] above, wherein ring A is a benzene ring substituted with one substituent selected from (a) hydroxy, (b) cyano, (c) carboxyl, (d) mono- or dialkylcarbamoyl, (e) acylamino, (f) alkylamino, and (g) alkoxycarbonyl, or a benzene ring substituted with a hydrocarbon group substituted with a substituent selected from the above substituent groups;
[13] The compound according to [8] above, wherein ring B², ring B⁵, ring B¹¹ and ring B¹⁴, which may be identical or different, are each a benzene ring which may be further substituted with 1 to 3 substituents selected from (a) C₆₋₁₄ aryl which may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen, (b) cyano, (c) hydroxy, (d) carboxy, (e) nitro, (f) C₁₋₆ alkyl which may be substituted with di-C₁₋₆ alkylamino, (g) C₁₋₆ alkoxy, (h) C₁₋₆ alkylcarbonyloxy, (i) C₁₋₆ alkylthio, (j) C₁₋₆ alkylsulfinyl, (k) C₁₋₆ alkylsulfonyl, (1) halogen, (m) amino, (n) mono-C₁₋₆ alkylamino, (o) di-C₁₋₆ alkylamino, (p) a 5-membered ring group containing 1 to 4 heteroatoms selected from oxygen, sulfur and nitrogen atoms and the like in addition to carbon atoms (wherein this 5-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (q) a 6-membered ring group containing 1 to 4 heteroatoms selected from oxygen, sulfur and nitrogen atoms and the like in addition to carbon atoms (wherein this 6-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (r) a bicyclic or tricyclic fused ring group containing 1 to 4 heteroatoms selected from oxygen, sulfur and nitrogen atoms and the like in addition to carbon atoms (wherein this bicyclic or tricyclic fused ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (s) C₁₋₆ alkoxycarbonyl, (t) carbamoyl, (u) N-mono-C₁₋₆ alkylcarbamoyl, (v) N,N-di-C₁₋₆ alkylcarbamoyl, and (w) C₁₋₂ alkylenedioxy (hereinafter (a) - (w) are briefly referred to as Substituent Group A);
   ring B⁷, ring B⁸, ring B¹⁶ and ring B¹⁷, which may be identical or different, are each an aromatic heterocycle which may be further substituted with 1 to 3 substituents selected from the Substituent Group A;
   Z², Z⁵, Z⁷, Z⁸, Z¹¹, Z¹⁴, Z¹⁶ and Z¹⁷, which may be identical or different, are each C₁₋₆ alkylene which may be substituted or a bond;
   R⁶, R¹², R¹⁶, R¹⁸, R²⁶, R³², R³⁶ and R³⁸, which may be identical or different, are each (a) a hydrogen atom, (b) C₁₋₆ alkyl which may be substituted with 1 to 3 substituents selected from the Substituent Group A, (c) C₂₋₆ alkenyl which may be substituted with 1 to 3 substituents selected from the Substituent Group A, (d) C₂₋₆ alkynyl which may be substituted with 1 to 3 substituents selected from the Substituent Group A, (e) C₃₋₈ cycloalkyl which may be substituted with 1 to 3 substituents selected from the Substituent Group A, (f) C₆₋₁₄ aryl which may be substituted with 1 to 3 substituents selected from the Substituent Group A, (g) C₇₋₁₄ aralkyl which may be substituted with 1 to 3 substituents selected from the Substituent Group A, or (h) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A), provided that R¹² is not a hydrogen atom; and
   R⁷, R¹³, R¹⁷, R¹⁹, R²⁷, R³³, R³⁷ and R³⁹, which may be identical or different, are each (a) C₁₋₁₀ alkyl which may be substituted with 1 to 3 substituents selected from the Substituent Group A, (b) C₆₋₁₄ aryl which may be substituted with 1 to 3 substituents selected from the Substituent Group A, or (c) a 5- or 6-membered aromatic ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or
   R⁶ and R⁷ , R¹² and R¹³ ,R¹⁶ and R¹⁷ , R¹⁸ and R¹⁹ , R²⁶ and R²⁷, R³² and R³³, R³⁶ and R³⁷, and R³⁸ and R³⁹ may respectively form a ring together with the adjacent nitrogen atom;
[14] The compound according to [8] above, wherein ring A is a benzene ring, X is an oxygen atom or NH, R¹ is a hydrogen atom or C₁₋₆ alkyl;
   ring B², ring B⁵, ring B¹¹ and ring B¹⁴, which may be identical or different, are each a benzene ring which may be further substituted with one substituent selected from halogen, lower alkyl and cyano;
   ring B⁷, ring B⁸, ring B¹⁶ and ring B¹⁷, which may be identical or different, are each a thiophene ring or a furan ring which may be further substituted;
   Z², Z⁵, Z⁷, Z⁸, Z¹¹, Z¹⁴, Z¹⁶ and Z¹⁷, which may be identical or different, are each C₁₋₆ alkylene which may be substituted or a bond;
   R⁶, R¹², R¹⁶, R¹⁸, R²⁶, R³², R³⁶ and R³⁸, which may be identical or different, are each C₁₋₆ alkyl which may be substituted with cyano; and
   R⁷, R¹³, R¹⁷, R¹⁹, R²⁷, R³³, R³⁷ and R³⁹, which may be identical or different, are each (a) C₁₋₁₀ alkyl which may be substituted with 1 to 3 substituents selected from the Substituent Group A, (b) C₆₋₁₄ aryl which may be substituted with halogen, or (c) pyridyl; or
   R⁶ and R⁷, R¹² and R¹³, R¹⁶ and R¹⁷, R¹⁸ and R¹⁹, R²⁶ and R²⁷, R³² and R³³, R³⁶ and R³⁷, and R³⁸ and R³⁹ may respectively form, together with the adjacent nitrogen atom, (1) 1,2,3,4-tetrahydroquinolin-1-yl which may be substituted with C₁₋₆ alkyl, halogen or C₁₋₆ alkoxy, (2) 2,3-dihydro-1H-indol-1-yl which may be substituted with C₁₋₆ alkyl, (3) 2,3,4,5-tetrahydro-1H-1-benzazepin-1-yl which may be substituted with C₁₋₆ alkyl, or (4) 3,4-dihydro-2H-1,4-benzoxazin-1-yl;
[15] The compound according to [13] or [14] above,
   wherein Z², Z⁵, Z⁷, Z⁸, Z¹¹, Z¹⁴, Z¹⁶ and Z¹⁷ are each a bond;
[16] The compound according to [9] above, wherein R² is (1) a group represented by the formula: 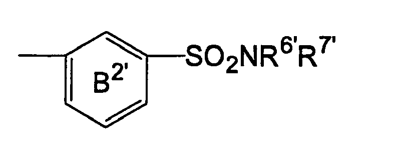 [wherein ring B^{2'} is a benzene ring which may be further substituted with 1 to 3 substituents selected from (a) C₆₋₁₄ aryl which may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen, (b) cyano, (c) hydroxy, (d) carboxy, (e) nitro, (f) C₁₋₆ alkyl which may be substituted with di-C₁₋₆ alkylamino, (g) C₁₋₆ alkoxy, (h) C₁₋₆ alkyl-carbonyloxy, (i) C₁₋₆ alkylthio, (j) C₁₋₆ alkylsulfinyl, (k) C₁₋₆ alkylsulfonyl, (1) halogen, (m) amino, (n) mono-C₁₋₆ alkylamino, (o) di-C₁₋₆ alkylamino, (p) a 5-membered ring group containing 1 to 4 heteroatoms selected from oxygen, sulfur and nitrogen atoms and the like in addition to carbon atoms (wherein this 5-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (q) a 6-membered ring group containing 1 to 4 heteroatoms selected from oxygen, sulfur and nitrogen atoms and the like in addition to carbon atoms (wherein this 6-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (r) a bicyclic or tricyclic fused ring group containing 1 to 4 heteroatoms selected from oxygen, sulfur and nitrogen atoms and the like in addition to carbon atoms (wherein this bicyclic or tricyclic fused ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (s) C₁₋₆ alkoxycarbonyl, (t) carbamoyl, (u) N-mono-C₁₋₆ alkylcarbamoyl, (v) N,N-di-C₁₋₆ alkylcarbamoyl, and (w) C₁₋₂ alkylenedioxy (hereinafter (a) - (w) are briefly referred to as Substituent Group A);
   R⁶' is (a) a hydrogen atom, (b) C₁₋₆ alkyl which may be substituted with 1 to 3 substituents selected from the Substituent Group A, (c) C₂₋₆ alkenyl which may be substituted with 1 to 3 substituents selected from the Substituent Group A, (d) C₂₋₆ alkynyl which may be substituted with 1 to 3 substituents selected from the Substituent Group A, (e) C₃₋₈ cycloalkyl which may be substituted with 1 to 3 substituents selected from the Substituent Group A, (f) C₆₋₁₄ aryl which may be substituted with 1 to 3 substituents selected from the Substituent Group A, (g) C₇₋₁₄ aralkyl which may be substituted with 1 to 3 substituents selected from the Substituent Group A, or (h) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); and
   R^{7'} is (a) C₆₋₁₄ aryl which may be substituted with 1 to 3 substituents selected from the Substituent Group A, or (b) a 5-or 6-membered aromatic ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or
   R^{6'} and R⁷' may form a ring together with the adjacent nitrogen atom],
(2) a group represented by the formula: 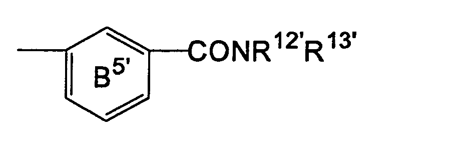 [wherein ring B⁵' is a benzene ring which may be further substituted with 1 to 3 substituents selected from the Substituent Group A;
   R^{12'} is (a) C₁₋₆ alkyl which may be substituted with 1 to 3 substituents selected from the Substituent Group A, (b) C₂₋₆ alkenyl which may be substituted with 1 to 3 substituents selected from the Substituent Group A, (c) C₂₋₆ alkynyl which may be substituted with 1 to 3 substituents selected from the Substituent Group A, (d) C₃₋₈ cycloalkyl which may be substituted with 1 to 3 substituents selected from the Substituent Group A, (e) C₆₋₁₄ aryl which may be substituted with 1 to 3 substituents selected from the Substituent Group A, (f) C₇₋₁₄ aralkyl which may be substituted with 1 to 3 substituents selected from the Substituent Group A, or (g) a 5-or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); and
   R^{13'} is (a) C₆₋₁₄ aryl which may be substituted with 1 to 3 substituents selected from the Substituent Group A, or (b) a 5-or 6-membered aromatic ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or
   R¹²' and R^{13'} may form a ring together with the adjacent nitrogen atom], or
(3) a group represented by the formula: 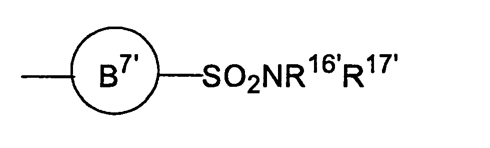 [wherein ring B⁷ is an aromatic heterocycle which may be further substituted with 1 to 3 substituents selected from the Substituent Group A;
   R¹⁶' is (a) a hydrogen atom, (b) C₁₋₆ alkyl which may be substituted with 1 to 3 substituents selected from the Substituent Group A, (c) C₂₋₆ alkenyl which may be substituted with 1 to 3 substituents selected from the Substituent Group A, (d) C₂₋₆ alkynyl which may be substituted with 1 to 3 substituents selected from the Substituent Group A, (e) C₃₋₈ cycloalkyl which may be substituted with 1 to 3 substituents selected from the Substituent Group A, (f) C₆₋₁₄ aryl which may be substituted with 1 to 3 substituents selected from the Substituent Group A, (g) C₇₋₁₄ aralkyl which may be substituted with 1 to 3 substituents selected from the Substituent Group A, or (h) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); and
   R¹⁷' is (a) C₆₋₁₄ aryl which may be substituted with 1 to 3 substituents selected from the Substituent Group A, or (b) a 5-or 6-membered aromatic ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or
   R^{16'} and R¹⁷' may form a ring together with the adjacent nitrogen atom];
[17] The compound according to [9] above, wherein ring A is a benzene ring, X is an oxygen atom or NH, R¹ is a hydrogen atom or C₁₋₆ alkyl, and R² is
   (1) a group represented by the formula: 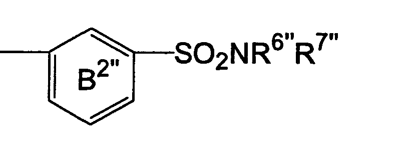 [wherein ring B²" is a benzene ring which may be further substituted with halogen, R⁶" is C₁₋₆ alkyl which may be substituted with cyano, and R⁷" is C₆₋₁₄ aryl or pyridyl which may be substituted with halogen, or R^{6"} and R⁷" form, together with the adjacent nitrogen atom, (1) 1,2,3,4-tetrahydroquinolin-1-yl which may be substituted with C₁₋₆ alkyl, halogen or C₁₋₆ alkoxy, (2) 2,3-dihydro-1H-indol-1-yl which may be substituted with C₁₋₆ alkyl, (3) 2,3,4,5-tetrahydro-1H-1-benzazepin-1-yl which may be substituted with C₁₋₆ alkyl, or (4) 3,4-dihydro-2H-1,4-benzoxazin-1-yl];
   (2) a group represented by the formula: 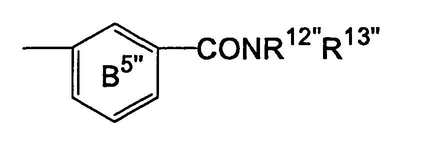 [wherein ring B^{5"} is a benzene ring which may be further substituted with halogen, R^{12"} is C₁₋₆ alkyl which may be substituted with cyano, and R^{13"} is C₆₋₁₄ aryl or pyridyl which may be substituted with halogen, or R^{12"} and R^{13"} form, together with the adjacent nitrogen atom, (1) 1,2,3,4-tetrahydroquinolin-1-yl which may be substituted with C₁₋₆ alkyl, halogen or C₁₋₆ alkoxy, (2) 2,3-dihydro-1H-indol-1-yl which may be substituted with C₁₋₆ alkyl, (3) 2,3,4,5-tetrahydro-1H-1-benzazepin-1-yl which may be substituted with C₁₋₆ alkyl, or (4) 3,4-dihydro-2H-1,4-benzoxazin-1-yl]; or
   (3) a group represented by the formula: 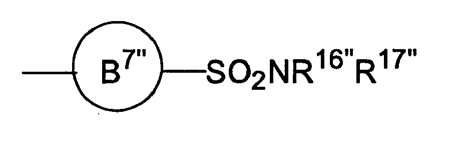 [wherein ring B^{7"} is a thiophene ring which may be further substituted with halogen, R^{16"} is C₁₋₆ alkyl which may be substituted with cyano, and R^{17"} is C₆₋₁₄ aryl or pyridyl which may be substituted with halogen, or R^{16"} and R^{17"} form, together with the adjacent nitrogen atom, (1) 1,2,3,4-tetrahydroquinolin-1-yl which may be substituted with C₁₋₆ alkyl, halogen or C₁₋₆ alkoxy, (2) 2,3-dihydro-1H-indol-1-yl which may be substituted with C₁₋₆ alkyl, (3) 2,3,4,5-tetrahydro-1H-1-benzazepin-1-yl which may be substituted with C₁₋₆ alkyl, or (4) 3,4-dihydro-2H-1,4-benzoxazin-1-yl];
[18] 3-(2-chloro-5-(3,4-dihydroquinolin-1(2H)-ylsulfonyl)phenyl)quinazoline-2,4(1H,3H)-dione,
   4-chloro-3-(2,4-dioxo-1,4-dihydro-3(2H)-quinazolinyl)-N-methyl-N-2-pyridinylbenzenesulfonamide trifluoroacetate,
   3-(2-chloro-5-(2,3,4,5-tetrahydro-1H-1-benzazepin-1-ylsulfonyl)phenyl)-2,4(1H,3H)-quinazolinedione,
   3-(2-chloro-5-(3,4-dihydro-1,5-benzoxazepin-5(2H)-ylsulfonyl)phenyl)-2,4(1H,3H)-quinazolinedione,
   4-chloro-3-(4-imino-2-oxo-1,4-dihydro-3(2H)-quinazolinyl)-N-methyl-N-phenylbenzenesulfonamide trifluoroacetate, or
   3-(2-chloro-5-(3,4-dihydro-1(2H)-quinolinylsulfonyl)phenyl)-4-imino-3,4-dihydro-2(1H)-quinazolinone trifluoroacetate;
[19] 3-[5-(3,4-dihydroquinolin-1(2H)-ylsulfonyl)-2-fluorophenyl]quinazoline-2,4(1H,3H)-dione,
   3-[2-chloro-5-(5,6,7,8-tetrahydro-4H-thieno[3,2-b]azepin-4-ylsulfonyl)phenyl]quinazoline-2,4(1H,3H)-dione,
   4-chloro-3-(2,4-dioxo-1,4-dihydroquinazolin-3(2H)-yl)-N-methyl-N-phenylbenzamide,
   5-chloro-4-[5-(hydroxymethyl)-2,4-dioxo-1,4-dihydroquinazolin-3(2H)-yl]-N-methyl-N-phenylthiophene-2-carboxamide,
   Methyl 3-[2-chloro-5-(3,4-dihydroquinolin-1(2H)-ylsulfonyl)phenyl]-2,4-dioxo-1,2,3,4-tetrahydroquinazoline-5-carboxylate,
   3-[2-chloro-5-(3,4-dihydroquinolin-1(2H)-ylsulfonyl)phenyl]-2,4-dioxo-1,2,3,4-tetrahydroquinazoline-5-carboxylic acid,
   3-[2-chloro-5-(3,4-dihydroquinolin-1(2H)-ylsulfonyl)phenyl]-5-(hydroxymethyl)quinazoline-2,4 (1H,3H)-dione,
   3-(2,4-dioxo-1,4-dihydroquinazolin-3(2H)-yl)-5-(2,3,4,5-tetrahydro-1H-1-benzazepin-1-ylsulfonyl)thiophene-2-carbonitrile,
   4-chloro-3-[5-(hydroxymethyl)-2,4-dioxo-1,4-dihydroquinazolin-3(2H)-yl]-N-methyl-N-phenylbenzamide,
   4-chloro-3-[5-(hydroxymethyl)-2,4-dioxo-1,4-dihydroquinazolin-3(2H)-yl]-N-methyl-N-pyridin-2-ylbenzamide,
   3-[2-chloro-5-(2,3,4,5-tetrahydro-1H-1-benzazepin-1-ylsulfonyl)-3-thienyl]-5-(hydroxymethyl)quinazoline-2,4(1H,3H)-dione,
   5-chloro-N-cyclohexyl-4-[5-(hydroxymethyl)-2,4-dioxo-1,4-dihydroquinazolin-3(2H)-yl]-N-methylthiophene-2-carboxamide,
   5-chloro-N-(2-chlorophenyl)-4-[5-(hydroxymethyl)-2,4-dioxo-1,4-dihydroquinazolin-3(2H)-yl]-N-methylthiophene-2-carboxamide,
   5-chloro-N-(3-chlorophenyl)-4-[5-(hydroxymethyl)-2,4-dioxo-1,4-dihydroquinazolin-3(2H)-yl]-N-methylthiophene-2-carboxamide,
   5-chloro-N-(4-chlorophenyl)-4-[5-(hydroxymethyl)-2,4-dioxo-1,4-dihydroquinazolin-3(2H)-yl]-N-methylthiophene-2-carboxamide,
   5-chloro-4-[5-(hydroxymethyl)-2,4-dioxo-1,4-dihydroquinazolin-3(2H)-yl]-N-methyl-N-(2-methylphenyl)thiophene-2-carboxamide,
   5-chloro-4-[5-(hydroxymethyl)-2,4-dioxo-1,4-dihydroquinazolin-3(2H)-yl]-N-methyl-N-(3-methylphenyl)thiophene-2-carboxamide,
   3-[2-chloro-5-(3,4-dihydroquinolin-1(2H)-ylsulfonyl)phenyl]-2,4-dioxo-1,2,3,4-tetrahydroquinazoline-5-carboxamide,
   3-[2-chloro-5-(3,4-dihydroquinolin-1(2H)-ylsulfonyl)phenyl]-3,4-dihydroquinazolin-2 (1H)-one,
   3-[2-chloro-5-(3,4-dihydroquinolin-1(2H)-ylsulfonyl)phenyl]-2-oxo-1,2,3,4-tetrahydroquinazoline-5-carboxylic acid,
   5-chloro-4-[5-(hydroxymethyl)-2,4-dioxo-1,4-dihydroquinazolin-3(2H)-yl]-N-isobutyl-N-methylthiophene-2-carboxamide, or
   3-[2-chloro-5-(3,4-dihydroquinolin-1(2H)-ylsulfonyl)phenyl]-5-(1-hydroxy-1-methylethyl)quinazoline-2,4(1H,3H)-dione;
[20] A prodrug of the compound according to [8] or [9] above;
[21] A medicine which comprises the compound according to [8] or [9] above, or a prodrug thereof;
[22] A prophylactic and/or therapeutic agent for sex hormone-dependent diseases, containing the compound according to [8] above or a prodrug thereof;
[23] A prophylactic and/or therapeutic agent for prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease, infertility, irritable bowel syndrome, prostate cancer, uterine cancer, breast cancer or pituitary tumor, a reproduction regulator, a contraceptive, an ovulation inducing agent, or a preventive agent for post-operative recurrence of sex hormone-dependent cancers, which contains the compound according to [8] above or a prodrug thereof;
[24] A prophylactic and/or therapeutic agent for prostate cancer, uterine cancer, breast cancer or pituitary tumor, or a preventive agent for post-operative recurrence of sex hormone-dependent cancers, which contains the compound according to [8] or [9] above, or a prodrug thereof;
[25] A method for prevention and/or treatment of prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease, infertility, irritable bowel syndrome, prostate cancer, uterine cancer, breast cancer or pituitary tumor, a method for reproduction regulation, a method for contraception, a method for ovulation induction, or a method for prevention of post-operative recurrence of sex hormone-dependent cancers, which comprises administering an effective amount of the compound according to [8] above or a prodrug thereof to a mammal;
[26] A method for prevention and/or treatment of prostate cancer, uterine cancer, breast cancer or pituitary tumor, or a method for prevention of post-operative recurrence of sex hormone-dependent cancers, which comprises administering an effective amount of the compound according to [8] or [9], or a prodrug thereof to a mammal;
[27] Use of the compound according to [8] above or a prodrug thereof for the manufacture of a prophylactic and/or therapeutic agent for prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease, infertility, irritable bowel syndrome, prostate cancer, uterine cancer, breast cancer or pituitary tumor, a reproduction regulator, a contraceptive, an ovulation inducing agent, or a preventive agent for post-operative recurrence of sex hormone-dependent cancers;
[28] Use of the compound according to [8] or [9] above, or a prodrug thereof for the manufacture of a prophylactic and/or therapeutic agent for prostate cancer, uterine cancer, breast cancer or pituitary tumor, or a preventive agent for post-operative recurrence of sex hormone-dependent cancers;
[29] The compound according to [9] above, wherein
   ring A is (1) a benzene ring which may be substituted with 1 to 4 substituents selected from (a) C₆₋₁₄ aryl which may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen, (b) cyano, (c) hydroxy, (d) carboxyl, (e) nitro, (f) C₁₋₆ alkyl which may be substituted with di-C₁₋₆ alkylamino, (g) C₁₋₆ alkoxy, (h) C₁₋₆ alkylcarbonyloxy, (i) C₁₋₆ alkylthio, (j) C₁₋₆ alkylsulfinyl, (k) C₁₋₆ alkylsulfonyl, (1) halogen, (m) amino, (n) mono-C₁₋₆ alkylamino, (o) di-C₁₋₆ alkylamino, (p) a 5-membered ring group containing 1 to 4 heteroatoms selected from oxygen, sulfur and nitrogen atoms and the like in addition to carbon atoms (wherein this 5-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (q) a 6-membered ring group containing 1 to 4 heteroatoms selected from oxygen, sulfur and nitrogen atoms and the like in addition to carbon atoms (wherein this 6-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (r) a bicyclic or tricyclic fused ring group containing 1 to 4 heteroatoms selected from oxygen, sulfur and nitrogen atoms and the like in addition to carbon atoms (wherein this bicyclic or tricyclic fused ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (s) C₁₋₆ alkoxycarbonyl, (t) carbamoyl, (u) N-mono-C₁₋₆ alkylcarbamoyl, (v) N,N-di-C₁₋₆ alkylcarbamoyl, and (w) C₁₋₂ alkylenedioxy (hereinafter (a) - (w) are briefly referred to as Substituent Group A), (2) a pyridine ring which may be substituted with 1 to 3 substituents selected from the Substituent Group A, (3) a pyrazine ring which may be substituted with 1 or 2 substituents selected from the Substituent Group A, (4) a pyrimidine ring which may be substituted with 1 or 2 substituents selected from the Substituent Group A, or (5) a pyridazine ring which may be substituted with 1 or 2 substituents selected from the Substituent Group A;
   X is an oxygen atom, a sulfur atom or NR³ (wherein R³ is (1) a hydrogen atom, (2) C₁₋₆ alkyl which may be substituted with 1 to 3 substituents selected from the Substituent Group A, (3) C₂₋₆ alkenyl which may be substituted with 1 to 3 substituents selected from the Substituent Group A, (4) C₂₋₆ alkynyl which may be substituted with 1 to 3 substituents selected from the Substituent Group A, (5) C₃₋₈ cycloalkyl which may be substituted with 1 to 3 substituents selected from the Substituent Group A, (6) C₆₋₁₄ aryl which may be substituted with 1 to 3 substituents selected from the Substituent Group A, or (7) C₇₋₁₄ aralkyl which may be substituted with 1 to 3 substituents selected from the Substituent Group A);
   R¹ is (1) a hydrogen atom, (2) C₁₋₆ alkyl which may be substituted with 1 to 3 substituents selected from the Substituent Group A, (3) C₂₋₆ alkenyl which may be substituted with 1 to 3 substituents selected from the Substituent Group A, (4) C₂₋₆ alkynyl which may be substituted with 1 to 3 substituents selected from the Substituent Group A, (5) C₃₋₈ cycloalkyl which may be substituted with 1 to 3 substituents selected from the Substituent Group A, (6) C₆₋₁₄ aryl which may be substituted with 1 to 3 substituents selected from the Substituent Group A, or (7) C₇₋₁₄ aralkyl which may be substituted with 1 to 3 substituents selected from the Substituent Group A;
   R² is (1) a group represented by the formula: 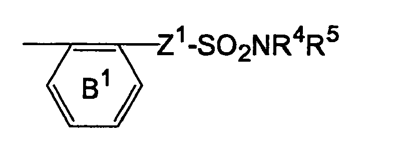 [wherein ring B¹ is a benzene ring which may be further substituted with 1 to 3 substituents selected from the Substituent Group A; Z¹ is C₁₋₆ alkylene which may be substituted with 1 to 3 substituents selected from the Substituent Group A, or a bond; R⁴ is (a) a hydrogen atom, (b) C₁₋₆ alkyl which may be substituted with 1 to 3 substituents selected from the Substituent Group A, (c) C₂₋₆ alkenyl which may be substituted with 1 to 3 substituents selected from the Substituent Group A, (d) C₂₋₆ alkynyl which may be substituted with 1 to 3 substituents selected from the Substituent Group A, (e) C₃₋₈ cycloalkyl which may be substituted with 1 to 3 substituents selected from the Substituent Group A, (f) C₆₋₁₄ aryl which may be substituted with 1 to 3 substituents selected from the Substituent Group A, (g) C₇₋₁₄ aralkyl which may be substituted with 1 to 3 substituents selected from the Substituent Group A, or (h) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); and R⁵ is (a) C₁₋₆ alkyl which may be substituted with 1 to 3 substituents selected from the Substituent Group A, (b) C₂₋₆ alkenyl which may be substituted with 1 to 3 substituents selected from the Substituent Group A, (c) C₂₋₆ alkynyl which may be substituted with 1 to 3 substituents selected from the Substituent Group A, (d) C₃₋₈ cycloalkyl which may be substituted with 1 to 3 substituents selected from the Substituent Group A, (e) C₆₋₁₄ aryl which may be substituted with 1 to 3 substituents selected from the Substituent Group A, (f) C₇₋₁₄ aralkyl which may be substituted with 1 to 3 substituents selected from the Substituent Group A, or (g) a 5- or 6-membered ring group containing 1 to 4 heteroatoms selected from oxygen, sulfur and nitrogen atoms and the like in addition to carbon atoms (wherein this 5- or 6-membered ring group may be substituted with a substituent selected from the Substituent Group A); or R⁴ and R⁵ may form a ring together with the adjacent nitrogen atom],
(2) a group represented by the formula: 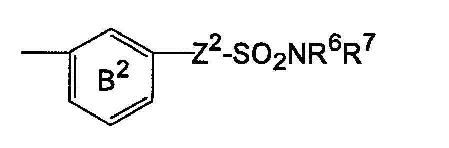 [wherein ring B² is a benzene ring which may be further substituted with 1 to 3 substituents selected from the Substituent Group A; Z² is C₁₋₆ alkylene which may be substituted with 1 to 3 substituents selected from the Substituent Group A, or a bond; R⁶ is (a) a hydrogen atom, (b) C₁₋₆ alkyl which may be substituted with 1 to 3 substituents selected from the Substituent Group A, (c) C₂₋₆ alkenyl which may be substituted with 1 to 3 substituents selected from the Substituent Group A, (d) C₂₋₆ alkynyl which may be substituted with 1 to 3 substituents selected from the Substituent Group A, (e) C₃₋₈ cycloalkyl which may be substituted with 1 to 3 substituents selected from the Substituent Group A, (f) C₆₋₁₄ aryl which may be substituted with 1 to 3 substituents selected from the Substituent Group A, (g) C₇₋₁₄ aralkyl which may be substituted with 1 to 3 substituents selected from the Substituent Group A, or (h) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); and R⁷ is (a) C₁₋₆ alkyl which may be substituted with 1 to 3 substituents selected from the Substituent Group A, (b) C₂₋₆ alkenyl which may be substituted with 1 to 3 substituents selected from the Substituent Group A, (c) C₂₋₆ alkynyl which may be substituted with 1 to 3 substituents selected from the Substituent Group A, (d) C₃₋₈ cycloalkyl which may be substituted with 1 to 3 substituents selected from the Substituent Group A, (e) C₆₋₁₄ aryl which may be substituted with 1 to 3 substituents selected from the Substituent Group A, (f) C₇₋₁₄ aralkyl which may be substituted with 1 to 3 substituents selected from the Substituent Group A, or (g) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or R⁶ and R⁷ may form a ring together with the adjacent nitrogen atom],
(3) a group represented by the formula: 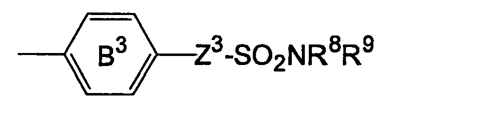 [wherein ring B³ is a benzene ring which may be further substituted with 1 to 3 substituents selected from the Substituent Group A; Z³ is C₁₋₆ alkylene which may be substituted with 1 to 3 substituents selected from the Substituent Group A, or a bond; R⁸ is (a) a hydrogen atom, (b) C₁₋₆ alkyl which may be substituted with 1 to 3 substituents selected from the Substituent Group A, (c) C₂₋₆ alkenyl which may be substituted with 1 to 3 substituents selected from the Substituent Group A, (d) C₂₋₆ alkynyl which may be substituted with 1 to 3 substituents selected from the Substituent Group A, (e) C₃₋₈ cycloalkyl which may be substituted with 1 to 3 substituents selected from the Substituent Group A, (f) C₆₋₁₄ aryl which may be substituted with 1 to 3 substituents selected from the Substituent Group A, (g) C₇₋₁₄ aralkyl which may be substituted with 1 to 3 substituents selected from the Substituent Group A, or (h) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); and R⁹ is (a) C₁₋₆ alkyl which may be substituted with 1 to 3 substituents selected from the Substituent Group A, (b) C₂₋₆ alkenyl which may be substituted with 1 to 3 substituents selected from the Substituent Group A, (c) C₂₋₆ alkynyl which may be substituted with 1 to 3 substituents selected from the Substituent Group A, (d) C₃₋₈ cycloalkyl which may be substituted with 1 to 3 substituents selected from the Substituent Group A, (e) C₆₋₁₄ aryl which may be substituted with 1 to 3 substituents selected from the Substituent Group A, (f) C₇₋₁₄ aralkyl which may be substituted with 1 to 3 substituents selected from the Substituent Group A, or (g) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or R⁸ and R⁹ may form a ring together with the adjacent nitrogen atom,
   provided that when R⁸ is a hydrogen atom, R⁹ is (a) C₆₋₁₄ aryl which may be substituted with 1 to 3 substituents selected from the Substituent Group A, (b) C₇₋₁₄ aralkyl which may be substituted with 1 to 3 substituents selected from the Substituent Group A, or (c) C₁₋₆ alkyl substituted with a heterocyclic group selected from (i) a 5-membered ring group containing 1 to 4 heteroatoms selected from oxygen, sulfur and nitrogen atoms and the like in addition to carbon atoms, (ii) a 6-membered ring group containing 1 to 4 heteroatoms selected from oxygen, sulfur and nitrogen atoms and the like in addition to carbon atoms, and (iii) a bicyclic or tricyclic fused ring group containing 1 to 4 heteroatoms selected from oxygen, sulfur and nitrogen atoms and the like in addition to carbon atoms (wherein this heterocyclic group may be substituted with 1 to 3 substituents selected from (i') hydroxy, (ii') amino, (iii') mono-C₁₋₆ alkylamino, (iv') di-C₁₋₆ alkylamino, (v') C₁₋₆ alkoxy, and (vi') halogen)],
(4) a group represented by the formula: 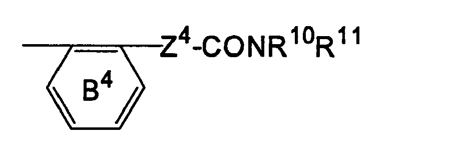 [wherein ring B⁴ is a benzene ring which may be further substituted with 1 to 3 substituents selected from the Substituent Group A; Z⁴ is C₁₋₆ alkylene which may be substituted with 1 to 3 substituents selected from the Substituent Group A, or a bond; R¹⁰ is (a) a hydrogen atom, (b) C₁₋₆ alkyl which may be substituted with 1 to 3 substituents selected from the Substituent Group A, (c) C₂₋₆ alkenyl which may be substituted with 1 to 3 substituents selected from the Substituent Group A, (d) C₂₋₆ alkynyl which may be substituted with 1 to 3 substituents selected from the Substituent Group A, (e) C₃₋₈ cycloalkyl which may be substituted with 1 to 3 substituents selected from the Substituent Group A, (f) C₆₋₁₄ aryl which may be substituted with 1 to 3 substituents selected from the Substituent Group A, (g) C₇₋₁₄ aralkyl which may be substituted with 1 to 3 substituents selected from the Substituent Group A, or (h) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); and R¹¹ is (a) C₆₋₁₄ aryl which may be substituted with 1 to 3 substituents selected from the Substituent Group A, (b) a 5- or 6-membered aromatic ring group containing 1 to 4 heteroatoms selected from oxygen, sulfur and nitrogen atoms and the like in addition to carbon atoms (wherein this 5- or 6-membered ring may be substituted with a substituents from the Substituent Group A), (c) C₇₋₁₄ aralkyl which may be substituted with 1 to 3 substituents selected from the Substituent Group A, or (d) C₁₋₆ alkyl substituted with a heterocyclic group selected from (i) a 5-membered ring group containing 1 to 4 heteroatoms selected from oxygen, sulfur and nitrogen atoms and the like in addition to carbon atoms, (ii) a 6-membered ring group containing 1 to 4 heteroatoms selected from oxygen, sulfur and nitrogen atoms and the like in addition to carbon atoms, and (iii) a bicyclic or tricyclic fused ring group containing 1 to 4 heteroatoms selected from oxygen, sulfur and nitrogen atoms and the like in addition to carbon atoms (wherein this heterocyclic group may be substituted with 1 to 3 substituents selected from (i') hydroxy, (ii') amino, (iii') mono-C₁₋₆ alkylamino, (iv') di-C₁₋₆ alkylamino, (v') C₁₋₆ alkoxy, and (vi') halogen) ; or R¹⁰ and R¹¹ may form, together with the adjacent nitrogen atom, a heterocyclic ring comprising carbon and nitrogen atoms as ring-constituting moieties],
(5) a group represented by the formula: 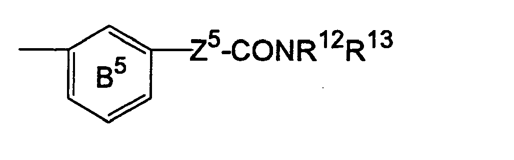 [wherein ring B⁵ is a benzene ring which may be further substituted with 1 to 3 substituents selected from the Substituent Group A; Z⁵ is C₁₋₆ alkylene which may be substituted with 1 to 3 substituents selected from the Substituent Group A, or a bond; and R¹² and R¹³, which may be identical or different, are each (a) a hydrogen atom, (b) C₁₋₆ alkyl which may be substituted with 1 to 3 substituents selected from the Substituent Group A, (c) C₂₋₆ alkenyl which may be substituted with 1 to 3 substituents selected from the Substituent Group A, (d) C₂₋₆ alkynyl which may be substituted with 1 to 3 substituents selected from the Substituent Group A, (e) C₃₋₈ cycloalkyl which may be substituted with 1 to 3 substituents selected from the Substituent Group A, (f) C₆₋₁₄ aryl which may be substituted with 1 to 3 substituents selected from the Substituent Group A, (g) C₇₋₁₄ aralkyl which may be substituted with 1 to 3 substituents selected from the Substituent Group A, or (h) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or R¹² and R¹³ may form a ring together with the adjacent nitrogen atom],
(6) a group represented by the formula: 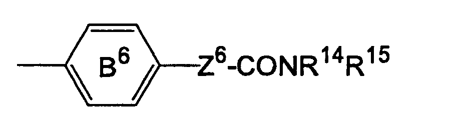 [wherein ring B⁶ is a benzene ring which may be further substituted with 1 to 3 substituents selected from the Substituent Group A; Z⁶ is C₁₋₆ alkylene which may be substituted with 1 to 3 substituents selected from the Substituent Group A, or a bond; and R¹⁴ and R¹⁵, which may be identical or different, are each (a) C₁₋₆ alkyl which may be substituted with 1 to 3 substituents selected from the Substituent Group A, (b) C₂₋₆ alkenyl which may be substituted with 1 to 3 substituents selected from the Substituent Group A, (c) C₂₋₆ alkynyl which may be substituted with 1 to 3 substituents selected from the Substituent Group A, (d) C₃₋₈ cycloalkyl which may be substituted with 1 to 3 substituents selected from the Substituent Group A, (e) C₆₋₁₄ aryl which may be substituted with 1 to 3 substituents selected from the Substituent Group A, (f) C₇₋₁₄ aralkyl which may be substituted with 1 to 3 substituents selected from the Substituent Group A, or (g) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or R¹⁴ and R¹⁵ may form a ring together with the adjacent nitrogen atom],
(7) a group represented by the formula: 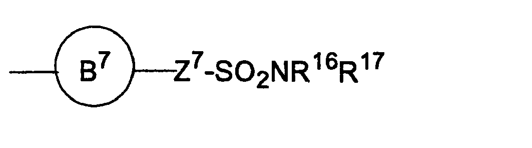 [wherein ring B⁷ is a C₁₀₋₁₄ aryl ring which may be further substituted with 1 to 3 substituents selected from the Substituent Group A, or an aromatic heterocycle which may be further substituted with 1 to 3 substituents selected from the Substituent Group A; Z⁷ is C₁₋₆ alkylene which may be substituted with 1 to 3 substituents selected from the Substituent Group A, or a bond; R¹⁶ is (a) a hydrogen atom, (b) C₁₋₆ alkyl which may be substituted with 1 to 3 substituents selected from the Substituent Group A, (c) C₂₋₆ alkenyl which may be substituted with 1 to 3 substituents selected from the Substituent Group A, (d) C₂₋₆ alkynyl which may be substituted with 1 to 3 substituents selected from the Substituent Group A, (e) C₃₋₈ cycloalkyl which may be substituted with 1 to 3 substituents selected from the Substituent Group A, (f) C₆₋₁₄ aryl which may be substituted with 1 to 3 substituents selected from the Substituent Group A, (g) C₇₋₁₄ aralkyl which may be substituted with 1 to 3 substituents selected from the Substituent Group A, or (h) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); and R¹⁷ is (a) C₁₋₆ alkyl which may be substituted with 1 to 3 substituents selected from the Substituent Group A, (b) C₂₋₆ alkenyl which may be substituted with 1 to 3 substituents selected from the Substituent Group A, (c) C₂₋₆ alkynyl which may be substituted with 1 to 3 substituents selected from the Substituent Group A, (d) C₃₋₈ cycloalkyl which may be substituted with 1 to 3 substituents selected from the Substituent Group A, (e) C₆₋₁₄ aryl which may be substituted with 1 to 3 substituents selected from the Substituent Group A, (f) C₇₋₁₄ aralkyl which may be substituted with 1 to 3 substituents selected from the Substituent Group A, or (g) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or R¹⁶ and R¹⁷ may form a ring together with the adjacent nitrogen atom], or
(8) a group represented by the formula: 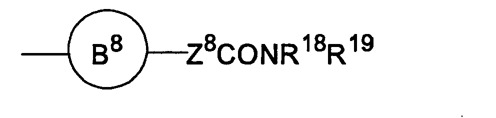 [wherein ring B⁸ is a C₁₀₋₁₄ aryl ring which may be further substituted with 1 to 3 substituents selected from the Substituent Group A, or an aromatic heterocycle which may be further substituted with 1 to 3 substituents selected from the Substituent Group A; Z⁸ is C₁₋₆ alkylene which may be substituted with 1 to 3 substituents selected from the Substituent Group A, or a bond; R¹⁸ is (a) a hydrogen atom, (b) C₁₋₆ alkyl which may be substituted with 1 to 3 substituents selected from the Substituent Group A, (c) C₂₋₆ alkenyl which may be substituted with 1 to 3 substituents selected from the Substituent Group A, (d) C₂₋₆ alkynyl which may be substituted with 1 to 3 substituents selected from the Substituent Group A, (e) C₃₋₈ cycloalkyl which may be substituted with 1 to 3 substituents selected from the Substituent Group A, (f) C₆₋₁₄ aryl which may be substituted with 1 to 3 substituents selected from the Substituent Group A, (g) C₇₋₁₄ aralkyl which may be substituted with 1 to 3 substituents selected from the Substituent Group A, or (h) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); and R¹⁹ is (a) C₁₋₆ alkyl which may be substituted with 1 to 3 substituents selected from the Substituent Group A, (b) C₂₋₆ alkenyl which may be substituted with 1 to 3 substituents selected from the Substituent Group A, (c) C₂₋₆ alkynyl which may be substituted with 1 to 3 substituents selected from the Substituent Group A, (d) C₃₋₈ cycloalkyl which may be substituted with 1 to 3 substituents selected from the Substituent Group A, (e) C₆₋₁₄ aryl which may be substituted with 1 to 3 substituents selected from the Substituent Group A, (f) C₇₋₁₄ aralkyl which may be substituted with 1 to 3 substituents selected from the Substituent Group A, or (g) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or R¹⁸ and R¹⁹ may form a ring together with the adjacent nitrogen atom];
[30] The compound according to [9] above, wherein ring A is a benzene ring which may be substituted with 1 to 4 substituents selected from the Substituent Group A; and
[31] The compound according to [9] above, wherein R² is (1) a group represented by the formula: 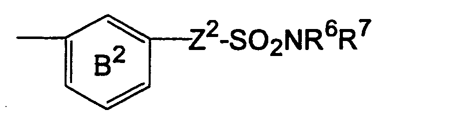 [wherein ring B² is a benzene ring which may be further substituted with 1 to 3 substituents selected from the Substituent Group A; Z² is C₁₋₆ alkylene which may be substituted with 1 to 3 substituents selected from the Substituent Group A, or a bond; R⁶ is (a) a hydrogen atom, (b) C₁₋₆ alkyl which may be substituted with 1 to 3 substituents selected from the Substituent Group A, (c) C₂₋₆ alkenyl which may be substituted with 1 to 3 substituents selected from the Substituent Group A, (d) C₂₋₆ alkynyl which may be substituted with 1 to 3 substituents selected from the Substituent Group A, (e) C₃₋₈ cycloalkyl which may be substituted with 1 to 3 substituents selected from the Substituent Group A, (f) C₆₋₁₄ aryl which may be substituted with 1 to 3 substituents selected from the Substituent Group A, (g) C₇₋₁₄ aralkyl which may be substituted with 1 to 3 substituents selected from the Substituent Group A, or (h) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); and R⁷ is (a) C₁₋₆ alkyl which may be substituted with 1 to 3 substituents selected from the Substituent Group A, (b) C₂₋₆ alkenyl which may be substituted with 1 to 3 substituents selected from the Substituent Group A, (c) C₂₋₆ alkynyl which may be substituted with 1 to 3 substituents selected from the Substituent Group A, (d) C₃₋₈ cycloalkyl which may be substituted with 1 to 3 substituents selected from the Substituent Group A, (e) C₆₋₁₄ aryl which may be substituted with 1 to 3 substituents selected from the Substituent Group A, (f) C₇₋₁₄ aralkyl which may be substituted with 1 to 3 substituents selected from the Substituent Group A, or (g) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or R⁶ and R⁷ may form a ring together with the adjacent nitrogen atom],
(2) a group represented by the formula: 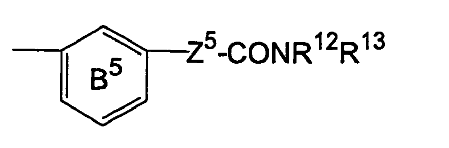 [wherein ring B⁵ is a benzene ring which may be further substituted with 1 to 3 substituents selected from the Substituent Group A; Z⁵ is C₁₋₆ alkylene which may be substituted with 1 to 3 substituents selected from the Substituent Group A, or a bond; and R¹² and R¹³, which may be identical or different, are each (a) a hydrogen atom, (b) C₁₋₆ alkyl which may be substituted with 1 to 3 substituents selected from the Substituent Group A, (c) C₂₋₆ alkenyl which may be substituted with 1 to 3 substituents selected from the Substituent Group A, (d) C₂₋₆ alkynyl which may be substituted with 1 to 3 substituents selected from the Substituent Group A, (e) C₃₋₈ cycloalkyl which may be substituted with 1 to 3 substituents selected from the Substituent Group A, (f) C₆₋₁₄ aryl which may be substituted with 1 to 3 substituents selected from the Substituent Group A, (g) C₇₋₁₄ aralkyl which may be substituted with 1 to 3 substituents selected from the Substituent Group A, or (h) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or R¹² and R¹³ may form a ring together with the adjacent nitrogen atom], or
(3) a group represented by the formula: 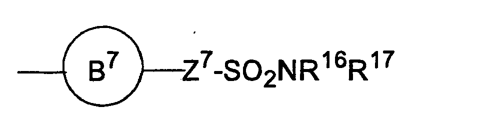 [wherein ring B⁷ is a C₁₀₋₁₄ aryl ring which may be further substituted with 1 to 3 substituents selected from the Substituent Group A, or an aromatic heterocycle which may be further substituted with 1 to 3 substituents selected from the Substituent Group A; Z⁷ is C₁₋₆ alkylene which may be substituted with 1 to 3 substituents selected from the Substituent Group A, or a bond; R¹⁶ is (a) a hydrogen atom, (b) C₁₋₆ alkyl which may be substituted with 1 to 3 substituents selected from the Substituent Group A, (c) C₂₋₆ alkenyl which may be substituted with 1 to 3 substituents selected from the Substituent Group A, (d) C₂₋₆ alkynyl which may be substituted with 1 to 3 substituents selected from the Substituent Group A, (e) C₃₋₈ cycloalkyl which may be substituted with 1 to 3 substituents selected from the Substituent Group A, (f) C₆₋₁₄ aryl which may be substituted with 1 to 3 substituents selected from the Substituent Group A, (g) C₇₋₁₄ aralkyl which may be substituted with 1 to 3 substituents selected from the Substituent Group A, or (h) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); and R¹⁷ is (a) C₁₋₆ alkyl which may be substituted with 1 to 3 substituents selected from the Substituent Group A, (b) C₂₋₆ alkenyl which may be substituted with 1 to 3 substituents selected from the Substituent Group A, (c) C₂₋₆ alkynyl which may be substituted with 1 to 3 substituents selected from the Substituent Group A, (d) C₃₋₈ cycloalkyl which may be substituted with 1 to 3 substituents selected from the Substituent Group A, (e) C₆₋₁₄ aryl which may be substituted with 1 to 3 substituents selected from the Substituent Group A, (f) C₇₋₁₄ aralkyl which may be substituted with 1 to 3 substituents selected from the Substituent Group A, or (g) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or R¹⁶ and R¹⁷ may form a ring together with the adjacent nitrogen atom].

### Detailed Explanation of The Invention

The compound of the invention has an excellent antagonistic activity against gonadotropin releasing hormone. Furthermore, the compound exhibits good oral absorption and is excellent in the aspects of stability and pharmacokinetics. It also has low toxicity and is excellent in view of safety. Therefore, the compound can be used as, for example, a prophylactic or therapeutic agent for hormone-dependent diseases. Specifically, the compound is effective as a medicine such as a prophylactic or therapeutic agent for sex hormone-dependent cancers (e.g., prostate cancer, uterine cancer, breast cancer, pituitary tumor, etc.), prostatomegaly, hysteromyoma, endometriosis, metrofibroma, precocious puberty, amenorrhea (syndrome), premenstrual syndrome, dysmenorrhea, polycystic ovary syndrome, acne, baldness, Alzheimer's disease and the like, or as a reproduction regulator, a birth control agent (e.g., a contraceptive (agent), an ovulation inducing agent, etc.), a prophylactic or therapeutic agent for infertility, a menstrual regulator, a prophylactic and/or therapeutic agent for irritable bowel syndrome, or a preventive agent for post-operative recurrence of sex hormone-dependent cancers. It is also effective as an agent for regulation of animal's estrus, improvement in the table meat quality or regulation of animal growth in the field of stockbreeding, or as a promoting agent for fish spawning in the field of fishery.

### Best Mode for Carrying Out the Invention

In Compound (I), ring A is "a 6-membered aromatic ring which may be further substituted." The "6-membered aromatic ring" of the "6-membered aromatic ring which may be further substituted" refers to a benzene ring, a pyridine ring, a pyrazine ring, a pyrimidine ring, a pyridazine ring or the like. Specifically, Compound (I) is a compound represented by the formulas: 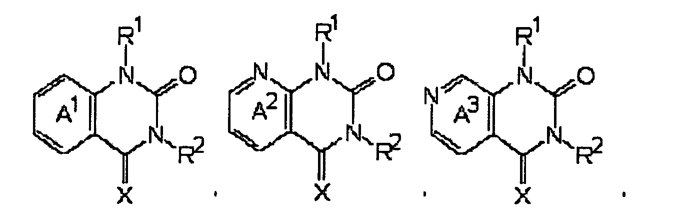 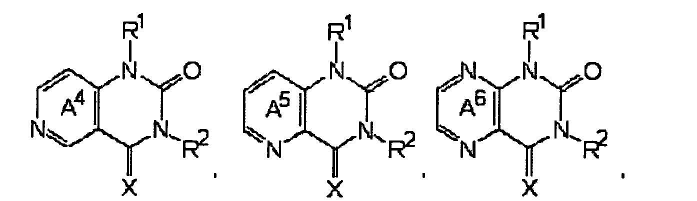 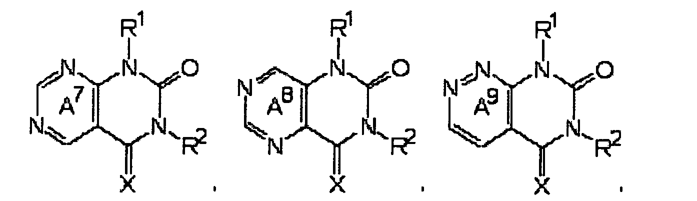 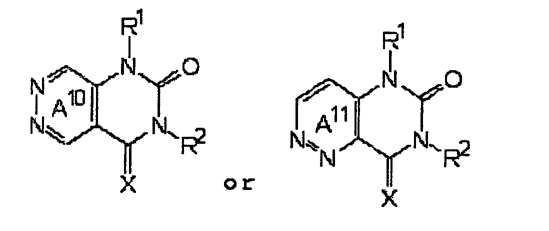 or 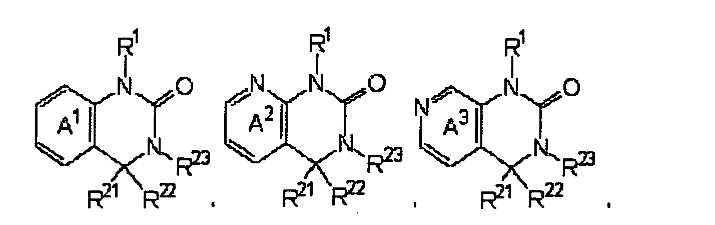 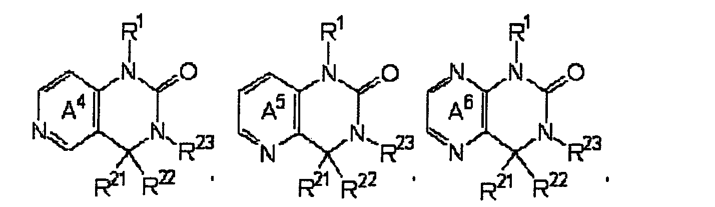 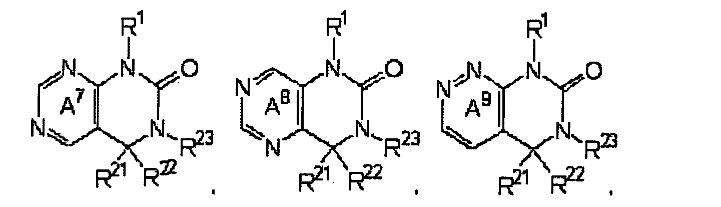 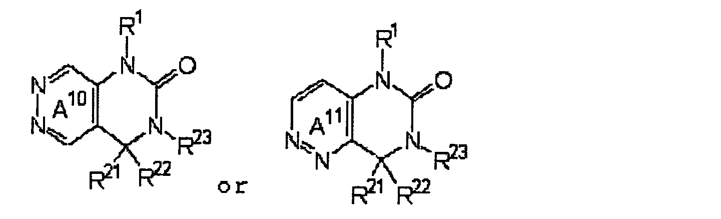 wherein ring A¹ through ring A¹¹ may be substituted, and other reference symbols have the meanings as defined above.

Examples of the substituent which may be further carried by ring A include:
(a) cyano,
(b) hydroxy which may be protected by a protective group (e.g., tert-butyl(dimethyl)silyl [TBDMS], etc.),
(c) carboxyl which may be protected by a protective group (e.g., trimethylsilylethyl [TMS-CH₂CH₂-], etc.),
(d) nitro,
(e) a hydrocarbon group
   [wherein the hydrocarbon group may be exemplified by a hydrocarbon group having 1 to 20 carbon atoms such as an alkyl group, a cycloalkyl group, an alkenyl group, a cycloalkenyl group, an alkynyl group, an aralkyl group or an aryl group.
   The "alkyl group" that can be used may be exemplified by a "straight-chained or branched C₁₋₁₅ alkyl group" such as methyl, ethyl, propyl, isopropyl, butyl, isobutyl, sec-butyl, tert-butyl, pentyl, hexyl, heptyl, octyl, nonyl, decyl, undecyl, tridecyl, tetradecyl or pentadecyl. Preferably, the "alkyl group" is C₁₋₆ alkyl (e.g., methyl, ethyl, propyl, isopropyl, butyl, isobutyl, sec-butyl, tert-butyl, pentyl, hexyl, etc.).
   The "cycloalkyl group" that can be used may be exemplified by a "C₃₋₈ cycloalkyl group" such as cyclopropyl, cyclobutyl, cyclopentyl, cyclohexyl, cycloheptyl or cyclooctyl.
   The "alkenyl group" that can be used may be exemplified by a "C₂₋₁₈ alkenyl group" such as vinyl, allyl, isopropenyl, 3-butenyl, 3-octenyl or 9-octadecenyl.
   The "cycloalkenyl group" that can be used may be exemplified by a "C₃₋₈ cycloalkenyl group" such as cyclopropenyl, cyclobutenyl, cyclopentenyl, cyclohexenyl, cycloheptenyl or cyclooctenyl.
   The "alkynyl group" that can be used may be exemplified by a "C₂₋₁₈ alkynyl group" such as ethynyl, propynyl, 1-butynyl, 2-butynyl, 1-pentynyl, 2-pentynyl or 3-pentynyl.
   The "aralkyl group" may be exemplified by C₇₋₁₄ aralkyl, for example, a phenyl-C₁₋₆ alkyl group such as benzyl, phenethyl, 3-phenylpropyl or 4-phenylbutyl, and a naphthyl-C₁₋₆ alkyl group such as (1-naphthyl)methyl, 2-(1-naphthyl)ethyl or 2-(2-naphthyl)ethyl.
   The "aryl group" that can be used may be exemplified by an aromatic monocyclic, bicyclic or tricyclic C₆₋₁₄ aryl group such as phenyl, 1-naphthyl, 2-naphthyl, phenanthryl or anthryl.],
(f) C₁₋₆ alkoxy (e.g., methoxy, ethoxy, propoxy, isopropoxy, butoxy, isobutoxy, sec-butoxy, tert-butoxy, pentyloxy, hexyloxy, etc.),
(g) C₆₋₁₄ aryloxy (e.g., phenyloxy, 1-naphthyloxy, 2-naphthyloxy, phenanthryloxy, anthryloxy, etc.),
(h) heterocyclic oxy
   [wherein the heterocyclic ring bound to the oxy may be exemplified by an aromatic heterocycle or a non-aromatic heterocycle.
   The aromatic heterocycle may be exemplified by a 5- to 7-membered monocyclic aromatic heterocycle or fused aromatic heterocycle containing 1 to 4 heteroatoms selected from oxygen, sulfur and nitrogen atoms in addition to carbon atoms, as the ring-constituting atom. The fused aromatic heterocycle may be exemplified by a ring in which one of these 5- to 7-membered monocyclic aromatic heterocycles is fused with a 6-membered ring containing 1 or 2 nitrogen atoms, a benzene ring or a 5-membered ring containing 1 sulfur atom, or the like. Suitable examples of the aromatic heterocycle include furan, thiophene, pyridine, pyrimidine, pyridazine, pyrazine, pyrrole, imidazole, pyrazole, isoxazole, isothiazole, oxazole, thiazole, oxadiazole, thiadiazole, triazole, tetrazole, quinoline, quinazoline, quinoxaline, benzofuran, benzothiophene, benzoxazole, benzothiazole, benzimidazole, indole, 1H-indazole, 1H-pyrrolo[2,3-b]pyrazine, 1H-pyrrolopyridine, 1H-imidazopyridine, 1H-imidazopyrazine, triazine, isoquinoline, benzothiadiazole and the like. The aromatic heterocycle is preferably a 5- or 6-membered aromatic heterocycle, and more preferably furan, thiophene, pyridine, pyrimidine, pyrazole, oxazole, thiazole or the like.
   The non-aromatic heterocycle may be exemplified by a 5- to 7-membered monocyclic non-aromatic heterocycle or fused non-aromatic heterocycle containing 1 to 4 heteroatoms selected from oxygen, sulfur and nitrogen atoms in addition to carbon atoms as the ring-constituting atom. The non-aromatic fused heterocyclic ring may be exemplified by a ring in which one of these 5- to 7-membered monocyclic non-aromatic heterocycles is fused with a 6-membered ring containing 1 or 2 nitrogen atom, a benzene ring or a 5-membered ring containing 1 sulfur atom, or the like. Suitable examples of the non-aromatic heterocycle include pyrrolidine, pyrroline, pyrazolidine, piperidine, piperazine, morpholine, thiomorpholine, piperazine, hexamethyleneimine, oxazolidine, thiazolidine, imidazolidine, imidazoline, tetrahydrofuran, azepane, tetrahydropyridine and the like.],
(i) C₃₋₆ alkyl-carbonyloxy (e.g., methylcarbonyloxy, ethylcarbonyloxy, propylcarbonyloxy, isopropylcarbonyloxy, butylcarbonyloxy, isobutylcarbonyloxy, sec-butylcarbonyloxy, tert-butylcarbonyloxy, pentylcarbonyloxy, hexylcarbonyloxy, etc.),
(j) C₁₋₆ alkylthio (e.g., methylthio, ethylthio, propylthio, isopropylthio, butylthio, isobutylthio, sec-butylthio, tert-butylthio, pentylthio, hexylthio, etc.),
(k) C₆₋₁₄ arylthio (e.g., phenylthio, 1-naphthylthio, 2-naphthylthio, phenanthrylthio, anthrylthio, etc.),
(1) heterocyclic thio
   [wherein the heterocyclic ring bound to the thio may be exemplified by the heterocyclic rings listed for the heterocyclic rings bound to the oxy in the above-described "heterocyclic oxy"],
(m) C₁₋₆ alkylsulfinyl (e.g., methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, hexylsulfinyl, etc.),
(n) C₆₋₁₄ arylsulfinyl (e.g. , phenylsulfinyl, 1-naphthylsulfinyl, 2-naphthylsulfinyl, phenanthrylsulfinyl, anthrylsulfinyl, etc.),
(o) heterocyclic sulfinyl
   [wherein the heterocyclic ring bound to the sulfinyl may be exemplified by the heterocyclic rings listed for the heterocyclic rings bound to the oxy in the above-described "heterocyclic oxy"],
(p) C₁₋₆ alkylsulfonyl (e.g., methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, hexylsulfonyl, etc.),
(q) C₆₋₁₄ arylsulfonyl (e. g. , phenylsulfonyl, 1-naphthylsulfonyl, 2-naphthylsulfonyl, phenanthrylsulfonyl, anthrylsulfonyl, etc.),
(r) heterocyclic sulfonyl
   [wherein the heterocyclic ring bound to the sulfonyl may be exemplified by the heterocyclic rings listed for the heterocyclic rings bound to the oxy in the above-described "heterocyclic oxy"],
(s) halogen (e.g., fluorine, chlorine, bromine, iodine, etc.),
(t) amino which may be protected by C₁₋₂₀ acyl (when the amino is protected by an acyl, it may be briefly referred to as C₁₋₂₀ acylamino)
   [wherein the "C₁₋₂₀ acyl" that can be used may be exemplified by a C₁₋₂₀ acyl group obtained by removing an OH group from a carboxylic acid such as R^{1'} COOH or R^{1'} OCOOH, a sulfonic acid such as R^{1'} SO₃H, a sulfinic acid such as R^{1'} SO₂H, a carbamic acid such as R^{1'} N (R^{2'}) COOH (wherein R^{1'} is a hydrogen atom, a hydrocarbon group which may be substituted, or a heterocyclic group which may be substituted, and R^{2'} is a hydrocarbon group which may be substituted or a hydrogen atom), or the like. Specifically, the "C₁₋₂₀ acyl" may be R^{1'}CO-, R^{1'}OCO-, R^{1'}SO₂-, R^{1'}SO-, R^{1'}N(R^{2'})CO- (wherein R^{1'} is a hydrocarbon group which may be substituted, or a heterocyclic group which may be substituted, and R^{2'} is a hydrocarbon group which may be substituted or a hydrogen atom).
   The "hydrocarbon group which may be substituted" represented by R^{1'} and R^{2'} may be exemplified by the "hydrocarbon groups" mentioned as the "substituent which may be further carried by ring A," or the like.
   The "heterocyclic group which may be substituted" represented by R^{1'} may be exemplified by the heterocyclic rings listed for the heterocyclic rings bound to the oxy in the "heterocyclic oxy" mentioned as the "substituent which may be further carried by ring A," or the like.],
(u) mono-C₁₋₆ alkylamino (e.g., methylamino, ethylamino, propylamino, isopropylamino, butylamino, isobutylamino, sec-butylamino, tert-butylamino, pentylamino, hexylamino, etc.),
(v) di-C₁₋₆ alkylamino (e.g., dimethylamino, diethylamino, dipropylamino, diisopropylamino, butylamino, dibutylamino, etc.),
(w) a 5-membered ring group containing 1 to 4 heteroatoms selected from oxygen, sulfur and nitrogen atoms and the like in addition to carbon atoms (e.g., 2-thienyl, 3-thienyl, 2-furyl, 3-furyl, 2-pyrrolyl, 3-pyrrolyl, 2-oxazolyl, 4-oxazolyl, 5-oxazolyl, 2-thiazolyl, 4-thiazolyl, 5-thiazolyl, 3-pyrazolyl, 4-pyrazolyl, 5-pyrazolyl, 2-imidazolyl, 4-imidazolyl, 5-imidazolyl, 3-isoxazolyl, 4-isoxazolyl, 5-isoxazolyl, 3-isothiazolyl, 4-isothiazolyl, 5-isothiazolyl, 3-(1,2,4-oxadiazolyl), 5-(1,2,4-oxadiazolyl), 1,3,4-oxadiazolyl, 3-(1,2,4-thiadiazolyl), 5-(1,2,4-thiadiazolyl), 1,3,4-thiadiazolyl, 4-(1,2,3-thiadiazolyl), 5-(1,2,3-thiadiazolyl), 1,2,5-thiadiazolyl, 1,2,3-triazolyl, 1,2,4-triazolyl, 1H-tetrazolyl, 2H-tetrazolyl, oxoimidazinyl, dioxotriazinyl, pyrrolidinyl, etc.) (wherein this 5-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen),
(x) a 6-membered ring group containing 1 to 4 heteroatoms selected from oxygen, sulfur and nitrogen atoms and the like in addition to carbon atoms (e.g., 2-pyridyl, 3-pyridyl, 4-pyridyl, N-oxido-2-pyridyl, N-oxido-3-pyridyl, N-oxido-4-pyridyl, 2-pyrimidinyl, 4-pyrimidinyl, 5-pyrimidinyl, N-oxido-2-pyrimidinyl, N-oxido-4-pyrimidinyl, N-oxido-5-pyrimidinyl, 2-thiomorpholinyl, 3-thiomorpholinyl, 2-morpholinyl, 3-morpholinyl, piperidinyl, pyranyl, thiopyranyl, 1,4-oxazinyl, 1,4-thiazinyl, 1,3-thiazinyl, 2-piperazinyl, 3-piperazinyl, triazinyl, oxotriazinyl, 3-pyridazinyl, 4-pyridazinyl, pyrazinyl, N-oxido-3-pyridazinyl, N-oxido-4-pyridazinyl, etc.) (wherein this 6-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen),
(y) a bicyclic or tricyclic fused ring group containing 1 to 4 heteroatoms selected from oxygen, sulfur and nitrogen atoms in addition to carbon atoms (e.g., benzofuryl, benzothiazolyl, benzoxazolyl, tetrazolo[1,5-b]pyridazinyl, triazolo[4,5-b]pyridazinyl, benzoimidazolyl, quinolyl, isoquinolyl, cinnolinyl, phthalazinyl, quinazolinyl, quinoxalinyl, indolizinyl, quinolizinyl, 1,8-naphthyridinyl, purinyl, pteridinyl, dibenzofuranyl, carbazolyl, acridinyl, phenanthridinyl, chromanyl, benzoxazinyl, phenazinyl, phenothiazinyl, phenoxazinyl, etc.) (wherein this bicyclic or tricyclic fused ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen),
(z) C₁₋₆ alkoxycarbonyl (e.g., methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl, etc.),

(A) carbamoyl,
(B) N-mono-C₁₋₆ alkylcarbamoyl (e.g., methylcarbamoyl, ethylcarbamoyl, propylcarbamoyl, isopropylcarbamoyl, butylcarbamoyl, isobutylcarbamoyl, sec-butylcarbamoyl, tert-butylcarbamoyl, pentylcarbamoyl, hexylcarbamoyl, etc.) [the C₁₋₆ alkyl moiety may be substituted with 1 to 5, preferably 1 or 2, substituents selected from (1) C₁₋₆ alkoxy (e.g., methoxy, ethoxy, propoxy, isopropoxy, butoxy, isobutoxy, sec-butoxy, tert-butoxy, pentyloxy, hexyloxy, etc.), (2) phenyl, (3) amino, (4) di-C₁₋₆ alkylamino (e.g., dimethylamino, diethylamino, dipropylamino, diisopropylamino, butylamino, dibutylamino, etc.), (5) hydroxy, (6) carboxy, (7) C₁₋₆ alkoxycarbonyl (e.g., tert-butoxycarbonyl, etc.) and the like],
(C) N,N-di-C₁₋₆ alkylcarbamoyl (e.g., dimethylcarbamoyl, diethylcarbamoyl, dipropylcarbamoyl, diisopropylcarbamoyl, butylcarbamoyl, dibutylcarbamoyl, etc.),
(D) N-mono-C₆₋₁₄ arylcarbamoyl (e.g. , phenylcarbamoyl, 1-naphthylcarbamoyl, 2-naphthylcarbamoyl, phenanthrylcarbamoyl, anthrylcarbamoyl, etc.),
(E) N-monoheterocyclic carbamoyl
   [wherein the heterocyclic ring bound to the nitrogen atom of the carbamoyl may be exemplified by the heterocyclic rings listed for the heterocyclic rings bound to the oxy of the "heterocyclic oxy"],
(F) N,N-di-C₆₋₁₄ arylcarbamoyl (e.g., diphenylcarbamoyl, di (1-naphthyl) carbamoyl, di(2-naphthyl)carbamoyl, di (phenanthryl) carbamoyl , di (anthryl) carbamoyl, N-phenyl-N-(1-naphthyl)carbamoyl, etc.),
(G) N,N-diheterocyclic carbamoyl
   [wherein the heterocyclic ring bound to the nitrogen atom of the carbamoyl may be exemplified by the heterocyclic rings listed for the heterocyclic rings bound to the oxy of the "heterocyclic oxy"],
(H) N-C₆₋₁₄ aryl-N-heterocyclic carbamoyl
   [wherein the heterocyclic ring bound to the nitrogen atom of the carbamoyl may be exemplified by the heterocyclic rings listed for the heterocyclic rings bound to the oxy of the "heterocyclic oxy", and the aryl bound to the nitrogen atom of the carbamoyl may be exemplified by phenyl, 1-naphthyl, 2-naphthyl, phenanthryl, anthryl or the like],
(I) C₁₋₂ alkylenedioxy (e.g., methylenedioxy, ethylenedioxy),
(J) oxo,
(K) formyl,
(L) C₁₋₆ alkyl-carbonyl (e.g., acetyl, ethylcarbonyl, propylcarbonyl, isopropylcarbonyl, butylcarbonyl, isobutylcarbonyl, sec-butylcarbonyl, tert-butylcarbonyl, pentylcarbonyl, hexylcarbonyl, etc.),
(M) C₆₋₁₄ arylcarbonyl (e.g., benzoyl, 1-naphthylcarbonyl, 2-naphthylcarbonyl, phenanthrylcarbonyl, anthrylcarbonyl, etc.),
(N) a 5-membered ring containing 1 to 4 heteroatoms selected from oxygen, sulfur and nitrogen atoms and the like in addition to carbon atoms (e.g., 2-thienyl, 3-thienyl, 2-furyl, 3-furyl, 2-pyrrolyl, 3-pyrrolyl, 2-oxazolyl, 4-oxazolyl, 5-oxazolyl, 2-thiazolyl, 4-thiazolyl, 5-thiazolyl, 3-pyrazolyl, 4-pyrazolyl, 5-pyrazolyl, 2-imidazolyl, 4-imidazolyl, 5-imidazolyl, 3-isoxazolyl, 4-isoxazolyl, 5-isoxazolyl, 3-isothiazolyl, 4-isothiazolyl, 5-isothiazolyl, 3-(1,2,4-oxadiazolyl), 5-(1,2,4-oxadiazolyl), 1,3,4-oxadiazolyl, 3-(1,2,4-thiadiazolyl), 5-(1,2,4-thiadiazolyl), 1,3,4-thiadiazolyl, 4-(1,2,3-thiadiazolyl), 5-(1,2,3-thiadiazolyl), 1,2,5-thiadiazolyl, 1,2,3-triazolyl, 1,2,4-triazolyl, 1H-tetrazolyl, 2H-tetrazolyl, oxoimidazinyl, dioxotriazinyl, pyrrolidinyl, etc.)-carbonyl,
(O) a 6-membered ring containing 1 to 4 heteroatoms selected from oxygen, sulfur and nitrogen atoms and the like in addition to carbon atoms (e.g., 2-pyridyl, 3-pyridyl, 4-pyridyl, N-oxido-2-pyridyl, N-oxido-3-pyridyl, N-oxido-4-pyridyl, 2-pyrimidinyl, 4-pyrimidinyl, 5-pyrimidinyl, N-oxido-2-pyrimidinyl, N-oxido-4-pyrimidinyl, N-oxido-5-pyrimidinyl, 2-thiomorpholinyl, 3-thiomorpholinyl, 2-morpholinyl, 3-morpholinyl, piperidinyl, pyranyl, thiopyranyl, 1,4-oxazinyl, 1,4-thiazinyl, 1,3-thiazinyl, 2-piperazinyl, 3-piperazinyl, triazinyl, oxotriazinyl, 3-pyridazinyl, 4-pyridazinyl, pyrazinyl, N-oxido-3-pyridazinyl, N-oxido-4-pyridazinyl, etc.)-carbonyl,
(P) a bicyclic or tricyclic fused ring containing 1 to 4 heteroatoms selected from oxygen, sulfur and nitrogen atoms in addition to carbon atoms (e.g., benzofuryl, benzothiazolyl, benzoxazolyl, tetrazolo[1,5-b]pyridazinyl, triazolo[4,5-b]pyridazinyl, benzoimidazolyl, quinolyl, isoquinolyl, cinnolinyl, phthalazinyl, quinazolinyl, quinoxalinyl, indolizinyl, quinolizinyl, 1,8-naphthyridinyl, purinyl, pteridinyl, dibenzofuranyl, carbazolyl, acridinyl, phenanthridinyl, chromanyl, benzoxazinyl, phenazinyl, phenothiazinyl, phenoxazinyl, etc.)-carbonyl,
(Q) R^{3'}O-N=CH- (wherein R^{3'} is a hydrocarbon group which may be substituted or a hydrogen atom, and the "hydrocarbon group which may be substituted" may be exemplified by the "hydrocarbon groups" mentioned as the "substituents which may be further carried by ring A"),
   and the like. These substituents may be present at substitutable positions, and the number of substituents is 1 to 4 when ring A is a benzene ring, and 1 to 3 when ring A is some other ring. These substituents may be further substituted with the above-defined substituents at the substitutable position thereof, and the number of substituents is not particularly limited.
   The "substituent" of the "hydrocarbon group" may be preferably exemplified by
   (1) mono- or di-C₁₋₆ alkylamino (e.g., methylamino, ethylamino, propylamino, isopropylamino, butylamino, isobutylamino, sec-butylamino, tert-butylamino, pentylamino, hexylamino, dimethylamino, diethylamino, dipropylamino, diisopropylamino, butylamino, dibutylamino, etc.), (2) C₆₋₁₄ aryl (e.g., phenyl, 1-naphthyl, 2-naphthyl, phenanthryl, anthryl, etc.), (3) hydroxy which may be protected by a protective group (e.g., tert-butyl(dimethyl)silyl [TBDMS], etc.), (4) halogen (e.g., fluorine, chlorine, bromine, iodine, etc.), (5) cyano, (6) carboxyl, (7) C₁₋₆ alkylthio (e.g., methylthio, ethylthio, propylthio, isopropylthio, butylthio, isobutylthio, sec-butylthio, tert-butylthio, pentylthio, hexylthio, etc.), (8) C₁₋₆ alkylsulfonyl (e.g., methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, hexylsulfonyl, etc.), (9) C₁₋₆ alkoxycarbonyl (e.g., methoxycarbonyl, ethoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl, etc.), (10) carbamoyl, (11) N-mono-C₁₋₆ alkylcarbamoyl (e.g. , methylcarbamoyl, ethylcarbamoyl, propylcarbamoyl, isopropylcarbamoyl, butylcarbamoyl, isobutylcarbamoyl, sec-butylcarbamoyl, tert-butylcarbamoyl, pentylcarbamoyl, hexylcarbamoyl, etc.), (12) N,N-di-C₁₋₆ alkylcarbamoyl (e.g., dimethylcarbamoyl, diethylcarbamoyl, dipropylcarbamoyl, diisopropylcarbamoyl, butylcarbamoyl, dibutylcarbamoyl, etc.), (13) a 5-membered ring group containing 1 to 4 heteroatoms selected from oxygen, sulfur and nitrogen atoms and the like in addition to carbon atoms (e.g., 2-thienyl, 3-thienyl, 2-furyl, 3-furyl, 2-pyrrolyl, 3-pyrrolyl, 2-oxazolyl, 4-oxazolyl, 5-oxazolyl, 2-thiazolyl, 4-thiazolyl, 5-thiazolyl, 3-pyrazolyl, 4-pyrazolyl, 5-pyrazolyl, 2-imidazolyl, 4-imidazolyl, 5-imidazolyl, 3-isoxazolyl, 4-isoxazolyl, 5-isoxazolyl, 3-isothiazolyl, 4-isothiazolyl, 5-isothiazolyl, 3-(1,2,4-oxadiazolyl), 5-(1,2,4-oxadiazolyl), 1,3,4-oxadiazolyl, 3-(1,2,4-thiadiazolyl), 5-(1,2,4-thiadiazolyl), 1,3,4-thiadiazolyl, 4-(1,2,3-thiadiazolyl), 5-(1,2,3-thiadiazolyl), 1,2,5-thiadiazolyl, 1,2,3-triazolyl, 1,2,4-triazolyl, 1H-tetrazolyl, 2H-tetrazolyl, oxoimidazinyl, dioxotriazinyl, pyrrolidinyl, etc.), (14) C₁₋₂₀ acylamino, or the like.

The "hydrocarbon group" is preferably C₆₋₁₄ aryl (e.g., phenyl, 1-naphthyl, 2-naphthyl, anthryl, phenanthryl, etc.) which may be substituted with 1 to 3 substituents selected from (i) hydroxy, (ii) amino, (iii) mono-C₁₋₆ alkylamino (e.g., methylamino, ethylamino, propylamino, isopropylamino, butylamino, isobutylamino, sec-butylamino, tert-butylamino, pentylamino, hexylamino, etc.), (iv) di-C₁₋₆ alkylamino (e.g., dimethylamino, diethylamino, dipropylamino, diisopropylamino, butylamino, dibutylamino, etc.), (v) C₁₋₆ alkoxy (e.g., methoxy, ethoxy, propoxy, isopropoxy, butoxy, isobutoxy, sec-butoxy, tert-butoxy, pentyloxy, hexyloxy, etc.), and (vi) halogen (e.g., fluorine, chlorine, bromine, iodine).

The hydrocarbon group is preferably C₁₋₆ alkyl or C₃₋₈ cycloalkyl.

Preferably, the substituents which may be further carried by ring A may be exemplified by
(a) C₆₋₁₄ aryl (e.g., phenyl, 1-naphthyl , 2-naphthyl, anthryl, phenanthryl, etc.) which may be substituted with 1 to 3 substituents selected from (i) hydroxy, (ii) amino, (iii) mono-C₁₋₆ alkylamino (e.g., methylamino, ethylamino, propylamino, isopropylamino, butylamino, isobutylamino, sec-butylamino, tert-butylamino, pentylamino, hexylamino, etc.), (iv) di-C₁₋₆ alkylamino (e.g., dimethylamino, diethylamino, dipropylamino, diisopropylamino, butylamino, dibutylamino, etc.), (v) C₁₋₆ alkoxy (e.g., methoxy, ethoxy, propoxy, isopropoxy, butoxy, isobutoxy, sec-butoxy, tert-butoxy, pentyloxy, hexyloxy, etc.), and (vi) halogen (e.g., fluorine, chlorine, bromine, iodine),
(b) cyano,
(c) hydroxy,
(d) carboxy,
(e) nitro,
(f) C₁₋₆ alkyl (e.g., methyl, ethyl, propyl, isopropyl, butyl, isobutyl, sec-butyl, tert-butyl, pentyl, hexyl, etc.) which may be substituted with di-C₁₋₆ alkylamino (e.g., dimethylamino, diethylamino, dipropylamino, diisopropylamino, butylamino, dibutylamino, etc.),
(g) C₁₋₆ alkoxy (e.g., methoxy, ethoxy, propoxy, isopropoxy, butoxy, isobutoxy, sec-butoxy, tert-butoxy, pentyloxy, hexyloxy, etc.),
(h) C₁₋₆ alkyl-carbonyloxy (e.g., methylcarbonyloxy, ethylcarbonyloxy, propylcarbonyloxy, isopropylcarbonyloxy, butylcarbonyloxy, isobutylcarbonyloxy, sec-butylcarbonyloxy, tert-butylcarbonyloxy, pentylcarbonyloxy, hexylcarbonyloxy, etc.),
(i) C₁₋₆ alkylthio (e.g., methylthio, ethylthio, propylthio, isopropylthio, butylthio, isobutylthio, sec-butylthio, tert-butylthio, pentylthio, hexylthio, etc.),
(j) C₁₋₆ alkylsulfinyl (e.g., methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, hexylsulfinyl, etc.),
(k) C₁₋₆ alkylsulfonyl (e.g., methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, hexylsulfonyl, etc.),
(1) halogen (e.g., fluorine, chlorine, bromine, iodine),
(m) amino,
(n) mono-C₁₋₆ alkylamino (e.g., methylamino, ethylamino, propylamino, isopropylamino, butylamino, isobutylamino, sec-butylamino, tert-butylamino, pentylamino, hexylamino, etc.),
(o) di-C₁₋₆ alkylamino (e.g., dimethylamino, diethylamino, dipropylamino, diisopropylamino, butylamino, dibutylamino, etc.),
(p) a 5-membered ring group containing 1 to 4 heteroatoms selected from oxygen, sulfur and nitrogen atoms and the like in addition to carbon atoms (e.g., 2-thienyl, 3-thienyl, 2-furyl, 3-furyl, 2-pyrrolyl, 3-pyrrolyl, 2-oxazolyl, 4-oxazolyl, 5-oxazolyl, 2-thiazolyl, 4-thiazolyl, 5-thiazolyl, 3-pyrazolyl, 4-pyrazolyl, 5-pyrazolyl, 2-imidazolyl, 4-imidazolyl, 5-imidazolyl, 3-isoxazolyl, 4-isoxazolyl, 5-isoxazolyl, 3-isothiazolyl, 4-isothiazolyl, 5-isothiazolyl, 3-(1,2,4-oxadiazolyl), 5-(1,2,4-oxadiazolyl), 1,3,4-oxadiazolyl, 3-(1,2,4-thiadiazolyl), 5-(1,2,4-thiadiazolyl), 1,3,4-thiadiazolyl, 4-(1,2,3-thiadiazolyl), 5-(1,2,3-thiadiazolyl), 1,2,5-thiadiazolyl, 1,2,3-triazolyl, 1,2,4-triazolyl, 1H-tetrazolyl, 2H-tetrazolyl, oxoimidazinyl, dioxotriazinyl, pyrrolidinyl, etc.) (wherein this 5-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen),
(q) a 6-membered ring group containing 1 to 4 heteroatoms selected from oxygen, sulfur and nitrogen atoms and the like in addition to carbon atoms (e.g., 2-pyridyl, 3-pyridyl, 4-pyridyl, N-oxido-2-pyridyl, N-oxido-3-pyridyl, N-oxido-4-pyridyl, 2-pyrimidinyl, 4-pyrimidinyl, 5-pyrimidinyl, N-oxido-2-pyrimidinyl, N-oxido-4-pyrimidinyl, N-oxido-5-pyrimidinyl, 2-thiomorpholinyl, 3-thiomorpholinyl, 2-morpholinyl, 3-morpholinyl, piperidinyl, pyranyl, thiopyranyl, 1,4-oxazinyl, 1,4-thiazinyl, 1,3-thiazinyl, 2-piperazinyl, 3-piperazinyl, triazinyl, oxotriazinyl, 3-pyridazinyl, 4-pyridazinyl, pyrazinyl, N-oxido-3-pyridazinyl, N-oxido-4-pyridazinyl, etc.) (wherein this 6-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen),
(r) a bicyclic or tricyclic fused ring group containing 1 to 4 heteroatoms selected from oxygen, sulfur and nitrogen atoms in addition to carbon atoms (e.g., benzofuryl, benzothiazolyl, benzoxazolyl, tetrazolo[1,5-b]pyridazinyl, triazolo[4,5-b]pyridazinyl, benzoimidazolyl, quinolyl, isoquinolyl, cinnolinyl, phthalazinyl, quinazolinyl, quinoxalinyl, indolizinyl, quinolizinyl, 1,8-naphthyridinyl, purinyl, pteridinyl, dibenzofuranyl, carbazolyl, acridinyl, phenanthridinyl, chromanyl, benzoxazinyl, phenazinyl, phenothiazinyl, phenoxazinyl, etc.) (wherein this bicyclic or tricyclic fused ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen),
(s) C₁₋₆ alkoxycarbonyl (e.g., methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl, etc.),
(t) carbamoyl,
(u) N-mono-C₁₋₆ alkylcarbamoyl (e.g., methylcarbamoyl, ethylcarbamoyl, propylcarbamoyl, isopropylcarbamoyl, butylcarbamoyl, isobutylcarbamoyl, sec-butylcarbamoyl, tert-butylcarbamoyl, pentylcarbamoyl, hexylcarbamoyl, etc.),
(v) N,N-di-C₁₋₆ alkylcarbamoyl (e.g., dimethylcarbamoyl, diethylcarbamoyl, dipropylcarbamoyl, diisopropylcarbamoyl, butylcarbamoyl, dibutylcarbamoyl, etc.), and
(w) C₁₋₂ alkylenedioxy (e.g., methylenedioxy and ethylenedioxy).

Ring A is preferably a benzene ring which may be substituted with 1 to 4 substituents selected from (a) C₆₋₁₄ aryl which may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen, (b) cyano, (c) hydroxy, (d) carboxyl, (e) nitro, (f) C₁₋₆ alkyl which may be substituted with di-C₁₋₆ alkylamino, (g) C₁₋₆ alkoxy, (h) C₁₋₆ alkyl-carbonyloxy, (i) C₁₋₆ alkylthio, (j) C₁₋₆ alkylsulfinyl, (k) C₁₋₆ alkylsulfonyl, (1) halogen, (m) amino, (n) mono-C₁₋₆ alkylamino, (o) di-C₁₋₆ alkylamino, (p) a 5-membered ring group containing 1 to 4 heteroatoms selected from oxygen, sulfur and nitrogen atoms and the like in addition to carbon atoms (wherein this 5-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (q) a 6-membered ring group containing 1 to 4 heteroatoms selected from oxygen, sulfur and nitrogen atoms and the like in addition to carbon atoms (wherein this 6-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (r) a bicyclic or tricyclic fused ring group containing 1 to 4 heteroatoms selected from oxygen, sulfur and nitrogen atoms in addition to carbon atoms (wherein this bicyclic or tricyclic fused ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (s) C₁₋₆ alkoxycarbonyl, (t) carbamoyl, (u) N-mono-C₁₋₆ alkylcarbamoyl, (v) N,N-di-C₁₋₆ alkylcarbamoyl, and (w) C₁₋₂ alkylenedioxy.

Furthermore, ring A is preferably a benzene ring which may be substituted with 1 to 4 substituents selected from (a) hydroxy, (b) cyano, (c) carboxyl, (d) mono- or dialkylcarbamoyl (i.e., the above-described N-mono-C₁₋₆ alkylcarbamoyl and N,N-di-C₁₋₆ alkylcarbamoyl), (e) acylamino (i.e., the above-described C₁₋₂₀ acylamino), (f) alkylamino (i.e., the above-described mono-C₁₋₆ alkylamino and di-C₁₋₆ alkylamino), and (g) alkoxycarbonyl (i.e., the above-described C₁₋₆ alkoxycarbonyl), or a benzene ring which may be substituted with a hydrocarbon group substituted with 1 to 4 of the aforementioned substituents. The positions of the substituents in the benzene ring are not particularly limited.

The "hydrocarbon group" of the above-described benzene ring which may be substituted with a hydrocarbon group is preferably, for example, (1) C₁₋₆ alkyl (e.g., methyl, ethyl, propyl, isopropyl, butyl, isobutyl, sec-butyl, tert-butyl, pentyl, hexyl, etc.), (2) C₂₋₆ alkenyl (e.g., vinyl, allyl, 1-butenyl, 2-butenyl, etc.), (3) C₂₋₆ alkynyl (e.g., ethynyl, propargyl, 2-butynyl, 5-hexynyl, etc.), (4) C₃₋₈ cycloalkyl (e.g., cyclopropyl, cyclobutyl, cyclopentyl, cyclohexyl, cycloheptyl, cyclooctyl, etc.), (5) C₆₋₁₄ aryl (e.g., phenyl, 1-naphthyl, 2-naphthyl, anthryl, phenanthryl, etc.), (6) C₇₋₁₄ aralkyl (e.g., benzyl, phenethyl, etc.), or the like.

In Compound (I-a), X is an oxygen atom, a sulfur atom or NR³, wherein R³ is a hydrocarbon group which may be substituted or a hydrogen atom. The "hydrocarbon group" of the "hydrocarbon group which may be substituted" represented by R³ may be exemplified by (1) C₁₋₆ alkyl (e.g., methyl, ethyl, propyl, isopropyl, butyl, isobutyl, sec-butyl, tert-butyl, pentyl, hexyl, etc.), (2) C₂₋₆ alkenyl (e.g., vinyl, allyl, 1-butenyl, 2-butenyl, etc.), (3) C₂₋₆ alkynyl (e.g., ethynyl, propargyl, 2-butynyl, 5-hexynyl, etc.), (4) C₃₋₈ cycloalkyl which may be fused with a C₆₋₁₄ aryl ring (e.g., benzene, naphthalene, anthracene, phenanthrene, etc.) (e.g., cyclopropyl, cyclobutyl, cyclopentyl, cyclohexyl, cycloheptyl, cyclooctyl, 1,2,3,4-tetrahydronaphthalen-1-yl, 2,3-dihydro-1H-inden-1-yl, 6,7,8,9-tetrahydro-5H-benzo[7]annulen-5-yl, etc.), (5) C₆₋₁₄ aryl which may be fused with a C₃₋₈ cycloalkane (e.g., cyclopropane, cyclobutane, cyclopentane, cyclohexane, cycloheptane, cyclooctane, etc.) (e.g., phenyl, 1-naphthyl, 2-naphthyl, anthryl, phenanthryl, 5,6,7,8-tetrahydronaphthalen-1-yl, etc.), (6) C₇₋₁₄ aralkyl (e.g., benzyl, phenethyl, etc.), or the like.

The "substituent" of the hydrocarbon group which may be substituted represented by R³ may be exemplified by those mentioned as the substituents which may be further carried by ring A, and the number of substituents is from 1 to 3.

X is preferably an oxygen atom or NR³, wherein R³ is a hydrocarbon group which may be substituted or a hydrogen atom.

More preferably, X is an oxygen atom or NR³, wherein R³ is (1) a hydrogen atom, (2) C₁₋₆ alkyl which may be substituted with 1 to 3 substituents selected from (a)C₆₋₁₄ aryl which may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen, (b) cyano, (c) hydroxy, (d) carboxy, (e) nitro, (f) C₁₋₆ alkyl which may be substituted with di-C₁₋₆ alkylamino, (g) C₁₋₆ alkoxy, (h) C₁₋₆ alkylcarbonyloxy, (i) C₁₋₆ alkylthio, (j) C₁₋₆ alkylsulfinyl, (k) C₁₋₆ alkylsulfonyl, (1) halogen, (m) amino, (n) mono-C₁₋₆ alkylamino, (o) di-C₁₋₆ alkylamino, (p) a 5-membered ring group containing 1 to 4 heteroatoms selected from oxygen, sulfur and nitrogen atoms and the like in addition to carbon atoms (wherein this 5-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (q) a 6-membered ring group containing 1 to 4 heteroatoms selected from oxygen, sulfur and nitrogen atoms and the like in addition to carbon atoms (wherein this 6-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (r) a bicyclic or tricyclic fused ring group containing 1 to 4 heteroatoms selected from oxygen, sulfur and nitrogen atoms and the like in addition to carbon atoms (wherein this bicyclic or tricyclic fused ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (s) C₁₋₆ alkoxycarbonyl, (t) carbamoyl, (u) N-mono-C₁₋₆ alkylcarbamoyl, (v) N,N-di-C₁₋₆ alkylcarbamoyl, and (w) C₁₋₂ alkylenedioxy (hereinafter (a) - (w) are briefly referred to as Substituent Group A), (3) C₂₋₆ alkenyl which may be substituted with 1 to 3 substituents selected from the Substituent Group A, (4) C₂₋₆ alkynyl which may be substituted with 1 to 3 substituents selected from the Substituent Group A, (5) C₃₋₈ cycloalkyl which may be substituted with 1 to 3 substituents selected from the Substituent Group A, (6) C₆₋₁₄ aryl which may be substituted with 1 to 3 substituents selected from the Substituent Group A, or (7) C₇₋₁₄ aralkyl which may be substituted with 1 to 3 substituents selected from the Substituent Group A. Among these, X is preferably an oxygen atom or NH.

In Compound (I), R¹ is a hydrocarbon group which may be substituted or a hydrogen atom. The "hydrocarbon group which may be substituted" represented by R¹ may be exemplified by those mentioned as the "hydrocarbon group which may be substituted" represented by R³.

R¹ is preferably (1) a hydrogen atom, or (2) C₁₋₆ alkyl which may be substituted with 1 to 3 substituents selected from (a) C₆₋₁₄ aryl which may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen, (b) cyano, (c) hydroxy, (d) carboxy, (e) nitro, (f) C₁₋₆ alkyl which may be substituted with di-C₁₋₆ alkylamino, (g) C₁₋₆ alkoxy, (h) C₁₋₆ alkyl-carbonyloxy, (i) C₁₋₆ alkylthio, (j) C₁₋₆ alkylsulfinyl, (k) C₁₋₆ alkylsulfonyl, (1) halogen, (m) amino, (n) mono-C₁₋₆ alkylamino, (o) di-C₁₋₆ alkylamino, (p) a 5-membered ring group containing 1 to 4 heteroatoms selected from oxygen, sulfur and nitrogen atoms and the like in addition to carbon atoms (wherein this 5-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (q) a 6-membered ring group containing 1 to 4 heteroatoms selected from oxygen, sulfur and nitrogen atoms and the like in addition to carbon atoms (wherein this 6-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (r) a bicyclic or tricyclic fused ring group containing 1 to 4 heteroatoms selected from oxygen, sulfur and nitrogen atoms and the like in addition to carbon atoms (wherein this bicyclic or tricyclic fused ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (s) C₁₋₆ alkoxycarbonyl, (t) carbamoyl, (u) N-mono-C₁₋₆ alkylcarbamoyl, (v) N,N-di-C₁₋₆ alkylcarbamoyl, and (w) C₁₋₂ alkylenedioxy, and among these, a hydrogen atom or C₁₋₆ alkyl, particularly a hydrogen atom, is preferred.

In Compound (I), R² is
(1) a group represented by the formula: 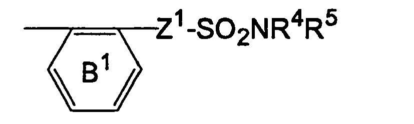 [wherein ring B¹ is a benzene ring which may be further substituted; Z¹ is C₁₋₆ alkylene which may be substituted or a bond; R⁴ is (a) a hydrogen atom, (b) a hydrocarbon group which may be substituted, or (c) a heterocyclic group which may be substituted; and R⁵ is (a) a hydrocarbon group which may be substituted, or (b) a heterocyclic group which may be substituted; or R⁴ and R⁵ may form a ring together with the adjacent nitrogen atom],
(2) a group represented by the formula: 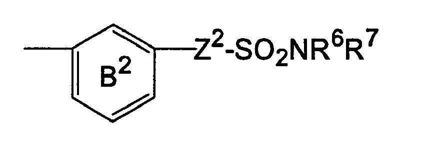 [wherein ring B² is a benzene ring which may be further substituted; Z² is C₁₋₆ alkylene which may be substituted or a bond; R⁶ is (a) a hydrogen atom, (b) a hydrocarbon group which may be substituted, or (c) a heterocyclic group which may be substituted; and R⁷ is (a) a hydrocarbon group which may be substituted, or (b) a heterocyclic group which may be substituted; or R⁶ and R⁷ may form a ring together with the adjacent nitrogen atom],
(3) a group represented by the formula: 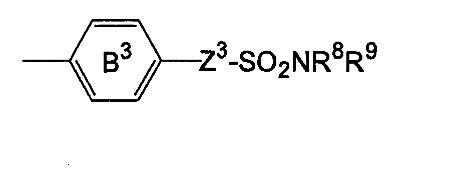 [wherein ring B³ is a benzene ring which may be further substituted; Z³ is C₁₋₆ alkylene which may be substituted or a bond; R⁸ is (a) a hydrogen atom, (b) a hydrocarbon group which may be substituted, or (c) a heterocyclic group which may be substituted; and R⁹ is (a) a hydrocarbon group which may be substituted, or (b) a heterocyclic group which may be substituted; or R⁸ and R⁹ may form a ring together with the adjacent nitrogen atom, provided that when R⁸ is a hydrogen atom, R⁹ is (a) an aryl group which may be substituted, (b) an aralkyl group which may be substituted, or (c) an alkyl group substituted with a heterocyclic group which may be substituted],
(4) a group represented by the formula: 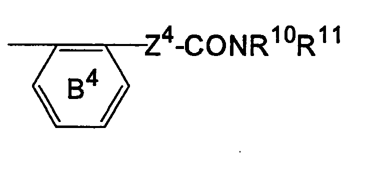 [wherein ring B⁴ is a benzene ring which may be further substituted; Z⁴ is C₁₋₆ alkylene which may be substituted or a bond; R¹⁰ is (a) a hydrogen atom, (b) a hydrocarbon group which may be substituted, or (c) a heterocyclic group which may be substituted; and R¹¹ is (a) an aromatic group which may be substituted, (b) an aralkyl group which may be substituted, or (c) an alkyl group substituted with a heterocyclic group which may be substituted; or R¹⁰ and R¹¹ may form, together with the adjacent nitrogen atom, a heterocyclic ring comprising carbon and nitrogen atoms as ring-constituting moieties],
(5) a group represented by the formula: 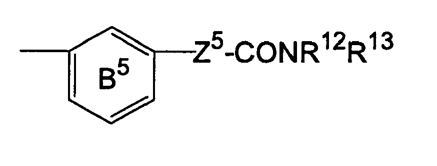 [wherein ring B⁵ is a benzene ring which may be further substituted; Z⁵ is C₁₋₆ alkylene which may be substituted or a bond; and R¹² and R¹³, which may be identical or different, are each (a) a hydrogen atom, (b) a hydrocarbon group which may be substituted, or (c) a heterocyclic group which may be substituted; or R¹² and R¹³ may form a ring together with the adjacent nitrogen atom],
(6) a group represented by the formula: 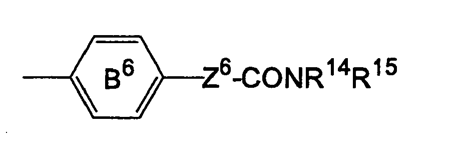 [wherein ring B⁶ is a benzene ring which may be further substituted; Z⁶ is C₁₋₆ alkylene which may be substituted or a bond; and R¹⁴ and R¹⁵, which may be identical or different, are each (a) a hydrocarbon group which may be substituted, or (b) a heterocyclic group which may be substituted; or R¹⁴ and R¹⁵ may form a ring together with the adjacent nitrogen atom],
(7) a group represented by the formula: 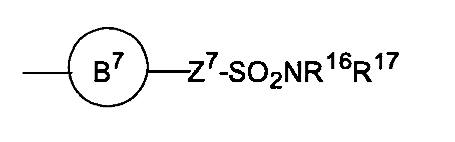 [wherein ring B⁷ is a C₁₀₋₁₄ aryl ring which may be further substituted, or an aromatic heterocycle which may be further substituted; Z⁷ is C₁₋₆ alkylene which may be substituted or a bond; R¹⁶ is (a) a hydrogen atom, (b) a hydrocarbon group which may be substituted, or (c) a heterocyclic group which may be substituted; and R¹⁷ is (a) a hydrocarbon group which may be substituted, or (b) a heterocyclic group which may be substituted; or R¹⁶ and R¹⁷ may form a ring together with the adjacent nitrogen atom],
(8) a group represented by the formula: 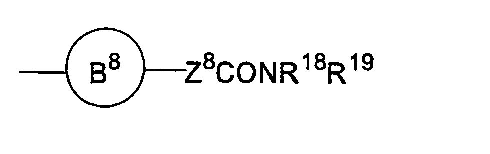 [wherein ring B⁸ is a C₁₀₋₁₄ aryl ring which may be further substituted, or an aromatic heterocycle which may be further substituted; Z⁸ is C₁₋₆ alkylene which may be substituted or a bond; R¹⁸ is (a) a hydrogen atom, (b) a hydrocarbon group which may be substituted, or (c) a heterocyclic group which may be substituted; and R¹⁹ is (a) a hydrocarbon group which may be substituted, or (b) a heterocyclic group which may be substituted; or R¹⁸ and R¹⁹ may form a ring together with the adjacent nitrogen atom], or
(9) a group represented by the formula: 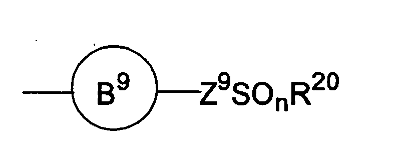 [wherein ring B⁹ is a C₆₋₁₄ aryl ring which may be further substituted, or an aromatic heterocycle which may be further substituted, provided that when X is an oxygen atom, ring B⁹ is not an indole ring; Z⁹ is C₁₋₆ alkylene which may be substituted or a bond; R²⁰ is a hydrocarbon group which may be substituted, or a heterocyclic group which may be substituted; and n is an integer from 0 to 2].

The "substituent" of the "benzene ring which may be further substituted" represented by ring B¹ through ring B⁶ may be exemplified by those mentioned as the substituents which may be further carried by ring A, and the number of substituents is from 1 to 3.

The "C₁₀₋₁₄ aryl ring" of the "C₁₀₋₁₄ aryl ring which may be further substituted" represented by ring B⁷ or ring B⁸ may be exemplified by a naphthalene ring, a phenanthrene ring, an anthracene ring or the like.

The "substituent" of the "C₁₀₋₁₄ aryl ring which may be further substituted" represented by ring B⁷ or ring B⁸ may be exemplified by those mentioned as the substituents which may be further carried by ring A, and the number of substituents is from 1 to 3.

The "C₆₋₁₄ aryl ring" of the "C₆₋₁₄ aryl ring which may be further substituted" represented by ring B⁹ may be exemplified by a benzene ring, a naphthalene ring, a phenanthrene ring, an anthracene ring or the like.

The "substituent" of the "C₆₋₁₄ aryl ring which may be further substituted" represented by ring B⁹ may be exemplified by those mentioned as the substituents which may be further carried by ring A, and the number of substituents is from 1 to 3.

The "aromatic heterocycle" of the "aromatic heterocycle which may be further substituted" represented by ring B⁷, ring B⁸ or ring B⁹ may be exemplified by a 5-membered aromatic heterocycle (e.g., a thiophene ring, a furan ring, a pyrrole ring, an oxazole ring, a thiazole ring, a pyrazole ring, an imidazole ring, an isoxazole ring, an isothiazole ring, a 1,2,4-oxadiazole ring, a 1,3,4-oxadiazole ring, a 1,2,4-thiadiazole ring, a 1,3,4-thiadiazole ring, a 1,2,3-triazole ring, a 1,2,4-triazole ring, a 1H-tetrazole ring, a 2H-tetrazole ring, etc.), a 6-membered aromatic heterocycle (e.g., a pyridine ring, a pyrimidine ring, a triazinine ring, a pyridazine ring, a pyrazine ring, etc.), a bicyclic or tricyclic fused aromatic heterocycle (e.g., a benzofuran ring, a benzothiazole ring, a benzoxazole ring, a tetrazolo [1,5-b]pyridazine ring, a triazolo[4,5-b]pyridazine ring, a benzoimidazole ring, a quinoline ring, an isoquinoline ring, a cinnoline ring, a phthalazine ring, a quinazoline ring, a quinoxaline ring, an indolizine ring, a quinolizine ring, a 1,8-naphthyridine ring, a purine ring, a pteridine ring, a dibenzofuran ring, a carbazole ring, an acridine ring, a phenanthridine ring, a chromane ring, a benzoxazine ring, a phenazine ring, a phenothiazine ring, a phenoxazine ring, etc.), an indole ring or the like.

The "substituent" of the aromatic heterocycle which may be further substituted represented by ring B⁷, ring B⁸ or ring B⁹ may be exemplified by those mentioned as the substituents which may be further carried by ring A, and the number of substituents is from 1 to 3.

The "C₁₋₆ alkylene" of the "C₁₋₆ alkylene which may be substituted" represented by Z¹ through Z⁹ may be exemplified by -CH₂-, -CH₂CH₂-, -CH₂CH₂CH₂-, -CH₂CH₂CH₂CH₂-, -CH₂CH₂CH₂CH_{Z}CH₂- or - CH₂CH₂CH₂CH₂CH₂CH₂-.

The "substituent" of the C₁₋₆ alkylene which may be substituted, represented by Z¹ through Z⁹ may be exemplified by those mentioned as the substituents which may be further carried by ring A, and the number of substituents is from 1 to 3.

The "hydrocarbon group which may be substituted" represented by R⁴, R⁵, R⁶, R⁷, R⁸ , R⁹ , R¹⁰, R¹² , R¹³, R¹⁴, R¹⁵,R¹⁶, R¹⁷, R¹⁸, R¹⁹ and R²⁰ may be exemplified by those mentioned as the "hydrocarbon group which may be substituted" represented by R³.

The "heterocyclic group" of the "heterocyclic group which may be substituted" represented by R⁴, R⁵, R⁶, R⁷, R⁸, R⁹, R¹⁰, R¹², R¹³, R¹⁴, R¹⁵, R¹⁶, R¹⁷, R¹⁸, R¹⁹ and R²⁰ may be exemplified by a 5- or 6-membered ring group containing 1 to 4 heteroatoms selected from oxygen, sulfur and nitrogen atoms and the like in addition to carbon atoms (e.g., a 5-membered ring group such as 2-thienyl, 3-thienyl, 2-furyl, 3-furyl, 2-pyrrolyl, 3-pyrrolyl, 2-oxazolyl, 4-oxazolyl, 5-oxazolyl, 2-thiazolyl, 4-thiazolyl, 5-thiazolyl, 3-pyrazolyl, 4-pyrazolyl, 5-pyrazolyl, 2-imidazolyl, 4-imidazolyl, 5-imidazolyl, 3-isoxazolyl, 4-isoxazolyl, 5-isoxazolyl, 3-isothiazolyl, 4-isothiazolyl, 5-isothiazolyl, 3-(1,2,4-oxadiazolyl), 5-(1,2,4-oxadiazolyl), 1,3,4-oxadiazolyl, 3-(1,2,4-thiadiazolyl), 5-(1,2,4-thiadiazolyl), 1,3,4-thiadiazolyl, 4-(1,2,3-thiadiazolyl), 5-(1,2,3-thiadiazolyl), 1,2,5-thiadiazolyl, 1,2,3-triazolyl, 1,2,4-triazolyl, 1H-tetrazolyl, 2H-tetrazolyl, oxoimidazinyl, dioxotriazinyl or pyrrolidinyl; a 6-membered ring group such as 2-pyridyl, 3-pyridyl, 4-pyridyl, N-oxido-2-pyridyl, N-oxido-3-pyridyl, N-oxido-4-pyridyl, 2-pyrimidinyl, 4-pyrimidinyl, 5-pyrimidinyl, N-oxido-2-pyrimidinyl, N-oxido-4-pyrimidinyl, N-oxido-5-pyrimidinyl, 2-thiomorpholinyl, 3-thiomorpholinyl, 2-morpholinyl, 3-morpholinyl, piperidinyl, pyranyl, thiopyranyl, 1,4-oxazinyl, 1,4-thiazinyl, 1,3-thiazinyl, 2-piperazinyl, 3-piperazinyl, triazinyl, oxotriazinyl, 3-pyridazinyl, 4-pyridazinyl, pyrazinyl, N-oxido-3-pyridazinyl or N-oxido-4-pyridazinyl), or the like.

The "substituent" of the "heterocyclic group which may be substituted" may be exemplified by those mentioned as the substituents which may be further carried by ring A, and the number of substituents is from 1 to 3.

When R⁴ and R⁵, R⁶ and R⁷, R⁸ and R⁹, R¹² and R¹³, R¹⁴ and R¹⁵, R¹⁶ and R¹⁷, and R¹⁸ and R¹⁹ respectively form a ring together with the adjacent nitrogen ring, the groups represented by - NR⁴R⁵, -NR⁶R⁷, -NR⁸R⁹, -NR¹²R¹³, -NR¹⁴R¹⁵, -NR¹⁶R¹⁷ and -NR¹⁸R¹⁹ are respectively a cyclic group represented by 1-pyrrolidinyl, 1-pyrrolinyl, 1-pyrrolyl, azepan-1-yl, 1-imidazolidinyl, 1-imidazolinyl, 1-imidazolyl, 2-pyrazolidinyl, 2-pyrazolinyl, 1-pyrazolyl, 1-indolinyl, 1-indolyl, 2-isoindolinyl, 2-isoindolyl, 2,3-dihydro-1H-indol-1-yl, 1-piperidyl, 3,4-dihydro-1,5-naphthyridin-1(2H)-yl, 1,2,3,4-tetrahydroquinolin-1-yl, 3,4-dihydroquinolin-1(2H)-yl, 3,4-dihydroisoquinolin-2(1H)-yl, 1-piperazinyl, octahydroquinolin-1(2H)-yl, octahydroisoquinolin-2(1H)-yl, 3,4-dihydroquinoxalin-1(2H)-yl, 1H-azepin-1-yl, 2,3,4,5,6,7-hexahydro-1H-azepin-1-yl, 2,3,4,5-tetrahydro-1H-1-benzazepin-1-yl, 1,3,4,5-tetrahydro-2H-2-benzazepin-2-yl, 3,4,5,6-tetrahydro-1-benzazocin-1(2H)-yl, 2,3-dihydro-4,1-benzothiazepin-1(5H)-yl, 3,4-dihydro-1,5-benzothiazepin-5(2H)-yl, 2,3-dihydro-4,1-benzothiazepin-1(5H)-yl, 2,3-dihydro-4,1-benzoxazepin-1(5H)-yl, 1,2,4,5-tetrahydro-3H-3-benzazepin-3-yl, 2,3,4,5-tetrahydro-1H-1,5-benzodiazepin-1-yl, 2,3,4,5-tetrahydro-1H-1,4-benzodiazepin-1-yl, 2,3-dihydro-4H-1,4-benzoxazin-4-yl, 3,4-dihydro-2H-1,4-benzoxazin-1-yl, 3,4-dihydro-1,5-benzoxazepin-5(2H)-yl, 5,6,7,8-tetrahydro-4H-thieno[3,2-b]azepin-4-yl, 4-morpholinyl, 4-thiomorpholinyl, 2,3-dihydro-4H-pyrido[3,2-b][1,4]oxazin-4-yl, 2,3-dihydro-4H-1,4-benzothiazin-4-yl, 5,6,7,8-tetrahydropyrazolo[4,3-b]azepin-4(1H)-yl and the like. The cyclic groups may be substituted, and the substituent for the cyclic groups may be exemplified by those mentioned as the substituents which may be further carried by ring A, while the number of substituents is from 1 to 3.

The "aromatic group" of the "aromatic group which may be substituted" represented by R¹¹ may be exemplified by an aromatic hydrocarbon group or an aromatic heterocyclic group.

The aromatic hydrocarbon group may be exemplified by C₆₋₁₄ aryl such as phenyl, 1-naphthyl, 2-naphthyl, anthryl or phenanthryl. The aromatic heterocyclic group may be exemplified by a 5- or 6-membered aromatic heterocyclic group containing 1 to 4 heteroatoms selected from oxygen, sulfur and nitrogen atoms and the like in addition to carbon atoms (e.g., a 5-membered aromatic heterocyclic group such as 2-thienyl, 3-thienyl, 2-furyl, 3-furyl, 2-pyrrolyl, 3-pyrrolyl, 2-oxazolyl, 4-oxazolyl, 5-oxazolyl, 2-thiazolyl, 4-thiazolyl, 5-thiazolyl, 3-pyrazolyl, 4-pyrazolyl, 5-pyrazolyl, 2-imidazolyl, 4-imidazolyl, 5-imidazolyl, 3-isoxazolyl, 4-isoxazolyl, 5-isoxazolyl, 3-isothiazolyl, 4-isothiazolyl, 5-isothiazolyl, 3-(1,2,4-oxadiazolyl), 5-(1,2,4-oxadiazolyl), 1,3,4-oxadiazolyl, 3-(1,2,4-thiadiazolyl), 5-(1,2,4-thiadiazolyl), 1,3,4-thiadiazolyl, 4-(1,2,3-thiadiazolyl), 5-(1,2,3-thiadiazolyl), 1,2,5-thiadiazolyl, 1,2,3-triazolyl, 1,2,4-triazolyl, 1H-tetrazolyl, 2H-tetrazolyl, oxoimidazinyl, dioxotriazinyl or pyrrolidinyl; a 6-membered aromatic heterocyclic group such as 2-pyridyl, 3-pyridyl, 4-pyridyl, N-oxido-2-pyridyl, N-oxido-3-pyridyl, N-oxido-4-pyridyl, 2-pyrimidinyl, 4-pyrimidinyl, 5-pyrimidinyl, N-oxido-2-pyrimidinyl, N-oxido-4-pyrimidinyl, N-oxido-5-pyrimidinyl, triazinyl, oxotriazinyl, 3-pyridazinyl, 4-pyridazinyl, pyrazinyl, N-oxido-3-pyridazinyl or N-oxido-4-pyridazinyl), or a fused ring in which such a 5- or 6-membered aromatic heterocycle is fused with a benzene ring, or the like.

The "substituent" of the "aromatic group which may be substituted" represented by R¹¹ may be exemplified by those mentioned as the substituents which may be further carried by ring A, and the number of substituents is from 1 to 3.

The "aralkyl group" of the "aralkyl group which may be substituted" represented by R¹¹ may be exemplified by C₇₋₁₄ aralkyl such as benzyl or phenethyl.

The "substituent" of the "aralkyl group which may be substituted" represented by R¹¹ may be exemplified by those mentioned as the substituents which may be further carried by ring A, and the number of substituents is from 1 to 3.

The "alkyl group" of the "alkyl group substituted with a heterocyclic group which may be substituted" represented by R¹¹ may be exemplified by C₁₋₆ alkyl such as methyl, ethyl, propyl, isopropyl, butyl, isobutyl, sec-butyl, tert-butyl, pentyl or hexyl.

The "heterocyclic group" of the "alkyl group substituted with a heterocyclic group which may be substituted" represented by R¹¹ may be exemplified by (i) a 5-membered ring group containing 1 to 4 heteroatoms selected from oxygen, sulfur and nitrogen atoms and the like in addition to carbon atoms (e.g., 2-thienyl, 3-thienyl, 2-furyl, 3-furyl, 2-pyrrolyl, 3-pyrrolyl, 2-oxazolyl, 4-oxazolyl, 5-oxazolyl, 2-thiazolyl, 4-thiazolyl, 5-thiazolyl, 3-pyrazolyl, 4-pyrazolyl, 5-pyrazolyl, 2-imidazolyl, 4-imidazolyl, 5-imidazolyl, 3-isoxazolyl, 4-isoxazolyl, 5-isoxazolyl, 3-isothiazolyl, 4-isothiazolyl, 5-isothiazolyl, 3-(1,2,4-oxadiazolyl), 5-(1,2,4-oxadiazolyl), 1,3,4-oxadiazolyl, 3-(1,2,4-thiadiazolyl), 5-(1,2,4-thiadiazolyl), 1,3,4-thiadiazolyl, 4-(1,2,3-thiadiazolyl), 5-(1,2,3-thiadiazolyl), 1,2,5-thiadiazolyl, 1,2,3-triazolyl, 1,2,4-triazolyl, 1H-tetrazolyl, 2H-tetrazolyl, oxoimidazinyl, dioxotriazinyl, pyrrolidinyl, etc.), (ii) a 6-membered ring group containing 1 to 4 heteroatoms selected from oxygen, sulfur and nitrogen atoms and the like in addition to carbon atoms (e.g., 2-pyridyl, 3-pyridyl, 4-pyridyl, N-oxido-2-pyridyl, N-oxido-3-pyridyl, N-oxido-4-pyridyl, 2-pyrimidinyl, 4-pyrimidinyl, 5-pyrimidinyl, N-oxido-2-pyrimidinyl, N-oxido-4-pyrimidinyl, N-oxido-5-pyrimidinyl, 2-thiomorpholinyl, 3-thiomorpholinyl, 2-morpholinyl, 3-morpholinyl, piperidinyl, pyranyl, thiopyranyl, 1,4-oxazinyl, 1,4-thiazinyl, 1,3-thiazinyl, 2-piperazinyl, 3-piperazinyl, triazinyl, oxotriazinyl, 3-pyridazinyl, 4-pyridazinyl, pyrazinyl, N-oxido-3-pyridazinyl, N-oxido-4-pyridazinyl, etc.), or (iii) a bicyclic or tricyclic fused ring group containing 1 to 4 heteroatoms selected from oxygen, sulfur and nitrogen atoms and the like in addition to carbon atoms (e.g., benzofuryl, benzothiazolyl, benzoxazolyl, tetrazolo[1,5-b]pyridazinyl, triazolo[4,5-b]pyridazinyl, benzoimidazolyl, quinolyl, isoquinolyl, cinnolinyl, phthalazinyl, quinazolinyl, quinoxalinyl, indolizinyl, quinolizinyl, 1,8-naphthyridinyl, purinyl, pteridinyl, dibenzofuranyl, carbazolyl, acridinyl, phenanthridinyl, chromanyl, benzoxazinyl, phenazinyl, phenothiazinyl, phenoxazinyl, etc.).

The "substituent" of the "alkyl group substituted with a heterocyclic group which may be substituted" represented by R¹¹ may be exemplified by (i) hydroxy, (ii) amino, (iii) mono-C₁₋₆ alkylamino (e.g., methylamino, ethylamino, propylamino, isopropylamino, butylamino, isobutylamino, sec-butylamino, tert-butylamino, pentylamino, hexylamino, etc.), (iv) di-C₁₋₆ alkylamino (e.g., dimethylamino, diethylamino, dipropylamino, diisopropylamino, butylamino, dibutylamino, etc.), (v) C₁₋₆ alkoxy (e.g., methoxy, ethoxy, propoxy, isopropoxy, butoxy, isobutoxy, sec-butoxy, tert-butoxy, pentyloxy, hexyloxy, etc.), (vi) halogen (e.g., fluorine, chlorine, bromine, iodine), or the like, and the heterocyclic group may have 1 to 3 of such substituents at substitutable positions.

When R¹⁰ and R¹¹ together with the adjacent nitrogen atom form a heterocyclic ring consisting of carbon and nitrogen atoms as ring-constituting moieties , the group represented by -NR¹⁰R¹¹ is a cyclic group selected from 1-pyrrolidinyl, 1-pyrrolinyl, 1-pyrrolyl, 1-imidazolidinyl, 1-imidazolinyl, 1-imidazolyl, 2-pyrazolidinyl, 2-pyrazolinyl, 1-pyrazolyl, 1-indolinyl, 1-indolyl, 2-isoindolinyl, 2-isoindolyl, 1-piperidyl, 3,4-dihydroquinolin-1(2H)-yl, 3,4-dihydroisoquinolin-2(1H)-yl, 1-piperazinyl, 3,4-dihydroquinoxalin-1(2H)-yl, 1H-azepin-1-yl, 2,3,4,5,6,7-hexahydro-1H-azepin-1-yl, 2,3,4,5-tetrahydro-1H-1-benzazepin-1-yl, 1,3,4,5-tetrahydro-2H-2-benzazepin-2-yl, 1,2,4,5-tetrahydro-3H-3-benzazepin-3-yl, and the like. These cyclic groups may be substituted, and the substituent for the cyclic groups may be exemplified by those mentioned as the substituents which may be further carried by ring A described above, while the number of substituents is from 1 to 3.

R² is preferably (1) a group represented by the formula: 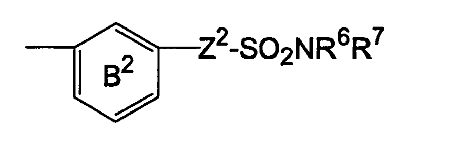 wherein each reference symbol has the meaning as defined above,
(2) a group represented by the formula: 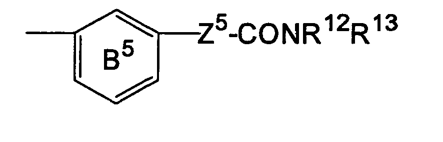 wherein each reference symbol has the meaning as defined above, or
(3) a group represented by the formula: 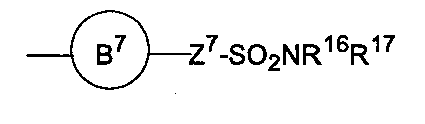 wherein each reference symbol has the meaning as defined above. Among these, R² is preferably
(1) a group represented by the formula: 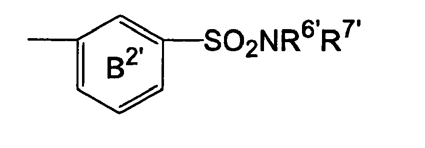 wherein ring B² is a benzene ring which may be further substituted with 1 to 3 substituents selected from (a) C₆₋₁₄ aryl which may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen, (b) cyano, (c) hydroxy, (d) carboxy, (e) nitro, (f) C₁₋₆ alkyl which may be substituted with di-C₁₋₆ alkylamino, (g) C₁₋₆ alkoxy, (h) C₁₋₆ alkyl-carbonyloxy, (i) C₁₋₆ alkylthio, (j) C₁₋₆ alkylsulfinyl, (k) C₁₋₆ alkylsulfonyl, (1) halogen, (m) amino, (n) mono-C₁₋₆ alkylamino, (o) di-C₁₋₆ alkylamino, (p) a 5-membered ring group containing 1 to 4 heteroatoms selected from oxygen, sulfur and nitrogen atoms and the like in addition to carbon atoms (wherein this 5-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (q) a 6-membered ring group containing 1 to 4 heteroatoms selected from oxygen, sulfur and nitrogen atoms and the like in addition to carbon atoms (wherein this 6-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (r) a bicyclic or tricyclic fused ring group containing 1 to 4 heteroatoms selected from oxygen, sulfur and nitrogen atoms and the like in addition to carbon atoms (wherein this bicyclic or tricyclic fused ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (s) C₁₋₆ alkoxycarbonyl, (t) carbamoyl, (u) N-mono-C₁₋₆ alkylcarbamoyl, (v) N,N-di-C₁₋₆ alkylcarbamoyl, and (w) C₁₋₂ alkylenedioxy (hereinafter (a) - (w) are briefly referred to as Substituent Group A); R^{6'} is (a) a hydrogen atom, (b) C₁₋₆ alkyl which may be substituted with 1 to 3 substituents selected from the Substituent Group A, (c) C₂₋₆ alkenyl which may be substituted with 1 to 3 substituents selected from the Substituent Group A, (d) C₂₋₆ alkynyl which may be substituted with 1 to 3 substituents selected from the Substituent Group A, (e) C₃₋₈ cycloalkyl which may be substituted with 1 to 3 substituents selected from the Substituent Group A, (f) C₆₋₁₄ aryl which may be substituted with 1 to 3 substituents selected from the Substituent Group A, (g) C₇₋₁₄ aralkyl which may be substituted with 1 to 3 substituents selected from the Substituent Group A, or (h) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); and R⁷' is (a) C₆₋₁₄ aryl which may be substituted with 1 to 3 substituents selected from the Substituent Group A, or (g) a 5- or 6-membered aromatic ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or R⁶' and R⁷' may form a ring together with the adjacent nitrogen atom,
(2) a group represented by the formula: 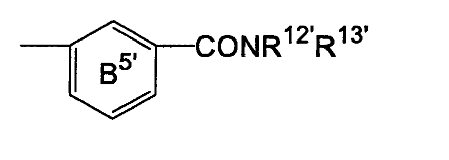 wherein ring B^{5'} is a benzene ring which may be further substituted with 1 to 3 substituents selected from the Substituent Group A; R^{12'} is (a) C₁₋₆ alkyl which may be substituted with 1 to 3 substituents selected from the Substituent Group A, (b) C₂₋₆ alkenyl which may be substituted with 1 to 3 substituents selected from the Substituent Group A, (c) C₂₋₆ alkynyl which may be substituted with 1 to 3 substituents selected from the Substituent Group A, (d) C₃₋₈ cycloalkyl which may be substituted with 1 to 3 substituents selected from the Substituent Group A, (e) C₆₋₁₄ aryl which may be substituted with 1 to 3 substituents selected from the Substituent Group A, (f) C₇₋₁₄ aralkyl which may be substituted with 1 to 3 substituents selected from the Substituent Group A, or (g) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); and R¹³ is (a) C₆₋₁₄ aryl which may be substituted with 1 to 3 substituents selected from the Substituent Group A, or (b) a 5- or 6-membered aromatic ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or R^{12'} and R^{13'} may form a ring together with the adjacent nitrogen atom, or
(3) a group represented by the formula: 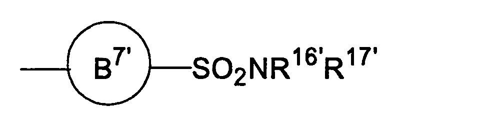 wherein ring B^{7'} is an aromatic heterocycle which may be substituted with 1 to 3 substituents selected from the Substituent Group A; R^{16'} is (a) a hydrogen atom, (b) C₁₋₆ alkyl which may be substituted with 1 to 3 substituents selected fro the Substituent Group A, (c) C₂₋₆ alkenyl which may be substituted with 1 to 3 substituents selected from the Substituent Group A, (d) C₂₋₆ alkynyl which may be substituted with 1 to 3 substituents selected from the Substituent Group A, (e) C₃₋₈ cycloalkyl which may be substituted with 1 to 3 substituents selected from the Substituent Group A, (f) C₆₋₁₄ aryl which may be substituted with 1 to 3 substituents selected from the Substituent Group A, (g) C₇₋₁₄ aralkyl which may be substituted with 1 to 3 substituents selected from the Substituent Group A, or (h) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); and R^{17'} is (a) C₆₋₁₄ aryl which may be substituted with 1 to 3 substituents selected from the Substituent Group A, or (b) a 5- or 6-membered aromatic ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or R^{16'} and R^{17'} may form a ring together with the adjacent nitrogen atom.

When R^{6'} and R^{7'}, R¹²' and R^{13'}, and R¹⁶' and R^{17'} respectively form a ring together with the adjacent nitrogen atom, the groups represented by -NR^{6'}R^{7'}, -NR^{12'}R^{13'} and -NR^{16'}R^{17'} are respectively a cyclic group selected from 1-pyrrolidinyl, 1-pyrrolinyl, 1-pyrrolyl, 1-imidazolidinyl, 1-imidazolinyl, 1-imidazolyl, 2-pyrazolidinyl, 2-pyrazolinyl, 1-pyrazolyl, 1-indolinyl, 1-indolyl, 2-isoindolinyl, 2-isoindolyl, 1-piperidyl, 3,4-dihydroquinolin-1(2H)-yl, 3,4-dihydroisoquinolin-2(1H)-yl, 1-piperazinyl, 3,4-dihydroquinoxalin-1(2H)-yl, 1H-azepin-1-yl, 2,3,4,5,6,7-hexahydro-1H-azepin-1-yl, 2,3,4,5-tetrahydro-1H-1-benzazepin-1-yl, 1,3,4,5-tetrahydro-2H-2-benzazepin-2-yl, 1,2,4,5-tetrahydro-3H-3-benzazepin-3-yl, 2,3-dihydro-4H-1,4-benzoxazin-4-yl, 3,4-dihydro-1,5-benzoxazepin-5(2H)-yl, 4-morpholinyl, 4-thiomorpholinyl, and the like. These cyclic groups may be substituted, and the substituents for the cyclic groups may be exemplified by those mentioned as the substituents which may be further carried by ring A described above, while the number of substituents is from 1 to 3.

Furthermore, in Formula (I-b): 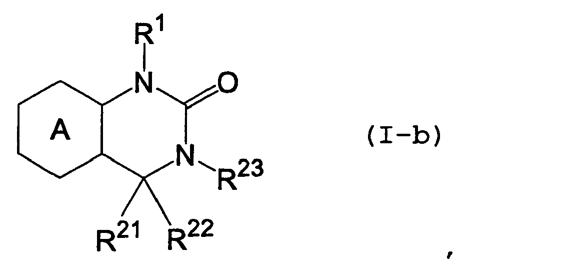 ring A and R¹ are as defined above.

R²¹ and R²², which may be identical or different, are each a hydrocarbon group which may be substituted or a hydrogen atom.

The "hydrocarbon group which may be substituted" represented by R²¹ and R²² may be exemplified by those mentioned as the "hydrocarbon group which may be substituted" represented by R³ described above.
R²³ is
(1) a group represented by the formula: 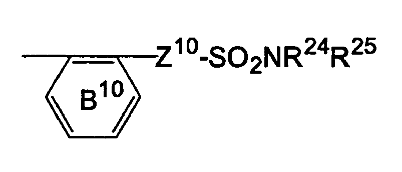 wherein ring B¹⁰ is a benzene ring which may be further substituted, Z¹⁰ is C₁₋₆ alkylene which may be substituted or a bond; and R²⁴ and R²⁵, which may be identical or different, are each (a) a hydrogen atom, (b) a hydrocarbon group which may be substituted, or (c) a heterocyclic group which may be substituted; or R²⁴ and R²⁵ may form a ring together with the adjacent nitrogen atom,
(2) a group represented by the formula: 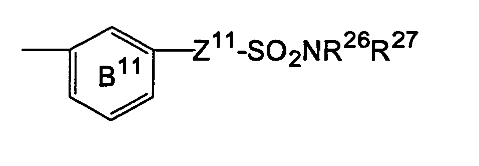 wherein ring B¹¹ is a benzene ring which may be further substituted; Z¹¹ is C₁₋₆ alkylene which may be substituted or a bond; and R²⁶ and R²⁷, which may be identical or different, are each (a) a hydrogen atom, (b) a hydrocarbon group which may be substituted, or (c) a heterocyclic group which may be substituted; or R²⁶ and R²⁷ may form a ring together with the adjacent nitrogen atom,
(3) a group represented by the formula: 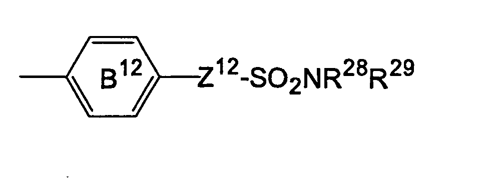 wherein ring B¹² is a benzene ring which may be further substituted; Z¹² is C₁₋₆ alkylene which may be substituted or a bond; and R²⁸ and R²⁹, which may be identical or different, are each (a) a hydrogen atom, (b) a hydrocarbon group which may be substituted, or (c) a heterocyclic group which may be substituted; or R²⁸ and R²⁹ may form a ring together with the adjacent nitrogen atom,
(4) a group represented by the formula: 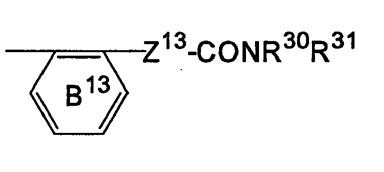 wherein ring B¹³ is a benzene ring which may be further substituted; Z¹³ is C₁₋₆ alkylene which may be substituted or a bond; and R³⁰ and R³¹, which may be identical or different, are each (a) a hydrogen atom, (b) a hydrocarbon group which may be substituted, or (c) a heterocyclic group which may be substituted; or R³⁰ and R³¹ may form a ring together with the adjacent nitrogen atom,
(5) a group represented by the formula: 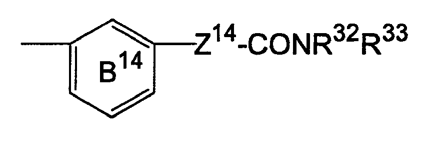 wherein ring B¹⁴ is a benzene ring which may be further substituted; Z¹⁴ is C₁₋₆ alkylene which may be substituted or a bond; and R³² and R³³, which may be identical or different, are each (a) a hydrogen atom, (b) a hydrocarbon group which may be substituted, or (c) a heterocyclic group which may be substituted; or R³² and R³³ may form a ring together with the adjacent nitrogen atom,
(6) a group represented by the formula: 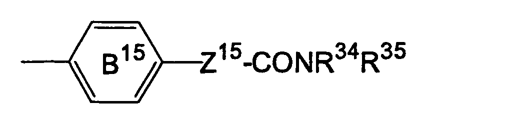 wherein ring B¹⁵ is a benzene ring which may be further substituted; Z¹⁵ is C₁₋₆ alkylene which may be substituted or a bond; and R³⁴ and R³⁵, which may be identical or different, are each (a) a hydrogen atom, (b) a hydrocarbon group which may be substituted, or (c) a heterocyclic group which may be substituted; or R³⁴ and R³⁵ may form a ring together with the adjacent nitrogen atom,
(7) a group represented by the formula: 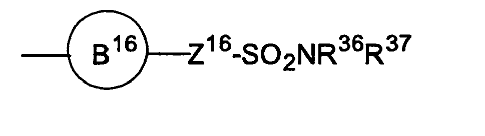 wherein ring B¹⁶ is a C₁₀₋₁₄ aryl ring which may be further substituted, or an aromatic heterocycle which may be further substituted; Z¹⁶ is C₁₋₆ alkylene which may be substituted or a bond; and R³⁶ and R³⁷, which may be identical or different, are each (a) a hydrogen atom, (b) a hydrocarbon group which may be substituted, or (c) a heterocyclic group which may be substituted; or R³⁶ and R³⁷ may form a ring together with the adjacent nitrogen atom,
(8) a group represented by the formula: 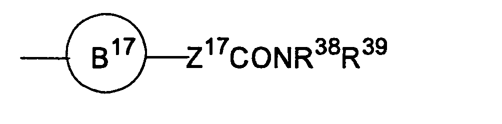 wherein ring B¹⁷ is a C₁₀₋₁₄ aryl ring which may be further substituted, or an aromatic heterocycle which may be further substituted; Z¹⁷ is C₁₋₆ alkylene which may be substituted or a bond; and R³⁸ and R³⁹, which may be identical or different, are each (a) a hydrogen atom, (b) a hydrocarbon group which may be substituted, or (c) a heterocyclic group which may be substituted; or R³⁸ and R³⁹ may form a ring together with the adjacent nitrogen atom, or
(9) a group represented by the formula: 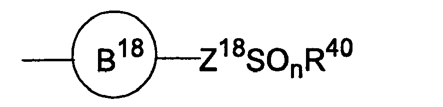 [wherein ring B¹⁸ is a C₆₋₁₄ aryl ring which may be further substituted, or an aromatic heterocycle which may be further substituted; Z¹⁸ is C₁₋₆ alkylene which may be substituted or a bond; R⁴⁰ is a hydrocarbon group which may be substituted, or a heterocyclic group which may be substituted; and n is an integer from 0 to 2, provided that the case where R²¹ and R²² are each a hydrogen atom, ring B¹⁸ is a thiazole ring, Z¹⁸ is methylene, and n is 2, and the case where R²¹ and R²² are each a dimethylaminophenyl, are excluded.

The "substituent" of the "benzene ring which may be further substituted" represented by ring B¹⁰ through ring B¹⁵ may be exemplified by those mentioned as the substituents which may be further carried by ring A described above, and the number of substituents is from 1 to 3.

The "C₁₀₋₁₄ aryl ring" of the "C₁₀₋₁₄ aryl ring which may be further substituted" represented by ring B¹⁶ and ring B¹⁷ may be exemplified by a naphthalene ring, a phenanthrene ring, an anthracene ring or the like.

The "substituent" of the "C₁₀₋₁₄ aryl ring which may be further substituted" represented by B¹⁶ and ring B¹⁷ may be exemplified by those mentioned as the substituents which may be further carried by ring A described above, and the number of substituents is from 1 to 3.

The "C₆₋₁₄ aryl ring" of the "C₆₋₁₄ aryl ring which may be further substituted" represented by ring B¹⁸ may be exemplified by a benzene ring, a naphthalene ring, a phenanthrene ring, an anthracene ring or the like.

The "substituent" of the "C₆₋₁₄ aryl ring which may be further substituted" represented by ring B¹⁸ may be exemplified by those mentioned as the substituents which may be further carried by ring A described above, and the number of substituents is from 1 to 3.

The "aromatic heterocycle" of the "aromatic cyclic ring which may be further substituted" represented by ring B¹⁶, ring B¹⁷ and ring B¹⁸ may be exemplified by a 5-membered aromatic heterocycle (e.g., a thiophene ring, a furan ring, a pyrrole ring, an oxazole ring, a thiazole ring, a pyrazole ring, an imidazole ring, an isoxazole ring, an isothiazole ring, a 1,2,4-oxadiazole ring, a 1,3,4-oxadiazole ring, a 1,2,4-thiadiazole ring, a 1,3,4-thiadiazole ring, a 1,2,3-triazole ring, a 1,2,4-triazole ring, a 1H-tetrazole ring, a 2H-tetrazole ring, etc.), a 6-membered aromatic heterocycle (e.g., a pyridine ring, a pyrimidine ring, a triazinine ring, a pyridazine ring, a pyrazine ring, etc.), a bicyclic or tricyclic fused aromatic heterocycle (e.g., a benzofuran ring, a benzothiazole ring, a benzoxazole ring, a tetrazolo[1,5-b]pyridazine ring, a triazolo[4,5-b]pyridazine ring, a benzoimidazole ring, a quinoline ring, an isoquinoline ring, a cinnoline ring, a phthalazine ring, a quinazoline ring, a quinoxaline ring, an indolizine ring, a quinolizine ring, a 1,8-naphthyridine ring, a purine ring, a pteridine ring, a dibenzofuran ring, a carbazole ring, an acridine ring, a phenanthridine ring, a chromane ring, a benzoxazine ring, a phenazine ring, a phenothiazine ring, a phenoxazine ring, an indole ring, etc.), or the like.

The "substituent" of the "aromatic heterocycle which may be further substituted" represented by ring B¹⁶, ring B¹⁷ and ring B¹⁸ may be exemplified by those mentioned as the substituents which may be further carried by ring A described above, and the number of substituents is from 1 to 3.

The "C₁₋₆ alkylene" of the "C₁₋₆ alkylene which may be substituted" represented by Z¹⁰ through Z¹⁸ may be exemplified by -CH₂-, -CH₂CH₂-, -CH₂CH₂CH₂-, -CH₂CH₂CH₂CH₂-, -CH₂CH₂CH₂CH₂CH₂-, or -CH₂CH₂CH₂CH₂CH₂CH₂-.

The "substituent" of the C₁₋₆ alkylene which may be substituted, represented by Z¹⁰ through Z¹⁸ may be exemplified by those mentioned as the substituents which may be further carried by ring A described above, and the number of substituents is from 1 to 3.

The "hydrocarbon group which may be substituted" represented by R²⁴ , R²⁵ , R²⁶ , R²⁷ , R²⁸ , R²⁹, R³⁰, R³¹, R³² , R³³ , R³⁴, R³⁵, R³⁶, R³⁷, R³⁸, R³⁹ and R⁴⁰ may be exemplified by those mentioned as the "hydrocarbon group which may be substituted" represented by R³ above.

The "heterocyclic group" of the "heterocyclic group which may be substituted" represented by R²⁴, R²⁵, R²⁶, R²⁷, R²⁸, R²⁹, R³⁰, R³¹, R³², R³³, R³⁴, R³⁵, R³⁶, R³⁷, R³⁸, R³⁹ and R⁴⁰ may be exemplified by a 5- or 6-membered ring group containing 1 to 4 heteroatoms selected from oxygen, sulfur and nitrogen atoms and the like in addition to carbon atoms (e.g., a 5-membered ring group such as 2-thienyl, 3-thienyl, 2-furyl, 3-furyl, 2-pyrrolyl, 3-pyrrolyl, 2-oxazolyl, 4-oxazolyl, 5-oxazolyl, 2-thiazolyl, 4-thiazolyl, 5-thiazolyl, 3-pyrazolyl, 4-pyrazolyl, 5-pyrazolyl, 2-imidazolyl, 4-imidazolyl, 5-imidazolyl, 3-isoxazolyl, 4-isoxazolyl, 5-isoxazolyl, 3-isothiazolyl, 4-isothiazolyl, 5-isothiazolyl, 3- (1,2,4-oxadiazolyl), 5-(1,2,4-oxadiazolyl), 1,3,4-oxadiazolyl, 3-(1,2,4-thiadiazolyl), 5-(1,2,4-thiadiazolyl), 1,3,4-thiadiazolyl, 4-(1,2,3-thiadiazolyl), 5-(1,2,3-thiadiazolyl), 1,2,5-thiadiazolyl, 1,2,3-triazolyl, 1,2,4-triazolyl, 1H-tetrazolyl, 2H-tetrazolyl, oxoimidazinyl, dioxotriazinyl or pyrrolidinyl; a 6-membered ring group such as 2-pyridyl, 3-pyridyl, 4-pyridyl, N-oxido-2-pyridyl, N-oxido-3-pyridyl, N-oxido-4-pyridyl, 2-pyrimidinyl, 4-pyrimidinyl, 5-pyrimidinyl, N-oxido-2-pyrimidinyl, N-oxido-4-pyrimidinyl, N-oxido-5-pyrimidinyl, 2-thiomorpholinyl, 3-thiomorpholinyl, 2-morpholinyl, 3-morpholinyl, piperidinyl, pyranyl, thiopyranyl, 1,4-oxazinyl, 1,4-thiazinyl, 1,3-thiazinyl, 2-piperazinyl, 3-piperazinyl, triazinyl, oxotriazinyl, 3-pyridazinyl, 4-pyridazinyl, pyrazinyl, N-oxido-3-pyridazinyl or N-oxido-4-pyridazinyl), or the like.

The "substituent" of the "heterocyclic group which may be substituted" may be exemplified by those mentioned as the substituents which may be further carried by ring A described above, and the number of substituents is from 1 to 3.

When R²⁴ and R²⁵, R²⁶ and R²⁷, R²⁸ and R²⁹, R³⁰ and R³¹, R³² and R³³, R³⁴ and R³⁵, R³⁶ and R³⁷, and R³⁸ and R³⁹ respectively form a ring together with the adjacent nitrogen atom, the group represented by -NR²⁴R²⁵, -NR²⁶R²⁷, -NR²⁸R²⁹, -NR³⁰R³¹, -NR³²R³³ - NR³⁴R³⁵, -NR³⁶R³⁷ and -NR³⁸R³⁹ is a cyclic group selected from 1-pyrrolidinyl, 1-pyrrolinyl, 1-pyrrolyl, azepan-1-yl, 1-imidazolidinyl, 1-imidazolinyl, 1-imidazolyl, 2-pyrazolidinyl, 2-pyrazolinyl, 1-pyrazolyl, 1-indolinyl, 1-indolyl, 2-isoindolinyl, 2-isoindolyl, 2,3-dihydro-1H-indol-1-yl, 1-piperidyl, 3,4-dihydro-1,5-naphthyridin-1(2H)-yl, 1,2,3,4-tetrahydroquinolin-1-yl, 3,4-dihydroquinolin-1(2H)-yl, 3,4-dihydroisoquinolin-2(1H)-yl, 1-piperazinyl, octahydroquinolin-1(2H)-yl, octahydroisoquinolin-2(1H)-yl, 3,4-dihydroquinoxalin-1(2H)-yl, 1H-azepin-1-yl, 2,3,4,5,6,7-hexahydro-1H-azepin-1-yl, 2,3,4,5-tetrahydro-1H-1-benzazepin-1-yl, 1,3,4,5-tetrahydro-2H-2-benzazepin-2-yl, 3,4,5,6-tetrahydro-1-benzazocin-1(2H)-yl, 2,3-dihydro-4,1-benzothiazepin-1(5H)-yl, 3,4-dihydro-1,5-benzothiazepin-5(2H)-yl, 2,3-dihydro-4,1-benzothiazepin-1(5H)-yl, 2,3-dihydro-4,1-benzoxazepin-1(5H)-yl, 1,2,4,5-tetrahydro-3H-3-benzazepin-3-yl, 2,3,4,5-tetrahydro-1H-1,5-benzodiazepin-1-yl, 2,3,4,5-tetrahydro-1H-1,4-benzodiazepin-1-yl, 2,3-dihydro-4H-1,4-benzoxazin-4-yl, 3,4-dihydro-2H-1,4-benzoxazin-1-yl, 3,4-dihydro-1,5-benzoxazepin-5(2H)-yl, 5,6,7,8-tetrahydro-4H-thieno[3,2-b]azepin-4-yl, 4-morpholinyl, 4-thiomorpholinyl, 2,3-dihydro-4H-pyrido[3,2-b][1,4]oxazin-4-yl, 2,3-dihydro-4H-1,4-benzothiazin-4-yl, 5,6,7,8-tetrahydropyrazolo[4,3-b]azepin-4(1H)-yl and the like. These cyclic groups may be substituted, and the substituent for the cyclic groups may be exemplified by those mentioned as the substituents which may be further carried by ring A described above, while the number of substituents is from 1 to 3.

As a preferred embodiment of Compound (I), mention may be made of Compound (I-a).

As a preferred embodiment of Compound (I), mention may be made of a compound represented by the formula: 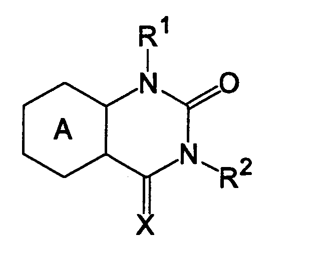 wherein ring A, X and R¹ are as defined above,
R² is
(1) a group represented by the formula: 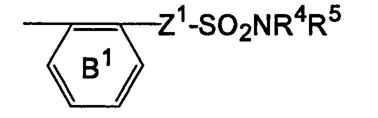 [wherein ring B¹, Z¹, R⁴ and R⁵ are as defined above],
(2) a group represented by the formula: 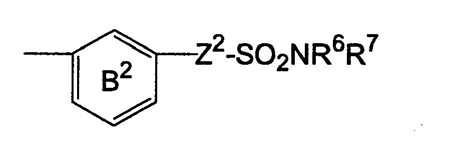 [wherein ring B², Z², R⁶ and R⁷ are as defined above],
(3) a group represented by the formula: 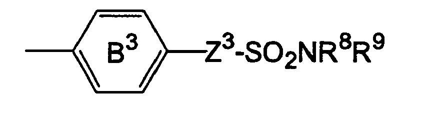 [wherein ring B³, Z³, R⁸ and R⁹ are as defined above, provided that when R⁸ is a hydrogen atom, R⁹ is (a) an aryl group which may be substituted, (b) an aralkyl group which may be substituted, or (c) an alkyl group substituted with a heterocyclic group which may be substituted],
(4) a group represented by the formula: 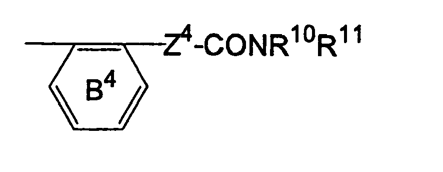 [wherein ring B⁴, Z⁴, R¹⁰ and R¹¹ are as defined above],
(5) a group represented by the formula: 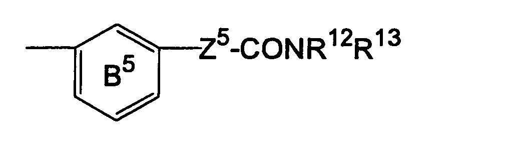 [wherein ring B⁵, Z⁵, R¹² and R¹³ are as defined above],
(6) a group represented by the formula: 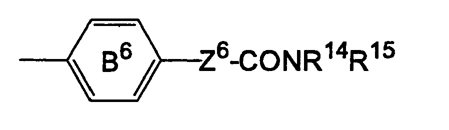 [wherein ring B⁶, Z⁶, R¹⁴ and R¹⁵ are as defined above],
(7) a group represented by the formula: 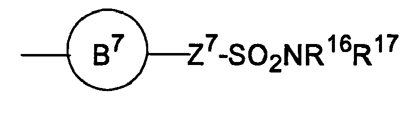 [wherein ring B⁷, Z⁷, R¹⁶ and R¹⁷ are as defined above], or
(8) a group represented by the formula: 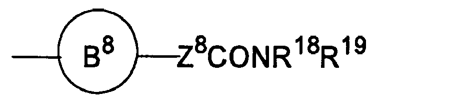 [wherein ring B⁸, Z⁸, R¹⁸ and R¹⁹ are as defined above]; provided that
   7-chloro-1-[4-(1,4-dihydro-2,4-dioxo-3(2H)-quinazolinyl)benzoyl]-2,3,4,5-tetrahydro-1H-1-benzazepine, and
   N,N-dibutyl-4-(1,4-dihydro-2,4-dioxo-3(2H)-quinazolinyl)benzenesulfonamide are excluded.

As another preferred embodiment of Compound (I), mention may be made of compounds wherein
ring B², ring B⁵, ring B¹¹ and ring B¹⁴, which may be identical or different, are further substituted with (a) C₆₋₁₄ aryl which may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen, (b) cyano, (c) hydroxy, (d) carboxy, (e) nitro, (f) C₁₋₆ alkyl which may be substituted with di-C₁₋₆ alkylamino, (g) C₁₋₆ alkoxy, (h) C₁₋₆ alkylcarbonyloxy, (i) C₁₋₆ alkylthio, (j) C₁₋₆ alkylsulfinyl, (k) C₁₋₆ alkylsulfonyl, (1) halogen, (m) amino, (n) mono-C₁₋₆ alkylamino, (o) di-C₁₋₆ alkylamino, (p) a 5-membered ring group containing 1 to 4 heteroatoms selected from oxygen, sulfur and nitrogen atoms and the like in addition to carbon atoms (wherein this 5-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (q) a 6-membered ring group containing 1 to 4 heteroatoms selected from oxygen, sulfur and nitrogen atoms and the like in addition to carbon atoms (wherein this 6-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (r) a bicyclic or tricyclic fused ring group containing 1 to 4 heteroatoms selected from oxygen, sulfur and nitrogen atoms and the like in addition to carbon atoms (wherein this bicyclic or tricyclic fused ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (s) C₁₋₆ alkoxycarbonyl, (t) carbamoyl, (u) N-mono-C₁₋₆ alkylcarbamoyl, (v) N, N-di-C₁₋₆ alkylcarbamoyl, and (w) C₁₋₂ alkylenedioxy (hereinafter (a) - (w) are briefly referred to as Substituent Group A);
ring B⁷, ring B⁸, ring B¹⁶ and ring B¹⁷, which may be identical or different, are each an aromatic heterocycle which may be further substituted with 1 to 3 substituents selected from the Substituent Group A;
Z², Z⁵, Z⁷, Z⁸, Z¹¹, Z¹⁴, Z¹⁶ and Z¹⁷, which may be identical or different, are each C₁₋₆ alkylene which may be substituted or a bond;
R⁶, R¹², R¹⁶, R¹⁸, R²⁶, R³², R³⁶ and R³⁸, which may be identical or different, are each (a) a hydrogen atom, (b) C₁₋₆ alkyl which may be substituted with 1 to 3 substituents selected from the Substituent Group A, (c) C₂₋₆ alkenyl which may be substituted with 1 to 3 substituents selected from the Substituent Group A, (d) C₂₋₆ alkynyl which may be substituted with 1 to 3 substituents selected from the Substituent Group A, (e) C₃₋₈ cycloalkyl which may be substituted with 1 to 3 substituents selected from the Substituent Group A, (f) C₆₋₁₄ aryl which may be substituted with 1 to 3 substituents selected from the Substituent Group A, (g) C₇₋₁₄ aralkyl which may be substituted with 1 to 3 substituents selected from the Substituent Group A, or (h) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A), provided that R¹² is not a hydrogen atom; and
R⁷, R¹³, R¹⁷, R¹⁹, R²⁷, R³³, R³⁷ and R³⁹, which may be identical or different, are each (a) C₁₋₁₀ alkyl which may be substituted with 1 to 3 substituents selected from the Substituent Group A, (b) C₆₋₁₄ aryl which may be substituted with 1 to 3 substituents selected from the Substituent Group A, (c) a 5- or 6-membered aromatic ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or
R⁶ and R⁷, R¹² and R¹³ R¹⁶ and R¹⁷, R¹⁸ and R¹⁹, R²⁶ and R²⁷, R³² and R³³, R³⁶ and R³⁷, and R³⁸ and R³⁹ may respectively form a ring together with the adjacent nitrogen atom.
As another preferred embodiment of Compound (I), mention may be made of compounds wherein
ring A is a benzene ring; X is an oxygen atom or NH; R¹ is a hydrogen atom or C₁₋₆ alkyl;
ring B², ring B⁵, ring B¹¹ and ring B¹⁴ may be identical or different, and are each a benzene ring which may be further substituted with one substituent selected from halogen (e.g., a fluorine atom, a chlorine atom, a bromine atom, an iodine atom, etc.), lower alkyl (e.g., C₁₋₆ alkyl, etc.) and cyano;
ring B⁷, ring B⁸, ring B¹⁶ and ring B¹⁷ may be identical or different, and are each a thiophene ring or furan ring which may be further substituted;
Z², Z⁵, Z⁷, Z⁸, Z¹¹, Z¹⁴, Z¹⁶ and Z¹⁷ may be identical or different, and are each C₁₋₆ alkylene which may be substituted or a bond;
R⁶, R¹², R¹⁶, R¹⁸, R²⁶, R³², R³⁶ and R³⁸ may be identical or different, and are each C₁₋₆ alkyl which may be substituted with cyano; and
R⁷, R¹³, R¹⁷, R¹⁹, R²⁷, R³³, R³⁷ and R³⁹ may be identical or different, and are each (a) C₁₋₁₀ alkyl which may be substituted with 1 to 3 substituents selected from the Substituent Group A, (b) C₆₋₁₄ aryl which may be substituted with halogen, or (c) pyridyl; or
R⁶ and R⁷ , R¹² and R¹³ , R¹⁶ and R¹⁷, R¹⁸ and R¹⁹, R²⁶ and R²⁷, R³² and R³³, R³⁶ and R³⁷, and R³⁸ and R³⁹ may respectively form, together with the adjacent nitrogen atom, (1) 1,2,3,4-tetrahydroquinolin-1-yl which may be substituted with C₁₋₆ alkyl, halogen (e.g., a fluorine atom, a chlorine atom, a bromine atom, an iodine atom, etc.) or C₁₋₆ alkoxy, (2) 2,3-dihydro-1H-indol-1-yl which may be substituted with C₁₋₆ alkyl, (3) 2,3,4,5-tetrahydro-1H-1-benzazepin-1-yl which may be substituted with C₁₋₆ alkyl, or (4) 3,4-dihydro-2H-1,4-benzoxazin-1-yl.

In particular, Compound (I) wherein X is NH and R¹ is a hydrogen atom, is preferred.

Further, Compound (I) wherein ring B², ring B⁵, ring B¹¹ and ring B¹⁴, which may be identical or different, are each a benzene ring which may be further substituted with one chlorine atom, is preferred, and Compound (I) wherein the above-mentioned rings are each an unsubstituted benzene ring, is particularly preferred.

Furthermore, Compound (I) wherein ring B⁷, ring B⁸, ring B¹⁶ and ring B¹⁷, which may be identical or different, are each a thiophene ring or furan ring which may be further substituted with one substituent selected from halogen, cyano and methyl, is preferred, and Compound (I) wherein the above-mentioned rings are each a thiophene ring or furan ring which may be substituted with one substituent selected from a chlorine atom and cyano, is particularly preferred.

As another preferred embodiment of Compound (I), mention may be made of compounds wherein Z², Z⁵, Z⁷, Z⁸, Z¹¹, Z¹⁴, Z¹⁶ and Z¹⁷ are each a bond.

As another preferred embodiment of Compound (I), for example, mention may be made of Compound (I) wherein ring A is a benzene ring; X is an oxygen atom or NH; R¹ is a hydrogen atom or C₁₋₆ alkyl; R² is
(1) a group represented by the formula: 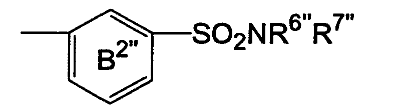 [wherein ring B²" is a benzene ring which may be further substituted with halogen, R⁶" is C₁₋₆ alkyl which may be substituted with cyano, and R^{7"} is C₆₋₁₄ aryl or pyridyl which may be substituted with halogen, or R^{6"} and R^{7"} form, together with the adjacent nitrogen atom, (1) 1,2,3,4-tetrahydroquinolin-1-yl which may be substituted with C₁₋₆ alkyl, halogen or C₁₋₆ alkoxy, (2) 2,3-dihydro-1H-indol-1-yl which may be substituted with C₁₋₆ alkyl, (3) 2,3,4,5-tetrahydro-1H-1-benzazepin-1-yl which may be substituted with C₁₋₆ alkyl, or (4) 3,4-dihydro-2H-1,4-benzoxazin-1-yl],
(2) a group represented by the formula: 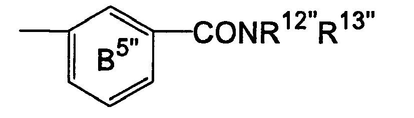 [wherein ring B^{5"} is a benzene ring which may be further substituted with halogen, R^{12"} is C₁₋₆ alkyl which may be substituted with cyano, and R^{13"} is C₆₋₁₄ aryl or pyridyl which may be substituted with halogen, or R^{12"} and R^{13"} form, together with the adjacent nitrogen atom, (1) 1,2,3,4-tetrahydroquinolin-1-yl which may be substituted with C₁₋₆ alkyl, halogen or C₁₋₆ alkoxy, (2) 2,3-dihydro-1H-indol-1-yl which may be substituted with C₁₋₆ alkyl, (3) 2,3,4,5-tetrahydro-1H-1-benzazepin-1-yl which may be substituted with C₁₋₆ alkyl, or (4) 3,4-dihydro-2H-1,4-benzoxazin-1-yl], or
(3) a group represented by the formula: 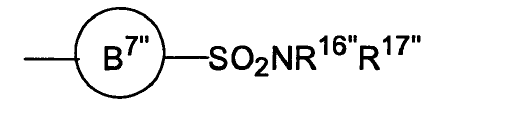 [wherein ring B⁷" is a thiophene ring which may be further substituted with halogen, R¹⁶" is C₁₋₆ alkyl which may be substituted with cyano, and R^{17"} is C₆₋₁₄ aryl or pyridyl which may be substituted with halogen, or R^{16"} and R^{17"} form, together with the adjacent nitrogen atom, (1) 1,2,3,4-tetrahydroquinolin-1-yl which may be substituted with C₁₋₆ alkyl, halogen or C₁₋₆ alkoxy, (2) 2,3-dihydro-1H-indol-1-yl which may be substituted with C₁₋₆ alkyl, (3) 2,3,4,5-tetrahydro-1H-1-benzazepin-1-yl which may be substituted with C₁₋₆ alkyl, or (4) 3,4-dihydro-2H-1,4-benzoxazin-1-yl], or the like.

More specifically, mention may be made of
3-(2-chloro-5-(3,4-dihydroquinolin-1(2H)-ylsulfonyl)phenyl)quinazoline-2,4(1H,3H)-dione,
4-chloro-3-(2,4-dioxo-1,4-dihydro-3(2H)-quinazolinyl)-N-methyl-N-2-pyridinylbenzenesulfonamide,
3-(2-chloro-5-((2-methyl-3,4-dihydro-1(2H)-quinolinyl)sulfonyl)phenyl)-2,4(1H,3H)-quinazolinedione,
3-(2-chloro-5-(2,3-dihydro-4H-1,4-benzoxazin-4-ylsulfonyl)phenyl)-2,4(1H,3H)-quinazolinedione,
3-(2-chloro-5-(2,3,4,5-tetrahydro-1H-1-benzazepin-1-ylsulfonyl)phenyl)-2,4(1H,3H)-quinazolinedione,
3-(2-chloro-5-(3,4-dihydro-1,5-benzoxazepin-5(2H)-ylsulfonyl)phenyl)-2,4(1H,3H)-quinazolinedione,
4-chloro-3-(4-imino-2-oxo-1,4-dihydro-3(2H)-quinazolinyl)-N-methyl-N-phenylbenzenesulfonamide trifluoroacetate,
3-(2-chloro-5-(3, 4-dihydro-1(2H) - quinolinylsulfonyl)phenyl)-4-imino-3,4-dihydro-2(1H)-quinazolinone trifluoroacetate,
3-(2-chloro-5-(2,3-dihydro-1H-indol-1-ylsulfonyl)phenyl)-4-imino-3,4-dihydro-2(1H)-quinazolinone,
3-(2-chloro-5-(3,4-dihydro-1(2H)-quinolinylcarbonyl)phenyl)-2,4(1H,3H)-quinazolinedione and the like.

More preferred compounds are
3-(2-chloro-5-(3,4-dihydroquinolin-1(2H)-ylsulfonyl)phenyl)quinazoline-2,4(1H,3H)-dione,
4-chloro-3-(2,4-dioxo-1,4-dihydro-3(2H)-quinazolinyl)-N-methyl-N-2-pyridinylbenzenesulfonamide trifluoroacetate,
3-(2-chloro-5-(2,3,4,5-tetrahydro-1H-1-benzazepin-1-ylsulfonyl)phenyl)-2,4(1H,3H)-quinazolinedione,
3-(2-chloro-5-(3,4-dihydro-1,5-benzoxazepin-5(2H)-ylsulfonyl)phenyl)-2,4(1H,3H)-quinazolinedione,
4-chloro-3-(4-imino-2-oxo-1,4-dihydro-3(2H)-quinazolinyl)-N-methyl-N-phenylbenzenesulfonamide trifluoroacetate, and
3-(2-chloro-5-(3,4-dihydro-1(2H)-quinolinylsulfonyl)phenyl)-4-imino-3,4-dihydro-2(1H)-quinazolinone trifluoroacetate.

For salts of Compound (I), salts of Compound (I) are preferably physiologically acceptable acid addition salts. Examples of such salt include salts with inorganic acids (e.g., hydrochloric acid, hydrobromic acid, nitric acid, sulfuric acid, phosphoric acid, etc.), or salts with organic acids (e.g., formic acid, acetic acid, trifluoroacetic acid (TFA), fumaric acid, oxalic acid, tartaric acid, maleic acid, citric acid, succinic acid, malic acid, methanesulfonic acid, benzenesulfonic acid, p-toluenesulfonic acid, etc.), and the like. When Compound (I) has an acidic group, the compound may form a physiologically acceptable salt with an inorganic base (e.g., alkali metals or alkaline earth metals such as sodium, potassium, calcium and magnesium, ammonia, etc.), or an organic base (e.g., trimethylamine, triethylamine, pyridine, picoline, ethanolamine, diethanolamine, triethanolamine, dicyclohexylamine, N,N'-dibenzylethylenediamine, etc.).

Compound (I) or a salt thereof is particularly preferably
3-[5-(3,4-dihydroquinolin-1 (2H) -ylsulfonyl) -2-fluorophenyl]quinazoline-2,4(1H,3H)-dione,
3-[2-chloro-5-(5,6,7,8-tetrahydro-4H-thieno[3,2-b]azepin-4-ylsulfonyl)phenyl]quinazoline-2,4(1H,3H)-dione,
4-chloro-3-(2,4-dioxo-1,4-dihydroquinazolin-3(2H)-yl)-N-methyl-N-phenylbenzamide,
5-chloro-4-[5-(hydroxymethyl)-2,4-dioxo-1,4-dihydroquinazolin-3(2H)-yl]-N-methyl-N-phenylthiophene-2-carboxamide,
Methyl 3-[2-chloro-5-(3,4-dihydroquinolin-1(2H)-ylsulfonyl)phenyl]-2,4-dioxo-1,2,3,4-tetrahydroquinazoline-5-carboxylate,
3-[2-chloro-5-(3,4-dihydroquinolin-1(2H)-ylsulfonyl)phenyl]-2,4-dioxo-1,2,3,4-tetrahydroquinazoline-5-carboxylic acid,
3-[2-chloro-5-(3,4-dihydroquinolin-1(2H)-ylsulfonyl)phenyl]-5-(hydroxymethyl)quinazoline-2,4(1H,3H)-dione,
3-(2,4-dioxo-1,4-dihydroquinazolin-3(2H)-yl)-5-(2,3,4,5-tetrahydro-1H-1-benzazepin-1-ylsulfonyl)thiophene-2-carbonitrile,
4-chloro-3-[5-(hydroxymethyl)-2,4-dioxo-1,4-dihydroquinazolin-3(2H)-yl]-N-methyl-N-phenylbenzamide,
4-chloro-3-[5-(hydroxymethyl)-2,4-dioxo-1,4-dihydroquinazolin-3(2H)-yl]-N-methyl-N-pyridin-2-ylbenzamide,
3-[2-chloro-5-(2,3,4,5-tetrahydro-1H-1-benzazepin-1-ylsulfonyl)-3-thienyl]-5-(hydroxymethyl)quinazoline-2,4(1H,3H)-dione,
5-chloro-N-cyclohexyl-4-[5-(hydroxymethyl)-2,4-dioxo-1,4-dihydroquinazolin-3(2H)-yl]-N-methylthiophene-2-carboxamide,
5-chloro-N-(2-chlorophenyl)-4-[5-(hydroxymethyl)-2,4-dioxo-1,4-dihydroquinazolin-3(2H)-yl]-N-methylthiophene-2-carboxamide,
5-chloro-N-(3-chlorophenyl)-4-[5-(hydroxymethyl)-2,4-dioxo-1,4-dihydroquinazolin-3(2H)-yl]-N-methylthiophene-2-carboxamide,
5-chloro-N-(4-chlorophenyl)-4-[5-(hydroxymethyl)-2,4-dioxo-1,4-dihydroquinazolin-3(2H)-yl]-N-methylthiophene-2-carboxamide,
5-chloro-4-[5-(hydroxymethyl)-2,4-dioxo-1,4-dihydroquinazolin-3(2H)-yl]-N-methyl-N-(2-methylphenyl)thiophene-2-carboxamide,
5-chloro-4-[5-(hydroxymethyl)-2,4-dioxo-1,4-dihydroquinazolin-3(2H)-yl]-N-methyl-N-(3-methylphenyl)thiophene-2-carboxamide,
3-[2-chloro-5-(3,4-dihydroquinolin-1(2H)-ylsulfonyl)phenyl]-2,4-dioxo-1,2,3,4-tetrahydroquinazoline-5-carboxamide,
3-[2-chloro-5-(3,4-dihydroquinolin-1(2H)-ylsulfonyl)phenyl]-3,4-dihydroquinazolin-2(1H)-one,
3-[2-chloro-5-(3,4-dihydroquinolin-1(2H)-ylsulfonyl)phenyl]-2-oxo-1,2,3,4-tetrahydroquinazoline-5-carboxylic acid,
5-chloro-4-[5-(hydroxymethyl)-2,4-dioxo-1,4-dihydroquinazolin-3(2H)-yl]-N-isobutyl-N-methylthiophenyl-2-carboxamide, and
3-[2-chloro-5-(3,4-dihydroquinolin-1(2H)-ylsulfonyl)phenyl]-5-(1-hydroxy-1-methylethyl)quinazoline-2,4(1H,3H)-dione.

Compound (I) can be produced by a method known per se, for example, the following production method. The solvent used for the production is not particularly limited as long as it is used in an amount that allows the mixture to be stirred. The compounds in the reaction schemes may be in the salt form, and the salts may be exemplified by those mentioned as the salts of Compound (I).

### [Method A]

Compound (I-a-a), which is Compound (I-a) of the invention wherein X is a sulfur atom, can be synthesized by, for example, the following method or the like: 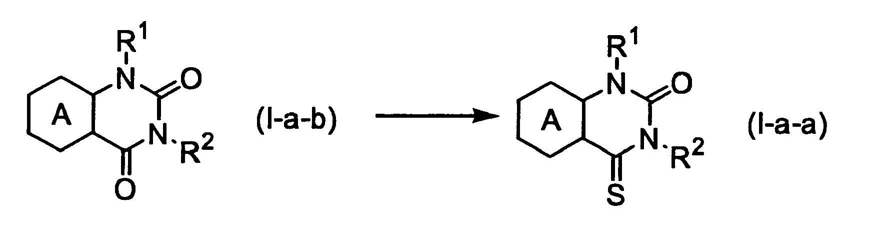 wherein the reference symbols have the meanings as defined above.

In the present method, Compound (I-a-a) is produced by reacting Compound (I-a-b) with an appropriate sulfide. This reaction is carried out according to a standard method in a solvent that has no adverse effect on the reaction.

The sulfide that can be used may be exemplified by Lawesson's Reagent, phosphorus pentasulfide or the like. The amount of the sulfide to be used is preferably 1 equivalent to a large excess (preferably 1 to 10 equivalents) with respect to Compound (I-a-b).

The solvent that has no adverse effect on the reaction may be exemplified by ethers (e.g., tetrahydrofuran, dioxane, diethyl ether, etc.), halogenated aliphatic hydrocarbons (e.g., dichloromethane, chloroform, etc.), aliphatic hydrocarbons (e.g., hexane, pentane, etc.), aromatic hydrocarbons (e.g., benzene, toluene, etc.), or the like. These solvents may be used in mixtures at appropriate ratios.

The reaction temperature is usually about 0°C to about 200°C. The reaction time may be usually about 0.5 to about 20 hours.

### [Method B]

Compound (I-a-c), which is Compound (I-a) of the invention wherein R¹ is not a hydrogen atom, can be synthesized by, for example, the following method or the like: 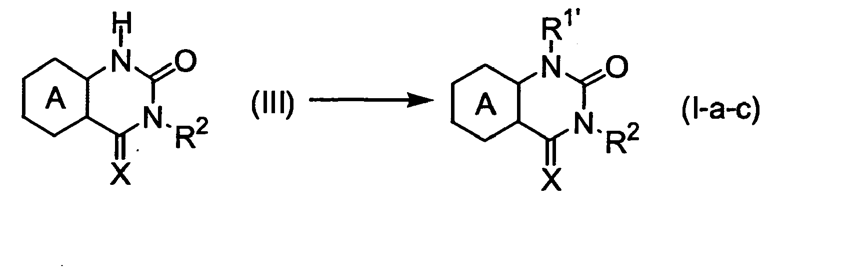 wherein R¹' is a hydrocarbon group which may be substituted, and the other reference symbols have the meanings as defined above.

The "hydrocarbon group which may be substituted" represented by R^{1'} may be exemplified by those mentioned as the "hydrocarbon group which may be substituted" represented by R¹.

The present method is a method of obtaining Compound (I-a-c) by reacting Compound (III) with a compound represented by the formula: R^{1'}-L [wherein R^{1'} has the meaning as defined above, L is a leaving group (e.g., a halogen atom (e.g., fluorine, chlorine, bromine, iodine, etc.), a C₁₋₆ alkylsulfonyloxy group which may be substituted with 1 to 3 halogen atoms (e.g., methanesulfonyloxy, ethanesulfonyloxy, trifluoromethanesulfonyloxy, etc.), an arylsulfonyloxy group which may be substituted (e.g., benzenesulfonyloxy, p-toluenesulfonyloxy, p-bromobenzenesulfonyloxy, etc.), etc.)]. This reaction is carried out according to a standard method in a solvent that has no adverse effect on the reaction.

The leaving group represented by L may be exemplified by halogen (e.g., fluorine, chlorine, bromine, iodine), C₁₋₆ alkylsulfonyl which may be substituted with halogen (e.g., fluorine, chlorine, bromine, iodine) (e.g., methanesulfonyl, ethanesulfonyl, trifluoromethanesulfonyl, 2,2,2-trifluoroethanesulfonyl, etc.), or the like.

In this reaction, the compound represented by the formula: R^{1'}-L is used in an amount of 1 equivalent to a large excess (preferably 1 to 10 equivalents) with respect to Compound (III). For this, for example, a basic compound such as sodium hydroxide, potassium hydroxide, sodium hydride, potassium carbonate, triethylamine, diisopropylethylamine, pyridine, 4-N,N-dimethylaminopyridine (DMAP), 1,8-diazabicyclo[5.4.0]-7-undecene, or 1,4-diazabicyclo[2.2.2]octane (DABCO) may be used in an amount of 1 to 10 equivalents. In addition, an alkali metal iodide such as sodium iodide may be added to the reaction as a reaction promoter, in an amount of 1 equivalent to a large excess (preferably 1 to 10 equivalents).

The solvent that has no adverse effect on the reaction may be exemplified by water, alcohols (e.g., methanol, ethanol, etc.), ethers (e.g., tetrahydrofuran, dioxane, diethyl ether, etc.), halogenated aliphatic hydrocarbons (e.g., methylene chloride, chloroform, etc.), ketones (e.g., acetone, methyl ethyl ketone, etc.), aliphatic hydrocarbons (e.g., hexane, pentane, etc.), aromatic hydrocarbons (e.g., benzene, toluene, etc.), aprotic polar solvents (e.g., N,N-dimethylformamide, acetonitrile, dimethylsulfoxide, etc.), or the like. These solvents may be used as a mixture at an appropriate ratio.

The reaction time is 10 minutes to 24 hours, preferably 0.5 to 6 hours. The reaction temperature may be -20°C to 200°C, preferably 0°C to 150°C.

### [Method B']

Compound (I-a-d), which is Compound (I-a) of the invention wherein R² is a group represented by the formula: 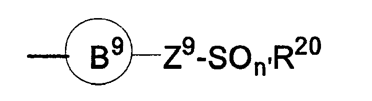 wherein n' is 1 or 2, and the other reference symbols have the meanings as defined above,
can be synthesized by, for example, the following method or the like: 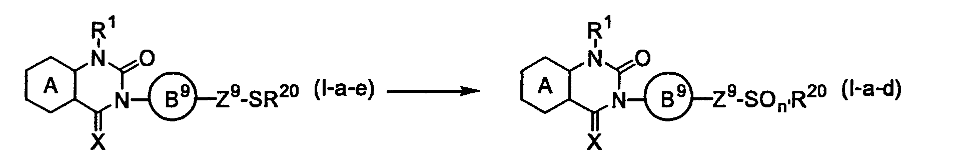 wherein the reference symbols have the meanings as defined above.

In the present method, Compound (I-a-d) is produced by oxidizing Compound (I-a-e). This oxidative reaction is carried out in the presence of an oxidizing agent. The oxidizing agent for this purpose may be exemplified by oxygen, hydrogen peroxide, organic peracids such as perbenzoic acid, m-chloroperbenzoic acid or peracetic acid; perchlorates such as lithium perchlorate, silver perchlorate or tetrabutylammonium perchlorate; periodates such as sodium periodate; periodic acid; manganese dioxide; lead tetraacetate; permanganates such as potassium permanganate; halogen such as iodine, bromine or chlorine; N-bromosuccinimide; N-chlorosuccinimide; sulfuryl chloride; chloramine T; or the like.

The present reaction is generally carried out in a solvent, and a solvent that does not impede the reaction is appropriately selected. Examples of such solvent include alcohols (e.g., methanol, ethanol, propanol, isopropanol, butanol, tert-butanol, etc.), ethers (e.g., dioxane, tetrahydrofuran, diethyl ether, tert-butyl methyl ether, diisopropyl ether, ethylene glycol, dimethyl ether, etc.), esters (e.g., ethyl formate, ethyl acetate, n-butyl acetate, etc.), carboxylic acids (e.g., formic acid, acetic acid, propionic acid, etc.), halogenated hydrocarbons (e.g., dichloromethane, chloroform, carbon tetrachloride, trichloroethylene, 1,2-dichloroethane, chlorobenzene, etc.), hydrocarbons (e.g., n-hexane, benzene, toluene, etc.), amides, (e.g., formamide, N,N-dimethylformamide, N,N-dimethylacetamide, etc.), ketones (e.g., acetone, methyl ethyl ketone, methyl isobutyl ketone, etc.), nitriles (e.g., acetonitrile, propionitrile, etc.), as well as sulfolane, hexamethyl phosphoramide, water and the like, which are used individually or in mixtures.

The present reaction can be carried out in the presence of base. Examples of such base that can be used include inorganic bases such as alkali metal hydroxides such as lithium hydroxide, sodium hydroxide and potassium hydroxide; alkaline earth metal hydroxides such as magnesium hydroxide and calcium hydroxide; alkali metal carbonates such as sodium carbonate and potassium carbonate; alkali metal hydrogen carbonates such as sodium hydrogen carbonate or potassium hydrogen carbonate, and the like.

The oxidizing agent is used in an amount of 0.1 to 20 equivalents, preferably about 0.4 to 10 equivalents, and the base is used in an amount of 0.1 to 20 equivalents, preferably about 0.4 to 10 equivalents, all with respect to Compound (I-a-e).

This reaction may be carried out in the presence of acid, if necessary, and examples of the acid that can be used include mineral acids such as hydrochloric acid, hydrobromic acid, sulfuric acid, phosphoric acid or perchloric acid; sulfonic acids such as methanesulfonic acid, ethanesulfonic acid, benzenesulfonic acid, toluenesulfonic acid or camphorsulfonic acid; and organic acids such as formic acid, acetic acid, propionic acid or trifluoroacetic acid. The amount of the acid to be used is 0.1 to 20 equivalents, preferably 0.5 to 10 equivalents, with respect to Compound (I-a-e).

The reaction temperature is about -10°C to about 250°C, preferably about -5°C to about 150°C. The reaction time may vary depending on Compound (I-a-e), the kind of base or solvent, reaction temperature or the like, but is usually about 1 minute to about 50 hours, preferably about 5 minutes to about 24 hours.

### [Method C]

Compound (III-a), which is Compound (I-a) of the invention wherein R¹ is a hydrogen atom and X is an oxygen atom (this compound includes Compound (I-a-b) wherein R¹ is a hydrogen atom, Compound (III) wherein X is an oxygen atom, or Compound (I-a-e) wherein R¹ is a hydrogen atom and X is an oxygen atom), can be synthesized by, for example, the following [Method C-1], [Method C-2] or the like. 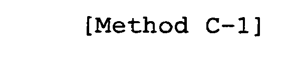 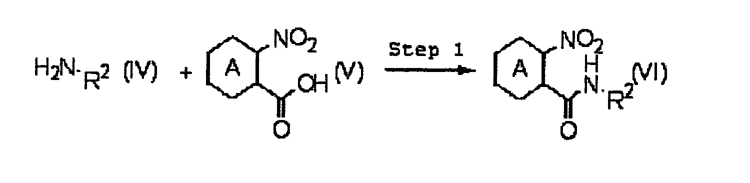 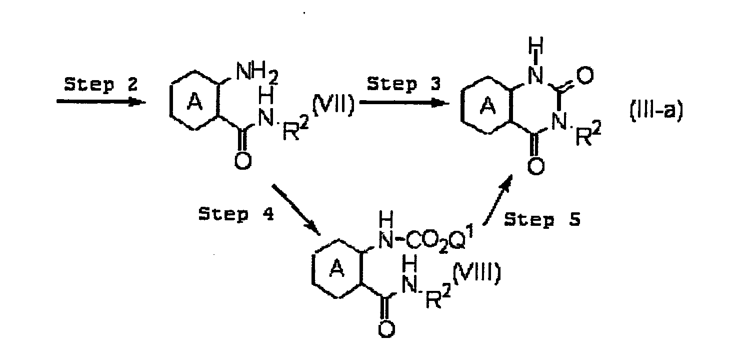 wherein Q¹ is a hydrocarbon group which may be substituted, and the other reference symbols have the meanings as defined above.

The "hydrocarbon group which may be substituted" represented by Q¹ may be exemplified by those mentioned as the "hydrocarbon group which may be substituted" represented by R³ above.

### [Step 1]

The present method is a method of obtaining Compound (VI) by subjecting Compound (IV) and Compound (V) to condensation (amidation). This reaction is carried out by a method known per se, for example,
(1) a method of directly condensing Compound (IV) and Compound (V) by using a condensing agent, or
(2) a method of allowing a reactive derivative of Compound (V) to undergo an appropriate reaction with Compound (IV).

First, Method (1) will be explained.

Examples of the condensing agent include carbodiimide-based condensing reagents such as dicyclohexylcarbodiimide, diisopropylcarbodiimide, 1-ethyl-3-(3-dimethylaminopropyl)carbodiimide and hydrochlorides thereof; phosphoric acid-based condensing reagents such as diethyl cyanophosphate and diphenylphosphoryl azide; and generally known condensing agents such as carbonyldiimidazole and 2-chloro-1,3-dimethylimidazolium tetrafluoroborate.

Method (1) is usually carried out in a solvent, and examples of the solvent include amides such as N,N-dimethylformamide and N,N-dimethylacetamide; halogenated aliphatic hydrocarbons such as chloroform and dichloromethane; aromatic hydrocarbons such as benzene and toluene; ethers such as tetrahydrofuran, dioxane and diethyl ether; ethyl acetate; water; and the like. These solvents may be used in mixtures at appropriate ratios.

The amount of Compound (IV) to be used is 0.1 to 10 molar equivalents, preferably 0.3 to 3 molar equivalents, with respect to Compound (V).

The amount of the condensing agent to be used is 0.1 to 10 molar equivalents, preferably 0.3 to 3 molar equivalents, with respect to Compound (V).

When a carbodiimide-based condensing reagent such as dicyclohexylcarbodiimide, diisopropylcarbodiimide, 1-ethyl-3-(3-dimethylaminopropyl)carbodiimide or a hydrochloride thereof is used as the condensing agent, an appropriate condensation promoting agent (e.g., 1-hydroxy-7-azabenzotriazole, 1-hydroxybenzotriazole, N-hydroxysuccinimide, N-hydroxyphthalimide, etc.) may be used, if desired. Further, when a phosphoric acid-based condensing agent such as diethyl cyanophosphate or diphenylphosphoryl azide is used as the condensing agent, an organic amine base such as triethylamine may be added.

The amount of the above-described condensation promoting agent or organic amine base to be used is 0.1 to 10 molar equivalents, preferably 0.3 to 3 molar equivalents, with respect to Compound (V).

The reaction temperature is usually -30°C to 100°C. The reaction time is usually 0.5 to 60 hours.

Next, Method (2) will be explained.

Examples of the reactive derivative of Compound (V) include acid anhydrides, acid halides (e.g., acid chloride, acid bromide), acid imidazolides, active esters (e.g., phenyl ester, nitro- or halogen-substituted phenyl ester (e.g., 4-nitrophenyl ester, pentafluorophenyl ester, etc.), 1-hydroxy-7-azabenzotriazole ester, 1-hydroxybenzotriazole ester, N-hydroxysuccinimide ester, N-hydroxyphthalimide ester, etc.), or mixed anhydride (e.g., anhydrides with methyl carbonate, ethyl carbonate or isobutyl carbonate, etc.), and the like.

Among the above-described reactive derivatives, for example, when an acid anhydride, an acid halide, an acid imidazolide, or an active ester is used, the reaction is carried out in the presence or absence of base, in a solvent that has no adverse reaction.

Examples of the base include amines such as triethylamine, N-methylmorpholine and N,N-dimethylaniline; alkali metal carbonates such as potassium carbonate and sodium carbonate; alkali metal hydrogen carbonates such as potassium hydrogen carbonate and sodium hydrogen carbonate; alkali metal hydroxides such as potassium hydroxide, sodium hydroxide and lithium hydroxide; and the like. The amount of the base to be used is 0.1 to 10 molar equivalents, preferably 0.3 to 3 molar equivalents, with respect to Compound (V) or a reactive derivative thereof.

Examples of the solvent that has no adverse effect on the reaction include water, ethers (e.g., tetrahydrofuran, dioxane, diethyl ether, etc.), halogenated aliphatic hydrocarbons (e.g., dichloromethane, chloroform, etc.), ketones (e.g., acetone, methyl ethyl ketone, etc.), esters (e.g., ethyl acetate, etc.), aprotic polar solvents (e.g., N,N-dimethylformamide, acetonitrile, dimethylsulfoxide, etc.), and the like. These solvents may be used in mixtures at appropriate ratios.

The amount of Compound (IV) to be used is 0.1 to 10 molar equivalents, preferably 0.3 to 3 molar equivalents, with respect to Compound (V) or a reactive derivative thereof.

The reaction temperature is usually -30°C to 100°C. The reaction time is usually 0.5 to 20 hours.

In addition, when an mixed anhydride is used, Compound (V) is reacted with a chlorocarbonate ester (e.g., methyl chlorocarbonate, ethyl chlorocarbonate, isobutyl chlorocarbonate, etc.) in the presence of base (e.g., amines such as triethylamine, N-methylmorpholine, N,N-dimethylaniline; alkali metal carbonates such as potassium carbonate and sodium carbonate; alkali metal hydrogen carbonates such as potassium hydrogen carbonate and sodium hydrogen carbonate; alkali metal hydroxides such as potassium hydroxide, sodium hydroxide and lithium hydroxide, etc.), and is further reacted with Compound (IV) .

The amount of Compound (IV) to be used is usually 0.1 to 10 molar equivalents, preferably 0.3 to 3 molar equivalents, with respect to Compound (V) or an mixed anhydride thereof.

The reaction temperature is usually -30°C to 100°C. The reaction time is usually 0.5 to 20 hours.

Compound (V) and the reactive derivative of Compound (V) that are used as the starting compounds in the present method can be produced by methods known per se, and are also commercially available.

### [Step 2]

In the present method, Compound (VII) is produced by reducing Compound (VI).

The reduction reaction is carried out by using a reducing agent such as, for example, a metal hydrogen complex (e.g., lithium aluminum hydride, etc.), a metal (e.g., zinc, iron, tin, etc.) in the co-presence of acid (e.g., hydrochloric acid, sulfuric acid, nitric acid, etc.), stannous chloride in the co-presence of acid (e.g., hydrochloric acid, sulfuric acid, nitric acid, etc.), or zinc under neutral or basic conditions. The reducing agent is used in an amount of 1 equivalent to a large excess (preferably 1 to 10 equivalents).

Further, this reduction reaction is also carried out as contact reduction at ambient pressure or under increased pressure, in the presence of a catalyst (a metal such as platinum, palladium, nickel or rhodium, or oxide, salt, complex and the like thereof. Such catalyst can be also used in the form of being supported on various supports such as carbon).

The solvent used in the reaction can be appropriately selected according to the method of reduction, and may be exemplified by water, alcohols (e.g., methanol, ethanol, etc.), ethers (e.g., tetrahydrofuran, dioxane, diethyl ether, etc.), halogenated aliphatic hydrocarbons (e.g., dichloromethane, chloroform, etc.), aliphatic hydrocarbons (e.g., hexane, pentane, etc.), aromatic hydrocarbons (e.g., benzene, toluene, etc.), aprotic polar solvents (e.g., N,N-dimethyl formamide, dimethylsulfoxide, acetonitrile, etc.), or the like. When the acid used in the reduction by a metal or stannous chloride is liquid, this acid can be used as the solvent. These solvents may be used in mixtures at appropriate ratios.

The reaction time is 0.1 to 72 hours, preferably 0.1 to 24 hours. The reaction temperature may be -30°C to 150°C, preferably 0°C to 80°C.

### [Step 3]

In the present method, Compound (III-a) is produced by reacting Compound (VII) with a reagent such as phosgene, diphosgene, triphosgene or N,N'-carbonyldiimidazole. The amount of such reagent to be used is preferably 1 equivalent to a large excess (preferably 1 to 5 equivalents) with respect to Compound (VII). The reaction is carried out in the presence or absence of base, in a solvent that has no adverse effect on the reaction.

Examples of the base include amines such as triethylamine, pyridine and N,N-dimethylaniline; alkali metal carbonates such as potassium carbonate and sodium carbonate; alkali metal hydrogen carbonates such as potassium hydrogen carbonate and sodium hydrogen carbonate; alkali metal hydroxides such as potassium hydroxide, sodium hydroxide and lithium hydroxide; and the like. The amount of the base to be used is 0.1 to 10 molar equivalents, preferably 0.3 to 3 molar equivalents, with respect to Compound (VII).

Examples of the solvent that has no adverse effect on the reaction include ethers (e.g., tetrahydrofuran, dioxane, diethyl ether, etc.), halogenated aliphatic hydrocarbons (e.g., dichloromethane, chloroform, etc.), ketones (e.g., acetone, methyl ethyl ketone, etc.), esters (e.g., ethyl acetate, etc.), aprotic polar solvents (e.g., N,N-dimethylformamide, acetonitrile, dimethylsulfoxide, etc.) and the like. These solvents may be used in mixtures at appropriate ratios.

The reaction temperature is usually -30°C to 100°C. The reaction time is usually 0.5 to 20 hours.

### [Step 4]

In the present method, Compound (VIII) is produced by reacting Compound (VII) with a halocarbonate ester represented by the formula: Hal-CO₂Q¹, wherein Hal is a halogen (e.g., fluorine, chlorine, bromine, iodine), and Q¹ has the meaning as defined above, or a compound represented by the formula: [Q¹OC(=O)]₂O, wherein Q¹ has the meaning as defined above. The amounts of these reagents to be used are preferably 1 equivalent to a large excess (preferably 1 to 5 equivalents) with respect to Compound (VII). The reaction is carried out in the presence or absence of base, in a solvent that has no adverse effect on the reaction.

Examples of the base include amines such as triethylamine, pyridine and N,N-dimethylaniline; alkali metal carbonates such as potassium carbonate and sodium carbonate; alkali metal hydrogen carbonates such as potassium hydrogen carbonate and sodium hydrogen carbonate; alkali metal hydroxides such as potassium hydroxide, sodium hydroxide and lithium hydroxide; and the like. The amount of the base to be used is 0.1 to 10 molar equivalents, preferably 0.3 to 3 molar equivalents, with respect to Compound (VII).

Examples of the solvent that has no adverse effect on the reaction include water, ethers (e.g., tetrahydrofuran, dioxane, diethyl ether, etc.), halogenated aliphatic hydrocarbons (e.g., dichloromethane, chloroform, etc.), ketones (e.g., acetone, methyl ethyl ketone, etc.), esters (e.g., ethyl acetate, etc.), aprotic polar solvents (e.g., N,N-dimethylformamide, acetonitrile, dimethylsulfoxide, etc.) and the like. These solvents may be used in mixtures at appropriate ratios.

The reaction temperature is usually -30°C to 100°C. The reaction time is usually 0.5 to 20 hours.

The product may be used in the subsequent reaction as a reaction liquid or as a crude product. However, the product may be also isolated from the reaction mixture by a standard method.

The halocarbonate ester represented by the formula: Hal-CO₂Q¹ [wherein the reference symbols have the meanings as defined above] and the compound represented by the formula: [Q¹OC(=O)]₂O [wherein Q¹ has the meaning as defined above] that are used as the starting compounds in the present method can be produced by methods known per se, and are also commercially available.

### [Step 5]

In the present method, Compound (III-a) is produced by cyclizing Compound (VIII). The reaction is carried out in the presence or absence of base, without solvent or in a solvent that has no adverse effect on the reaction.

Examples of the base include amines such as triethylamine, pyridine and N,N-dimethylaniline; alkali metal carbonates such as potassium carbonate and sodium carbonate; alkali metal hydrogen carbonates such as potassium hydrogen carbonate and sodium hydrogen carbonate; alkali metal hydroxides such as potassium hydroxide, sodium hydroxide and lithium hydroxide; and the like. The amount of the base to be used is 0.1 to 10 molar equivalents, preferably 0.3 to 3 molar equivalents, with respect to Compound (VIII).

Examples of the solvent that has no adverse effect on the reaction include ethers (e.g., tetrahydrofuran, dioxane, diethyl ether, etc.), halogenated aliphatic hydrocarbons (e.g., dichloromethane, chloroform, etc.), ketones (e.g., acetone, methyl ethyl ketone, etc.), esters (e.g., ethyl acetate, etc.), aprotic polar solvents (e.g., N,N-dimethylformamide, acetonitrile, dimethylsulfoxide, etc.) and the like. These solvents may be used in mixtures at appropriate ratios.

The reaction temperature is usually 0°C to 150°C. The reaction time is usually 0.5 to 36 hours. 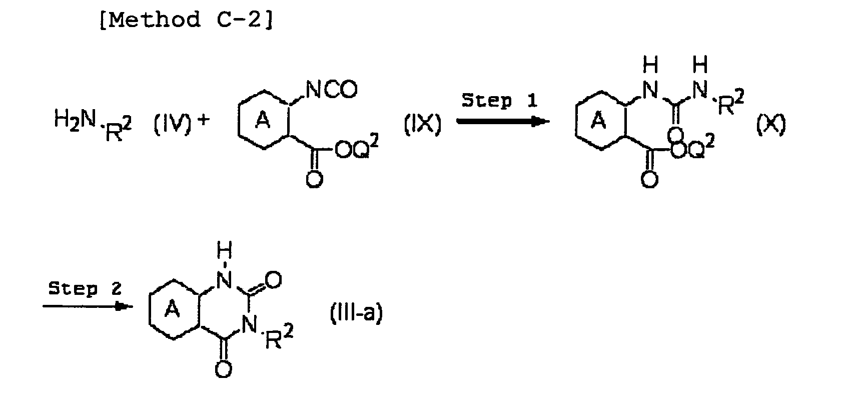 wherein Q² is a hydrocarbon group which may be substituted, and the other reference symbols have the meanings as defined above.

The "hydrocarbon group which may be substituted" represented by Q² may be exemplified by those mentioned as the "hydrocarbon group which may be substituted" represented by R³.

### [Step 1]

The present method is a method of obtaining Compound (X) by condensing Compound (IV) with Compound (IX).

The reaction is carried out in the presence or absence of base, in a solvent that has no adverse effect on the reaction.

The amount of Compound (IX) to be used is 0.1 to 10 molar equivalents, preferably 0.3 to 3 molar equivalents, with respect to Compound (IV).

Examples of the base include amines such as triethylamine, pyridine and N,N-dimethylaniline; alkali metal carbonates such as potassium carbonate and sodium carbonate; alkali metal hydrogen carbonates such as potassium hydrogen carbonate and sodium hydrogen carbonate; alkali metal hydroxides such as potassium hydroxide, sodium hydroxide and lithium hydroxide; and the like. The amount of the base to be used is 0.1 to 10 molar equivalents, preferably 0.3 to 3 molar equivalents, with respect to Compound (IV).

Examples of the solvent that has no adverse effect on the reaction include ethers (e.g., tetrahydrofuran, dioxane, diethyl ether, etc.), halogenated aliphatic hydrocarbons (e.g., dichloromethane, chloroform, etc.), ketones (e.g., acetone, methyl ethyl ketone, etc.), esters (e.g., ethyl acetate, etc.), aprotic polar solvents (e.g., N,N-dimethylformamide, acetonitrile, dimethylsulfoxide, etc.) and the like. These solvents may be used in mixtures at appropriate ratios.

The reaction temperature is usually 0°C to 150°C. The reaction time is usually 0.5 to 36 hours.

Compound (IX) that is used as the starting compound in the present method can be produced by a method known per se, and is also commercially available.

The product can be used in the subsequent reaction as a reaction liquid or as a crude product, but it can be also isolated from the reaction mixture by a standard method.

### [Step 2]

In the present method, Compound (III-a) is produced by cyclizing Compound (X). The reaction is carried out in the presence or absence of base, without solvent or in a solvent that has no adverse effect on the reaction.

Examples of the base include amines such as triethylamine, pyridine and N,N-dimethylaniline; alkali metal carbonates such as potassium carbonate and sodium carbonate; alkali metal hydrogen carbonates such as potassium hydrogen carbonate and sodium hydrogen carbonate; alkali metal hydroxides such as potassium hydroxide, sodium hydroxide and lithium hydroxide; and the like. The amount of the base to be used is 0.1 to 10 molar equivalents, preferably 0.3 to 3 molar equivalents, with respect to Compound (X).

Examples of the solvent that has no adverse effect on the reaction include ethers (e.g., tetrahydrofuran, dioxane, diethyl ether, etc.), halogenated aliphatic hydrocarbons (e.g., dichloromethane, chloroform, etc.), ketones (e.g., acetone, methyl ethyl ketone, etc.), esters (e.g., ethyl acetate, etc.), aprotic polar solvents (e.g., N,N-dimethylformamide, acetonitrile, dimethylsulfoxide, etc.) and the like. These solvents may be used in mixtures at appropriate ratios.

The reaction temperature is usually 0°C to 150°C. The reaction time is usually 0.5 to 36 hours.

### [Method D]

Compound (III-b), which is Compound (I-a) of the invention wherein X is NH, or Compound (III-c), which is Compound (I-a) of the invention wherein X is NR³ (wherein R³ is a hydrocarbon group which may be substituted) [Compound (III-b) or Compound (III-c) includes the compound which is Compound (III) wherein X is NR² (wherein R² has the meaning as defined above), the compound which is Compound (I-a-e) wherein X is NR² (wherein R² has the meaning as defined above), or the compound which is Compound (I-a-f) wherein X is NR² (R² has the meaning as defined above)], can be synthesized by, for example, the following method or the like. 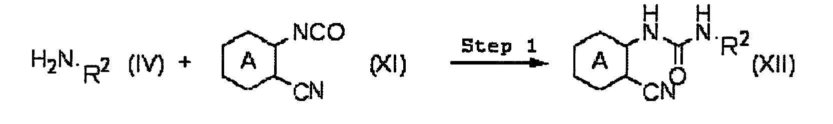 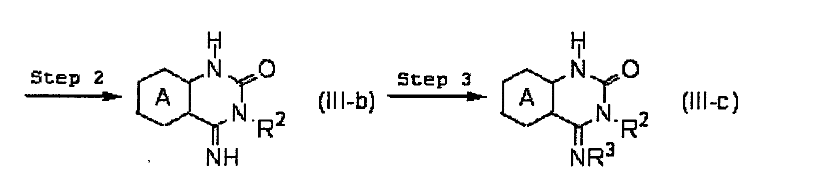 wherein the reference symbols have the meanings as defined above.

### [Step 1]

The present method is a method of obtaining Compound (XII) by condensing Compound (IV) with Compound (XI).

The present production method is carried out under the same reaction conditions as, for example, those of the method of producing Compound (X) by condensing Compound (IV) with Compound (IX) in [Step 1] of [Method C-2] described above.

Compound (XI) that is used as the starting compound in the present method can be produced by a method known per se, and is also commercially available.

The product can be used in the subsequent reaction as a reaction liquid or as a crude product, but it can be also isolated from the reaction mixture by a standard method.

### [Step 2]

In the present method, Compound (III-b) is produced by cyclizing Compound (XII).

The present production method is carried out under the same reaction conditions as, for example, those of the method of producing Compound (III-a) by cyclizing Compound (X) in [Step 2] of [Method C-2] described above.

### [Step 3]

In the present method, Compound (III-c) is produced by reacting Compound (III-b) with a compound represented by the formula: R³-L, wherein the reference symbols have the meanings as defined above.

The present production method is carried out under the same reaction conditions as, for example, those for the method of producing Compound (I-a-c) by reacting Compound (III) with a compound represented by the formula: R^{1'}-L (wherein the reference symbols have the meanings as defined above) in [Method B] described above.

Compound (IV) that is used as the starting compound in [Method C-1], [Method C-2] and [Method D] is produced by, for example, Method E as described below:

### [Method E]

O₂N-_{R}2 (XIII) → H₂N_{R}2 (IV)

wherein the reference symbol has the meaning as defined above.

In the present method, Compound (IV) is produced by reducing Compound (XIII).

The reduction method is carried out under the same reaction conditions as, for example, those of the method of producing Compound (VII) by reducing Compound (VI) in [Step 2] of [Method C-1] described above.

Compound (XIII) that is used as the starting compound in [Method E] is produced by, for example, [Method F] through [Method O] described below.

### [Method F]

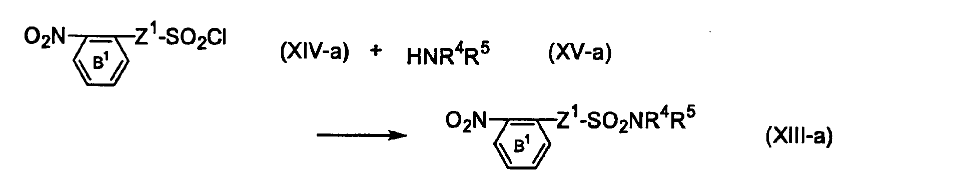

wherein the reference symbols have the meanings as defined above.

The present method is a method of obtaining Compound (XIII-a) by condensing Compound (XIV-a) with Compound (XV-a).

The present method is carried out in the presence or absence of base, in a solvent that has no adverse effect on the reaction.

Examples of the base include amines such as triethylamine, N-methylmorpholine and N,N-dimethylaniline; alkali metal carbonates such as potassium carbonate and sodium carbonate; alkali metal hydrogen carbonates such as potassium hydrogen carbonate and sodium hydrogen carbonate; alkali metal hydroxides such as potassium hydroxide, sodium hydroxide and lithium hydroxide; and the like. The amount of the base to be used is 0.1 to 10 molar equivalents, preferably 0.3 to 3 molar equivalents, with respect to Compound (XIV-a).

Examples of the solvent that has no adverse effect on the reaction include water, ethers (e.g., tetrahydrofuran, dioxane, diethyl ether, etc.), halogenated aliphatic hydrocarbons (e.g., dichloromethane, chloroform, etc.), ketones (e.g., acetone, methyl ethyl ketone, etc.), esters (e.g., ethyl acetate, etc.), aprotic polar solvents (e.g., N,N-dimethylformamide, acetonitrile, dimethylsulfoxide, etc.) and the like. These solvents may be used in mixtures at appropriate ratios.

The amount of Compound (XV-a) to be used is 0.1 to 10 molar equivalents, preferably 0.3 to 3 molar equivalents, with respect to Compound (XIV-a).

The reaction temperature is usually -30°C to 100°C. The reaction time is usually 0.5 to 20 hours.

Compound (XIV-a) and Compound (XV-a) that are used as the starting compounds in the present method can be produced by methods known per se, and are also commercially available. 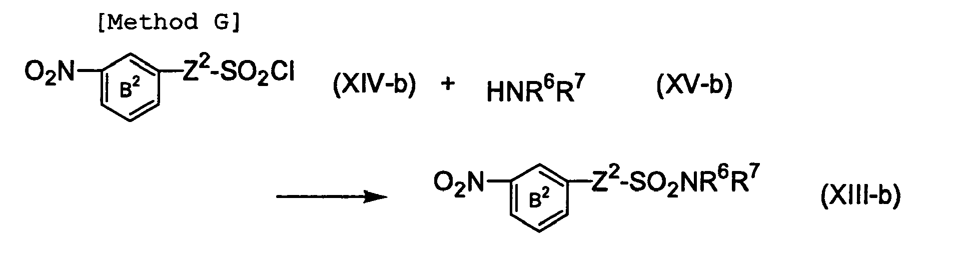 wherein the reference symbols have the meanings as defined above.

The present method is a method of obtaining Compound (XIII-b) by condensing Compound (XIV-b) with Compound (XV-b).

The present production method is carried out under the same reaction conditions as, for example, those of the method of producing Compound (XIII-a) by condensing Compound (XIV-a) with Compound (XV-a) in [Method F] described above.

Compound (XIV-b) and Compound (XV-b) that are used as the starting compounds in the present method can be produced by methods known per se, and are also commercially available. 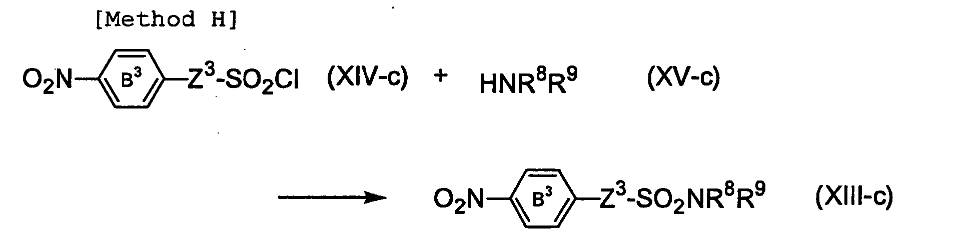 wherein the reference symbols have the meanings as defined above.

The present method is a method of obtaining Compound (XIII-c) by condensing Compound (XIV-c) with Compound (XV-c).

The present production method is carried out under the same reaction conditions as, for example, those of the method of producing Compound (XIII-a) by condensing Compound (XIV-a) with Compound (XV-a) in [Method F] described above.

Compound (XIV-c) and Compound (XV-c) that are used as the starting compounds in the present method can be produced by methods known per se, and are also commercially available. 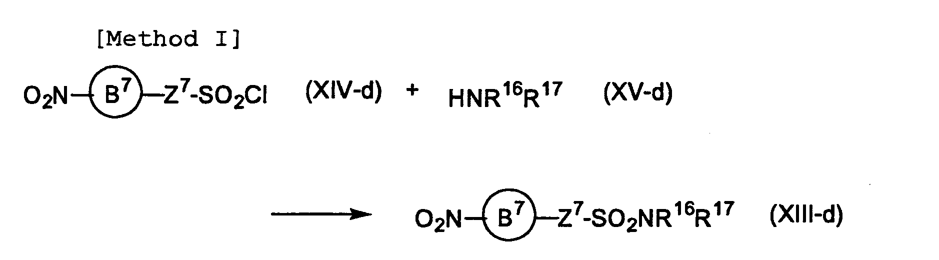 wherein the reference symbols have the meanings as defined above.

The present method is a method of obtaining Compound (XIII-d) by condensing Compound (XIV-d) with Compound (XV-d).

The present production method is carried out under the same reaction conditions as, for example, those of the method of producing Compound (XIII-a) by condensing Compound (XIV-a) with Compound (XV-a) in [Method F] described above.

Compound (XIV-d) and Compound (XV-d) that are used as the starting compounds in the present method can be produced by methods known per se, and are also commercially available. 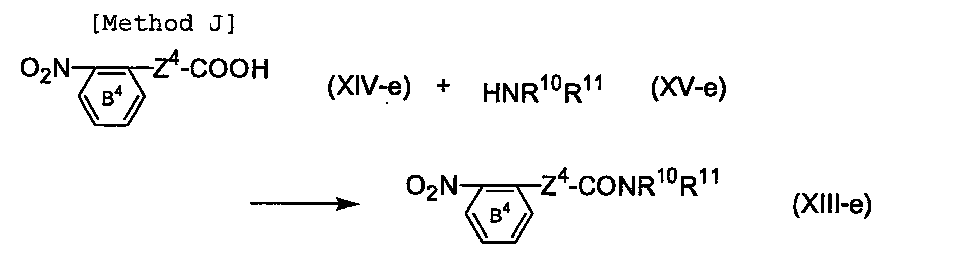 wherein the reference symbols have the meanings as defined above.

The present method is a method of obtaining Compound (XIII-e) by condensing Compound (XIV-e) with Compound (XV-e).

The present production method is carried out under the same reaction conditions as, for example, those of the method of producing Compound (VI) by condensing Compound (IV) with Compound (V) in [Step 1] of [Method C-1] described above.

Compound (XIV-e) (or a reactive derivative of Compound (XIV-e) and Compound (XV-e) that are used as the starting compounds in the present method can be produced by methods known per se, and are also commercially available. 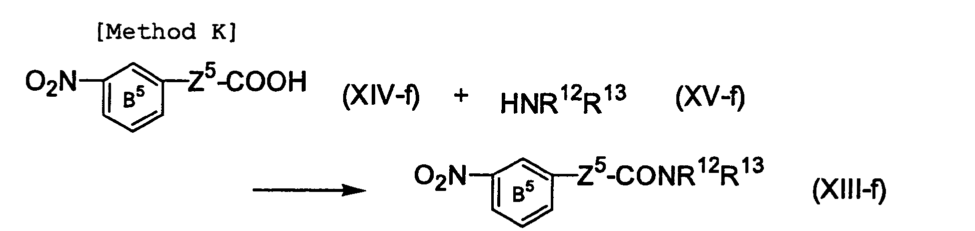 wherein the reference symbols have the meanings as defined above.

The present method is a method of obtaining Compound (XIII-f) by condensing Compound (XIV-f) with Compound (XV-f).

The present production method is carried out under the same reaction conditions as, for example, those of the method of producing Compound (VI) by condensing Compound (IV) with Compound (V) in [Step 1] of [Method C-1] described above.

Compound (XIV-f) (or a reactive derivative of Compound (XIV-f) and Compound (XV-f) that are used as the starting compounds in the present method can be produced by methods known per se, and are also commercially available. 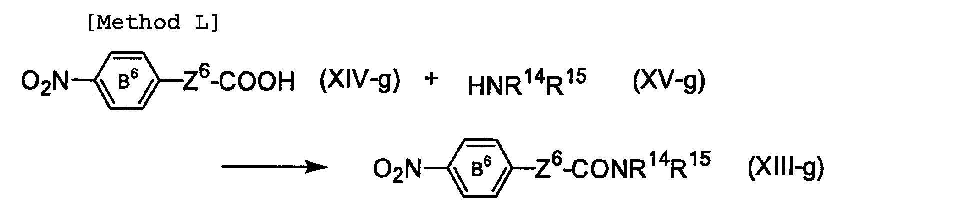 wherein the reference symbols have the meanings as defined above.

The present method is a method of obtaining Compound (XIII-g) by condensing Compound (XIV-g) with Compound (XV-g).

The present production method is carried out under the same reaction conditions as, for example, those of the method of producing Compound (VI) by condensing Compound (IV) with Compound (V) in [Step 1] of [Method C-1] described above.

Compound (XIV-g) (or a reactive derivative of Compound (XIV-g) and Compound (XV-g) that are used as the starting compounds in the present method can be produced by methods known per se, and are also commercially available. 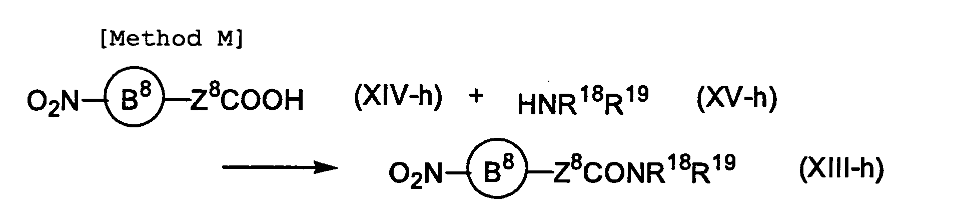 wherein the reference symbols have the meanings as defined above.

The present method is a method of obtaining Compound (XIII-h) by condensing Compound (XIV-h) with Compound (XV-h).

The present production method is carried out under the same reaction conditions as, for example, those of the method of producing Compound (VI) by condensing Compound (IV) with Compound (V) in [Step 1] of [Method C-1] described above.

Compound (XIV-h) (or a reactive derivative of Compound (XIV-h)) and Compound (XV-h) that are used as the starting compounds in the present method can be produced by methods known per se, and are also commercially available. 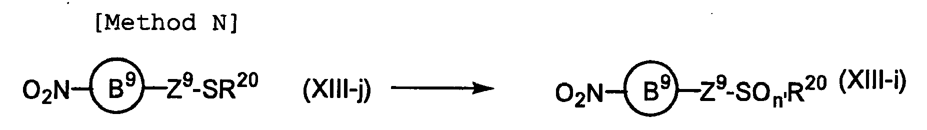 wherein the reference symbols have the meanings as defined above.

In the present method, Compound (XIII-i) is produced by oxidizing Compound (XIII-j).

The present production method is carried out under the same reaction conditions as, for example, those of the method of producing Compound (I-a-d) by oxidizing Compound (I-a-e) in [Method B'] described above.

Compound (XIII-j) that is used as the starting compounds in [Method N] is produced by, for example, Method O as described below. 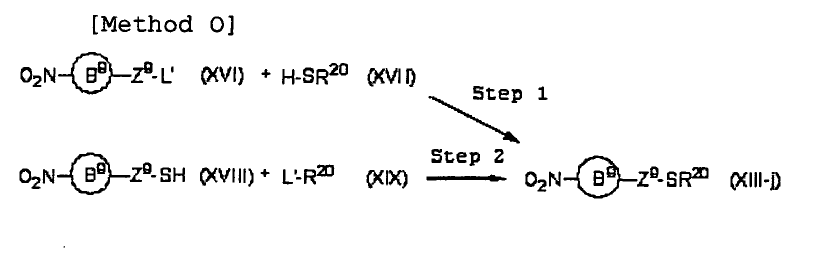 wherein L' is a hydroxyl group or a leaving group (e.g., a halogen atom (e.g., fluorine, chlorine, bromine, iodine, etc.), a C₁₋₆ alkylsulfonyloxy group which may be substituted with 1 to 3 halogen atoms (e.g., methanesulfonyloxy, ethanesulfonyloxy, trifluoromethanesulfonyloxy, etc.), an arylsulfonyloxy group which may be substituted (e.g., benzenesulfonyloxy, p-toluenesulfonyloxy, p-bromobenzenesulfonyloxy, etc.), etc.), and the other reference symbols have the meanings as defined above.

### [Step 1]

The present method is a method of obtaining Compound (XIII-j) by condensing Compound (XVI) with Compound (XVII).

### [When L' in Compound (XVI) is a leaving group]

The present production method is carried out under the same reaction conditions as, for example, those of the method of producing Compound (I-a-c) by reacting Compound (III) with a compound represented by R^{1'}-L (wherein the reference symbols have the meanings as defined above) in [Method B] described above.

### [When L' in Compound (XVI) is a hydroxyl group]

The present production method is carried out by a method known per se as the so-called Mitsunobu Reaction, for example, the method described in [Synthesis, p.1 (1981)], or a method equivalent thereto. That is, the present reaction is generally carried out in the presence of an organic phosphorus compound and an electrophile, in a solvent that has no adverse effect on the reaction.

The amount of Compound (XVII) to be used is 0.1 to 10 molar equivalents, preferably 0.3 to 3 molar equivalents, with respect to Compound (XVI).

Examples of the organic phosphorus compound include triphenylphosphine, tributylphosphine and the like. Examples of the electrophile include diethyl azodicarboxylate, diisopropyl azodicarboxylate, azodicarbonyldipiperazine, 1,1'-(azodicarbonyl)-dipiperidine and the like. The amounts of the organic phosphorus compound and the electrophile to be used are preferably about 1 to about 5 molar equivalents, respectively, with respect to Compound (XVI).

Examples of the solvent that has no adverse effect on the reaction include ethers such as diethyl ether, tetrahydrofuran and dioxane; halogenated hydrocarbons such as chloroform and dichloromethane; aromatic hydrocarbons such as benzene, toluene and xylene; amides such as N,N-dimethylformamide; sulfoxides such as dimethylsulfoxide; and the like. These solvents maybe used in mixtures at appropriate ratios.

The reaction temperature is usually about -50 to about 150°C, preferably about -10 to about 100°C. The reaction time is usually about 0.5 to about 20 hours.

Compound (XVI) and Compound (XVII) that are used as the starting compounds in the present method can be produced by methods known per se, and are also commercially available.

### [Step 2]

The present method is a method of obtaining Compound (XIII-j) by condensing Compound (XVIII) with Compound (XIX).

The production method is carried out under the same reaction conditions as, for example, those of the method producing Compound (XIII-j) by condensing Compound (XVI) with Compound (XVII) in [Step 1] of [Method 0] described above.

Compound (XVIII) and Compound (XIX) that are used as the starting compounds in the present method can be produced by methods known per se, and are also commercially available.

### [Method P]

Compound (XX-a), which is Compound (I-b) of the invention wherein R²³ is a group represented by the formula: 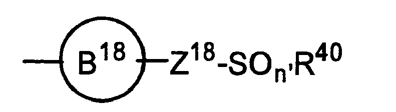 [wherein the reference symbols have the meanings as defined above],
can be synthesized by the following method or the like: 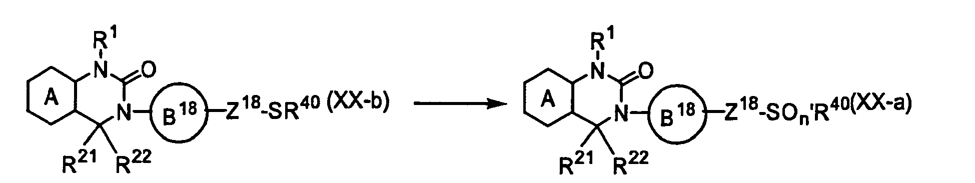 wherein the reference symbols have the meanings as defined above.

In the present method, Compound (XX-a) is produced by oxidizing Compound (XX-b).

The present production method is carried out under the same reaction conditions as, for example, those of the method of producing Compound (I-a-d) by oxidizing Compound (I-a-e) in [Method B'] described above.

### [Method Q]

Compound (XX-c), which is Compound (I-b) of the invention wherein R¹ is not a hydrogen atom, can be synthesized by, for example, the following method or the like: 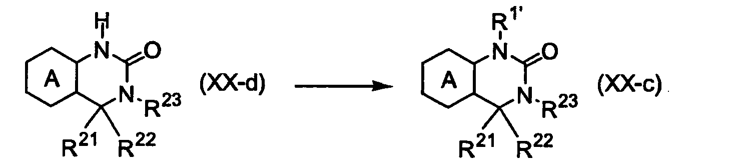 wherein the reference symbols have the meanings as defined above.

This method is a method of obtaining Compound (XX-c) by reacting Compound (XX-d) with a compound represented by the formula: R^{1'}-L, wherein the reference symbols have the meanings as defined above.

The present production method is carried out under the same reaction conditions as, for example, those of the method of producing Compound (I-a-c) by reacting Compound (III) with a compound represented by the formula: R^{1'}-L, wherein the reference symbols have the meanings as defined above, in [Method B] above.

### [Method R]

Compound (XX-e), which is Compound (I-b) of the invention wherein R¹, R²¹ and R²² are all hydrogen atoms (this compound includes Compound (XX-b) wherein R¹ is a hydrogen atom, and Compound (XX-d)), can be synthesized by, for example, the following method or the like: 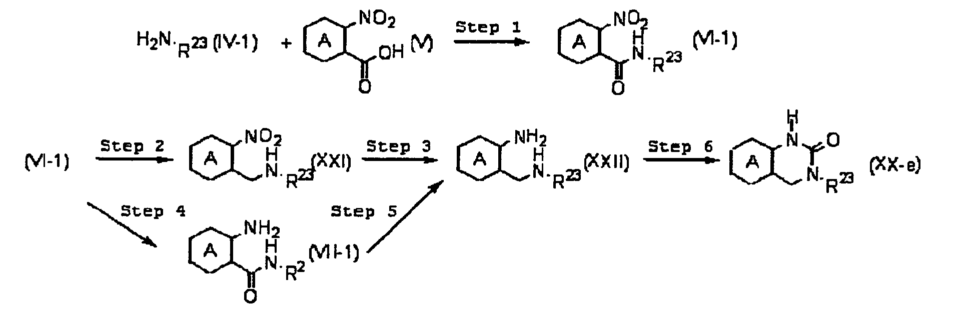 wherein the reference symbols have the meanings as defined above.

### [Step 1]

The present method is a method of obtaining Compound (VI-1) by condensing Compound (IV-1) with Compound (V) (amidation).

The present production method is carried out under the same reaction conditions as, for example, those of the method of producing Compound (VI) by condensing Compound (IV) with Compound (V) in [Step 1] of [Method C-1] above.

Compound (V) and a reactive derivative thereof that are used as the starting compounds in the present method can be produced by methods known per se, and are also commercially available.

### [Step 2]

In the present method, Compound (XXI) is produced by reducing Compound (VI-1).

In the present reduction method, a reducing agent is used in an amount of 1 equivalent to a large excess (preferably 1 to 10 equivalents) with respect to Compound (VI-1). Examples of the reducing agent include metal hydrogen complexes such as sodium borohydride, sodium cyanoborohydride, lithium aluminum hydride and diisobutylaluminum hydride, diborane and the like.

This reaction is usually carried out in a solvent. The solvent used for this purpose can be appropriately selected in accordance with the type of reducing agent, and examples thereof include alcohols (e.g., methanol, ethanol, etc.), ethers, (e.g., tetrahydrofuran, dioxane, diethyl ether, etc.), hydrocarbons (e.g., hexane, pentane, etc.), aromatic hydrocarbons (e.g., benzene, toluene, etc.), halogenated hydrocarbons (e.g., dichloromethane, chloroform, etc.) and the like.

The reaction time is 0.5 to 72 hours, preferably 1 to 24 hours. The reaction temperature may be -30 to 100°C.

### [Step 3]

In the present method, Compound (XXII) is produced by reducing Compound (XXI).

This reduction reaction is carried out under the same reaction conditions as, for example, those of the method of producing Compound (VII) by reducing Compound (VI) in [Step 2] of [Method C-1] described above.

The serial reactions involving Compound (VI-1) through Compound (XXII) via [Step 2] and [Step 3] can be also carried out within the same system.

### [Step 4]

In the present method, Compound (VII-1) is produced by reducing Compound (VI-1).

This reduction reaction is carried out under the same reaction conditions as, for example, those of the method of producing Compound (VII) by reducing Compound (VI) in [Step 2] of [Method C-1] described above.

### [Step 5]

In the present method, Compound (XXII) is produced by reducing Compound (VII-1).

This reduction reaction is carried out under the same reaction conditions as, for example, those of the method of producing Compound (XXI) by reducing Compound (VI-1) in [Step 2] of [Method R] described above.

The serial reactions involving Compound (VI-1) through Compound (XXII) via [Step 4] and [Step 5] can be also carried out within the same system.

### [Step 6]

In the present method, Compound (XX-e) is produced by reacting Compound (XXII) with a reagent such as phosgene, diphosgene, triphosgene or N,N-carbonyldiimidazole.

The present production method is carried out under the same reaction conditions as, for example, those of the method of producing Compound (III-a) from Compound (VII) in [Step 3] of [Method C-1] described above.

### [Method S]

Compound (XX-d), which is Compound (I-b) of the invention wherein R¹ is a hydrogen atom, can be synthesized by, for example, the following method or the like: 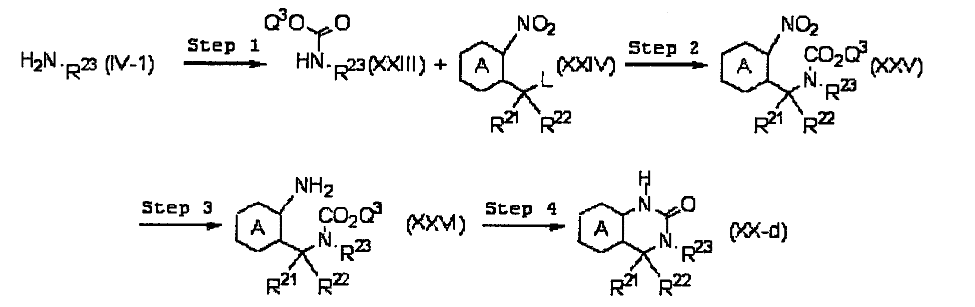 wherein Q³ is a hydrocarbon group which may be substituted, and the other reference symbols have the meanings as defined above.

The "hydrocarbon group which may be substituted" represented by Q³ may be exemplified by those mentioned as the "hydrocarbon group which may be substituted" represented by R³ described above.

### [Step 1]

In the present method, Compound (XXIII) is produced by reacting Compound (IV-1) with a halocarbonic acid ester represented by the formula: Hal-CO₂Q³ (wherein Hal is halogen (e.g., fluorine, chlorine, bromine, iodine), and Q³ has the meaning as defined above), or with a compound represented by the formula: [Q³OC(=O)]₂O (wherein Q³ has the meaning as defined above).

The present production method is carried out under the same reaction conditions as, for example, those of the method of producing Compound (VIII) by reacting Compound (VII) with a halocarboxylic acid ester represented by Hal-CO₂Q¹ (wherein the reference symbols have the meanings as defined above), or a compound represented by the formula: [Q¹OC(=O)]₂O (wherein Q¹ has the meaning as defined above), in [Step 4] of [Method C-1] described above.

The halocarbonic acid ester represented by the formula: Hal-CO₂Q³ (wherein the reference symbols have the meanings as defined above), and the compound represented by the formula: [Q³OC(=O)]₂O (wherein Q³ has the meaning as defined above) that are used as the starting compounds in the present method can be produced by methods known per se, and are also commercially available.

### [Step 2]

In the present method, Compound (XXV) is produced by reacting Compound (XXIII) with Compound (XXIV).

The present production method is carried out under the same reaction conditions as, for example, those of the method of producing Compound (I-a-c) by reacting Compound (III) with a compound represented by the formula: R¹'-L (wherein the reference symbols have the meanings as defined above) in [Method B] described above.

Compound (XXIV) that is used as the starting compound in the present method can be produced by a method known per se, and is also commercially available.

### [Step 3]

In the present method, Compound (XXVI) is produced by reducing Compound (XXV).

This reduction reaction is carried out under the same reaction conditions as, for example, those of the method of producing Compound (VII) by reducing Compound (VI) in [Step 2] of [Method C-1] described above.

### [Step 4]

In the present method, Compound (XX-d) is produced by reducing Compound (XXVI). The present method is carried out under the same conditions as, for example, those of the method of producing Compound (III-a) by reducing Compound (VIII) in [Step 5] of [Method C-1] described above.

Compound (IV-1) that is used as the starting compound in [Method R] and [Method S] is produced by, for example, the following Method T.

### [Method T]

O₂N_{R}23 (XIII-1) → H₂N _{R}23 (IV-1)

wherein the reference symbols have the meanings as defined above.

In the present method, Compound (IV-1) is produced by reducing Compound (XIII-1).

This reduction method is carried out under the same reaction conditions as, for example, those of the method of producing Compound (VII) by reducing Compound (VI) in [Step 2] of [Method C-1] described above.

Compound (XIII-1) that is used as the starting compound in [Method T] is produced by, for example, the following [Method U] through [Method EE]. 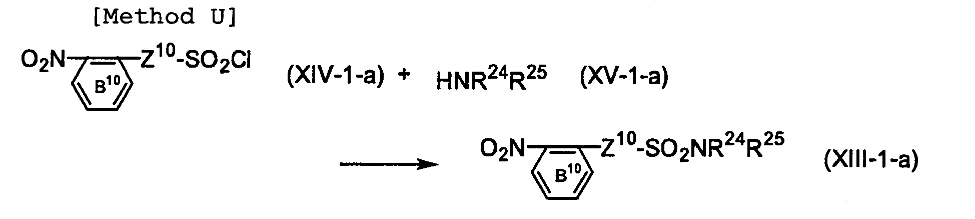 wherein the reference symbols have the meanings as defined above.

The present method is a method of obtaining Compound (XIII-1-a) by condensing Compound (XIV-1-a) with Compound (XV-1-a).

The present production method is carried out under the same reaction conditions as, for example, those of the method of producing Compound (XIII-a) by condensing Compound (XIV-a) with Compound (XV-a) in [Method F] described above.

Compound (XIV-1-a) and Compound (XV-1-a) that are used as the starting compounds in the present method can be produced by methods known per se, and are also commercially available.

### [Method V]

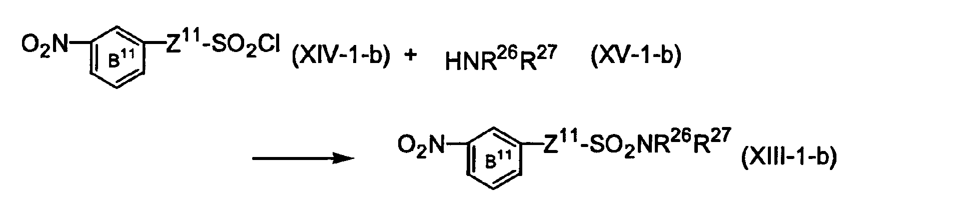

wherein the reference symbols have the meanings as defined above.

The present method is a method of obtaining Compound (XIII-1-b) by condensing Compound (XIV-1-b) with Compound (XV-1-b).

The present production method is carried out under the same reaction conditions as, for example, those of the method of producing Compound (XIII-a) by condensing Compound (XIV-a) with Compound (XV-a) in [Method F] described above.

Compound (XIV-1-b) and Compound (XV-1-b) that are used as the starting compounds in the present method can be produced by methods known per se, and are also commercially available. 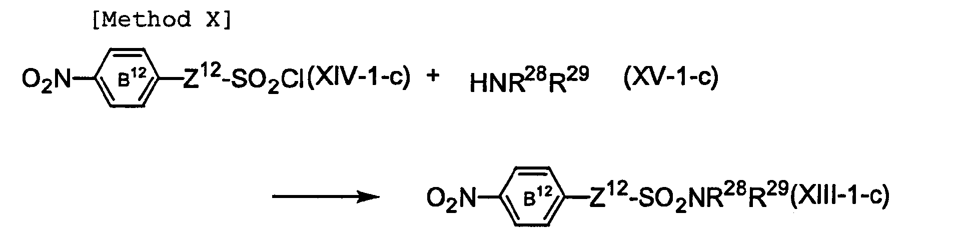 wherein the reference symbols have the meanings as defined above.

The present method is a method of obtaining Compound (XIII-1-c) by condensing Compound (XIV-1-c) with Compound (XV-1-c) .

The present production method is carried out under the same reaction conditions as, for example, those of the method of producing Compound (XIII-a) by condensing Compound (XIV-a) with Compound (XV-a) in [Method F] described above.

Compound (XIV-1-c) and Compound (XV-1-c) that are used as the starting compounds in the present method can be produced by methods known per se, and are also commercially available. 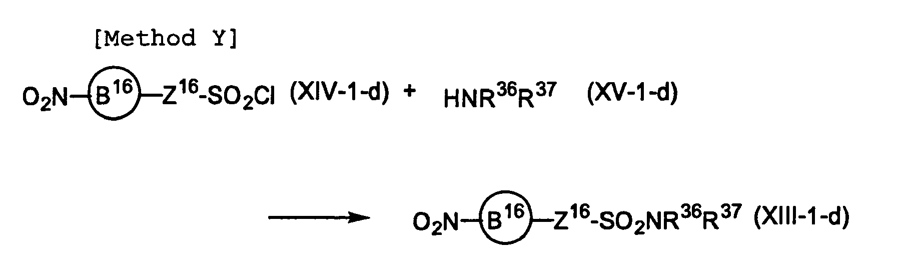 wherein the reference symbols have the meanings as defined above.

The present method is a method of obtaining Compound (XIII-1-d) by condensing Compound (XIV-1-d) with Compound (XV-1-d).

The present production method is carried out under the same reaction conditions as, for example, those of the method of producing Compound (XIII-a) by condensing Compound (XIV-a) with Compound (XV-a) in [Method F] described above.

Compound (XIV-1-d) and Compound (XV-1-d) that are used as the starting compounds in the present method can be produced by methods known per se, and are also commercially available. 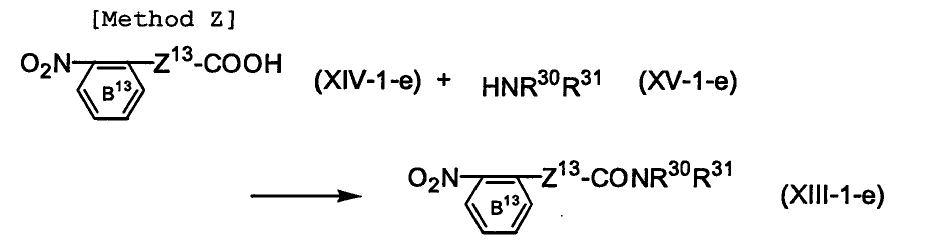 wherein the reference symbols have the meanings as defined above.

The present method is a method of obtaining Compound (XIII-1-e) by condensing Compound (XIV-1-e) with Compound (XV-1-e).

The present production method is carried out under the same reaction conditions as, for example, those of the method of producing Compound (VI) by condensing Compound (IV) with Compound (V) in [Step 1] of [Method C-1] described above.

Compound (XIV-1-e) (or a reactive derivative of Compound (XIV-1-e)) and Compound (XV-1-e) that are used as the starting compounds in the present method can be produced by methods known per se, and are also commercially available. 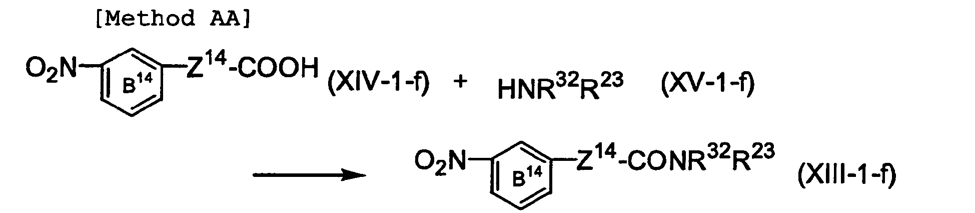 wherein the reference symbols have the meanings as defined above.

The present method is a method of obtaining Compound (XIII-1-f) by condensing Compound (XIV-1-f) with Compound (XV-1-f) .

The present production method is carried out under the same reaction conditions as, for example, those of the method of producing Compound (VI) by condensing Compound (IV) with Compound (V) in [Step 1] of [Method C-1] described above.

Compound (XIV-1-f) (or a reactive derivative of Compound (XIV-1-f)) and Compound (XV-1-f) that are used as the starting compounds in the present method can be produced by methods known per se, and are also commercially available. 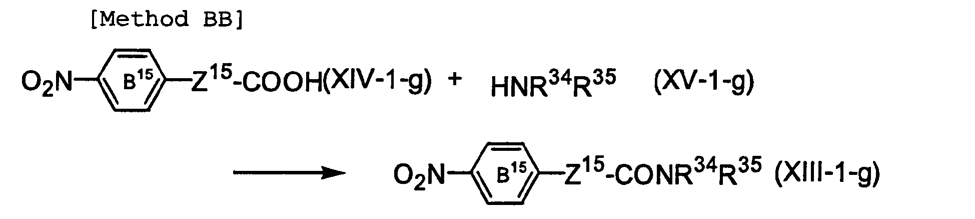 wherein the reference symbols have the meanings as defined above.

The present method is a method of obtaining Compound (XIII-1-g) by condensing Compound (XIV-1-g) with Compound (XV-1-g).

The present production method is carried out under the same reaction conditions as, for example, those of the method of producing Compound (VI) by condensing Compound (IV) with Compound (V) in [Step 1] of [Method C-1] described above.

Compound (XIV-1-g) (or a reactive derivative of Compound (XIV-1-g)) and Compound (XV-1-g) that are used as the starting compounds in the present method can be produced by methods known per se, and are also commercially available. 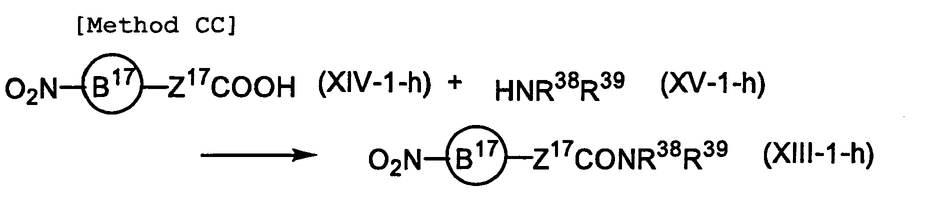 wherein the reference symbols have the meanings as defined above.

The present method is a method of obtaining Compound (XIII-1-h) by condensing Compound (XIV-1-h) with Compound (XV-1-h).

The present production method is carried out under the same reaction conditions as, for example, those of the method of producing Compound (VI) by condensing Compound (IV) with Compound (V) in [Step 1] of [Method C-1] described above.

Compound (XIV-1-h) (or a reactive derivative of Compound (XIV-1-h)) and Compound (XV-1-h) that are used as the starting compounds in the present method can be produced by methods known per se, and are also commercially available. 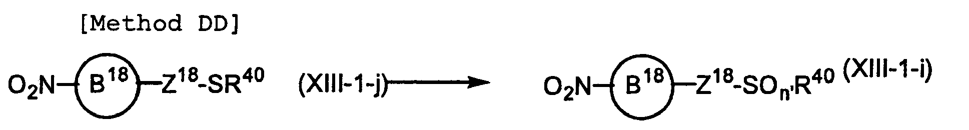 wherein the reference symbols have the meanings as defined above.

In the present method, Compound (XIII-1-i) is produced by oxidizing Compound (XIII-1-j).

The present production method is carried out under the same reaction conditions as, for example, those of the method of producing Compound (I-a-d) by oxidizing Compound (I-a-e) in [Method B'] described above.

Compound (XIII-1-j) that is used as the starting compound in [Method DD] is produced by, for example, the following Method EE. 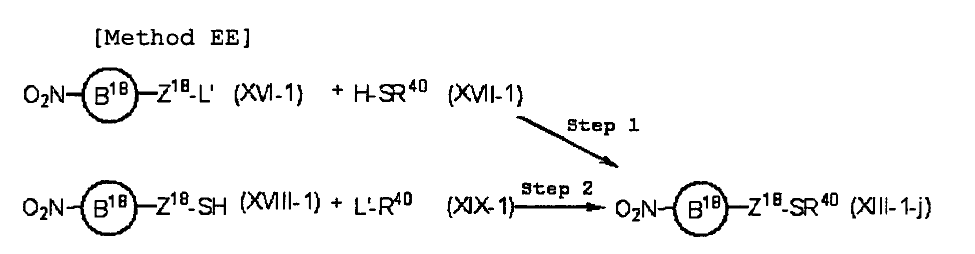 wherein the reference symbols have the meanings as defined above.

### [Step 1]

The present method is a method of obtaining Compound (XIII-1-j) by condensing Compound (XVI-1) with Compound (XVII-1).

The present production method is carried out under the same reaction conditions as, for example, those of the method of producing Compound (XIII-j) by condensing Compound (XVI) with Compound (XVII) in [Step 1] of [Method 0] described above.

Compound (XVI-1) and Compound (XVII-1) that are used as the starting compounds in the present method can be produced by methods known per se, and are also commercially available.

### [Step 2]

The present method is a method of obtaining Compound (XIII-a-j) by condensing Compound (XVIII-1) with Compound (XIX-1).

The present production method is carried out under the same reaction conditions as, for example, those of the method of producing Compound (XIII-j) by condensing Compound (XVI) with Compound (XVII) in [Step 1] of [Method 0] described above.

Compound (XVIII-1) and Compound (XIX-1) that are used as the starting compounds in the present method can be produced by methods known per se, and are also commercially available.

Furthermore, in the above-described respective reactions for synthesizing target compounds and starting compounds, when the starting compounds to be used have amino, carboxy, hydroxy or mercapto as the substituent, the starting compounds may have such substituents protected by protective groups such as those generally used in peptide chemistry or the like, and the target compounds can be obtained, if necessary, by removing the protective groups after the reaction.

The amino-protective group that can be used may be exemplified by formyl, or C₁₋₆ alkyl-carbonyl (e.g., acetyl, ethylcarbonyl, etc.), benzoyl, C₁₋₆ alkyloxy-carbonyl (e.g., methoxycarbonyl, ethoxycarbonyl, etc.), C₆₋₁₀ aryloxy-carbonyl (e.g., phenoxycarbonyl group, etc.), C₇₋₁₀ aralkyloxy-carbonyl (e.g., benzyloxycarbonyl, etc.), trityl or phthaloyl, each of which may be substituted, or the like. These protective groups may be substituted with about 1 to 4 substituents such as halogen (e.g., fluorine, chlorine, bromine, iodine, etc.), C₁₋₆ alkyl-carbonyl (e.g., acetyl, ethylcarbonyl, butylcarbonyl, etc.), nitro or the like.

The carboxyl-protective group that can be used may be exemplified by C₁₋₆ alkyl (e.g., methyl, ethyl, propyl, isopropyl, butyl, tert-butyl, etc.), phenyl, trityl, silyl, or the like. These protective groups maybe substituted with about 1 to 4 substituents such as halogen (e.g., fluorine, chlorine, bromine, iodine), C₁₋₆ alkyl-carbonyl (e.g., acetyl, ethylcarbonyl, butylcarbonyl, etc.), formyl or nitro, each of which may be substituted, or the like.

The hydroxyl-protective group that can be used may be exemplified by formyl, C₁₋₆ alkyl (e.g., methyl, ethyl, propyl, isopropyl, butyl, tert-butyl, etc.), phenyl, C₇₋₁₀ aralkyl (e.g., benzyl, etc.), C₁₋₆ alkyl-carbonyl (e.g., acetyl, ethylcarbonyl, etc.), C₆₋₁₀ aryloxy-carbonyl (e.g., phenoxycarbonyl, etc.), C₇₋₁₀ aralkyloxy-carbonyl (e.g., benzyloxycarbonyl, etc.), tetrahydropyranyl, tetrahydrofuranyl or silyl, each of which may be substituted, or the like. These protective groups may be substituted with about 1 to 4 substituents such as halogen (e.g., fluorine, chlorine, bromine, iodine, etc.), C₁₋₆ alkyl (e.g., methyl, ethyl, propyl, isopropyl, butyl, tert-butyl, etc.), phenyl, C₇₋₁₀ aralkyl (e.g., benzyl, etc.), nitro, or the like. The mercapto-protective group that can be used may be exemplified by those mentioned as the protective groups that are used as the hydroxyl-protective groups.

Removal of the protective group is carried out using a method known per se or a method equivalent thereto, and for example, methods of treating with an acid, a base, a ultraviolet light, hydrazine, phenylhydrazine, sodium N-methyldithiocarbamate, tetrabutylammonium fluoride, palladium acetate or the like, or of using a reduction reaction.

Precursors of Compound (I) respectively refer to a synthetic intermediate of Compound (I). In particular, the precursor is preferably a compound that is converted to Compound (I) by carrying out intramolecular ring closure. Preferred examples of the precursor of Compound (I-a) include compounds (VIII), (X) and (XII) mentioned above, and a preferred example of the precursor of Compound (I-b) is Compound (XXVI) mentioned above: 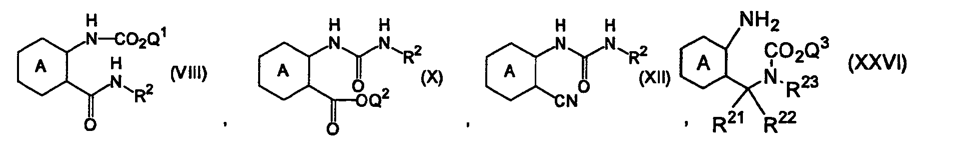 wherein each reference symbol has the meaning as defined above. These compounds (VIII), (X), (XII) and (XXVI) are converted to Compound (I) in the mixtures during testing (evaluation of the compound activity) or in vivo, and exhibit the same effect as Compound (I).

Compound (I) can be isolated and purified by a separation technique known per se, such as recrystallization, distillation or chromatography.

When Compound (I) is obtained in its free from, the compound can be converted to a desired salt by a method known per se or a method equivalent thereto. In contrast, when Compound (I) is obtained in a salt form, the salt can be converted to the free form compound or to another desired salt by a method known per se or a method equivalent thereto.

Compound (I) may be a hydrate or non-hydrate. Examples of the hydrate include a 1-hydrate, a 1.5-hydrate, a 2-hydrate and the like.

When Compound (I) is obtained as a mixture of optically active isomers, the compound can be separated into the desired R-isomer or S-isomer by an optical resolution method known per se.

Compound (I) may be used in its prodrug form, and such prodrug refers to a compound which is converted to Compound (I) by an in vivo reaction of enzyme, gastric acid or the like under the physiological conditions, that is, a compound which changes to Compound (I) upon enzymatic oxidation, reduction, hydrolysis or the like, or a compound which changes to Compound (I) upon hydrolysis by gastric acid or the like. The prodrug of Compound (I) may be exemplified by a compound resulting from acylation, alkylation or phosphorylation of the amino group of Compound (I) [e.g., a compound in which the amino group of Compound (I) is in the form of eicosanoyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl, tert-butyl or the like]; a compound resulting from acetylation, alkylation, phosphorylation or boration of the hydroxyl group of Compound (I) [e.g., a compound in which the hydroxyl group of Compound (I) is in the form of acetyl, palmitoyl, propanoyl, pivaloyl, succinyl, fumaryl, alanyl, dimethylaminomethylcarbonyl or the like); or the like. These compounds can be produced from Compound (I) by methods known per se.

The prodrug of Compound (I) may also be a compound which changes to the compound of the invention under the physiological conditions, as described in "Development of Pharmaceutical Products", Vol.7, Design of Molecules, Hirokawa Publisher, pp.163-198 (1990).

Compound (I) may be also labeled with an isotope (e.g., ³H, ¹⁴C, ³⁵S) or the like.

Furthermore, Compound (II) represented by the formula: 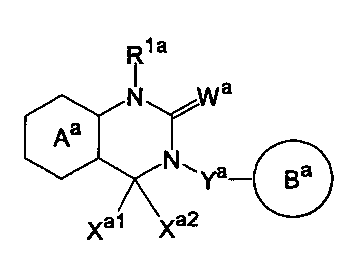 wherein R^{1a} is a hydrocarbon group which may be substituted or a hydrogen atom,
ring A^{a} is a 6-membered aromatic ring which may be further substituted,
ring B^{a} is a homocyclic or heterocyclic ring which may be further substituted,
W^{a} is an oxygen atom or a sulfur atom,
X^{a1} and X^{a2}, which may be identical or different, are each a hydrogen atom, a hydrocarbon group which may be substituted, or a heterocyclic group which may be substituted, or X^{a1} and X^{a2} together may form an oxygen atom, a sulfur atom or NR^{3a} (wherein R^{3a} is a hydrocarbon group which may be substituted or a hydrogen atom), and
Y^{a} is C₁₋₆ alkylene which may be substituted or a bond, or a salt thereof, which includes Compound (I) and known compounds, has an excellent GnRH antagonizing activity.

In Compound (II), R^{1a} is a hydrocarbon group which may be substituted or a hydrogen atom. The "hydrocarbon group which may be substituted" represented by R^{1a} may be exemplified by those mentioned as the "hydrocarbon group which may be substituted" represented by R¹ mentioned above.

R^{1a} is preferably (1) a hydrogen atom, or (2) C₁₋₆ alkyl which may be substituted with 1 to 3 substituents selected from (a) C₆₋₁₄ aryl which may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen, (b) cyano, (c) hydroxy, (d) carboxy, (e) nitro, (f) C₁₋₆ alkyl which may be substituted with di-C₁₋₆ alkylamino, (g) C₁₋₆ alkoxy, (h) C₁₋₆ alkylcarbonyloxy, (i) C₁₋₆ alkylthio, (j) C₁₋₆ alkylsulfinyl, (k) C₁₋₆ alkylsulfonyl, (1) halogen, (m) amino, (n) mono-C₁₋₆ alkylamino, (o) di-C₁₋₆ alkylamino, (p) a 5-membered ring containing 1 to 4 heteroatoms selected from oxygen, sulfur and nitrogen atoms and the like in addition to carbon atoms (wherein this 5-membered ring may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (q) a 6-membered ring containing 1 to 4 heteroatoms selected from oxygen, sulfur and nitrogen atoms and the like in addition to carbon atoms (wherein this 6-membered ring may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (r) a bicyclic or tricyclic fused ring containing 1 to 4 heteroatoms selected from oxygen, sulfur and nitrogen atoms and the like in addition to carbon atoms (wherein this bicyclic or tricyclic ring may be substituted with 1 to 3 substituents selected from (i) hydroxyl, (ii) amino, (iii) mono-C₁₋₆ alkylamino, (iv) di-C₁₋₆ alkylamino, (v) C₁₋₆ alkoxy and (vi) halogen), (s) C₁₋₆ alkoxycarbonyl, (t) carbamoyl, (u) N-mono-C₁₋₆ alkylcarbamoyl, (v) N,N-di-C₁₋₆ alkylcarbamoyl, and (w) C₁₋₂ alkylenedioxy. Among these, R^{1a} is preferably a hydrogen atom or C₁₋₆ alkyl, particularly preferably a hydrogen atom.

The "6-membered aromatic ring which may be further substituted" represented by ring A^{a} may be exemplified by those mentioned as the "6-membered aromatic ring which may be further substituted" represented by ring A mentioned above.

The "homocyclic ring" of the "homocyclic or heterocyclic ring which may be further substituted" represented by ring B^{a} may be exemplified by a C₃₋₈ cycloalkyl ring (e.g., a cyclopropane ring, a cyclobutane ring, a cyclopentane ring, a cyclohexane ring, a cycloheptane ring, a cyclooctane ring, etc.), a C₆₋₁₄ aryl ring (e.g., a benzene ring, a naphthalene ring, a phenanthrene ring, an anthracene ring), a fused ring formed by condensation of the C₃₋₈ cycloalkyl ring and the C₆₋₁₄ aryl ring, and the like.

The "heterocyclic ring" of the "homocyclic or heterocyclic ring which may be further substituted" represented by ring B^{a} may be exemplified by a 5-membered aromatic heterocycle (e.g., a thiophene ring, a furan ring, a pyrrole ring, an oxazole ring, a thiazole ring, a pyrazole ring, an imidazole ring, an isoxazole ring, an isothiazole ring, a 1,2,4-oxadiazole ring, a 1,3,4-oxadiazole ring, a 1,2,4-thiadiazole ring, a 1,3,4-thiadiazole ring, a 1,2,3-triazole ring, a 1,2,4-triazole ring, a 1H-tetrazole ring, a 2H-tetrazole ring, etc.), a 6-membered aromatic heterocycle (e.g., a pyridine ring, a pyrimidine ring, a triazinine ring, a pyridazine ring, a pyrazine ring, etc.), a bicyclic or tricyclic fused aromatic heterocycle (e.g., a benzofuran ring, a benzothiazole ring, a benzoxazole ring, a tetrazolo[1,5-b]pyridazine ring, a triazolo[4,5-b]pyridazine ring, a benzoimidazole ring, a quinoline ring, an isoquinoline ring, a cinnoline ring, a phthalazine ring, a quinazoline ring, a quinoxaline ring, an indolizine ring, a quinolizine ring, a 1,8-naphthyridine ring, a purine ring, a pteridine ring, a dibenzofuran ring, a carbazole ring, an acridine ring, a phenanthridine ring, a chromane ring, a benzoxazine ring, a phenazine ring, a phenothiazine ring, a phenoxazine ring, etc.), an indole ring, rings formed by partial or total saturation of double bonds of these aromatic heterocycles, or the like.

The "substituent" of the "homocyclic or heterocyclic ring which may be further substituted" represented by ring B^{a} may be exemplified by those mentioned as the "substituent which may be further carried by ring A," or
(i) a group represented by the formula: -Z^{a1}-SO₂NR^{4a}R^{5a} (wherein Z^{a1} is C₁₋₆ alkylene which may be substituted or a bond and R^{4a} and R^{5a}, which may be identical or different, are each a hydrogen atom, a hydrocarbon group which may be substituted or a heterocyclic group which may be substituted, or R^{4a} and R^{5a} may form a ring together with the adjacent nitrogen atom),
(ii) a group represented by the formula: -Z^{a2}-CONR^{6a}R^{7a} (wherein Z^{a2} is C₁₋₆ alkylene which may be substituted or a bond, and R^{6a} and R^{7a}, which may be identical or different, are each a hydrogen atom, a hydrocarbon group which may be substituted or a heterocyclic group which may be substituted, or R^{6a} and R^{7a} may form a ring together with the adjacent nitrogen atom),
(iii) a group represented by the formula: -Z^{a3}-SOₜR^{8a} (wherein Z^{a3} is C₁₋₆ alkylene which may be substituted or a bond, R^{8a} is a hydrocarbon group which may be substituted or a heterocyclic group which may be substituted, and t is an integer from 0 to 2), or the like, while the number of substituents is from 1 to 3.

These substituents may be substituted with the above-defined substituents at their substitutable positions, and the number of substituents is not particularly limited.

The "C₁₋₆ alkylene which may be substituted" represented by Z^{a1}, Z^{a2} and Z^{a3} may be exemplified by those mentioned as the "C₁₋₆ alkylene which may be substituted" represented by Z¹ through Z⁹ and Z¹⁰ through Z¹⁸ mentioned above.

The "hydrocarbon group which may be substituted" represented by R^{4a}, R^{5a}, R^{6a}, R^{7a} and R^{8a} may be exemplified by those mentioned as the "hydrocarbon group which may be substituted" represented by R³ mentioned above.

The "heterocyclic group which may be substituted" represented by R^{4a}, R^{5a}, R^{6a}, R^{7a} and R^{8a} may be exemplified by those mentioned as the "heterocyclic group which may be substituted" represented by R⁴, R⁵, R⁶, R⁷, R⁸, R⁹, R¹⁰, R¹², R¹³, R¹⁴ , R¹⁵, R¹⁶, R¹⁷, R¹⁸, R¹⁹ and R²⁰ and R²⁴ through R⁴⁰.

When R^{4a} and R^{5a} and R^{6a} and R^{7a} respectively form a ring together with the adjacent nitrogen atom, the group represented by -NR ^{4a}R^{5a} or -NR^{6a}R^{7a} is a cyclic group selected from 1-pyrrolidinyl, 1-pyrrolinyl, 1-pyrrolyl, azepan-1-yl, 1-imidazolidinyl, 1-imidazolinyl, 1-imidazolyl, 2-pyrazolidinyl, 2-pyrazolinyl, 1-pyrazolyl, 1-indolinyl, 1-indolyl, 2-isoindolinyl, 2-isoindolyl, 2,3-dihydro-1H-indol-1-yl, 1-piperidyl, 3,4-dihydro-1,5-naphthyridin-1(2H)-yl, 1,2,3,4-tetrahydroquinolin-1-yl, 3,4-dihydroquinolin-1(2H)-yl, 3,4-dihydroisoquinolin-2(1H)-yl, 1-piperazinyl, octahydroquinolin-1(2H)-yl, octahydroisoquinolin-2(1H)-yl, 3,4-dihydroquinoxalin-1(2H)-yl, 1H-azepin-1-yl, 2,3,4,5,6,7-hexahydro-1H-azepin-1-yl, 2,3,4,5-tetrahydro-1H-1-benzazepin-1-yl, 1,3,4,5-tetrahydro-2H-2-benzazepin-2-yl, 3,4,5,6-tetrahydro-1-benzazocin-1(2H)-yl, 2,3-dihydro-4,1-benzothiazepin-1(5H)-yl, 3,4-dihydro-1,5-benzothiazepin-5(2H)-yl, 2,3-dihydro-4,1-benzothiazepin-1(5H)-yl, 2,3-dihydro-4,1-benzoxazepin-1(5H)-yl, 1,2,4,5-tetrahydro-3H-3-benzazepin-3-yl, 2,3,4,5-tetrahydro-1H-1,5-benzodiazepin-1-yl, 2,3,4,5-tetrahydro-1H-1,4-benzodiazepin-1-yl, 2,3-dihydro-4H-1,4-benzoxazin-4-yl, 3,4-dihydro-2H-1,4-benzoxazin-1-yl, 3,4-dihydro-1,5-benzoxazepin-5(2H)-yl, 5,6,7,8-tetrahydro-4H-thieno[3,2-b]azepin-4-yl, 4-morpholinyl, 4-thiomorpholinyl, 2,3-dihydro-4H-pyrido[3,2-b][1,4]oxazin-4-yl, 2,3-dihydro-4H-1,4-benzothiazin-4-yl, 5,6,7,8-tetrahydropyrazolo[4,3-b]azepin-4(1H)-yl and the like. These cyclic groups may be substituted, and the substituent for the cyclic groups may be exemplified by those mentioned as the substituents which may be further carried by ring A described above, while the number of substituents is from 1 to 3.

Preferably, the group may be exemplified by
(a) C₆₋₁₄ aryl (e.g. , phenyl, 1-naphthyl , 2-naphthyl, anthryl, phenanthryl, etc.) which may be substituted with 1 to 3 substituents selected from (i) hydroxy, (ii) amino, (iii) mono-C₁₋₆ alkylamino (e.g., methylamino, ethylamino, propylamino, isopropylamino, butylamino, isobutylamino, sec-butylamino, tert-butylamino, pentylamino, hexylamino, etc.), (iv) di-C₁₋₆ alkylamino (e.g., dimethylamino, diethylamino, dipropylamino, diisopropylamino, butylamino, dibutylamino, etc.), (v) C₁₋₆ alkoxy (e.g., methoxy, ethoxy, propoxy, isopropoxy, butoxy, isobutoxy, sec-butoxy, tert-butoxy, pentyloxy, hexyloxy, etc.), and (vi) halogen (e.g., fluorine, chlorine, bromine, iodine),
(b) cyano,
(c) hydroxy,
(d) carboxyl,
(e) nitro,
(f) C₁₋₆ alkyl (e.g., methyl, ethyl, propyl, isopropyl, butyl, isobutyl, sec-butyl, tert-butyl, pentyl, hexyl, etc.) which may be substituted with di-C₁₋₆ alkylamino (e.g., dimethylamino, diethylamino, dipropylamino, diisopropylamino, butylamino, dibutylamino, etc.),
(g) C₁₋₆ alkoxy (e.g., methoxy, ethoxy, propoxy, isopropoxy, butoxy, isobutoxy, sec-butoxy, tert-butoxy, pentyloxy, hexyloxy, etc.),
(h) C₁₋₆ alkyl-carbonyloxy (e.g., methylcarbonyloxy, ethylcarbonyloxy, propylcarbonyloxy, isopropylcarbonyloxy, butylcarbonyloxy, isobutylcarbonyloxy, sec-butylcarbonyloxy, tert-butylcarbonyloxy, pentylcarbonyloxy, hexylcarbonyloxy, etc.),
(i) C₁₋₆ alkylthio (e.g., methylthio, ethylthio, propylthio, isopropylthio, butylthio, isobutylthio, sec-butylthio, tert-butylthio, pentylthio, hexylthio, etc.),
(j) C₁₋₆ alkylsulfinyl (e.g., methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, hexylsulfinyl, etc.),
(k) C₁₋₆ alkylsulfonyl (e.g., methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, hexylsulfonyl, etc.),
(1) halogen (e.g., fluorine, chlorine, bromine, iodine),
(m) amino,
(n) mono-C₁₋₆ alkylamino (e.g., methylamino, ethylamino, propylamino, isopropylamino, butylamino, isobutylamino, sec-butylamino, tert-butylamino, pentylamino, hexylamino, etc.),
(o) di-C₁₋₆ alkylamino (e.g., dimethylamino, diethylamino, dipropylamino, diisopropylamino, butylamino, dibutylamino, etc.),
(p) a 5-membered ring containing 1 to 4 heteroatoms selected from oxygen, sulfur and nitrogen atoms and the like in addition to carbon atoms (e.g., 2-thienyl, 3-thienyl, 2-furyl, 3-furyl, 2-pyrrolyl, 3-pyrrolyl, 2-oxazolyl, 4-oxazolyl, 5-oxazolyl, 2-thiazolyl, 4-thiazolyl, 5-thiazolyl, 3-pyrazolyl, 4-pyrazolyl, 5-pyrazolyl, 2-imidazolyl, 4-imidazolyl, 5-imidazolyl, 3-isoxazolyl, 4-isoxazolyl, 5-isoxazolyl, 3-isothiazolyl, 4-isothiazolyl, 5-isothiazolyl, 3-(1,2,4-oxadiazolyl), 5-(1,2,4-oxadiazolyl), 1,3,4-oxadiazolyl, 3-(1,2,4-thiadiazolyl), 5-(1,2,4-thiadiazolyl), 1,3,4-thiadiazolyl, 4-(1,2,3-thiadiazolyl), 5-(1,2,3-thiadiazolyl), 1,2,5-thiadiazolyl, 1,2,3-triazolyl, 1,2,4-triazolyl, 1H-tetrazolyl, 2H-tetrazolyl, oxoimidazinyl, dioxotriazinyl, pyrrolidinyl, etc.) (wherein this 5-membered ring may be substituted with 1 to 3 substituents selected from (i) hydroxyl, (ii) amino, (iii) mono-C₁₋₆ alkylamino, (iv) di-C₁₋₆ alkylamino, (v) C₁₋₆ alkoxy and (vi) halogen),
(q) a 6-membered ring containing 1 to 4 heteroatoms selected from oxygen, sulfur and nitrogen atoms and the like in addition to carbon atoms (e.g., 2-pyridyl, 3-pyridyl, 4-pyridyl, N-oxido-2-pyridyl, N-oxido-3-pyridyl, N-oxido-4-pyridyl, 2-pyrimidinyl, 4-pyrimidinyl, 5-pyrimidinyl, N-oxido-2-pyrimidinyl, N-oxido-4-pyrimidinyl, N-oxido-5-pyrimidinyl, 2-thiomorpholinyl, 3-thiomorpholinyl, 2-morpholinyl, 3-morpholinyl, piperidinyl, pyranyl, thiopyranyl, 1,4-oxazinyl, 1,4-thiazinyl, 1,3-thiazinyl, 2-piperazinyl, 3-piperazinyl, triazinyl, oxotriazinyl, 3-pyridazinyl, 4-pyridazinyl, pyrazinyl, N-oxido-3-pyridazinyl, N-oxido-4-pyridazinyl, etc.) (wherein this 6-membered ring may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen),
(r) a bicyclic or tricyclic fused ring containing 1 to 4 heteroatoms selected from oxygen, sulfur and nitrogen atoms and the like in addition to carbon atoms (e.g., benzofuryl, benzothiazolyl, benzoxazolyl, tetrazolo[1,5-b]pyridazinyl, triazolo[4,5-b]pyridazinyl, benzoimidazolyl, quinolyl, isoquinolyl, cinnolinyl, phthalazinyl, quinazolinyl, quinoxalinyl, indolizinyl, quinolizinyl, 1,8-naphthyridinyl, purinyl, pteridinyl, dibenzofuranyl, carbazolyl, acridinyl, phenanthridinyl, chromanyl, benzoxazinyl, phenazinyl, phenothiazinyl, phenoxazinyl, etc.) (wherein this bicyclic or tricyclic ring may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen),
(s) C₁₋₆ alkoxycarbonyl (e.g., methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl, etc.),
(t) C₁₋₂ alkylenedioxy (e.g., methylenedioxy, ethylenedioxy)
(u) a group represented by the formula: -Z^{a1}-SO₂NR^{4a}R^{5a} (wherein Z^{a1} is C₁₋₆ alkylene which may be substituted or a bond, R^{4a} and R^{5a}, which may be identical or different, are each a hydrogen atom, a hydrocarbon group which may be substituted or a heterocyclic group which may be substituted, or R^{4a} and R^{5a} may form a ring together with the adjacent nitrogen atom),
(v) a group represented by the formula: -Z^{a2} -CONR^{6a}R^{7a} (wherein Z^{a2} is C₁₋₆ alkylene which may be substituted or a bond, R^{6a} and R^{7a}, which may be identical or different, are each a hydrogen atom, a hydrocarbon group which may be substituted or a heterocyclic group which may be substituted, or R^{6a} and R^{7a} may form a ring together with the adjacent nitrogen atom), or the like, and the number of substituents is from 1 to 3.
   X^{a1} and X^{a2}, which may be identical or different, are each a hydrogen atom, a "hydrocarbon group which may be substituted" or a "heterocyclic group which may be substituted", and X^{a1} and X^{a2} together may form an oxygen atom, a sulfur atom or NR^{3a} (wherein R^{3a} is a hydrogen atom or a "hydrocarbon group which may be substituted".

The "hydrocarbon group which may be substituted" represented by X^{a1} and X^{a2} may be exemplified by those mentioned as the "hydrocarbon group which may be substituted" represented by R²¹ and R²².

The "heterocyclic group which may be substituted" represented by X^{a1} and X^{a2} may be exemplified by the heterocyclic rings listed for the heterocyclic rings bound to the oxy in the "heterocyclic oxy" mentioned as the "substituent which may be further carried by ring A" described above.

The "substituent" of the "heterocyclic group which may be substituted" may be exemplified by those mentioned as the "substituent" of the "6-membered aromatic ring which may be further substituted" represented by ring A.

The "hydrocarbon group which may be substituted" represented by R^{3a} may be exemplified by those mentioned as the "hydrocarbon group which may be substituted" represented by R³ described above.

The C₁₋₆ alkylene of the "C₁₋₆ alkylene which may be substituted" represented by Y^{a} may be exemplified by -CH₂-, - CH₂CH₂-, -CH₂CH₂CH₂-, -CH₂CH₂CH₂CH₂-, -CH₂CH₂CH₂CH₂CH₂- or - CH₂CH₂CH₂CH₂CH₂CH₂-. The substituent of the "C₁₋₆ alkylene which may be substituted" may be exemplified by those mentioned as the substituents which may be further carried by ring A described above, and the number of substituents is from 1 to 3.

The salt of Compound (II) may be exemplified by those mentioned as the salts of Compound (I).

The prodrug of Compound (II) may be exemplified by those mentioned as the prodrugs of Compound (I).

The precursor of Compound (II) may be exemplified by those mentioned as the precursors of Compound (I). That is, compounds represented by the formulas: 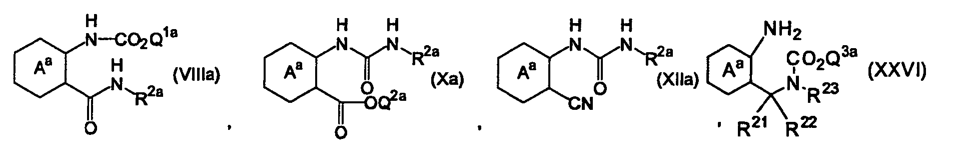 wherein Q^{1a}, Q^{2a} and Q^{3a} are each a hydrocarbon group which may be substituted, and the other reference symbols have the meanings as defined above,
may be mentioned as preferred examples.

The "hydrocarbon group which may be substituted" represented by Q^{1a}, Q^{2a} and Q^{3a} may be exemplified by those mentioned as the "hydrocarbon group which may be substituted" represented by R³ described above.

Compound (II) of the invention (includes Compound (I). Hereinafter, may be briefly referred to as the "compound of the invention") has excellent GnRH antagonizing activity and has low toxicity. Moreover, the compound excels in oral absorption or sustained action, and is also excellent in the aspects of stability or pharmacokinetics. The compound of the invention can be safely used in prevention and/or treatment of male hormone- or female hormone-dependent diseases (sex hormone-dependent diseases), and in prevention and/or treatment of diseases attributable to excessive secretion of those hormones, since the compound can inhibit secretion of gonadotropin by means of its antagonistic activity against a GnRH receptor in a mammal (e.g., a human, a monkey, a cow, a horse, a dog, a cat, a rabbit, a rat, a mouse, etc.), thus regulating the sex hormone concentration in the blood.

For example, the compound of the invention is useful in prevention and/or treatment of sex hormone-dependent diseases such as sex hormone-dependent cancers (e.g., prostate cancer, uterine cancer, breast cancer, pituitary tumor, etc.), bone metastasis of sex hormone-dependent cancers, prostatomegaly, hysteromyoma, endometriosis, metrofibroma, precocious puberty, amenorrhea, premenstrual syndrome, dysmenorrhea, polycystic ovary syndrome, acne, baldness, and Alzheimer's disease (Alzheimer's disease, Alzheimer type senile dementia and mixed type thereof). The compound of the invention is also useful in regulation of male and female reproduction (e.g., agent for birth control, menstrual cycle regulator, etc.). The compound of the invention can be also used as a contraceptive for both male and female, and further as an ovulation inducing agent for female. The compound of the invention can be used in prevention and/or treatment of infertility by using its rebound effect after cessation of medication. The compound can be also used as a prophylactic and/or therapeutic agent for benign or malignant tumors which are sex hormone-independent and LH-RH-sensitive. The compound of the invention can be also used as a prophylactic and/or therapeutic agent for irritable bowel syndrome and as a prophylactic agent for post-operative recurrence of sex hormone-dependent cancers (a prophylactic agent for post-operative recurrence of prostate cancer, a prophylactic agent for post-operative recurrence of breast cancer or ovarian cancer before and after menopause, etc.; particularly preferably a prophylactic agent for post-operative recurrence of breast cancer or ovarian cancer before menopause). Moreover, the compound of the invention prevents disorder in the ovary or testicle due to the treatment using chemotherapeutic agents, and thus can be used for the preservation of the reproductive function after the treatment.

In addition, the compound of the invention is also useful for the regulation of animal's estrus, improvement in the table meat quality, regulation of animal growth and the like. The compound of the invention is also useful as a promoting agent for fish spawning.

The compound of the invention can be used to suppress a transient increase in the blood concentration of testosterone (flare phenomenon) that is observed upon administration of a GnRH superagonist such as leuprorelin acetate. The compound of the invention can be used in combination with a GnRH superagonist such as leuprorelin acetate, gonadorelin, buserelin, triptorelin, goserelin, nafarelin, histrelin, deslorelin, meterelin or lecirelin (preferably leuprorelin acetate).

The compound of the invention can be also effectively used in combination with at least one selected from steroidal or nonsteroidal antiandrogenic agents or antiestrogenic agents, chemotherapeutic agents, peptidic GnRH antagonists, α-reductase inhibitors, α-receptor inhibitors, aromatase inhibitors, 17β-hydroxysteroid dehydrogenase inhibitors, adrenal androgen production inhibitors, phosphorylase inhibitors, hormonotherapeutic agents, cell growth factors or drugs inhibiting the action of receptors thereof, and the like.

Examples of the "chemotherapeutic agent" include ifosfamide, Adriamycin, peplomycin, cisplatin, cyclophosphamide, 5-FU, UFT, methotrexate, mitomycin C, mitoxantrone and the like.

Examples of the "peptidic GnRH antagonist" include parenterally administered peptidic GnRH antagonists such as cetrorelix, ganirelix and abarelix.

Examples of the "adrenal androgen production inhibitor" may be exemplified by lyase (C_{17.20}-lyase) inhibitors, and the like.

Examples of the "phosphorylase inhibitor" include tyrosine phosphorylases and the like.

Examples of the "hormonotherapeutic agent" include antiestrogenic agents, luteinizing hormones (e.g., MPA, etc.), androgenic agents, estrogenic agents, antiandrogenic agents and the like.

The "cell growth factor" may be any substance that facilitates cell growth, and may be exemplified by a peptide having a molecular weight of usually 20,000 or less, which is a factor expressing its action at low concentrations by receptor binding. Specific examples thereof include (1) EGF (epidermal growth factor) or substances having an activity substantially identical therewith (e.g., EGF, heregulin (HER2 ligand), etc.), (2) insulin or substances having an activity substantially identical therewith (e.g., insulin, IGF (insulin-like growth factor)-1, IGF-2, etc.), (3) FGF (fibroblast growth factor) or substances having an activity substantially identical therewith (e.g., aFGF, bFGF, KGF (keratindcyte growth factor), HGF (hepatocyte growth factor), FGF-10, etc.), (4) other cell growth factors (e.g., CSF (colony stimulating factor), EPO (erythropoietin), IL-2 (interleukin-2), NGF (nerve growth factor), PDGF (platelet-derived growth factor), TGFβ (transforming growth factor β), etc.), and the like.

The "receptor for cell growth factor" may be any receptor having binding ability with the above-described cell growth factors, and specific examples thereof include EGF receptors, heregulin receptors (HER2), insulin receptor-1, insulin receptor-2, IGF receptors, FGF receptor-1, FGF receptor-2, and the like.

The drug inhibiting the action of the above-described cell growth factors may be exemplified by herceptin (HER2 receptor antibody) or the like.

The drug inhibiting the action of the cell growth factor or its receptor may be exemplified by herbimycin, PD153035 (Science, 265 (5175), p.1093 (1994)), or the like.

As the drug inhibiting the cell growth factor or its receptor, mention may be also made of HER2 inhibitors. The HER2 inhibitor may be any substance inhibiting the HER2 activity (e.g., phosphorylating activity), including antibodies, low molecular weight compounds (synthetic compounds, natural compounds), antisenses, HER2 ligands, heregulin, or products resulting from partial modification or alteration of their structures. Further, the drug may be also a substance inhibiting the HER2 activity by inhibiting a HER2 receptor (e.g., HER2 receptor antibody). Examples of the low molecular weight compound having HER2 inhibiting activity include the compounds described in WO 98/03505, specifically 1-[3-[4-[2-((E)-2-phenylethenyl)-4-oxazolylmethoxy]phenyl]propyl]-1,2,4-triazole, and the like.

With respect to prostatomegaly, the compound of the invention can be used in combination with a drug such as GnRH superagonist, antiandrogenic agent, antiestrogenic agent, peptidic GnRH antagonist, α-reductase inhibitor, α-receptor inhibitor, aromatase inhibitor, 17β-hydroxysteroid dehydrogenase inhibitor, adrenal androgen production inhibitor, or phosphorylase inhibitor.

With respect to prostate cancer, the compound of the invention can be used in combination with a drug such as GnRH superagonist, antiandrogenic agent, antiestrogenic agent, chemotherapeutic agent [e.g., ifosfamide, UFT, Adriamycin, peplomycin, cisplatin, etc.], peptidic GnRH antagonist, aromatase inhibitor, 17β-hydroxysteroid dehydrogenase inhibitor, adrenal androgen production inhibitor, phosphorylase inhibitor, hormonotherapeutic agent [e.g., estrogenic agent (e.g., DSB, EMP, etc.), antiandrogenic agent (e.g., CMA, etc.), etc.], cell growth factor or a drug inhibiting the activity of receptor thereof.

With respect to breast cancer, the compound of the invention can be used in combination with a drug such as GnRH superagonist, antiestrogenic agent, chemotherapeutic agent [e.g., cyclophosphamide, 5-FU, UFT, methotrexate, Adriamycin, mitomycin C, mitoxantrone, etc.], peptidic GnRH antagonist, aromatase inhibitor, adrenal androgen production inhibitor, phosphorylase inhibitor, hormonotherapeutic agent [e.g., antiestrogenic agent (e.g., tamoxifen, etc.), luteinizing hormone (e.g., MPA, etc.), androgenic agent, estrogenic agent, etc.], cell growth factor or a drug inhibiting the activity of receptor thereof.

The administration mode for the compound of the invention and the combination drug is not particularly limited, and it will be sufficient to combine the compound of the invention and the combination drug upon administration. Examples of such administration mode include (1) administration of a single preparation obtained by simultaneously formulating the compound of the invention and the combination drug; (2) simultaneous administration of two preparations obtained by separately formulating the compound of the invention and the combination drug, via the same route of administration; (3) administration of two preparations obtained by separately formulating the compound of the invention and the combination drug, with a time interval via the same route of administration; (4) simultaneous administration of two preparations obtained by separately formulating the compound of the invention and the combination drug, via different routes of administration; (5) administration of two preparations obtained by separately formulating the compound of the invention and the combination drug, with a time interval via different routes of administration (e.g., administration in order of the compound of the invention and the combination drug, or administration in the inverse order); and the like.

When the compound of the invention is used as a prophylactic and/or therapeutic agent for the above-described diseases, or in the field of stockbreeding or fishery, the compound of the invention can be administered both orally or parenterally by a method known per se, and the compound can be orally administered after being mixed with a pharmaceutically acceptable carrier, usually as a solid preparation such as a tablet, a capsule, a granule or a powder, or can be parenterally administered as an intravenous, subcutaneous, or intramuscular injectable preparation, suppository, sublingual tablet or the like. The compound can be also administered sublingually, subcutaneously, intramuscularly and the like as a sustained release preparation such as a sublingual tablet, a microcapsule or the like. The daily dose may vary depending on the severity of symptom; the age, the gender, the body weight, the sensitivity of the subject of administration; the administration time and interval, properties, prescription or kind of the pharmaceutical preparation; the type of the active ingredient or the like. The daily dose is not particularly limited as long as the compound achieves the object of the invention. However, when the compound is used in the treatment of the above-described sex hormone-dependent cancers (e.g., prostate cancer, uterine cancer, breast cancer, pituitary tumor, etc.), prostatomegaly, hysteromyoma, endometriosis, precocious puberty or the like, the active ingredient (the compound of the invention) is administered as an oral preparation, usually in an amount of about 0.01 to 30 mg, preferably about 0.02 to 10 mg, more preferably 0.1 to 10 mg, and most preferably 0.1 to 5 mg, relative to 1 kg of body weight of a mammal, usually administered in 1 to 4 portions a day.

The dosage in the case of using the compound of the invention in the field of stockbreeding or fishery is also equivalent to the above-described range, but the active ingredient (the compound of the invention) is usually administered as an oral preparation, in an amount of about 0.01 to 30 mg, preferably about 0.1 to 10 mg, relative to 1 kg of body weight of the subject organism of administration, usually administered in 1 to 3 portions a day.

The content of Compound (I) in the pharmaceutical composition of the invention is about 0.01 to 100% by weight of the total composition.

The pharmaceutically acceptable carriers that can be used may be exemplified by various organic or inorganic carrier substances that are conventionally used as the materials for preparation, and are mixed as excipient, glidant, binding agent, disintegrant and the like in solid preparations, and as solvent, dissolution aid, suspending agent, isotonic agent, buffer, soothing agent and the like in liquid preparations.
Furthermore, if necessary, preparation additives such as antiseptic agent, antioxidant, colorant and sweetener can be also used.

Suitable examples of the excipient include lactose, sucrose, D-mannitol, starch, crystalline cellulose, light anhydrous silicic acid and the like. Suitable examples of the glidant include magnesium stearate, calcium stearate, talc, colloidal silica and the like. Suitable examples of the binding agent include crystalline cellulose, sucrose, D-mannitol, dextrin, hydroxypropylcellulose, hydroxypropylmethylcellulose, polyvinylpyrrolidone and the like. Suitable examples of the disintegrant include starch, carboxymethylcellulose, carboxymethylcellulose calcium, croscarmellose sodium, carboxymethylstarch sodium and the like. Suitable examples of the solvent include water for injection, alcohol, propylene glycol, macrogol, sesame oil, corn oil and the like. Suitable examples of the dissolution aid include polyethylene glycol, propylene glycol, D-mannitol, benzyl benzoate, ethanol, trisaminomethane, cholesterol, triethanolamine, sodium carbonate, sodium citrate and the like. Suitable examples of the suspending agent include surfactants such as stearyltriethanolamine, sodium laurylsulfate, laurylaminopropionic acid, lecithin, benzalkonium chloride, benzethonium chloride and glycerin monostearate; hydrophilic polymers such as polyvinyl alcohol, polyvinylpyrrolidone, carboxymethylcellulose sodium, methylcellulose, hydroxymethylcellulose, hydroxyethylcellulose, and hydroxypropylcellulose, and the like. Suitable examples of the isotonic agent include sodium chloride, glycerin, D-mannitol and the like. Suitable examples of the buffer include buffer solutions of phosphates, acetates, carbonates, citrates and the like. Suitable examples of the soothing agent include benzyl alcohol and the like. Suitable examples of the antiseptic agent include paraoxybenzoates, chlorobutanol, benzyl alcohol, phenethyl alcohol, dehydroacetic acid, sorbic acid and the like. Suitable examples of the antioxidant include sulfites, ascorbic acid and the like.

An intravenous, subcutaneous or intramuscular injectable preparation can be produced by adding a suspending agent, a dissolving aid, a stabilizer, an isotonic agent, a preservative or the like to the compound of the invention, according to a method known per se. Here, if necessary, the preparation can be produced as a lyophilization product by a method known per se. When the compound of the invention is administered to human, for example, the compound can be safely administered orally or parenterally, for example, as it is or as a pharmaceutical composition in which the compound of the invention is mixed with an appropriate pharmaceutically acceptable carrier, excipient or diluent.

The pharmaceutical composition may be exemplified by an oral preparation (e.g., powder, granule, capsule, tablet), or a parenteral preparation [e.g., injectable preparation, dip infusion, external agent (e.g., intranasal preparation, transdermal preparation, etc.), suppository (e.g., rectal suppository, vaginal suppository, etc.), etc.].

These preparations can be produced by methods known per se that are generally used in the processes for producing preparations.

The compound of the invention can be produced into an injectable preparation by formulating the compound into aqueous injectable preparations together with dispersants (e.g., Tween 80 (Atlas Powder Co., US), HC060 (Nikko Chemicals Co., Ltd.), polyethylene glycol, carboxymethylcellulose, sodium alginate, etc.), preservatives (e.g., methylparaben, propylparaben, benzyl alcohol, etc.), isotonic agents (e.g., sodium chloride, mannitol, sorbitol, glucose, etc.) and the like, or into an oil-based injectable preparation by dissolving, suspending or emulsifying the compound in plant oils such as olive oil, sesame oil, cotton seed oil or corn oil, propylene glycol or the like.

The compound of the invention can be produced into an oral preparation according to a method known per se by mixing the compound with, for example, excipients (e.g., lactose, sucrose, starch, etc.), disintegrants (e.g., starch, calcium carbonate, etc.), binding agents (e.g., starch, gum arabic, carboxymethylcellulose, polyvinylpyrrolidone, hydroxypropylcellulose, etc.), glidants (e.g., talc, magnesium stearate, polyethylene glycol 6000, etc.) or the like; molding the mixture by compression; and then coating the compression product by a method known per se, if necessary, for the purposes of masking of taste, or enteric or sustained release. Examples of the coating agent that can be used for the purpose include hydroxypropylmethylcellulose, ethylcellulose, hydroxymethylcellulose, hydroxypropylcellulose, polyoxyethylene glycol, Tween 80, Pluronic F68, cellulose acetate phthalate, hydroxypropylmethylcellulose phthalate, hydroxymethylcellulose acetate succinate, Eudragit (Röhm Pharma GmbH, Germany; copolymer of methacrylic acid and acrylic acid), colorants (e.g., red iron oxide, titanium dioxide, etc.) and the like.
In order to use Compound (I) of the invention as enteric preparations, an intermediate phase can be provided in between an enteric phase and a drug-containing phase of the preparation by a method known per se, for the purpose of separation of the enteric phase and the drug-containing phase.

In order to use the compound of the invention as external preparations, the compound or a salt thereof can be formulated into solid, semi-solid or liquid externally administered preparations by methods known per se. For the solid preparation for example, the compound of the invention or a salt thereof is directly used or mixed with excipients (e.g., glycol, mannitol, starch, microcrystalline cellulose, etc.), thickening agents (e.g., natural gums, cellulose derivatives, acrylic acid polymers, etc.) and the like, to be produced into a pulverized composition. For the liquid preparation, an oil-based preparation or an aqueous suspension is produced, in the virtually same manner as in the preparation of injectable preparations. For the semi-solid preparation, aqueous or oil-based gel preparations, or ointments are preferable. All of these preparations may also have pH adjusting agents (e.g., carbonate, phosphate, citrate, chloride, hydroxide of sodium, etc.), antiseptic agents (e.g., paraoxybenzoic acid esters, chlorobutanol, benzalkonium chloride, etc.) and the like added therein.

For example, in order to use the compound of the invention as suppositories, the compound can be formulated into oil-based or aqueous, solid, semi-solid or liquid suppositories by methods known per se. The oil-based base that can be used in the compositions may be exemplified by glycerides of high fatty acids [e.g., cacao butter, Witepsols (Dynamit Nobel, Inc., Germany), etc.], intermediate fatty acids [e.g., miglyols (Dynamit Nobel, Inc., Germany), etc.], plant oils (e.g., sesame oil, soybean oil, cotton seed oil, etc.) or the like. The aqueous base that can be used in the compositions may be exemplified by polyethylene glycols, propylene glycol or the like, and the aqueous gel base may be exemplified by natural gums, cellulose derivatives, vinyl polymers, acrylic acid polymers or the like.

The present invention is further described in detail in with reference to Reference Examples, Examples, Preparation Examples and Experimental Examples which are not intended to restrict the invention.

¹H-NMR spectra were measured with a Varian GEMINI 200 (200 MHz) spectrometer, a MERCURY 300 (300 MHz) spectrometer or a BRUKER AVANCE 300 spectrometer (300 MHz) using tetramethylsilane as an internal standard. All of the δ values are represented in ppm.

Preparative HPLC was measured under the following conditions.
Instrument: LC-10Avp, Shimadzu Corporation.
Column: Capcell Pak C18UG 120, S-3 µm, 2.0 × 50 mm
Solvent: Solution A; 0.1% trifluoroacetic acid-containing water, Solution B; 0.1% trifluoroacetic acid-containing acetonitrile
Gradient cycle: 0.00 minute (Solution A/Solution B = 90/10), 4.00 minutes (Solution A/Solution B = 5/95), 5.50 minutes (Solution A/Solution B = 5/95), 5.51 minutes (Solution A/Solution B = 90/10), 8.00 minutes (Solution A/Solution B = 90/10)
Flow rate: 0.5 ml/min

The number indicated in the mixed solvent means a mixing ratio by volume of each solvent unless otherwise stated. Further, the eluting solvent in silica gel chromatography means a ratio by volume unless otherwise stated. % means weight% unless otherwise stated. Provided that, yield means mol/mol%. Other symbols used in the present text indicate the following meanings.
s: Singlet
d: Doublet
t: Triplet
q: Quartet
dd: Double doublet
dt: Double triplet
td: Triple doublet
dq: Double quartet
ddd: Double double doublet
m: Multiplet
br: Broad
Hz: Hertz
CDCl₃: deutero chloroform
DMSO-d₆: deutero dimethylsulfoxide
CD₃OD: deutero methanol
AIBN: 2,2-azobisisobutyronitrile
DMF: N,N-dimethylformamide
NBS: N-bromosuccinimide
TFA: trifluoroacetic acid
THF: tetrahydrofuran
DMAP: 4-N,N-dimethylaminopyridine
Me: Methyl
Et: Ethyl
Ph: Phenyl
TMS: Trimethylsilyl
TBDMS: tert-Butyl(dimethyl)silyl
Cbz: Benzyloxycarbonyl

Room temperature represents a temperature of about 15°C to about 30°C, but is not particularly exactly limited.

As for corn starch, magnesium stearate, lactose and distilled water for injection, which are used in Preparation Examples, products in conformity to the 14^{th} revision of the Japanese Pharmacopoeia were used.

### EXAMPLES

### Reference Example 1

### 4-Chloro-N-ethyl-3-nitro-N-phenylbenzenesulfonamide

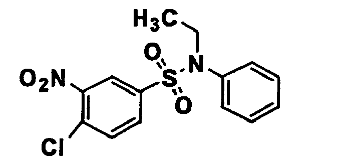

While stirring N-ethyl aniline (5.22 g) and sodium hydrogen carbonate (4.93 g) in THF (100 ml) and water (10 ml), a solution of 4-chloro-3-nitrobenzensulfonyl chloride (10.0 g) in THF (50 ml) was added at room temperature, and the mixture was stirred overnight as it was. The reaction solution was poured into water, and extracted twice with ethyl acetate. The collected organic layers were dried over anhydrous magnesium sulfate, passed through silica gel, and then the solvent was distilled off under reduced pressure. The resulting residue was crystallized from diisopropyl ether/hexane to give the desired product (11.9 g) as crystals.

¹H-NMR (CDCl₃) δ 1. 13 (3H, t), 3.65 (2H, q), 7.04-7.08 (2H, m), 7.35-7.39 (3H, m), 7.62-7.69 (2H, m), 8. 07-8. 08 (1H, m)

### Reference Examples 1(1) to 1(14)

In the same manner as in Reference Example 1, the corresponding sulfonyl chlorides (commercially available or conventional) were reacted with the corresponding amine (commercially available or conventional) to obtain the following compounds.

### Reference Example 1(1)

### N-Ethyl-4-methyl-3-nitro-N-phenylbenzenesulfonamide

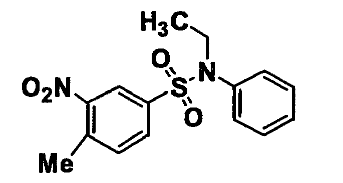

¹H-NMR (CDCl₃) δ 1.11 (3H, t), 2.68 (3H, s), 3.65 (2H, q), 7.04-7.07 (2H, m), 7.32-7.37 (3H, m), 7.44 (1H, d), 7.65 (1H, dd), 8.20 (1H, d)

### Reference Example 1(2)

### N-Ethyl-3-nitro-N-phenylbenzenesulfonamide

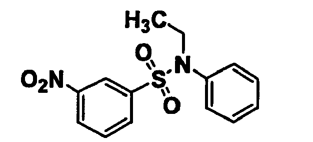

¹ H-NMR (CDCl₃) δ 1. 13 (3H, t), 3.67 (2H, q), 7.01-7.06 (2H, m), 7.31-7.36 (3H, m), 7.65 (1H, t), 7. 87 (1H, td), 8.41 (1H, ddd), 8.46 (1H, t)

### Reference Example 1(3)

### 4-Chloro-3-nitro-N-phenylbenzenesulfonamide

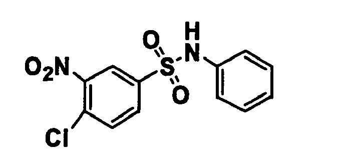

¹H-NMR (CDCl₃) δ 6.66 (1H, br s), 7.07-7.12 (2H, m), 7.19-7.35 (3H, m), 7.63 (1H, d), 7.81 (1H, dd, 8.6Hz), 8.24 (1H, d)

### Reference Example 1(4)

### N-(2-Chloro-4-((ethyl(phenyl)amino)sulfonyl)phenyl)acetamide

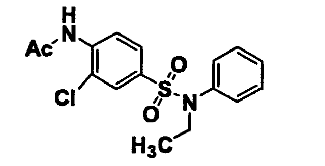

¹H-NMR (CDCl₃) δ 1.08 (3H, t), 2.29 (3H, s), 3.60 (2H, q), 7.03-7.07 (2H, m), 7.30-7.35 (3H, m), 7.48 (1H, dd), 7.60 (1H, d), 7.77 (1H, br s), 8.55 (1H, d)

### Reference Example 1(5)

### N-(2-Methylphenyl)-3-nitrobenzenesulfonamide

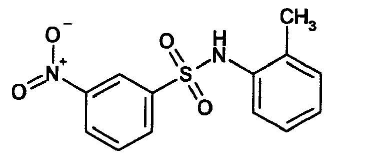

¹H-NMR (CDCl₃) δ 2.04 (3H, s), 6.47 (1H, s), 7.12-7.21 (3H, m), 7.25-7.28 (1H, m), 7.66 (1H, t), 8.02 (1H, ddd), 8.41 (1H, ddd), 8.61 (1H, t)

### Reference Example 1(6)

### N-(2-Butylphenyl)-3-nitrobenzenesulfonamide

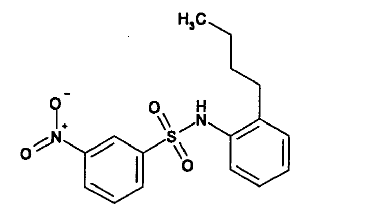

¹H-NMR (CDCl₃) δ 0.85 (3H, t), 1.20-1.37 (4H, m), 2.32 (2H, t), 6.52 (1H, s), 7. 11-7.20 (3H, m), 7.29-7.33 (1H, m), 7.66 (1H, t), 8.02 (1H, ddd), 8.41 (1H, ddd), 8.61 (1H, t)

### Reference Example 1(7)

### N-(2-Methoxyphenyl)-3-nitrobenzenesulfonamide

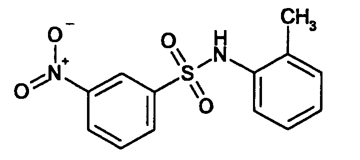

¹H-NMR (CDCl₃) δ 3.64 (3H, s), 6.72 (1H, dd), 6.93 (1H, dt), 7.06 (1H, s), 7. 09 (1H, dt), 7. 54 (1H, dd), 7.59 (1H, t), 8.02-8.05 (1H, m), 8.32-8.35 (1H, m), 8.60 (1H, t)

### Reference Example 1(8)

### N-(2-Fluorophenyl)-3-nitrobenzenesulfonamide

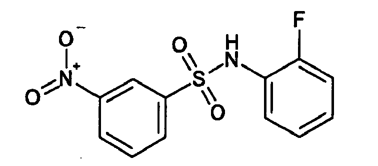

¹H-NMR (CDCl₃) δ 6.81 (1H, s), 6.94-7.01 (1H, m), 7.14-7.20 (2H, m), 7.59-7.65 (1H, m), 7.67 (1H, t), 8.05-8.08 (1H, m), 8.41 (1H, ddd), 8.61 (1H, t)

### Reference Example 1(9)

### 3-Nitro-N-5,6,7,8-tetrahydronaphthalen-1-ylbenzenesulfonamide

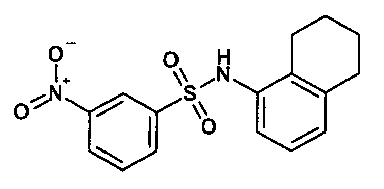

¹H-NMR (CDCl₃) δ 1.63-1.68 (4H, m), 2.34-2.36 (2H, m), 2.69-2.71 (2H, m), 6.41 (1H, s), 6.93-6.96 (1H, m), 7.02-7.10 (2H, m), 7.65 (1H, t), 8.01-8.04 (1H, m), 8.39 (1H, ddd), 8.59 (1H, t)

### Reference Example 1(10)

### 3-Nitro-N-1,2,3,4-tetrahydronaphthalen-1-ylbenzenesulfonamide

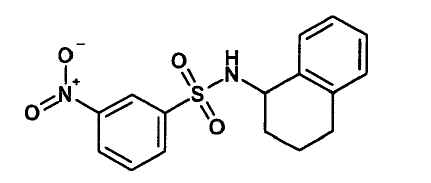

¹H-NMR (CDCl₃) δ 1.75-1.89 (4H, m), 2.69-2.77 (2H, m), 4. 56-4.59 (1H, m), 4.85 (1H, d), 6.95 (1H, d), 7. 04-7. 09 (2H, m), 7.13-7.18 (1H, m), 7.77 (1H, t), 8.26 (1H, ddd), 8.46 (1H, ddd), 8.77 (1H, t)

### Reference Example 1(11)

### N-[2-(1-Hydroxyethyl)phenyl]-3-nitrobenzenesulfonamide

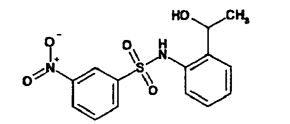

¹H-NMR (CDCl₃) δ 1.39 (3H, d), 2.35 (1H, s), 4.87-4.88 (1H, m), 7.07-7.09 (2H, m), 7.21-7.25 (1H, m), 7.49 (1H, d), 7.68 (1H, t), 8.14-8.17 (1H, m), 8.39 (1H, ddd), 8.70 (1H, t), 8.83 (1H, s)

### Reference Example 1(12)

### 2-{[(3-Nitrophenyl)sulfonyl]amino}benzamide

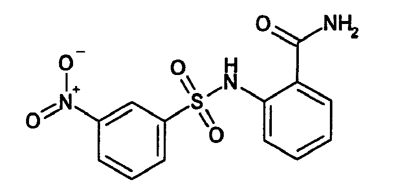

¹H-NMR (DMSO-d₆) δ 7.15 (1H, ddd), 7.46-7.53 (2H, m), 7.74-7.76 (1H, m), 7.84 (1H, t), 7.86 (1H, s), 8.15 (1H, ddd), 8.29 (1H, s), 8.39 (1H, t), 8.45 (1H, ddd), 12.31 (1H, s)

### Reference Example 1(13)

### N-[2-(Benzyloxy)phenyl]-3-nitrobenzenesulfonamide

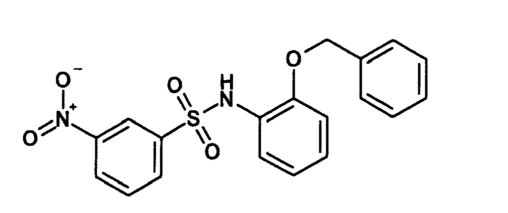

¹H-NMR (CDCl₃) δ 4.83 (2H, s), 6.81 (1H, dd), 6.97 (1H, dd), 7.00-7.01 (1H, m), 7.08-7.15 (3H, m), 7.35-7.37 (3H, m), 7.54 (1H, t), 7.61 (1H, dd), 7.92-7.95 (1H, m), 8.33 (1H, ddd), 8.54 (1H, t)

### Reference Example 1(14)

### Methyl 2-{[(3-nitrophenyl)sulfonyl]amino}benzoate

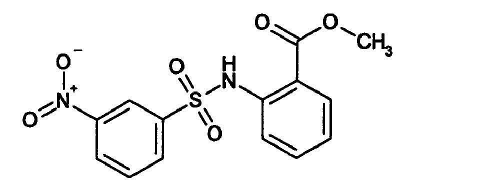

¹H-NMR (CDCl₃) δ 3.89 (3H, s), 7. 10 (1H, ddd), 7.51 (1H, ddd), 7.66 (1H, t), 7.73 (1H, dd), 7.94 (1H, dd), 8.18 (1H, ddd), 8.37 (1H, ddd), 8.68 (1H, t), 10.84 (1H, s)

### Reference Example 2

### 4-Chloro-N-methyl-3-nitro-N-phenylbenzenesulfonamide

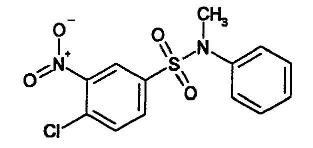

To a solution of N-methylaniline (1.54 g), triethylamine (1.46 g) and DMAP (147 mg) in dichloromethane (50 ml), 4-chloro-3-nitrobenzensulfonyl chloride (3.07 g) was added and the mixture was agitated for 2 hours at room temperature, after which the reaction mixture was poured into water and extracted with dichloromethane. The organic layer was sequentially washed with 1 N hydrochloric acid, water, a saturated sodium bicarbonate solution, water and saturated brine, and dried over anhydrous magnesium sulfate. The solvent was distilled off under reduced pressure, and the crystals were collected by filtration to give the desired product (3.49 g).

¹H-NMR (CDCl₃) δ 3.24 (3H, s), 7.08-7.13 (2H, m), 7.33-7.37 (3H, m), 7.60 (1H, dd), 7.65 (1H, d), 8.02 (1H, d)
MS (ESI+, m/e) 327 (M+1)

### Reference Examples 2(1) to 2(78)

In the same manner as in Reference Example 2, the corresponding sulfonyl chlorides (commercially available or conventional) were reacted with the corresponding amine (commercially available, conventional or synthesized in Reference Examples) to obtain the following compounds.

### Reference Example 2(1)

### 4-Chloro-N-isopropyl-3-nitro-N-phenylbenzenesulfonamide

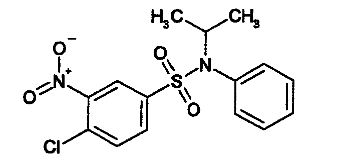

¹H-NMR (CDCl₃) δ 1.10 (6H, d), 4.65 (1H, sevenplet), 7.01-7.05 (2H, m), 7.35-7.43 (3H, m), 7.65 (1H, d), 7.81 (1H, dd), 8.19 (1H, d)
MS (ESI+, m/e) 355 (M+1)

### Reference Example 2(2)

### N-1,3-Benzodioxol-5-yl-4-chloro-N-ethyl-3-nitrobenzenesulfonamide

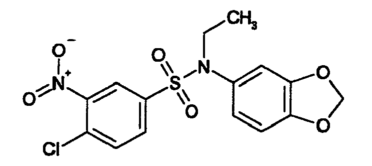

¹H-NMR (CDCl₃) δ 1.13 (3H, t), 3.58 (2H, q), 6.02 (2H, s), 6.44 (1H, dd), 6.58 (1H, d), 6.74 (1H, d), 7.66 (1H, d), 7.74 (1H, dd), 8.11 (1H, d)
MS (ESI+, m/e) 385 (M+1)

### Reference Example 2(3)

### 4-Chloro-N-(2-cyanoethyl)-3-nitro-N-phenylbenzenesulfonamide

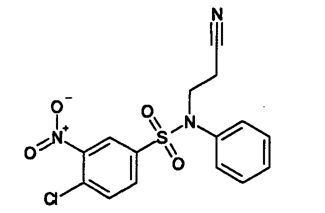

¹H-NMR (CDCl₃) δ 2.64 (2H, t), 3.90 (2H, t), 7.09-7.13 (2H, m), 7.39-7.44 (3H, m), 7.65-7.75 (2H, m), 8.06-8.07 (1H, m)
MS (ESI+, m/e) 366 (M+1)

### Reference Example 2(4)

### 1-((4-Chloro-3-nitrophenyl)sulfonyl)-1,2,3,4-tetrahydroquinoline

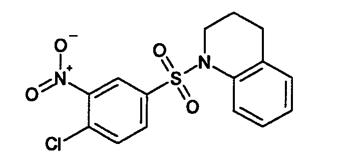

1 H-NMR (CDCl₃) δ 1.66-1.78 (2H, m), 2.49 (2H, t), 3.85 (2H, t), 7.03-7.07 (1H, m), 7.14 (1H, dt), 7.23-7.27 (1H, m), 7.57 (1H, d), 7.63 (1H, dd), 7.74-7.78 (1H, m), 8.08 (1H, d)
MS (ESI+, m/e) 353 (M+1)

### Reference Example 2(5)

### 2-((4-Chloro-3-nitrophenyl)sulfonyl)-1,2,3,4-tetrahydroisoquinoline

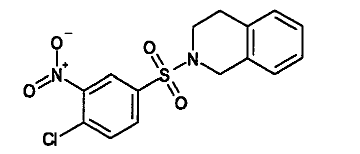

¹H-NMR (CDCl₃) δ 2.94 (2H, t), 3.49 (2H, t), 4.37 (2H, s), 7.05-7.20 (4H, m), 7.71 (1H, d), 7.94 (1H, dd), 8.30 (1H, d)
MS (ESI+, m/e) 353 (M+1)

### Reference Example 2(6)

### 1-((4-Chloro-3-nitrophenyl)sulfonyl)-4-ethylpiperazine

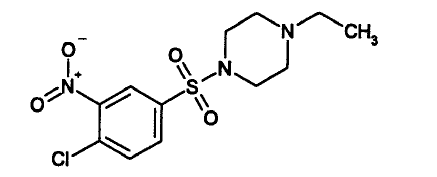

¹H-NMR (CDCl₃) δ 1.04 (3H, t), 2.42 (2H, q), 2.54 (4H, t), 3.10 (4H, t), 7.74 (1H, d), 7.87 (1H, dd), 8.22 (1H, d)
MS (ESI+, m/e) 334 (M+1)

### Reference Example 2(7)

### 4-Chloro-N,N-diethyl-3-nitrobenzenesulfonamide

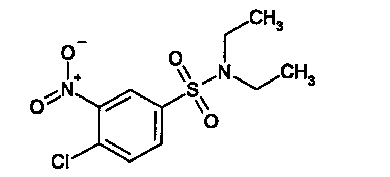

¹H-NMR (CDCl₃) δ 1.18 (6H, t), 3.29 (4H, q), 7.70 (1H, d), 7.93 (1H, dd), 8.28 (1H, d)
MS (ESI+, m/e) 293 (M+1)

### Reference Example 2(8)

### N-Benzyl-4-chloro-N-ethyl-3-nitrobenzenesulfonamide

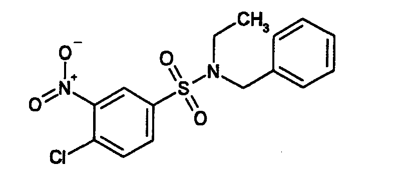

¹H-NMR (CDCl₃) δ 1.03 (3H, t), 3.28 (2H, q), 4.41 (2H, s), 7.26-7.35 (5H, m), 7.68 (1H, d), 7.90 (1H, dd), 8.22 (1H, d)
MS (ESI+, m/e) 355 (M+1)

### Reference Example 2(9)

### 1-((4-Chloro-3-nitrophenyl)sulfonyl)-6-methyl-1,2,3,4-tetrahydroquinoline

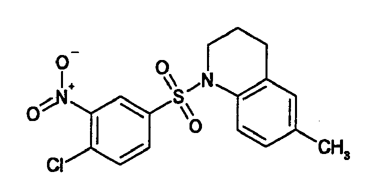

¹H-NMR (CDCl₃) δ 1.61-1.74 (2H, m), 2.30 (3H, s), 2.43 (2H, t), 3.83 (2H, t), 6.86 (1H, s), 7.03 (1H, d), 7.57 (1H, d), 7.63 (1H, dd), 7.63 (1H, d), 8.06 (1H, d)
MS (ESI+, m/e) 367 (M+1)

### Reference Example 2(10)

### 1-((4-Chloro-3-nitrophenyl)sulfonyl)-2-methyl-1,2,3,4-tetrahydroquinoline

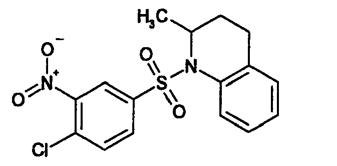

¹H-NMR (CDCl₃) δ 1.33 (3H, d), 1.38-1.49 (1H, m), 1.70-2.00 (2H, m), 2.40-2. 53 (1H, m), 4.37 (1H, q), 7.02 (1H, d), 7.16 (1H, dt), 7.28 (1H, dt), 7.49 (1H, dd), 7.54 (1H, d), 7.72 (1H, d), 7.96 (1H, d)
MS (ESI+, m/e) 367.(M+J)

### Reference Example 2(11)

### 1-((4-Chloro-3-nitrophenyl)sulfonyl)-2-methylindoline

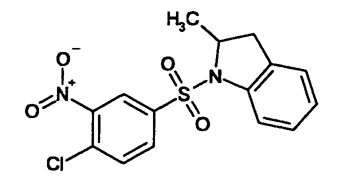

¹H-NMR (CDCl₃) δ 1.47 (3H, d), 2.54 (1H, dd), 2.99 (1H, dd), 4.34-4.41 (1H, m), 7.06-7.12 (2H, m), 7.23-7.28 (1H, m), 7.57 (1H, d), 7.64 (1H, d), 7.75 (1H, dd), 8.19 (1H, d)
MS (ESI+, m/e) 353 (M+1)

### Reference Example 2(12)

### 1-((4-Chloro-3-nitrophenyl)sulfonyl)-5-methylindoline

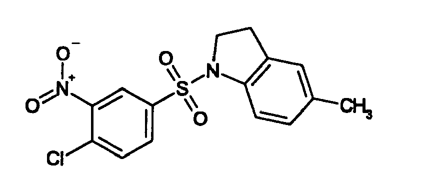

¹H-NMR (CDCl₃) δ 2.28 (3H, s), 2.90 (2H, t), 3.94 (2H, t), 6.94 (1H, s), 7.03 (1H, d), 7.48 (1H, d), 7.62 (1H, d), 7.86 (1H, dd), 8.26 (1H, d)
MS (ESI+, m/e) 353 (M+1)

### Reference Example 2(13)

### 4-((4-Chloro-3-nitrophenyl)sulfonyl)-3,4-dihydro-2H-1,4-benzoxazine

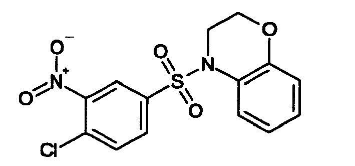

¹H-NMR (CDCl₃) δ 3.88-3.96 (4H, m), 6.84 (1H, dd), 6.97 (1H, dt), 7.12 (1H, dt), 7.62 (1H, d), 7.69 (1H, dd), 7.79 (1H, dd), 8.15 (1H, d)
MS (ESI+, m/e) 355 (M+1)

### Reference Example 2(14)

### 1-((4-Chloro-3-nitrophenyl)sulfonyl)-2,3,4,5-tetrahydro-1H-1-benzazepine

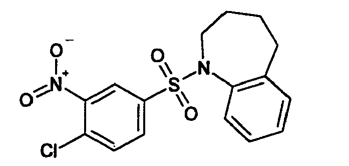

¹H-NMR (CDCl₃) δ 1.60-1.62 (2H, m), 1.79-1.87 (2H, m), 2.40-2.45 (2H, m), 3.73-3.75 (2H, m), 7.13-7.24 (4H, m), 7.65 (1H, d), 7.80 (1H, dd), 8.20 (1H, d)
MS (ESI+, m/e) 367 (M+1)

### Reference Example 2(15)

### 4-Chloro-N-methyl-3-nitro-N-4-pyridinylbenzenesulfonamide

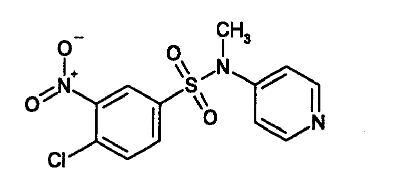

¹H-NMR (CDCl₃) δ 3.29 (3H, s), 7.17 (2H, d), 7.60 (1H, dd), 7.67 (1H, d), 8.12 (1H, d), 8.59 (2H, d)
MS (ESI+, m/e) 328 (M+1)

### Reference Example 2(16)

### 1-((4-Chloro-3-nitrophenyl)sulfonyl)-1,2,3,4-tetrahydro-1,5-naphthylidine

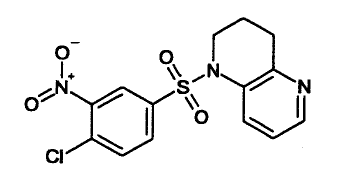

¹H-NMR (CDCl₃) δ 1.80 (2H, quintet), 2.80 (2H, t), 3.86 (2H, t), 7.19 (1H, dd), 7.63-7.64 (2H, m), 8.12 (1H, dd), 8.17 (1H, s), 8.37 (1H, dd)
MS (ESI+, m/e) 354 (M+1)

### Reference Example 2(17)

### 5-((4-Chloro-3-nitrophenyl)sulfonyl)-2,3,4,5-tetrahydro-1,5-benzoxazepine

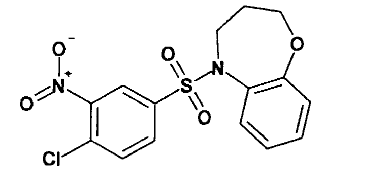

¹H-NMR (CDCl₃) δ 1.86 (2H, quintet), 3.84-3.85 (4H, m), 6.98 (1H, dd), 7.15 (1H, dt), 7.29 (1H, dt), 7.51 (1H, dd), 7.56 (1H, d), 7.59 (1H, dd), 8.03 (1H, d)
MS (ESI+, m/e) 369 (M+1)

### Reference Example 2(18)

### 1-((4-Chloro-3-nitrophenyl)sulfonyl)-1,2,3,4,5,6-hexahydro-1-benzazocine

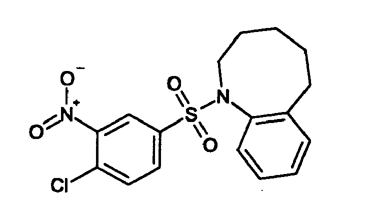

¹H-NMR (CDCl₃) δ 1.23-4.08 (10H, m), 6.56 (1H, d), 7.10-7.16 (1H, m), 7.28-7.31 (2H, m), 7.71 (1H, d), 7.89 (1H, dd), 8.27 (1H, d)
MS (ESI+, m/e) 381 (M+1)

### Reference Example 2(19)

### 1-((2-Chloro-5-nitrophenyl)sulfonyl)-1,2,3,4-tetrahydroquinoline

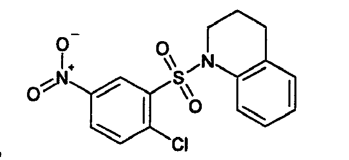

¹H-NMR (CDCl₃) δ 1. 96 (2H, quintet), 2.76 (2H, t), 3.94 (2H, t), 7.04-7.12 (3H, m), 7.33-7.38 (1H, m), 7.67 (1H, d), 8.32 (1H, dd), 8.96 (1H, d)
MS (ESI+, m/e) 353 (M+1)

### Reference Example 2(20)

### 4-Chloro-N-cyclohexyl-N-methyl-3-nitrobenzenesulfonamide

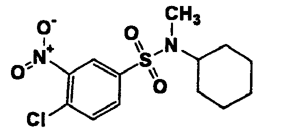

¹H-NMR (CDCl₃) δ 0.86-1.79 (10H, m), 2.79 (3H, s), 3.78 (1H, m), 7.71 (1H, d), 7.93 (1H, dd), 8.28 (1H, d)
MS (ESI+, m/e) 333 (M+1)

### Reference Example 2(21)

### 1-((4-Chloro-3-nitrophenyl)sulfonyl)indoline

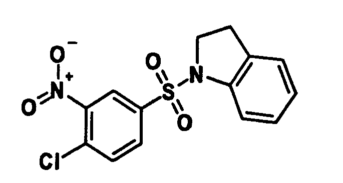

¹H-NMR (CDCl₃) δ 2. 98 (2H, t), 3. 97 (2H, t), 7.01-7.28 (3H, m), 7.59-7.65 (2H, m), 7.89 (1H, dd), 8.28 (1H, d)
MS (ESI+, m/e) 339 (M+1)

### Reference Example 2(22)

### 4-Chloro-N-ethyl-3-nitro-N-phenylbenzenesulfonamide

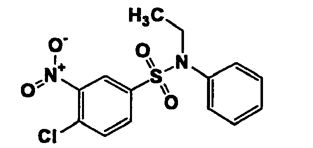

¹H-NMR (CDCl₃) δ 1.13 (3H, t), 3.65 (2H, q), 7.04-7.08 (2H, m), 7.35-7.39 (3H, m), 7.62-7.69 (2H, m), 8.07 (1H, d)
MS (ESI+, m/e) 341 (M+1)

### Reference Example 2(23)

### 4-Chloro-N-(4-chlorophenyl)-N-ethyl-3-nitrobenzenesulfonamide

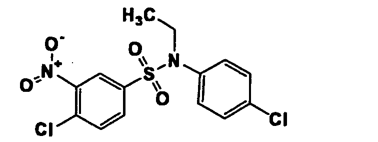

¹H-NMR (CDCl₃) δ 1.12 (3H, t), 3.62 (2H, q), 7.01 (2H, d), 7.35 (2H, d), 7.65-7.67 (2H, m), 8.11 (1H, s)
MS (ESI+, m/e) 375 (M+1)

### Reference Example 2(24)

### 4-Benzyl-1-((4-chloro-3-nitrophenyl)sulfonyl)piperidine

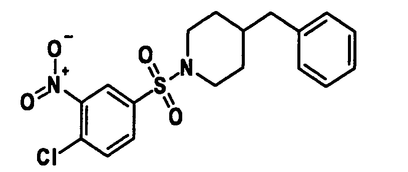

¹H-NMR (CDCl₃) δ 1.33-3.83 (7H, m), 2.53 (2H, d), 7.07-7.09 (5H, m), 7.71 (1H, d), 7.85 (1H, dd), 8.20 (1H, d)
MS (ESI+, m/e) 395 (M+1)

### Reference Example 2(25)

### 4-Chloro-N-(2-(dimethylamino)-1-phenylethyl)-N-methyl-3-nitrobenzenesulfonamide

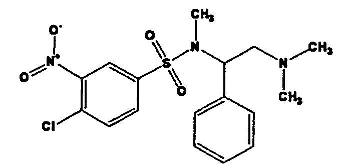

¹H-NMR (CDCl₃) δ 2.25 (6H, s), 2.29 (3H, s), 2.32-2.96 (2H, m), 5.33-5.38 (1H, m), 7.26-7.40 (5H, m); 7.61 (1H, d), 7.98 (1H, d), 8.62 (1H, s)
MS (ESI+, m/e) 398 (M+1)

### Reference Example 2(26)

### 4-Chloro-N-methyl-3-nitro-N-2-pyridinylbenzenesulfonamide

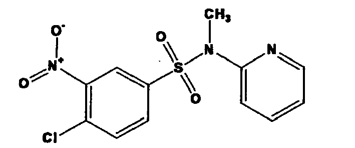

¹H-NMR (CDCl₃) δ 3.30 (3H, s), 7.22 (1H, dd), 7.58 (1H, d), 7.64 (1H, d), 7.73 (1H, dd), 7.79 (1H, d), 8.09 (1H, d), 8.32 (1H, d)
MS (ESI+, m/e) 328 (M+1)

### Reference Example 2(27)

### N-Butyl-4-chloro-3-nitro-N-phenylbenzenesulfonamide

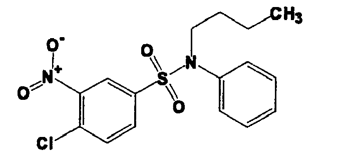

¹H-NMR (CDCl₃) δ 0.88 (3H, t), 1.30-1.48 (4H, m), 3.57 (2H, t), 7.03-7.08 (2H, m), 7.34-7.39 (3H, m), 7.61-7.67 (2H, m), 8.05 (1H, s)
MS (ESI+, m/e) 369 (M+1)

### Reference Example 2(28)

### 1-((2-Methyl-5-nitrophenyl)sulfonyl)-1,2,3,4-tetrahydroquinoline

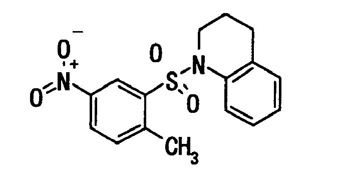

¹H-NMR (CDCl₃) δ 1.78-1.94 (2H, m), 2.49 (3H, s), 2.69 (2H, t), 3.84 (2H, t), 7.05-7.21 (3H, m), 7.42 (1H, d), 7.47 (1H, d), 8.27 (1H, dd), 8.74 (1H, d)
MS (ESI+, m/e) 333 (M+1)

### Reference Example 2(29)

### 1-[(3-Nitrophenyl)sulfonyl]indoline

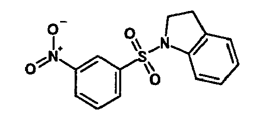

¹H-NMR (CDCl₃) δ 2.94 (2H, t), 3.99 (2H, t), 6.98-7.12 (2H, m), 7.20-7.27 (1H, m), 7.64 (1H, d), 7.66 (1H, t), 8.08-8.12 (1H, m), 8.37-8.42 (1H, m), 8.65 (1H, t)

### Reference Example 2(30)

### 1-[(3-Nitrophenyl)sulfonyl]-1,2,3,4-tetrahydroquinoline

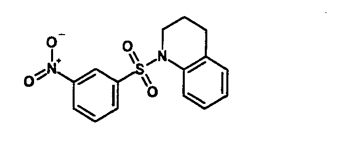

¹H-NMR (CDCl₃) δ 1.63-1.77 (2H, m), 2.44 (2H, t), 3.87 (2H, t), 7.00-7.27 (3H, m), 7.60 (1H, t), 7.76-7.88 (2H, m), 8.37 (1H, ddd), 8.47 (1H, t)

### Reference Example 2(31)

### 1-[(3-Nitrophenyl)sulfonyl]-2,3,4,5-tetrahydro-1H-1-benzazepine

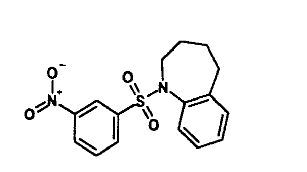

¹H-NMR (CDCl₃) δ 1. 57-1. 79 (2H, m), 1.84 (2H, quintet), 2.38 (2H, t), 3.75-3.77 (2H, m), 7.12-7.26 (4H, m), 7.67 (1H, t), 7.98-8.01 (1H, m), 8.39-8.43 (1H, m), 8.59 (1H, t)

### Reference Example 2(32)

### 1-[(3-Nitrophenyl)sulfonyl]-1,2,3,4,5,6-hexahydro-1-benzazocine

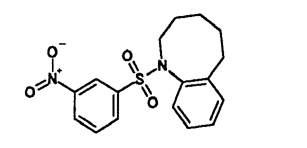

¹H-NMR (CDCl₃) δ 1.24-1.70 (6H, m), 2.94-4.10 (4H, m), 6.52 (1H, d), 7.07-7.13 (1H, m), 7.28-7.31 (2H, m), 7.75 (1H, t), 8.10-8.13 (1H, m), 8.46 (1H, ddd), 8.67 (1H, t)

### Reference Example 2(33)

### 2-Methyl-1-[(3-nitrophenyl)sulfonyl]-1,2,3,4-tetrahydroquinoline

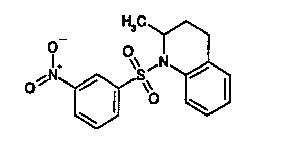

¹H-NMR (CDCl₃) δ 1.34 (3H, d), 1.37-1.42 (1H, m), 1.64-1.70 (1H, m), 1.86-1.92 (1H, m), 2.36-2.43 (1H, m), 4.40 (1H, sextet), 6.98 (1H, d), 7.16 (1H, t), 7.28 (1H, t), 7.53-7.59 (1H, m), 7.69-7.73 (1H, m), 7. 75 (1H, d), 8.34-8.37 (2H, m)

### Reference Example 2(34)

### 1-[(4-Fluoro-3-nitrophenyl)sulfonyl]-1,2,3,4-tetrahydroquinoline

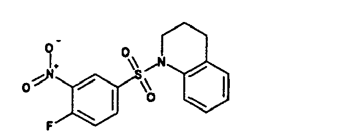

¹H-NMR (CDCl₃) δ 1.72 (2H, quintet), 2.48 (2H, t), 3.86 (2H, t), 7.03 (1H, d), 7.12 (1H, t), 7.20-7.23 (1H, m), 7.31 (1H, t), 7.74-7.78 (2H, m), 8.29-8.32 (1H, m)

### Reference Example 2(35)

### 4-[(3-Nitrophenyl)sulfonyl]-5,6,7,8-tetrahydro-4H-thieno[3,2-b]azepine

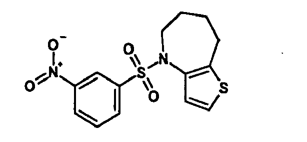

¹H-NMR (CDCl₃) δ 1.48-1.53 (2H, m), 1.72-1.77 (2H, m), 2.19-2.22 (2H, m), 3.77-3.79 (2H, m), 6.98 (1H, d), 7.04 (1H, d), 7.66 (1H, t), 7.90-7.94 (1H, m), 8.41 (1H, ddd), 8.54 (1H, t)

### Reference Example 2(36)

### 1-[(3-nitrophenyl)sulfonyl]-1,2,3,5-tetrahydro-4,1-benzothiazepine

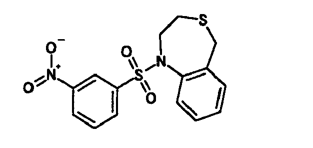

¹H-NMR (CDCl₃) δ 2.99-3.64 (6H, m), 6.97 (1H, d), 7.18 (1H, dt), 7.22-7.31 (2H, m), 7.74 (1H, t), 8.07-8.10 (1H, m), 8.45-8.48 (1H, m), 8.63 (1H, t)

### Reference Example 2(37)

### 5-[(3-Nitrophenyl)sulfonyl]-2,3,4,5-tetrahydro-1,5-benzoxazepine

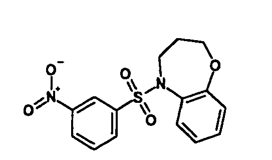

¹H-NMR (CDCl₃) δ 1.83-1.88 (2H, m), 3.79-3.81 (2H, m), 3.88-3.90 (2H, m), 6.95 (1H, dd), 7.16 (1H, dt), 7.29 (1H, dt), 7.54 (1H, dd), 7.59 (1H, t), 7.80-7.83 (1H, m), 8.35-8.39 (1H, m), 8.45 (1H, t)

### Reference Example 2(38)

### 5-[(3-Nitrophenyl)sulfonyl]-2,3,4,5-tetrahydro-1,5-benzothiazepine

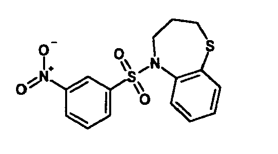

¹H-NMR (CDCl₃) δ 2.07 (2H, quintet), 2.58-2.60 (2H, m), 3.65-4.05 (2H, m), 7.24 (1H, dt), 7.34 (1H, dt), 7.47 (1H, dd), 7.57 (1H, t), 7.58 (1H, dd), 7.85-7.89 (1H, m), 8.34-8.37 (1H, m), 8.50 (1H, t)

### Reference Example 2(39)

### 5-Methyl-1-[(3-nitrophenyl)sulfonyl]-1,2,3,4-tetrahydroquinoline

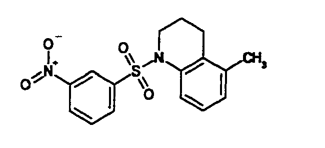

¹H-NMR (CDCl₃) δ 1.72 (2H, quintet), 2.15 (3H, s), 2.39 (2H, t), 3.87 (2H, t), 7.02 (1H, d), 7. 14 (1H, t), 7.61 (1H, d), 7.61 (1H, t), 7.86-7.90 (1H, m), 8.37 (1H, ddd), 8.49 (1H, t)

### Reference Example 2(40)

### 4-Methyl-1-[(3-nitrophenyl)sulfonyl]-1,2,3,4-tetrahydroquinoline

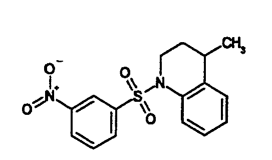

¹H-NMR (CDCl₃) δ 1.01 (3H, d), 1.31-1.37 (1H, m), 1.77-1.84 (1H, m), 2.55 (1H, sextet), 3.80-3.92 (2H, m), 7.12-7.27 (3H, m), 7.61 (1H, t), 7.78-7.81 (1H, m), 7.82-7.86 (1H, m), 8.37 (1H, ddd), 8.47 (1H, t)

### Reference Example 2(41)

### 1-[(3-Nitrophenyl)sulfonyl]-1,2,3,5-tetrahydro-4,1-benzoxazepine

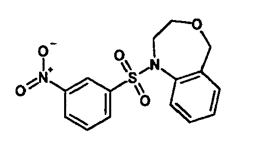

¹H-NMR (CDCl₃) δ 3.87-3.89 (4H, m), 4.28-4.30 (2H, m), 7.20-7.33 (4H, m), 7.69 (1H, t), 7.99-8.03 (1H, m), 8.43 (1H, ddd), 8.57 (1H, t)

### Reference Example 2(42)

### 1-[(3-Nitrophenyl)sulfonyl]-1,2,3,4-tetrahydro-5H-1-benzazepin-5-one

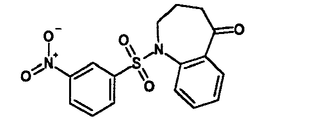

¹H-NMR (CDCl₃) δ 1.96 (2H, quintet), 2.39 (2H, t), 3.94 (2H, t), 7.38-7.41 (1H, m), 7.43-7.48 (1H, m), 7.58 (1H, dt), 7.63 (1H, dd), 7.67 (1H, t), 7.88-7.91 (1H, m), 8.42-8.45 (1H, m), 8.50 (1H, t)

### Reference Example 2(43)

### 5-Methyl-1-[(3-nitrophenyl)sulfonyl]-2,3,4,5-tetrahydro-1H-1-benzazepine

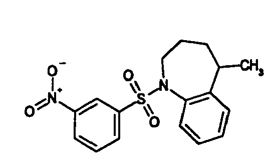

¹H-NMR (CDCl₃) δ 1.21 (3H, d), 1.70-1.77 (2H, m), 1.92-1.94 (1H, m), 2.63-2.65 (1H, m), 3.22-3.24 (1H, m), 4.10-4.13 (2H, m), 7.12-7.32 (4H, m), 7.69 (1H, t), 8.03-8.06 (1H, m), 8.40-8.43 (1H, m), 8.62 (1H, t)

### Reference Example 2(44)

### N-(2-Cyanoethyl)-3-nitro-N-phenylbenzenesulfonamide

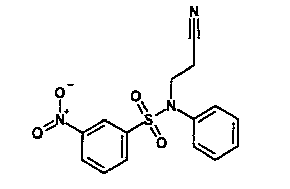

¹H-NMR (DMSO-d₆) δ 2.63 (2H, t), 3.91 (2H, t), 7.10-7.14 (2H, m), 7.39-7.41 (3H, m), 7.88 (1H, t), 7.96 (1H, dt), 8.20 (1H, t), 8.54 (1H, ddd)

### Reference Example 2(45)

### N-(2-Hydroxyethyl)-3-nitro-N-phenylbenzenesulfonamide

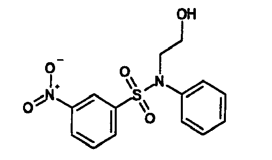

¹H-NMR (CDCl₃) δ 1. 81 (1H, t), 3.70 (2H, q), 3.79 (2H, t), 7.06-7.09 (2H, m), 7.25-7.37 (3H, m), 7.69 (1H, t), 7.91 (1H, ddd), 8.43 (1H, ddd), 8.50 (1H, t)

### Reference Example 2(46)

### Ethyl N-[(3-nitrophenyl)sulfonyl]-N-phenylglycinate

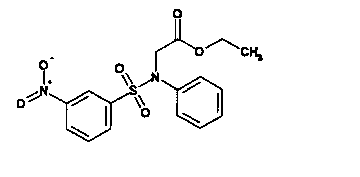

¹H-NMR (CDCl₃) δ 1.24 (3H, t), 4.17 (2H, q), 4.47 (2H, s), 7.21-7.24 (2H, m), 7.30-7.33 (3H, m), 7.64 (1H, t), 7.97 (1H, ddd), 8.40 (1H, ddd), 8.53 (1H, t)

### Reference Example 2(47)

### 4-[(3-Nitrophenyl)sulfonyl]-3,4-dihydroquinoxalin-2(1H)-one

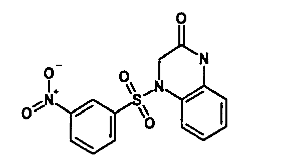

¹H-NMR (CDCl₃) δ 4.41 (2H, s), 6.69 (1H, dd), 7.20-7.33 (2H, m), 7.57 (1H, t), 7.75-7.82 (2H, m), 8.11 (1H, s), 8.23 (1H, t), 8.32-8.36 (1H, m)

### Reference Example 2(48)

### 5-[(3-Nitrophenyl)sulfonyl]-1,3,4,5-tetrahydro-2H-1,5-benzodiazepin-2-one

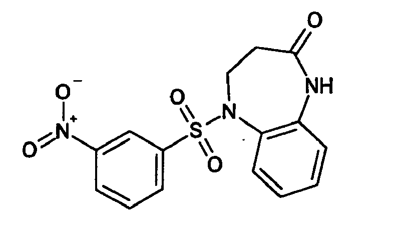

¹H-NMR (DMSO-d₆) δ 2.32 (2H, t), 4.21 (2H, t), 6.93 (1H, d), 7.25 (1H, t), 7.37-7.44 (2H, m), 7.83 (1H, t), 8.01-8.04 (2H, m), 8.52 (1H, dd), 9.14 (1H, s)

### Reference Example 2(49)

### 3-Methyl-4-[(3-nitrophenyl)sulfonyl]-3,4-dihydroquinoxalin-2(1H)-one

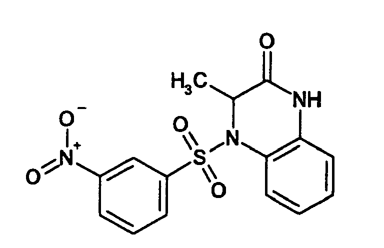

¹H-NMR (DMSO-d₆) δ 1.16 (3H, d), 4.61 (1H, q), 6.76 (1H, dd), 7.17 (1H, dt), 7.31 (1H, dt), 7.58 (1H, d), 7. 7 6 (1H, dt), 7.82 (1H, t), 7.90 (1H, t), 8.53 (1H, dt), 10.26 (1H, s)

### Reference Example 2(50)

### 4-Methyl-5-[(3-nitrophenyl)sulfonyl]-1,3,4,5-tetrahydro-2H-1,5-benzodiazepin-2-one

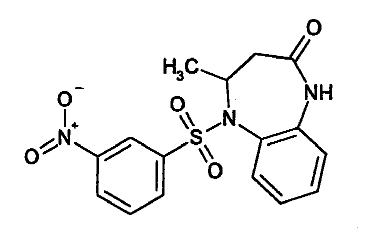

¹H-NMR (DMSO-d₆) δ 1.25 (3H, d), 2.16-2.20 (2H, m), 4.83-4. 86 (1H, m), 6.91 (1H, dd), 7.27 (1H, dt), 7.39-7.45 (2H, m), 7.81 (1H, t), 7.95-7.99 (2H, m), 8.49-8.53 (1H, m), 9.08 (1H, s)

### Reference Example 2(51)

### 4-[(3-Nitrophenyl)sulfonyl]-3,4-dihydro-2H-pyrido[3,2-b][1,4]oxazine

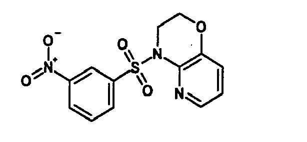

¹H-NMR (CDCl₃) δ 4.16 (2H, t), 4.38 (2H, t), 6.92 (1H, dd), 7.14-7.17 (1H, m), 7.72 (1H, t), 7.86-7.88 (1H, m), 8.40-8.44 (1H, m), 8.48-8.52 (1H, m), 9.04 (1H, t)

### Reference Example 2(52)

### 4-[(3-Nitrophenyl)sulfonyl]-3,4-dihydro-2H-1,4-benzothiazine

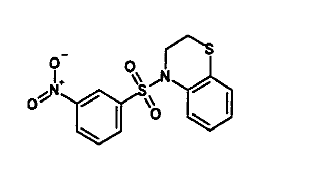

¹H-NMR (CDCl₃) δ 2.95 (2H, t), 4.06 (2H, t), 7.05-7.08 (1H, m), 7.14-7.21 (2H, m), 7.60 (1H, t), 7.67-7.70 (1H, m), 7.79-7.82 (1H, m), 8.38-8.43 (2H, m)

### Reference Example 2(53)

### N-{1-[(3-Nitrophenyl)sulfonyl]-1,2,3,4-tetrahydroquinolin-3-yl}acetamide

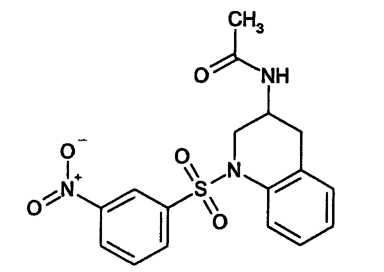

¹H-NMR (CDCl₃) δ 1.97 (3H, s), 2.58 (1H, dd), 2.84 (1H, dd), 3.68 (1H, dd), 4.11 (2H, dd), 5.50 (1H, d), 7.07 (1H, d), 7. 14 (1H, t), 7.26 (1H, dt), 7. 69-7. 75 (2H, m), 8.12-8.16 (1H, m), 8.40-8.44 (1H, m), 8.53 (1H, t)

### Reference Example 2(54)

### Ethyl 4-[(3-nitrophenyl)sulfonyl]-3,4-dihydro-2H-1,4-benzoxazine-2-carboxylate

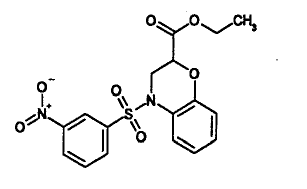

¹H-NMR (CDCl₃) δ 1.31 (3H, t), 3.72 (1H, dd), 4.13 (1H, dd), 4.25 (2H, q), 4.48 (1H, dd), 6.97-7.04 (2H, m), 7.14 (1H, dt), 7.71 (1H, t), 7.77 (1H, dd), 7.99 (1H, d), 8.43-8.46 (1H, m), 8.58 (1H, t)

### Reference Example 2(55)

### 1-Methyl-4-[(3-nitrophenyl)sulfonyl]-1,4,5,6,7,8-hexahydropyrazolo[4,3-b]azepine

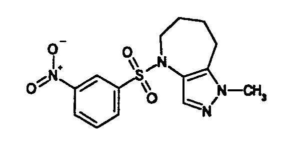

¹H-NMR (CDCl₃) δ 1.52-1.58 (2H, m), 1.71-1.77 (2H, m), 2.30 (2H, t), 3.75 (3H, s), 3.76-3.80 (2H, m), 7.38 (1H, s), 7.67 (1H, t), 7.97-8.00 (1H, m), 8.41 (1H, ddd), 8.55 (1H, t)

### Reference Example 2(56)

### 1,7,7-Trimethyl-4-[(3-nitrophenyl)sulfonyl]-1,4,5,6,7,8-hexahydropyrazolo[4,3-b]azepine

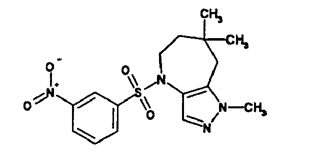

¹H-NMR (CDCl₃) δ 0.84 (6H, s), 1.59-1.64 (2H, m), 2.15 (2H, s), 3.72 (3H, s), 3.74-3.79 (2H, m), 7.29 (1H, s), 7.68 (1H, t), 8.01-8.06 (1H, m), 8.42 (1H, ddd), 8.58 (1H, t)

### Reference Example 2(57)

### 8-Methoxy-1-[(3-nitrophenyl)sulfonyl]-2,3,4,5-tetrahydro-1H-1-benzazepine

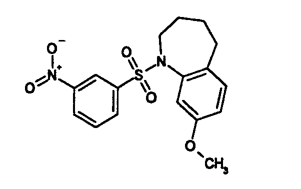

¹H-NMR (CDCl₃) δ 1.50-1.56 (2H, m), 1.82 (2H, quintet), 2.25-2.28 (2H, m), 3.69-3.76 (2H, m), 3.76 (3H, s), 6.75-6.81 (2H, m), 7.02 (1H, d), 7.67 (1H, t), 8.01 (1H, ddd), 8.41 (1H, ddd), 8.61 (1H, t)

### Reference Example 2(58)

### 6,9-Dimethoxy-1-[(3-nitrophenyl)sulfonyl]-2,3,4,5-tetrahydro-1H-1-benzazepine

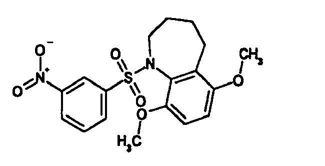

¹H-NMR (CDCl₃) δ 1.24-1.28 (1H, m), 1.73-1.78 (1H, m), 2.01-2.05 (1H, m), 2.26-2.30 (1H, m), 2.72 (1H, t), 2.90-3.00 (1H, m), 3.36 (1H, dd), 3.50 (3H, s), 3.78 (3H, s), 4.00-4.04 (1H, m), 6.66 (1H, d), 6. 83 (1H, d), 7.72 (1H, t), 8.27-8.30 (1H, m), 8.41-8.45 (1H, m), 8.91 (1H, t)

### Reference Example 2(59)

### 6-Methoxy-1-[(3-nitrophenyl)sulfonyl]-2,3,4,5-tetrahydro-1H-1-benzazepine

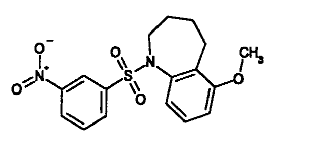

¹H-NMR (CDCl₃) δ 1.50-1.55 (2H, m), 1.82-1.88 (2H, m), 2.54-2.56 (2H, m), 3.76-3.79 (2H, m), 3.79 (3H, s), 6.77-6.85 (2H, m), 7.12 (1H, t), 7.68 (1H, t), 8.04-8.07 (1H, m), 8.39-8.43 (1H, m), 8.62 (1H, t)

### Reference Example 2(60)

### 6,8-Dimethyl-1-[(3-nitrophenyl)sulfonyl]-2,3,4,5-tetrahydro-1H-1-benzazepine

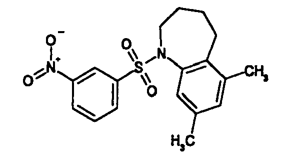

¹H-NMR (CDCl₃) δ 1.55-2.24 (8H, m), 2.23 (3H, s), 2.24 (3H, s), 6.79 (1H, s), 6.94 (1H, s), 7.69 (1H, t), 8.05-8.08 (1H, m), 8.41-8.44 (1H, m), 8.65 (1H, t)

### Reference Example 2(61)

### Methyl 1-[(3-nitrophenyl)sulfonyl]-1,2,3,4-tetrahydroquinoline-6-carboxylate

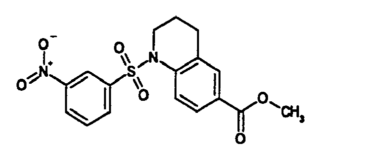

¹H-NMR (CDCl₃) δ 1.77 (2H, quintet), 2.59 (2H, t), 3.90 (3H, s), 3.92 (2H, t), 7.64 (1H, t), 7.73 (1H, s), 7.87-7.91 (3H, m), 8.39-8.42 (1H, m), 8.56 (1H, t)

### Reference Example 2(62)

### 8-Methyl-1-[(3-nitrophenyl)sulfonyl]-2,3,4,5-tetrahydro-1H-1-benzazepine

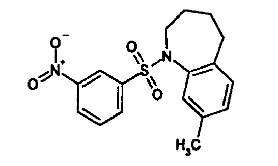

¹H-NMR (CDCl₃) δ 1.53-1.55 (2H, m), 1.80 (2H, quintet), 2.28-2.30 (2H, m), 2.30 (3H, s), 3.73-7.77 (2H, m), 6.98-7.07 (3H, m), 7.65 (1H, t), 7.96-7.99 (1H, m), 8.38-8.41 (1H, m), 8.58 (1H, t)

### Reference Example 2(63)

### 7,8-Dimethoxy-1-[(3-nitrophenyl)sulfonyl]-2,3,4,5-tetrahydro-1H-1-benzazepine

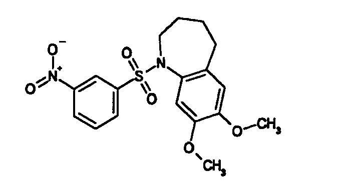

¹H-NMR (CDCl₃) δ 1. 50-1. 55 (2H, m), 1.79 (2H, quintet), 2.14-2.18 (2H, m), 3.81 (3H, s), 3.83-8.85 (2H, m), 3.86 (3H, s), 6.57 (1H, s), 6.84 (1H, s), 7.65 (1H, t), 7.95-7.98 (1H, m), 8.39-8.42 (1H, m), 8.60 (1H, t)

### Reference Example 2(64)

### 6-[(3-Nitrophenyl)sulfonyl]-2,3,7,8,9,10-hexahydro-6H-[1,4]dioxino[2,3-h][1]benzazepine

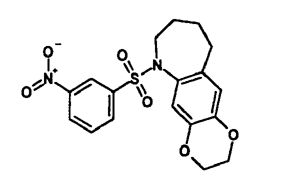

¹H-NMR (CDCl₃) δ 1.50-1.55 (2H, m), 1.80 (2H, quintet), 2.22-2.24 (2H, m), 3.68-3.72 (2H, m), 4.22-4.25 (4H, m), 6.61 (1H, s), 6.73 (1H, s), 7.68 (1H, t), 8.01-8.04 (1H, m), 8.39-8.43 (1H, m), 8.61 (1H, t)

### Reference Example 2(65)

### 1-[(3-Nitrophenyl)sulfonyl]-8-phenyl-2,3,4,5-tetrahydro-1H-1-benzazepine

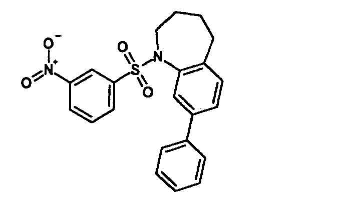

¹H-NMR (CDCl₃) δ 1.58-1.62 (2H, m), 1.86 (2H, quintet), 2.39-2.44 (2H, m), 3.76-3.80 (2H, m), 7.20 (1H, d), 7.34-7.54 (7H, m), 7.68 (1H, t), 8.02-8.06 (1H, m), 8.42 (1H, ddd), 8.67 (1H, t)

### Reference Example 2(66)

### 8-Cyclohexyl-1-[(3-nitrophenyl)sulfonyl]-2,3,4,5-tetrahydro-1H-1-benzazepine

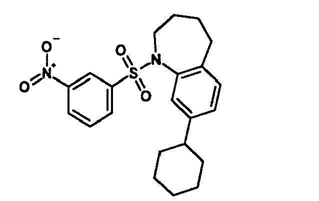

¹H-NMR (CDCl₃) δ 1.17-1.42 (5H, m), 1.55-1.57 (2H, m), 1.71-1.88 (7H, m), 2.35-2.43 (3H, m), 3.70-3.74 (2H, m), 6.99-7.06 (3H, m), 7.66 (1H, t), 7.98-8.01 (1H, m), 8.38-8.41 (1H, m), 8.57 (1H, t)

### Reference Example 2(67)

### 8-tert-Butyl-1-[(3-nitrophenyl)sulfonyl]-2,3,4,5-tetrahydro-1H-1-benzazepine

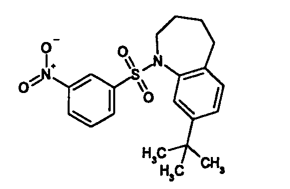

¹H-NMR (CDCl₃) δ 1.22 (9H, s), 1.57-1.61 (2H, m), 1.88 (2H, quintet), 2.43-2.47 (2H, m), 3.73-3.77 (2H, m), 7.05-7.08 (2H, m), 7.22 (1H, dd), 7.68 (1H, t), 8.01-8.05 (1H, m), 8.41 (1H, ddd), 8.60 (1H, t)

### Reference Example 2(68)

### 1-[(3-Nitrophenyl)sulfonyl]-8-phenoxy-2,3,4,5-tetrahydro-1H-1-benzazepine

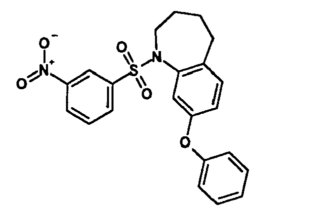

¹H-NMR (CDCl₃) δ 1.58-1.62 (2H, m), 1.89 (2H, quintet), 2.44-2.50 (2H, m), 3.71-3.75 (2H, m), 6.69 (1H, d), 6.88 (1H, dd), 6.94-6.99 (2H, m), 7.08-7.16 (2H, m), 7.29-7. 37 (2H, m), 7.59 (1H, t), 7.96-8.01 (1H, m), 8.33-8.39 (1H, m), 8.57 (1H, t)

### Reference Example 2(69)

### 8-Fluoro-1-[(3-nitrophenyl)sulfonyl]-2,3,4,5-tetrahydro-1H-1-benzazepine

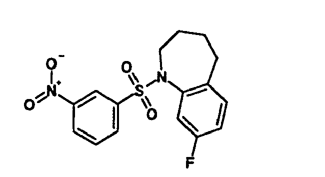

¹H-NMR (CDCl₃) δ 1.54-1.57 (2H, m), 1.83 (2H, quintet), 2.30-2.35 (2H, m), 3.74-3.78 (2H, m), 6.89-7.13 (3H, m), 7.69 (1H, t), 7.99-8.04 (1H, m), 8.43 (1H, ddd), 8.60 (1H, t)

### Reference Example 2(70)

### 8-Bromo-1-[(3-nitrophenyl)sulfonyl]-2,3,4,5-tetrahydro-1H-1-benzazepine

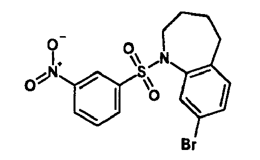

¹H-NMR (CDCl₃) δ 1.54-1.56 (2H, m), 1.83 (2H, quintet), 2.32-2.35 (2H, m), 3.70-3.74 (2H, m), 7.02 (1H, d), 7.36 (1H, dd), 7.38 (1H, d), 7.70 (1H, t), 8.00-8.03 (1H, m), 8.42-8.46 (1H, m), 8.62 (1H, t)

### Reference Example 2(71)

### 1-[(5-Chloro-4-nitro-2-thienyl)sulfonyl]-2,3,4,5-tetrahydro-1H-1-benzazepine

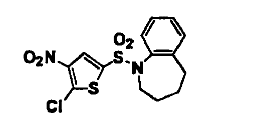

¹H-NMR (CDCl₃) δ 1.66 (2H, br s), 1.88-1.96 (2H, m), 2.59 (2H, t), 3.71 (2H, br s), 7.18-7.30 (4H, m), 7.88 (1H, s)

### Reference Example 2(72)

### 4-[(5-Chloro-4-nitro-2-thienyl)sulfonyl]-5,6,7,8-tetrahydro-4H-thieno[3,2-b]azepine

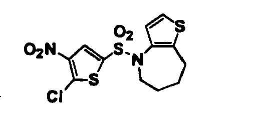

¹H-NMR (CDCl₃) δ 1.58-1.66 (2H, m), 1.85-1.93 (2H, m), 2.52 (2H, t), 3.77 (2H, br s), 6.99 (1H, d), 7.03 (1H, d), 7.84 (1H, s)

### Reference Example 2(73)

### 5-Chloro-N-ethyl-4-nitro-N-phenylthiophene-2-sulfonamide

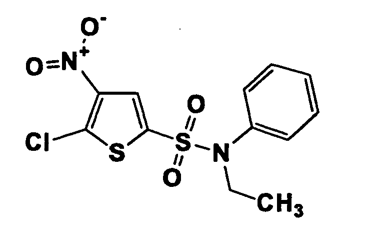

¹H-NMR (CDCl₃) δ 1.16 (3H, t), 3.71 (2H, q), 7.14-7.18 (2H, m), 7.39-7.43 (3H, m), 7.78 (1H, s)

### Reference Example 2(74)

### 1-[(4-Chloro-3-nitrophenyl)sulfonyl]-7-(trifluoromethyl)-1,2,3,4-tetrahydroquinoline

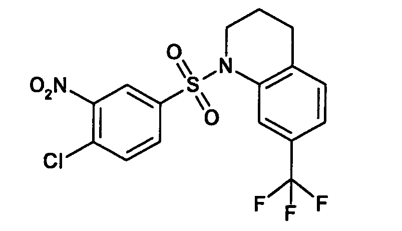

¹H-NMR (CDCl₃) δ 1.77 (2H, quintet), 2.60 (2H, t), 3.87 (2H, t), 7. 18 (1H, d), 7.37 (1H, d), 7. 61 (1H, d), 7. 66 (1H, dd), 8.06 (1H, s), 8.13 (1H, d)

### Reference Example 2(75)

### 4-[(4-Chloro-3-nitrophenyl)sulfonyl]-5,6,7,8-tetrahydro-4H-thieno[3,2-b]azepine

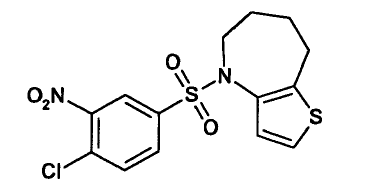

¹H-NMR (CDCl₃) δ 1.51-1.55 (2H, m), 1.74-1.78 (2H, m), 2.26-2.30 (2H, m), 3.74-3.78 (2H, m), 6.97 (1H, d), 7.01 (1H, d), 7.62 (1H, d), 7.71 (1H, dd), 8.11 (1H, d)

### Reference Example 2(76)

### 1-[(4-Chloro-3-nitrophenyl)sulfonyl]-1,2,3,5-tetrahydro-4,1-benzothiazepine

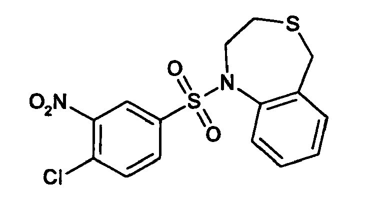

¹H-NMR (CDCl₃) δ 3.00-3.68 (6H, m), 6.96 (1H, d), 7.16-7.31 (3H, m), 7.72 (1H, d), 7.89 (1H, dd), 8.24 (1H, d)

### Reference Example 2(77)

### 1-[(4-Chloro-3-nitrophenyl)sulfonyl]-6-fluoro-1,2,3,4-tetrahydroquinoline

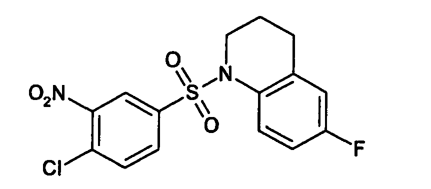

¹H-NMR (CDCl₃) δ 1.69 (2H, quintet), 2.46 (2H, t), 3.83 (2H, t), 6.77 (1H, dd), 6.95 (1H, dt), 7.60 (2H, d), 7.74 (1H, dd), 8.09 (1H, t)

### Reference Example 2(78)

### 1-[(4-Chloro-3-nitrophenyl)sulfonyl]-5-methyl-1,2,3,4-tetrahydroquinoline

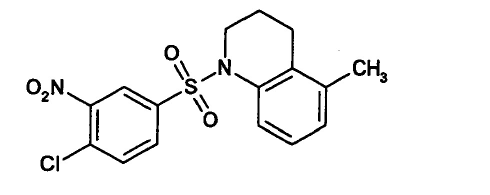

¹H-NMR (CDCl₃) δ 1.73 (2H, quintet), 2.17 (3H, s), 2.42 (2H, t), 3.85 (2H, t), 7.03 (1H, d), 7.14 (1H, t), 7.57 (1H, d), 7.59 (1H, d), 7.67 (1H, dd), 8.04 (1H, d)

### Reference Example 3

### N-Ethyl-4-methoxy-3-nitro-N-phenylbenzenesulfonamide

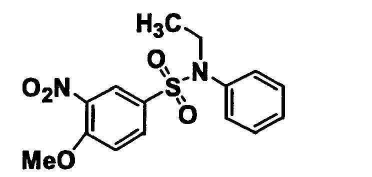

To chlorosulfonic acid (45.0 g), 1-methoxy-2-nitrobenzene (13.4 g) was added dropwise with ice cooling, and the mixture was stirred for 10 minutes as it was and further stirred for 1 hour at room temperature. The reaction solution was poured onto crushed ice, and extracted twice with diethyl ether. The collected organic layers were dried over anhydrous magnesium sulfate. The solvent was distilled off under reduced pressure to give crude 4-methoxy-3-nitrobenzensulfonyl chloride as an oily matter.

While stirring a solution of N-ethyl aniline (10.6 g) and sodium hydrogen carbonate (14.7 g) in THF (50 ml) and water (15 ml), a solution of the above-obtained oily matter in THF (50 ml) was added thereto at room temperature, and the mixture was stirred overnight as it was. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (3 : 1 to 1 : 1)), and crystallized from diisopropyl ether/diethyl ether to give the desired product (13.4 g) as crystals.

¹H-NMR (CDCl₃) δ 1.11 (3H, t), 3.63 (2H, q), 4.03 (3H, s), 7.04-7.07 (2H, m), 7.10 (1H, d), 7.31-7.36 (3H, m), 7.70 (1H, dd), 8.06 (1H, d).

### Reference Example 4

### 2-Chloro-5-(3,4-dihydro-1(2H)-quinolinylcarbonyl)aniline

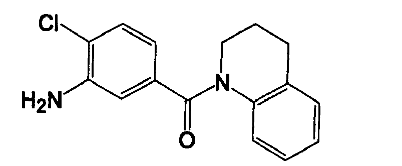

To a solution of 3-amino-4-chlorobenzoic acid (5.03 g) and 1,2,3,4-tetrahydroquinoline (4.69 g) in dichloromethane (110 ml), a solution of 1-(3-dimethylaminopropyl)-3-ethylcarbodiimide hydrochloride (6.78 g) and N-methylimidazole (2.91 g) in dichloromethane (37 ml) was added, and the mixture was stirred for 16 hours at room temperature. Then, the reaction mixture was poured into a 5% sodium bicarbonate solution, and extracted with dichloromethane. The organic layer was washed with water and dried over anhydrous magnesium sulfate, and the solvent was distilled off under reduced pressure. Then, the crystals, which were crystallized from toluene, were collected by filtration to give the desired product (8.41 g).
¹H-NMR (CDCl₃) δ 1.99-2.08 (2H, m), 2.83 (2H, t), 3 .88 (2H, t), 4.07 (2H, br s), 6.54 (1H, dd), 6.76-7. 15 (4H, m), 6. 89 (1H, d), 7.08 (1H, d)
MS (ESI+, m/e) 287 (M+1)

### Reference Examples 4(1) to 4(8)

In the same manner as in Reference Example 4, the corresponding carboxylic acids (commercially available or conventional) were reacted with 1,2,3,4-tetrahydroquinoline to obtain the following compounds.

### Reference Example 4(1)

### 3-(3,4-Dihydro-1(2H)-quinolinylcarbonyl)-2-methylaniline

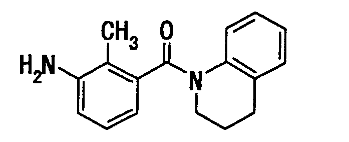

¹H-NMR (CDCl₃) δ 1.85-2.22 (5H, br s), 2.83 (2H, t), 3.51-4.08 (2H, br s), 6.50-7.21 (7H, m)
MS (ESI+, m/e) 267 (M+1)

### Reference Example 4 (2)

### 2-Chloro-3-(3,4-dihydro-1(2H)-quinolinylcarbonyl)aniline

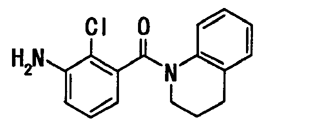

¹H-NMR (CDCl₃) δ 1.82-2.23 (2H, br s), 2.70-2.95 (2H, br s), 3.40-3.65 (1H, br s), 3.78-4.23 (1H, br s), 6.46-7.25 (6H, m), 8.04-8.24 (1H, br s)
MS (ESI+, m/e) 287 (M+1)

### Reference Example 4(3)

### 1-(4-Fluoro-3-nitrobenzoyl)-1,2,3,4-tetrahydroquinoline

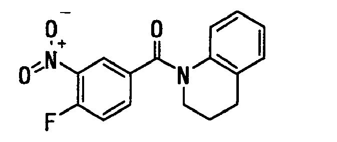

¹H-NMR (CDCl₃) δ 2.09 (2H, quintet), 2.87 (2H, t), 3.94 (2H, t), 6.55 (1H, d), 6.91 (1H, dt), 7.07 (1H, dt), 7.12-7.24 (2H, m), 7.56 (1H, ddd), 8.08 (1H, dd)
MS (ESI+, m/e) 301 (M+1)

### Reference Example 4(4)

### 1-(2-Fluoro-5-nitrobenzoyl)-1,2,3,4-tetrahydroquinoline

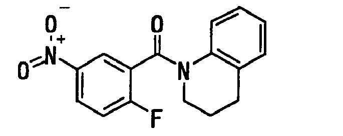

¹H-NMR (CDCl₃) δ 2.00-2 . 17 (2H, m), 2 . 85 (2H, t), 3 . 77-4.13 (2H, br s), 6.35-6.58 (1H, br s), 6.74-7.13 (3H, m), 7.18 (1H, d), 8.15-8.29 (1H, m), 8.44 (1H, dd)
MS (ESI+, m/e) 301 (M+1)

### Reference Example 4(5)

### (2,6-Dichloro-4-(3,4-dihydroquinolin-1(2H)-ylcarbonyl)phenyl)amine

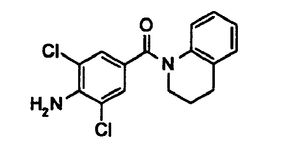

¹H-NMR (CDCl₃) δ 2.04 (2H, quintet), 2.83 (2H, t), 3.88 (2H, t), 6.72 (1H, d), 6.94 (1H, dt), 7.03 (1H, dt), 7.17 (1H, d), 7.21 (2H, s)
MS (ESI+, m/e) 321 (M+1)

### Reference Example 4(6)

### 1-[(5-Nitro-2-thienyl)carbonyl]-1,2,3,4-tetrahydroquinoline

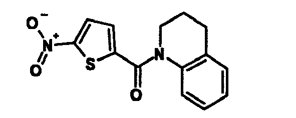

¹H-NMR (DMSO-d₆) δ 2.06 (2H, dt), 2.82 (2H, t), 3.93 (2H, t), 6.81 (1H, d), 6.91 (1H, d), 7.04 (1H, t), 7.17 (1H, t), 7.25 (1H, d), 7.63 (1H, d)

### Reference Example 4(7)

### 1-((5-Nitro-1H-pyrazol-3-yl)carbonyl)-1,2,3,4-tetrahydroquinoline

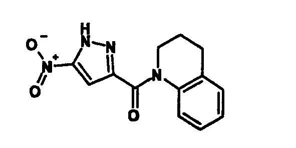

¹H-NMR (CDCl₃) δ 2. 10 (2H, dt), 2.82 (2H, t), 4.01 (2H, t), 6.20 (1H, br s), 7.08-7.34 (4H, m), 12.79 (1H, br s)

### Reference Example 4 (8)

### 1-((5-Nitro-3-thienyl)carbonyl)-1,2,3,4-tetrahydroquinoline

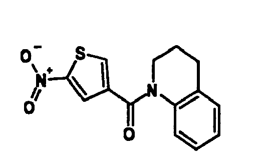

¹H-NMR (DMSO-d₆) δ 2.07 (2H, dt), 2.83 (2H, t), 3.92 (2H, t), 6. 72 (1H, d), 6.99 (1H, td), 7.12 (1H, td), 7.23 (1H, d), 7.61 (2H, dd)

### Reference Example 5

### 3-Amino-4-chloro-N-methyl-N-phenylbenzenesulfonamide

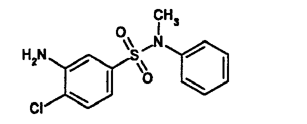

A mixture of 4-chloro-N-methyl-3-nitro-N-phenylbenzenesulfonamide (3.43 g), nickel bromide (II) (115 mg), methanol (50 ml) and THF (50 ml) was ice cooled, and sodium borohydride (1.19 g) was gradually added thereto. The mixture was agitated for 30 minutes at 0°C and then for 30 minutes at room temperature, after which the reaction mixture was poured into a saturated sodium bicarbonate solution, and extracted with ethyl acetate. The organic layer was sequentially washed with water and saturated brine, dried over anhydrous magnesium sulfate, and then the solvent was distilled off under reduced pressure. The residue was subjected to silica gel column chromatography, and the fraction eluted with ethyl acetate/hexane (1 : 3) was concentrated under reduced pressure. Thus obtained crystals were collected by filtration to give the desired product (2.72 g).

¹H-NMR (CDCl₃) δ 3.17 (3H, s), 4.23 (2H, s), 6.82 (1H, dd), 6.90 (1H, d), 7.10-7.15 (2H, m), 7.26-7.32 (4H, m)
MS (ESI+, m/e) 297 (M+1)

### Reference Examples 5(1) to 5(107)

In the same manner as in Reference Example 5, the corresponding nitro compound (synthesized in Reference Examples) was reduced to obtain the following compounds.

### Reference Example 5(1)

### 3-Amino-4-chloro-N-isopropyl-N-phenylbenzenesulfonamide

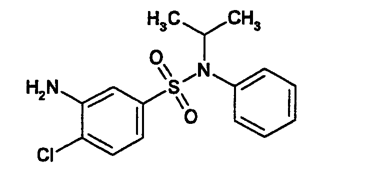

¹H-NMR (CDCl₃) δ 1.05 (6H, d), 4.23 (2H, s), 4.58 (1H, sevenplet), 7.01-7.11 (4H, m), 7.30-7.39 (4H, m)
MS (ESI+, m/e) 325 (M+1)

### Reference Example 5(2)

### 3-Amino-N-(1,3-benzodioxol-5-yl)-4-chloro-N-ethylbenzenesulfonamide

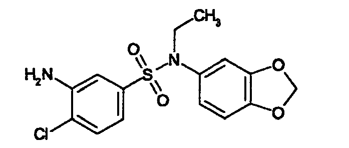

¹H-NMR (CDCl₃) δ 1.07 (3H, t), 3.53 (2H, q), 4.26 (2H, s), 5.99 (2H, s), 6.48 (1H, dd), 6.59 (1H, d), 6.72 (1H, d), 6.93 (1H, dd), 7.02 (1H, d), 7.33 (1H, d)
MS (ESI+, m/e) 355 (M+1)

### Reference Example 5(3)

### 3-Amino-4-chloro-N-(2-cyanoethyl)-N-phenylbenzenesulfonamide

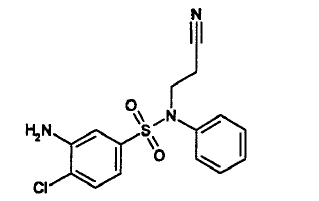

¹H-NMR (CDCl₃) δ 2.61 (2H, t), 3. 84 (2H, t), 4.29 (2H, s), 6.87 (1H, dd), 6.97 (1H, d), 7.07-7.12 (2H, m), 7.31-7.38 (4H, m)
MS (ESI+, m/e) 336 (M+1)

### Reference Example 5(4)

### 2-Chloro-5-(3,4-dihydro-2(1H)-isoquinolinylsulfonyl)aniline

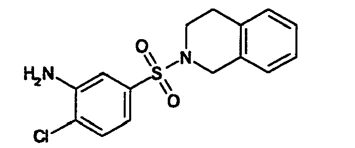

¹H-NMR (CDCl₃) δ 2 . 93 (2H, t), 3 . 37 (2H, t), 4 . 27 (2H, s), 4.33 (2H, s), 7.00-7.28 (6H, m), 7.37 (1H, d)
MS (ESI+, m/e) 323 (M+1)

### Reference Example 5(5)

### 2-Chloro-5-((4-ethyl-1-piperazinyl)sulfonyl)aniline

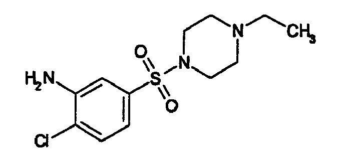

¹H-NMR (CDCl₃) δ 1.03 (3H, t), 2.40 (2H, q), 2.51 (4H, t), 3. 04 (4H, t), 4.31 (2H, s), 7.02 (1H, dd), 7.11 (1H, d), 8.37 (1H, d)
MS (ESI+, m/e) 304 (M+1)

### Reference Example 5(6)

### 3-Amino-4-chloro-N,N-diethylbenzenesulfonamide

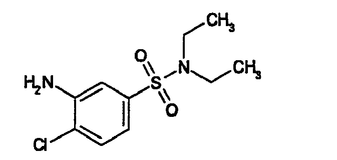

¹H-NMR (CDCl₃) δ 1.14 (6H, t), 3.23 (4H, q), 4.30 (2H, s), 7.08 (1H, dd), 7.21 (1H, d), 7.34 (1H, d)
MS (ESI+, m/e) 263 (M+1)

### Reference Example 5(7)

### 3-Amino-N-benzyl-4-chloro-N-ethylbenzenesulfonamide

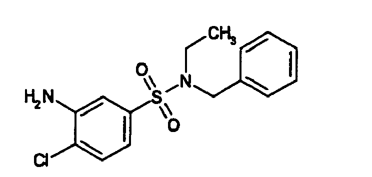

¹H-NMR (CDCl₃) δ 0.94 (3H, t), 3.18 (2H, q), 4.30 (2H, s), 4.33 (2H, s), 7.12 (1H, dd), 7.22 (1H, d), 7.27-7.32 (5H, m), 7.37 (1H, d)
MS (ESI+, m/e) 325 (M+1)

### Reference Example 5(8)

### 2-Chloro-5-((6-methyl-3,4-dihydro-1(2H)-quinolinyl) sulfonyl) aniline

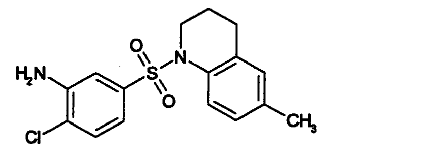

¹H-NMR (CDCl₃) δ 1.58-1.70 (2H, m), 2.29 (3H, s), 2.44 (2H, t), 3.76 (2H, t), 4.19 (2H, s), 6.82-6.87 (2H, m), 6.96-7.00 (2H, m), 7.24 (1H, d), 7.63 (1H, d)
MS (ESI+, m/e) 337 (M+1)

### Reference Example 5(9)

### 2-Chloro-5-((2-methyl-3,4-dihydro-1(2H)-quinolinyl)sulfonyl)aniline

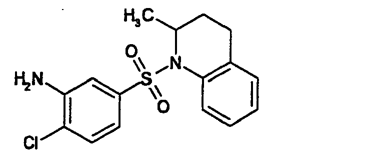

¹H-NMR (CDCl₃) δ 1.28 (3H, d), 1.30-1.40 (1H, m), 1.82-1.93 (2H, m), 2.73-2.46 (1H, m), 4.16 (2H, s), 4.33 (1H, q), 6.76 (1H, dd), 6.83 (1H, d), 7.00 (1H, d), 7.12 (1H, dt), 7.20-7.26 (2H, m), 7.71 (1H, d)
MS (ESI+, m/e) 337 (M+1)

### Reference Example 5(10)

### 2-Chloro-5-((2-methyl-2,3-dihydro-1H-indol-1-yl)sulfonyl)aniline

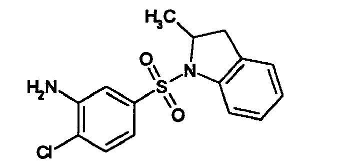

¹H-NMR (CDCl₃) δ1.42 (3H, d), 2.46 (1H, dd), 2.98 (1H, dd), 4.19 (2H, s), 4.30-4.37 (1H, m), 6.93 (1H, dd), 7.01-7.09 (3H, m), 7.18-7.26 (2H, m), 7.63 (1H, d)
MS (ESI+, m/e) 323 (M+1)

### Reference Example 5(11)

### 2-Chloro-5-((5-methyl-2,3-dihydro-1H-indol-1-yl) sulfonyl) aniline

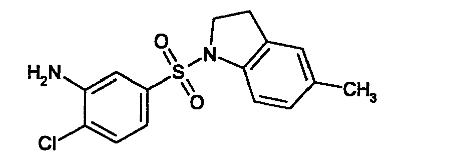

¹H-NMR (CDCl₃) δ 2.27 (3H, s), 2.86 (2H, t), 3.89 (2H, t), 4.23 (2H, s), 6.91-7.01 (2H, m), 7.03 (1H, dd), 7.15 (1H, d), 7.26 (1H, d), 7.48 (1H, d)
MS (ESI+, m/e) 323 (M+1)

### Reference Example 5(12)

### 2-Chloro-5-(2,3-dihydro-4H-1,4-benzoxazin-4-ylsulfonyl)aniline

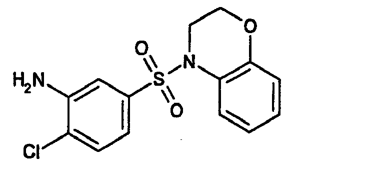

¹H-NMR (CDCl₃) δ 3.76-3.87 (4H, m), 4.24 (2H, s), 6.80-7.12 (5H, m), 7.29 (1H, d), 7.81 (1H, dd)
MS (ESI+, m/e) 325 (M+1)

### Reference Example 5(13)

### 2-Chloro-5-(2,3,4,5-tetrahydro-1H-1-benzazepin-1-ylsulfonyl)aniline

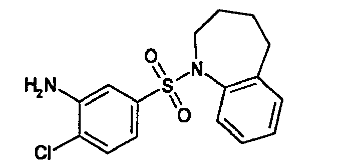

¹H-NMR (CDCl₃) δ 1.54-1.58 (2H, m), 1.75-1.84 (2H, m), 2.43-2.48 (2H, m), 3.67-3.71 (2H, m), 4.23 (2H, s), 7.02 (1H, dd), 7.09-7.27 (5H, m), 7.31 (1H, d)
MS (ESI+, m/e) 337 (M+1)

### Reference Example 5(14)

### 3-Amino-4-chloro-N-methyl-N-4-pyridinylbenzenesulfonamide

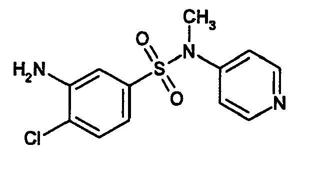

¹H-NMR (CDCl₃) δ 3.23 (3H, s), 4.31 (2H, s), 6.84 (1H, dd), 6.92 (1H, d), 7.18 (2H, d), 7.31 (1H, d), 8.53 (2H, d)
MS (ESI+, m/e) 298 (M+1)

### Reference Example 5(15)

### 2-Chloro-5-(3,4-dihydro-1,5-naphthylidin-1(2H)-ylsulfonyl)aniline

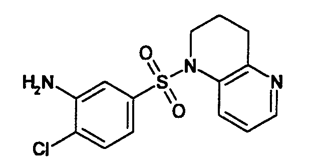

¹H-NMR (CDCl₃) δ 1.73 (2H, quintet), 2.75 (2H, t), 3.79 (2H, t), 4.27 (2H, s), 6.84 (1H, dd), 6.98 (1H, d), 7.14 (1H, dd), 7.28 (1H, d), 8.11 (1H, dd), 8.31 (1H, dd)
MS (ESI+, m/e) 324 (M+1)

### Reference Example 5(16)

### 2-Chloro-5-(3,4-dihydro-1,5-benzoxazepin-5(2H)-ylsulfonyl)aniline

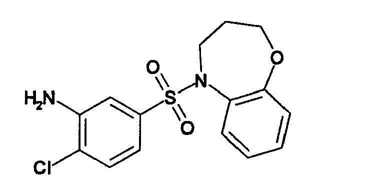

¹H-NMR (CDCl₃) δ 1.88 (2H, quintet), 3.85-3.90 (4H, m), 4.19 (2H, s), 6.93-7.11 (4H, m), 7.18-7.23 (1H, m), 7.28 (1H, d), 7.46 (1H, dd)
MS (ESI+, m/e) 339 (M+1)

### Reference Example 5(17)

### 2-Chloro-5-(3,4,5,6-tetrahydro-1-benzazocin-1(2H)-ylsulfonyl)aniline

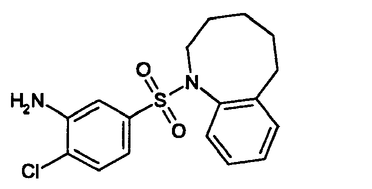

¹H-NMR (CDCl₃) δ 1.26-3.86 (10H, m), 4.28 (2H, s), 6.65 (1H, d), 7.05-7.26 (5H, m), 7.38 (1H, d)
MS (ESI+, m/e) 351 (M+1)

### Reference Example 5(18)

### 4-Chloro-3-(3,4-dihydro-1(2H)-quinolinylsulfonyl)aniline

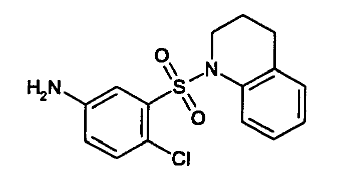

¹H-NMR (CDCl₃) δ 1. 95 (2H, quintet), 2.76 (2H, t), 3.87-3.93 (4H, m), 6.73 (1H, dd), 6.95-7.11 (3H, m), 7.20 (1H, d), 7.33-7.38 (1H, m), 7.43 (1H, d)
MS (ESI+, m/e) 323 (M+1)

### Reference Example 5(19)

### 3-(3,4-Dihydro-1(2H)-quinolinylsulfonyl)-4-methylaniline

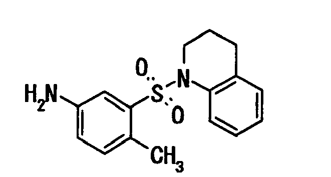

¹H-NMR (CDCl₃) δ 1.74-1.89 (2H, m), 2.23 (3H, s), 2.68 (2H, t), 3.76 (2H, t), 6.74 (1H, dd), 6.97-7.16 (4H, m), 7.25 (1H, d), 7.45 (1H, d)
MS (ESI+, m/e) 303 (M+1)

### Reference Example 5(20)

### 3-Amino-4-chloro-N-cyclohexyl-N-methylbenzenesulfonamide

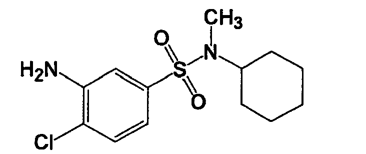

¹H-NMR (CDCl₃) δ 0.97-1.74 (10H, m), 2.74 (3H, s), 3.72 (1H, m), 4.27 (2H, br s), 7.07 (1H, dd), 7.18 (1H, d), 7.33 (1H, d)
MS (ESI+, m/e) 303 (M+1)

### Reference Example 5(21)

### 2-Chloro-5-(2,3-dihydro-1H-indol-1-ylsulfonyl)aniline

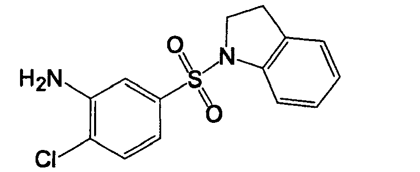

¹H-NMR (CDCl₃) δ 2.93 (2H, t), 3.92 (2H, t), 4.24 (2H, br s), 6.95-7.28 (4H, m), 7.05 (1H, dd), 7.15 (1H, d), 7.61 (1H, d)
MS (ESI+, m/e) 309 (M+1)

### Reference Example 5(22)

### 3-Amino-4-chloro-N-ethyl-N-phenylbenzenesulfonamide

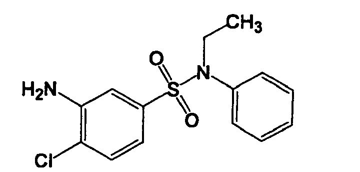

¹H-NMR (CDCl₃) δ 1.06 (3H, t), 3.59 (2H, q), 4.21 (2H, br s), 6.88 (1H, dd), 8.94 (1H, d), 7.05-7.09 (2H, m), 7.28-7.35 (4H, m)
MS (ESI+, m/e) 311 (M+1)

### Reference Example 5(23)

### 3-Amino-4-chloro-N-(4-chlorophenyl)-N-ethylbenzenesulfonamide

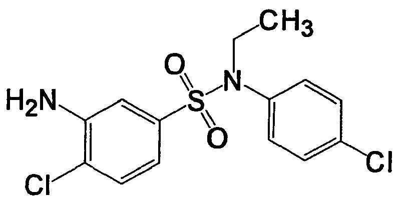

¹H-NMR (CDCl₃) δ 1.05 (3H, t), 3.56 (2H, q), 4.24 (2H, br s), 6.86 (1H, dd), 6.93 (1H, d), 7.00 (2H, d), 7.29 (2H, d), 7.31 (1H, d)
MS (ESI+, m/e) 345 (M+1)

### Reference Example 5(24)

### 5-((4-Benzylpiperidin-1-yl)sulfonyl)-2-chloroaniline

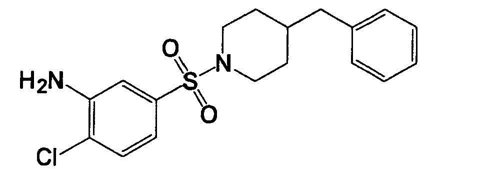

¹H-NMR (CDCl₃) δ 1.36 (2H, td), 1.39-1.55 (1H, m), 1.68 (2H, dd), 2.23 (2H, td), 2.51 (2H, d), 3.72 (2H, d), 4.28 (2H, br s), 7.00 (1H, dd), 7.04-7.10 (2H, m), 7. 10 (1H, d), 7. 15-7.30 (2H, m), 7.35 (1H, d)
MS (ESI+, m/e) 365 (M+1)

### Reference Example 5(25)

### 3-Amino-4-chloro-N-(2-(dimethylamino)-1-phenylethyl)-N-methylbenzenesulfonamide

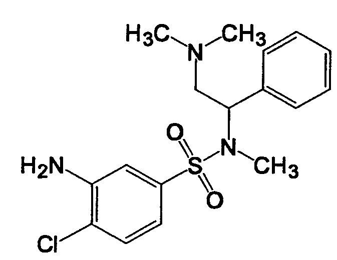

¹H-NMR (CDCl₃) δ 2.24 (6H, s), 2.65 (3H, s), 2. 26-2. 76 (2H, m), 4.19 (2H, br s), 5.25 (1H, t), 7.10 (1H, dd), 7.16 (1H, d), 7.17-7.34 (5H, m), 7.28 (1H, d)
MS (ESI+, m/e) 368 (M+1)

### Reference Example 5(26)

### 3-Amino-4-chloro-N-methyl-N-2-pyridinylbenzenesulfonamide

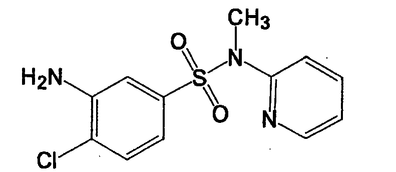

¹H-NMR (CDCl₃) δ 3.27 (3H, s), 4.23 (2H, br s), 6.82 (1H, dd), 6.98 (1H, d), 7.12 (1H, ddd, 5.0), 7.24-7.27 (1H, m), 7.63-7.72 (2H, m), 8.28 (1H, dd)
MS (ESI+, m/e) 298 (M+1)

### Reference Example 5(27)

### 3-Amino-N-butyl-4-chloro-N-phenylbenzenesulfonamide

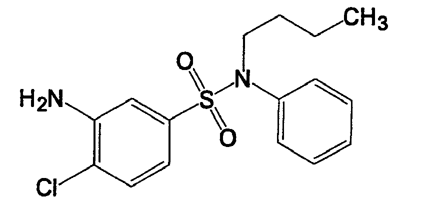

¹H-NMR (CDCl₃) δ 0.86 (3H, t), 1.28-1.39 (4H, m), 3.51 (2H, t), 4.21 (2H, br s), 6.86 (1H, dd), 6.93 (1H, d), 7.05-7.10 (2H, m), 7.28-7.35 (4H, m)
MS (ESI+, m/e) 339 (M+1)

### Reference Example 5(28)

### 2-Chloro-5-(3,4-dihydro-1(2H)-quinolinylsulfonyl)aniline

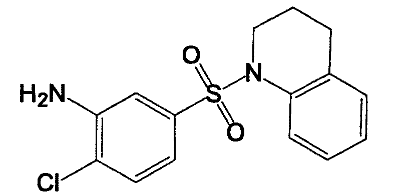

¹H-NMR (CDCl₃) δ 1.63-1.71 (2H, m), 2.48 (2H, t), 3.76-3.80 (2H, m), 4.19 (2H, br s), 6.84 (1H, dd), 6.95 (1H, d), 7.00-7.22 (4H, m), 7.74 (1H, d)
MS (ESI+, m/e) 323 (M+1)

### Reference Example 5(30)

### 3-Amino-N-ethyl-4-methyl-N-phenylbenzenesulfonamide

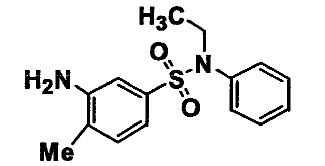

¹H-NMR (CDCl₃) δ 1.06 (3H, t), 2.20 (3H, s), 3.59 (2H, q), 3.73 (2H, br s), 6.85 (1H, d), 6.90 (1H, dd), 7.04-7.10 (3H, m), 7.24-7.34 (3H, m)

### Reference Example 5(31)

### 3-Amino-N-ethyl-4-methoxy-N-phenylbenzenesulfonamide

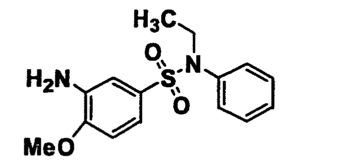

¹H-NMR (CDCl₃) δ 1.05 (3H, t), 3.58 (2H, q), 3.90 (5H, s), 6.76 (1H, d), 6.89 (1H, d), 6.98 (1H, dd), 7.05-7.10 (2H, m), 7.26-7.33 (3H, m)

### Reference Example 5(32)

### 3-Amino-N-ethyl-N-phenylbenzenesulfonamide

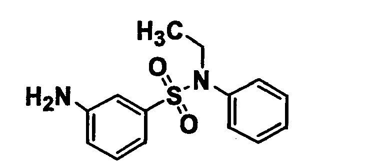

¹H-NMR (CDCl₃) δ 1.07 (3H, t), 3.61 (2H, q), 3.81 (1H, br s), 6.80-6.83 (1H, m), 6.86-6.86 (1H, m), 6.94-6.97 (1H, m), 7.05-7.09 (2H, m), 7.21 (1H, t), 7.27-7.34 (3H, m)

### Reference Example 5(33)

### 3-Amino-4-chloro-N-phenylbenzenesulfonamide

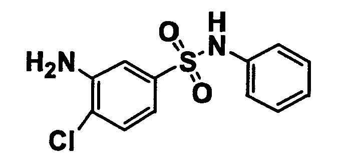

¹H-NMR (CDCl₃) δ 4.23 (1H, br s), 6. 45 (1H, br s), 7.02 (1H, dd), 7.05-7.09 (2H, m), 7.12-7.18 (2H, m), 7.24-7.30 (3H, m)

### Reference Example 5(34)

### (5-(3,4-Dihydroquinolin-1(2H)-ylcarbonyl)-2-fluorophenyl)amine

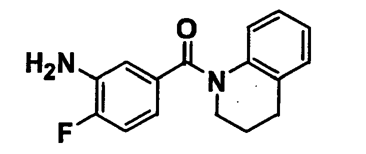

HPLC (220 nm) purity 100% (retention time: 1.90 minutes)
MS (ESI+, m/e) 271 (M+1)

### Reference Example 5(35)

### (3-(3,4-Dihydroquinolin-1(2H)-ylcarbonyl)-4-fluorophenyl)amine

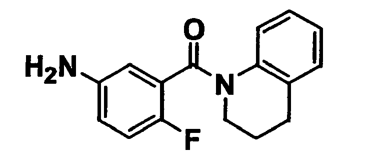

HPLC (220 nm) purity 100% (retention time: 1.57 minutes)
MS (ESI+, m/e) 271 (M+1)

### Reference Example 5(36)

### 3-(2,3-Dihydro-1H-indol-1-ylsulfonyl)aniline

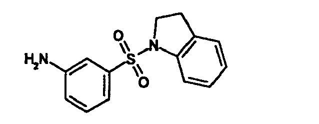

¹H-NMR (CDCl₃) δ 2.90 (2H, t), 3.83 (2H, s), 3.91 (2H, t), 6.76-6.80 (1H, m), 6.93-6.98 (1H, m), 7.04-7.19 (5H, m), 7.61 (1H, d)

### Reference Example 5(37)

### 3-(3,4-Dihydro-1(2H)-quinolinylsulfonyl)aniline

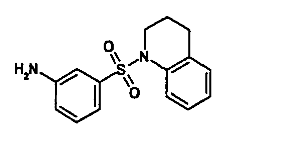

¹H-NMR (CDCl₃) δ 1.67 (2H, quintet), 2.48 (2H, t), 3. 79 (2H, t), 3.79 (2H, s), 6.75-7.22 (7H, m), 7.76 (1H, d)

### Reference Example 5(38)

### 3-(2,3,4,5-Tetrahydro-1H-1-benzazepin-1-ylsulfonyl)aniline

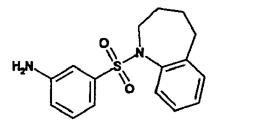

¹H-NMR (CDCl₃) δ 1. 55-1. 59 (2H, m), 1.81 (2H, quintet), 2.45 (2H, t), 3.67-3.71 (2H, m), 3.83 (2H, s), 6.80 (1H, ddd), 7.00 (1H, t), 7.06-7.31 (6H, m)

### Reference Example 5(39)

### 3-(3,4,5,6-Tetrahydro-1-benzazocin-1(2H)-ylsulfonyl)aniline

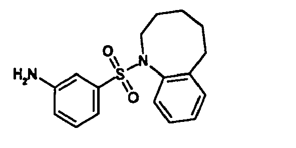

¹H-NMR (CDCl₃) δ 1.22-1.63 (6H, m), 2.80-3.00 (4H, m), 3.87 (2H, s), 6.65 (1H, d), 6.86 (1H, ddd), 7.04-7.33 (6H, m)

### Reference Example 5(40)

### 3-[(2-Methyl-3,4-dihydroquinolin-1(2H)-yl)sulfonyl]aniline

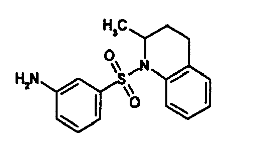

¹H-NMR (CDCl₃) δ 1.27 (3H, d), 1.31-1.37 (1H, m), 1.80-1.94 (2H, m), 2.36-2.43 (1H, m), 3.75 (2H, s), 4.29-4.36 (1H, m), 6.72-6.86 (3H, m), 6.99 (1H, d), 7.06-7.22 (3H, m), 7.71 (1H, d)

### Reference Example 5(41)

### 5-(3,4-Dihydroquinolin-1(2H)-ylsulfonyl)-2-fluoroaniline

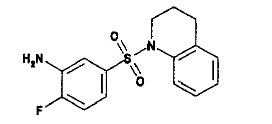

¹H-NMR (CDCl₃) δ 1.67 (2H, quintet), 2.48 (2H, t), 3.78 (2H, t), 3.85 (2H, s), 6.88-7.02 (4H, m), 7.07 (1H, dt), 7.15-7.20 (1H, m), 7.74 (1H, d)

### Reference Example 5(42)

### 3-(5,6,7,8-Tetrahydro-4H-thieno[3,2-b]azepin-4-ylsulfonyl)aniline

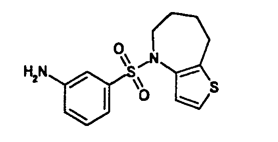

¹H-NMR (CDCl₃) δ 1.46-1.49 (2H, m), 1.71-1.76 (2H, m), 2.31 (2H, t), 3.69-3.71 (2H, m), 3.82 (2H, s), 6.80 (1H, ddd), 6.91 (1H, d), 6.92 (1H, t), 7.01-7.05 (1H, m), 7.04 (1H, d), 7.20 (1H, d)

### Reference Example 5(43)

### 3-(2,3-Dihydro-4,1-benzothiazepin-1(5H)-ylsulfonyl)aniline

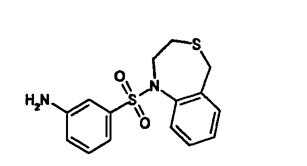

¹H-NMR (CDCl₃) δ 2.95-3.59 (6H, m), 3.88 (2H, s), 6.83-6.86 (1H, m), 7.02 (1H, t), 7.10-7.13 (1H, m), 7.16-7.29 (5H, m)

### Reference Example 5(44)

### 3-(3,4-Dihydro-1,5-benzoxazepin-5(2H)-ylsulfonyl)aniline

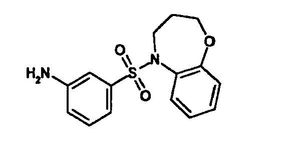

¹H-NMR (CDCl₃) δ 1.90 (2H, quintet), 3.81 (2H, s), 3.86-3.87 (4H, m), 6.80 (1H, ddd), 6.91 (1H, t), 6.99 (1H, dd), 7.02-7.09 (2H, m), 7.20 (1H, t), 7.21 (1H, dt), 7.46 (1H, dd)

### Reference Example 5(45)

### 3-(3,4-Dihydro-1,5-benzothiazepin-5(2H)-ylsulfonyl)aniline

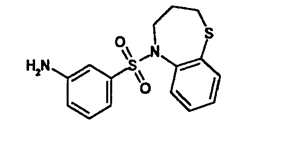

¹H-NMR (CDCl₃) δ 2.09 (2H, quintet), 2.64-2.68 (2H, m), 3.70-3.82 (2H, m), 3.82 (2H, s), 6.81 (1H, ddd), 7.04 (1H, t), 7.11-7.28 (4H, m), 7.43 (1H, dd), 7.52 (1H, dd)

### Reference Example 5(46)

### 3-[(5-Methyl-3,4-dihydroquinolin-1(2H)-yl)sulfonyl]aniline

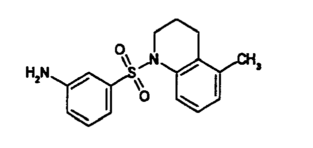

¹H-NMR (CDCl₃) δ 1.69 (2H, quintet), 2.16 (3H, s), 2.41 (2H, t), 3.78 (2H, t), 3. 80 (2H, s), 6. 78 (1H, ddd), 6.90 (1H, t), 6.94-6.98 (2H, m), 7.09 (1H, t), 7.16 (1H, t), 7.60 (1H, d)

### Reference Example 5(47)

### 3-[(4-Methyl-3,4-dihydroquinolin-1(2H)-yl)sulfonyl]aniline

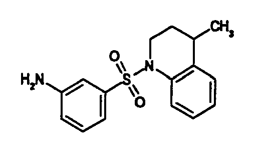

¹H-NMR (CDCl₃) δ 1.05 (3H, d), 1.30-1.32 (1H, m), 1.73-1.79 (1H, m), 2.61 (1H, sextet), 3.70-3.84 (4H, m), 6.76 (1H, ddd), 6.84 (1H, t), 6.90-6.93 (1H, m), 7.07-7.20 (4H, m), 7.76 (1H, d)

### Reference Example 5(48)

### 3-(2,3-Dihydro-4,1-benzoxazepin-1(5H)-ylsulfonyl)aniline

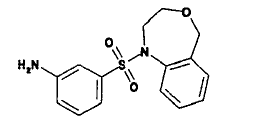

¹H-NMR (CDCl₃) δ 3.83-3.84 (4H, m), 3.87 (2H, s), 4.27-4.29 (2H, m), 6.83 (1H, dd), 6.98 (1H, t), 7.06 (1H, d), 7.19-7.32 (4H, m), 7.41-7.44 (1H, m)

### Reference Example 5(49)

### 1-[(3-Aminophenyl)sulfonyl]-2,3,4,5-tetrahydro-1H-1-benzazepin-5-ol

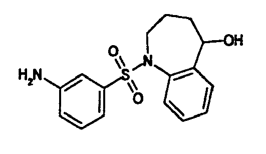

¹H-NMR (CDCl₃) δ 1.65-1.69 (1H, m), 1.91-1.96 (3H, m), 2.32-2.36 (1H, m), 3.25-3.29 (1H, m), 3.88 (2H, s), 3.88-3.92 (1H, m), 4.65 (1H, d), 6.81-6.85 (1H, m), 7.03 (1H, t), 7.11-7.32 (5H, m), 7.49 (1H, d)

### Reference Example 5(50)

### 3-Amino-N-(2-methylphenyl)benzenesulfonamide

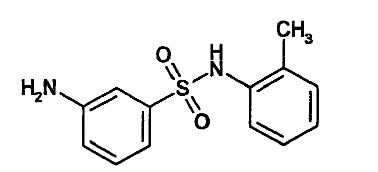

¹H-NMR (CDCl₃) δ 2.02 (3H, s), 3.82 (2H, s), 6.26 (1H, s), 6.79 (1H, ddd), 6.97 (1H, t), 7.04-7.16 (4H, m), 7.18 (1H, t), 7.29 (1H, d)

### Reference Example 5(51)

### 3-Amino-N-(2-butylphenyl)benzenesulfonamide

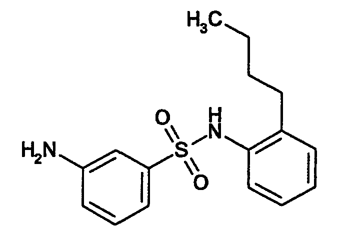

¹H-NMR (CDCl₃) δ 0.87 (3H, t), 1.24-1.37 (4H, m), 2.32 (2H, t), 3.82 (2H, s), 6.29 (1H, s), 6.76-6.80 (1H, m), 6.97 (1H, t), 7.05-7.16 (4H, m), 7.17 (1H, t), 7.32-7.35 (1H, m)

### Reference Example 5(52)

### 3-Amino-N-(2-methoxyphenyl)benzenesulfonamide

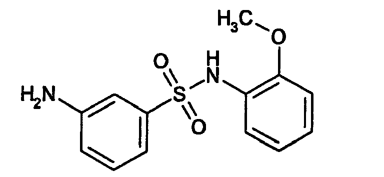

¹H-NMR (CDCl₃) δ 3.66 (3H, s), 3.80 (2H, s), 6.73-6.76 (2H, m), 6.88 (1H, dt), 6.97 (1H, s), 6.99-7.17 (4H, m), 7.49 (1H, dd)

### Reference Example 5(53)

### 3-Amino-N-(2-fluorophenyl)benzenesulfonamide

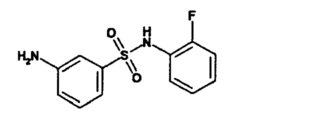

¹H-NMR (CDCl₃) δ 3.85 (2H, s), 6.68 (1H, s), 6.77-6.80 (1H, m), 6.93-7.13 (4H, m), 7.18 (1H, t), 7.53-7.62 (2H, m)

### Reference Example 5(54)

### 3-Amino-N-5,6,7,8-tetrahydronaphthalen-1-ylbenzenesulfonamide

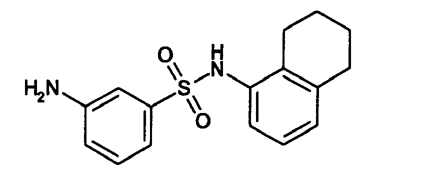

¹H-NMR (CDCl₃) δ 1.64-1.68 (4H, m), 2.33-2.35 (2H, m), 2.69-2.71 (2H, m), 3.82 (2H, s), 6.20 (1H, s), 6.78 (1H, dd), 6.89 (1H, d), 6.98 (1H, t), 7.03 (1H, d), 7.07 (1H, d), 7.11 (1H, d), 7.18 (1H, t)

### Reference Example 5(55)

### 3-Amino-N-1,2,3,4-tetrahydronaphthalen-1-ylbenzenesulfonamide

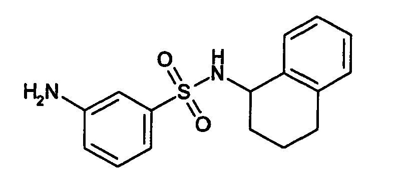

¹H-NMR (CDCl₃) δ 1.73-1.88 (4H, m), 2.68-2.73 (2H, m), 3.93 (2H, s), 4.46-4.47 (1H, m), 4.57 (1H, d), 6.86 (1H, dt), 6.92 (1H, d), 7.02-7.07 (2H, m), 7.13 (1H, dt), 7.22-7.33 (3H, m)

### Reference Example 5(56)

### 3-Amino-N-[2-(1-hydroxyethyl)phenyl]benzenesulfonamide

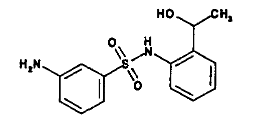

¹H-NMR (CDCl₃) δ 1.38 (3H, d), 2.39 (1H, s), 3.87 (2H, s), 4.84-4.86 (1H, m), 6.76-6.80 (1H, m), 7.03-7.23 (6H, m), 7.44 (1H, d), 8.37 (1H, s)

### Reference Example 5(57)

### 2-{[(3-Aminophenyl)sulfonyl]amino}benzamide

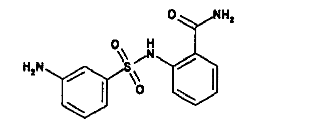

¹H-NMR (DMSO-d₆) δ 5.60 (2H, s), 6.70 (1H, dd), 6.84 (1H, d), 6.98 (1H, t), 7.06 (1H, ddd), 7.12 (1H, t), 7.41-7.48 (2H, m), 7.78 (1H, d), 7.85 (1H, s), 8.34 (1H, s), 12.14 (1H, s)

### Reference Example 5(58)

### 3-[(5-Methyl-2,3,4,5-tetrahydro-1H-1-benzazepin-1-yl) sulfonyl] aniline

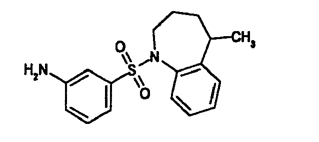

¹H-NMR (CDCl₃) δ 1.22 (3H, d), 1.66-1.70 (2H, m), 1.88-1.92 (1H, m), 2.71-2.75 (1H, m), 3.18-3.22 (1H, m), 3.85 (2H, s), 3.85-4.05 (2H, m), 6.82 (1H, ddd), 7.04 (1H, s), 7.15-7.26 (6H, m)

### Reference Example 5(59)

### 3-Amino-N-(2-cyanoethyl)-N-phenylbenzenesulfonamide

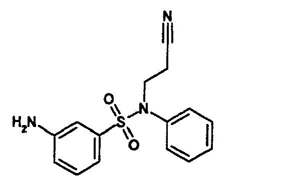

¹H-NMR (CDCl₃) δ 2.61 (2H, t), 3.83 (2H, t), 3.86 (2H, s), 6.82-6.86 (2H, m), 6.92-6.95 (1H, m), 7.07-7.10 (2H, m), 7.19-7.24 (1H, m), 7.32-7.36 (3H, m)

### Reference Example 5(60)

### 3-Amino-N-(2-hydroxyethyl)-N-phenylbenzenesulfonamide

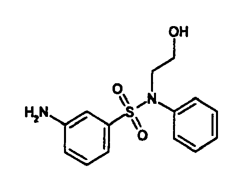

¹H-NMR (CDCl₃) δ 2.05 (1H, t), 3.62-3.74 (4H, m), 3.85 (2H, s), 6. 83 (1H, ddd), 6.88 (1H, dd), 6.96 (1H, ddd), 7. 08-7. 12 (2H, m), 7.22 (1H, t), 7.29-7.35 (3H, m)

### Reference Example 5(61)

### Ethyl N-[(3-aminophenyl)sulfonyl]-N-phenylglycinate

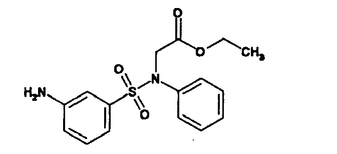

¹H-NMR (CDCl₃) δ 1.21 (3H, t), 3.84 (2H, s), 4.13 (2H, q), 4.37 (2H, s), 6.81 (1H, ddd), 6.92 (1H, t), 6.98-7.02 (1H, m), 7.17-7.31 (6H, m)

### Reference Example 5(62)

### 4-[(3-Aminophenyl)sulfonyl]-3,4-dihydroquinoxalin-2(1H)-one

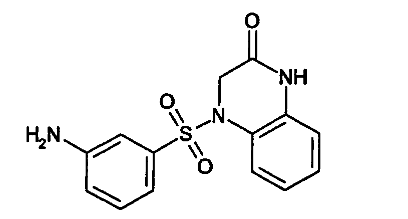

¹H-NMR (CDCl₃) δ 3.81 (2H, s), 4.34 (2H, s), 6.68 (1H, dd), 6.73-6.76 (3H, m), 7.06 (1H, dd), 7.12 (1H, dt), 7.22 (1H, dt), 7.70 (1H, d), 7.87 (1H, s)

### Reference Example 5(63)

### 5-[(3-Aminophenyl)sulfonyl]-1,3,4,5-tetrahydro-2H-1,5-benzodiazepin-2-one

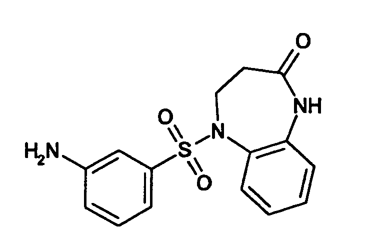

¹H-NMR (DMSO-d₆) δ 2.38 (2H, t), 4.01 (2H, t), 5.59 (2H, s), 6.72-6.79 (2H, m), 6.92 (1H, t), 7.03 (1H, d), 7.11-7.19 (2H, m), 7.24 (1H, dd), 7.31 (1H, dt), 9.52 (1H, s)

### Reference Example 5(64)

### 4-[(3-Aminophenyl)sulfonyl]-3-methyl-3,4-dihydroquinoxalin-2(1H)-one

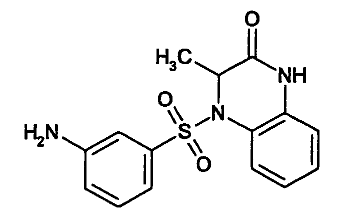

¹H-NMR (DMSO-d₆) δ 1. 14 (3H, d), 4.53 (1H, q), 5.56 (2H, s), 6.38-6.41 (1H, m), 6.63 (1H, t), 6.72 (1H, dd), 6. 82 (1H, dd), 7.02-7.11 (2H, m), 7.23 (1H, dt), 7.52 (1H, d), 10.36 (1H, s)

### Reference Example 5(65)

### 5-[(3-Aminophenyl)sulfonyl]-4-methyl-1,3,4,5-tetrahydro-2H-1,5-benzodiazepin-2-one

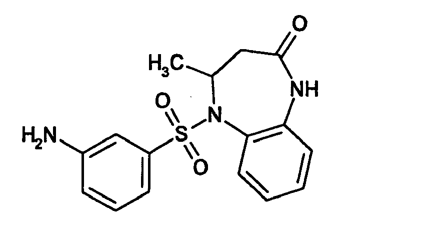

¹H-NMR (DMSO-d₆) δ 1.14 (3H, d), 2.15-2.23 (2H, m), 4.70 (1H, sextet), 5.52 (2H, s), 6.64-6.67 (1H, m), 6.71-6.75 (1H, m), 6.85 (1H, t), 7.00 (1H, d), 7.11 (1H, t), 7.14-7.19 (1H, m), 7.27 (1H, d), 7.32-7.38 (1H, m), 9.40 (1H, s)

### Reference Example 5 (66)

### 3-(2,3-Dihydro-4H-pyrido[3,2-b][1,4]oxazin-4-ylsulfonyl)aniline

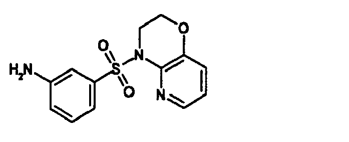

¹H-NMR (CDCl₃) δ 3.88 (2H, s), 4.10 (2H, t), 4.30 (2H, t), 6.80-6.84 (1H, m), 6.88 (1H, ddd), 7.11 (1H, ddd), 7.23 (1H, t), 7.38-7.41 (2H, m), 7.91 (1H, ddd)

### Reference Example 5(67)

### 3-(2,3-Dihydro-4H-1,4-benzothiazin-4-ylsulfonyl)aniline

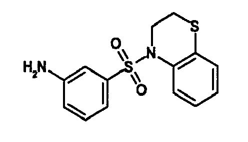

¹H-NMR (CDCl₃) δ 2.89 (2H, t), 3.81 (2H, s), 3.97 (2H, t), 6.79-6.85 (2H, m), 6.94-6.98 (1H, m), 7.07-7.11 (3H, m), 7.18 (1H, t), 7.63-7.67 (1H, m)

### Reference Example 5 (68)

### N-{1-[(3-Aminophenyl)sulfonyl]-1,2,3,4-tetrahydroquinolin-3-yl}acetamide

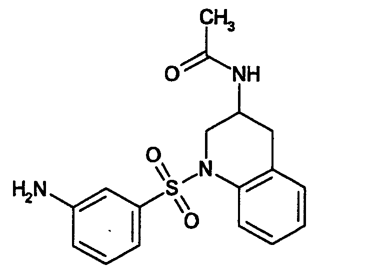

¹H-NMR (CDCl₃) δ 1.96 (3H, s), 2.58 (1H, dd), 2.86 (1H, dd), 3.70 (1H, dd), 3.96 (2H, s), 3.99 (1H, dd), 4.26-4.32 (1H, m), 5.58 (1H, d), 6.82 (1H, dd), 7.01-7.10 (3H, m), 7.13 (1H, t), 7.17-7.22 (2H, m), 7.73 (1H, d)

### Reference Example 5(69)

### 3-[(1-Methyl-5,6,7,8-tetrahydropyrazolo[4,3-b]azepin-4(1H)-yl)sulfonyl]aniline

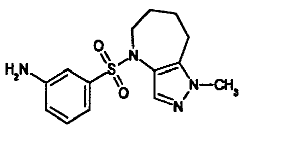

¹H-NMR (CDCl₃) δ 1. 49-1. 51 (2H, m), 1.72-1. 74 (2H, m), 2.33 (2H, t), 3.69-3.74 (2H, m), 3.74 (3H, s), 3.84 (2H, s), 6.79-6.82 (1H, m), 6.95 (1H, t), 7.03-7.06 (1H, m), 7.20 (1H, t), 7.41 (1H, s)

### Reference Example 5(70)

### 3-[(1,7,7-Trimethyl-5,6,7,8-tetrahydropyrazolo[4,3-b]azepin-4(1H)-yl)sulfonyl]aniline

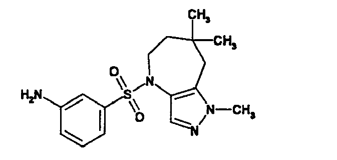

¹H-NMR (CDCl₃) δ 0.83 (6H, s), 1.58-1.61 (2H, m), 2.14 (2H, s), 3.68-3.70 (2H, m), 3.71 (3H, s), 3. 84 (2H, s), 6.80-6.84 (1H, m), 6.96 (1H, t), 7.07-7.10 (1H, m), 7.22 (1H, t), 7.35 (1H, s)

### Reference Example 5(71)

### 3-[(8-Methoxy-2,3,4,5-tetrahydro-1H-1-benzazepin-1-yl)sulfonyl]aniline

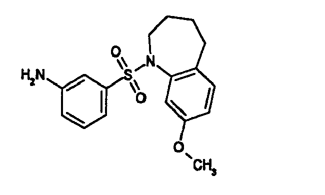

¹H-NMR (CDCl₃) δ 1.50-1.56 (2H, m), 1.81 (2H, quintet), 2.36-2.41 (2H, m), 3.70-3.73 (2H, m), 3.73 (3H, s), 3.83 (2H, s), 6.72 (1H, dd), 6.78-6.84 (2H, m), 6.99-7.03 (2H, m), 7.11 (1H, dt), 7.22 (1H, t)

### Reference Example 5(72)

### 3-[(6,9-Dimethoxy-2,3,4,5-tetrahydro-1H-1-benzazepin-1-yl)sulfonyl]aniline

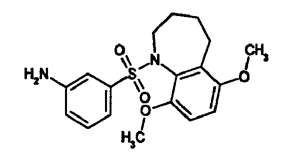

¹H-NMR (CDCl₃) δ 1.16-1.20 (1H, m), 1.60-1.66 (1H, m), 1.90-2.05 (2H, m), 2.55 (1H, t), 2.94 (1H, ddd), 3.25 (1H, dd), 3.60 (3H, s), 3.76 (3H, s), 3.84 (2H, s), 4.10 (1H, dt), 6.67 (1H, d), 6.79 (1H, d), 6.82-6.85 (1H, m), 7.23-7.34 (3H, m)

### Reference Example 5(73)

### 3-[(6-Methoxy-2,3,4,5-tetrahydro-1H-1-benzazepin-1-yl)sulfonyl]aniline

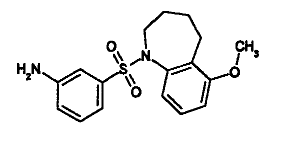

¹H-NMR (CDCl₃) δ 1.50-1.55 (2H, m), 1.80-1.82 (2H, m), 2.61-2.65 (2H, m), 3.54-3.79 (2H, m), 3.79 (3H, s), 3.85 (2H, s), 6.80 (1H, d), 6.80-6.83 (1H, m), 6.88 (1H, d), 7.06-7.11 (2H, m), 7.13-7.15 (1H, m), 7.24 (1H, t)

### Reference Example 5(74)

### 3-[(6,8-Dimethyl-2,3,4,5-tetrahydro-1H-1-benzazepin-1-yl)sulfonyl]aniline

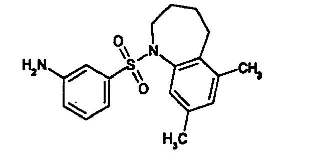

¹H-NMR (CDCl₃) δ 1.55-1.78 (6H, m), 2.22 (3H, s), 2.24 (3H, s), 2.48-2.52 (2H, m), 3. 84 (2H, s), 6. 79-6. 82 (1H, m), 6.87-6.88 (2H, m), 7.06 (1H, t), 7.12-7.15 (1H, m), 7.23 (1H, t)

### Reference Example 5(75)

### 3-[(8-Methyl-2,3,4,5-tetrahydro-1H-1-benzazepin-1-yl)sulfonyl]aniline

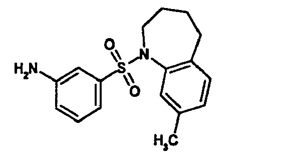

¹H-NMR (CDCl₃) δ 1.49-1.53 (2H, m), 1.77 (2H, quintet), 2.29 (3H, s), 2.36 (2H, t), 3.66-3.70 (2H, m), 3.83 (2H, s), 6.81 (1H, ddd), 6.99-7.01 (3H, m), 7.08-7.11 (1H, m), 7.14 (1H, s), 7.22 (1H, t)

### Reference Example 5(76)

### 3-[(7,8-Dimethoxy-2,3,4,5-tetrahydro-1H-1-benzazepin-1-yl)sulfonyl]aniline

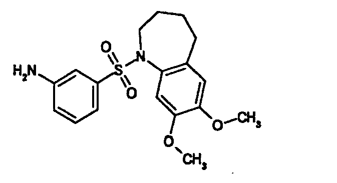

¹H-NMR (CDCl₃) δ 1.50-1.55 (2H, m), 1.80 (2H, quintet), 2.34 (2H, t), 3.76 (3H, s), 3.76-3.85 (2H, m), 3.82 (2H, s), 3.85 (3H, s), 6.57 (1H, s), 6.76 (1H, s), 6.79 (1H, ddd), 6.98 (1H, t), 7.07-7.10 (1H, m), 7.21 (1H, t)

### Reference Example 5(77)

### 3-(2,3,7,8,9,10-hexahydro-6H-[1,4]dioxino[2,3-h][1]benzazepin-6-ylsulfonyl)aniline

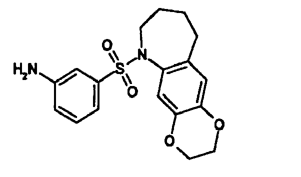

¹H-NMR (CDCl₃) δ 1.49-1.53 (2H, m), 1.79 (2H, quintet), 2.32 (2H, t), 3.64-3.66 (2H, m), 3.84 (2H, s), 4.19-4.24 (4H, m), 6.59 (1H, s), 6.79 (1H, s), 6.79 (1H, ddd), 7.02 (1H, t), 7.09 (1H, dt), 7.21 (1H, t)

### Reference Example 5(78)

### 3-[(8-Phenyl-2,3,4,5-tetrahydro-1H-1-benzazepin-1-yl)sulfonyl]aniline

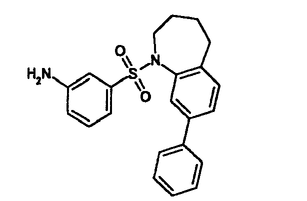

¹H-NMR (CDCl₃) δ 1. 56-1. 60 (2H, m), 1.84 (2H, quintet), 2.51 (2H, t), 3.72-3.76 (2H, m), 3.84 (2H, s), 6.82 (1H, ddd), 7.04 (1H, t), 7. 13 (1H, ddd), 7.18 (1H, d), 7.23 (1H, t), 7.28-7.34 (1H, m), 7.37-7.42 (3H, m), 7.49-7.53 (3H, m)

### Reference Example 5(79)

### 3-[(8-Cyclohexyl-2,3,4,5-tetrahydro-1H-1-benzazepin-1-yl)sulfonyl]aniline

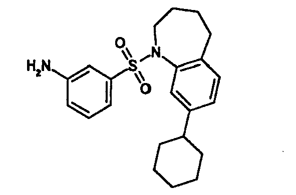

¹H-NMR (CDCl₃) δ 1.18-1.42 (5H, m), 1.53-1.55 (2H, m), 1.70-1.82 (7H, m), 2.40-2.47 (3H, m), 3.65-3.69 (2H, m), 3.81 (2H, s), 6.78-6.82 (1H, m), 6.97-7.05 (4H, m), 7.07-7.10 (1H, m), 7.21 (1H, t)

### Reference Example 5(80)

### 3-[(8-tert-Butyl-2,3,4,5-tetrahydro-1H-1-benzazepin-1-yl)sulfonyl]aniline

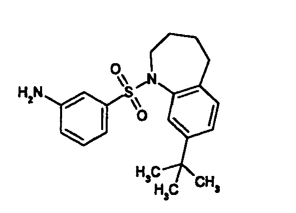

¹H-NMR (CDCl₃) δ 1.22 (9H, s), 1.55-1.57 (2H, m), 1.86 (2H, quintet), 2.55 (2H, t), 3.66-3.70 (2H, m), 3.82 (2H, s), 6.81 (1H, ddd), 7.03-7.20 (5H, m), 7.23 (1H, t)

### Reference Example 5 (81)

### 3-[(8-Phenoxy-2,3,4,5-tetrahydro-1H-1-benzazepin-1-yl)sulfonyl]aniline

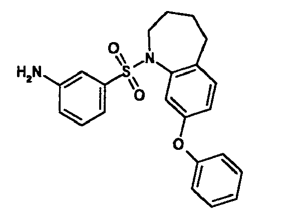

¹H-NMR (CDCl₃) δ 1.55-1.59 (2H, m), 1.85 (2H, quintet), 2.53 (2H, t), 3.63-3.65 (2H, m), 3.76 (2H, s), 6.75-6.78 (1H, m), 6.83-6.86 (1H, m), 6.91-7.19 (8H, m), 7.32-7.37 (2H, m)

### Reference Example 5(82)

### 3-[(8-Fluoro-2,3,4,5-tetrahydro-1H-1-benzazepin-1-yl)sulfonyl]aniline

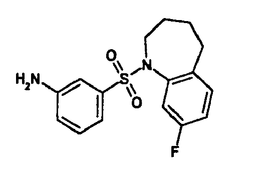

¹H-NMR (CDCl₃) δ 1.51-1.55 (2H, m), 1.81 (2H, quintet), 2.40-2.45 (2H, m), 3.66-3.70 (2H, m), 3.85 (2H, s), 6.82 (1H, ddd), 6.90 (1H, dd), 7.00-7.12 (4H, m), 7.24 (1H, t)

### Reference Example 5(83)

### 3-[(8-Bromo-2,3,4,5-tetrahydro-1H-1-benzazepin-1-yl)sulfonyl]aniline

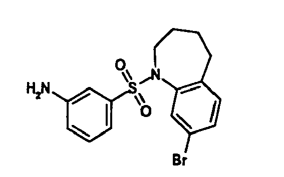

¹H-NMR (CDCl₃) δ 1.50-1.54 (2H, m), 1.80 (2H, quintet), 2.37-2.43 (2H, m), 3.63-3.67 (2H, m), 3.86 (2H, s), 6.82 (1H, ddd), 6.97-7.01 (2H, m), 7.07-7.12 (1H, m), 7.24 (1H, t), 7.30 (1H, dd), 7.45 (1H, d)

### Reference Example 5(84)

### 4-[(3-Aminophenyl)sulfonyl]-1-methyl-3,4-dihydroquinoxalin-2(1H)-one

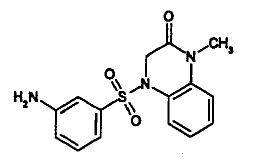

¹H-NMR (CDCl₃) δ 2.79 (3H, s), 3.81 (2H, s), 4.36 (2H, s), 6.69-6.77 (3H, m), 6.85 (1H, dd), 7.09 (1H, t), 7.17 (1H, dt), 7.32 (1H, dt), 7.70 (1H, dd)

### Reference Example 5(85)

### 5-[(3-Aminophenyl)sulfonyl]-1-methyl-1,3,4,5-tetrahydro-2H-1,5-benzodiazepin-2-one

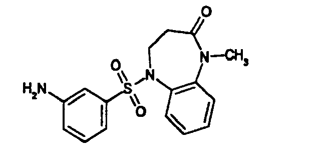

¹H-NMR (CDCl₃) δ 2.44-2.48 (2H, m), 2.67 (3H, s), 3.87 (2H, s), 4.58-4.62 (2H, m) , 6.77 (1H, ddd), 6.84 (1H, t), 6.92 (1H, ddd), 7.07 (1H, dd), 7.17 (1H, t), 7.28 (1H, dt), 7.38 (1H, dt), 7.61 (1H, dd)

### Reference Example 5(86)

### 1-[(3-Aminophenyl)sulfonyl]-2,3-dihydro-4,1-benzoxazepin-5 (1H)-one

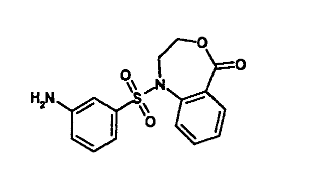

¹H-NMR (CDCl₃) δ 3.85 (2H, s), 4.02-4.06 (2H, m), 4.11-4.15 (2H, m), 6.74 (1H, t), 6.85 (1H, dd), 6.93-6.96 (1H, m), 7.21 (1H, t), 7.49 (1H, ddd), 7.64-7.67 (3H, m)

### Reference Example 5(87)

### 1-[(3-Aminophenyl)sulfonyl]-2,3,4,5-tetrahydro-1H-1-benzazepine-8-carbonitrile

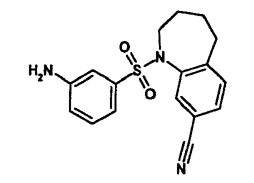

¹H-NMR (CDCl₃) δ 1.55-1.59 (2H, m), 1.84 (2H, quintet), 2.51-2.57 (2H, m), 3.65-3.69 (2H, m), 3.89 (2H, s), 6.82-6.87 (1H, m), 7.00 (1H, t), 7.06-7. 10 (1H, m), 7.22-7.30 (2H, m), 7.47 (1H, dd), 7.58 (1H, d)

### Reference Example 5(88)

### 5- (3, 4-Dihydroquinolin-1 (2H) -ylcarbonyl) -2-(trifluoromethyl)aniline

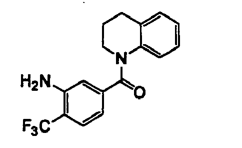

¹H-NMR (CDCl₃) δ 2.00-2.17 (2H, m), 2.79-2.92 (2H, m), 3.84-4.00 (2H, m), 6.54-7.25 (4H, m), 7.30-7.94 (3H, m)

### Reference Example 5(89)

### 2-Amino-N-[5-(3,4-dihydroquinolin-1(2H)-ylcarbonyl)-2-(trifluoromethyl)phenyl]benzamide

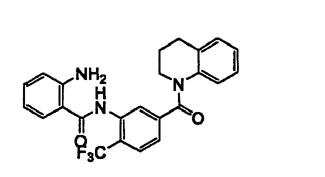

¹H-NMR (CDCl₃) δ 2.01-2.14 (2H, m), 2.87 (2H, t), 3.92 (2H, t), 5.58 (2H, br s), 6.67-6.79 (2H, m), 6.88-7.21 (5H, m), 7.27-7.33 (1H, m), 7.41 (1H, dd), 7.54 (1H, d), 8.13 (1H, s), 8.52 (1H, s)

### Reference Example 5(90)

### 2-Amino-N-(2-chloro-5-(3,4-dihydro-1(2H)-quinolinylsulfonyl)phenyl)benzamide

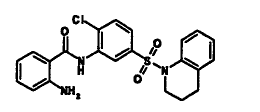

¹H-NMR (CDCl₃) δ 1.76-1.85 (2H, m), 2.52 (2H, t), 3.88-3.92 (2H, m), 5.64 (2H, br s), 6.69-6.74 (2H, m), 7.00-7.08 (2H, m), 7.15-7.31 (3H, m), 7.40 (1H, d), 7.48 (1H, dd), 7. 77 (1H, d), 8.36 (1H, br s), 8.94 (1H, d)

### Reference Example 5(91)

### 2-Amino-5-chloro-N-(2-chloro-5-(3, 4-dihydro-1(2H)-quinolinylsulfonyl)phenyl)benzamide

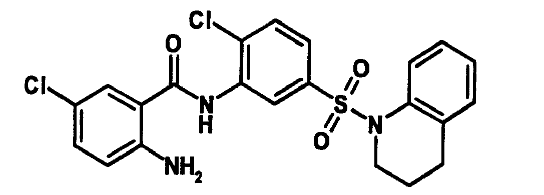

¹H-NMR (CDCl₃) δ 1.74-1.83 (2H, m), 2.53 (2H, t), 3.86-3.90 (2H, m), 5.60 (1H, br s), 6.68 (1H, d), 7.00-7.08 (2H, m), 7.15-7.22 (2H, m), 7.40-7.43 (2H, m), 7.77 (1H, d), 8.25 (1H, br s), 8.86 (1H, d)

### Reference Example 5(92)

### 2-Amino-N-(2-chloro-5-(3,4-dihydro-1(2H)-quinolinylsulfonyl)phenyl)-5-methoxybenzamide

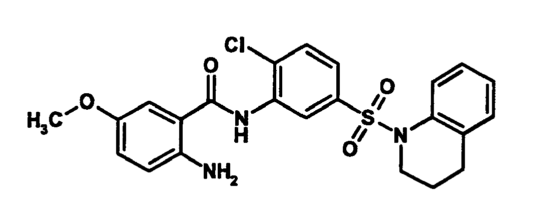

¹H-NMR (CDCl₃) δ 1.76-1.85 (2H, m), 2.54 (2H, t), 3.79 (1H, s), 3.88-3.92 (2H, m), 6.74 (1H, d), 6.94-7.08 (4H, m), 7.15-7.20 (1H, m), 7.21 (1H, dd), 7.42 (1H, d), 7.77 (1H, d), 8.67 (1H, br s), 8.95 (1H, d)

### Reference Example 5(93)

### 2-Amino-3-chloro-N-(2-chloro-5-(3,4-dihydro-1(2H)-quinolinylsulfonyl)phenyl)benzamide

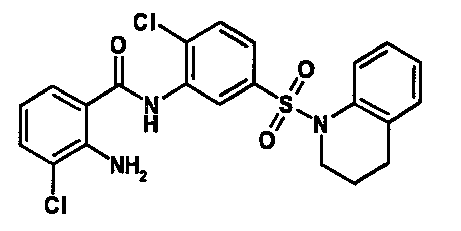

¹H-NMR (CDCl₃) δ 1.81 (2H, t), 2.55 (2H, t), 3.91 (2H, t), 6.18 (1H, br s), 6.67 (1H, t), 7.01-7.11 (2H, m), 7.20 (1H, td), 7.26 (1H, dd), 7.41-7.45 (3H, m), 7.78 (1H, d), 8.32 (1H, br s), 8.90 (1H, d)

### Reference Example 5(94)

### 2-Amino-N-(2-chloro-5-(3,4-dihydro-1(2H)-quinolinylsulfonyl)phenyl)-3-methylbenzamide

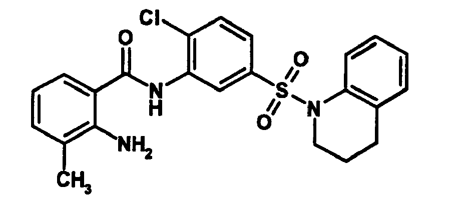

¹H-NMR (CDCl₃) δ 1.81 (2H, t), 2.55 (2H, t), 3.91 (2H, t), 5.72 (1H, br s), 6.67 (1H, t), 7.00-7.09 (2H, m), 7.16-7.23 (2H, m), 7.23 (1H, dd), 7.39 (1H, d), 7.40 (1H, d), 7.78 (1H, d), 8.36 (1H, br s), 8.96 (1H, d)

### Reference Example 5(95)

### 5-(3,4-Dihydroquinolin-1(2H)-ylcarbonyl)thiophen-2-amine

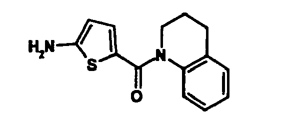

¹H-NMR (DMSO-d₆) δ 2.01 (2H, dt), 2.77 (2H, t), 3.87 (2H, t), 4.16 (2H, br s), 5.88 (1H, d), 6.66 (1H, d), 7.00-7.09 (2H, m), 7.13-7.18 (2H, m)

### Reference Example 5(96)

### 4-(3,4-Dihydro-1(2H)-quinolinylcarbonyl)thiophen-2-amine

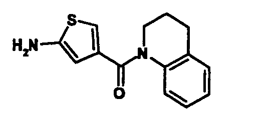

¹H-NMR (DMSO-d₆) δ 1.97-2.06 (2H, m), 2.79 (2H, t), 3.68 (2H, br s), 3.87 (2H, t), 6.05 (1H, d), 6.65 (1H, d), 6.91-7.06 (3H, m), 7.14 (1H, d)

### Reference Example 5(97)

### 2-Amino-N-[3- (3,4-dihydroquinolin-1(2H) - ylsulfonyl)phenyl]-3-methoxybenzamide

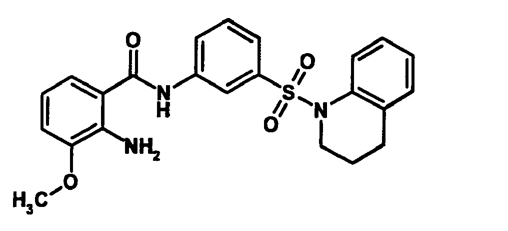

¹H-NMR (CDCl₃) δ 1.69 (2H, ddd), 2.47 (2H, t), 3.81 (2H, t), 3.87 (3H, s), 5.82 (2H, br s), 6.65 (1H, t), 6.85 (1H, d), 6.99-7.11 (3H, m), 7.16 (1H, t), 7.26 (1H, d), 7.37 (1H, t), 7.74-7.78 (2H, m), 8.02 (1H, dd), 8.06 (1H, br s)

### Reference Example 5(98)

### 2-Amino-N-[2-chloro-5-(3,4-dihydroquinolin-1(2H)-ylsulfonyl)phenyl]-3-methoxybenzamide

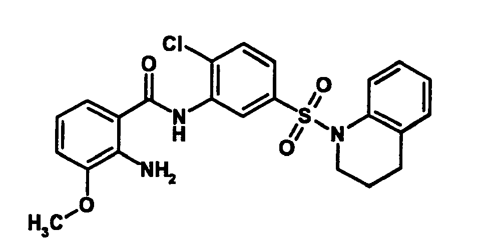

¹H-NMR (CDCl₃) δ 1.81 (2H, ddd), 2.54 (2H, t), 3.90 (3H, s), 3.91 (2H, t), 5.97 (2H, br s), 6.67 (1H, t), 6.87 (1H, dd), 7.00-7.12 (3H, m), 7.16-7.24 (2H, m), 7.40 (1H, d), 7.78 (1H, d), 8.40 (1H, br s), 8.97 (1H, d)

### Reference Example 5(99)

### 2-Chloro-5-{[7-(trifluoromethyl)-3,4-dihydroquinolin-1 (2H)-yl]sulfonyl}aniline

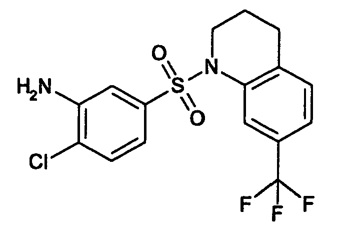

¹H-NMR (CDCl₃) δ 1.72 (2H, quintet), 2.58 (2H, t), 3.81 (2H, t), 4.24 (2H, s), 6.88 (1H, dd), 7.00 (1H, d), 7.14 (1H, d), 7.26-7.33 (2H, m), 8.08 (1H, s)

### Reference Example 5(100)

### 2-Chloro-5-(5,6,7,8-tetrahydro-4H-thieno[3,2-b]azepin-4-ylsulfonyl)aniline

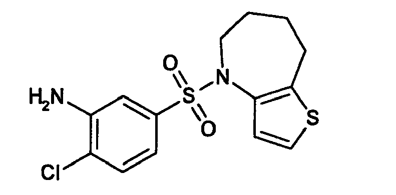

¹H-NMR (CDCl₃) δ 1.47-1.52 (2H, m), 1.69-1.75 (2H, m), 2.30-2.36 (2H, m), 3.68-3.73 (2H, m), 4.22 (2H, s), 6.91 (1H, d), 6.96 (1H, dd), 7.01 (1H, d), 7.02 (1H, d), 7.30 (1H, d)

### Reference Example 5(101)

### 2-Chloro-5-(2,3-dihydro-4,1-benzothiazepin-1(5H)-ylsulfonyl)aniline

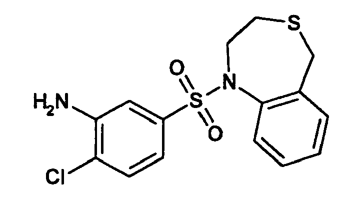

¹H-NMR (CDCl₃) δ 2.97-3.61 (6H, m), 4.28 (2H, s), 7.04 (1H, dd), 7.09-7.28 (5H, m), 7.36 (1H, d)

### Reference Example 5(102)

### 2-Chloro-5-[(6-fluoro-3,4-dihydroquinolin-1(2H)-yl)sulfonyl]aniline

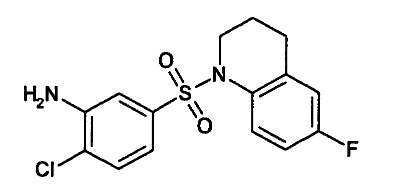

¹H-NMR (CDCl₃) δ 1.64 (2H, quintet), 2.44 (2H, t), 3.76 (2H, t), 4.22 (2H, s), 6.74 (1H, dd), 6.82 (1H, dd), 6.87-6.95 (2H, m), 7.26 (1H, d), 7.73 (1H, dd)

### Reference Example 5(103)

### 2-Chloro-5-[(5-methyl-3,4-dihydroquinolin-1(2H)-yl)sulfonyl]aniline

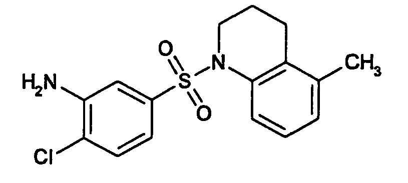

¹H-NMR (CDCl₃) δ 1.70 (2H, quintet), 2. 17 (3H, s), 2.42 (2H, t), 3.78 (2H, t), 4.20 (2H, s), 6.87 (1H, dd), 6.98 (1H, d), 6.99 (1H, d), 7.09 (1H, t), 7.25 (1H, d), 7.59 (1H, d)

### Reference Example 5(104)

### 2-Amino-N-[2-chloro-5-(3,4-dihydroquinolin-1(2H)-ylsulfonyl)phenyl]-4-fluorobenzamide

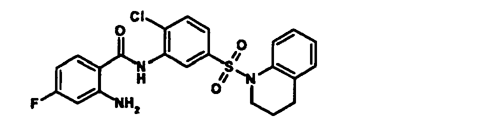

MS (ESI+, m/e) 460 (M+1)

### Reference Example 5(105)

### 2-Amino-4-chloro-N-[2-chloro-5-(3,4-dihydroquinolin-1(2H)-ylsulfonyl)phenyl]benzamide

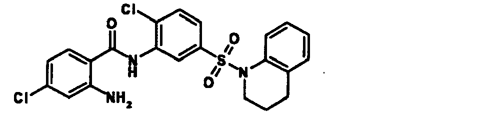

MS (ESI+, m/e) 476 (M+1)

### Reference Example 5(106)

### 2-Amino-N-[2-chloro-5-(3,4-dihydroquinolin-1(2H)-ylsulfonyl)phenyl]-4-(trifluoromethyl)benzamide

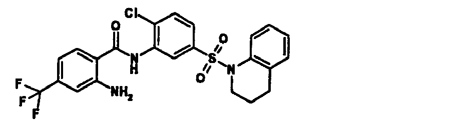

MS (ESI+, m/e) 510 (M+1)

### Reference Example 5(107)

### 5-(Acetylamino)-2-amino-N-[2-chloro-5-(3,4-dihydroquinolin-1(2H)-ylsulfonyl)phenyl]benzamide

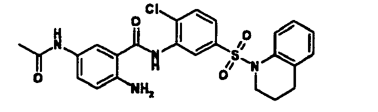

MS (ESI+, m/e) 499 (M+1)

### Reference Example 5-1

In the same manner as in Reference Example 5, ethyl 4-[(3-nitrophenyl)sulfonyl]-3,4-dihydro-2H-1,4-benzoxazine-2-carboxylate was reduced to obtain the following two components of Reference Examples 5-1 and 5-2, which were subjected to silica gel column chromatography (ethyl acetate/hexane, 1 : 1 to 2 : 1) for separation.

### Reference Example 5-1

### Ethyl 4-[(3-aminophenyl)sulfonyl]-3,4-dihydro-2H-1,4-benzoxazine-2-carboxylate

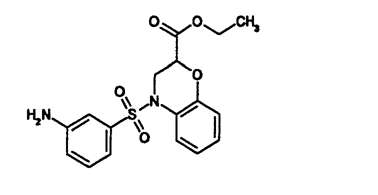

¹H-NMR (CDCl₃) δ 1.31 (3H, t), 3.47 (1H, dd), 3.86 (2H, s), 3.96 (1H, dd), 4.26 (2H, q), 4.47 (1H, dd), 6.82 (1H, ddd), 6.89 (1H, t), 6.94-7.03 (3H, m), 7.11 (1H, dt), 7.22 (1H, t), 7.81 (1H, dd)

### Reference Example 5-2

### {4-[(3-Aminophenyl)sulfonyl]-3,4-dihydro-2H-1,4-benzoxazin-2-yl}methanol

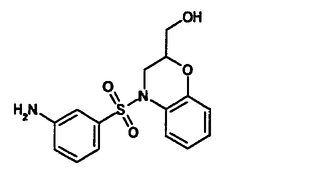

¹H-NMR (CDCl₃) δ 1.90 (1H, t), 3.37 (1H, dd), 3.53-3.56 (1H, m), 3.66-3.76 (2H, m), 3.86 (2H, s), 4.24 (1H, dd), 6.79-6.83 (1H, m), 6.84-6.97 (3H, m), 6.98-7.01 (1H, m), 7.05-7.11 (1H, m), 7.20 (1H, t), 7.80 (1H, dt)

### Reference Example 6

### 3-Chloro-5-(3,4-dihydroquinolin-1(2H)-ylcarbonyl)benzoic acid

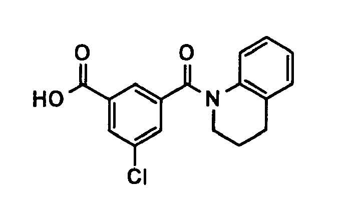

To a solution of 5-chloroisophthalic acid (602 mg), 1-ethyl-3-(3-dimethylaminopropyl)carbodiimide hydrochloride (575 mg) and N-methylimidazole (239 µl) in THF (15 ml), a solution of 1,2,3,4-tetrahydroquinoline in 0.30 M THF (10 ml) was added, and the reaction mixture was agitated overnight at room temperature. To the reaction mixture, ethyl acetate was added, and the organic layer was sequentially washed with 1 N hydrochloric acid and saturated brine and dried over anhydrous sodium sulfate. The solvent was distilled off under reduced pressure, and the residue was subjected to reversed-phase preparative HPLC (Gilson Inc., UniPoint System, YMC ODS column 30 × 75 mm). The fraction eluted with 0.1% TFA-containing acetonitrile/water (10 : 90 to 100 : 0) was concentrated under reduced pressure to give the desired product (143 mg).

¹H-NMR (CDCl₃) δ 2.08 (2H, quintet), 2.88 (2H, t), 3.92 (2H, t), 6.60-6.75 (1H, br s), 6.91 (1H, t), 7.05 (1H, t), 7.20 (1H, d), 7.58 (1H, s), 7.89 (1H, s), 8.04 (1H, s)
MS (ESI+, m/e) 316 (M+1)

### Reference Example 7

### N- (2-Chloro-5- (3 , 4-dihydro-1 (2H) - quinolinylcarbonyl)phenyl)-5-methoxy-2-nitrobenzamide

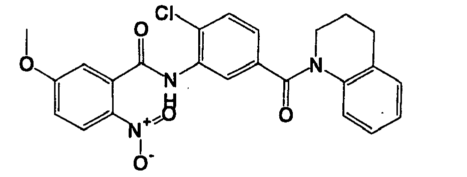

To a solution of 5-methoxy-2-nitrobenzoic acid (198 mg) in THF (5.03 ml), a 0.36 N solution of oxalyl chloride in THF (3.35 ml) was added. DMF (2 drops) was further added thereto, and the mixture was stirred at room temperature until the gas evolution ceased. After distilling off the volatile substance under reduced pressure, the residue, which was diluted with THF (4.90 ml), was gradually added dropwise to a solution of 2-chloro-5-(3,4-dihydro-1(2H)-quinolinylcarbonyl)aniline (0.20 N) and pyridine (0.25 N) in THF (4.10 ml). The reaction mixture was stirred for 14 hours at room temperature, and then was poured into 0.5 N hydrochloric acid and extracted with ethyl acetate. The organic layer was washed with a 5% sodium bicarbonate solution and saturated brine, and dried over anhydrous magnesium sulfate. Then, the solvent was distilled off under reduced pressure, and the crystals were collected by filtration to give the desired product (278 mg).

¹H-NMR (CDCl₃) δ 2.01-2.19 (2H, m), 2.85 (2H, t), 3.88-3.93 (2H, m), 3.93 (3H, s), 6.90-7.06 (5H, m), 7.10 (1H, dd), 7.17 (1H, d), 7.30 (1H, d), 7.76 (1H, s), 7.17-7.20 (1H, m), 8.53 (1H, br s)
MS (ESI+, m/e) 466 (M+1)

### Reference Examples 7(1) to 7(11)

In the same manner as in Reference Example 7, the corresponding carboxylic acid (commercially available or conventional) was reacted with 2-chloro-5-(3,4-dihydro-1(2H)-quinolinylcarbonyl)aniline in the case of Reference Examples 7 (1) to 7 (2), and reacted with 2-chloro-5-(3,4-dihydro-1(2H)-quinolinylsulfonyl)aniline in the case of Reference Examples 7(3) to 7 (11), to obtain the following compounds.

### Reference Example 7(1)

### 5-Chloro-N-(2-chloro-5-(3,4-dihydro-1(2H)-quinolinylcarbonyl)phenyl)-2-nitrobenzamide

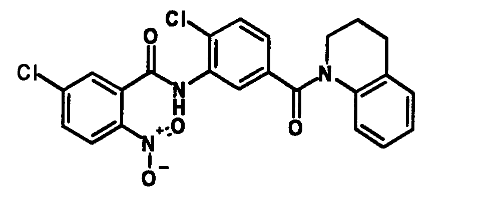

¹H-NMR (CDCl₃) δ 2.05-2.09 (2H, m), 2.86 (2H, t), 3.92 (2H, t), 6.84-7.20 (5H, m), 7.33 (1H, d), 7.60-7.64 (2H, m), 7.82 (1H, s), 8.11-8.14 (1H, m), 8.47 (1H, br s)

### Reference Example 7 (2)

### N- (2-Chloro-5- (3,4-dihydro-1 (2H) -quinolinylcarbonyl)phenyl)-2-nitrobenzamide

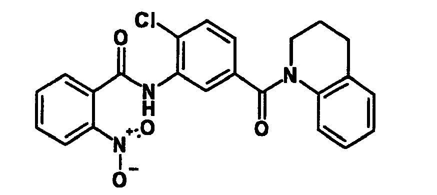

¹H-NMR (CDCl₃) δ 2.01-2.10 (2H, m), 2.85 (2H, t), 3.91 (2H, t), 6.87-7.18 (4H, m), 7.31 (1H, d), 7.62-7.85 (5H, m), 7.14 (1H, d), 8.51 (1H, br s)

### Reference Example 7(3)

### N-(2-Chloro-5-(3,4-dihydro-1(2H)-quinolinylsulfonyl)phenyl)-2-nitrobenzamide

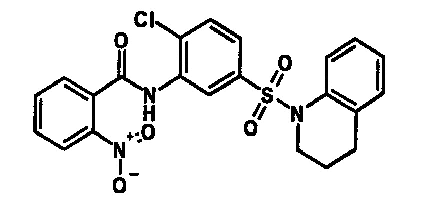

¹H-NMR (CDCl₃) δ 1.77-1.86 (2H, m), 2.56 (2H, t), 3.88-3.92 (2H, m), 7.01-7.44 (5H, m), 7.61-7.92 (5H, m), 8.16 (1H, d), 8.78-8.80 (1H, m)

### Reference Example 7(4)

### 3-Chloro-N-[2-chloro-5-(3,4-dihydroquinolin-1(2H)-ylsulfonyl)phenyl]-2-nitrobenzamide

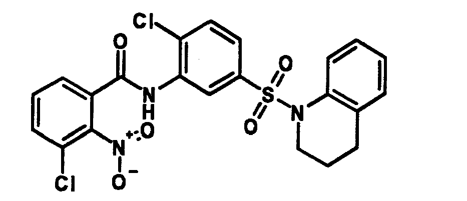

MS (ESI+, m/e) 506 (M+1)

### Reference Example 7(5)

### N-[2-Chloro-5-(3,4-dihydroquinolin-1(2H)-ylsulfonyl)phenyl]-3-methyl-2-nitrobenzamide

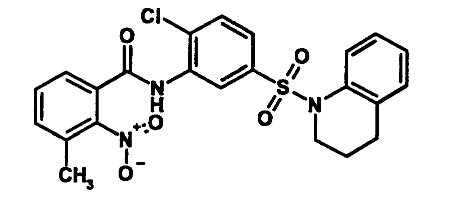

MS (ESI+, m/e) 486 (M+1)

### Reference Example 7(6)

### 4-Chloro-N-[2-chloro-5-(3,4-dihydroquinolin-1(2H)-ylsulfonyl)phenyl]-2-nitrobenzamide

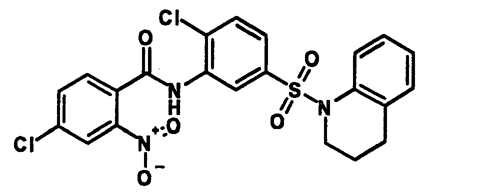

MS (ESI+, m/e) 506 (M+1)

### Reference Example 7(7)

### N-[2-Chloro-5-(3,4-dihydroquinolin-1(2H)-ylsulfonyl)phenyl]-4-fluoro-2-nitrobenzamide

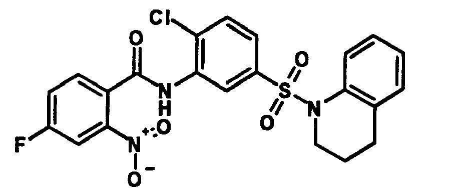

MS (ESI+, m/e) 490 (M+1)

### Reference Example 7(8)

### N-[2-Chloro-5-(3,4-dihydroquinolin-1(2H)-ylsulfonyl)phenyl]-2-nitro-4-(trifluoromethyl)benzamide

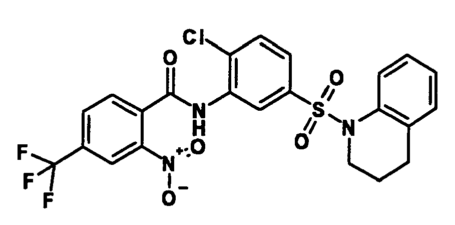

MS (ESI+, m/e) 540 (M+1)

### Reference Example 7(9)

### 5-Chloro-N-[2-chloro-5-(3,4-dihydroquinolin-1(2H)-ylsulfonyl)phenyl]-2-nitrobenzamide

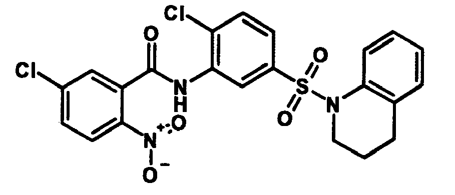

MS (ESI+, m/e) 506 (M+1)

### Reference Example 7(10)

### N-[2-Chloro-5-(3,4-dihydroquinolin-1(2H)-ylsulfonyl)phenyl]-5-methoxy-2-nitrobenzamide

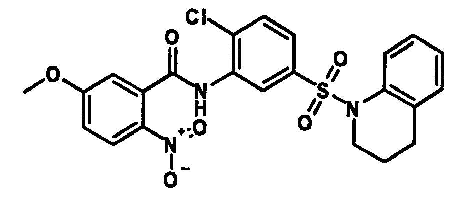

MS (ESI+, m/e) 502 (M+1)

### Reference Example 7(11)

### 5-(Acetylamino)-N-[2-chloro-5-(3,4-dihydroquinolin-1(2H)-ylsulfonyl)phenyl]-2-nitrobenzamide

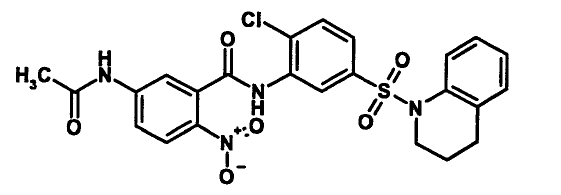

MS (ESI+, m/e) 529 (M+1)

### Reference Example 8

### 2-Amino-N-(2-chloro-5-(3,4-dihydro-1(2H)-quinolinylcarbonyl)phenyl)-5-methoxybenzamide

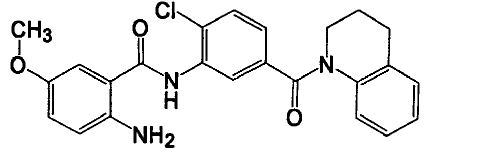

To a solution of N-(2-chloro-5-(3,4-dihydro-1(2H)-quinolinylcarbonyl)phenyl)-5-methoxy-2-nitrobenzamide (112 mg) in THF (4.80 ml), a 0.25 N solution of tin chloride (II) dehydrate in methanol (4.80 ml) was added, and the mixture was stirred for 15 hours at 50°C. Tin chloride (II) dihydrate (250 mg) was further added thereto, and the reaction mixture was stirred for 7.5 hours at 50°C. After distilling off the volatile substance under reduced pressure, a saturated sodium bicarbonate solution was added at 0°C, and the precipitates generated therefrom was added to ethyl acetate to form powder. The mixture was stirred for 16 hours at room temperature. The suspension was passed through Celite column and washed with ethyl acetate. The organic layer was separated, dried over anhydrous magnesium sulfate, and then the solvent was distilled off under reduced pressure. The residue was subjected to silica gel column chromatography, and the fraction eluted with ethyl acetate/hexane (1: 9 to 1: 1) was concentrated under reduced pressure. Thus obtained crystals were collected by filtration to give the desired product (64 mg).

¹H-NMR (CDCl₃) δ 2.08 (2H, t), 2.85 (2H, t), 3.94 (2H, t), 6.72-7.24 (8H, m), 7.51-7.53 (1H, m), 8.58 (1H, s), 8.67 (1H, br s)
MS (ESI+, m/e) 436 (M+1)

### Reference Example 9

### Ethyl 4-chloro-3-(2,4-dioxo-1,4-dihydroquinazolin-3(2H)-yl)benzoate

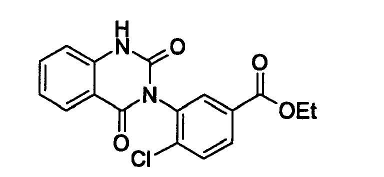

To a solution of 3-amino-4-chlorobenzoic acid (8.6 g) in ethanol (60 ml) and THF (40 ml), concentrated sulfuric acid (2.5 ml) was added, and the mixture was subjected to a reflux overnight. The solvent was distilled off under reduced pressure and ethyl acetate was added thereto. The organic layer was washed with a saturated sodium bicarbonate solution and saturated brine and dried over anhydrous sodium sulfate. The solvent thereof was then distilled off under reduced pressure to give ethyl 3-amino-4-chlorobenzoate (10 g).

To a solution of obtained ethyl 3-amino-4-chlorobenzoate (3.99 g) in acetonitrile (100 ml), methyl 2-isocyanatobenzoate (4.25 g) and DMAP (2.93 g) were added, and the mixture was stirred overnight at 50°C. Ethyl acetate was added thereto, and the organic layer was sequentially washed with 1 N hydrochloric acid, a saturated sodium bicarbonate solution and saturated brine and dried over anhydrous sodium sulfate. Then, the solvent was distilled off under reduced pressure. The residue was washed with ethanol to give the desired product (4.29 g).

¹H-NMR (DMSO-d₆) δ 1.32 (3H, t), 4.33 (2H, q), 7.20-7.30 (2H, m), 7.74 (1H, dt), 7.81 (1H, d), 7.95 (1H, dd), 8.03 (1H, dd), 8.17 (1H, d)
MS (ESI+, m/e) 297 (M+1)

### Reference Example 10

### 4-Chloro-3-(2,4-dioxo-1,4-dihydroquinazolin-3(2H)-yl)benzoic acid

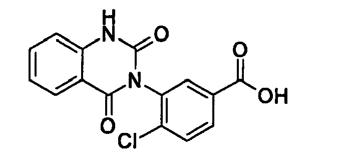

To a solution of ethyl 4-chloro-3-(2,4-dioxo-1,4-dihydroquinazolin-3(2H)-yl)benzoate (3.45 g) in ethanol (10.0 ml), THF (10.0 ml) and water (11.3 ml), an 8 N aqueous sodium hydroxide solution (3.75 ml) was added, and the mixture was stirred for 1 hour at 50°C. The reaction mixture was poured into 1 N hydrochloric acid and extracted with ethyl acetate. The organic layer was washed with 1 N hydrochloric acid and saturated brine, and dried over anhydrous sodium sulfate. Then, the solvent was distilled off under reduced pressure. The residue was washed with diethyl ether to give the desired product (2.30 g) .

¹H-NMR (DMSO-d₆) δ 7.23-7.30 (2H, m), 7.70-7.78 (1H, m), 7.79 (1H, d), 7.96 (1H, dd), 8.02 (1H, dd), 8.11 (1H, d)
MS (ESI+, m/e) 317 (M+1)

### Reference Example 11

### 4-Amino-5-chloro-N-ethyl-N-phenylthiophene-2-sulfonamide

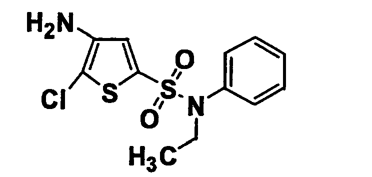

A mixture of 5-chloro-N-ethyl-4-nitro-N-phenylthiophene-2-sulfonamide (1.20 g), zinc (4.5 g), ammonium chloride (0.93 g) and methanol (300 ml) was stirred for 2 hours at 55°C. After filtering the reaction solution, the filtrate was distilled off under reduced pressure. The resulting residue was purified with silica gel column chromatography (hexane/ethyl acetate (6 : 1 to 3 : 1)) to give the desired product (0.43 g) as an oily matter.

¹H-NMR (CDCl₃) δ 1.11 (3H, t), 3.65 (2H, q), 3.72 (2H, br s), 6.79 (1H, s), 7.14-7.17 (2H, m), 7.33-7.39 (3H, m)

### Reference Example 11(1)

In the same manner as in Reference Example 11, the following compound was obtained from 4-[(5-chloro-4-nitro-2-thienyl)sulfonyl]-5,6,7,8-tetrahydro-4H-thieno[3,2-b]azepine.

### 2-Chloro-5-(5,6,7,8-tetrahydro-4H-thieno[3,2-b]azepin-4-ylsulfonyl)thiophen-3-amine

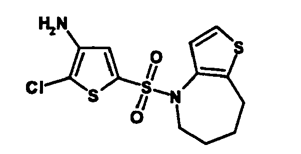

¹H-NMR (CDCl₃) δ 1.58 (2H, ddd), 1.83-1.91 (2H, m), 2.52 (2H, t), 3.68 (2H, br s), 3.73 (2H, br s), 6.86 (1H, s), 6.93 (1H, d), 7.04 (1H, d)

### Reference Example 12

### 4-Amino-3-chloro-N-ethyl-N-phenylbenzenesulfonamide

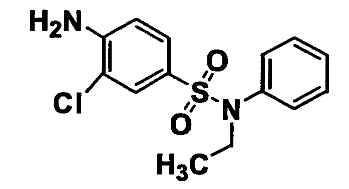

A mixture of N-(2-chloro-4-((ethyl(phenyl)amino)sulfonyl)phenyl)acetamide (1.48 g), concentrated hydrochloric acid (1 ml) and 2-propanol (20 ml) was stirred for 5 hours at 70°C. The reaction solution was distilled off under reduced pressure, poured into an aqueous sodium hydrogen carbonate solution, and then extracted twice with ethyl acetate. The collected organic layers were dried over anhydrous magnesium sulfate and passed through silica gel. Then, the solvent was distilled off under reduced pressure to give the desired product (1.35 g) as an oily matter.

¹H-NMR (CDCl₃) δ 1.07 (3H, t), 3.58 (2H, q), 4.50 (2H, br s), 6.71 (1H, d), 7.05-7.11 (2H, m), 7.22-7.35 (4H, m), 7.51 (1H, d)

### Reference Example 13

### 4-Chloro-3-((((2-cyanophenyl)amino)carbonyl)amino)-N-ethyl-N-phenylbenzenesulfonamide

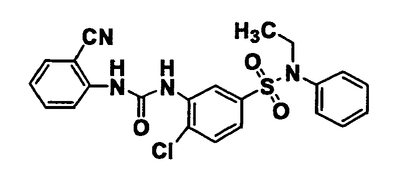

To a solution of 3-amino-4-chloro-N-ethyl-N-phenylbenzenesulfonamide (1.02 g) and DMAP (0.60 g) in THF (30 ml), 2-isocyanatobenzonitrile (0.71 g) was added at room temperature, and the mixture was stirred for 1.5 hours as it was. The reaction solution was diluted with ethyl acetate and washed with diluted hydrochloric acid and water. The resulting ethyl acetate solution was dried over anhydrous magnesium sulfate, and passed through silica gel, after which the solvent was distilled off under reduced pressure. The residue was crystallized from THF/diethyl ether/diisopropyl ether to give the desired product (0.73 g) as crystals.

¹H-NMR (CDCl₃) δ 1.09 (3H, t), 3.70 (2H, q), 7.05-7.14 (4H, m), 7.29-7.40 (4H, m), 7.55-7.61 (2H, m), 8.25 (1H, dd), 8.74 (1H, d), 9.13 (1H, s), 9.39 (1H, s)

### Reference Example 13(1)

In the same manner as in Reference Example 13, 3-amino-N-ethyl-4-methyl-N-phenylbenzenesulfonamide was reacted with 2-isocyanatobenzonitrile to give the following compound.

### 3-((((2-Cyanophenyl)amino)carbonyl)amino)-N-ethyl-4-methyl-N-phenylbenzenesulfonamide

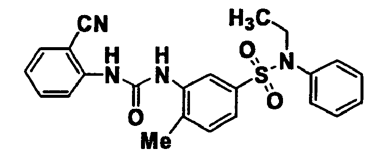

¹H-NMR (CDCl₃-DMSO-d₆) δ 1.07 (3H, t), 3.68 (2H, q), 7.05-7.14 (4H, m), 7.20 (1H, d), 7.27-7.35 (3H, m), 7.54-7.59 (2H, m), 7.31-7.34 (2H, m), 8.66 (1H, s), 8.87 (1H, s)

### Reference Example 14

### Methyl 2-((((2-chloro-5-(3,4-dihydro-1(2H)-quinolinylcarbonyl)phenyl)amino)carbonyl)amino)benzoate

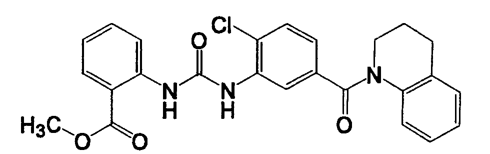

To a solution of 2-chloro-5-(3,4-dihydro-1(2H)-quinolinylcarbonyl)aniline (511 mg) in THF (4.5 ml), a solution of DMAP (331 mg) in THF (4.5 ml) and a solution of methyl 2-isocyanatobenzoate (476 mg) in THF (4.5 ml) were added, and the mixture was stirred for 16 hours at room temperature. Next, the reaction mixture was poured into 0.2 N hydrochloric acid, and extracted with dichloromethane. The organic layer was dried over anhydrous magnesium sulfate, and the solvent was distilled off under reduced pressure. The crystals, which were crystallized from ethyl acetate/toluene, were collected by filtration, washed twice with ethyl acetate and dried under reduced pressure to give the desired product (530 mg).

¹H-NMR (CDCl₃) δ 1.99-2.08 (2H, m), 2.83 (2H, t), 3.88 (2H, t), 3.92 (3H, s), 6.90-7.27 (8H, m), 7.54 (1H, ddd), 8.01 (1H, dd), 8.32 (1H, d), 8.48 (1H, d), 10.73 (1H, br s)
MS (ESI+, m/e) 464 (M+1)

### Reference Example 15

### Methyl 4-(3,4-dihydroquinolin-1(2H)-ylcarbonyl)-2-nitrobenzoate

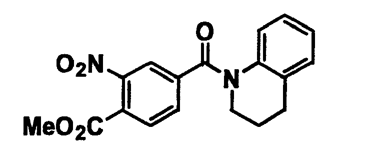

To a solution of 4-(methoxycarbonyl)-3-nitrobenzoic acid (5.12 g) and DMF (2 drops) in THF (30 ml), oxalyl chloride (2.97 ml) was added at room temperature, and the mixture was stirred for 0.5 hour as it was. The solvent of the reaction solution was distilled off under reduced pressure to obtain an oily matter.

While stirring 1,2,3,4-tetrahydroquinoline (3.33 g) and sodium hydrogen carbonate (2.86 g) in THF (40 ml), a solution of the above-obtained oily matter in THF (30 ml) was added thereto at room temperature, and the mixture was stirred overnight as it was. The reaction solution was poured into water, and extracted twice with ethyl acetate. The collected organic layers were dried over anhydrous magnesium sulfate and passed through silica gel. Then, the solvent was distilled off under reduced pressure. The resulting residue was crystallized from diethyl ether/diisopropyl ether to give the desired product (6.37 g) as crystals.

¹H-NMR (CDCl₃) δ 2.05-2.14 (2H, m), 2.87 (2H, t), 3.91 (3H, s), 3.94 (2H, t), 6.57 (1H, br s), 6.91 (1H, t), 7.07 (1H, dt), 7.21 (1H, dd), 7.55 (1H, dd), 7.61 (1H, d), 7.90 (1H, d)

### Reference Example 15(1)

In the same manner as in Reference Example 15, 3-(methoxycarbonyl)-5-nitrobenzoic acid was reacted with 1,2,3,4-tetrahydroquinoline to obtain the following compound.

### Methyl 3-(3,4-dihydroquinolin-1(2H)-ylcarbonyl)-5-nitrobenzoate

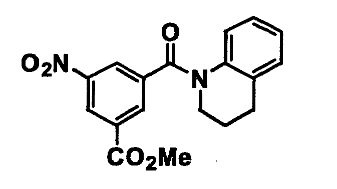

¹H-NMR (CDCl₃) δ 2.06-2.15 (2H, m), 2.90 (2H, t), 3.95 (3H, s), 3.96 (2H, t), 6.56 (1H, br s), 6.86 (1H, t), 7.05 (1H, dt), 7.22 (1H, dd), 8.28-8.32 (2H, m), 8.82 (1H, t)

### Reference Example 16

### 4-(3,4-Dihydroquinolin-1(2H)-ylcarbonyl)-2-nitrobenzoic acid

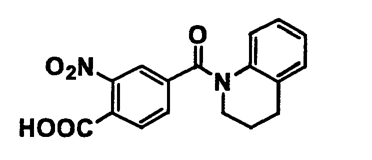

To a solution of 4-(methoxycarbonyl)-3-nitrobenzoic acid (5.12 g) and DMF (2 drops) in THF (30 ml), oxalyl chloride (2.97 ml) was added at room temperature, and the mixture was stirred for 0.5 hour as it was. The solvent of the reaction solution was distilled off under reduced pressure to give an oily matter.

While stirring 1,2,3,4-tetrahydroquinoline (3.33 g) and sodium hydrogen carbonate (2.86 g) in THF (40 ml), a solution of the above-obtained oily matter in THF (30 ml) was added thereto at room temperature, and the mixture was further stirred overnight as it was. The reaction solution was poured into water, and extracted twice with ethyl acetate. The collected organic layers were dried over anhydrous magnesium sulfate and passed through silica gel. Then, the solvent was distilled off under reduced pressure. The resulting residue was crystallized from diethyl ether/diisopropyl ether to obtain crystals (6.37 g).

A mixture of the above-obtained crystals, a 1 N aqueous sodium hydroxide solution (34 ml), THF (30 ml) and methanol (10 ml) was stirred overnight at room temperature. After acidifying the reaction solution with 1 N hydrochloric acid, the solution was extracted twice with ethyl acetate. The collected organic layers were dried over anhydrous magnesium sulfate, and the solvent was distilled off under reduced pressure. The resulting residue was crystallized from diethyl ether/ethyl acetate to give the desired product (4.96 g) as a powder.

¹H-NMR (CDCl₃) δ 2.05-2.14 (2H, m), 2.87 (2H, t), 3.94 (2H, t), 6.56 (1H, br s), 6.89-6.94 (1H, m), 7.07 (1H, dt), 7.20 (1H, d), 7.55 (1H, dd), 7.71 (1H, d), 7.83 (1H, d)

### Reference Example 17

### 4-(3,4-Dihydroquinolin-1(2H)-ylcarbonyl)-2-nitrobenzonitrile

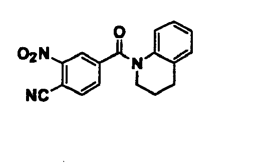

To a solution of 4-(3,4-dihydroquinolin-1(2H)-ylcarbonyl)-2-nitrobenzoic acid (1.51 g) and DMF (2 drops) in THF (40 ml), oxalyl chloride (0.61 ml) was added at room temperature, and the mixture was stirred for 2 hours as it was. The solvent of the reaction solution was distilled off under reduced pressure to obtain an oily matter.

While stirring 25 % ammonia water (1.26 g) in THF (20 ml), a solution of the above-obtained oily matter in THF (30 ml) was added thereto at room temperature, and the mixture was stirred for 1 hour as it was. The reaction solution was poured into water, and extracted twice with ethyl acetate. The collected organic layers were dried over anhydrous magnesium sulfate and passed through silica gel. The solvent was distilled off under reduced pressure to obtain a solid matter. 5 A mixture of the above-obtained solid matter, DMF (1 ml), thionyl chloride (0.51 ml), THF (20 ml) and toluene (20 ml) was stirred for 10 minutes at 80°C. The reaction solution was poured into an aqueous sodium hydrogen carbonate solution, and extracted twice with ethyl acetate. The collected organic layers were dried over anhydrous magnesium sulfate, and the solvent was distilled off under reduced pressure. The resulting residue was crystallized from ethyl acetate/diethyl ether to give the desired product (0.94 g) as crystals.

¹H-NMR (CDCl₃) δ 2.07-2.16 (2H, m), 2.88 (2H, t), 3.96 (2H, t), 6.48 (1H, br s), 6.89 (1H, t), 7.09 (1H, dt), 7.24 (1H, d), 7.65 (1H, dd), 7.78 (1H, d), 8.26 (1H, d)

### Reference Example 18

### Methyl 2-amino-4-(3,4-dihydroquinolin-1(2H)-ylcarbonyl)benzoate

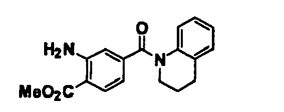

A solution of methyl 4-(3,4-dihydroquinolin-1(2H)-ylcarbonyl)-2-nitrobenzoate (3.47 g) in THF (30 ml) and methanol (30 ml) was hydrogenated overnight in presence of 10% palladium carbon (50% water content, 1 g), at room temperature and at normal pressure. The catalyst was separated by filtration, and then the filtrate was concentrated. The resulting residue was purified with silica gel column chromatography (hexane/ethyl acetate (1 : 1)), and crystallized from diisopropyl ether to give the desired product (2.89 g) as crystals.

¹H-NMR (CDCl₃) δ 1.99-2.08 (2H, m), 2.83 (2H, t), 3.84 (3H, s), 3.87 (2H, t), 5.73 (2H, br s), 6.45 (1H, dd), 6.75 (1H, d), 6.82 (1H, br s), 6.90 (1H, dt), 7.00 (1H, dt), 7.13 (1H, d), 7.70 (1H, d)

### Reference Examples 18(1) to 18(3)

In the same manner as in Reference Example 18, the corresponding nitro compounds (Reference Examples 15(1), 17 and 2(61)) were reduced to obtain the following compounds.

### Reference Example 18(1)

### Methyl 3-amino-5-(3,4-dihydroquinolin-1(2H)-ylcarbonyl)benzoate

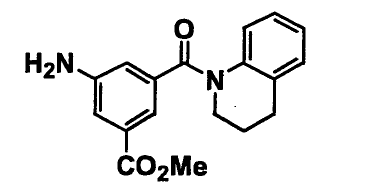

¹H-NMR (CDCl₃) δ 1.99-2.08 (2H, m), 2.85 (2H, t), 3.78 (2H, br s), 3.84 (3H, s), 3.87 (2H, t), 6.82-6.93 (3H, m), 6.99 (1H, dt), 7.13 (1H, d), 7.31 (1H, dd), 7.35 (1H, t)

### Reference Example 18(2)

### 2-Amino-4-(3,4-dihydroquinolin-1(2H)-ylcarbonyl)benzonitrile

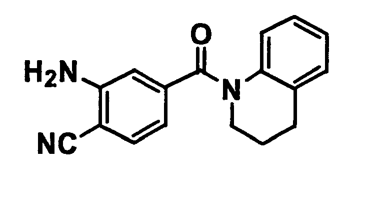

¹H-NMR (CDCl₃) δ 2.00-2.09 (2H, m), 2.83 (2H, t), 3.88 (2H, t), 4.44 (2H, s), 6.55 (1H, d), 6.73 (1H, br s), 6.84 (1H, d), 6.93 (1H, t), 7.04 (1H, t), 7.16 (1H, dd), 7.24 (1H, d)

### Reference Example 18(3)

### Methyl 1-[(3-aminophenyl)sulfonyl]-1,2,3,4-tetrahydroquinoline-6-carboxylate

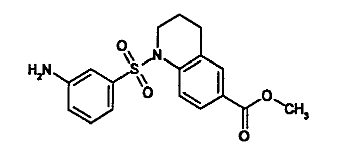

¹H-NMR (CDCl₃) δ 1.72 (2H, quintet), 2.60 (2H, t), 3.83 (2H, t), 3.83 (2H, s), 3.89 (3H, s), 6.78-6.81 (1H, m), 6.91 (1H, t), 6.95-6.98 (1H, m), 7.18 (1H, t), 7.73 (1H, s), 7.81 (1H, dd), 7.87 (1H, d)

### Reference Example 19

### [4-(3,4-Dihydroquinolin-1(2H)-ylcarbonyl)-2-nitrophenyl]methanol

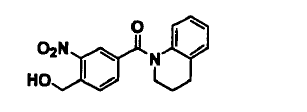

To a solution of 4-(3,4-dihydroquinolin-1(2H)-ylcarbonyl)-2-nitrobenzoic acid (3.32 g), triethylamine (1.70 ml) in THF (100 ml), with ice cooling, ethyl chlorocarbonate (1.07 ml) was added, and the mixture was stirred for 0.5 hour as it was. Sodium borohydride (0.96 g) and methanol (20 ml) were added to the mixture with ice cooling, and was stirred for 2 hours at 0°C. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (2 : 1 to 1 : 1)) to give the desired product (1.14 g) as an oily matter.

¹H-NMR (CDCl₃) δ 2.04-2.13 (2H, m), 2.67 (1H, t), 2.87 (2H, t), 3.93 (2H, t), 4.97 (2H, d), 6.59 (1H, br d), 6.88 (1H, t), 7.04 (1H, dt), 7.20 (1H, d), 7.53 (1H, dd), 7.66 (1H, d), 8.10 (1H, d)

### Reference Example 20

### 1-[4-({[tert-Butyl(dimethyl)silyl]oxy}methyl)-3-nitrobenzoyl]-1,2,3,4-tetrahydroquinoline

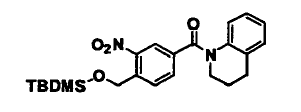

To a solution of [4-(3,4-dihydroquinolin-1(2H)-ylcarbonyl)-2-nitrophenyl]methanol (1.14 g), DMAP (44 mg), triethylamine (0.61 ml) in THF (40 ml), tert-butylchlorodimethylsilane (0.60 g) was added at room temperature, and the mixture was stirred for 1 day as it was. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (9: 1 to 6: 1))to give the desired product (1.35 g) as a solid.

¹H-NMR (CDCl₃) δ 0.12 (6H, s), 0.95 (9H, s), 2.04-2.13 (2H, m), 2. 87 (2H, t), 3.94 (2H, t), 5.07 (2H, s), 6.62 (1H, br d), 6.88 (1H, t), 7.04 (1H, dt), 7. 17 (1H, d), 7. 59 (1H, dd), 7. 81 (1H, d), 8.11 (1H, d)

### Reference Example 21

### Methyl 2-[(2-aminobenzoyl)amino]-4-(3,4-dihydroquinolin-1(2H)-ylcarbonyl)benzoate

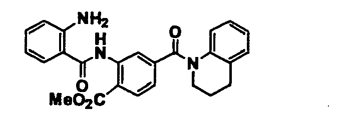

1) While stirring methyl 2-amino-4-(3,4-dihydroquinolin-1(2H)-ylcarbonyl)benzoate (2.62 g) and sodium hydrogen carbonate (1.42 g) in THF (60 ml), 2-nitrobenzoyl chloride (1.88 g) was added at room temperature, and the mixture was stirred overnight as it was. After concentrating the reaction solution, water and diisopropyl ether were added thereto, and the reaction mixture was stirred. Formed precipitates were collected and washed with water, methanol and diisopropyl ether to obtain methyl 4-(3,4-dihydroquinolin-1(2H)-ylcarbonyl)-2-[(2-nitrobenzoyl)amino]benzoate (3.78 g) as crystals.
2) A solution of methyl 4-(3,4-dihydroquinolin-1(2H)-ylcarbonyl)-2-[(2-nitrobenzoyl)amino]benzoate (1.12 g) in THF (20 ml), methanol (10 ml) and DMF (10 ml) was hydrogenated overnight in presence of 10% palladium carbon (50% water content, 0.5 g) at room temperature at normal pressure. The catalyst was separated by filtration, and the filtrate was concentrated. The resulting residue was crystallized from ethyl acetate/diisopropyl ether to give the desired product (0.86 g) as a powder.

¹H-NMR (CDCl₃) δ 2.02-2.11 (2H, m), 2.87 (2H, t), 3.91 (2H, t), 3.94 (3H, s), 5.73 (2H, s), 6.70 (1H, d), 6.76 (1H, dt), 6.90-7.04 (4H, m), 7.16 (1H, d), 7.25 (1H, dt), 7.68 (1H, d), 7.97 (1H, d), 8.98 (1H, d), 11.77 (1H, s)

### Reference Example 21(1)

In the same manner as in Reference Example 21, the following compound was obtained from 2-amino-4-(3,4-dihydroquinolin-1(2H)-ylcarbonyl)benzonitrile.

### 2-Amino-N-[2-cyano-5-(3,4-dihydroquinolin-1(2H)-ylcarbonyl)phenyl]benzamide

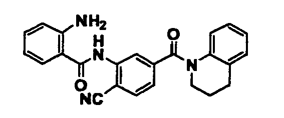

¹H-NMR (CDCl₃) δ 2.03-2.11 (2H, m), 2.86 (2H, t), 3.91 (2H, t), 5.70 (1H, br s), 6.71-6.76 (2H, m), 6.83 (1H, br s), 6.93 (1H, t), 7.01-7.06 (2H, m), 7.17 (1H, d), 7.29 (1H, dt), 7.49 (1H, d), 7.53 (1H, dd), 8.42 (1H, s), 8.60 (1H, d)

### Reference Example 22

### 2-Chloro-N-[2-chloro-5-(3,4-dihydroquinolin-1(2H)-ylsulfonyl)phenyl]-6-nitrobenzamide

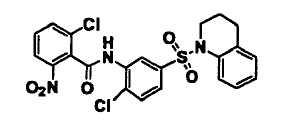

To a solution of 2-chloro-6-nitrobenzoic acid (2.60 g) and DMF (2 drops) in THF (30 ml), oxalyl chloride (1.24 ml) was added at room temperature, and the mixture was stirred for 2 hours as it was. The solvent of the reaction solution was distilled off under reduced pressure to obtain an oily matter.

While stirring 2-chloro-5-(3,4-dihydro-1(2H)-quinolinylsulfonyl)aniline (3.07 g) and sodium hydrogen carbonate (1.60 g) in THF (30 ml), a solution of the above-obtained oily matter in THF (10 ml) was added at room temperature, and the mixture was stirred overnight as it was, and further stirred for 3 days at 60°C. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (2 : 1 to 1 : 1)) to give the desired product (0.43 g) as foam.

¹H-NMR (CDCl₃) δ 1.77-1.86 (2H, m), 2.56 (2H, t), 3.91 (2H, t), 7.02-7.10 (2H, m), 7.20 (1H, dt), 7.37 (1H, dd), 7.44 (1H, d), 7.61 (1H, t), 7. 78-7. 82 (2H, m), 7. 86 (1H, s), 8.17 (1H, dd), 8.78 (1H, d)

### Reference Example 23

### 2-Amino-6-chloro-N-[2-chloro-5-(3,4-dihydroquinolin-1(2H)-ylsulfonyl)phenyl]benzamide

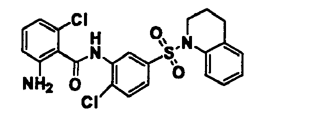

To a mixture of 2-chloro-N-[2-chloro-5-(3,4-dihydroquinolin-1(2H)-ylsulfonyl)phenyl]-6-nitrobenzamide (0.43 g), nickel bromide (II) (9 mg), methanol (10 ml) and THF (10 ml), sodium borohydride (96 mg) was added at room temperature, and the resulting mixture was stirred for 15 minutes as it was. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (2 : 1 to 1 : 1)) to give the desired product (0.20 g) as an oily matter. Also, 2-amino-6-chloro-N-[3-(3,4-dihydroquinolin-1(2H)-ylsulfonyl)phenyl]benzamide (75 mg) as an oily matter was obtained as a by-product.

¹H-NMR (CDCl₃) δ 1.77-1.85 (2H, m), 2.56 (2H, t), 3.90 (2H, t), 4.82 (1H, s), 6.64 (1H, dd), 6.77 (1H, d), 7.01-7.15 (3H, m), 7.20 (1H, dt), 7.30 (1H, dd), 7.42 (1H, d), 7.79 (1H, d), 8.40 (1H, s), 8.94 (1H, s)

### Reference Example 24

### 2-Amino-6-chloro-N-[3-(3,4-dihydroquinolin-1(2H)-ylsulfonyl)phenyl]benzamide

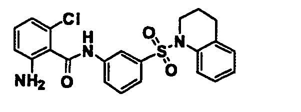

The desired product was obtained in Reference Example 23 as a by-product.

¹H-NMR (CDCl₃) δ 1.66-1.75 (2H, m), 2.48 (2H, t), 3.84 (2H, t), 4.73 (1H, s), 6.63 (1H, dd), 6.76 (1H, d), 7.00-7.14 (3H, m), 7.20 (1H, dt), 7.37-7.45 (2H, m), 7.71 (1H, s), 7.77 (1H, s), 7.78 (1H, d), 7.98 (1H, d)

### Reference Example 25

### Methyl 2-({[2-chloro-5-(3,4-dihydroquinolin-1(2H)-ylsulfonyl)phenyl]amino}carbonyl)-3-nitrobenzoate

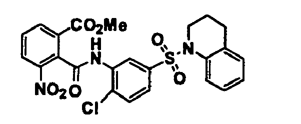

To a solution of 2-(methoxycarbonyl)-6-nitrobenzoic acid (3.05 g) and DMF (2 drops) in THF (50 ml), oxalyl chloride (1.77 ml) was added at room temperature, and the mixture was stirred for 1 hour as it was. The solvent of the reaction solution was distilled off under reduced pressure to obtain a solid.

While stirring 2-chloro-5-(3,4-dihydro-1(2H)-quinolinylsulfonyl)aniline (2.18 g) and sodium hydrogen carbonate (1.71 g) in THF (30 ml), a solution of the above-obtained solid in THF (20 ml) was added at room temperature, and the mixture was stirred for 4 days as it was. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (2 : 1 to 1 : 1)) to give the desired product (2.49 g) as foam.

¹H-NMR (CDCl₃) δ 1.79-1.88 (2H, m), 2.57 (2H, t), 3.87 (3H, s), 3.92 (2H, t), 7.02-7.09 (2H, m), 7.19 (1H, t), 7.32 (1H, dd), 7.41 (1H, d), 7.73-7.82 (3H, m), 8.37-8.41 (2H, m), 8.84 (1H, d)

### Reference Example 26

### N-[2-Chloro-5-(3,4-dihydroquinolin-1(2H)-ylsulfonyl)phenyl]-2-(hydroxymethyl)-6-nitrobenzamide

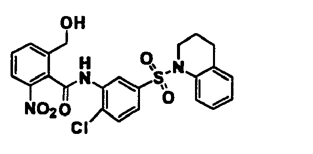

To a solution of methyl 2-({[2-chloro-5-(3,4-dihydroquinolin-1(2H)-ylsulfonyl)phenyl]amino}carbonyl)-3-nitrobenzoate (1.48 g) in toluene (20 ml) and dichloromethane (20 ml), a 1.5 N solution of diisobutylaluminum hydride in toluene (7.43 ml) was added dropwise at -78°C, and the mixture was stirred for 0.5 hour at -78°C. Methanol (2 ml) was added dropwise to the reaction solution at -78°C, and the reaction mixture was stirred for 0.5 hour as it was. A small amount of water was then added thereto at -78°C, and the temperature was gradually elevated to room temperature while stirring, where the mixture was stirred for 1 hour at room temperature. Formed precipitates were separated by filtration and washed with ethyl acetate. The solvent of the collected filtrate was distilled off under reduced pressure. The resulting residue was purified with silica gel column chromatography (hexane/ethyl acetate (1 : 1 to 1 : 2)) to give the desired product (0.26 g) as an oily matter.

¹H-NMR (CDCl₃) δ 1.76-1.84 (2H, m), 2.43 (1H, t), 2.56 (2H, t), 3.90 (2H, t), 4.77 (2H, d), 7.03-7.11 (2H, m), 7.20 (1H, dt), 7.36 (1H, dd), 7.43 (1H, d), 7.66 (1H, t), 7.79 (1H, d), 7.87 (1H, d), 8.03 (1H, s), 8.15 (1H, d), 8.76 (1H, d)

### Reference Example 27

### 2-({[tert-Butyl(dimethyl)silyl]oxy}methyl)-N-[2-chloro-5-(3,4-dihydroquinolin-1(2H)-ylsulfonyl)phenyl]-6-nitrobenzamide

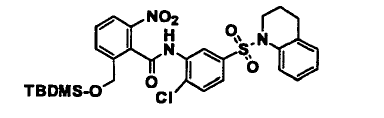

To a solution of N-[2-chloro-5-(3,4-dihydroquinolin-1(2H)-ylsulfonyl)phenyl]-2-(hydroxymethyl)-6-nitrobenzamide (0.26 g), DMAP (6 mg) and triethylamine (0.11 ml) in THF (20 ml), tert-butylchlorodimethylsilane (85 mg) was added at room temperature, and the mixture was stirred for 1 day as it was. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (9 : 1 to 3 : 1)) to give the desired product (0.12 g) as an oily matter.

¹H-NMR (CDCl₃) δ 0.07 (6H, s), 0.89 (9H, s), 1.77-1.85 (2H, m), 2.56 (2H, t), 3.91 (2H, t), 4.81 (2H, s), 7.01-7.09 (2H, m), 7.19 (1H, dt), 7.34 (1H, dd), 7.41 (1H, d), 7.64 (1H, t), 7.78 (1H, d), 7.85 (1H, br s), 7.92 (1H, d), 8.12 (1H, d), 8.82 (1H, d)

### Reference Example 28

### Methyl 5-(2,4-dioxo-1,4-dihydroquinazolin-3(2H)-yl)-2-furoate

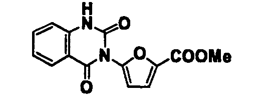

To a solution of methyl 5-amino-2-furoate (1.01 g) and DMAP (1.75 g) in THF (40 ml), methyl 2-isocyanatobenzoate (1.90 g) was added at room temperature, and the mixture was stirred for 2 days as it was. The solvent of the reaction solution was then distilled off under reduced pressure. The residue was purified with silica gel column chromatography (hexane/ethyl acetate (1: 1) to ethyl acetate), and crystallized from ethyl acetate/diethyl ether to give the desired product (0.98 g) as crystals.

¹H-NMR (CDCl₃-DMSO-d₆) δ 3.90 (3H, s), 6.57 (1H, d), 7.18-7.23 (2H, m), 7.32 (1H, d), 7.61 (1H, dt), 8.08 (1H, dd), 11.38 (1H, br s)

### Reference Example 29

### Methyl 5-(2,4-dioxo-1,4-dihydroquinazolin-3(2H)-yl)thiophene-3-carboxylate

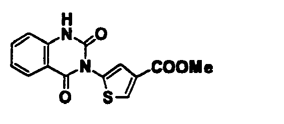

To a mixture of methyl 5-nitrothiophene-3-carboxylate (3.38 g), ammonium chloride (4.83 g) and methanol (100 ml), zinc (11.8 g) was added portionwise at 60°C, and the mixture was stirred for 30 minutes at 60°C. The insolubles were removed by subjecting the reaction solution to filtration, and the filtrate was distilled off under reduced pressure. The resulting residue was passed through silica gel column chromatography (hexane/ethyl acetate (6 : 1 to 2 : 1)) to obtain a solid.

To a solution of the above-obtained solid and DMAP (1.92 g) in THF (50 ml), methyl 2-isocyanatobenzoate (2.09 g) was added at room temperature, and the mixture was stirred for 1 day as it was. A 28% solution of sodium methoxide in methanol (1.52 g) was added to the reaction solution at room temperature, and the mixture was stirred for 10 minutes as it was. The reaction solution was poured into diluted hydrochloric acid, and extracted twice with ethyl acetate. The collected organic layers were dried over anhydrous magnesium sulfate and passed through silica gel, and the solvent was distilled off under reduced pressure. The resulting residue was crystallized from ethyl acetate/diethyl ether to give the desired product (1.36 g) as a powder.

¹H-NMR (CDCl₃-DMSO-d₆) δ 3.85 (3H, s), 7.17-7.24 (2H, m), 7.47 (1H, d), 7.59 (1H, ddd), 8.09 (1H, dd), 8.15 (1H, d), 11.39 (1H, s)

### Reference Example 30

### 5-(2,4-Dioxo-1,4-dihydroquinazolin-3(2H)-yl)-2-furoic acid

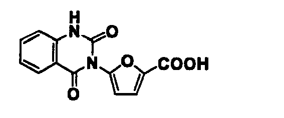

A mixture of methyl 5-(2,4-dioxo-1,4-dihydroquinazolin-3(2H)-yl)-2-furoate (0.82 g), lithium hydroxide monohydrate (0.36 g), THF (10 ml) and water (3 ml) was stirred for 0.5 hour at room temperature. The reaction solution was concentrated, and then diluted with water. 1 N Hydrochloric acid (8 ml) was added thereto, and the reaction mixture was stirred. Formed precipitates were collected and washed with water and diethyl ether to give title product (0.77 g) as a powder.

¹H-NMR (CDCl₃) δ 6.55 (1H, d), 7. 18-7.25 (2H, m), 7.29 (1H, d), 7.61 (1H, dt), 8.07 (H, d), 11.43 (1H, s)

### Reference Example 30(1)

In the same manner as in Reference Example 30, the following compound was obtained from methyl 5-(2,4-dioxo-1,4-dihydroquinazolin-3(2H)-yl)thiophene-3-carboxylate.

### 5-(2,4-dioxo-1,4-dihydroquinazolin-3(2H)-yl)thiophene-3-carboxylic acid

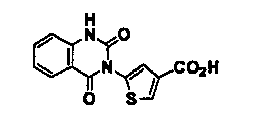

¹H-NMR (DMSO-d₆) δ 7.20-7.25 (2H, m), 7.36 (1H, d), 7.70 (1H, dt), 7.93 (1H, d), 8.28 (1H, d), 11.64 (1H, s), 12.79 (1H, s)

### Reference Example 31

### Methyl 5-[5-({[tert-butyl(dimethyl)silyl]oxy}methyl)-2,4-dioxo-1,4-dihydroquinazolin-3(2H)-yl]thiophene-3-carboxylate

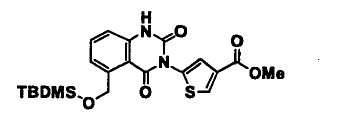

1) To a mixture of methyl 5-nitrothiophene-3-carboxylate (6.52 g), ammonium chloride (9.32 g) and methanol (150 ml), zinc (22.8 g) was added portionwise at 55°C , and the mixture was stirred for 30 minutes at 55°C. The insolubles were removed by subjecting the reaction solution to filtration, and the filtrate was distilled off under reduced pressure. The resulting residue was passed through silica gel column chromatography (hexane/ethyl acetate (6 : 1 to 2 : 1)) to give methyl 5-aminothiophene-3-carboxylate (1.34 g) as a solid.
2) To a solution of 7-amino-2-benzofuran-1(3H)-one (1.46 g) and triethylamine (2.27 ml) in THF (30 ml), a solution of triphosgene (0.97 g) in THF (10 ml) was added dropwise at -20°C, and the mixture was stirred for 0.5 hour at room temperature. The reaction solution was diluted with ethyl acetate, and the precipitates were separated by filtration. The solvent of the filtrate was distilled off under reduced pressure to obtain a wet solid.

To a solution of methyl 5-aminothiophene-3-carboxylate (1.28 g) and DMAP (1.99 g) in THF (50 ml), a solution of the above-obtained solid in THF (20 ml) was added at room temperature, and the mixture was stirred overnight as it was. The reaction solution was diluted with water and ethyl acetate, and stirred. Formed precipitates were collected, and washed with water, ethyl acetate and diethyl ether to obtain a powder.

While stirring the above-obtained powder in methanol (20 ml) and THF (30 ml), a 28% solution of sodium methoxide in methanol (0.77 g) was added at room temperature, and the mixture was stirred for 3 days at room temperature. The reaction solution was poured into diluted hydrochloric acid. Formed precipitates were collected, and washed with water to obtain a powder.

To a solution of the above-obtained powder and imidazole (0.83 g) in DMF (20 ml), tert-butylchlorodimethylsilane (0.61 g) was added at room temperature, and the mixture was stirred overnight as it was. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (3 : 1 to 1 : 1)) to give the desired product (0.73 g) as a solid.

¹H-NMR (CDCl₃) δ 0.12 (6H, s), 0.97 (9H, s), 3.86 (3H, s), 5.25 (2H, s), 6.92 (1H, dd), 7.47 (1H, d), 7.64 (1H, t), 7.70 (1H, dd), 8.18 (1H, d), 8.70 (1H, s)

### Reference Example 32

### 5-[5-(Hydroxymethyl)-2,4-dioxo-1,4-dihydroquinazolin-3(2H)-yl]thiophene-3-carboxylic acid

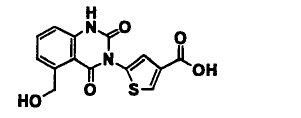

To a solution of methyl 5-[5-({[tert-butyl(dimethyl)silyl]oxy}methyl)-2,4-dioxo-1,4-dihydroquinazolin-3(2H)-yl]thiophene-3-carboxylate (0.67 g) in THF (30 ml), a 1 N solution of tetrabutylammonium fluoride in THF (2.25 ml) was added at room temperature, and the mixture was stirred for 2 hours as it was. Lithium hydroxide monohydrate (0.19 g) and water (10 ml) were added to the mixture, and the reaction mixture was stirred for 5 hours at room temperature. The reaction solution was poured into water, and the reaction mixture was acidified with 1 N hydrochloric acid, and then extracted twice with ethyl acetate. The collected organic layers were dried over anhydrous magnesium sulfate, and the solvent was distilled off under reduced pressure. The resulting residue was crystallized from ethyl acetate/diethyl ether to give the desired product (0.44 g) as crystals.

¹H-NMR (DMSO-d₆) δ 4.94 (2H, d), 5.27 (1H, t), 7. 10 (1H, d), 7.33 (1H, d), 7.48 (1H, d), 7.66 (1H, t), 8.27 (1H, s), 11.57 (1H, s), 12.73 (1H, br s)

### Reference Example 33

### Methyl 5-(3,4-dihydroquinolin-1(2H)-ylsulfonyl)-2-furoate

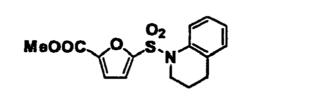

To a solution of methyl 5-(chlorosulfonyl)-2-furoate (0.90 g) and 1,2,3,4-tetrahydroquinoline (0.59 g) in THF (50 ml), pyridine (2 ml) and DMAP (10 mg) were added, and the mixture was stirred for 3 hours at 50°C. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (6 : 1 to 3 : 1)) to give the desired product (0.94 g) as an oily matter.

¹H-NMR (CDCl₃) δ 1.90-1.99 (2H, m), 2.67 (2H, t), 3.89 (3H, s), 3.94 (2H, t), 6.89 (1H, d), 7.02-7.10 (3H, m), 7.12-7.18 (1H, m), 7.66 (1H, d)

### Reference Examples 33 (1) to 33(2)

In the same manner as in Reference Example 33, the corresponding sulfonyl chlorides (commercially available or conventional) were reacted with 1,2,3,4-tetrahydroquinoline to obtain the following compounds.

### Reference Example 33 (1)

### Methyl 5-(3,4-dihydroquinolin-1(2H)-ylsulfonyl)-2-methyl-3-furoate

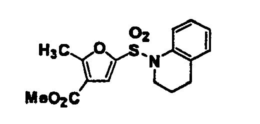

¹H-NMR (CDCl₃) δ 1.86-1.94 (2H, m), 2.57 (3H, s), 2.68 (2H, t), 3.82 (3H, s), 3.87 (2H, t), 7.05-7.11 (2H, m), 7.15-7.20 (2H, m), 7.67 (1H, d)

### Reference Example 33(2)

### Methyl 4-(3,4-dihydroquinolin-1(2H)-ylsulfonyl)-3-methoxythiophene-2-carboxylate

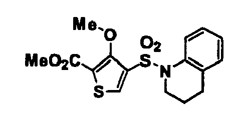

¹H-NMR (CDCl₃) δ 1.86-1.94 (2H, m), 2.64 (2H, t), 3.86 (3H, s), 3.90 (3H, s), 3.96 (2H, t), 7.03-7.04 (2H, m), 7.12-7.17 (1H, m), 7.70 (1H, d), 8.12 (1H, s)

### Reference Example 34

### Methyl 5-(3,4-dihydroquinolin-1(2H)-ylsulfonyl)-2-methylthiophene-3-carboxylate

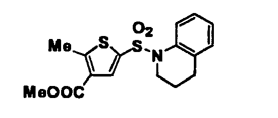

To a solution of methyl 2-methylthiophene-3-carboxylate (3.67 g) in chloroform (10 ml), chlorosulfonic acid (6.25 ml) was added dropwise with ice cooling, and the mixture was stirred for 20 minutes at room temperature. The reaction solution was poured onto crushed ice, and extracted twice with ethyl acetate. The collected organic layers were dried over anhydrous magnesium sulfate, and the solvent was distilled off under reduced pressure to obtain an oily matter.

To a solution of 1,2,3,4-tetrahydroquinoline (3.44 g), pyridine (3.80 ml) and DMAP (0.29 g) in THF (30 ml), a solution of the above-obtained oily matter in THF (10 ml) was added at room temperature, and the mixture was stirred overnight as it was. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (9 : 1 to 6 : 1)) to give the desired product (0.93 g) as an oily matter.

¹H-NMR (CDCl₃) δ 1. 74-1. 83 (2H, m), 2.57 (2H, t), 2.69 (3H, s), 3.83 (3H, s), 3.84 (2H, t), 7.04 (1H, dd), 7.10 (1H, dt), 7.20 (1H, dt), 7.71 (1H, s), 7. 76 (1H, dd)

### Reference Example 34(1)

In the same manner as in Reference Example 34, the following compound was obtained from methyl 3-methylthiophene-2-carboxylate.

### Methyl 4-(3,4-dihydroquinolin-1(2H)-ylsulfonyl)-3-methylthiophene-2-carboxylate

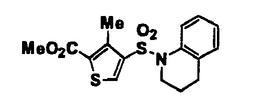

¹H-NMR (CDCl₃) δ 1.68-1.80 (2H, m), 2.34 (3H, s), 2.61 (2H, t), 3.80 (2H, t), 3.87 (3H, s), 7.03-7.20 (3H, m), 7.59 (1H, d), 8.07 (1H, s)

### Reference Example 35

### 5-(3,4-Dihydroquinolin-1(2H)-ylsulfonyl)-2-furoic acid

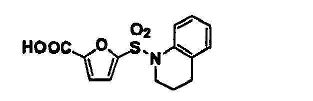

A mixture of methyl 5-(3,4-dihydroquinolin-1(2H)-ylsulfonyl)-2-furoate (0.94 g), a 1 N aqueous sodium hydroxide solution (5.86 ml), THF (10 ml) and methanol (3 ml) was stirred overnight at room temperature. The reaction solution was acidified with 1 N hydrochloric acid, and then extracted twice with ethyl acetate. The collected organic layers were dried over anhydrous magnesium sulfate, and the solvent was distilled off under reduced pressure. The resulting residue was crystallized from diethyl ether/diisopropyl ether to give the desired product (0.76 g) as a powder.

¹H-NMR (CDCl₃) δ 1.92-2. 01 (2H, m), 2.69 (2H, t), 3.96 (2H, t), 6.94 (1H, d), 7. 04-7.11 (2H, m), 7.14-7.20 (1H, m), 7.22 (1H, d), 7.68 (1H, d)

### Reference Examples 35 (1) to 35(4)

In the same manner as in Reference Example 35, the following compounds were obtained by hydrolyzing the compounds of Reference Examples 33(1), 33(2), 34 and 34(1).

### Reference Example 35(1)

### 5-(3,4-Dihydroquinolin-1(2H)-ylsulfonyl)-2-methyl-3-furoic acid

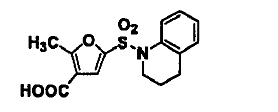

¹H-NMR (CDCl₃) δ 1.87-1.95 (2H, m), 2.59 (3H, s), 2.69 (2H, t), 3.88 (2H, t), 7.05-7.11 (2H, m), 7.15-7.21 (2H, m), 7.67 (1H, d)

### Reference Example 35(2)

### 4-(3,4-Dihydroquinolin-1(2H)-ylsulfonyl)-3-methoxythiophene-2-carboxylic acid

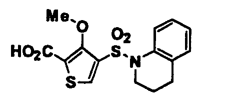

¹H-NMR (CDCl₃) δ 1. 86-1.94 (2H, m), 2.64 (2H, t), 3.92 (3H, s), 3.96 (2H, t), 7.03-7.07 (2H, m), 7.12-7.17 (1H, m), 7.69 (1H, d), 8.25 (1H, s)

### Reference Example 35(3)

### 5-(3,4-Dihydroquinolin-1(2H)-ylsulfonyl)-2-methylthiophene-3-carboxylic acid

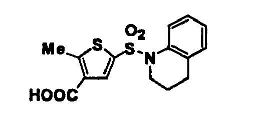

¹H-NMR (CDCl₃) δ 1. 76-1. 84 (2H, m), 2.58 (2H, t), 2.71 (3H, s), 3.85 (2H, t), 7.05 (1H, dd), 7. 11 (1H, dt), 7.21 (1H, dt), 7.75 (1H, s), 7.76 (1H, dd)

### Reference Example 35(4)

### 4-(3,4-Dihydroquinolin-1(2H)-ylsulfonyl)-3-methylthiophene-2-carboxylic acid

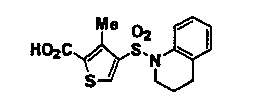

¹H-NMR (CDCl₃) δ 1.71-1.79 (2H, m), 2.34 (3H, s), 2.61 (2H, t), 3.81 (2H, t), 7.04-7.21 (3H, m), 7.58 (1H, d), 8.13 (1H, s)

### Reference Example 36

### N-[5-(3,4-Dihydroquinolin-1(2H)-ylsulfonyl)-4-methyl-1,3-thiazol-2-yl]acetamide

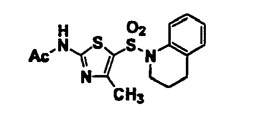

While stirring 1,2,3,4-tetrahydroquinoline (0.71 g) and sodium hydrogen carbonate (0.81 g) in THF (50 ml) and DMF (10 ml), 2-(acetylamino)-4-methyl-1,3-thiazol-5-sulfonyl chloride (1.23 g) was added at room temperature, and the mixture was stirred overnight at 60°C. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (2 : 1 to 1 : 1)), and crystallized from diisopropyl ether to give the desired product (0.31 g) as crystals.

¹H-NMR (CDCl₃) δ 1.78-1.86 (2H, m), 2.15 (3H, s), 2.24 (3H, s), 2.57 (2H, t), 3.88 (2H, t), 7.04 (1H, d), 7.10 (1H, dt), 7.16-7.22 (1H, m), 7.74 (1H, dd), 9.07 (1H, br s)

### Reference Example 37

### 6-Chloro-3-(3,4-dihydroquinolin-1(2H)-ylsulfonyl)-2-methylbenzamide

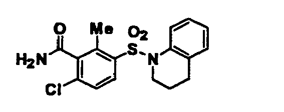

Chlorosulfonic acid (11.0 ml) was added dropwise to 2-chloro-6-methylbenzonitrile (5.01 g) with ice cooling, and the mixture was stirred for 3 hours at 100°C. After cooling, the reaction solution was poured onto crushed ice, and extracted twice with ethyl acetate. The collected organic layers were dried over anhydrous magnesium sulfate, and the solvent was distilled off under reduced pressure to obtain an oily matter.

To a solution of 1,2,3,4-tetrahydroquinoline (4.83 g), triethylamine (5.51 ml) and DMAP (0.40 g) in THF (100 ml) and pyridine (15 ml), a solution of the above-obtained oily matter in THF (30 ml) was added at room temperature, and the mixture was stirred for 2 hours at 65°C. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (2 : 1 to 1 : 1)), and crystallized from ethyl acetate/diethyl ether/diisopropyl ether to give the desired product (3.97 g) as crystals.

¹H-NMR (CDCl₃) δ 1.84-1.92 (2H, m), 2.48 (3H, s), 2.74 (2H, t), 3.78 (2H, t), 5.78 (1H, br s), 6. O1 (1H, br s), 7.03-7.12 (3H, m), 7.33 (1H, d), 7.36 (1H, d), 7.88 (1H, d)

### Reference Example 38

### 6-Chloro-3-(3,4-dihydroquinolin-1(2H)-ylsulfonyl)-2-methylaniline

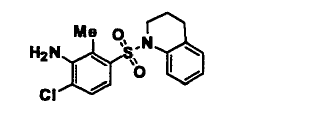

To a solution of sodium hydroxide (0.51 g) in water (5 ml), bromine (0.17 ml) was added dropwise with ice cooling, and the mixture was stirred for 10 minutes as it was. 6-Chloro-3-(3,4-dihydroquinolin-1(2H)-ylsulfonyl)-2-methylbenzamide (0.78 g) was further added to the mixture with ice cooling, and then stirred for 45 minutes at 70°C. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (3 : 1)), and crystallized from diisopropyl ether/hexane to give the desired product (0.49 g) as crystals.

¹H-NMR (CDCl₃) δ 1.76-1.84 (2H, m), 2.17 (3H, s), 2.67 (2H, t), 3.77 (2H, t), 4.23 (2H, br s), 7.01-7.13 (3H, m), 7.22 (1H, d), 7.30 (1H, d), 7.44 (1H, d)

### Reference Example 39

### 1-[2-(Trimethylsilyl)ethyl]2-methyl 3-nitrophthalate

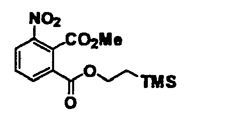

To a solution of 2-(methoxycarbonyl)-3-nitrobenzoic acid (13.7 g) and DMF (2 drops) in THF (100 ml), oxalyl chloride (10.6 ml) was added at room temperature, and the mixture was stirred for 1 hour as it was. The solvent of the reaction solution was distilled off under reduced pressure to obtain a solid.

To a solution of 2-(trimethylsilyl)ethanol (7.89 g) and triethylamine (12.7 ml) in THF (50 ml), a solution of the above-obtained solid in THF (50 ml) was added dropwise at room temperature, and the mixture was stirred overnight as it was. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (6 : 1)) to give the desired product (19.4 g) as an oily matter.

¹H-NMR (CDCl₃) δ 0.08 (9H, s), 1.09-1.18 (2H, m), 4.02 (3H, s), 4.39-4.48 (2H, m), 7.68 (1H, t), 8.31-8.39 (2H, m)

### Reference Example 40

### Dimethyl 3-aminophthalate

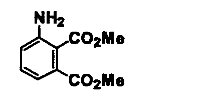

A solution of dimethyl 3-nitrophthalate (7.43 g) in THF (30 ml) and methanol (20 ml) was hydrogenated overnight in presence of 10% palladium carbon (50% water content, 2 g) at room temperature at normal pressure. The catalyst was separated by filtration, and the filtrate was concentrated. The resulting residue was purified with silica gel column chromatography (hexane/ethyl acetate (3: 1 to 1: 1)) to give the desired product (6.66 g) as an oily matter.

¹H-NMR (CDCl₃) δ 3.85 (3H, s), 3.86 (3H, s), 5.20 (2H, br s), 6.78 (1H, dd), 6.90 (1H, dd), 7.24 (1H, t)

### Reference Example 40(1)

In the same manner as in Reference Example 40, the following compound was obtained from 1-[2-(trimethylsilyl)ethyl]2-methyl 3-nitrophthalate.

### 1-[2-(Trimethylsilyl)ethyl]2-methyl 3-aminophthalate

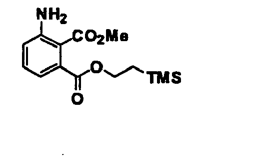

¹H-NMR (CDCl₃) δ 0.06 (9H, s), 1.05-1. 11 (2H, m), 3.83 (3H, s), 4.31-4.36 (2H, m), 5. 16 (2H, br s), 6.76 (1H, dd), 6.89 (1H, dd), 7.23 (1H, dd)

### Reference Example 41

### Methyl 2-({[tert-butyl(dimethyl)silyl]oxy}methyl)-6-nitrobenzoate

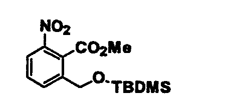

To a solution of 2-(methoxycarbonyl)-3-nitrobenzoic acid (93.9 g) in THF (300 ml), a 1 N solution of borane/THF complex in THF (500 ml) was added with ice cooling, and the mixture was stirred for 1 day at room temperature. Water was added dropwise to the reaction solution, and an aqueous ammonium chloride solution was further added dropwise thereto. The mixture was stirred for 5 minutes, and then extracted twice with ethyl acetate. The collected organic layers were dried over anhydrous magnesium sulfate, and the solvent was distilled off under reduced pressure to obtain an oily matter.

To a solution of the above-obtained oily matter and imidazole (46.8 g) in DMF (500 ml), tert-butylchlorodimethylsilane (94.2 g) wad added at room temperature, and the mixture was stirred overnight as it was. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (20 : 1 to 9 : 1)) to give the desired product (115 g) as an oily matter.

¹H-NMR (CDCl₃) δ 0.10 (6H, s), 0.93 (9H, s), 3.93 (3H, s), 4.78 (2H, s), 7.57 (1H, t), 7.89 (1H, dd), 8.00 (1H, d)

### Reference Example 42

### Methyl 2-amino-6-({[tert-butyl(dimethyl)silyl]oxy}methyl)benzoate

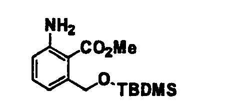

A solution of methyl 2-({[tert-butyl(dimethyl)silyl]oxy}methyl)-6-nitrobenzoate (23.3 g) in THF (100 ml) was hydrogenated for 1 day in presence of platinum dioxide (0.17 g) at room temperature at normal pressure. The catalyst was separated by filtration, and the filtrate was concentrated. The resulting residue was purified with silica gel column chromatography (hexane/ethyl acetate (9 : 1 to 6 : 1))to give the desired product (20.6 g) as an oily matter.

¹H-NMR (CDCl₃) δ 0.08 (6H, s), 0.93 (9H, s), 3.87 (3H, s), 4.87 (2H, s), 5.16 (2H, br s), 6.58 (1H, dd), 6.96 (1H, dd), 7.19 (1H, t)

### Reference Example 43

### Methyl 4-amino-5-chlorothiophene-2-carboxylate

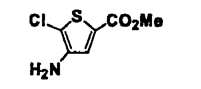

While stirring methyl 5-chloro-4-nitrothiophene-2-carboxylate (4.77 g) and reduced iron (9.2 g) in ethanol (120 ml), concentrated hydrochloric acid (30 ml) was added dropwise thereto at 60°C. After completion of the dropwise addition, the reaction solution was neutralized with an aqueous sodium hydrogen carbonate solution, and extracted with ethyl acetate. The organic layer was dried over anhydrous magnesium sulfate, and the solvent was distilled off under reduced pressure to give the desired product (3.94 g) as a powder.

¹H-NMR (CDCl₃) δ 3.73 (2H, br s), 3.85 (3H, s), 7.24 (1H, s)

### Reference Example 44

### Methyl 4-[5-({[tert-butyl(dimethyl)silyl]oxy}methyl)-2,4-dioxo-1,4-dihydroquinazolin-3(2H)-yl]-5-chlorothiophene-2-carboxylate

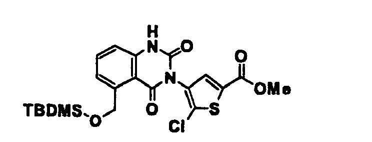

To a solution of methyl 2-amino-6-({[tert-butyl(dimethyl)silyl]oxy}methyl)benzoate (16.0 g) and triethylamine (10.1 ml) in THF (100 ml), a solution of triphosgene (5.35 g) in THF (30 ml) was added dropwise at -20°C, and the mixture was stirred for 0.5 hour at room temperature. The reaction solution was diluted with ethyl acetate, the precipitates were separated by filtration, and the filtrate was concentrated to obtain an oily matter.

To a solution of methyl 4-amino-5-chlorothiophene-2-carboxylate (6.91 g) and DMAP (8.81 g) in THF (150 ml), a solution of the above-obtained oily matter in THF (50 ml) was added at room temperature, and the mixture was stirred for 3 days at room temperature. The reaction solution was poured into an aqueous ammonium chloride solution, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (3 : 1 to 2 : 1)), and crystallized from ethyl acetate/diethyl ether/diisopropyl ether to give the desired product (12.5 g) as crystals.

¹H-NMR (CDCl₃) δ 0.13 (6H, s), 0.97 (9H, s), 3.89 (3H, s), 5.24 (2H, s), 6.94 (1H, d), 7.62-7.71 (2H, m), 7.64 (1H, s), 9.30 (1H, s)

### Reference Example 45

### 3,4-Dihydro-2H-1,4-benzoxazin-3-ylmethanol

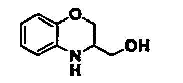

To a solution of N-(2-hydroxyphenyl)acetamide (25.5 g) in DMF (150 ml), a suspension of 60% sodium hydride and paraffin (6.76 g) was added portionwise with ice cooling, and the mixture was stirred for 1 hour at room temperature. Epichlorohydrin (15.6 g) was added to the mixture at room temperature, and then stirred for 8 hours at 90°C. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (2: 1 to 1: 1)) to give the desired product (7.78 g) as an oily matter.

¹H-NMR (CDCl₃) δ 1.82 (1H, t), 3.56-3.62 (1H, m), 3.63-3.78 (2H, m), 4.05 (1H, br s), 4.09 (1H, dd), 4.21 (1H, dd), 6.64-6.71 (2H, m), 6.76-6.82 (2H, m)

### Reference Example 46

### {4-[(3-Nitrophenyl)sulfonyl]-3,4-dihydro-2H-1,4-benzoxazin-3-yl}methanol

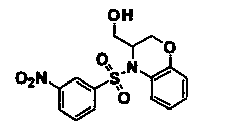

While stirring 3,4-dihydro-2H-1,4-benzoxazin-3-ylmethanol (5.70 g) and sodium hydrogen carbonate (4.35 g) in THF (50 ml), a solution of 3-nitrobenzensulfonyl chloride (7.64 g) in THF (30 ml) was added at room temperature, and the mixture was stirred for 1 week at 60°C. The reaction solution was poured into water, and extracted twice with ethyl acetate. The collected organic layers were washed with diluted hydrochloric acid, and dried over anhydrous magnesium sulfate. Then, the solvent was distilled off under reduced pressure. The resulting residue was purified with silica gel column chromatography (hexane/ethyl acetate (2 : 1 to 1 : 1)) to give the desired product (7.56 g) as an oily matter.

¹H-NMR (CDCl₃) δ 1.80 (1H, dd), 3.37 (1H, dd), 3.57-3.66 (1H, m), 3.69-3.77 (1H, m), 4.21 (1H, dd), 4.48-4.55 (1H, m), 6.80 (1H, dd), 7.00 (1H, ddd), 7.11 (1H, ddd), 7.66 (1H, t), 7.85 (1H, dd), 7.91 (1H, ddd), 8.41 (1H, ddd), 8.51 (1H, t)

### Reference Example 47

### 3-({[tert-Butyl(dimethyl)silyl]oxy}methyl)-4-[(3-nitrophenyl)sulfonyl]-3,4-dihydro-2H-1,4-benzoxazine

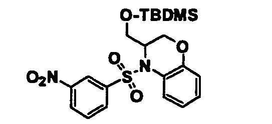

To a solution of (4-[(3-nitrophenyl)sulfonyl]-3,4-dihydro-2H-1,4-benzoxazin-3-yl)methanol (0.90 g), DMAP (31 mg) and triethylamine (0.43 ml) in THF (20 ml), tert-butylchlorodimethylsilane (0.43 g) was added at room temperature, and the mixture was stirred overnight as it was. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (3 : 1)), and crystallized from diisopropyl ether/hexane to give the desired product (0.68 g) as crystals.

¹H-NMR (CDCl₃) 80.01 (3H, s), 0.04 (3H, s), 0.87 (9H, s), 3.28 (1H, dd), 3.50 (1H, t), 3.72 (1H, dd), 4.27 (1H, dd), 4.38-4.45 (1H, m), 6.80 (1H, dd), 6.97 (1H, dt), 7.09 (1H, dt), 7.64 (1H, t), 7.84-7.91 (2H, m), 8.40 (1H, ddd), 8.48 (1H, t)

### Reference Example 48

### 4-(3,4-Dihydroquinolin-1(2H)-ylsulfonyl)thiophen-2-amine

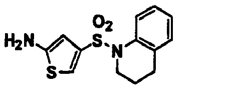

1) Half amount of a solution of commercially available 2-nitrothiophene (containing about 15% of 3-nitrothiophene) (50.1 g) in chloroform (100 ml) was added dropwise to a solution of chlorosulfonic acid (113 g) in chloroform (100 ml) while subjecting to reflux. Chlorosulfonic acid (113 g) was further added thereto, and then the remaining solution of 2-nitrothiophene in chloroform was added dropwise thereto. The mixture was stirred overnight at 60°C. The reaction solution was poured into water, the chloroform layer was separated, and the aqueous layer was extracted twice with ethyl acetate. The collected organic layers were dried over anhydrous magnesium sulfate, and the solvent was distilled off under reduced pressure to give an isomeric mixture of nitrothiophenesulfonyl chloride (88.6 g) as an oily matter.
2) To a solution of 1,2,3,4-tetrahydroquinoline (11.7 g) in acetonitrile (50 ml), a solution of the above-obtained oily matter (13.4 g) in acetonitrile (50 ml) was added at room temperature, and the mixture was stirred overnight as it was. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (9 : 1 to 3 : 1)) to give an isomeric mixture of 1-[(nitrothienyl)sulfonyl]-1,2,3,4-tetrahydroquinoline (7.75 g) as crystals.
3) To a mixture of the above-obtained crystals (7.06 g), ammonium chloride (5.82 g) and methanol (200 ml), zinc (28.5 g) was added portionwise at 60°C, and the mixture was stirred for 30 minutes at 60°C. The insolubles were removed by subjecting the reaction solution to filtration, and the filtrate was distilled off under reduced pressure. The resulting residue was purified with silica gel column chromatography (hexane/ethyl acetate (3 : 1 to 2 : 1)) to give an isomeric mixture of the desired product (1.5 g) as crystals. Subsequently, the isomers were separated using NH-silica gel column chromatography to give the desired product (0.94 g) as crystals, as well as its isomer, 5-(3,4-dihydroquinolin-1(2H)-ylsulfonyl)thiophen-2-amine (0.22 g), as crystals.

¹H-NMR (CDCl₃) δ 1.71-1.79 (2H, m), 2.59 (2H, t), 3.77-3.81 (2H, m), 3.84 (2H, br s), 5.96 (1H, d), 6.98 (1H, d), 7.03-7.11 (2H, m), 7.15-7.20 (1H, m), 7.75 (1H, ddd)

### Reference Example 49

### 5-(3,4-Dihydroquinolin-1(2H)-ylsulfonyl)thiophen-2-amine

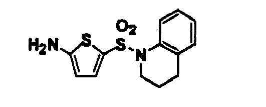

See Reference Example 48.

¹H-NMR (CDCl₃) δ 1.72-1.81 (2H, m), 2.58 (2H, t), 3.80 (2H, t), 4.17 (2H, br s), 5.99 (1H, d), 7.03 (1H, d), 7.05-7.10 (2H, m), 7.15-7.21 (1H, m), 7.77 (1H, d)

### Reference Example 50

### 1-[(4-Nitro-2-thienyl)sulfonyl]-2,3,4,5-tetrahydro-1H-1-benzazepine

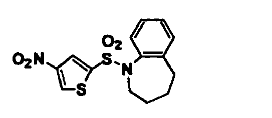

A solution of 1-[(5-chloro-4-nitro-2-thienyl)sulfonyl]-2,3,4,5-tetrahydro-1H-1-benzazepine (0.54 g), zinc cyanide (0.17 g) and tetrakis(triphenylphosphine)palladium (0) (0.17 g) in DMF (10 ml) was stirred for 4 hours at 90°C. Tetrakis(triphenylphosphine)palladium (0) (0.17 g) was added thereto, and the mixture was further stirred for 3 hours at 90°C. The reaction solution was poured into an aqueous sodium hydroxide solution, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (9 : 1 to 3 : 1)) to give the desired product (0.36 g) as an oily matter.

¹H-NMR (CDCl₃) δ 1.64 (2H, br s), 1.87-1.95 (2H, m), 2.51 (2H, t), 3.74 (2H, br s), 7.15-7.32 (4H, m), 7.96 (1H, d), 8.43 (1H, d)

### Reference Example 51

### 3-Nitro-5-(2,3,4,5-tetrahydro-1H-1-benzazepin-1-ylsulfonyl)thiophene-2-carbonitrile

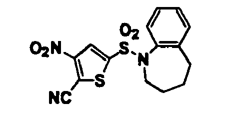

A solution of 1-[(5-chloro-4-nitro-2-thienyl)sulfonyl]-2,3,4,5-tetrahydro-1H-1-benzazepine (0.55 g) and copper cyanide (I) (0.20 g) in DMF (5 ml) was stirred for 2 hours at 150°C. The reaction solution was poured into water, ethylenediamine (1 ml) was added thereto. The mixture was stirred, and then extracted twice with ethyl acetate. The collected organic layers were dried over anhydrous magnesium sulfate, and the solvent was distilled off under reduced pressure. The resulting residue was purified with silica gel column chromatography (hexane/ethyl acetate (9 : 1 to 6 : 1)) to give the desired product (0.15 g) as a solid.

¹H-NMR (CDCl₃) δ 1.67 (2H, br s), 1.88-1.95 (2H, m), 2.47-2.53 (2H, m), 3.77 (2H, br s), 7.19-7.32 (4H, m), 7.96 (1H, s)

### Reference Example 52

### 3-Amino-5-(2,3,4,5-tetrahydro-1H-1-benzazepin-1-ylsulfonyl)thiophene-2-carboxamide

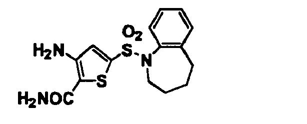

To a mixture of 3-nitro-5-(2,3,4,5-tetrahydro-1H-1-benzazepin-1-ylsulfonyl)thiophene-2-carbonitrile (0.91 g), ammonium chloride (0.67 g) and methanol (100 ml), zinc (3.26 g) was added at room temperature, and the mixture was stirred for 40 minutes at 60°C. The insolubles were removed by subjecting the reaction solution to filtration, and the filtrate was distilled off under reduced pressure. The resulting residue was purified with silica gel column chromatography (hexane/ethyl acetate (1 : 1) to ethyl acetate), and crystallized from ethyl acetate/diisopropyl ether to give the desired product (0.48 g) as crystals.

¹H-NMR (CDCl₃) δ 1.63 (2H, br s), 1. 87-1. 94 (2H, m), 2.58 (2H, t), 3.71 (2H, br s), 5.32 (2H, br s), 5.67 (2H, br s), 6.88 (1H, s), 7.16-7.24 (3H, m), 7.32-7.35 (1H, m)

### Reference Example 53

### 2-[(Trifluoroacetyl)amino]thiophene-3-carboxamide

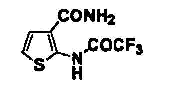

While stirring 2-aminothiophene-3-carboxamide (20.0 g) and sodium hydrogen carbonate (23.6 g) in THF (150 ml), a solution of anhydrous trifluoroacetic acid (31.0 g) in THF (50 ml) was added dropwise with ice cooling, and the mixture was stirred for 3 hours at room temperature. The reaction solution was poured into water, and the reaction mixture was acidified with diluted hydrochloric acid, and then extracted three times with ethyl acetate. The collected organic layers were dried over anhydrous magnesium sulfate and passed through silica gel. Then, the solvent was distilled off under reduced pressure. The resulting residue was crystallized from diisopropyl ether to give the desired product (28.5 g) as crystals.

¹H-NMR (CDCl₃-DMSO-d₆) δ 6.29 (1H, br s), 6.96 (1H, d), 7.32 (1H, d), 7.35 (1H, br s), 13.38 (1H, br s)

### Reference Example 54

### 5-(2,3,4,5-Tetrahydro-1H-1-benzazepin-1-ylsulfonyl)-2-[(trifluoroacetyl)amino]thiophene-3-carboxamide

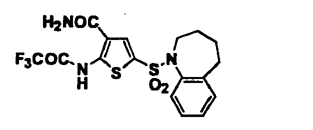

To 2-[(trifluoroacetyl)amino]thiophene-3-carboxamide (5.12 g), chlorosulfonic acid (7.14 ml) was added dropwise with ice cooling, and the mixture was stirred for 1 hour at room temperature followed by 2 hours at 50°C. After cooling, the reaction solution was poured onto crushed ice, and extracted twice with ethyl acetate. The collected organic layers were dried over anhydrous magnesium sulfate, and the solvent was distilled off under reduced pressure to obtain a solid.

To a solution of 2,3,4,5-tetrahydro-1H-1-benzazepine (3.16 g), pyridine (3.48 ml), DMAP (0.26 g) in acetonitrile (50 ml), a solution of the above-obtained solid in THF (20 ml) was added at room temperature, and the mixture was stirred overnight as it was. The reaction solution was poured into diluted hydrochloric acid, and extracted twice with ethyl acetate. The collected organic layers were dried over anhydrous magnesium sulfate and passed through silica gel. Then, the solvent was distilled off under reduced pressure. The resulting residue was crystallized from ethyl acetate/diethyl ether/diisopropyl ether to give the desired product (7.73 g) as crystals.

¹H-NMR (CDCl₃-DMSO-d₆) δ 1.47 (2H, br s), 1.71-1.78 (2H, m), 2.46-2.51 (2H, m), 3.51 (2H, br s), 6.45 (1H, br s), 7.01-7.10 (4H, m), 8.01 (1H, s), 8.11 (1H, br s), 13.65 (1H, br s)

### Reference Example 55

### N'-[3-Cyano-5-(2,3,4,5-tetrahydro-1H-1-benzazepin-1-ylsulfonyl)-2-thienyl]-N,N-dimethylimidoformamide

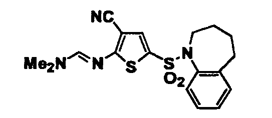

A mixture of 5-(2,3,4,5-tetrahydro-1H-1-benzazepin-1-ylsulfonyl)-2-[(trifluoroacetyl)amino]thiophene-3-carboxamide (2.07 g), DMF (3 ml), thionyl chloride (1.01 ml) and THF (40 ml) was stirred for 2 hours at room temperature. The reaction solution was poured into water, and the reaction mixture was neutralized with sodium hydrogen carbonate, and then extracted twice with ethyl acetate. The collected organic layers were dried over anhydrous magnesium sulfate and passed through silica gel. Then, the solvent was distilled off under reduced pressure. The resulting residue was crystallized from diethyl ether/diisopropyl ether to give the desired product (1.34 g) as crystals.

¹H-NMR (CDCl₃) δ 1.63 (2H, br s), 1.85-1.93 (2H, m), 2.59-2.63 (2H, m), 3.16 (6H, s), 3.68 (2H, br s), 7.14-7.22 (3H, m), 7.30 (1H, s), 7.31-7.33 (1H, m), 7.72 (1H, s)

### Reference Example 56

### 2-Amino-5-(2,3,4,5-tetrahydro-1H-1-benzazepin-1-ylsulfonyl)thiophene-3-carbonitrile

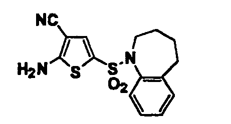

A solution of N'-[3-cyano-5-(2,3,4,5-tetrahydro-1H-1-benzazepin-1-ylsulfonyl)-2-thienyl]-N,N-dimethylimidoformamide (0.11 g), concentrated hydrochloric acid (1 ml) and water(1 ml) in methanol (5 ml) was stirred for 3 hours at 65°C. The reaction solution was poured into water, and the reaction mixture was neutralized with sodium hydrogen carbonate, and then extracted twice with ethyl acetate. The collected organic layers were dried over anhydrous magnesium sulfate, and the solvent was distilled off under reduced pressure to give the desired product (96 mg) as crystals.

¹H-NMR (CDCl₃) δ 1.64 (2H, br s), 1.85-1.92 (2H, m), 2.61 (2H, t), 3.65 (2H, br s), 5. 19 (2H, br s), 7. 15 (1H, s), 7.16-7.23 (3H, m), 7.27-7.31 (1H, m)

### Reference Example 57

### N-[3-Cyano-5-(2,3,4,5-tetrahydro-1H-1-benzazepin-1-ylsulfonyl)-2-thienyl]-2-nitrobenzamide

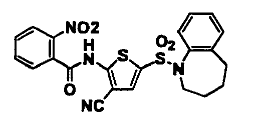

A solution of 2-amino-5-(2,3,4,5-tetrahydro-1H-1-benzazepin-1-ylsulfonyl)thiophene-3-carbonitrile (0.31 g), DMAP (0.24 g) and 2-nitrobenzoyl chloride (0.42 g) in THF (20 ml) and acetonitrile (20 ml) was stirred overnight at room temperature. The reaction solution was diluted with ethyl acetate, and then sequentially washed with diluted hydrochloric acid and an aqueous sodium hydrogen carbonate solution. The organic layer of the resulting ethyl acetate solution was dried over anhydrous magnesium sulfate and passed through silica gel. Then, the solvent was distilled off under reduced pressure. The resulting residue was crystallized from diethyl ether/diisopropyl ether to give the desired product (0.30 g) as crystals.

¹H-NMR (CDCl₃-DMSO-d₆) δ 1.66 (2H, br s), 1.89-1.96 (2H, m), 2.67 (2H, t), 3.63-3.75 (2H, m), 7.17-7.29 (4H, m), 7.41 (1H, s), 7.63 (1H, dd, J = 1.5 H), 7.70 (1H, dt), 7.79 (1H, dt), 8.21 (1H, d)

### Reference Example 58

### Benzyl 3-{[tert-butyl(dimethyl)silyl]oxy}-3,4-dihydro-1,5-benzoxazepine-5(2H)-carboxylate

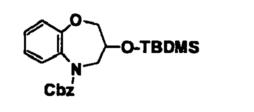

To a solution of 5-(methylsulfonyl)-2,3,4,5-tetrahydro-1,5-benzoxazepin-3-ol (5.62 g) in toluene (100 ml), a 70% solution of sodium bis(2-methoxyethoxy)aluminum hydride in toluene (28.4 g) was added at room temperature, and the mixture was stirred for 1 hour at 85°C. A 15% aqueous potassium carbonate solution was added dropwise to the reaction solution, and the mixture was extracted three times with ethyl acetate. The collected organic layers were dried over anhydrous magnesium sulfate, and the solvent was distilled off under reduced pressure to obtain an oily matter.

While stirring the above-obtained oily matter and sodium hydrogen carbonate (2.48 g) in THF (50 ml), a solution of benzyl chlorocarbonate (3.69 g) in THF (20 ml) was added at room temperature, and the mixture was stirred overnight at 50°C. The reaction solution was poured into water, and extracted twice with ethyl acetate. The collected organic layers were washed with diluted hydrochloric acid, dried over anhydrous magnesium sulfate, and passed through silica gel. Then, the solvent was distilled off under reduced pressure to obtain an oily matter.

To a solution of the above-obtained oily matter and imidazole (2.01 g) in DMF (50 ml), tert-butylchlorodimethylsilane (3.56 g) was added at room temperature, and the mixture was stirred overnight as it was. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (15 : 1)) to give the desired product (8.97 g) as an oily matter.

¹H-NMR (CDCl₃) δ 0.14 (6H, br s), 0.89 (9H, br s), 2.95-3.20 (1H, m), 3.55-3.78 (1H, m), 4.08-4.40 (3H, m), 4.97-5.18 (1H, m), 5.27 (1H, d), 6.98-7.03 (2H, m), 7.11-7.40 (7H, m)

### Reference Example 59

### 3-{[tert-Butyl(dimethyl)silyl]oxy}-2,3,4,5-tetrahydro-1,5-benzoxazepine

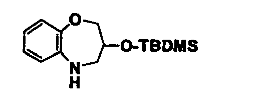

A solution of benzyl 3-{[tert-butyl(dimethyl)silyl]oxy}-3,4-dihydro-1,5-benzoxazepine-5(2H)-carboxylate (8.97 g) in THF (25 ml) and methanol (25 ml) was hydrogenated for 8 hours in presence of 10% palladium carbon (50% water content, 5 g) at room temperature at normal pressure. The catalyst was separated by filtration, and the filtrate was concentrated. The resulting residue was purified with silica gel column chromatography (hexane/ethyl acetate (9 : 1)) to give the desired product (4.79 g) as an oily matter.

¹H-NMR (CDCl₃) δ 0.10 (6H, s), 0.90 (9H, s), 3.20 (1H, dd), 3.47 (1H, dd), 3.56 (1H, br s), 3.91 (1H, dd), 4.08-4.17 (1H, m), 4.32 (1H, dd), 6.62 (1H, dd), 6.71 (1H, dt), 6.82 (1H, dt), 6.88 (1H, dd)

### Reference Example 60

### 3-{[tert-Butyl(dimethyl)silyl]oxy}-5-[(3-nitrophenyl)sulfonyl]-2,3,4,5-tetrahydro-1,5-benzoxazepine

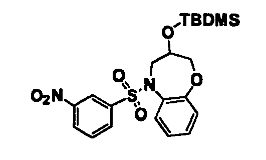

To a solution of 3-{[tert-butyl(dimethyl)silyl]oxy}-2,3,4,5-tetrahydro-1,5-benzoxazepine (4.27 g), triethylamine (3.20 ml) and DMAP (0.19 g) in THF (50 ml), a solution of 3-nitrobenzensulfonyl chloride (4.75 g) in THF (30 ml) was added at room temperature, and the mixture was stirred overnight at room temperature. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (6 : 1)) to give the desired product (6.37 g) as a solid.

¹H-NMR (CDCl₃) δ 0.10 (3H, s), 0.15 (3H, s), 0.88 (9H, s), 3.07-3.16 (1H, m), 3.23-3.30 (1H, m), 3.88-4.01 (2H, m), 4.34-4.41 (1H, m), 6.97 (1H, dd), 7.15 (1H, dt), 7.28 (1H, dt), 7.50 (1H, dd), 7.59 (1H, t), 7.83 (1H, d), 8.38 (1H, ddd), 8.51 (1H, t)

### Reference Example 61

### 3-{[3-{[tert-Butyl(dimethyl)silyl]oxy}-3,4-dihydro-1,5-benzoxazepin-5(2H)-yl]sulfonyl}aniline

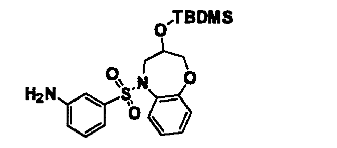

To a mixture of 3-{[tert-butyl(dimethyl)silyl]oxy}-5-[(3-nitrophenyl)sulfonyl]-2,3,4,5-tetrahydro-1,5-benzoxazepine (0.63 g), nickel bromide (II) (15 mg), methanol (10 ml) and THF (10 ml), sodium borohydride (0.15 g) was added at room temperature, and the mixture was stirred for 1 hour as it was. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (6 : 1 to 2 : 1)) to give the desired product (0.55 g) as a solid.

¹H-NMR (CDCl₃) δ 0.08 (3H, s), 0.12 (3H, s), 0.87 (9H, s), 3.01 (1H, dd), 3.32 (1H, dd), 3.81 (2H, br s), 3.96-4.05 (1H, m), 4.09 (1H, ddd), 4.30 (1H, ddd), 6.81 (1H, ddd), 6.92 (1H, t), 7.00-7.08 (3H, m), 7.20 (1H, dt), 7.21 (1H, t), 7.39 (1H, dd)

### Reference Example 62

### 5-[(3-nitrophenyl)sulfonyl]-2,3,4,5-tetrahydro-1,5-benzoxazepin-3-ol

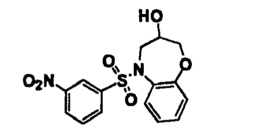

To a solution of 3-{[tert-butyl(dimethyl)silyl]oxy}-5-[(3-nitrophenyl)sulfonyl]-2,3,4,5-tetrahydro-1,5-benzoxazepine (5.52 g) in THF (40 ml), a 1 N solution of tetrabutylammonium fluoride in THF (17.8 ml) was added at room temperature, and the mixture was stirred overnight as it was. The solvent of the reaction solution was distilled off under reduced pressure. The residue was purified with silica gel column chromatography (hexane/ethyl acetate (3: 1 to 1: 2)) to give the desired product (3.80 g) as a solid.

¹H-NMR (CDCl₃) δ 2.12 (1H, d), 3.70-3.86 (2H, m), 3.91-4.02 (2H, m), 4.14 (1H, br s), 7.02 (1H, dd), 7.14 (1H, dt), 7.29 (1H, dt), 7.51 (1H, dd), 7.62 (1H, t), 7.96 (1H, d), 8.38 (1H, ddd), 8.52 (1H, t)

### Reference Example 63

### 3-Methoxy-5-[(3-nitrophenyl)sulfonyl]-2,3,4,5-tetrahydro-1,5-benzoxazepine

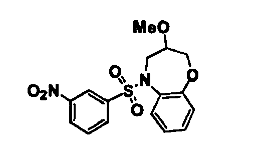

To a solution of 5-[(3-nitrophenyl)sulfonyl]-2,3,4,5-tetrahydro-1,5-benzoxazepin-3-ol (0.43 g) in THF (15 ml), a suspension of 60% sodium hydride and liquid paraffin (54 mg) was added at room temperature, and the mixture was stirred for 15 minutes as it was. Methyl iodide (0.11 ml) was added to the mixture at room temperature and the reaction mixture was stirred overnight as it was. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (9 : 1 to 2 : 1)), and crystallized from diisopropyl ether/hexane to give the desired product (0.30 g) as crystals.

¹H-NMR (CDCl₃) δ 3.43 (4H, s), 3.64-3.70 (2H, m), 3.93-4.00 (1H, m), 4.25 (1H, br s), 6.95 (1H, dd), 7.15 (1H, dt), 7.26 (1H, dt), 7.51 (1H, t), 7.62 (1H, dd), 7.73 (1H, d), 8.32 (1H, ddd), 8.55 (1H, t)

### Reference Example 64

### 3-Fluoro-5-[(3-nitrophenyl)sulfonyl]-2,3,4,5-tetrahydro-1,5-benzoxazepine

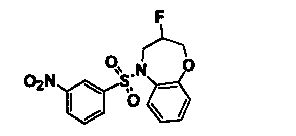

To a solution of 5-[(3-nitrophenyl)sulfonyl]-2,3,4,5-tetrahydro-1,5-benzoxazepin-3-ol (0.83 g) and pyridine (0.95 ml) in dichloromethane (30 ml), diethylaminosulfur trifluoride (0.62 ml) was added at -78°C, and the mixture was stirred overnight at room temperature. The reaction solution was poured into an aqueous sodium hydrogen carbonate solution, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (6: 1 to 2: 1)) to give the desired product (0.28 g) as crystals.

¹H-NMR (CDCl₃) δ 3.42-3.68 (2H, m), 4.15-4.26 (1H, m), 4.58-4.76 (2H, m), 7.00 (1H, dd), 7.22 (1H, dt), 7.32 (1H, dt), 7.54 (1H, t), 7.67 (1H, dd), 7.82 (1H, ddd), 8.32-8.38 (2H, m)

### Reference Example 65

### 5-Nitro-N-phenyl-N-(2,2,2-trifluoroethyl)thiophene-3-carboxamide

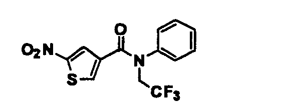

To a solution of 5-nitrothiophene-3-carboxylic acid (1.98 g) and DMF (2 drops) in THF (50 ml), oxalyl chloride (2.00 ml) was added at room temperature, and the mixture was stirred for 1 hour as it was. The solvent of the reaction solution was distilled off under reduced pressure to obtain an oily matter.

To a solution of N-(2,2,2-trifluoroethyl)aniline (2.20 g), pyridine (1.37 ml) and DMAP (0.14 g) in acetonitrile (30 ml), a solution of the above-obtained oily matter in THF (20 ml) was added at room temperature, and the mixture was stirred overnight at room temperature. The reaction solution was poured into diluted hydrochloric acid, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (6 : 1 to 3 : 1)), and crystallized from hexane to give the desired product (1.63 g) as crystals.

¹H-NMR (CDCl₃) δ 4.52 (2H, q), 7.21-7.25 (3H, m), 7.38 (1H, d), 7.42-7.44 (2H, m), 7.51 (1H, d)

### Reference Example 65 (1)

### In the same manner as in Reference Example 65, 5-nitrothiophene-3-carboxylic acid was reacted with N-ethylaniline to obtain the following compound.

N-Ethyl-5-nitro-N-phenylthiophene-3-carboxamide

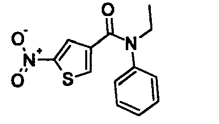

¹H-NMR (CDCl₃) δ 1.22 (3H, t), 3.94 (2H, q), 7.11-7.19 (2H, m), 7.34-7.45 (4H, m), 7.52 (1H, d)

### Reference Example 66

### 3-Amino-4-chloro-N-methyl-N-phenylbenzamide

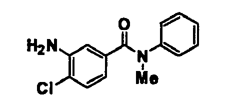

While stirring N-methylaniline (5.51 g) and sodium hydrogen carbonate (7.86 g) in THF (150 ml), 4-chloro-3-nitrobenzoyl chloride (10.3 g) was added portionwise at room temperature, and the mixture was stirred overnight as it was. The reaction solution was poured into water, and extracted twice with ethyl acetate. The collected organic layers were dried over anhydrous magnesium sulfate and passed through silica gel. The solvent was distilled off under reduced pressure to obtain an oily matter.

To a mixture of the above-obtained oily matter, nickel bromide (II) (0.51 g), methanol (100 ml) and THF (100 ml), sodium borohydride (5.31 g) was added portionwise with water cooling, and then the mixture was stirred for 2 hours as it was. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (3 : 1 to 1 : 1)) to give the desired product (10.3 g) as a solid.

¹H-NMR (CDCl₃) δ 3.46 (3H, s), 3.98 (2H, br s), 6.45 (1H, dd), 6.84 (1H, d), 6.95 (1H, d), 7. O1-7.04 (2H, m), 7.12-7.18 (1H, m), 7.20-7.27 (2H, m)

### Reference Example 66 (1)

In the same manner as in Reference Example 66, the following compound was obtained from 2-(methylamino)pyridine and 4-chloro-3-nitrobenzoyl chloride.

### 3-Amino-4-chloro-N-methyl-N-pyridin-2-ylbenzamide

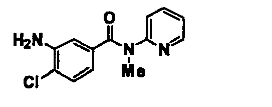

¹H-NMR (CDCl₃) δ 3.55 (3H, s), 4.03 (2H, br s), 6.50 (1H, dd), 6.82 (1H, td), 6.87 (1H, d), 7.02 (1H, d), 7.05 (1H, ddd), 7.47 (1H, ddd), 8.43 (1H, ddd)

### Reference Example 67

### N-Methyl-2-(3-nitrophenyl)-N-phenylacetamide

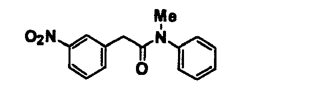

While stirring (3-nitrophenyl)acetic acid (1.08 g), N-methylaniline (0.77 g), N-methylimidazole (0.71 ml) in THF (40 ml) and acetonitrile (20 ml), 1-ethyl-3-(3-dimethylaminopropyl)carbodiimidehydrochloride (1.71 g) was added at room temperature, and the mixture was stirred overnight as it was. The reaction solution was diluted with ethyl acetate, and then sequentially washed with diluted hydrochloric acid and an aqueous sodium hydrogen carbonate solution. The resulting ethyl acetate solution was dried over anhydrous magnesium sulfate, and the solvent was distilled off under reduced pressure. The resulting residue was purified with silica gel column chromatography (hexane/ethyl acetate (3 : 1 to 2 : 1)) to give the desired product (1.55 g) as an oily matter.

¹H-NMR (CDCl₃) δ 3.29 (3H, s), 3.54 (2H, s), 7.14-7.17 (2H, m), 7.36-7.50 (5H, m), 7.85 (1H, s), 8.06 (1H, ddd)

### Reference Example 68

### N-Methyl-2-(3-nitrophenyl)-N-phenylpropanamide

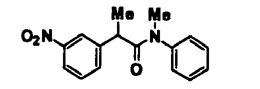

To a solution of N-methyl-2-(3-nitrophenyl)-N-phenylacetamide (0.68 g) in THF (20 ml), a suspension of 60% sodium hydride and liquid paraffin (0.25 g) was added at room temperature, and the mixture was stirred for 15 minutes as it was. Methyl iodide (0.62 ml) was added to the mixture at room temperature, and the mixture was stirred for 1 hour as it was. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (9 : 1 to 3 : 1)) to give the desired product (0.60 g) as an oily matter.

¹H-NMR (CDCl₃) δ 1.43 (3H, d), 3.25 (3H, s), 3.77 (1H, q), 7.01 (2H, br s), 7.35-7.42 (4H, m), 7.49 (1H, td), 7.77 (1H, t), 8.04 (1H, ddd)

### Reference Example 69

### 1-Methyl-4-[(3-nitrophenyl)sulfonyl]-3,4-dihydroquinoxalin-2(1H)-one

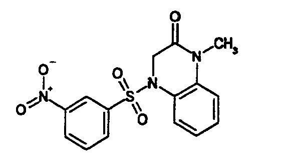

A mixture of 4-[(3-nitrophenyl)sulfonyl]-3,4-dihydroquinoxalin-2(1H)-one (983 mg), methyl iodide (2.51 g), potassium carbonate (1.22 g) and DMF (10 ml) was agitated for 6 hours at 50°C. The reaction mixture was then poured into water, and extracted with ethyl acetate. The organic layer was sequentially washed with water and saturated brine, and dried over anhydrous magnesium sulfate. Then, the solvent was distilled off under reduced pressure, and the crystals were collected by filtration to give the desired product (792 mg). The product was recrystallized from ethyl acetate/diisopropyl ether.

¹H-NMR (CDCl₃) δ 2.70 (3H, s), 4.40 (2H, s), 6.84 (1H, d), 7.22-7.27 (1H, m), 7.34-7.40 (1H, m), 7.64 (1H, t), 7.74 (1H, dd), 7.85-7.88 (1H, m), 8.09 (1H, t), 8.33-8.37 (1H, m)

### Reference Example 69(1)

In the same manner as in Reference Example 69, the following compound was obtained from 5-[(3-nitrophenyl)sulfonyl]-1,3,4,5-tetrahydro-2H-1,5-benzodiazepin-2-one.

### 1-Methyl-5-[(3-nitrophenyl)sulfonyl]-1,3,4,5-tetrahydro-2H-1,5-benzodiazepin-2-one

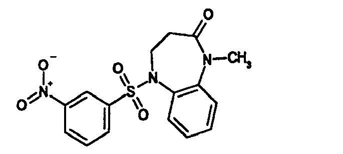

¹H-NMR (CDCl₃) δ 2.47-2.51 (2H, m), 2.63 (3H, s), 4.00-4.60 (2H, m), 7.12 (1H, dd), 7.35 (1H, dt), 7.47 (1H, dt), 7.62 (1H, dd), 7.74 (1H, t), 8.01-8.05 (1H, m), 8.35 (1H, t), 8.41 (1H, ddd)

### Reference Example 70

### N-[2-(Benzyloxy)phenyl]-N-[(3-nitrophenyl)sulfonyl]alanine methyl ester

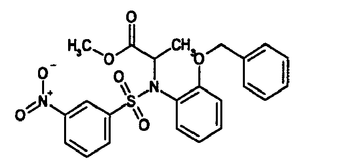

A mixture of N-[2-(benzyloxy)phenyl]-3-nitrobenzenesulfonamide (1.54 g), methyl 2-bromopropionate (1.00 g), potassium carbonate (995 mg) and DMF (5 ml) was agitated for 1 hour at 70°C. The reaction mixture was then poured into water and extracted with ethyl acetate. The organic layer was sequentially washed with water and saturated brine, and dried over anhydrous magnesium sulfate. Then, the solvent was distilled off under reduced pressure, and the crystals were collected by filtration to give the desired product (1.74 g).

¹H-NMR (CDCl₃) δ 1.31 (3H, d), 3.67 (3H, s), 4. 73-4. 86 (3H, m), 6.92-7.01 (2H, m), 7.13-7.15 (2H, m), 7.29-7.39 (4H, m), 7.42 (1H, t), 7.49 (1H, dd), 7.91-7.93 (1H, m), 8.15-8.18 (1H, m), 8.49 (1H, s)

### Reference Example 71

### Methyl 2-{(2-hydroxyethyl)[(3-nitrophenyl)sulfonyl]amino}benzoate

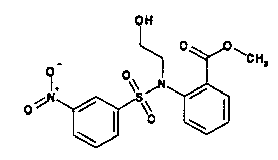

A mixture of methyl 2-{[(3-nitrophenyl)sulfonyl]amino}benzoate (1.00 g), 2-bromoethanol (2.23 g), potassium carbonate (1.23 g) and DMF (5 ml) was agitated for 1 hour at 100°C. The reaction mixture was then poured into water and extracted with ethyl acetate. The organic layer was sequentially washed with water and saturated brine, and dried over anhydrous magnesium sulfate. Then, the solvent was distilled off under reduced pressure. The residue was subjected to silica gel column chromatography, and the fraction eluted with ethyl acetate/hexane (1 : 1 to 1.5 : 1) was concentrated under reduced pressure to give the desired product (929 mg) as an oily matter.

¹H-NMR (CDCl₃) δ 3.44-3.81 (4H, m), 3.99 (3H, s), 4.66 (1H, t), 6.69-6.72 (1H, m), 7.40-7.49 (2H, m), 7.69 (1H, t), 7.87-7.91 (2H, m), 8.40 (1H, t), 8.43-8.46 (1H, m)

### Reference Example 72

### 2-{(2-Hydroxyethyl)[(3-nitrophenyl)sulfonyl]amino}benzoic acid

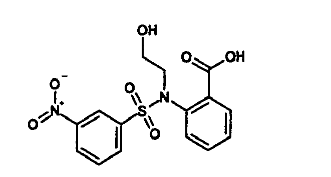

Methyl 2-{(2-hydroxyethyl)[(3-nitrophenyl)sulfonyl]amino}benzoate (1.10 g) was dissolved in methanol (9 ml), a 2 N aqueous sodium hydroxide solution (4.3 ml) was added thereto, and the mixture was agitated for 30 minutes at 50°C. The reaction solution was poured into water, and the reaction mixture was acidified with 2 N hydrochloric acid, and then extracted with ethyl acetate and THF (3 : 1). The organic layer was sequentially washed with water and saturated brine, and dried over anhydrous magnesium sulfate. Then, the solvent was distilled off under reduced pressure, and the crystals were collected by filtration to give the desired product (938 mg).

¹H-NMR (DMSO-d₆) δ 3.44 (2H, t), 3.74 (2H, t), 7.03-7.07 (1H, m), 7.49-7.53 (2H, m), 7.81-7.99 (3H, m), 8.23 (1H, t), 8.49-8.54 (1H, m)

### Reference Example 73

### 1-[(3-Nitrophenyl)sulfonyl]-2,3-dihydro-4,1-benzoxazepin-5(1H)-one

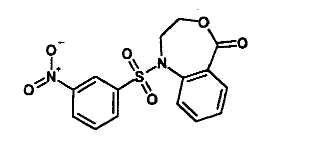

While dehydrating a mixture of 2-{(2-hydroxyethyl)[(3-nitrophenyl)sulfonyl]amino}benzoic acid (886 mg), p-toluene sulfonic acid monohydrate (552 mg) and toluene (50 ml) using a Dean-Stark trap, the mixture was heated under reflux for 1 hour. Then, the mixture was poured into a saturated sodium bicarbonate solution, and extracted with ethyl acetate and THF (3 : 1). The organic layer was sequentially washed with water and saturated brine, and dried over anhydrous magnesium sulfate. Then, the solvent was distilled off under reduced pressure, and the crystals were collected by filtration to give the desired product (797 mg).

¹H-NMR (CDCl₃) δ 4.13 (4H, s), 7.51-7.82 (6H, m), 8.33 (1H, t), 8.42-8.46 (1H, m)

### Reference Example 74

### 1-[(3-Nitrophenyl)sulfonyl]-2,3,4,5-tetrahydro-1H-1-benzazepine-8-carbonitrile

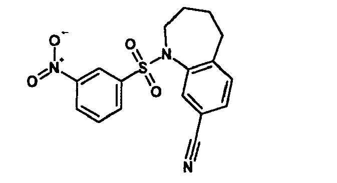

A mixture of 8-bromo-1-[(3-nitrophenyl)sulfonyl]-2,3,4,5-tetrahydro-1H-1-benzazepine (1.15 g), zinc cyanide (985 mg), tetrakis(triphenylphosphine)palladium (0) (485 mg) and DMF (20 ml) was agitated for 18 hours at 80°C under argon gas flow. Subsequently, the mixture was poured into 2.5% ammonia water, and extracted with ethyl acetate. The organic layer was sequentially washed with water and saturated brine, and dried over anhydrous magnesium sulfate. Then, the solvent was distilled off under reduced pressure. The residue was subjected to silica gel column chromatography, and the fraction eluted with ethyl acetate/ chloroform/hexane (1 : 2 : 3) was concentrated under reduced pressure. Thus obtained crystals were collected by filtration to give the desired product (604 mg) .

¹H-NMR (CDCl₃) δ 1.59-1.61 (2H, m), 1.85 (2H, quintet), 2.46 (2H, t), 3.72-3.76 (2H, m), 7.27 (1H, d), 7.50-7.54 (2H, m), 7.72 (1H, t), 7.99-8.02 (1H, m), 8.43-8.47 (1H, m), 8.60 (1H, s)

### Reference Example 75

### 4-Methyl-3,4-dihydronaphthalen-1(2H)-one oxime

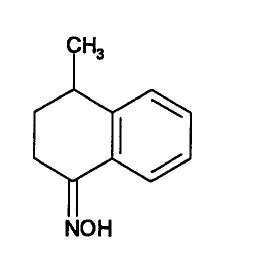

A mixture of 4-methyl-3,4-dihydronaphthalen-1(2H)-one (2.77 g), hydroxylamine hydrochloride (1.32 g), sodium acetate (1.56 g) and ethanol/water (4 : 1, 50 ml) was heated under reflux for 1 hour. The reaction mixture was then poured into water and extracted with ethyl acetate. The organic layer was sequentially washed with a diluted sodium hydrogen carbonate solution and saturated brine, and dried over anhydrous magnesium sulfate. Then, the solvent was distilled off under reduced pressure, and the crystals were collected by filtration to give the desired product (2.15 g).

¹H-NMR (CDCl₃) δ 1.30 (3H, d), 1.61-1.77 (1H, m), 1.89-2.03 (1H, m), 2.83-2.96 (3H, m), 7.17-7.36 (3H, m), 7.88 (1H, dd), 8.58 (1H, s)

### Reference Examples 75(1) to 75(12)

In the same manner as in Reference Example 75, the corresponding cyclohexanone derivatives (commercially available or conventional) were reacted with hydroxylamine hydrochloride to obtain the following compounds.

### Reference Example 75(1)

### 1-Methyl-1,5,6,7-tetrahydro-4H-indazol-4-one oxime

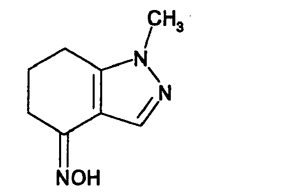

¹H-NMR (CDCl₃) δ 2.07 (2H, quintet), 2.50 (2H, t), 2.73 (2H, t), 3.81 (3H, s), 8.21 (1H, s), 8.44 (1H, br s)

### Reference Example 75(2)

### 1,6,6-Trimethyl-1,5,6,7-tetrahydro-4H-indazol-4-one oxime

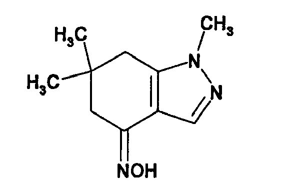

¹H-NMR (CDCl₃) δ 1.09 (6H, s), 2.31 (2H, s), 2.53 (2H, s), 3.79 (3H, s), 8.20 (1H, s), 8.46 (1H, br s)

### Reference Example 75(3)

### 5,8-Dimethoxy-3,4-dihydronaphthalen-1(2H)-one oxime

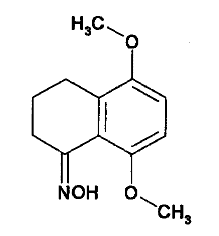

¹H-NMR (CDCl₃) δ 1.79 (2H, quintet), 2.72 (2H, t), 2.90 (2H, t), 3.80 (3H, s), 3.86 (3H, s), 6.79 (2H, s), 11.24 (1H, br s)

### Reference Example 75(4)

### 5,7-Dimethyl-3,4-dihydronaphthalen-1(2H)-one oxime

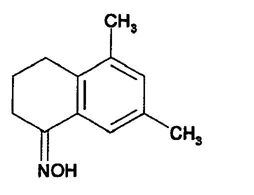

¹H-NMR (CDCl₃) δ 1.87 (2H, quintet), 2.25 (3H, s), 2.30 (3H, s), 2.65 (2H, t), 2.79 (2H, t), 7.00 (1H, s), 7.60 (1H, s), 7.89 (1H, s)

### Reference Example 75(5)

### 7-Methyl-3,4-dihydronaphthalen-1(2H)-one oxime

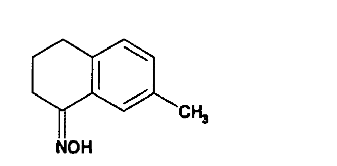

¹H-NMR (CDCl₃) δ 1.86 (2H, quintet), 2.33 (3H, s), 2.72 (2H, t), 2.81 (2H, t), 7.02-7.12 (2H, m), 7.71 (1H, s), 8.11 (1H, br s)

### Reference Example 75(6)

### 6,7-Dimethoxy-3,4-dihydronaphthalen-1(2H)-one oxime

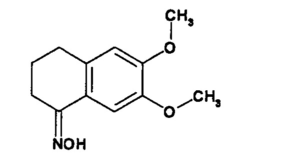

¹H-NMR (CDCl₃) δ 1.87 (2H, quintet), 2.71 (2H, t), 2.78 (2H, t), 3.89 (6H, s), 6.61 (1H, s), 7.40 (1H, s), 7.71 (1H, br s)

### Reference Example 75(7)

### 2,3,8,9-Tetrahydronaphtho[2,3-b] [1,4]dioxin-6(7H)-one oxime

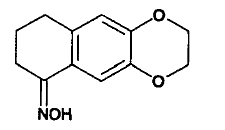

¹H-NMR (CDCl₃) δ 1.83 (2H, quintet), 2.64 (2H, t), 2.75 (2H, t), 4.23-4.27 (4H, m), 6.64 (1H, s), 7.42 (1H, s), 7.89 (1H, br s)

### Reference Example 75(8)

### 7-Phenyl-3,4-dihydronaphthalen-1(2H)-one oxime

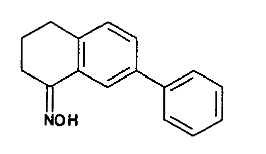

¹H-NMR (CDCl₃) δ 1.90 (2H, quintet), 2.80 (2H, t), 2.85 (2H, t), 7.22 (1H, d), 7.29-7.34 (1H, m), 7.38-7.44 (2H, m), 7.50 (1H, dd), 7.58-7.61 (2H, m), 8.10 (1H, br s), 8.14 (1H, d)

### Reference Example 75(9)

### 7-Cyclohexyl-3,4-dihydronaphthalen-1(2H)-one oxime

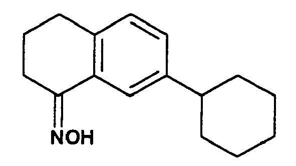

¹H-NMR (CDCl₃) δ 1.24-1.50 (5H, m), 1.71-1.90 (7H, m), 2.45-2.52 (1H, m), 2.73 (2H, t), 2.80 (2H, t), 7.08 (1H, d), 7.13 (1H, dd), 7.76 (1H, s), 7.76 (1H, br s)

### Reference Example 75(10)

### 7-tert-Butyl-3,4-dihydronaphthalen-1(2H)-one oxime

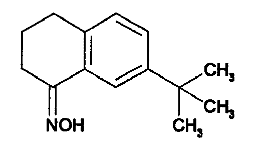

¹H-NMR (CDCl₃) δ 1.33 (9H, s), 1.87 (2H, quintet), 2.73 (2H, t), 2.81 (2H, t), 7.10 (1H, d), 7.32 (1H, dd), 7.76 (1H, br s), 7.95 (1H, s)

### Reference Example 75(11)

### 7-Phenoxy-3,4-dihydronaphthalen-1(2H)-one oxime

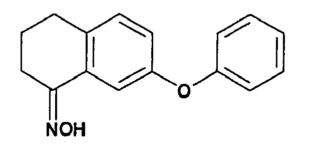

¹H-NMR (CDCl₃) δ 1. 87 (2H, quintet), 2.74 (2H, t), 2.78 (2H, t), 6.95 (1H, dd), 7.00 (2H, dd), 7.05-7.13 (2H, m), 7.32 (2H, t), 7.55 (1H, d), 7.74 (1H, s)

### Reference Example 75(12)

### 7-Fluoro-3,4-dihydronaphthalen-1(2H)-one oxime

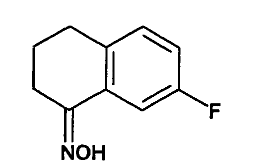

¹H-NMR (CDCl₃) δ 1. 87 (2H, quintet), 2.73 (2H, t), 2.80 (2H, t), 6.97 (1H, dt), 7.11 (1H, dd), 7.57 (1H, dd), 8.35 (1H, br s)

### Reference Example 76

### 5-Methyl-2,3,4,5-tetrahydro-1H-1-benzazepine

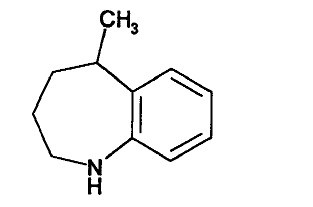

4-Methyl-3,4-dihydronaphthalen-1(2H)-one oxime (1.75 g) was dissolved in dichloromethane (100 ml), and the solution was ice cooled. A 1.0 M solution of diisobutylaluminum hydride in hexane (50.0 ml) was added dropwise thereto over 10 minutes. The mixture was agitated for 1 hour at 0°C, and further agitated for 2 hours at room temperature. The reaction solution was ice cooled and then diluted with dichloromethane (200 ml), which was then added sequentially with sodium fluoride (8.40 g) and water (2.7 ml). The reaction mixture was agitated for 20 minutes while returning the temperature to room temperature. Insolubles were filtered off, and the filtrate was concentrated under reduced pressure. The residue was subjected to silica gel column chromatography, and the fraction eluted with ethyl acetate/hexane (1 :6) was concentrated under reduced pressure to give the desired product (1.05 g) as an oily matter.

¹H-NMR (CDCl₃) δ 1.32 (3H, d), 1.52-1.94 (4H, m), 2.98-3.09 (1H, m), 3.03 (2H, t), 3.65 (1H, br s), 6.70 (1H, dd), 6.85 (1H, dt), 7.02 (1H, dt), 7.14 (1H, dd)

### Reference Examples 76(1) to 76(12)

In the same manner as in Reference Example 76, the following compounds were obtained from the oximes synthesized in Reference Examples 75(1) to 75(12).

### Reference Example 76(1)

### 1-Methyl-1,4,5,6,7,8-hexahydropyrazolo[4,3-b]azepine

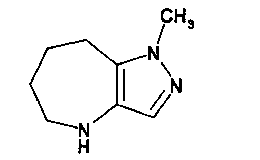

¹H-NMR (CDCl₃) δ 1.64-1.71 (2H, m), 1.81-1.88 (2H, m), 2.70 (2H, t), 2.99 (2H, t), 3.23 (1H, br s), 3.74 (3H, s), 7.03 (1H, s)

### Reference Example 76(2)

### 1,7,7-Trimethyl-1,4,5,6,7,8-hexahydropyrazolo[4,3-b]azepine

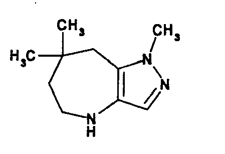

¹H-NMR (CDCl₃) δ 0.97 (6H, s), 1.70 (2H, t), 2.53 (2H, s), 2.98 (2H, t), 3.71 (3H, s), 7.03 (1H, s)

### Reference Example 76(3)

### 6,9-Dimethoxy-2,3,4,5-tetrahydro-1H-1-benzazepine

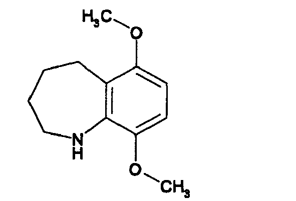

¹H-NMR (CDCl₃) δ 1.60-1.68 (2H, m), 1.75-1.84 (2H, m), 2.85 (2H, t), 3.05 (2H, t), 3.76 (3H, s), 3.78 (3H, s), 4.67 (1H, br s), 6.35 (1H, d), 6.61 (1H, d)

### Reference Example 76(4)

### 6,8-Dimethyl-2,3,4,5-tetrahydro-1H-1-benzazepine

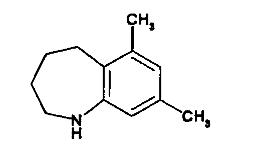

¹H-NMR (CDCl₃) δ 1.57-1.62 (2H, m), 1.76-1.78 (2H, m), 2.21 (3H, s), 2.27 (3H, s), 2.73 (2H, t), 3.03 (2H, t), 3.65 (1H, br s), 6.44 (1H, s), 6.58 (1H, s)

### Reference Example 76(5)

### 8-Methyl-2,3,4,5-tetrahydro-1H-1-benzazepine

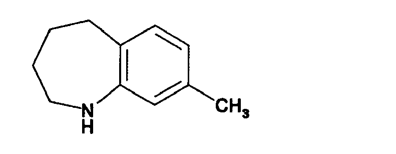

¹H-NMR (CDCl₃) δ 1.58-1.65 (2H, m), 1.75-1.82 (2H, m), 2.25 (3H, s), 2.73 (2H, t), 3.03 (2H, t), 3.71 (1H, br s), 6.56 (1H, s), 6.64 (1H, d), 6.99 (1H, d)

### Reference Example 76(6)

### 7,8-Dimethoxy-2,3,4,5-tetrahydro-1H-1-benzazepine

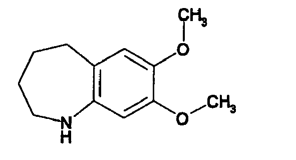

¹H-NMR (CDCl₃) δ 1.61-1.63 (2H, m), 1.75-1.78 (2H, m), 2.70 (2H, t), 3.01 (2H, t), 3.83 (6H, s), 6.33 (1H, s), 6.65 (1H, s)

### Reference Example 76(7)

### 2,3,7,8,9,10-Hexahydro-6H-[1,4]dioxino[2,3-h][1]benzazepine

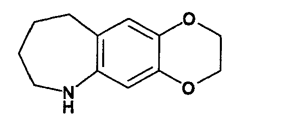

¹H-NMR (CDCl₃) δ 1.57-1.62 (2H, m), 1.72-1.78 (2H, m), 2.65 (2H, t), 2.97 (2H, t), 3.53 (1H, br s), 4.17-4.23 (4H, m), 6.28 (1H, s), 6.61 (1H, s)

### Reference Example 76(8)

### 8-Phenyl-2,3,4,5-tetrahydro-1H-1-benzazepine

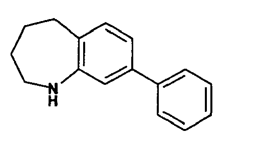

¹H-NMR (CDCl₃) δ 1. 63-1. 71 (2H, m), 1.79-1.86 (2H, m), 2.81 (2H, t), 3.09 (2H, t), 3.88 (1H, br s), 6.95 (1H, d), 7.04 (1H, dd), 7.16 (1H, d), 7.27-7.32 (1H, m), 7.37-7.42 (2H, m), 7.52-7.56 (2H, m)

### Reference Example 76(9)

### 8-Cyclohexyl-2,3,4,5-tetrahydro-1H-1-benzazepine

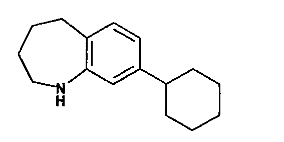

¹H-NMR (CDCl₃) δ 1.24-1.45 (5H, m), 1.61-1.66 (2H, m), 1.71-1.87 (7H, m), 2.38-2.42 (1H, m), 2.73 (2H, t), 3.04 (2H, t), 3.72 (1H, br s), 6.57 (1H, d), 6.67 (1H, dd), 7.01 (1H, d)

### Reference Example 76(10)

### 8-tert-Butyl-2,3,4,5-tetrahydro-1H-1-benzazepine

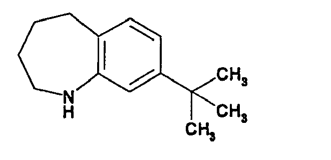

¹H-NMR (CDCl₃) δ 1.28 (9H, s), 1.61-1.67 (2H, m), 1.75-1.80 (2H, m), 2.73 (2H, t), 3.04 (2H, t), 3.77 (1H, br s), 6.73 (1H, d), 6.83 (1H, dd), 7.02 (1H, d)

### Reference Example 76(11)

### 8-Phenoxy-2,3,4,5-tetrahydro-1H-1-benzazepine

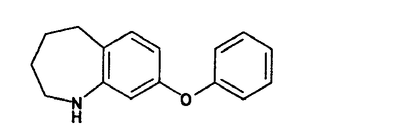

¹H-NMR (CDCl₃) δ 1.60-1.67 (2H, m), 1.75-1.80 (2H, m), 2.73 (2H, t), 3.04 (2H, t), 3.73 (1H, br s), 6.38 (1H, d), 6.45 (1H, dd), 6.98-7.09 (4H, m), 7.27-7.33 (2H, m)

### Reference Example 76(12)

### 8-Fluoro-2,3,4,5-tetrahydro-1H-1-benzazepine

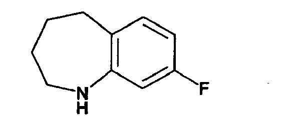

¹H-NMR (CDCl₃) δ 1.56-1.67 (2H, m), 1.73-1.81 (2H, m), 2.72 (2H, t), 3.06 (2H, t), 3.78 (1H, br s), 6.40-6.54 (2H, m), 7.01 (1H, dd)

### Reference Example 77

### N-[(3-Aminophenyl)sulfonyl]-N-(2-hydroxyphenyl)alanine methyl ester

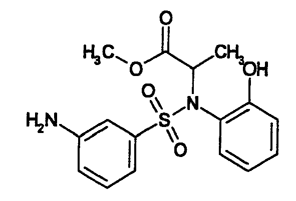

N-[2-(Benzyloxy)phenyl]-N-[(3-nitrophenyl)sulfonyl]alanine methyl ester (1.68 g) was dissolved in methanol/THF (1 : 1, 50 ml), 10% palladium carbon (50% water content, 1.0 g) was added thereto, and the mixture was subjected to catalytic reduction at normal pressure for 3 hours at room temperature. The catalyst was filtered off, and the filtrate was concentrated under reduced pressure. The residue was subjected to silica gel column chromatography, and the fraction eluted with ethyl acetate/hexane (1 : 1) was concentrated under reduced pressure. Thus obtained crystals were collected by filtration to give the desired product (1.13 g).

¹H-NMR (CDCl₃) δ 1.13 (3H, d), 3.84 (2H, s), 3.87 (3H, s), 4.94 (1H, q), 6.71-6.85 (3H, m), 6.93-6.95 (2H, m), 7.04 (1H, d), 7.19-7.23 (2H, m), 8.94 (1H, s)

### Reference Example 78

### N-[(3-Aminophenyl)sulfonyl]-N-(2-hydroxyphenyl)alanine

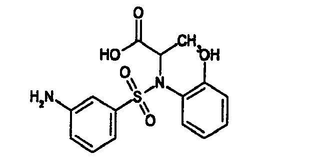

N-[(3-Aminophenyl)sulfonyl]-N-(2-hydroxyphenyl)alanine methyl ester (1.08 g) was suspended in methanol (9 ml), a 2 N aqueous sodium hydroxide solution (4.6 ml) was added thereto, and the mixture was agitated for 30 minutes at 50°C. The reaction solution was poured into water, and the reaction mixture was acidified with 2 N hydrochloric acid to be weakly acidic (pH 4), saturated with sodium chloride and extracted with ethyl acetate/THF (2 : 1). The organic layer was washed with saturated brine, and dried over anhydrous magnesium sulfate. Then, the solvent was distilled off under reduced pressure, and thus obtained crystals were collected by filtration to give the desired product (593 mg).

¹H-NMR (DMSO-d₆) δ 0.98 (3H, d), 4.47 (1H, q), 5.60 (3H, br s), 6. 55-6. 58 (3H, m), 6.74-6.80 (3H, m), 6. 91 (1H, s), 7.09-7.18 (2H, m)

### Reference Example 79

### 4-[(3-Aminophenyl)sulfonyl]-3-methyl-3,4-dihydro-2H-1,4-benzoxazin-2-one

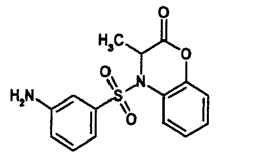

While dehydrating a mixture of N-[(3-aminophenyl)sulfonyl]-N-(2-hydroxyphenyl)alanine (549 mg), p-toluenesulfonic acid monohydrate (621 mg) and toluene (40 ml) using a Dean-Stark trap, the mixture was heat under reflux for 1 hour. Then, the mixture was poured into a saturated sodium bicarbonate solution, and extracted with ethyl acetate and THF (3 : 1). The organic layer was sequentially washed with water and saturated brine, and dried over anhydrous magnesium sulfate. Then, the solvent was distilled off under reduced pressure. The residue was subjected to silica gel column chromatography, and the fraction eluted with ethyl acetate/hexane (1 : 2) was concentrated under reduced pressure. Thus obtained crystals were collected by filtration to give the desired product (320 mg).

¹H-NMR (CDCl₃) δ 1.35 (3H, d), 3.81 (2H, s), 4.98 (1H, q), 6.69 (1H, t), 6.71-6.74 (1H, m), 6.77-6.80 (1H, m), 6.94 (1H, dd), 7.11 (1H, t), 7.23 (1H, dt), 7.29 (1H, dt), 7.75 (1H, dd)

### Reference Example 80

### Methyl 3-(2,4-dioxo-1,4-dihydroquinazolin-3(2H)-yl)benzoate

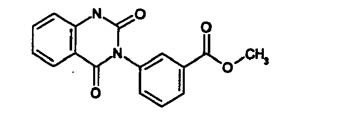

A solution of methyl 3-aminobenzoate (4.53 g), methyl 2-isocyanatobenzoate (6.38 g), DMAP (4.40 g) and THF (140 ml) was agitated for 18 hours at room temperature. The mixture was concentrated under reduced pressure until the total amount was reduced in about half. Sodium methoxide (28% methanol solution, 6.95 g) was added thereto, and the mixture was heated for 15 minutes at 70°C, and then poured into ice water. The reaction mixture was acidified with 1 N hydrochloric acid to be weakly acidic. The precipitated crystals were collected by filtration, and sequentially washed with water, ethanol and diethyl ether to give the desired product (8.11 g).

¹H-NMR (DMSO-d₆) δ 3.88 (3H, s), 7.19-7.26 (2H, m), 7.63-7.74 (3H, m), 7.92-8.04 (3H, m), 11.59 (1H, s)

### Reference Example 81

### 3-(2,4-dioxo-1,4-dihydroquinazolin-3(2H)-yl)benzoic acid

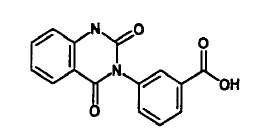

Methyl 3-(2,4-dioxo-1,4-dihydroquinazolin-3(2H)-yl)benzoate (8.01 g) was suspended in methanol (60 ml), a 2 N aqueous sodium hydroxide solution (40.6 ml) was added thereto, and the mixture was agitated for 2 hours at 60°C. The reaction solution was poured into water, and the reaction mixture was acidified with concentrated hydrochloric acid. Then, the precipitated crystals were collected by filtration, and sequentially washed with water, ethanol and diethyl ether to give the desired product (6.95 g).

¹H-NMR (DMSO-d₆) δ 7.21-7.26 (2H, m), 7.58-7.65 (2H, m), 7.70 (1H, t), 7.90 (1H, s), 7.95 (1H, d), 7.99-8.01 (1H, m), 11.60 (1H, s)

### Reference Examples 81(1) to 81(3)

In the same manner as in Reference Example 81, the following compounds were obtained from the corresponding esters (Reference Examples 101, 99(1) and 100).

### Reference Example 81(1)

### 6-(2,4-Dioxo-1,4-dihydroquinazolin-3(2H)-yl)pyridine-2-carboxylic acid

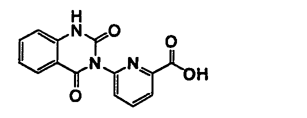

¹H-NMR (DMSO-d₆) δ 7.23-7.33 (2H, m), 7.70-7.82 (2H, m), 7.96 (1H, dd), 8.13-8.25 (2H, m), 11.73 (1H, s)

### Reference Example 81(2)

### 5-Chloro-4-(2,4-dioxo-1,4-dihydroquinazolin-3(2H)-yl)thiophene-2-carboxylic acid

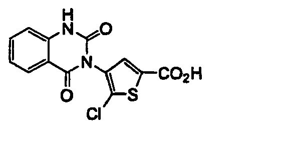

¹H-NMR (DMSO-d₆) δ 7.21-7.32 (2H, m), 7.70-7.81 (2H, m), 7.94-8.00 (1H, m), 11.77 (1H, br s), 13.65 (1H, br s)

### Reference Example 81(3)

### 5-(2,4-Dioxo-1,4-dihydroquinazolin-3(2H)-yl)-4-methylthiophene-3-carboxylic acid

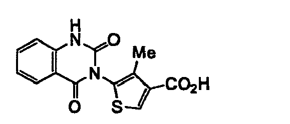

¹H-NMR (DMSO-d₆) δ 2.08 (3H, s), 7.21-7.29 (2H, m), 7.68-7.77 (1H, m), 7.95 (1H, dd), 8.31 (1H, s), 11.69 (1H, br s), 12.71 (1H, br s)

### Reference Example 82

### 2-Bromo-6-nitrobenzoic acid

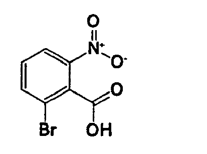

1-Bromo-2-methyl-3-nitrobenzene (15.0 g) and sodium carbonate (34.0 g) were suspended in water (1.05 1), and then potassium permanganate (43.8 g) was added portionwise thereto at 75°C. After stirring the mixture overnight at 110°C, the temperature was returned to room temperature, and insolubles were filtered off. The filtrate was acidified with 6 N hydrochloric acid at 0°C, and then extracted with ethyl acetate. The organic layer was washed with saturated brine and dried over magnesium sulfate, and the solvent was distilled off under reduced pressure. The resulting residue was crystallized from diisopropyl ether to give the desired product (5.11 g) as crystals.

¹H-NMR (DMSO-d₆) δ 7.65 (1H, t), 8.15 (1H, dd), 8.23 (1H, dd), 14.28 (1H, s)

### Reference Example 83

### 2-(2-Ethoxy-2-oxoethyl)-6-nitrobenzoic acid

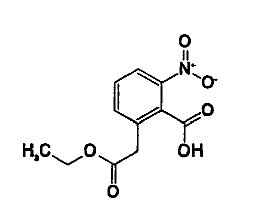

To a solution of sodium ethoxide (11.1 g, 20% ethanol solution) in ethanol (240 ml), 2-bromo-6-nitrobenzoic acid (4.0 g), ethyl acetoacetate (2.12 g) and copper bromide (I) (70 mg) were added, and then the mixture was stirred overnight at 90°C under argon atmosphere. After returning the temperature to room temperature, the solvent was distilled off under reduced pressure. The resulting residue was acidified with 1 N hydrochloric acid, diluted with water, and extracted with ethyl acetate. The organic layer was washed with saturated brine and dried over magnesium sulfate, and the solvent was distilled off under reduced pressure. The resulting residue was crystallized from hexane/diisopropyl ether to give the desired product (1.93 g) as crystals.

¹H-NMR (CDCl₃) δ 1.26 (3H, t), 3.87 (2H, s), 4.19 (2H, q), 7.49-7.70 (2H, m), 8.06 (1H, dd)

### Reference Example 84

### Methyl 2-(2-ethoxy-2-oxoethyl)-6-nitrobenzoate

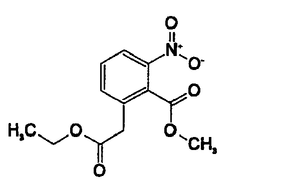

To a suspension of 2-(2-ethoxy-2-oxoethyl)-6-nitrobenzoic acid (1.9 g) and potassium carbonate (1.35 g) in DMF (20 ml), methyl iodide (0.61 ml) was added, and the mixture was stirred for 2 hours at room temperature under nitrogen atmosphere. Water was added to the reaction solution, and the mixture was extracted with ethyl acetate, washed saturated brine and dried over magnesium sulfate. The solvent was distilled off under reduced pressure, and the residue was crystallized from hexane to give the desired product (1.51 g) as crystals.

¹H-NMR (CDCl₃) δ 1.25 (3H, t), 3.78 (2H, s), 3.93 (3H, s), 4.16 (2H, q), 7. 56 (1H, t), 7.65 (1H, dd), 8.03 (1H, dd)

### Reference Example 85

### Methyl 2-(2-ethoxy-1-methyl-2-oxoethyl)-6-nitrobenzoate

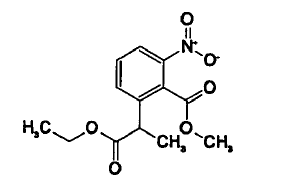

To a solution of methyl 2-(2-ethoxy-2-oxoethyl)-6-nitrobenzoate (500 mg) in dry THF (20 ml), lithium diisopropylamide (1.22 ml, 2.0 N heptane-THF-ethylbenzene solution) was added dropwise at -78°C under argon atmosphere. After stirring the mixture for 1.5 hours as it was, methyl iodide (0.16 ml) was added thereto and the mixture was stirred overnight returning the temperature to room temperature. The reaction solution was acidified with 0.1 N hydrochloric acid at 0°C and extracted with ethyl acetate. The organic layer was washed with saturated brine and dried over magnesium sulfate. The solvent was distilled off under reduced pressure, and the residue was purified with silica gel column chromatography to give the desired product (396 mg) as an oily matter.

¹H-NMR (CDCl₃) δ 1.21 (3H, t), 1.54 (3H, d), 3.90 (1H, q), 3.97 (3H, s), 4.08-4.18 (2H, m), 7.58 (1H, t), 7.77 (1H, dd), 8.06 (1H, dd)

### Reference Example 86

### 3-(6,7,8,9-Tetrahydro-5H-benzo[7]annulen-5-ylthio)aniline

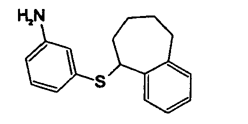

To a solution of 6,7,8,9-tetrahydro-5H-benzo[7]annulen-5-ol (500 mg), 3-aminothiophenol (463 mg), tributylphosphine (1.87 g) in THF (40 ml), 1,1'-(azodicarbonyl)dipiperidine (2.33 g) was added portionwise at 0°C under nitrogen atmosphere, and the mixture was stirred overnight. Water was added to the reaction solution, and the mixture was extracted with ethyl acetate. The organic layer was washed with saturated brine and dried over magnesium sulfate. After distilling off the solvent thereof under reduced pressure, ethyl acetate was added thereto and insolubles were filtered off. The filtrate was concentrated under reduced pressure, and then the residue was purified with silica gel column chromatography to give the desired product (607 mg) as an oily matter.

¹H-NMR (CDCl₃) δ 1.40-1.54 (1H, m), 1.76-2.02 (3H, m), 2.08-2.26 (2H, m), 2.77 (1H, dd), 3.25-3.41 (1H, m), 3.59 (2H, br s), 4.49 (1H, dd), 6.49-6.55 (1H, m), 6.61-6.64 (1H, m), 6.71-6.75 (1H, m), 6.98-7.13 (5H, m)

### Reference Example 86(1)

In the same manner as in Reference Example 86, the following compound was obtained from reacting 1-phenylethanethiol with 3-aminothiophenol.

### 3-[(1-Phenylethyl)thio]aniline

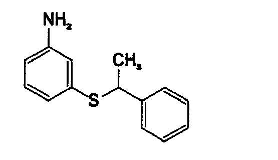

¹H-NMR (CDCl₃) δ 1.62 (3H, d), 3.59 (2H, br s), 4.33 (1H, q), 6.52 (1H, dd), 6.62 (1H, t), 6.71 (1H, d), 7.01 (1H, t), 7.15-7.42 (5H, m)

### Reference Example 87

### 2-Chloro-5-(2,3,4,5-tetrahydro-1H-1-benzazepin-1-ylsulfonyl)thiophen-3-amine

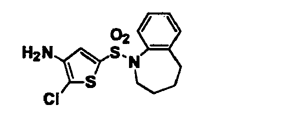

To a mixture of 1-[(5-chloro-4-nitro-2-thienyl)sulfonyl]-2,3,4,5-tetrahydro-1H-1-benzazepine (2.31 g), ammonium chloride (1.66 g) and methanol (150 ml), zinc (8.11 g) was added several times portionwise at 60°C and the mixture was stirred for 30 minutes at 60°C. The insolubles were removed by subjecting the reaction solution to filtration, and the filtrate was distilled off under reduced pressure. The resulting residue was purified with silica gel column chromatography (hexane/ethyl acetate (6 : 1 to 2 : 1)) to give the desired product (1.07 g) as an oily matter.

¹H-NMR (CDCl₃) δ 1.55-1.66 (2H, m), 1.83-1.95 (2H, m), 2.58 (2H, t), 3.68 (2H, br s), 3.73 (2H, br s), 6.92 (1H, s), 7.16-7.36 (4H, m)

### Reference Example 87(1)

In the same manner as in Reference Example 87, the following compound was obtained from N-ethyl-5-nitro-N-phenylthiophene-3-carboxamide.

### 5-Amino-N-ethyl-N-phenylthiophene-3-carboxamide

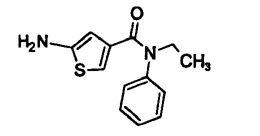

¹H-NMR (CDCl₃) δ 1.19 (3H, t), 3.58 (2H, br s), 3.90 (2H, q), 6.05 (1H, d), 6.29 (1H, d), 7.10-7.15 (2H, m), 7.27-7.43 (3H, m)

### Reference Example 88

### Methyl 2-amino-6-(2-ethoxy-2-oxoethyl)benzoate

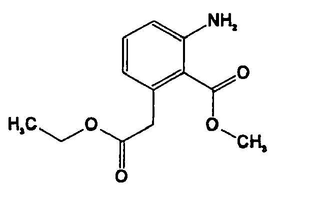

To a solution of methyl 2-(2-ethoxy-2-oxoethyl)-6-nitrobenzoate (1.45 g) in methanol (20 ml), 10% palladium carbon (700 mg, hydrated) was added, and then the mixture was stirred overnight under hydrogen atmosphere. Insolubles were filtered off, and then the solvent was distilled off under reduced pressure to give the desired product (1.28 g) as an oily matter.

¹H-NMR (CDCl₃) δ 1.25 (3H, t), 3.81 (2H, s), 3.83 (3H, s), 4.15 (2H, q), 5.38 (2H, br s), 6.51-6.55 (1H, m), 6.63 (1H, dd), 7.12-7.19 (1H, m)

### Reference Example 88(1)

In the same manner as in Reference Example 88, the following compound was obtained from methyl 2-(2-ethoxy-1-methyl-2-oxoethyl)-6-nitrobenzoate.

### Methyl 2-amino-6-(2-ethoxy-1-methyl-2-oxoethyl)benzoate

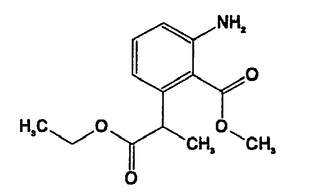

¹H-NMR (CDCl₃) δ 1.19 (3H, t), 1.49 (3H, d), 3.87 (3H, s), 4.02-4.19 (3H, m), 4.96 (2H, br s), 6.60 (1H, dd), 6.68 (1H, dd), 7.17 (1H, t)

### Reference Example 89

### Methyl 5-chloro-4-[5-(hydroxymethyl)-2,4-dioxo-1,4-dihydroquinazolin-3(2H)-yl]thiophene-2-carboxylate

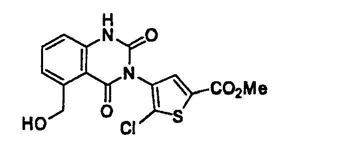

To a solution of methyl 4-[5-({[tert-butyl(dimethyl)silyl]oxy}methyl)-2,4-dioxo-1,4-dihydroquinazolin-3(2H)-yl]-5-chlorothiophene-2-carboxylate (11.0 g) in THF (200 ml), tetrabutylammonium fluoride (45 ml, 1 N THF solution) was added dropwise under nitrogen atmosphere, and then the mixture was stirred for 1 day as it was. Water was added thereto, and the mixture was extracted twice with ethyl acetate. The organic layers were washed with saturated brine and dried over magnesium sulfate. The solvent was distilled off under reduced pressure. The resulting residue was purified with silica gel column chromatography (ethyl acetate), and thus obtained solid was washed with diisopropyl ether to give the desired product (8.4 g) as crystals.

¹H-NMR (DMSO-d₆) δ 3.86 (3H, s), 4.94 (2H, d), 5.28 (1H, t), 7.14 (1H, d), 7.51 (1H, d), 7.70 (1H, t), 7.86 (1H, s), 11.70 (1H, br s)

### Reference Example 90

### Methyl 5-chloro-4-(5-formyl-2,4-dioxo-1,4-dihydroquinazolin-3(2H)-yl)thiophene-2-carboxylate

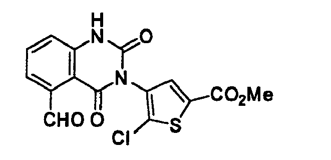

To a solution of methyl 5-chloro-4-[5-(hydroxymethyl)-2,4-dioxo-1,4-dihydroquinazolin-3(2H)-yl]thiophene-2-carboxylate (8.4 g) in DMF (200 ml), manganese dioxide (50 g) was added, and then the mixture was stirred for 5 hours at room temperature. Insolubles were filtered off, and then the filtrate was diluted with ethyl acetate and washed with water. The organic layer was washed with saturated brine and dried over magnesium sulfate. The solvent was distilled off under reduced pressure, and the resulting residue was washed with hexane and diisopropyl ether to give the desired product (7.75 g) as crystals.

¹H-NMR (DMSO-d₆) δ 3.86 (3H, s), 7.45 (1H, dd), 7.50 (1H, dd), 7.86 (1H, t), 7.88 (1H, s), 10.78 (1H, s), 12.06 (1H, br s)

### Reference Example 91

### 1-[3-Nitro-4-(trifluoromethyl)benzoyl]-1,2,3,4-tetrahydroquinoline

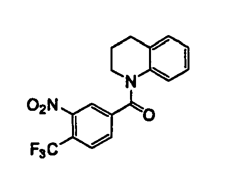

3-Nitro-4-trifluoromethylbenzoic acid (1.54 g), thionyl chloride (0.81 ml) and DMF (0.02 ml) was heated in toluene (30 ml) at 100°C. The reaction solution was stirred for 7 hours followed by distilling off the solvent thereof under reduced pressure, and dissolving the resulting residue in THF (10 ml). This solution was added to a mixture of 1,2,3,4-tetrahydroquinoline (1.07 ml), sodium hydrogen carbonate (1.10 g) and THF (20 ml), which were cooled to 5°C, and then the reaction mixture was stirred for 16 hours at room temperature. The reaction solution was diluted with ethyl acetate and an aqueous sodium hydrogen carbonate solution. Subsequently, the organic layer was washed with an aqueous sodium hydrogen carbonate solution, 1 N hydrochloric acid and saturated brine and dried over anhydrous magnesium sulfate. After filtering the drying agent, the solvent was distilled off under reduced pressure to give the desired product (1.90 g) as crystals.

¹H-NMR (CDCl₃) δ 2.05-2.16 (2H, m), 2.87 (2H, t), 3.94 (2H, t), 6.56 (1H, br s), 6.93 (1H, t), 7.09 (1H, t), 7.22 (1H, d), 7.55-7.64 (1H, m), 7.67-7.75 (1H, m), 7.85 (1H, s)

### Reference Example 92

### N-[2-Chloro-5-(3,4-dihydroquinolin-1(2H)-ylsulfonyl)phenyl]-5-fluoro-2-nitrobenzamide

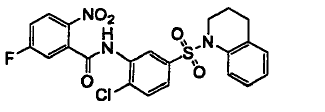

5-Fluoro-2-nitrobenzoic acid (0.70 g), thionyl chloride (1.0 ml) and DMF (0.01 ml) were heated under reflux in toluene (15 ml) for 3 hours. The reaction solution was concentrated under reduced pressure, and the resulting residue was dissolved in THF (10 ml). This solution was added to a solution of 2-chloro-5-(3,4-dihydro-1(2H)-quinolinylsulfonyl)aniline (1.00 g) and sodium hydrogen carbonate (0.80 g) in THF (10 ml), which was cooled to 5°C, and the mixture was stirred for 16 hours at room temperature. The reaction solution was diluted with ethyl acetate and an aqueous sodium hydrogen carbonate solution. The organic layer was separated and dried over anhydrous magnesium sulfate. After filtering the drying agent, the solvent was distilled off under reduced pressure. The resulting residue was purified with silica gel column chromatography, and recrystallized from hexane/ethyl acetate to give the desired product (0.92 g) as crystals.

¹H-NMR (CDCl₃) δ 1.75-1.90 (2H, m), 2.56 (2H, t), 3.85-3.96 (2H, m), 7.01-7.25 (3H, m), 7.28-7.41 (3H, m), 7.41-7.50 (1H, m), 7.77 (1H, d), 7. 85 (1H, s), 8.25 (1H, dd), 8.78 (1H, s)

### Reference Examples 92 (1) to 92(4)

In the same manner as in Reference Example 92, the following compounds were obtained from the corresponding carboxylic acid (commercially available or conventional) and 2-chloro-5-(3,4-dihydro-1(2H)-quinolinylsulfonyl)aniline.

### Reference Example 92 (1)

### N-[2-Chloro-5-(3,4-dihydroquinolin-1(2H)-ylsulfonyl)phenyl]-5-methoxy-2-nitrobenzamide

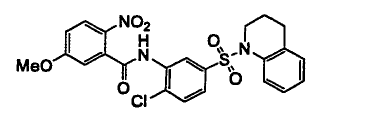

¹H-NMR (CDCl₃) δ 1.77-1.90 (2H, m), 2.53-2.62 (2H, m), 3.87-4.00 (5H, m), 7.00-7.13 (4H, m), 7.15-7.24 (1H, m), 7.31-7.38 (1H, m), 7.43 (1H, dd), 7.73-7.84 (2H, m), 8.22 (1H, dd), 8.80-8.89 (1H, m)

### Reference Example 92(2)

### 5-Bromo-N-[2-chloro-5-(3,4-dihydroquinolin-1(2H)-ylsulfonyl)phenyl]-2-nitrobenzamide

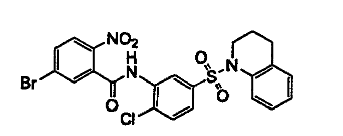

¹H-NMR (CDCl₃) δ 1.74-1.87 (2H, m), 2.56 (2H, t), 3.86-3.95 (2H, m), 7.01-7.12 (2H, m), 7.15-7.24 (1H, m), 7.36 (1H, dd), 7.44 (1H, d), 7.72-7.90 (4H, m), 8.06 (1H, d), 8.77 (1H, s)

### Reference Example 92(3)

### N-[2-Chloro-5-(3,4-dihydroquinolin-1(2H)-ylsulfonyl)phenyl]-4-fluoro-2-nitrobenzamide

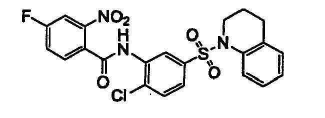

¹H-NMR (CDCl₃) δ 1.76-1.88 (2H, m), 2.56 (2H, t), 3.86-3.95 (2H, m), 7.00-7.12 (2H, m), 7.14-7.23 (1H, m), 7.33-7.39 (1H, m), 7.41-7.54 (2H, m), 7.66 (1H, dd), 7.76 (1H, d), 7.83-7.91 (2H, m), 8.78 (1H s)

### Reference Example 92(4)

### 4-Bromo-N-[2-chloro-5-(3,4-dihydroquinolin-1(2H)-ylsulfonyl)phenyl]-2-nitrobenzamide

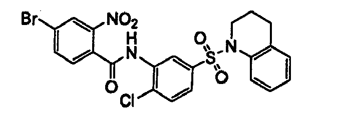

¹H-NMR (DMSO-d₆) δ 1.64-1.78 (2H, m), 2.46-2.57 (2H, m), 3.75-3.88 (2H, m), 7.06-7.25 (3H, m), 7.39 (1H, dd), 7.60 (1H, d), 7.69-7.83 (2H, m), 8.13 (1H, dd), 8.19 (1H, s), 8.41 (1H, d), 10.66 (1H, s)

### Reference Example 93

### N-[5-(3,4-Dihydroquinolin-1(2H)-ylcarbonyl)-2-(trifluoromethyl)phenyl]-2-nitrobenzamide

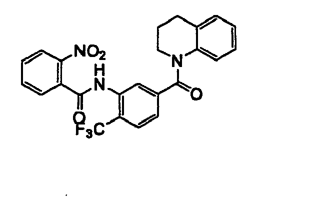

A mixture of 5-(3,4-dihydroquinolin-1(2H)-ylcarbonyl)-2-(trifluoromethyl)aniline (150 mg), sodium hydrogen carbonate (130 mg) and THF (5 ml) was cooled to 5°C, and a solution of 2-nitrobenzoyl chloride (95 mg) in THF (2 ml) was slowly added thereto. After stirring for 48 hours at room temperature, the reaction solution was diluted with ethyl acetate and an aqueous sodium hydrogen carbonate solution, and extracted with ethyl acetate. The organic layer was dried over anhydrous magnesium sulfate, and then the solvent was distilled off under reduced pressure. The resulting residue was purified with silica gel column chromatography, and recrystallized from hexane/ethyl acetate to give the desired product (120 mg) as crystals.

¹H-NMR (CDCl₃) δ 2. 02-2. 14 (2H, m), 2.87 (2H, t), 3.91 (2H, t), 6.91-7.11 (3H, m), 7.19 (1H, d), 7.24-7.33 (1H, m), 7.53-7. 82 (5H, m), 8.16 (1H, d), 8.31 (1H, s)

### Reference Example 93 (1)

In the same manner as in Reference Example 93, the following compound was obtained from reacting 2-chloro-5-(3,4-dihydro-1(2H)-quinolinylsulfonyl)aniline with 2-nitrobenzoyl chloride.

### N-[2-Chloro-5-(3,4-dihydroquinolin-1(2H)-ylsulfonyl)phenyl]-2-nitrobenzamide

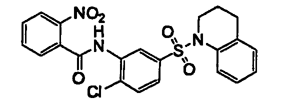

¹H-NMR (CDCl₃) δ 1.76-1.89 (2H, m), 2.57 (2H, t), 3.85-3.97 (2H, m), 7.00-7.12 (2H, m), 7.14-7.24 (1H, m), 7.35 (1H, dd), 7.43 (1H, d), 7.60-7.83 (4H, m), 7.88 (1H, br s), 8.17 (1H, dd), 8.83 (1H, br s)

### Reference Example 94

### 2-Chloro-5-(3,4-dihydroquinolin-1(2H)-ylsulfonyl)-N-(2-nitrobenzyl)aniline .

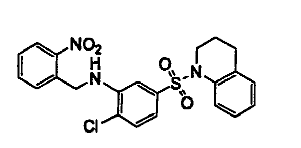

A solution of N-[2-chloro-5-(3,4-dihydroquinolin-1(2H)-ylsulfonyl)phenyl]-2-nitrobenzamide (650 mg) in THF (15 ml) was cooled to 5°C, and then a 1.1 mol/L solution of borane in THF (5 ml) was added thereto. The reaction solution was stirred for 36 hours at 55°C followed by diluting with an aqueous sodium hydrogen carbonate solution. The reaction solution was extracted with ethyl acetate. Then, the organic layer was dried over anhydrous magnesium sulfate, and the solvent was distilled off under reduced pressure. The resulting residue was recrystallized from hexane/ethyl acetate to give the desired product (496 mg) as crystals.

¹H-NMR (CDCl₃) δ 1.49-1.62 (2H, m), 2.40 (2H, t), 3.66-3.74 (2H, m), 4.56 (2H, d), 5.06-5.16 (1H, m), 6.50 (1H, d), 6.90-6.99 (2H, m), 7.00-7.15 (2H, m), 7.31 (1 H, d), 7.37 (1H, d), 7.42-7.60 (2H, m), 7.70 (1H, dd), 8.12 (1 H, dd)

### Reference Examples 94(1) to 94(5)

In the same manner as in Reference Example 94, the following compounds were obtained from the compounds of Reference Examples 92 and 92 (1) to 92 (4) .

### Reference Example 94(1)

### 2-Chloro-5-(3,4-dihydroquinolin-1(2H)-ylsulfonyl)-N-(5-fluoro-2-nitrobenzyl)aniline

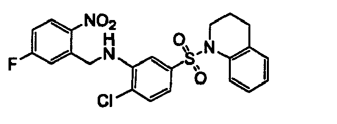

¹H-NMR (CDCl₃) δ 1.49-1.63 (2H, m), 2.40 (2H, t), 3.66-3.75 (2H, m), 4.60 (2H, d), 5.10 (1H, t), 6.38 (1H, d), 6.86-7.18 (6H, m), 7.35 (1H, d), 7.69 (1H, dd), 8.24 (1H, dd)

### Reference Example 94(2)

### 2-Chloro-5-(3,4-dihydroquinolin-1(2H)-ylsulfonyl)-N-(5-methoxy-2-nitrobenzyl)aniline

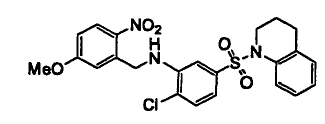

¹H-NMR (CDCl₃) δ 1. 50-1.61 (2H, m), 2.39 (2H, t), 3.66-3.75 (2H, m), 3.83 (3H, s), 4.59 (2H, d), 5.16 (1H, t), 6.51 (1H, d), 6.82-6.97 (4H, m), 6.99-7.13 (2H, m), 7.31 (1H, d), 7.68 (1H, dd), 8.23 (1H, d)

### Reference Example 94(3)

### N-(5-Bromo-2-nitrobenzyl)-2-chloro-5-(3,4-dihydroquinolin-1(2H)-ylsulfonyl)aniline

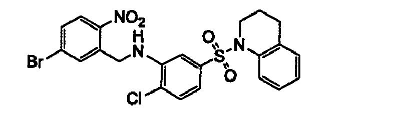

¹H-NMR (CDCl₃) δ 1.48-1. 62 (2H, m), 2.40 (2H, t), 3. 67-3. 75 (2H, m), 4.56 (2H, d), 5.07 (1H, t), 6.43 (1H, d), 6.89-7.15 (4H, m), 7.34 (1H, d), 7.53 (1H, d), 7.61 (1H, dd), 7.72 (1H, dd), 8.02 (1H, d)

### Reference Example 94(4)

### 2-Chloro-5-(3,4-dihydroquinolin-1(2H)-ylsulfonyl)-N-(4-fluoro-2-nitrobenzyl)aniline

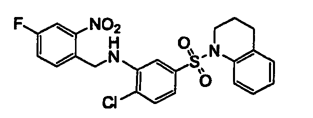

¹H-NMR (CDCl₃) δ 1. 50-1. 63 (2H, m), 2.41 (2H, t), 3.68-3.75 (2H, m), 4.52 (2H, d), 5.09 (1H, t), 6.43 (1H, d), 6.93-7.14 (4H, m), 7.22-7.39 (3H, m), 7.69 (1H, dd), 7.85 (1H, dd)

### Reference Example 94(5)

### N-(4-Bromo-2-nitrobenzyl)-2-chloro-5-(3,4-dihydroquinolin-1 (2H)-ylsulfonyl) aniline

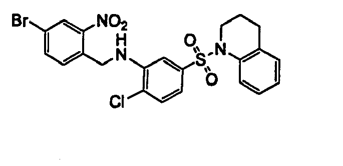

¹H-NMR (CDCl₃) δ 1.46-1.68 (2H, m), 2.40 (2H, t), 3.63-3.78 (2H, m), 4.51 (2H, d), 5.04-5.15 (1H, m), 6.41 (1H, s), 6.92-7.15 (4H, m), 7.18-7.39 (2H, m), 7.60-7.74 (2H, m), 8.26 (1H, s)

### Reference Example 95

### 5-Bromo-2-nitrobenzoic acid

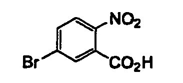

A solution of 5-amino-2-nitrobenzoic acid (25.3 g) in hydrobromic acid (300 ml) was cooled to 5°C, and 80 ml of an aqueous solution of sodium nitrite (10 g) was added thereto over 30 minutes. The reaction solution was stirred for 30 minutes at 5°C. This reaction solution was added dropwise to a solution of copper bromide (I) (11.50 g) in hydrobromic acid (100 ml), and the reaction mixture was heated to 70°C and further stirred for 1 hour at 70°C. After cooling the reaction solution to room temperature, it was diluted with water. The obtained solid was separated by filtration and air-dried. The resulting solid was purified with silica gel column chromatography and recrystallized from hexane/ethyl acetate to give the desired product (11.5 g) as crystals.

¹H-NMR (CDCl₃) δ 7.77-7.86 (2H, m), 8.00 (1H, d), 8.22 (1H, s)

### Reference Example 96

### Ethyl [2-chloro-5-(3,4-dihydroquinolin-1(2H)-ylsulfonyl)phenyl]carbamate

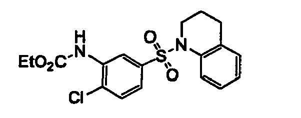

A solution of 2-chloro-5-(3,4-dihydro-1(2H)-quinolinylsulfonyl)aniline (8.06 g) and ethyl chlorocarbonate (4.8 ml) in toluene (50 ml) was stirred for 2 hours at 95°C, ethyl chlorocarbonate (2.4 ml) was added thereto, and then the mixture was further stirred for 4 hours. The reaction solution was concentrated under reduced pressure, and the resulting residue was recrystallized from ethyl acetate/isopropyl ether to give the desired product (8.84 g) as crystals.

¹H-NMR (DMSO-d₆) δ 1.24 (3H, t), 1.58-1.72 (2H, m), 2.42-2.50 (2H, m), 3.73-3.81 (2H, m), 4.14 (2H, q), 7.06-7.12 (2H, m), 7.14-7.22 (1H, m), 7.24 (1H, dd), 7.57 (1H, d), 7.63 (1H, d), 8.01 (1H, d), 9.33 (1H, br s)

### Reference Example 97

### 1-Bromo-2-(bromomethyl)-3-nitrobenzene

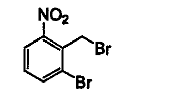

A solution of 2-bromo-6-nitrotoluene (20.70 g), N-bromosuccinimide (24.0 g) and 2,2'-azobis(isobutyronitrile) (900 mg) in chlorobenzene (120 ml) was stirred for 3 hours at 100°C, and then the reaction solution was concentrated under reduced pressure. The resulting residue was diluted with ethyl acetate and water, and the organic layer was then washed with an aqueous sodium hydrogen carbonate solution and saturated brine. The organic layer was dried over anhydrous magnesium sulfate and concentrated, and the resulting residue was washed with hexane to give the desired product (24.50 g) as crude crystals.

¹H-NMR (CDCl₃) δ 4.89 (2H, s), 7.36 (1H, t), 7.84-7.92 (2H, m)

### Reference Example 98

### Ethyl (2-bromo-6-nitrobenzyl)[2-chloro-5-(3,4-dihydroquinolin-1(2H)-ylsulfonyl)phenyl]carbamate

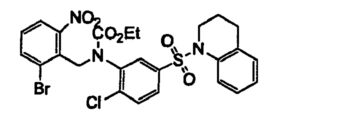

A solution of 1-bromo-2-(bromomethyl)-3-nitrobenzene (3.60 g), ethyl [2-chloro-5-(3,4-dihydroquinolin-1(2H)-ylsulfonyl)phenyl]carbamate (4.50 g) and cesium carbonate (4.80 g) in acetone (60 ml) was stirred for 6 hours at 60°C, and then the reaction solution was concentrated under reduced pressure. The resulting residue was diluted with ethyl acetate and water, and the organic layer was then washed with an aqueous sodium hydrogen carbonate solution and saturated brine. The organic layer was dried over anhydrous magnesium sulfate, concentrated, and the resulting residue was purified with silica gel column chromatography to give the desired product (5.40 g) as an amorphous.

¹H-NMR (CDCl₃) δ 1.02-1.41 (3H, m), 1.58-1.70 (2H, m), 2.44 (2H, t), 3.52-3.82 (2H, m), 3.98-4.31 (2H, m), 4.92 (1H, d), 5.45 (1H, d), 6.93-7.04 (2H, m), 7.05-7.22 (2H, m), 7.26 (1H, t), 7.34 (1H, dd), 7.43 (1H, d), 7.52-7.75 (3H, m)

### Reference Example 99

### 3-(6-Bromopyridin-2-yl)quinazoline-2,4(1H,3H)-dione

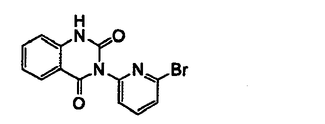

A solution of 2-amino-6-bromopyridine (6.17 g), methyl 2-isocyanatobenzoate (6.40 g) and 4-dimethylaminopyridine (520 mg) in THF (30 ml) was stirred for 16 hours at room temperature. The reaction solution was diluted with ethyl acetate and an aqueous sodium hydrogen carbonate solution. Then, the organic layer was separated and dried over anhydrous magnesium sulfate. The resulting residue was dissolved in THF (50 ml), whereto a 28% solution of sodium methoxide in methanol (5 ml) was added, and the mixture was stirred for 30 minutes at 60°C. The reaction solution was diluted with ethyl acetate and 1 N hydrochloric acid. Then, the organic layer was separated, dried over anhydrous magnesium sulfate and concentrated. The resulting residue was recrystallized from ethyl acetate/THF to give the desired product (8.70 g) as crystals.

¹H-NMR (DMSO-d₆) δ 7.20-7.32 (2H, m), 7.63 (1H, d), 7.69-7.78 (1H, m), 7.81 (1H, dd), 7.91-7.98 (1H, m), 8.01 (1H, d)

### Reference Example 99(1)

In the same manner as in Reference Example 99, the following compound was obtained from reacting methyl 4-amino-5-chlorothiophene-2-carboxylate with methyl 2-isocyanatobenzoate.

### Methyl 5-chloro-4-(2,4-dioxo-1,4-dihydroquinazolin-3(2H)-yl)thiophene-2-carboxylate

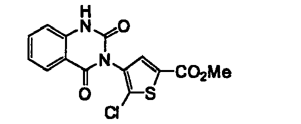

¹H-NMR (DMSO-d₆) δ 3.86 (3H, s), 7.23-7.32 (2H, m), 7.71-7.79 (1H, m), 7.89 (1H, s), 7.97 (1H, dd), 11.79 (1H, br s)

### Reference Example 100

### Ethyl 5-(2,4-dioxo-1,4-dihydroquinazolin-3(2H)-yl)-4-methylthiophene-3-carboxylate

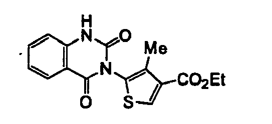

To a solution of ethyl 4-methyl-5-nitrothiophene-3-carboxylate (594 mg) and reduced iron (900 mg) in methanol (15 ml), which was cooled to 5°C, concentrated hydrochloric acid (3 ml) was added and the mixture was stirred for 2 minutes. The reaction solution was diluted with ethyl acetate and an aqueous sodium hydrogen carbonate solution, and then the organic layer was separated and dried over anhydrous magnesium sulfate. After the drying agent was separated by filtration, the organic layer was concentrated under reduced pressure, and the resulting residue was dissolved in THF (15 ml). Methyl 2-isocyanatobenzoate (540 mg) and 4-dimethylaminopyridine (170 mg) were added to this solution and the mixture was stirred for 1 hour at room temperature. The reaction solution was diluted with ethyl acetate, and then the organic layer was washed with 1 N hydrochloric acid and an aqueous sodium hydrogen carbonate solution. The organic layer was dried over anhydrous magnesium sulfate and concentrated, and the resulting residue was purified with NH silica gel column chromatography. The resulting solid was dissolved in THF (15 ml), a 28% solution of sodium methoxide in methanol (1.0 ml) was added thereto and the mixture was stirred for 5 minutes at room temperature. Then, the reaction solution was diluted with ethyl acetate and water. The organic layer was separated, washed with 1 N hydrochloric acid and saturated brine, and then dried over anhydrous magnesium sulfate. After the drying agent was separated by filtration, the solvent was distilled off under reduced pressure. The resulting residue was purified with silica gel column chromatography, and recrystallized from hexane/ethyl acetate to give the desired product (110 mg) as crystals.

¹H-NMR (DMSO-d₆) δ 1.31 (3H, t), 2.09 (3H, s), 4.28 (2H, q), 7.20-7.30 (2H,- m), 7.68-7.77 (1H, m), 7.95 (1H, dd), 8.36 (1H, s), 11.69 (1H, br s)

### Reference Example 101

### Ethyl 6-(2,4-dioxo-1,4-dihydroquinazolin-3(2H)-yl)pyridine-2-carboxylate

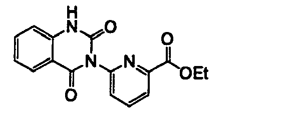

Under carbon monoxide atmosphere, a solution of 3-(6-bromopyridin-2-yl)quinazoline-2,4(1H,3H)-dione (7.00 g), palladium acetate (II) (500 mg), 1,1'-bis(diphenylphosphino)ferrocene (1.10 g) and triethylamine (6.1 ml) in ethanol (100 ml) was stirred for 16 hours at 70°C. After filtering the catalyst, the filtrate was diluted with ethyl acetate and an aqueous sodium hydrogen carbonate solution. The organic layer was separated, dried over anhydrous magnesium sulfate, and the solvent was distilled off under reduced pressure. The resulting residue was purified with NH silica gel column chromatography, and recrystallized from ethyl acetate/THF to give the desired product (3.10 g).

¹H-NMR (DMSO-d₆) δ 1.33 (3H, t), 4.37 (2H, q), 7.23-7.32 (2H, m), 7.70-7.79 (1H, m), 7.82 (1H, dd), 7.96 (1H, dd), 8.14-8.27 (2H, m), 11.75 (1H, br s)

### Reference Example 101(1)

In the same manner as in Reference Example 101, the following compound was obtained from 3-bromo-4-methylthiophene.

### Ethyl 4-methylthiophene-3-carboxylate

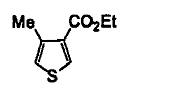

¹H-NMR (CDCl₃) δ 1.37 (3H, t), 2.46 (3H, d), 4.31 (2H, q), 6.90-6.95 (1H, m), 8.08 (1H, d)

### Reference Example 102

### 2-(2,4-Dioxo-1,4-dihydroquinazolin-3(2H)-yl)isonicotinic acid

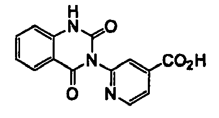

A solution of methyl 2-aminopyridine-4-carboxylate (3.76 g), methyl 2-isocyanatobenzoate (4.40 g) and 4-dimethylaminopyridine (400 mg) in THF (50 ml) was stirred for 16 hours at room temperature, and then a 28% solution of sodium methoxide in methanol (5 ml) was added to the reaction solution. The reaction solution was stirred for 30 minutes at 60°C, water was added thereto, and the reaction mixture was further stirred for 30 minutes. The reaction solution was diluted with 1 N hydrochloric acid, and then the resulting solid was separated by filtration and air-dried to give the desired product (4.17 g) as crystals.

¹H-NMR (DMSO-d₆) δ 7.20-7.32 (2H, m), 7.67-7.80 (1H, m), 7.88-8.02 (3H, m), 8.80 (1H, d), 11.69 (1H, s), 13.83 (1 H, br s)

### Reference Example 103

### Ethyl 5-aminonicotinate

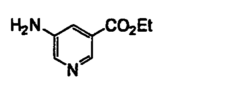

Under nitrogen atmosphere, a solution of ethyl 5-bromonicotinate (4.65 g), benzophenone imine (5.0 ml), sodium t-butoxide (3.0 g), tris(dibenzylideneacetone)dipalladium (0) (200 mg) and 2,2'-bis(diphenylphosphino)-1,1'-binaphthyl (400 mg) in toluene (50 ml) was stirred for 48 hours at 110°C. The reaction solution was diluted with ethyl acetate and an aqueous sodium hydrogen carbonate solution. Then, the organic layer was separated, washed with saturated brine and dried over anhydrous magnesium sulfate. The reaction solution was concentrated under reduced pressure, and the resulting residue was dissolved in a solution of 1 N hydrochloric acid/THF (50 ml/50 ml). The reaction mixture was stirred for 3 hours at room temperature. The reaction solution was diluted with ethyl acetate and water followed by extraction with 1 N hydrochloric acid. The collected aqueous layer was basified with an aqueous sodium hydrogen carbonate solution, and then extracted with ethyl acetate. The organic layer was dried over anhydrous magnesium sulfate and concentrated, and the resulting residue was purified with silica gel column chromatography to give the desired product (800 mg) as crystals.

¹H-NMR (CDCl₃) δ 1.40 (3H, t), 3.86 (2H, s), 4.39 (2H, q), 7.57 (1H, dd), 8.23 (1H, d), 8.62 (1H, d)

### Reference Example 103(1)

In the same manner as in Reference Example 103, the following compound was obtained from 1-[(5-bromopyridin-3-yl)sulfonyl]-1,2,3,4-tetrahydroquinoline.

### 5-(3,4-Dihydroquinolin-1(2H)-ylsulfonyl)pyridin-3-amine

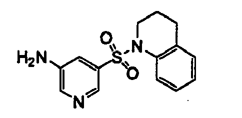

¹H-NMR (CDCl₃) δ 1.65-1.78 (2H, m), 2.49 (2H, t), 3.79-3.90 (4H, m), 7.00-7.06 (2H, m), 7.06-7.14 (1H, m), 7.16-7.24 (1H, m), 7.76 (1H, dd), 8.17 (1H, d), 8.20 (1H, d)

### Reference Example 104

### Methyl 5-[5-(hydroxymethyl)-2,4-dioxo-1,4-dihydroquinazolin-3(2H)-yl]nicotinate

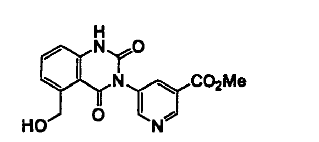

To a solution of 7-amino-2-benzofuran-1(3H)-one (270 mg) and triethylamine (0.30 ml) in THF (10 ml), which was cooled to 5°C, a solution of triphosgene (180 mg) in THF (10 ml) was added and the mixture was stirred for 1 hour at room temperature. A solution of ethyl 5-aminonicotinate (300 mg) in THF (10 ml) was added to the reaction solution, and the mixture was further stirred for 16 hours. The reaction solution was diluted with ethyl acetate and an aqueous sodium hydrogen carbonate solution. Then, the organic layer was separated and dried over anhydrous magnesium sulfate. The drying agent was separated by filtration, the filtrate was concentrated under reduced pressure, and the residue was then recrystallized from ethyl acetate. Thus obtained solid (290 mg) was dissolved in THF (10 ml), and a 28% solution of sodium methoxide in methanol (1 ml) was added thereto. After stirring the mixture for 1 hour at room temperature, the reaction solution was diluted with ethyl acetate and an aqueous ammonium chloride solution. The organic layer was separated, dried over anhydrous magnesium sulfate, and then concentrated under reduced pressure. The resulting residue was purified with silica gel column chromatography, and recrystallized from ethyl acetate to give the desired product (210 mg) as crystals.

¹H-NMR (DMSO-d₆) δ 3.92 (3H, s), 4.95 (2H, d), 5.26 (1H, t), 7.15 (1H, d), 7.50 (1H, d), 7.69 (1H, t), 8.38 (1H, t), 8.81 (1H, d), 9.11 (1H, d), 11.61 (1H, br s)

### Reference Example 104(1)

In the same manner as in Reference Example 104, the following compound was obtained from reacting ethyl 3-aminobenzoate with 7-amino-2-benzofuran-1(3H)-one.

### Ethyl 3-[5-(hydroxymethyl)-2,4-dioxo-1,4-dihydroquinazolin-3(2H)-yl]benzoate

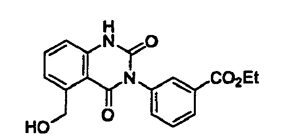

¹H-NMR (DMSO-d₆) δ 1.33 (3H, t), 4.34 (2H, q), 4.94 (2H, d), 5.24 (1H, t), 7.14 (1H, d), 7.48 (1H, d), 7.57-7.74 (3H, m), 7.91 (1H, s), 7.96-8.05 (1H, m), 11.53 (1H, s)

### Reference Example 105

### 5-(2,4-Dioxo-1,4-dihydroquinazolin-3(2H)-yl)nicotinic acid

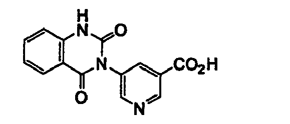

A solution of ethyl 5-aminonicotinate (224 mg), methyl 2-isocyanatobenzoate (287 mg) and 4-dimethylaminopyridine (250 mg) in THF (10 ml) was stirred for 4 hours at room temperature, and then a 28% solution of sodium methoxide in methanol (1 ml) was added thereto. The reaction mixture was further stirred for 3 hours. The reaction solution was diluted with an aqueous ammonium chloride solution, and then the formed precipitates were separated by filtration and washed with water and ethyl acetate. Thus obtained solid was dissolved in ethanol (20 ml), a 1 N aqueous sodium hydroxide solution (10 ml) was added thereto and the mixture was stirred for 3 hours at room temperature. The reaction solution was diluted with 1 N hydrochloric acid and concentrated under reduced pressure. The resulting solid was separated by filtration and washed with water to give the desired product (200 mg) as crystals.

¹H-NMR (DMSO-d₆) δ 7.22-7.33 (2H, m), 7.73 (1H, t), 7.96 (1H, d), 8.32-8.38 (1H, m), 8.80 (1H, d), 9.10 (1H, d), 11.69 (1H, br s), 13.61 (1H, br s)

### Reference Example 106

### 1-[(5-Bromopyridin-3-yl)sulfonyl]-1,2,3,4-tetrahydroquinoline

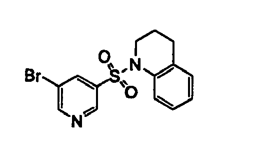

Pyridine-3-sulfonyl chloride hydrochloride (5.00 g) synthesized according to the article (Bioorganic & Medicinal Chemistry Letters, 12, 2097-2100, (2002)) and bromine (4.50 g) 5 was stirred for 8 hours at 120°C. After cooling, the reaction solution was diluted with THF (15 ml), and triethylamine (4.9 ml) and 1,2,3,4-tetrahydroquinoline (2.9 ml) were added thereto. Then, the reaction mixture was stirred for 24 hours at room temperature. The reaction solution was diluted with ethyl acetate and an aqueous sodium hydrogen carbonate solution. The organic layer was then separated, dried over anhydrous magnesium sulfate and concentrated. The resulting residue was purified with silica gel column chromatography to give the desired product (450 mg) as an oily matter.

¹H-NMR (CDCl₃) δ 1.65-1.79 (2H, m), 2.48 (2H, t), 3.79-3.90 (2H, m), 7.00-7.27 (3H, m), 7.75 (1H, dd), 7.97 (1H, t), 8.68 (1H, d), 8.79 (1H, d)

### Reference Example 107

### Ethyl 4-methyl-5-nitrothiophene-3-carboxylate

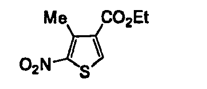

To a solution of fuming nitric acid (6 ml) and anhydrous acetic acid (20 ml), which was cooled to -10°C, a solution of ethyl 4-methylthiophene-3-carboxylate (3.20 g) in anhydrous acetic acid (10 ml) was added slowly. After stirring for 30 minutes at -10°C, the reaction solution was added to ice water. The mixture was extracted with a mixed solvent of hexane and ethyl acetate, and the organic layer was washed with water, an aqueous sodium hydrogen carbonate solution and saturated brine, dried over anhydrous magnesium sulfate, and concentrated. The resulting residue was dissolved in hexane, the insolubles were separated by filtration, and the filtrate was concentrated under reduced pressure to give the desired product (3.38 g) as crystals.

¹H-NMR (CDCl₃) δ 1.39 (3H, t), 2.90 (3H, s), 4.36 (2H, q), 8.21 (1H, s)

### Reference Example 108

### 3-[5-(Hydroxymethyl)-2,4-dioxo-1,4-dihydroquinazolin-3(2H)-yl]benzoic acid

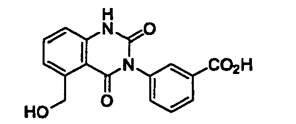

To a solution of ethyl 3-[5-(hydroxymethyl)-2,4-dioxo-1,4-dihydroquinazolin-3(2H)-yl]benzoate (150 mg) in methanol (10 ml) and THF (10 ml), a 1 N aqueous sodium hydroxide solution (1.32 ml) was added, and the mixture was stirred for 2 hours at room temperature. The reaction mixture was acidified with 0.1 N hydrochloric acid at 0°C, and then extracted with ethyl acetate. The organic layer was washed with saturated brine and dried over magnesium sulfate. The solvent was distilled off under reduced pressure, and the resulting residue was crystallized from hexane/diisopropyl ether to give the desired product (108 mg) as crystals.

¹H-NMR (DMSO-d₆) δ 4.94 (2H, d), 5.23 (1H, t), 7. 13 (1H, d), 7.45-7.70 (4H, m), 7.84-7.88 (1H, m), 7.96-8.01 (1H, m), 11.53 (1H, s), 13.13 (1H, br s)

### Reference Examples 108(1) to 108(4)

In the same manner as in Reference Example 108, the following compounds were obtained from the corresponding esters (Reference Examples 90, 104, 89 and 44).

### Reference Example 108(1)

### 5-Chloro-4-(5-formyl-2,4-dioxo-1,4-dihydroquinazolin-3(2H)-yl)thiophene-2-carboxylic acid

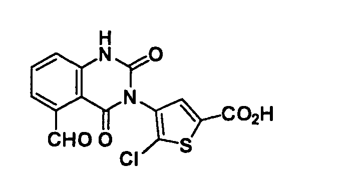

¹H-NMR (DMSO-d₆) δ 7.45 (1H, dd), 7.50 (1H, dd), 7.77 (1H, s), 7.87 (1H, t), 10.78 (1H, s), 12.06 (1H, s), 13.71 (1H, br s)

### Reference Example 108(2)

### 5- [5- (Hydroxymethyl) -2,4-dioxo-1,4-dihydroquinazolin-3 (2H) -yl] nicotinic acid

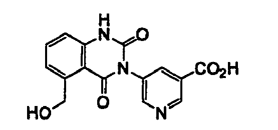

¹H-NMR (DMSO-d₆) δ 4.95 (2H, s), 7. 16 (1H, d), 7.50 (1H, d), 7.68 (1H, t), 8.17-8.22 (1H, m), 8.61 (1H, d), 9.04 (1H, d), 11.65 (1H, br s)

### Reference Example 108(3)

### 5-Chloro-4-[5-(hydroxymethyl)-2,4-dioxo-1,4-dihydroquinazolin-3(2H)-yl]thiophene-2-carboxylic acid

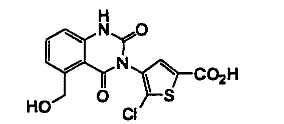

¹H-NMR (DMSO-d₆) δ 4.91-5.00 (2H, m), 5.23-5.37 (1H, m), 7.15 (1H, d), 7.52 (1H, d), 7.66-7.78 (2H, m), 11.69 (1H, br s), 13.62 (1H, br s)

### Reference Example 108(4)

### 4-[5-({[tert-Butyl(dimethyl)silyl]oxy}methyl)-2,4-dioxo-1,4-dihydroquinazolin-3(2H)-yl]-5-chlorothiophene-2-carboxylic acid

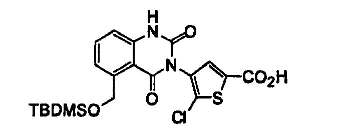

¹H-NMR (DMSO-d₆) δ 0.10 (6H, s), 0.94 (9H, s), 5.16 (2H, s), 7. 14 (1H, s), 7.17 (1H, d), 7.48 (1H, d), 7.72 (1H, t), 8.28 (2H, br s)

### Reference Example 109

### 3-(3,4-Dihydro-1(2H)-quinolinylcarbonyl)-1H-pyrazol-5-amine

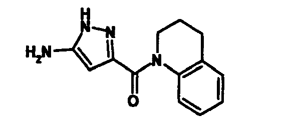

1-((5-Nitro-1H-pyrazol-3-yl)carbonyl)-1,2,3,4-tetrahydroquinoline (141 mg) was dissolved in ethanol/THF (3 : 1 solution, 14.6 ml), 10% palladium carbon (45 mg) was added thereto. While stirring for 4 days at room temperature at normal pressure, hydrogenation was carried out. The reaction solution was filtered through Celite, and the solvent was distilled off under reduced pressure. The residue was purified with silica gel column chromatography (0 to 7.5% methanol/ethyl acetate) to give the desired product (47 mg).

¹H-NMR (DMSO-d₆) δ 1.98-2.07 (2H, m), 2.77 (2H, t), 3.92 (2H, t), 5.11 (1H, br s), 7.04-7.21 (4H, m), 7.26 (1H, s)

### Reference Example 110

### N-[3-(3,4-Dihydroquinolin-1(2H)-ylsulfonyl)phenyl]-5-fluoro-2-nitrobenzamide

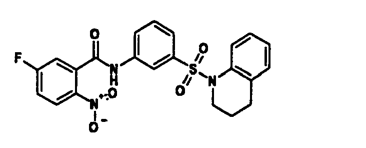

To a 0.24 N solution of 5-fluoro-2-nitrobenzoic acid in THF (2.5 ml), a 0.43 N solution of oxalyl chloride in THF (1.67 ml) was added. DMF (2 drops) was further added thereto, and the mixture was stirred at room temperature until the gas evolution ceased. After distilling off the volatile matter under reduced pressure, 2.5 ml of a solution obtained by diluting the residue with THF (7.5 ml) was gradually added dropwise to a solution of 3-(3,4-dihydro-1(2H)-quinolinylsulfonyl)aniline (0.20 N) and pyridine (0.25 N) in THF (2.5 ml), and the mixture was stirred for 15 hours at room temperature. Next, a trisamine resin (Argonaut Technologies, Inc., 4.36 mmol/g, 165 mg) was added thereto and the mixture was further stirred for 3 hours at room temperature. The resin was filtered off, and then washed with THF. The filtrate and a washing liquor were combined and concentrated under reduced pressure using a GeneVac's centrifugal concentrator. The residue was purified with silica gel column chromatography (20 to 60% ethyl acetate/hexane) to give the desired product (213 mg).
MS (ESI+, m/e) 456 (M+1)

### Reference Examples 110 (1) to 110(2)

In the same manner as in Reference Example 110, 3-(3,4-dihydro-1(2H)-quinolinylsulfonyl)aniline or 2-chloro-5-(3,4-dihydro-1(2H)-quinolinylsulfonyl)aniline was reacted with 3-methoxy-2-nitrobenzoic acid to obtain the following compounds.

### Reference Example 110(1)

### N-[3-(3,4-Dihydroquinolin-1(2H)-ylsulfonyl)phenyl]-3-methoxy-2-nitrobenzamide

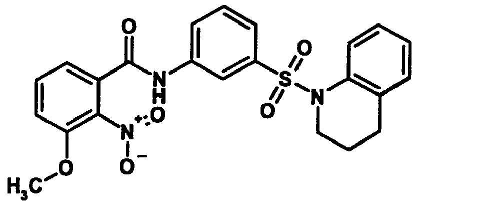

MS (ESI+, m/e) 468 (M+1)

### Reference Example 110(2)

### N-[2-Chloro-5-(3,4-dihydroquinolin-1(2H)-ylsulfonyl)phenyl]-3-methoxy-2-nitrobenzamide

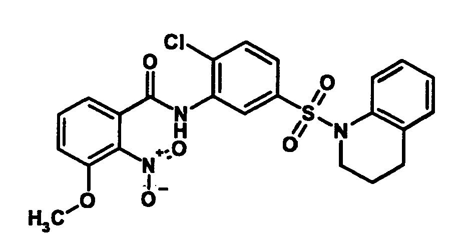

MS (ESI+, m/e) 502 (M+1)

### Reference Example 111

### 2-Amino-N- [3- (3, 4-dihydroquinolin-1 (2H) - ylsulfonyl)phenyl]-5-fluorobenzamide

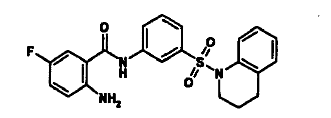

3-(3,4-Dihydro-1(2H)-quinolinylsulfonyl)aniline (120 mg), methyl 2-amino-5-fluorobenzoate (143 mg) and potassium t-butoxide (95 mg) were mixed. The reaction mixture was subjected to microwave irradiation for 10 minutes at 140°C in a closed vessel using a Smith Synthesizer, a microwave reactor, available from Personal Chemistry. The same reaction was newly carried out two more times. The three reaction mixtures obtained from the reactions were combined, partitioned into water and ethyl acetate and extracted with ethyl acetate. The organic layer was washed with saturated brine and dried over anhydrous magnesium sulfate. Then, the solvent was distilled off under reduced pressure. The residue was purified with silica gel column chromatography (20 to 55% ethyl acetate/hexane) to give the desired product (88 mg).

¹H-NMR (CDCl₃) δ 1. 70 (2H, ddd), 2.47 (2H, t), 3.82 (2H, t), 5.26 (2H, br s), 6.69 (1H, dd), 6.99-7.10 (3H, m), 7.15-7.23 (2H, m), 7.26-7.30 (1H, m), 7.38 (1H, t), 7.74-7.79 (2H, m), 7.97 (1H, dt), 8.12 (1H, br s)

### Reference Example 111(1)

In the same manner as in Reference Example 111, the following compound was obtained from reacting 3-(3,4-dihydro-1(2H)-guinolinylsulfonyl)aniline with methyl 3-aminopyrazine-2-carboxylate.

### 3-Amino-N-[3-(3,4-dihydroquinolin-1(2H)-ylsulfonyl)phenyl]pyrazine-2-carboxamide

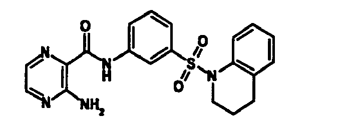

¹H-NMR (CDCl₃) δ 1.72 (2H, ddd), 2.49 (2H, t), 3.86 (2H, t), 7.02 (1H, d), 7.08 (1H, td), 7.21 (1H, td), 7.29 (1H, ddd), 7.37-7.43 (1H, m), 7.79 (1H, dd), 7.87 (1H, d), 7.96-7.99 (1H, m), 7.99 (1H, d), 8.24 (1H, d), 9.88 (1H, br s)

### Reference Example 112

### 3-(1,2,3,4-Tetrahydronaphthalen-1-ylthio)aniline

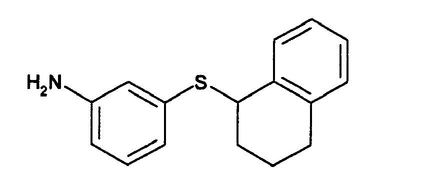

1-Hydroxy-1,2,3,4-tetrahydronaphthalene (1.48 g) and N,N-diisopropylethylamine (2.58 g) were dissolved in THF (20 ml), and a solution of methanesulfonyl chloride (1.26 g) in THF (1 ml) was added dropwise thereto with ice cooling. After stirring the mixture for 18 hours at room temperature, water was added thereto, and the resulting mixture was extracted with ethyl acetate, dried over magnesium sulfate, and then concentrated under reduced pressure to obtain 1,2,3,4-tetrahydronaphthalen-1-yl methanesulfonate (1.48 g) as an oily matter. Thus obtained oily matter was dissolved in DMF (10 ml), and 3-aminothiophenol (1.39 g) and potassium carbonate (1.38 g) were added thereto. The reaction mixture was stirred for 18 hours at 100°C. Water was added to the reaction solution, and the mixture was extracted with ethyl acetate, dried over magnesium sulfate, and then concentrated under reduced pressure. The residue was purified with silica gel column chromatography (hexane/ethyl acetate) to give the desired product (1.74 g) as an oily matter.
MS (ESI+, m/e) 256 (M+1)

### Reference Example 113

### 6-Methoxy-2,3,4,5-tetrahydro-1H-1-benzazepine

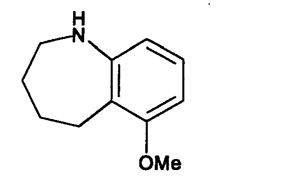

5-methoxy-3,4-dihydronaphthalen-1(2H)-one (1.76 g), hydroxyamine hydrochloride (0.83 g) and sodium acetate (0.86 g) were dissolved in ethanol/water (10 ml/1 ml), and the mixture was heated under reflux for 1 hour. The reaction solution was poured into water, and the mixture was extracted with ethyl acetate, dried over magnesium sulfate, and then concentrated under reduced pressure to obtain 5-methoxy-3,4-dihydronaphthalen-1(2H)-one oxime (1.91 g) as crystals.

Thus obtained crystals were dissolved in dichloromethane (20 ml), diisobutylaluminum hydride (1.5 N toluene solution, 30 ml) was added thereto with ice cooling, and the mixture was stirred for 1 hour at room temperature. Water was added to the reaction solution, and the mixture was extracted with ethyl acetate, dried over magnesium sulfate, and then concentrated under reduced pressure. The residue was purified with silica gel column chromatography (hexane/ethyl acetate) to give the desired product (0.95 g) as an oily matter.
MS (ESI+, m/e) 178 (M+1)

### Reference Example 113(1)

In the same manner as in Reference Example 113, the following compound was obtained from 7-methoxy-3,4-dihydronaphthalen-1(2H)-one.

### 8-Methoxy-2,3,4,5-tetrahydro-1H-1-benzazepine

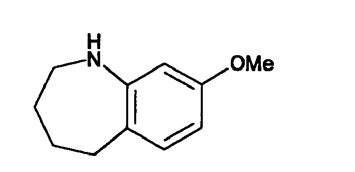

MS (ESI+, m/e) 178 (M+1)

### Reference Example 114

### 1-[(4-Chloro-3-nitrophenyl)sulfonyl]-6-methoxy-2,3,4,5-tetrahydro-1H-1-benzazepine

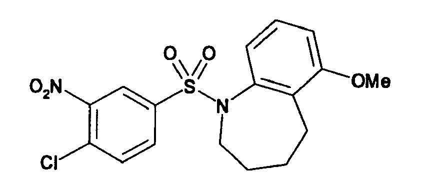

6-Methoxy-2,3,4,5-tetrahydro-1H-1-benzazepine (0.35 g) and N,N-diisopropylethylamine (0.65 g) were dissolved in THF (10 ml), and a solution of 4-chloro-3-nitrobenzensulfonyl chloride (0.51 g) in THF (2 ml) was added dropwise thereto with ice cooling. After stirring the mixture for 8 hours at room temperature, water was added thereto, and the mixture was extracted with ethyl acetate, dried over magnesium sulfate, and then concentrated under reduced pressure. The resulting residue was crystallized from diethyl ether to give the desired product (0.79 g) as a powder.
MS (ESI+, m/e) 397 (M+1)

### Reference Example 114(1)

In the same manner as in Reference Example 114, the following compound was obtained from reacting 8-methoxy-2,3,4,5-tetrahydro-1H-1-benzazepine with 4-chloro-3-nitrobenzensulfonyl chloride.

### 1-[(4-Chloro-3-nitrophenyl)sulfonyl]-8-methoxy-2,3,4,5-tetrahydro-1H-1-benzazepine

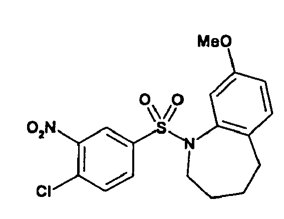

MS (ESI+, m/e) 397 (M+1)

### Reference Example 115

### 2-Chloro-5-[(6-methoxy-2,3,4,5-tetrahydro-1H-1-benzazepin-1-yl)sulfonyl]aniline

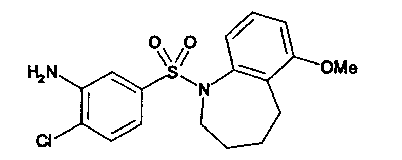

1-[(4-Chloro-3-nitrophenyl)sulfonyl]-6-methoxy-2,3,4,5-tetrahydro-1H-1-benzazepine (0.79 g) and reduced iron (0.8 g) were suspended in ethanol (10 ml), and concentrated hydrochloric acid (10 ml) was added dropwise thereto with ice cooling. After stirring for 1 hour with ice cooling, the reaction solution was poured into water, and the reaction mixture was neutralized with a 10 N aqueous sodium hydroxide solution, extracted with ethyl acetate, dried over magnesium sulfate, and then concentrated under reduced pressure. The residue was crystallized from diethyl ether to give the desired product (0.24 g) as a powder.
MS (ESI+, m/e) 367 (M+1)

### Reference Example 115(1)

In the same manner as in Reference Example 115, the following compound was obtained from 1-[(4-chloro-3-nitrophenyl)sulfonyl]-8-methoxy-2,3,4,5-tetrahydro-1H-1-benzazepine.

### 2-Chloro-5-[(8-methoxy-2,3,4,5-tetrahydro-1H-1-benzazepin-1-yl)sulfonyl]aniline

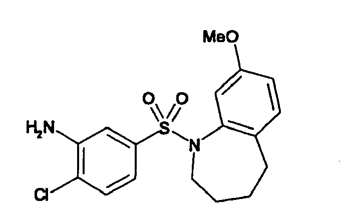

MS (ESI+, m/e) 367 (M+1)

### Reference Example 116

### 2-[(2-Hydroxyethyl)amino]benzamide

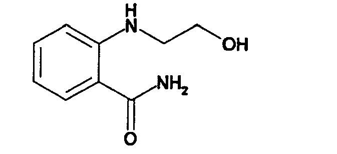

Isatoic anhydride (1.63 g), ethyl bromoacetate (1.83g), potassium carbonate (1.38 g) and potassium iodide (1.60 g) were suspended in DMF (10 ml), and the suspension was stirred for 1 hour at 80°C. The reaction solution was poured into water, and the mixture was extracted with ethyl acetate, dried over magnesium sulfate, and then was concentrated under reduced pressure. The residue was crystallized from diethyl ether to obtain ethyl (2,4-dioxo-2H-3,1-benzoxazin-1(4H)-yl)acetate (1.55 g) as crystals.

Thus obtained crystals were dissolved in DMF (5 ml), 25% ammonia water (5 ml) was added thereto, and the mixture was stirred for 0.5 hour at 40°C. The reaction solution was poured into water, and the mixture was extracted with ethyl acetate, dried over magnesium sulfate, and then concentrated under reduced pressure. The residue was crystallized from diethyl ether to obtain N-[2-(aminocarbonyl)phenyl]glycine ethyl ester (1.25 g) as crystals.

Thus obtained crystals were dissolved in THF (5 ml), lithium borohydride (0.44 g) was added thereto with ice cooling, and the mixture was stirred for 15 minutes at room temperature. The reaction solution was poured into water, and the mixture was extracted with ethyl acetate, dried over magnesium sulfate, and then concentrated under reduced pressure. The residue was crystallized from diethyl ether to give the desired product (0.80 g) as a powder.

¹H-NMR (CDCl₃) δ 2.01 (1H, br s), 3.35-3.45 (2H, m), 3.85 (2H, t), 5.70 (2H, br s), 6.61 (1H, t), 6.76 (1H, d), 7.28-7.41 (2H, m), 7.90 (1H, br s)

### Reference Example 117

### 1-Benzyl-1,2,3,4-tetrahydro-5H-1,4-benzodiazepin-5-one

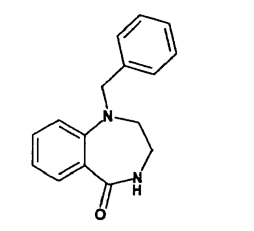

2-[(2-Hydroxyethyl)amino]benzamide (1.20 g), benzyl bromide (1.73g), potassium carbonate (1.38 g) and potassium iodide (1.60 g) were suspended in DMF (10 ml), and the suspension was stirred for 18 hours at 60°C. The reaction solution was poured into water, and the mixture was extracted with ethyl acetate, dried over magnesium sulfate, and then concentrated under reduced pressure. The residue was purified with silica gel column chromatography (hexane/ethyl acetate) to obtain 2-[benzyl(2-hydroxyethyl)amino]benzamide (1.80 g) as a powder.

Thus obtained powder was dissolved in thionyl chloride (10 ml), and the solution was stirred for 1 hour at room temperature. The reaction solution was concentrated under reduced pressure, water was added thereto, and the mixture was extracted with ethyl acetate, dried over magnesium sulfate, and then concentrated under reduced pressure. The residue was purified with silica gel column chromatography (hexane/ethyl acetate) to obtain 2-[benzyl(2-chloroethyl)amino]benzamide (1.00 g) as an oily matter.

Thus obtained oily matter was dissolved in THF (10 ml), sodium hydride (0.40 g) was added thereto, and the mixture was stirred for 1 hour at 70°C. Water was added to the reaction solution, and the mixture was extracted with ethyl acetate, dried over magnesium sulfate, and then concentrated under reduced pressure. The residue was purified with silica gel column chromatography (hexane/ethyl acetate) to give the desired product (0.72 g) as a powder.

¹H-NMR (CDCl₃) δ 3.24 (2H, t), 3.34-3.43 (2H, m), 4.38 (2H, s), 6.53 (1H, br s), 6.93-7.04 (2H, m), 7.25-7.40 (6H, m), 7.73 (1H, dd)

### Reference Example 118

### 1-[(4-Chloro-3-nitrophenyl)sulfonyl]-1,2,3,4-tetrahydro-5H-1,4-benzodiazepin-5-one

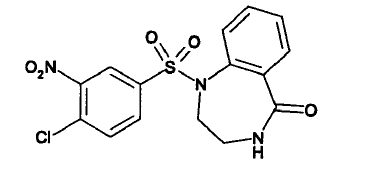

1-Benzyl-1,2,3,4-tetrahydro-5H-1,4-benzodiazepin-5-one (1.0 g) was dissolved in methanol (10 ml), 10% palladium carbon (50% water content, 1.0 g) was added thereto, and the mixture was stirred for 18 hours at room temperature under hydrogen atmosphere. Palladium carbon was filtered off, and then the filtrate was concentrated under reduced pressure to obtain 1,2,3,4-tetrahydro-5H-1,4-benzodiazepin-5-one (0.64 g) as an oily matter. Thus obtained oily matter was dissolved in THF (10 ml), N,N-diisopropylethylamine (1.29 g) was added thereto and a solution of 4-chloro-3-nitrobenzensulfonyl chloride (1.0 g) in THF (2 ml) was further added dropwise with ice cooling. After stirring the mixture for 8 hours at room temperature, water was added thereto, and the mixture was extracted with ethyl acetate, dried over magnesium sulfate, and then concentrated under reduced pressure. The residue was purified with silica gel column chromatography (hexane/ethyl acetate) to give the desired product (0.92 g) as a powder.
MS (ESI+, m/e) 382 (M+1)

### Reference Example 119

### 1-[(3-Amino-4-chlorophenyl)sulfonyl]-1,2,3,4-tetrahydro-5H-1,4-benzodiazepin-5-one

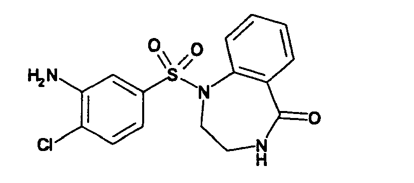

1-[(4-Chloro-3-nitrophenyl)sulfonyl]-1,2,3,4-tetrahydro-5H-1,4-benzodiazepin-5-one (0.24 g) and reduced iron (0.5 g) were suspended in ethanol (5 ml), and concentrated hydrochloric acid (5 ml) was added dropwise thereto with ice cooling. After stirring for 1 hour with ice cooling, the reaction solution was poured into water, and the reaction mixture was acidified with a 10 N aqueous sodium hydroxide solution, extracted with ethyl acetate, dried over magnesium sulfate, and then concentrated under reduced pressure. The residue was crystallized from diethyl ether to give the desired product (0.18 g) as a powder.
MS (ESI+, m/e) 352 (M+1)

### Reference Example 120

### Methyl 2-[({[3-(3,4-dihydroquinolin-1(2H)-ylsulfonyl)phenyl]amino}carbonyl)amino]-6-nitrobenzoate

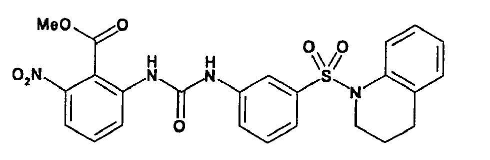

Methyl 2-amino-6-nitrobenzoate (2.75 g) and N,N-diisopropylethylamine (2.58 g) were dissolved in THF (30 ml), and a solution of triphosgene (1.48 g) in THF (5 ml) was added dropwise thereto with ice cooling. After stirring for 0.5 hour at room temperature, the insolubles were filtered off, and the filtrate was concentrated under reduced pressure. The resulting residue was dissolved in pyridine (50 ml), 3-(3,4-dihydro-1(2H)-quinolinylsulfonyl)aniline (4.04 g) was added thereto, and the mixture was stirred for 18 hours at 60°C. The reaction solution was concentrated under reduced pressure, and water was added to the residue. The mixture was extracted with ethyl acetate, dried over magnesium sulfate, and then concentrated under reduced pressure. The residue was purified with silica gel column chromatography (hexane/ethyl acetate), and recrystallized from ethyl acetate to give the desired product (2.94 g) as a powder.

¹H-NMR (CDCl₃) δ 1.60 (3H, s), 1.61 (2H, m), 2.45 (2H, t), 3.78 (2H, d), 7.06-7.17 (5H, m), 7.21 (1H, d), 7.43-7.49 (2H, m), 7.54 (1H, t), 7.62-7.73 (3H, m), 10.08 (1H, br s)

### Reference Example 121

### Methyl 2-cyano-6-nitrobenzoate

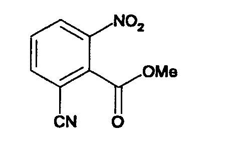

4-Nitro-2-benzofuran-1,3-dione (5.80 g) was heated under reflux for 1 hour in methanol (100 ml). After standing to cool, the reaction solution was concentrated under reduced pressure. Water was added to the residue, and the precipitated crystals were collected by filtration. The resulting crystals were dissolved in ethyl acetate, dried over magnesium sulfate, and then concentrated under reduced pressure to obtain 2-(methoxycarbonyl)-3-nitrobenzoic acid (6.08 g) as a solid. Thus obtained solid was dissolved in THF (10 ml), and oxalyl chloride (5 ml) was added thereto. The mixture was ice cooled, and several drops of DMF were further added thereto. The reaction mixture was stirred for 0.5 hour at room temperature. The reaction solution was concentrated under reduced pressure, and the residue was dissolved in THF (20 ml). 10 ml of an aqueous solution of ammonium carbonate (1.92 g) was added thereto at a time at -78°C. The reaction solution was again ice cooled and stirred for 15 minutes. Water was added thereto and the organic solvent was distilled off under reduced pressure. A powder precipitated from the residual aqueous suspension was collected by filtration, washed with water, and dried over diphosphorus pentoxide under reduced pressure to obtain methyl 2-(aminocarbonyl)-6-nitrobenzoate (4.60 g) as a powder.

The above-obtained methyl 2-(aminocarbonyl)-6-nitrobenzoate (2.02 g) was dissolved in a mixed solution of THF/DMF (10 ml/1 ml), oxalyl chloride (2.282 g) was added dropwise thereto with ice cooling, and the mixture was stirred for 1 hour at room temperature. Water was added to the reaction solution, and the mixture was extracted with ethyl acetate, dried over magnesium sulfate, and then concentrated under reduced pressure. The residue was purified with silica gel column chromatography (hexane/ethyl acetate) to give the desired product (1.86 g) as crystals.

¹H-NMR (CDCl₃) δ 4.07 (3H, s), 7.78 (1H, t), 8.02 (1H, d), 8.35 (1H, d)

### Reference Example 122

### Methyl 2-amino-6-(1H-tetrazol-5-yl)benzoate

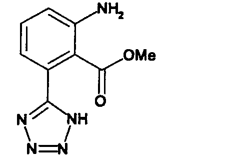

Methyl 2-cyano-6-nitrobenzoate (1.55 g) and ammonium chloride (0.80 g) were dissolved in DMF (10 ml), sodium azide (0.98 g) was added thereto, and the mixture was stirred for 0.5 hour at 60°C. Water was added to the reaction solution, and the mixture was extracted with ethyl acetate, dried over magnesium sulfate, and then concentrated under reduced pressure to obtain methyl 2-nitro-6-(1H-tetrazol-5-yl)benzoate (1.78 g) as an oily matter. Thus obtained oily matter was suspended in methanol (10 ml), 10% palladium carbon (50% water content, 1.0 g) was added thereto, and the mixture was stirred for 1 hour at room temperature under hydrogen atmosphere. Palladium carbon was filtered off, and then the filtrate was concentrated under reduced pressure to give the desired product (1.19 g) as an oily matter.

¹H-NMR (CDCl₃-DMSO-d₆) δ 3.55 (3H, s), 5.73 (2H, br s), 6.84 (1H, d), 7.31 (1H, t), 7.38 (1H, d)

### Example 1

### 4-Chloro-N-ethyl-3-(4-imino-2-oxo-1,4-dihydroquinazolin-3(2H)-yl)-N-phenylbenzenesulfonamide

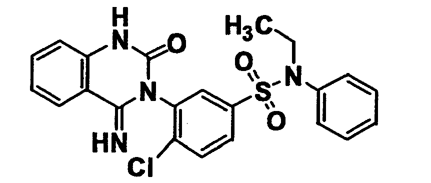

A solution of 4-chloro-3-((((2-cyanophenyl)amino)carbonyl)amino)-N-ethyl-N-phenylbenzenesulfonamide (613 mg) and DMAP (0.16 g) in THF (30 ml) was stirred for 3 days at room temperature, and then the solvent of the reaction solution was distilled off under reduced pressure. The residue was purified with silica gel column chromatography (hexane/ethyl acetate (3 : 1 to 2 : 3)), and crystallized from ethyl acetate/diethyl ether/hexane to give the desired product (276 mg) as crystals.

¹H-NMR (CDCl₃) δ 1.08 (3H, t), 3.62 (2H, dq), 6.93 (1H, d), 7.08-7. 11 (2H, m), 7.21 (1H, dt), 7.24-7.37 (4H, m), 7. 50 (1H, dt), 7.62 (1H, d), 7.65-7.72 (2H, m), 8.00 (1H, br s), 8.75 (1H, br s)

### Example 1 (1)

In the same manner as in Example 1, the following compound was obtained from 3-((((2-cyanophenyl)amino)carbonyl)amino)-N-ethyl-4-methyl-N-phenylbenzenesulfonamide.

### N-Ethyl-3-(4-imino-2-oxo-1,4-dihydroquinazolin-3(2H)-yl)-4-methyl-N-phenylbenzenesulfonamide

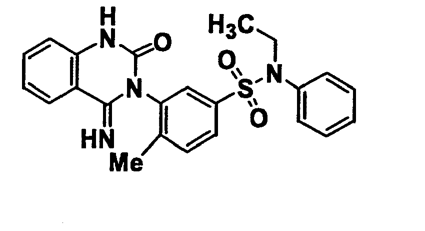

¹H-NMR (CDCl₃-DMSO-d₆) δ 1.07 (3H, t), 2.27 (3H, s), 3.60 (2H, q), 7.07-7.11 (4H, m), 7.16 (1H, dt), 7.27-7.35 (3H, m), 7.44-7.52 (3H, m), 7.62 (1H, dd), 8.10 (1H, br s), 10.14 (1H, s)

### Example 2

### 5-Chloro-4-(2,4-dioxo-1,4-dihydroquinazolin-3(2H)-yl)-N-ethyl-N-phenylthiophene-2-sulfonamide

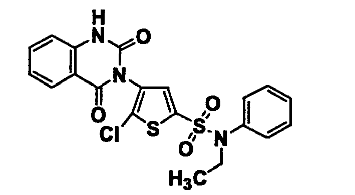

To a solution of 4-amino-5-chloro-N-ethyl-N-phenylthiophene-2-sulfonamide (214 mg) and DMAP (0.17 g) in THF (30 ml), methyl 2-isocyanatobenzoate (0.18 g) was added at room temperature, and the mixture was stirred for 3 days as it was. Subsequently, the solvent of the reaction solution was distilled off under reduced pressure. The residue was purified with silica gel column chromatography (hexane/ethyl acetate (3 : 1 to 1 : 2)), and crystallized from ethyl acetate/diisopropyl ether to give the desired product (189 mg) as crystals.

¹H-NMR (CDCl₃-DMSO-d₆) δ 1.12 (3H, t), 3.68 (2H, q), 7.18-7.25 (5H, m), 7.33-7.42 (3H, m), 7.62 (1H, dt), 8.11 (1H, dd), 11.20 (1H, br s)

### Example 2(1)

In the same manner as in Example 2, the following compound was obtained from reacting 3-amino-5-(2,3,4,5-tetrahydro-1H-1-benzazepin-1-ylsulfonyl)thiophene-2-carboxamide with methyl 2-isocyanatobenzoate.

### 3-(2,4-Dioxo-1,4-dihydroquinazolin-3(2H)-yl)-5-(2,3,4,5-tetrahydro-1H-1-benzazepin-1-ylsulfonyl)thiophene-2-carboxamide

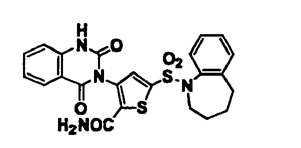

¹H-NMR (CDCl₃-DMSO-d₆) δ 1.65 (2H, brs), 1.89-1.96 (2H, m), 2.62 (2H, t), 3.71 (2H, br s), 6.71 (2H, br s), 7.15-7.24 (5H, m), 7.30-7.34 (1H, m), 7.35 (1H, s), 7.60 (1H, dt), 8.06 (1H, dd), 11.45 (1H, s)

### Example 3

### 3-Chloro-4-(2,4-dioxo-1,4-dihydroquinazolin-3(2H)-yl)-N-ethyl-N-phenylbenzenesulfonamide

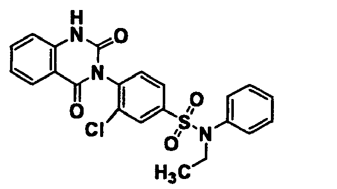

To a solution of 4-amino-3-chloro-N-ethyl-N-phenylbenzenesulfonamide (315 mg) and DMAP (0.25 g) in THF (30 ml), methyl 2-isocyanatobenzoate (0.27 g) was added at room temperature, and the mixture was stirred for 3 days as it was. The solvent of the reaction solution was distilled off under reduced pressure. The residue was stirred overnight at 80°C, purified with silica gel column chromatography (hexane/ethyl acetate (3 : 1 to 1 : 2)), and then crystallized from ethyl acetate/diethyl ether to give the desired product (78 mg) as crystals.

¹H-NMR (CDCl₃-DMSO-d₆) δ 1.12 (3H, t), 3.65 (2H, q), 7.10-7.14 (2H, m), 7.21-7.40 (5H, m), 7.45 (1H, s), 7.59-7.69 (2H, m), 7.81 (1H, d), 8.12 (1H, dd), 11.20 (1H, s)

### Example 4

### 3-(2,4-Dioxo-1,4-dihydroquinazolin-3(2H)-yl)-N-ethyl-4-methoxy-N-phenylbenzenesulfonamide

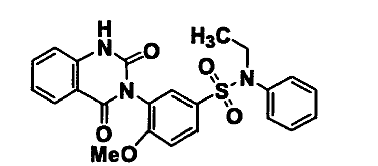

To a solution of 3-amino-N-ethyl-4-methoxy-N-phenylbenzenesulfonamide (226 mg) and DMAP (0.18 g) in THF (30 ml), methyl 2-isocyanatobenzoate (0.20 g) was added at room temperature, and the mixture was stirred for 3 days as it was. The solvent of the reaction solution was distilled off under reduced pressure. The residue was stirred for 0.5 hour at 80°C, purified with silica gel column chromatography (hexane/ethyl acetate (3 : 1 to 1 : 2)), and then crystallized from ethyl acetate/diethyl ether/diisopropyl ether to give the desired product (49 mg) as crystals.

¹H-NMR (CDCl₃) δ 1.06 (3H, t), 3.60 (2H, q), 3.85 (3H, s), 6.98 (1H, d), 7.06 (1H, d), 7.10-7.13 (2H, m), 7.19-7.35 (4H, m), 7.54-7.59 (2H, m), 7.67 (1H, dd), 8.11 (1H, dd), 9.89 (1H, s)

### Example 4(1)

In the same manner as in Example 4, the following compound was obtained from reacting 3-amino-N-ethyl-N-phenylbenzenesulfonamide with methyl 2-isocyanatobenzoate.

### 3-(2,4-Dioxo-1,4-dihydroquinazolin-3(2H)-yl)-N-ethyl-N-phenylbenzenesulfonamide

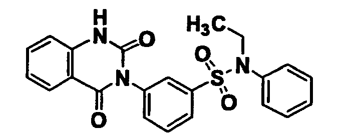

¹H-NMR (CDCl₃-DMSO-d₆) δ 1.07 (3H, t), 3.61 (2H, q), 7.09-7.12 (2H, m), 7.20-7.35 (5H, m), 7.52-7.65 (5H, m), 8.10 (1H, dd), 11.29 (1H, br s)

### Example 5

### 3-(2,4-Dioxo-1,4-dihydroquinazolin-3(2H)-yl)-N-ethyl-4-methyl-N-phenylbenzenesulfonamide

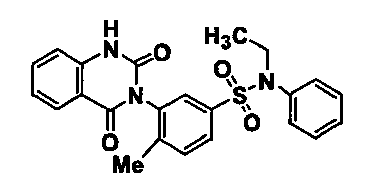

To a solution of 3-amino-N-ethyl-4-methyl-N-phenylbenzenesulfonamide (210 mg) and DMAP (0.18 g) in THF (30 ml), methyl 2-isocyanatobenzoate (0.19 g) was added at room 5 temperature, and the mixture was stirred for 3 days as it was and further stirred overnight at 65°C. Subsequently, the solvent of the reaction solution was distilled off under reduced pressure. The residue was purified with silica gel column chromatography (hexane/ethyl acetate (3 : 1 to 1 : 2)), and crystallized from diethyl ether/diisopropyl ether to give the desired product (107 mg) as crystals.

¹H-NMR (CDCl₃) δ 1.06 (3H, t), 2.26 (3H, s), 3.60 (2H, q), 7.01 (1H, d), 7.08-7.12 (2H, m), 7.23-7.35 (4H, m), 7.45-7.48 (2H, m), 7.59-7.66 (2H, m), 8.14 (1H, d), 9.29 (1H, s)

### Examples 5 (1) to 5(4)

In the same manner as in Example 5, the corresponding amines (Reference Examples 5(33), 18(1), 11(1) and 48) were reacted with methyl 2-isocyanatobenzoate to obtain the following compounds.

### Example 5(1)

### 4-Chloro-3-(2,4-dioxo-1,4-dihydroquinazolin-3(2H)-yl)-N-phenylbenzenesulfonamide

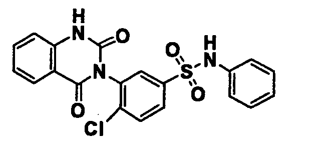

¹H-NMR (CDCl₃-DMSO-d₆) δ 7.05-7.10 (1H, m), 7.13-7.26 (6H, m), 7.55 (1H, dd), 7. 58-7. 64 (1H, m), 7.69 (1H, ddd), 7.83 (1H, d), 8.07 (1H, d), 9.37 (1H, br s), 11.30 (1H, br s)

### Example 5(2)

### Methyl 3-(3,4-dihydroquinolin-1(2H)-ylcarbonyl)-5-(2,4-dioxo-1,4-dihydroquinazolin-3(2H)-yl)benzoate

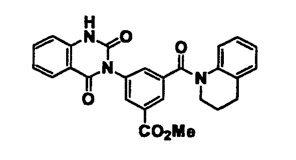

¹H-NMR (CDCl₃-DMSO-d₆) δ 1.99-2.11 (2H, m), 2.86 (2H, t), 3.87 (3H, s), 3.90 (2H, t), 6.93-7.03 (3H, m), 7.13-7.26 (3H, m), 7.52 (1H, t), 7.60 (1H, dt), 7.98-8.10 (3H, m), 11.31 (1H, s)

### Example 5(3)

### 3-[2-Chloro-5-(5,6,7,8-tetrahydro-4H-thieno[3,2-b]azepin-4-ylsulfonyl)-3-thienyl]quinazoline-2,4(1H,3H)-dione

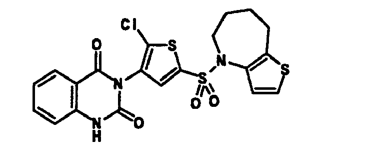

MS (ESI+, m/e) 494 (M+1)

### Example 5(4)

### 3-[4-(3,4-Dihydroquinolin-1(2H)-ylsulfonyl)-2-thienyl]quinazoline-2,4(1H,3H)-dione

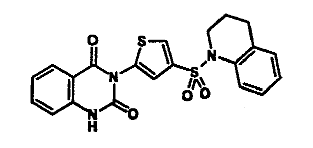

¹H-NMR (DMSO-d₆) δ 1.71 (2H, ddd), 2.58 (2H, t), 3.75 (2H, t), 7.08-7.12 (3H, m), 7.15-7.25 (3H, m), 7.58 (1H, d), 7.67-7.72 (1H, m), 7.92 (1H, dd), 8.13 (1H, d), 11.62 (1H, br s)

### Example 6

### 4-Chloro-3-(2,4-dioxo-1,4-dihydroquinazolin-3(2H)-yl)-N-methyl-N-phenylbenzenesulfonamide

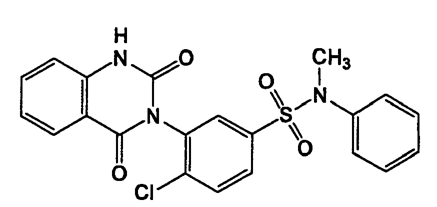

In a 48-well FlexChem reactor (Robbins Scientific Corp.), methyl 2-isocyanatobenzoate (0.90 M THF solution, 500 µl) was added to a mixed solution of 3-amino-4-chloro-N-methyl-N-phenylbenzenesulfonamide (0.60 M THF solution, 500 µl) and DMAP (0.56 M THF solution, 800 µl), and the mixture was shaken for 3 hours at room temperature. Then, a trisamine resin (Argonaut Technologies, Inc., 4.36 mmol/g, 100 mg) was added thereto, and the mixture was further shaken for 15 hours at room temperature. The resin was filtered off, and then the filtrate was concentrated under reduced pressure using a GeneVac's centrifugal concentrator. The residue was subjected to reversed-phase preparative HPLC (Gilson Inc., UniPoint System, YMC ODS column 30 × 75 mm), and the fraction eluted with 0.1% TFA-containing acetonitrile/water (10 : 90 to 100 : 0) was concentrated under reduced pressure to give the desired product (96 mg).

¹H-NMR (DMSO-d₆) δ 3.18 (3H, s), 7.09-7.13 (2H, m), 7.24-7.39 (5H, m), 7.57 (1H, dd), 7.71-7.77 (1H, m), 7.86 (1H, d), 7.96 (1H, d), 7.98 (1H, dd), 11.75 (1H; s)
HPLC (220 nm) purity 96 % (retention time: 3.53 minutes)
MS (ESI+, m/e) 442 (M+1)

### Examples 6(1) to 6(16)

In the same manner as in Example 6, the corresponding amines (Reference Examples 5 (1) to 5(7) and 5(20) to 5 (28)) were reacted with methyl 2-isocyanatobenzoate to obtain the following compounds.

### Example 6(1)

### 4-Chloro-3-(2,4-dioxo-1,4-dihydroquinazolin-3(2H)-yl)-N-isopropyl-N-phenylbenzenesulfonamide

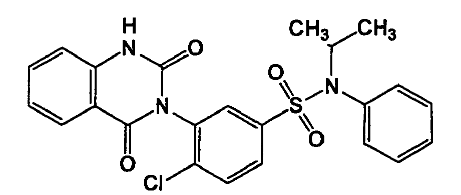

HPLC (220 nm) purity 100% (retention time: 3.74 minutes)
MS (ESI+, m/e) 470 (M+1)

### Example 6(2)

### N-1,3-Benzodioxol-5-yl-4-chloro-3-(2,4-dioxo-1,4-dihydroquinazolin-3(2H)-yl)-N-ethylbenzenesulfonamide

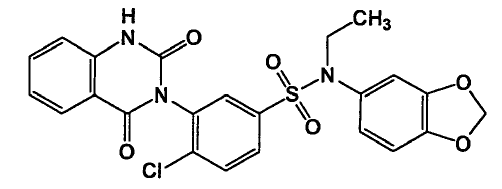

HPLC (220 nm) purity 100% (retention time: 3.63 minutes)
MS (ESI+, m/e) 500 (M+1)

### Example 6(3)

### 4-Chloro-N-(2-cyanoethyl)-3-(2,4-dioxo-1,4-dihydroquinazolin-3(2H)-yl)-N-phenylbenzenesulfonamide

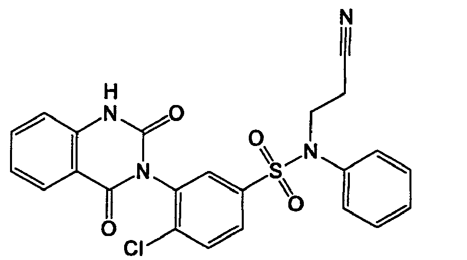

HPLC (220 nm) purity 99% (retention time: 3.39 minutes)
MS (ESI+, m/e) 481 (M+1)

### Example 6(4)

### 3-(2-Chloro-5-(3,4-dihydroquinolin-1(2H)-ylsulfonyl)phenyl)quinazoline-2,4(1H,3H)-dione

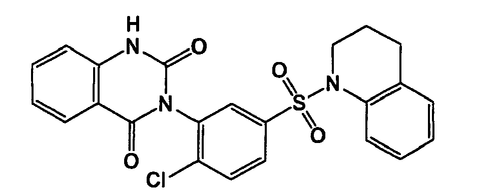

¹H-NMR (DMSO-d₆) δ 1.68 (2H, quintet), 2. 50 (2H, t), 3. 78 (2H, t), 7.09-7.11 (2H, m), 7.16-7.21 (1H, m), 7.24-7.29 (2H, m), 7.55 (1H, dd), 7.57 (1H, d), 7.71-7.77 (1H, m), 7. 80 (1H, d), 7.96 (1H, dd), 8.07 (1H, d), 11.74 (1H, s)
HPLC (220 nm) purity 100% (retention time: 3.72 minutes)
MS (ESI+, m/e) 468 (M+1)

### Example 6(5)

### 3-(2-Chloro-5-(3,4-dihydroisoquinolin-2(1H)-ylsulfonyl)phenyl)quinazoline-2,4(1H,3H)-dione

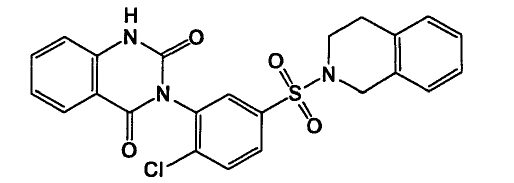

¹H-NMR (DMSO-d₆) δ 2.89 (2H, t), 3.31 (2H, t), 4.24 (2H, s), 7.10-7.30 (7H, m), 7.70-7.79 (1H, m), 7.91-7.98 (3H, m), 8.17 (1H, s), 11.76 (1H, s)
HPLC (220 nm) purity 100% (retention time: 3.68 minutes)
MS (ESI+, m/e) 468 (M+1)

### Example 6(6)

### 3-(2-Chloro-5-((4-ethylpiperazin-1-yl)sulfonyl)phenyl)quinazoline-2,4(1H,3H)-dione trifluoroacetate

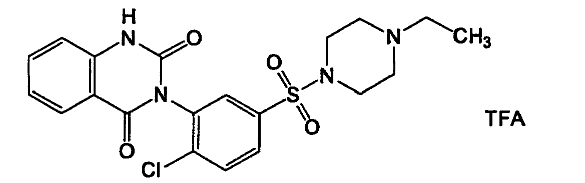

HPLC (220 nm) purity 100% (retention time: 2.41 minutes)
MS (ESI+, m/e) 449 (M+1)

### Example 6(7)

### 4-Chloro-3-(2,4-dioxo-1,4-dihydroquinazolin-3(2H)-yl)-N,N-diethylbenzenesulfonamide

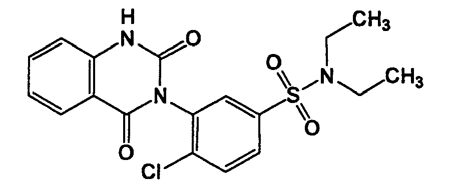

HPLC (220 nm) purity 100% (retention time: 3.31 minutes)
MS (ESI+, m/e) 408 (M+1)

### Example 6(8)

### 4-Chloro-3-(2,4-dioxo-1,4-dihydro-3(2H)-quinazolinyl)-N-ethyl-N-phenylbenzenesulfonamide

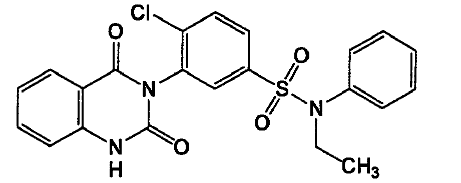

HPLC (220 nm) purity 83% (retention time: 2.19 minutes)
MS (ESI+, m/e) 456 (M+1)

### Example 6(9)

### N-Butyl-4-chloro-3-(2,4-dioxo-1,4-dihydro-3(2H)-quinazolinyl)-N-phenylbenzenesulfonamide

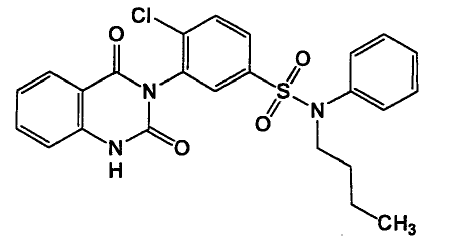

HPLC (220 nm) purity 95% (retention time: 2.37 minutes)
MS (ESI+, m/e) 484 (M+1)

### Example 6(10)

### 4-Chloro-N-(4-chlorophenyl)-3-(2,4-dioxo-1,4-dihydro-3(2H)-quinazolinyl)-N-ethylbenzenesulfonamide

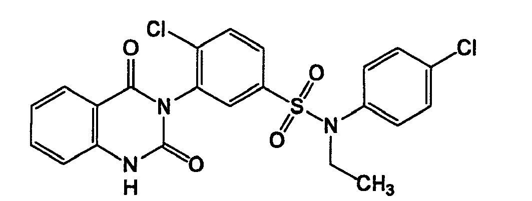

HPLC (220 nm) purity 99% (retention time: 2.18 minutes)
MS (ESI+, m/e) 490 (M+1)

### Example 6(11)

### 3-(2-Chloro-5-(2,3-dihydro-1H-indol-1-ylsulfonyl)phenyl)-2,4(1H,3H)-quinazolinedione

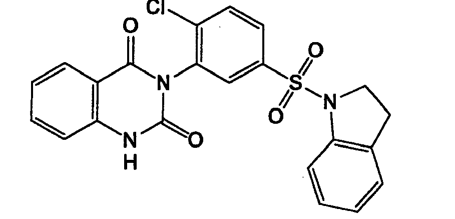

HPLC (220 nm) purity 95% (retention time: 2.16 minutes)
MS (ESI+, m/e) 454 (M+1)

### Example 6(12)

### 3-(5-((4-Benzyl-1-piperidinyl)sulfonyl)-2-chlorophenyl)-2,4(1H,3H)-quinazolinedione

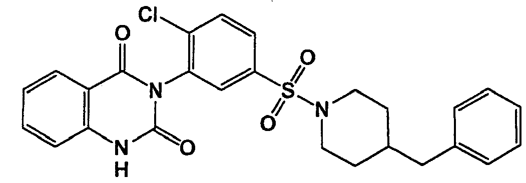

HPLC (220 nm) purity 99% (retention time: 2.39 minutes)
MS (ESI+, m/e) 510 (M+1)

### Example 6(13)

### 4-Chloro-N-(2-(dimethylamino)-1-phenylethyl)-3-(2,4-dioxo-1,4-dihydro-3(2H)-quinazolinyl)-N-methylbenzenesulfonamide trifluoroacetate

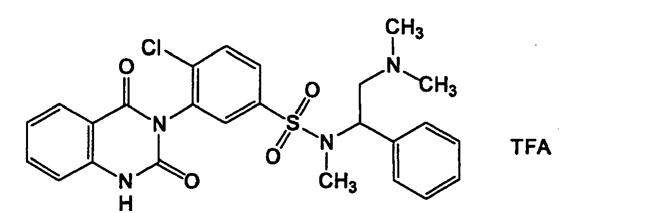

HPLC (220 nm) purity 81% (retention time: 1.60 minutes)
MS (ESI+, m/e) 513 (M+1)

### Example 6(14)

### 4-Chloro-N-cyclohexyl-3-(2,4-dioxo-1,4-dihydro-3(2H)-quinazolinyl)-N-methylbenzenesulfonamide

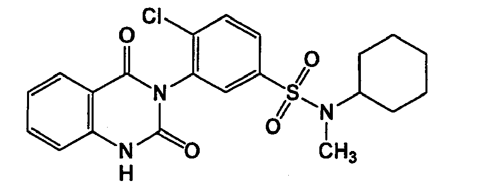

HPLC (220 nm) purity 97% (retention time: 2.12 minutes)
MS (ESI+, m/e) 448 (M+1)

### Example 6(15)

### N-Benzyl-4-chloro-3-(2,4-dioxo-1,4-dihydro-3(2H)-quinazolinyl)-N-ethylbenzenesulfonamide

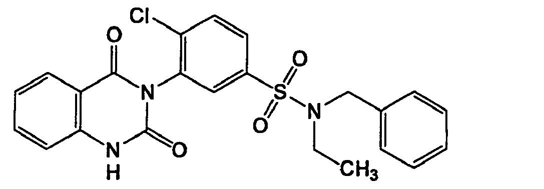

HPLC (220 nm) purity 95% (retention time: 1.99 minutes)
MS (ESI+, m/e) 470 (M+1)

### Example 6(16)

### 4-Chloro-3-(2,4-dioxo-1,4-dihydro-3(2H)-quinazolinyl)-N-methyl-N-2-pyridinylbenzenesulfonamide trifluoroacetate

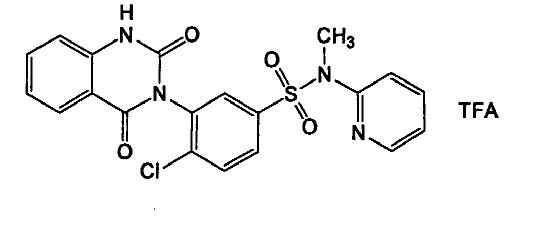

HPLC (220 nm) purity 84% (retention time: 1.95 minutes)
MS (ESI+, m/e) 443 (M+1)

### Example 7

### 3-(2-Chloro-5-((6-methyl-3,4-dihydro-1(2H)-quinolinyl)sulfonyl)phenyl)-2,4(1H,3H)-quinazolinedione

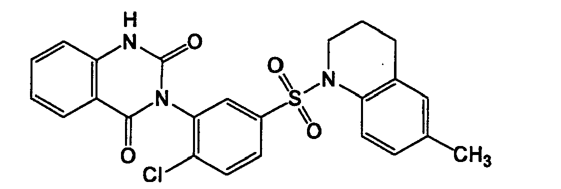

A solution of 2-chloro-5-((6-methyl-3,4-dihydro-1(2H)-quinolinyl)sulfonyl)aniline (505 mg), methyl 2-isocyanatobenzoate (399 mg) and DMAP (275 mg) in THF (9 ml) was shaken for 2 hours at room temperature, and then a trisamine resin (Argonaut Technologies, Inc., 4.36 mmol/g, 516 mg) was added thereto, and the mixture was further shaken for 15 hours at room temperature. The resin was filtered off, and then washed with acetonitrile. The filtrate and a washing liquor were combined and concentrated under reduced pressure. The residue was heated for 30 minutes at 80°C, and then subjected to silica gel column chromatography. The fraction eluted with ethyl acetate/hexane (1 : 2 to 2 : 1) was concentrated under reduced pressure. Thus obtained crystals were collected by filtration to give the desired product (512 mg).

¹H-NMR (DMSO-d₆) δ 1.64 (2H, quintet), 2.22 (3H, s), 2.44 (2H, t), 3.74 (2H, t), 6.91 (1H, s), 6.99 (1H, dd), 7.24-7.29 (2H, m), 7.47 (1H, d), 7. 51 (1H, dd), 7.71-7.77 (1H, m), 7. 79 (1H, d), 7.97 (1H, dd), 8.03 (1H, d), 11.74 (1H, s)
HPLC (220 nm) purity 95% (retention time: 3.87 minutes)
MS (ESI+, m/e) 482 (M+1)

### Examples 7(1) to 7(73)

In the same manner as in Example 7, the corresponding amines (Synthesized in Reference Examples) were reacted with methyl 2-isocyanatobenzoate to obtain the following compounds.

### Example 7(1)

### 3-(2-Chloro-5-((2-methyl-3,4-dihydro-1(2H)-quinolinyl) sulfonyl) phenyl) -2, 4 (1H, 3H)-quinazolinedione

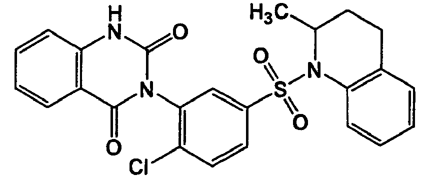

¹H-NMR (DMSO-d₆) δ 1.30 (3H, d), 1.33-1.39 (1H, m), 1.88-2.00 (2H, m), 2.41-2.47 (1H, m), 4.30-4.37 (1H, m), 6.98-7.77 (10H, m), 8.12 (1H, d), 9.89 (0.5H, s), 10.00 (0.5H, s)
HPLC (220 nm) purity 99% (retention time: 3.84 minutes)
MS (ESI+, m/e) 482 (M+1)

### Example 7(2)

### 3-(2-Chloro-5-((2-methyl-2,3-dihydro-1H-indol-1-yl)sulfonyl)phenyl)-2,4(1H,3H)-quinazolinedione

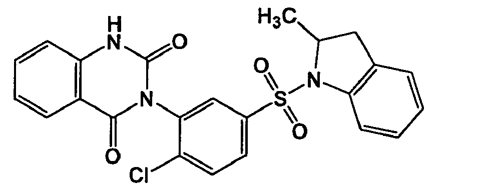

¹H-NMR (CDCl₃) δ 1.41 (3H, d), 2.45 (1H, d), 2.97-3.06 (1H, m), 4.35-4.39 (1H, m), 6.98-7.89 (10H, m), 8.10-8.14 (1H, m), 9.80 (0.5H, s), 10.05 (0.5H, s)
HPLC (220 nm) purity 95% (retention time: 3.73 minutes)
MS (ESI+, m/e) 468 (M+1)

### Example 7(3)

### 3-(2-Chloro-5-((5-methyl-2,3-dihydro-1H-indol-1-yl)sulfonyl)phenyl)-2,4(1H,3H)-quinazolinedione

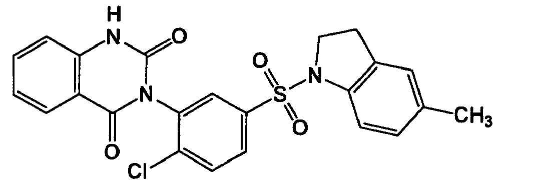

1 H-NMR (DMSO-ds) δ 2.22 (3H, s), 2.86 (2H, t), 3.96 (2H, t), 6.97-7.00 (2H, m), 7.23-7.36 (3H, m), 7.71-7.83 (3H, m), 7.96 (1H, dd), 8.22 (1H, d), 11.74 (1H, s)
HPLC (220 nm) purity 96% (retention time: 3.77 minutes)
MS (ESI+, m/e) 468 (M+1)

### Example 7(4)

### 3-(2-Chloro-5-(2,3-dihydro-4H-1,4-benzoxazin-4-ylsulfonyl)phenyl)-2,4(1H,3H)-quinazolinedione

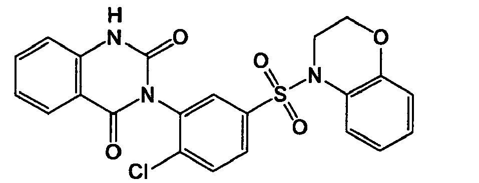

¹H-NMR (DMSO-d₆) δ 3.85-3.91 (4H, m), 6.85 (1H, d), 6.93 (1H, t), 7.10 (1H, t), 7.25-7.29 (2H, m), 7.61 (1H, dd), 7.69 (1H, d), 7.75 (1H, t), 7.84 (1H, d), 7.97 (1H, d), 8.23 (1H, d), 11.79 (1H, s)
HPLC (220 nm) purity 92% (retention time: 3.62 minutes)
MS (ESI+, m/e) 470 (M+1)

### Example 7(5)

### 3-(2-Chloro-5-(2,3,4,5-tetrahydro-1H-1-benzazepin-1-ylsulfonyl)phenyl)-2,4(1H,3H)-quinazolinedione

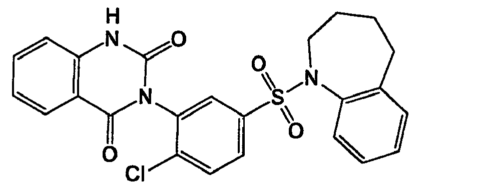

¹H-NMR (CDCl₃) δ 1.58-1.62 (2H, m), 1.82-1.84 (2H, m), 2.48-2.52 (2H, m), 3.71-3.75 (2H, m), 7.02 (1H, d), 7.12-7.32 (5H, m), 7.59-7.73 (3H, m), 7.82 (1H, d), 8.14 (1H, d), 9.93 (1H, s)
HPLC (220 nm) purity 98% (retention time: 3.77 minutes)
MS (ESI+, m/e) 482 (M+1)

### Example 7(6)

### 4-Chloro-3-(2,4-dioxo-1,4-dihydro-3(2H)-quinazolinyl)-N-methyl-N-4-pyridinylbenzenesulfonamide

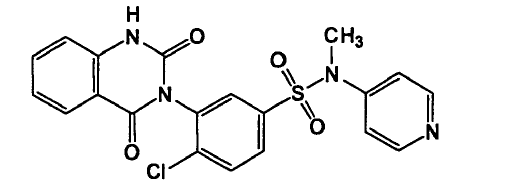

¹H-NMR (CDCl₃) δ 3.22 (3H, s), 7.04 (1H, d), 7.20 (2H, d), 7.27 (1H, t), 7.50 (1H, d), 7.60-7.71 (3H, m), 8.13 (1H, dd), 8.59 (2H, d), 10.04 (1H, s)
HPLC (220 nm) purity 99% (retention time: 2.49 minutes)
MS (ESI+, m/e) 443 (M+1)

### Example 7(7)

### 3-(2-Chloro-5-(3,4-dihydro-1,5-naphthylidin-1(2H)-ylsulfonyl)phenyl)-2,4(1H,3H)-quinazolinedione

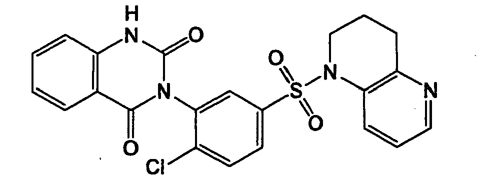

¹H-NMR (CDCl₃) δ 1. 72-1. 81 (2H, m), 2. 80 (2H, t), 3 . 76-3.85 (2H, m), 7.01 (1H, d), 7.16 (1H, dd), 7.23-7.31 (1H, m), 7. 51-7. 70 (4H, m), 8.11 (1H, d), 8.15 (1H, d), 8.34 (1H, dd), 9.92 (1H, s)
HPLC (220 nm) purity 100% (retention time: 2.57 minutes)
MS (ESI+, m/e) 469 (M+1)

### Example 7(8)

### 3-(2-Chloro-5-(3,4-dihydro-1,5-benzoxazepin-5(2H)-ylsulfonyl)phenyl)-2,4(1H,3H)-quinazolinedione

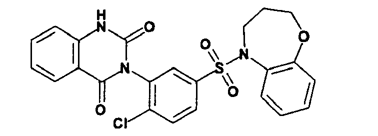

¹H-NMR (DMSO-d₆) δ 1.89-1.93 (2H, m), 3.74-3.78 (2H, m), 3.90 (2H, t), 7.02-7.12 (2H, m), 7.23-7.31 (4H, m), 7.59 (1H, dd), 7.71-7.78 (1H, m), 7.83 (1H, d), 7.98 (1H, dd), 8.16 (1H, d), 11.74 (1H, s)
HPLC (220 nm) purity 99% (retention time: 4.15 minutes)
MS (ESI+, m/e) 484 (M+1)

### Example 7 (9)

### 3-(2-Chloro-5-(3,4,5,6-tetrahydro-1-benzazocin-1(2H)-ylsulfonyl)phenyl)-2,4(1H,3H)-quinazolinedione

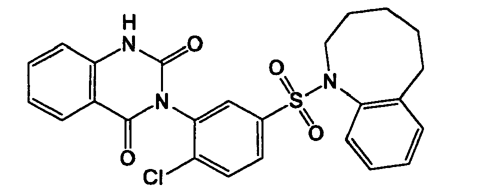

¹H-NMR (CDCl₃) δ 1.21-3.98 (10H, m), 6.71 (1H, d), 7.03 (1H, d), 7.12-7.29 (4H, m), 7.56-7.64 (1H, m), 7.73 (1H, d), 7.85-7.88 (2H, m), 8.14 (1H, d), 9.73 (1H, s)
HPLC (220 nm) purity 96% (retention time: 4.60 minutes)
MS (ESI+, m/e) 496 (M+1)

### Example 7(10)

### 3-(4-Chloro-3-(3,4-dihydro-1(2H)-quinolinylsulfonyl)phenyl)-2,4(1H,3H)-quinazolinedione

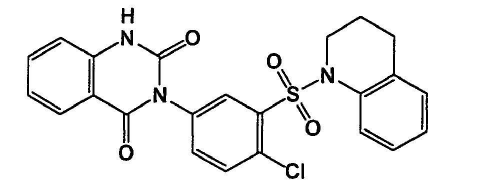

¹H-NMR (DMSO-d₆) δ 1.88 (2H, quintet), 2.75 (2H, t), 3.85 (2H, t), 7.01-7.16 (3H, m), 7.20-7.27 (3H, m), 7.66-7.74 (2H, m), 7. 81 (1H, d), 7.96 (1H, dd), 8.21 (1H, d), 11. 61 (1H, s)
HPLC (220 nm) purity 96% (retention time: 4.30 minutes)
MS (ESI+, m/e) 468 (M+1)

### Example 7(11)

### 3-(3-(3,4-Dihydro-1(2H)-quinolinylsulfonyl)-4-methylphenyl)-2,4(1H,3H)-quinazolinedione

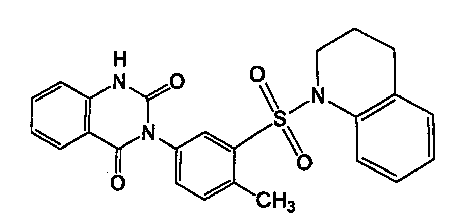

¹H-NMR (DMSO) δ 1.65-1.78 (2H, m), 2.30 (3H, s), 2.61 (2H, t), 3.72 (2H, t), 7.01-7.33 (6H, m), 7.45-7.57 (2H, m), 7.63-7.74 (1H, m), 7.78-7.97 (2H, m)
HPLC (220 nm) purity 98% (retention time: 2.05 minutes)
MS (ESI+, m/e) 448 (M+1)

### Example 7(12)

### 3-(3-(3,4-Dihydro-1(2H)-quinolinylcarbonyl)-2-methylphenyl)-2,4(1H,3H)-quinazolinedione

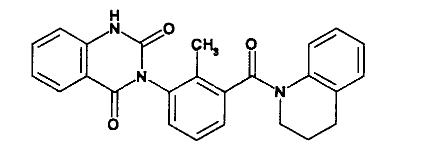

¹H-NMR (CDCl₃) δ 1.90-2.11 (2H, br s), 2.15 (3H, s), 2.85 (2H, t), 3.5-4.0 (2H, br s), 6.88-7.42 (9H, m), 7.63 (1H, dt), 8.13 (1H, d)
HPLC (220 nm) purity 100% (retention time: 1.89 minutes)
MS (ESI+, m/e) 412 (M+1)

### Example 7(13)

### 3-(2-Chloro-3-(3,4-dihydro-1(2H)-quinolinylcarbonyl)phenyl)-2,4(1H,3H)-quinazolinedione

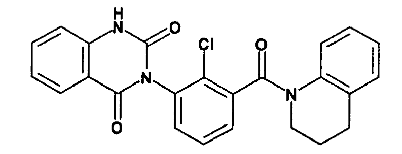

¹H-NMR (CDCl₃) δ 1.85-2.20 (2H, br s), 2.71-2.96 (2H, br s), 3.40-4.24 (2H, br s), 6.50-7.72 (10H, m), 8.14 (1H, d)
HPLC (220 nm) purity 98% (retention time: 1.92 minutes)
MS (ESI+, m/e) 432 (M+1)

### Example 7(14)

### 3-(5-(3,4-Dihydro-1(2H)-quinolinylcarbonyl)-2-fluorophenyl)-2,4(1H,3H)-quinazolinedione

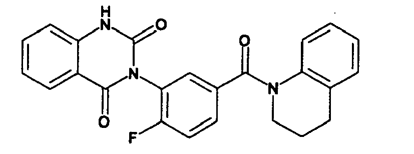

¹H-NMR (CDCl₃) δ 1.92-2.20 (2H, m), 2.84 (2H, t), 3.75-4.10 (2H, m), 6.80-7.29 (6H, m), 7.35-7.44 (1H, m), 7.61 (2H, dd), 8.10 (1H, dd)
HPLC (220 nm) purity 95% (retention time: 2.03 minutes)
MS (ESI+, m/e) 416 (M+1)

### Example 7(15)

### 3-(3-(3,4-Dihydro-1(2H)-quinolinylcarbonyl)-4-fluorophenyl)-2,4(1H,3H)-quinazolinedione

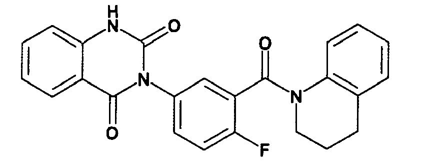

¹ H-NMR (CDCl₃) δ 1.95-2.21 (2H, br s), 2.85 (2H, t), 3.66-4.10 (2H, br s), 6.85-7.34 (8H, m), 7.55-7.20 (2H, m), 8.12 (1H, d)
HPLC (220 nm) purity 97% (retention time: 2.02 minutes)
MS (ESI+, m/e) 416 (M+1)

### Example 7(16)

### 3-(2,6-Dichloro-4-(3,4-dihydro-1(2H)-quinolinylcarbonyl)phenyl)-2,4(1H,3H)-quinazolinedione

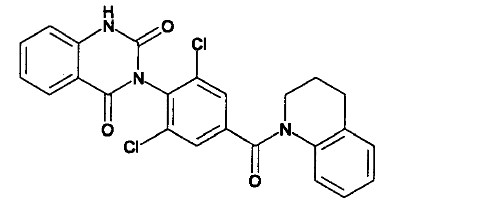

¹H-NMR (CDCl₃) δ 2.08 (2H, quintet), 2.88 (2H, t), 3. 93 (2H, t), 6.85-7.34 (6H, m), 7.48 (2H, s), 7.67 (1H, dt), 8.16 (1H, d) HPLC (220 nm) purity 92% (retention time: 2.18 minutes)
MS (ESI+, m/e) 466 (M+1)

### Example 7(17)

### 3-[3-(2,3-Dihydro-1H-indol-1-ylsulfonyl)phenyl]quinazoline-2,4(1H,3H)-dione

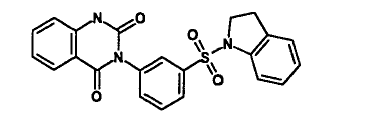

¹H-NMR (DMSO-d₆) δ 2.93 (2H, t), 3.96 (2H, t), 6.99 (1H, dt), 7.15-7.27 (4H, m), 7.47 (1H, d), 7.58-7.79 (4H, m), 7.92-7.97 (2H, m), 11.58 (1H, s)

### Example 7(18)

### 3-[3-(3,4-Dihydroquinolin-1(2H)-ylsulfonyl)phenyl]quinazoline-2,4(1H,3H)-dione

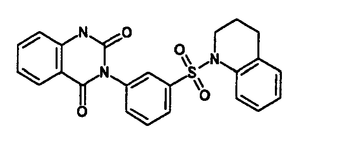

¹H-NMR (DMSO-d₆) δ 1.66 (2H, quintet), 2.48 (2H, t), 3. 76 (2H, t), 7.06-7.26 (5H, m), 7.50-7.77 (6H, m), 7.94 (1H, d), 11.58 (1H, s)

### Example 7(19)

### 3-[3-(2,3,4,5-Tetrahydro-1H-1-benzazepin-1-ylsulfonyl)phenyl]quinazoline-2,4(1H,3H)-dione

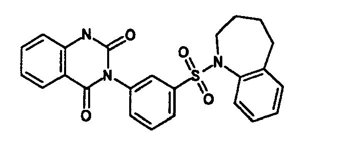

¹H-NMR (CDCl₃) δ 1.59-1.61 (2H, m), 1. 83-1. 85 (2H, m), 2.48 (2H, t), 3.71-3.73 (2H, m), 6.98 (1H, d), 7. 11-7.34 (5H, m), 7.50-7.53 (1H, m), 7.59 (1H, dt), 7.60 (1H, t), 7. 75-7. 80 (2H, m), 8.13 (1H, d), 9.73 (1H, s)

### Example 7(20)

### 3-[3-(3,4,5,6-Tetrahydro-1-benzazocin-1(2H)-ylsulfonyl)phenyl]quinazoline-2,4(1H,3H)-dione

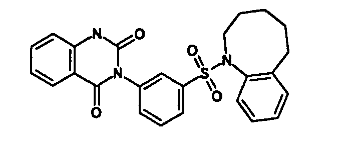

¹H-NMR (CDCl₃) δ 1.14-2.05 (6H, m), 2.60-4.08 (4H, m), 6.72 (1H, d), 6.97 (1H, d), 7.10-7.16 (1H, m), 7.22-7.25 (3H, m), 7.55-7.58 (2H, m), 7.69 (1H, t), 7.82 (1H, s), 7.92 (1H, d), 8.13 (1H, d), 9.76 (1H, s)

### Example 7 (21)

### 3-{3-[(2-Methyl-3,4-dihydroquinolin-1(2H)-yl)sulfonyl]phenyl}quinazoline-2,4(1H,3H)-dione

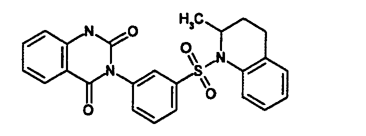

¹H-NMR (CDCl₃) δ 1.29 (3H, d), 1.26-1.37 (1H, m), 1.88-1.96 (2H, m), 2.39-2.45 (1H, m), 4.37 (1H, sextet), 6.95-7.01 (2H, m), 7.10 (1H, t), 7.20-7.28 (2H, m), 7.42-7.52 (3H, m), 7.58-7.64 (2H, m), 7.74 (1H, d), 8.11 (1H, d), 9.70 (1H, s)

### Example 7 (22)

### 3-[5-(3,4-Dihydroquinolin-1(2H)-ylsulfonyl)-2-fluorophenyl]quinazoline-2,4(1H,3H)-dione

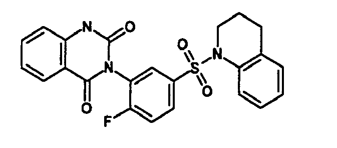

¹H-NMR (DMSO-d₆) δ 1.69 (2H, quintet), 2. 51 (2H, t), 3.77 (2H, t), 7.08-7.29 (5H, m), 7.50-7.60 (3H, m), 7.73 (1H, t), 7.96 (1H, d), 8.04 (1H, dd), 11.75 (1H, s)

### Example 7 (23)

### 3-[3-(5,6,7,8-Tetrahydro-4H-thieno[3,2-b]azepin-4-ylsulfonyl)phenyl]quinazoline-2,4(1H,3H)-dione

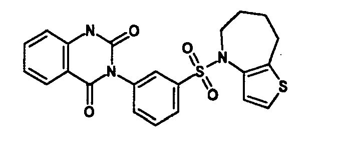

¹H-NMR (DMSO-d₆) δ 1.41-1.43 (2H, m), 1.72-1.74 (2H, m), 2.26-2.30 (2H, m), 3.62-3.64 (2H, m), 6.92 (1H, d), 7.15 (1H, d), 7.19-7.24 (2H, m), 7.51-7.55 (1H, m), 7.61-7.74 (4H, m), 7.94 (1H, d), 11.59 (1H, s)

### Example 7(24)

### 3-[3-(2,3-Dihydro-4,1-benzothiazepin-1(5H)-ylsulfonyl)phenyl]quinazoline-2,4(1H,3H)-dione

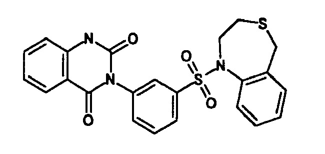

¹H-NMR (DMSO-d₆) δ 2.94-3.66 (6H, m), 7.09 (1H, dd), 7.17-7.28 (4H, m), 7.33 (1H, dd), 7.67-7.75 (3H, m), 7. 81 (1H, dt), 7.91 (1H, dd), 7.96 (1H, dd), 11.61 (1H, s)

### Example 7(25)

### 3-[3-(3,4-Dihydro-1,5-benzoxazepin-5(2H)-ylsulfonyl)phenyl]quinazoline-2,4(1H,3H)-dione

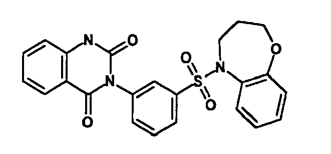

¹H-NMR (DMSO-d₆) δ 1.92 (2H, quintet), 3.73-3.75 (2H, m), 3.88-3.90 (2H, m), 7.00-7.08 (2H, m), 7.20-7.27 (4H, m), 7.58-7. 72 (4H, m), 7.90-7.92 (1H, m), 7.95 (1H, dd), 11.60 (1H, s)

### Example 7 (26)

### 3-[3-(3,4-Dihydro-1,5-benzothiazepin-5(2H)-ylsulfonyl)phenyl]quinazoline-2,4(1H,3H)-dione

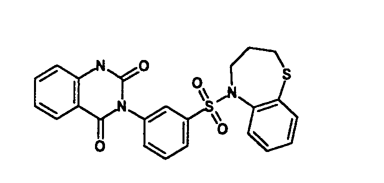

¹H-NMR (DMSO-d₆) δ 2.08-2.10 (2H, m), 2.73-2.75 (2H, m), 3.62-3.66 (2H, m), 7.17-7.28 (5H, m), 7.54-7.57 (1H, m), 7.66-7.74 (3H, m), 7. 81-7. 85 (1H, m), 7.90 (1H, t), 7.96 (1H, dd), 11.61 (1H, s)

### Example 7 (27)

### 3-{3-[(5-Methyl-3,4-dihydroquinolin-1(2H)-yl)sulfonyl]phenyl}quinazoline-2,4(1H,3H)-dione

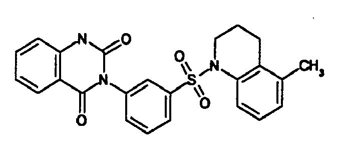

¹H-NMR (DMSO-d₆) δ 1.63 (2H, quintet), 2.13 (3H, s), 2.40 (2H, t), 3.72 (2H, t), 6.97 (1H, d), 7.06 (1H, t), 7.19-7.24 (2H, m), 7.41 (1H, d), 7.55-7.64 (4H, m), 7.66-7.72 (1H, m), 7.92 (1H, dd), 11.57 (1H, s)

### Example 7(28)

### 3-{3-[(4-Methyl-3,4-dihydroquinolin-1(2H)-yl)sulfonyl]phenyl}quinazoline-2,4(1H,3H)-dione

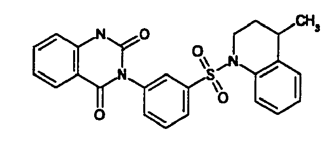

¹H-NMR (DMSO-d₆) δ 1.04 (3H, d), 1.32-1.34 (1H, m), 1.75-1.77 (1H, m), 2.61 (1H, sextet), 3.74-3.79 (2H, m), 7.08-7.24 (5H, m), 7.48-7.50 (1H, m), 7.57-7.65 (3H, m), 7.68 (1H, t), 7.76 (1H, s), 7.93 (1H, d), 11.58 (1H, s)

### Example 7 (29)

### 3-[3-(2,3-Dihydro-4,1-benzoxazepin-1(5H)-ylsulfonyl)phenyl]quinazoline-2,4(1H,3H)-dione

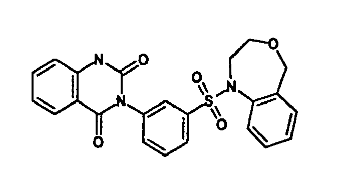

¹H-NMR (DMSO-d₆) δ 3.74-4.25 (6H, m), 7.20-7.34 (6H, m), 7.66-7.72 (4H, m), 7.89 (1H, s), 7.95 (1H, dd), 11.60 (1H, s)

### Example 7 (30)

### 3-{3-[(5-Hydroxy-2,3,4,5-tetrahydro-1H-1-benzazepin-1-yl)sulfonyl]phenyl}quinazoline-2,4(1H,3H)-dione

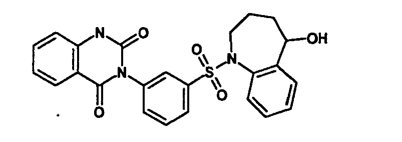

¹H-NMR (DMSO-d₆) δ 1.36-1.40 (1H, m), 1.68-1.70 (1H, m), 1.96-1.98 (2H, m), 2.88-2.95 (1H, m), 4.06-4.11 (1H, m), 4.63-4.68 (1H, m), 5.33 (1H, d), 7.00 (1H, dd), 7.16 (1H, dt), 7.20-7.25 (2H, m), 7.31 (1H, dt), 7.57 (1H, d), 7.67-7.73 (2H, m), 7.74 (1H, t), 7.86 (1H, dt), 7.92 (1H, t), 7.96 (1H, dd), 11.62 (1H, s)

### Example 7(31)

### 3-(2,4-Dioxo-1,4-dihydroquinazolin-3(2H)-yl)-N-(2-methylphenyl)benzenesulfonamide

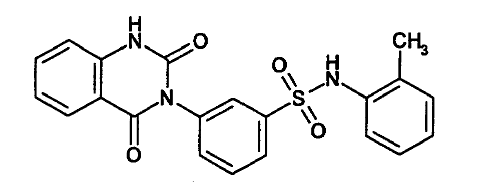

¹H-NMR (DMSO-d₆) δ. 2.07 (3H, s), 6.98-7.24 (6H, m), 7.60-7.69 (4H, m), 7.71 (1H, t), 7.93 (1H, dd), 9.64 (1H, s), 11.59 (1H, s)

### Example 7(32)

### N-(2-Butylphenyl)-3-(2,4-dioxo-1,4-dihydroquinazolin-3 (2H) -yl) benzenesulfonamide

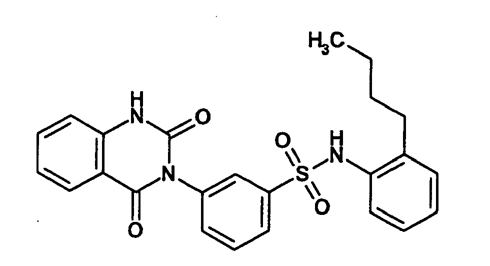

¹H-NMR (DMSO-d₆) δ 0.87 (3H, t), 1.24-1.31 (2H, m), 1.41-1.46 (2H, m), 2.55 (2H, t), 6.87 (1H, dd), 7.03 (1H, dt), 7.13 (1H, dt), 7.17-7.24 (3H, m), 7.60-7.74 (5H, m), 7.93 (1H, dd), 9.62 (1H, s), 11.59 (1H, s)

### Example 7(33)

### 3-(2,4-Dioxo-1,4-dihydroquinazolin-3(2H)-yl)-N-(2-methoxyphenyl)benzenesulfonamide

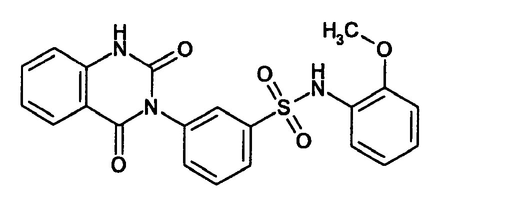

¹H-NMR (DMSO-d₆) δ 3.51 (3H, s), 6.83-6.91 (2H, m), 7.13 (1H, dt), 7.19-7.24 (3H, m), 7.56-7.71 (5H, m) , 7.93 (1H, d), 9.56 (1H, s), 11.59 (1H, s)

### Example 7(34)

### 3-(2,4-Dioxo-1,4-dihydroquinazolin-3(2H)-yl)-N-(2-fluorophenyl)benzenesulfonamide

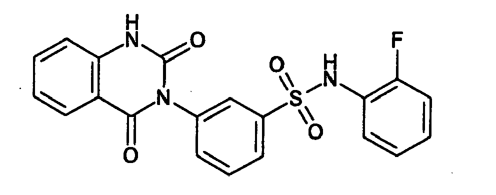

¹H-NMR (DMSO-d₆) δ 7.06-7.25 (6H, m), 7.60-7.71 (4H, m), 7.77 (1H, dd), 7.93 (1H, d), 10.24 (1H, s), 11.59 (1H, s)

### Example 7(35)

### 3-(2,4-Dioxo-1,4-dihydroquinazolin-3(2H)-yl)-N-5,6,7,8-tetrahydronaphthalen-1-ylbenzenesulfonamide

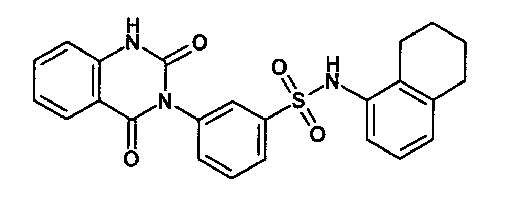

¹H-NMR (DMSO-d₆) δ 1.59-1.61 (4H, m), 2.48-2.51 (2H, m), 2.65-2.67 (2H, m), 6.80 (1H, d), 6.89-6.99 (2H, m), 7.19-7.24 (2H, m), 7.59-7.71 (5H, m), 7.93 (1H, dd), 9.48 (1H, s), 11.59 (1H, s)

### Example 7(36)

### 3-(2,4-Dioxo-1,4-dihydroquinazolin-3(2H)-yl)-N-1,2,3,4-tetrahydronaphthalen-1-ylbenzenesulfonamide

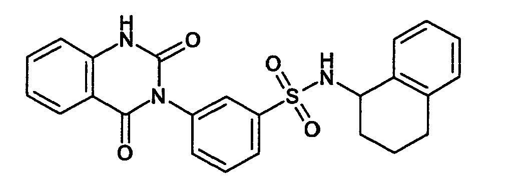

¹H-NMR (DMSO-d₆) δ 1.60-1.80 (4H, m), 2.63-2.67 (2H, m), 4.36-4.39 (1H, m), 7.02-7.04 (1H, m), 7.10-7.12 (3H, m), 7.20-7.25 (2H, m), 7.62-7.75 (3H, m), 7.89-7.97 (3H, m), 8.15 (1H, d), 11.61 (1H, s)

### Example 7(37)

### 3-(2,4-Dioxo-1,4-dihydroquinazolin-3(2H)-yl)-N-[2-(1-hydroxyethyl)phenyl]benzenesulfonamide

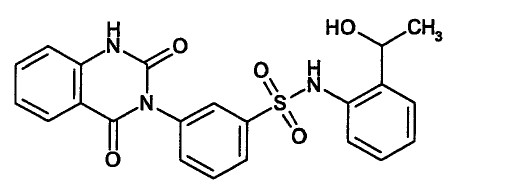

¹H-NMR (DMSO-d₆) δ 1.23 (3H, d), 5.03 (1H, q), 5.31 (1H, br s), 6.93 (1H, d), 7.10 (1H, t), 7.17 (1H, d), 7.20-7.26 (2H, m), 7.41 (1H, d), 7.64-7.74 (3H, m), 7.77-7.80 (2H, m), 7.95 (1H, d), 9.71 (1H, br s), 11.63 (1H, s)

### Example 7 (38)

### 2-({[3-(2,4-Dioxo-1,4-dihydroquinazolin-3(2H)-yl)phenyl]sulfonyl}amino)benzamide

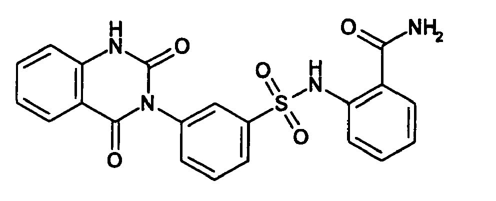

¹H-NMR (DMSO-d₆) δ 7.10 (1H, ddd), 7.20-7.24 (2H, m), 7.41-7.49 (2H, m), 7.61-7.72 (3H, m), 7.81-7.93 (5H, m), 8.36 (1H, s), 11.57 (1H, s), 12.40 (1H, s)

### Example 7(39)

### 3-{3-[(5-Methyl-2,3,4,5-tetrahydro-1H-1-benzazepin-1-yl)sulfonyl]phenyl}quinazoline-2,4(1H,3H)-dione

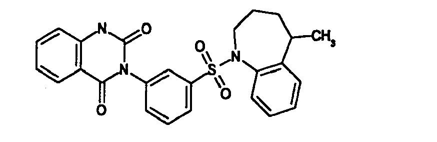

¹H-NMR (CDCl₃) δ 1.22 (3H, d), 1.25-1.27 (2H, m), 1.73-1.77 (1H, m), 1.94-1.98 (1H, m), 2.76-2.80 (1H, m), 3.12-3.16 (1H, m), 4.11-4.13 (1H, m), 6.95 (1H, d), 7.14-7.22 (5H, m), 7.51-7.65 (3H, m), 7.80-7.86 (2H, m), 8.11 (1H, d), 9.84 (1H, s)

### Example 7(40)

### N-(2-Cyanoethyl)-3-(2,4-dioxo-1,4-dihydroquinazolin-3(2H)-yl)-N-phenylbenzenesulfonamide

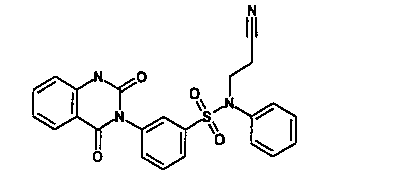

¹H-NMR (DMSO-d₆) δ 2.64 (2H, t), 3.86 (2H, t), 7.07-7.10 (2H, m), 7.22-7.27 (2H, m), 7.35-7.40 (3H, m), 7.59-7.63 (1H, m), 7.67-7.76 (4H, m), 7.97 (1H, d), 11.62 (1H, s)

### Example 7(41)

### 3-(2,4-Dioxo-1,4-dihydroquinazolin-3(2H)-yl)-N-(2-hydroxyethyl)-N-phenylbenzenesulfonamide

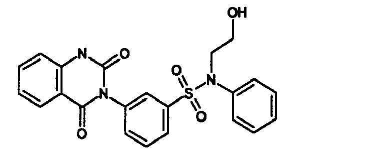

¹H-NMR (DMSO-d₆) δ 3.37 (2H, t), 3.61 (2H, t), 4.77 (1H, t), 7.06-7.09 (2H, m), 7.22-7.26 (2H, m), 7.31-7.36 (3H, m), 7.57-7.60 (1H, m), 7.63-7.73 (4H, m), 7.95 (1H, d), 11.59 (1H, s)

### Example 7(42)

### Ethyl N-{[3-(2,4-dioxo-1,4-dihydroquinazolin-3(2H)-yl)phenyl]sulfonyl}-N-phenylglycinate

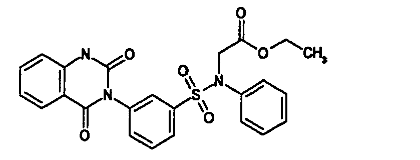

¹H-NMR (DMSO-d₆) δ 1.13 (3H, t), 4.07 (2H, q), 4.53 (2H, s), 7.17-7.34 (7H, m), 7.68-7.74 (4H, m), 7.85 (1H, s), 7.97 (1H, d), 11.63 (1H, s)

### Example 7(43)

### 3-{3-[(3-Oxo-3,4-dihydroquinoxalin-1(2H)-yl)sulfonyl]phenyl}quinazoline-2,4(1H,3H)-dione

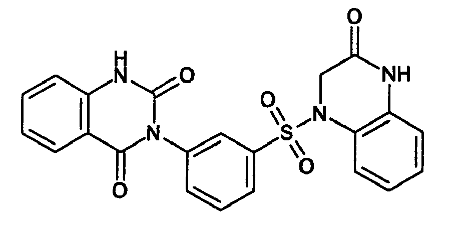

¹H-NMR (DMSO-d₆) δ 4.33 (2H, s), 6.89 (1H, dd), 7.03 (1H, t), 7.17-7.26 (3H, m), 7.46-7.49 (2H, m), 7.59-7.73 (4H, m), 7.92 (1H, d), 10.42 (1H, s), 11.59 (1H, s)

### Example 7(44)

### 3-{3-[(4-Oxo-2,3,4,5-tetrahydro-1H-1,5-benzodiazepin-1-yl)sulfonyl]phenyl}quinazoline-2,4(1H,3H)-dione

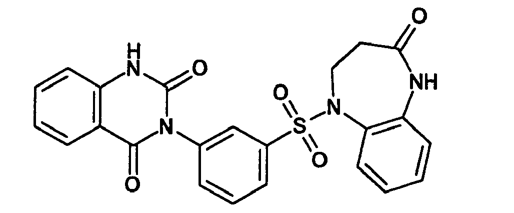

¹H-NMR (DMSO-d₆) δ 2.38 (2H, t), 4.09 (2H, t), 7.06 (1H, d), 7.18 (1H, t), 7.23-7.28 (3H, m), 7.36 (1H, dt), 7.67-7.77 (4H, m), 7.87-7.90 (1H, m), 7.97 (1H, d), 9.21 (1H, s), 11.67 (1H, s)

### Example 7(45)

### 3-{3-[(2-Methyl-3-oxo-3,4-dihydroquinoxalin-1(2H)-yl)sulfonyl]phenyl}quinazoline-2,4(1H,3H)-dione

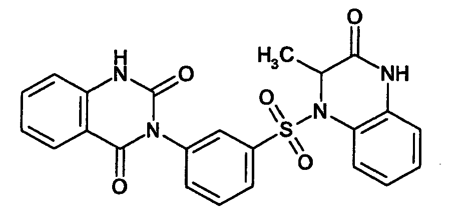

¹H-NMR (DMSO-d₆) δ 1.14 (3H, d), 4.62 (1H, q), 6.89 (1H, d), 7.05 (1H, t), 7.18-7.24 (3H, m), 7.47 (1H, t), 7.50 (1H, d), 7.57-7.62 (1H, m), 7.65-7.71 (3H, m), 7.91 (1H, d), 10.44 (1H, s), 11.57 (1H, s)

### Example 7(46)

### 3-{3-[(2-Methyl-4-oxo-2,3,4,5-tetrahydro-1H-1,5-benzodiazepin-1-yl) sulfonyl]phenyl}quinazoline-2,4 (1H,3H) -dione

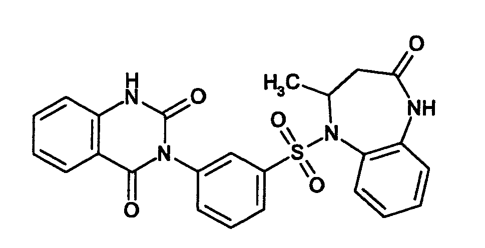

¹H-NMR (DMSO-d₆) δ 1.14 (3H, d), 2.16 (1H, t), 2.25 (1H, dd), 4.77 (1H, sevenplet), 7.03 (1H, dd), 7. 18-7.31 (4H, m), 7.38 (1H, dt), 7.47 (1H, t), 7.68-7.74 (3H, m), 7.82-7.85 (1H, m), 7.96 (1H, dd), 8.92 (1H, s), 11.66 (1H, s)

### Example 7(47)

### 3-[3-(2,3-Dihydro-4H-pyrido[3,2-b][1,4]oxazin-4-ylsulfonyl)phenyl]quinazoline-2,4(1H,3H)-dione

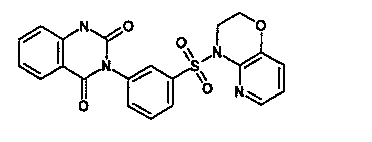

¹H-NMR (DMSO-d₆) δ 4.11 (2H, t), 4.33 (2H, t), 6.99 (1H, dd), 7.22-7.29 (3H, m), 7.63-7.74 (3H, m), 7.80 (1H, dd), 7.95 (1H, dd), 8.05-8.08 (2H, m), 11.62 (1H, s)

### Example 7 (48)

### 3-[3-(2,3-Dihydro-4H-1,4-benzothiazin-4-ylsulfonyl)phenyl]quinazoline-2,4(1H,3H)-dione

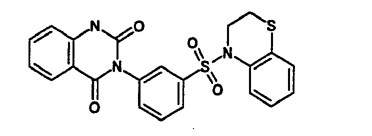

¹H-NMR (DMSO-d₆) δ 3.04 (2H, t), 3.92 (2H, t), 7.07-7.14 (3H, m), 7.21-7.25 (2H, m), 7.44-7.48 (2H, m), 7.63 (1H, t), 7.68-7.73 (2H, m), 7.92-7.96 (2H, m), 11.59 (1H, s)

### Example 7(49)

### N-(1-{[3-(2,4-Dioxo-1,4-dihydroquinazolin-3(2H)-yl)phenyl]sulfonyl}-1,2,3,4-tetrahydroquinolin-3-yl)acetamide

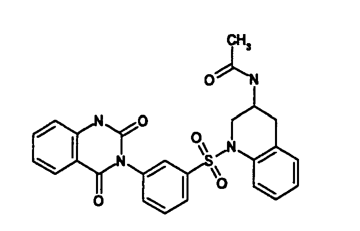

¹H-NMR (DMSO-d₆) δ 1.77 (3H, s), 2.44 (1H, dd), 2.78 (1H, dd), 3.34 (1H, dd), 3.90-3.92 (1H, m), 4.17 (1H, dd), 7.04-7.25 (5H, m), 7.53 (1H, d), 7.61-7.72 (4H, m), 7. 87-7.93 (3H, m), 11.60 (1H, s)

### Example 7(50)

### Ethyl 4-{[3-(2,4-dioxo-1,4-dihydroquinazolin-3(2H)-yl)phenyl]sulfonyl}-3,4-dihydro-2H-1,4-benzoxazine-2-carboxylate

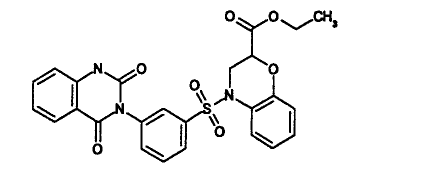

¹H-NMR (DMSO-d₆) δ 1.20 (3H, t), 4.02-4.21 (4H, m), 4.74-4.77 (1H, m), 6.92 (1H, t), 6.98 (1H, d), 7.09 (1H, t), 7.22-7.27 (2H, m), 7.48 (1H, d), 7.69-7.79 (4H, m), 7.93 (1H, d), 8.01 (1H, s), 11.63 (1H, s)

### Example 7(51)

### 3-{3-[(1-Methyl-5,6,7,8-tetrahydropyrazolo[4,3-b]azepin-4(1H)-yl)sulfonyl]phenyl}quinazoline-2,4(1H,3H)-dione

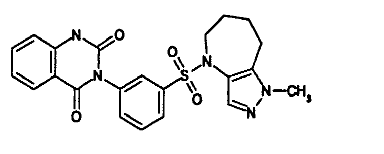

¹H-NMR (DMSO-d₆) δ 1.37-1.39 (2H, m), 1.64-1.66 (2H, m), 2.21-2.23 (2H, m), 3.65-3.67 (2H, m), 3.68 (3H, s), 7.22-7.29 (3H, m), 7.51 (1H, d), 7.65-7.74 (4H, m), 7.94 (1H, d), 11.60 (1H, s)

### Example 7(52)

### 3-{3-[(1,7,7-Trimethyl-5,6,7,8-tetrahydropyrazolo[4,3-b]azepin-4(1H)-yl)sulfonyl]phenyl}quinazoline-2,4(1H,3H)-dione

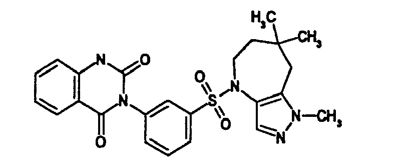

¹H-NMR (DMSO-d₆) δ 0.76 (6H, s), 1.48-1.50 (2H, m), 2.01 (2H, s), 3.65-3.67 (2H, m), 3.67 (3H, s), 7.23 (1H, d), 7.24 (1H, t), 7.29 (1H, s), 7.58-7.74 (5H, m), 7.94 (1H, dd), 11.61 (1H, s)

### Example 7(53)

### 3-{3-[(8-Methoxy-2,3,4,5-tetrahydro-1H-1-benzazepin-1-yl)sulfonyl]phenyl}quinazoline-2,4(1H,3H)-dione

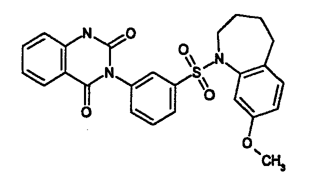

¹H-NMR (DMSO-d₆) δ 1.48-1.50 (2H, m), 1.77-1.79 (2H, m), 2.43-2.45 (2H, m), 3.61-3.63 (2H, m), 3.63 (3H, s), 6.59 (1H, d), 6. 78 (1H, dd), 7.13 (1H, d), 7.23 (1H, d), 7.23 (1H, t), 7. 67-7. 74 (4H, m), 7.93 (1H, s), 7.95 (1H, d), 11.62 (1H, s)

### Example 7(54)

### 3-{3-[(6,9-Dimethoxy-2,3,4,5-tetrahydro-1H-1-benzazepin-1-yl)sulfonyl]phenyl}quinazoline-2,4(1H,3H)-dione

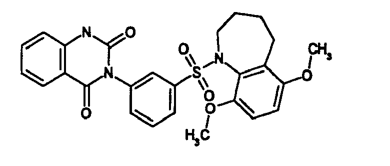

¹H-NMR (DMSO-d₆) δ 1.09-1.17 (1H, m), 1.62-1.67 (1H, m), 1.94-2.02 (2H, m), 2.44 (1H, t), 2.87 (1H, t), 3.19 (1H, dd), 3.45 (3H, s), 3.73 (3H, s), 4.06 (1H, d), 6.77 (1H, d), 6.91 (1H, d), 7.24 (1H, t), 7.25 (1H, d), 7.64-7.75 (3H, m), 7.90-7.92 (2H, m), 7.96 (1H, d), 11.62 (1H, s)

### Example 7 (55)

### 3-{3-[(6-Methoxy-2,3,4,5-tetrahydro-1H-1-benzazepin-1-yl)sulfonyl]phenyl}quinazoline-2,4(1H,3H)-dione

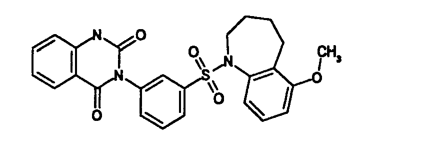

¹H-NMR (DMSO-d₆) δ 1.44-1.48 (2H, m), 1.76-1.78 (2H, m), 2.55-2.59 (2H, m), 3.56-3.60 (2H, m), 3.76 (3H, s), 6.72 (1H, d), 6.94 (1H, d), 7.11 (1H, t), 7.24 (1H, d), 7.24 (1H, t), 7.67-7.78 (4H, m), 7.93 (1H, s), 7.96 (1H, d), 11.62 (1H, s)

### Example 7(56)

### 3-{3-[(6,8-Dimethyl-2,3,4,5-tetrahydro-1H-1-benzazepin-1-yl)sulfonyl]phenyl}quinazoline-2,4(1H,3H)-dione

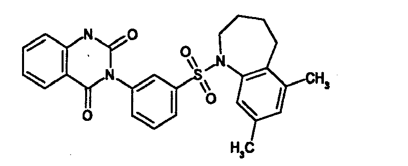

¹H-NMR (DMSO-d₆) δ 1.40-1.76 (6H, m), 2.17 (3H, s), 2.23 (3H, s), 2.48-2.52 (2H, m), 6.71 (1H, s), 6.92 (1H, s), 7.24 (1H, d), 7.24 (1H, t), 7.67-7. 75 (3H, m), 7. 83-7. 84 (2H, m), 7.95 (1H, d), 11.62 (1H, s)

### Example 7(57)

### 3-{3-[(8-Methyl-2,3,4,5-tetrahydro-1H-1-benzazepin-1-yl)sulfonyl]phenyl}quinazoline-2,4(1H,3H)-dione

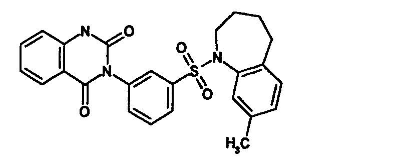

¹H-NMR (DMSO-d₆) δ 1.48-1.50 (2H, m), 1.75-1. 79 (2H, m), 2.45 (2H, t), 2.50 (3H, s), 3.58-3.62 (2H, m), 6.92 (1H, s), 7.00 (1H, d), 7.09 (1H, d), 7.23 (1H, d), 7.23 (1H, t), 7.67-7.72 (4H, m), 7.87 (1H, s), 7.94 (1H, d), 11.59 (1H, s)

### Example 7(58)

### 3-{3-[(7,8-Dimethoxy-2,3,4,5-tetrahydro-1H-1-benzazepin-1-yl)sulfonyl]phenyl}quinazoline-2,4(1H,3H)-dione

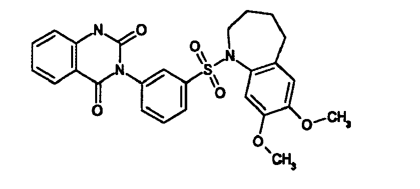

¹H-NMR (DMSO-d₆) δ 1.48-1.52 (2H, m), 1.78-1.80 (2H, m), 2.44-2.46 (2H, m), 3.56 (3H, s), 3.64-3.68 (2H, m), 3.72 (3H, s), 6.49 (1H, s), 6.79 (1H, s), 7.22 (1H, d), 7.23 (1H, t), 7.65-7.72 (4H, m), 7.89 (1H, s), 7.93 (1H, d), 11.59 (1H, s)

### Example 7(59)

### 3-[3-(2,3,7,8,9,10-Hexahydro-6H-[1,4]dioxino[2,3-h] [1]benzazepin-6-ylsulfonyl)phenyl]quinazoline-2,4(1H,3H)-dione

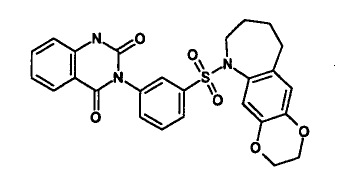

¹H-NMR (DMSO-d₆) δ 1.42-1.46 (2H, m), 1.71-1.73 (2H, m), 2.26-2.28 (2H, m), 3.56-3.60 (2H, m), 4.19 (4H, s), 6.61 (1H, s), 6.68 (1H, s), 7.22 (1H, d), 7.22 (1H, t), 7.60-7.72 (4H, m), 7.87 (1H, s), 7.94 (1H, dd), 11.59 (1H, s)

### Example 7(60)

### 3-{3-[(8-Phenyl-2,3,4,5-tetrahydro-1H-1-benzazepin-1-yl)sulfonyl]phenyl}quinazoline-2,4(1H,3H)-dione

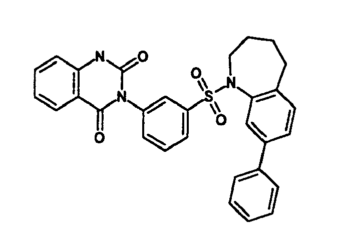

¹H-NMR (DMSO-d₆) δ 1.53-1.57 (2H, m), 1.79-1.83 (2H, m), 2.49-2.53 (2H, m), 3.68-3.72 (2H, m), 7.20-7.58 (10H, m), 7.58-7.75 (4H, m), 7.93-7.97 (2H, m), 11.61 (1H, s)

### Example 7(61)

### 3-{3-[(8-Cyclohexyl-2,3,4,5-tetrahydro-1H-1-benzazepin-1-yl)sulfonyl]phenyl}quinazoline-2,4(1H,3H)-dione

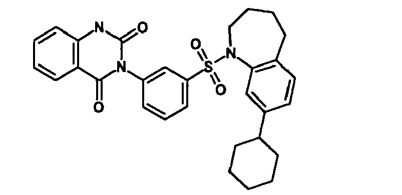

¹H-NMR (DMSO-d₆) δ 1.26-1.31 (5H, m), 1.50-1.54 (2H, m), 1.69-1.72 (7H, m), 2.38-2.42 (3H, m), 3.59-3.63 (2H, m), 6.90 (1H, s), 7.03 (1H, d), 7.12 (1H, d), 7.24 (1H, t), 7.25 (1H, d), 7.68-7.75 (4H, m), 7.88 (1H, s), 7.96 (1H, dd), 11.60 (1H, s)

### Example 7 (62)

### 3-{3-[(8-tert-Butyl-2,3,4,5-tetrahydro-1H-1-benzazepin-1-yl)sulfonyl]phenyl}quinazoline-2,4(1H,3H)-dione

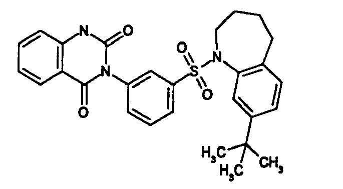

¹H-NMR (DMSO-d₆) δ 1. 16 (9H, s) , 1. 51-1. 55 (2H, m), 1.78-1. 82 (2H, m), 2.50-2.54 (2H, m), 3.61-3.65 (2H, m), 6.99 (1H, d), 7.12-7.27 (4H, m), 7.68-7.75 (4H, m), 7.92-7.98 (2H, m), 11.61 (1H, s)

### Example 7(63)

### 3-{3-[(8-Phenoxy-2,3,4,5-tetrahydro-1H-1-benzazepin-1-yl)sulfonyl]phenyl}quinazoline-2,4(1H,3H)-dione

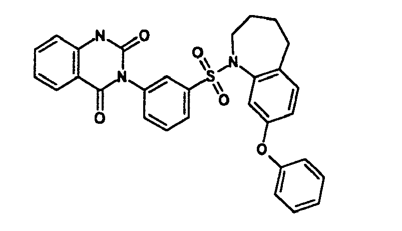

¹H-NMR (DMSO-d₆) δ 1.49-1.53 (2H, m), 1.74-1.78 (2H, m), 2.41-2.45 (2H, m), 3.60-3.64 (2H, m), 6.80 (1H, d), 6.88 (1H, dd), 7.01 (2H, d), 7. 10 (1H, t), 7.22 (1H, d), 7.24 (1H, d), 7.24 (1H, t), 7.37 (2H, t), 7.64-7.76 (4H, m), 7.85 (1H, s), 7.94 (1H, dd), 11.60 (1H, s)

### Example 7(64)

### 3-{3-[(8-Fluoro-2,3,4,5-tetrahydro-1H-1-benzazepin-1-yl)sulfonyl]phenyl}quinazoline-2,4(1H,3H)-dione

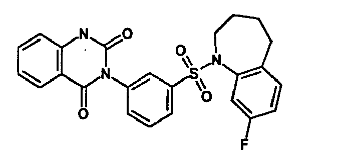

¹H-NMR (DMSO-d₆) δ 1.47-1.51 (2H, m), 1.74-1.78 (2H, m), 2.41-2.45 (2H, m), 3.61-3.65 (2H, m), 6.96 (1H, dd), 7.08 (1H, dt), 7.20-7.30 (3H, m), 7.68-7.74 (4H, m), 7.93-7.98 (2H, m), 11.61 (1H, s)

### Example 7(65)

### 3-{3-[(8-Bromo-2,3,4,5-tetrahydro-1H-1-benzazepin-1-yl)sulfonyl]phenyl}quinazoline-2,4(1H,3H)-dione

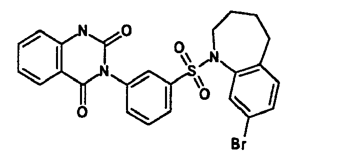

¹H-NMR (DMSO-d₆) δ 1.45-1.49 (2H, m), 1.70-1.74 (2H, m), 2.33-2.37 (2H, m), 3.63-3.67 (2H, m), 7.19 (1H, d), 7.23 (1H, d), 7.24 (1H, t), 7.35 (1H, d), 7.43 (1H, dd), 7.69-7.74 (4H, m), 7.90 (1H, s), 7.95 (1H, dd), 11. 60 (1H, s)

### Example 7 (66)

### 3-{3-[(5-Oxo-2,3-dihydro-4,1-benzoxazepin-1(5H)-yl)sulfonyl]phenyl}quinazoline-2,4(1H,3H)-dione

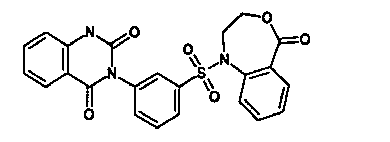

¹H-NMR (DMSO-d₆) δ 3.96 (2H, t), 4.21 (2H, t), 7.22-7.27 (3H, m), 7.53-7.77 (7H, m), 7.95-7.98 (2H, m), 11.63 (1H, s)

### Example 7(67)

### Methyl 1-{[3-(2,4-dioxo-1,4-dihydroquinazolin-3(2H)-yl)phenyl]sulfonyl}-1,2,3,4-tetrahydroquinoline-6-carboxylate

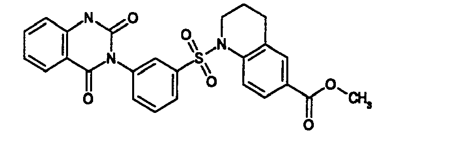

¹H-NMR (DMSO-d₆) δ 1.70 (2H, quintet), 2.64 (2H, t), 3.81 (3H, s), 3.83 (2H, t), 7.23 (1H, d), 7.24 (1H, t), 7.67-7.73 (7H, m), 7.88 (1H, s), 7.93 (1H, d), 11.61 (1H, s)

### Example 7 (68)

### 1-{[3-(2,4-Dioxo-1,4-dihydroquinazolin-3(2H)-yl)phenyl]sulfonyl}-2,3,4,5-tetrahydro-1H-1-benzazepine-8-carbonitrile

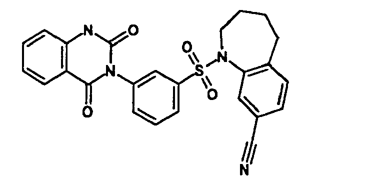

¹H-NMR (DMSO-d₆) δ 1.48-1.52 (2H, m), 1. 73-1. 77 (2H, m), 2.46-2.50 (2H, m), 3.61-3.65 (2H, m), 7.23 (1H, d), 7.24 (1H, t), 7.45 (1H, d), 7.61 (1H, d), 7.68-7.74 (5H, m), 7.92 (1H, s), 7.96 (1H, dd), 11.59 (1H, s)

### Example 7(69)

### 3-(2-Chloro-5-{[7-(trifluoromethyl)-3,4-dihydroquinolin-1(2H)-yl]sulfonyl}phenyl]quinazoline-2,4(1H,3H)-dione

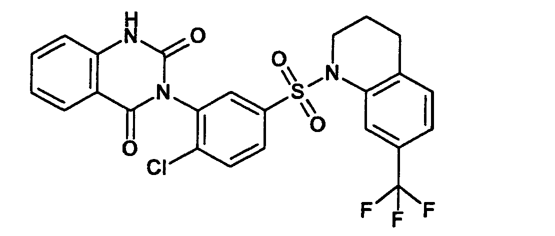

¹H-NMR (CDCl₃) δ 1.71 (2H, quintet), 2. 58-2. 59 (2H, m), 3.78-3.84 (2H, m), 7.00 (1H, d), 7. 15 (1H, d), 7.26 (1H, t), 7.32 (1H, d), 7. 54 (1H, dd), 7.60 (1H, d), 7.61 (1H, d), 7.66 (1H, d), 8.09 (1H, s), 8.10 (1H, d), 9.74 (1H, s)

### Example 7(70)

### 3-[2-Chloro-5-(5,6,7,8-tetrahydro-4H-thieno[3,2-b]azepin-4-ylsulfonyl)phenyl]quinazoline-2,4(1H,3H)-dione

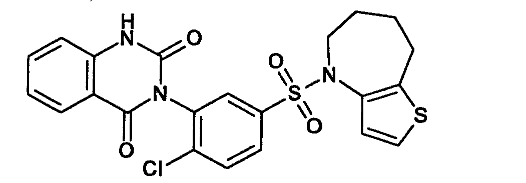

¹H-NMR (DMSO-d₆) δ 1.43-1.47 (2H, m), 1.71-1.75 (2H, m), 2.32-2.36 (2H, m), 3.62-3.66 (2H, m), 6.92 (1H, d), 7.17 (1H, d), 7.26 (1H, t), 7.26 (1H, d), 7.58 (1H, dd), 7.73 (1H, dt), 7.84 (1H, d), 7.97 (1H, dd), 8.03 (1H, d), 11.74 (1H, s)

### Example 7(71)

### 3-[2-Chloro-5-(2,3-dihydro-4,1-benzothiazepin-1(5H)-ylsulfonyl)phenyl]quinazoline-2,4(1H,3H)-dione

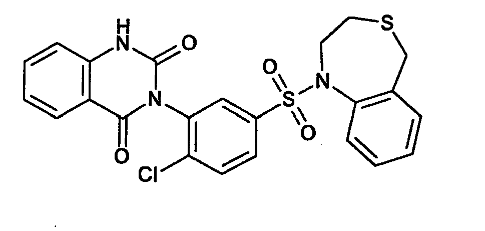

¹H-NMR (CDCl₃) δ 3.00-3.68 (6H, m), 7.03 (1H, d), 7.14-7.31 (5H, m), 7.59-7.85 (4H, m), 8.15 (1H, d), 9.72 (1H, s)

### Example 7(72)

### 3-{2-Chloro-5-[(6-fluoro-3,4-dihydroquinolin-1(2H)-yl)sulfonyl]phenyl}quinazoline-2,4(1H,3H)-dione

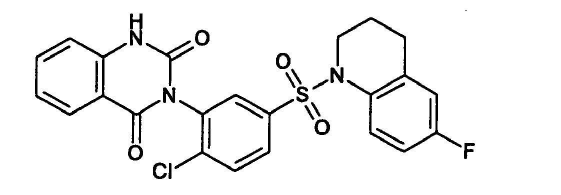

¹H-NMR (DMSO-d₆) δ 1. 63 (2H, quintet), 2.46 (2H, t), 3.75 (2H, t), 6.96 (1H, dd), 7.06 (1H, dd), 7.26 (1H, t), 7.27 (1H, d), 7. 53 (1H, dd), 7. 59 (1H, dd), 7. 74 (1H, t), 7. 81 (1H, d), 7.96 (1H, d), 8.01 (1H, d), 11.73 (1H, s)

### Example 7(73)

### 3-{2-Chloro-5-[(5-methyl-3,4-dihydroquinolin-1(2H)-yl)sulfonyl]phenyl}quinazoline-2,4(1H,3H)-dione

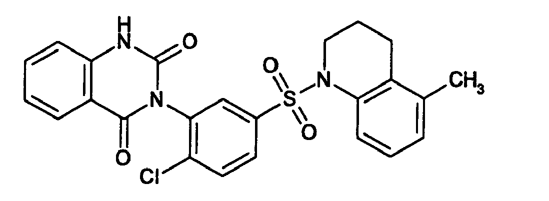

¹H-NMR (DMSO-d₆) δ 1.67 (2H, quintet), 2.15 (3H, s), 2.44 (2H, t), 3.74 (2H, t), 6.99 (1H, d), 7.08 (1H, t), 7.26 (1H, t), 7.27 (1H, d), 7.41 (1H, d), 7.62 (1H, dd), 7.74 (1H, dt), 7.82 (1H, d), 7.89 (1H, d), 7.95 (1H, dd), 11.72 (1H, s)

### Example 8

### 4-Chloro-3-(4-imino-2-oxo-1,4-dihydro-3(2H)-quinazolinyl)-N-methyl-N-phenylbenzenesulfonamide trifluoroacetate

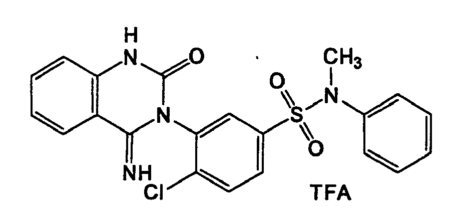

A mixture of 3-amino-4-chloro-N-methyl-N-phenylbenzenesulfonamide (445 mg), 2-isocyanatobenzonitrile (324 mg), DMAP (275 mg) and THF (9 ml) was agitated for 2 hours at room temperature, and then concentrated under reduced pressure. The residue was left to stand for 1 hour at room temperature, and then subjected to reversed-phase preparative HPLC (Gilson Inc., UniPoint System, YMC ODS column 30 × 75 mm). The fraction eluted with 0.1% TFA-containing acetonitrile/water (10 : 90 to 100 : 0) was concentrated under reduced pressure to give the desired product (328 mg).

¹H-NMR (DMSO-d₆) δ 3.17 (3H, s), 7.16-7.21 (2H, m), 7.32-7.49 (6H, m), 7.81 (1H, dd), 7.93 (1H, t), 8.03-8.07 (2H, m), 8.43 (1H, d)
HPLC (220 nm) purity 98% (retention time: 2.58 minutes)
MS (ESI+, m/e) 441 (M+1)

### Example 8(1)

In the same manner as in Example 8, the following compound was obtained from reacting 2-chloro-5-(3,4-dihydro-1(2H)-quinolinylsulfonyl)aniline with 2-isocyanatobenzonitrile.

### 3-(2-Chloro-5-(3,4-dihydro-1(2H)-quinolinylsulfonyl)phenyl)-4-imino-3,4-dihydro-2(1H)-quinazoline trifluoroacetate

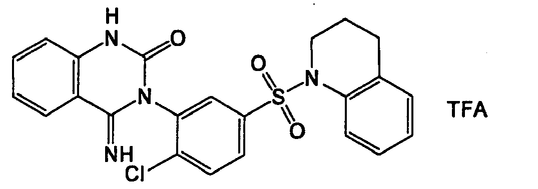

¹H-NMR (DMSO-d₆) δ 1.68-1.75 (2H, m), 2.49-2.57 (2H, m), 3.72-3.83 (2H, m), 7.10-7.23 (3H, m), 7.36-7.48 (2H, m), 7.59 (1H, d), 7.74 (1H, dd), 7.88-7.97 (2H, m), 8.30 (1H, s), 8.41 (1H, d) HPLC (220 nm) purity 98% (retention time: 3.16 minutes)
MS (ESI+, m/e) 467 (M+1)

### Example 9

### N-Butyl-4-chloro-3-(4-imino-2-oxo-1,4-dihydro-3(2H)-quinazolinyl)-N-phenylbenzenesulfonamide trifluoroacetate

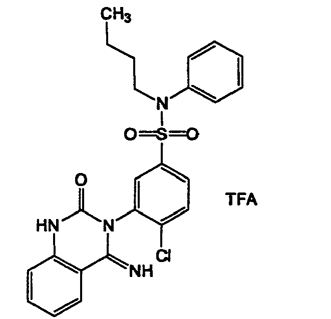

In 48-well FlexChem reactor (Robins Scientific Corp.), 2-isocyanatobenzonitrile (0.60 M THF solution, 750 µl) was added to a mixed solution of 3-amino-N-butyl-4-chloro-N-phenylbenzenesulfonamide (0.40 M THF solution, 500 µl) and DMAP (0.38 M THF solution, 800 µl), and the mixture was shaken overnight at room temperature. Subsequently, a trisamine resin (Argonaut Technologies, Inc., 4.36 mmol/g, 100 mg) was added thereto, and the mixture was further shaken for 3 hours at room temperature. The resin was filtered off, and then the filtrate was concentrated under reduced pressure using a GeneVac's centrifugal concentrator. The residue was subjected to reversed-phase preparative HPLC (Gilson Inc., UniPoint System, YMC ODS column 30 × 75 mm), and the fraction eluted with 0.1% TFA-containing acetonitrile/water (10 : 90 to 100 : 0) was concentrated under reduced pressure to give the desired product (18.5 mg).

¹H-NMR (DMSO-d₆) δ 0.76-0.90 (3H, m), 1.19-1.37 (4H, m), 3.43-3.64 (2H, m), 7.17 (2H, d), 7.32-7.52 (5H, m), 7.83-8.01 (2H, m), 8.02-8.13 (2H, m), 8.48 (1H, d)
HPLC (220 nm) purity 100% (retention time: 1.74 minutes)
MS (ESI+, m/e) 483 (M+1)

### Examples 9(1) to 9(6)

In the same manner as in Example 9, the corresponding amines (Reference Examples 5(7), 5(20), 5(21), 5(23), 5(24) and 5(26)) were reacted with 2-isocyanatobenzonitrile to obtain the following compounds.

### Example 9(1)

### 4-Chloro-N-(4-chlorophenyl)-N-ethyl-3-(4-imino-2-oxo-1,4-dihydro-3(2H)-quinazolinyl)benzenesulfonamide trifluoroacetate

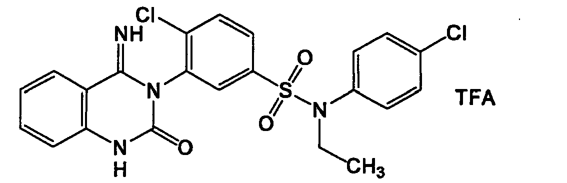

HPLC (220 nm) purity 83% (retention time: 1.70 minutes)
MS (ESI+, m/e) 489 (M+1)

### Example 9 (2)

### 3-(2-Chloro-5-(2,3-dihydro-1H-indol-1-ylsulfonyl)phenyl)-4-imino-3,4-dihydro-2(1H)-quinazoline trifluoroacetate

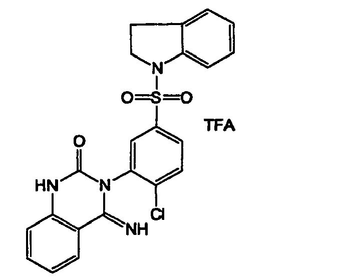

¹H-NMR (CD₃OD) δ 2.81-2.99 (2H, m), 3.88-4.06 (2H, m), 6.92-8.09 (10H, m), 8.20-8.35 (1H, m)
HPLC (220 nm) purity 99% (retention time: 1.56 minutes)
MS (ESI+, m/e) 453 (M+1)

### Example 9 (3)

### 3-(5-((4-Benzyl-1-piperidinyl)sulfonyl)-2-chlorophenyl)-4-imino-3,4-dihydro-2(1H)-quinazoline trifluoroacetate

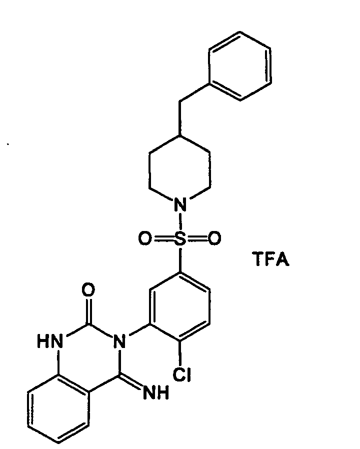

HPLC (220 nm) purity 96% (retention time: 1.79 minutes)
MS (ESI+, m/e) 509 (M+1)

### Example 9(4)

### 4-Chloro-N-cyclohexyl-3-(4-imino-2-oxo-1,4-dihydro-3(2H)-quinazolinyl)-N-methylbenzenesulfonamide trifluoroacetate

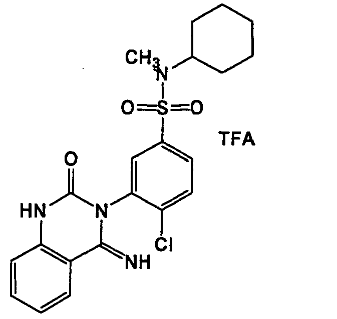

HPLC (220 nm) purity 94% (retention time: 1.56 minutes)
MS (ESI+, m/e) 447 (M+1)

### Example 9(5)

### N-Benzyl-4-chloro-N-ethyl-3-(4-imino-2-oxo-1,4-dihydro-3(2H)-quinazolinyl)benzenesulfonamide trifluoroacetate

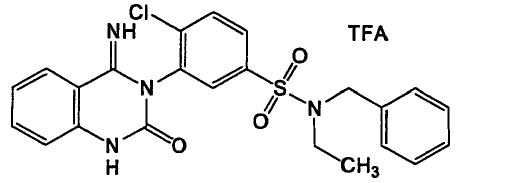

HPLC (220 nm) purity 92% (retention time: 1.66 minutes)
MS (ESI+, m/e) 455 (M+1)

### Example 9(6)

### 4-Chloro-3-(4-imino-2-oxo-1,4-dihydro-3(2H)-quinazolinyl)-N-methyl-N-2-pyridinylbenzenesulfonamide ditrifluoroacetate

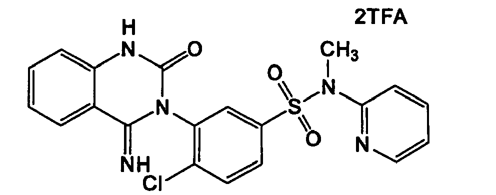

HPLC (220 nm) purity 91% (retention time: 1.43 minutes)
MS (ESI+, m/e) 442 (M+1)

### Example 10

### 4-Chloro-N-methyl-3-(1-methyl-2,4-dioxo-1,4-dihydro-3(2H)-quinazolinyl)-N-phenylbenzenesulfonamide

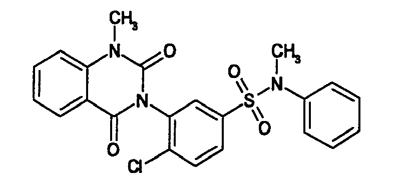

To a solution of 4-chloro-3-(2,4-dioxo-1,4-dihydroquinazolin-3(2H)-yl)-N-methyl-N-phenylbenzenesulfonamide (44.2 mg) in THF (1.0 ml), potassium carbonate (20.7 mg) and methyl iodide (22.8 µl) were added, and the mixture was reacted overnight at room temperature. Ethyl acetate was added thereto, and the mixture was sequentially washed with a saturated sodium bicarbonate solution, 1 N hydrochloric acid and saturated brine and dried over anhydrous sodium sulfate. Then, the solvent was distilled off under reduced pressure, and the residue was washed with diethyl ether to give the desired product (31.6 mg).

¹H-NMR (CDCl₃) δ 3.18 (3H, s), 3.66 (3H, s), 7.10-7.18 (2H, m), 7.22-7.39 (5H, m), 7.48-7.57 (2H, m), 7.64 (1H, d), 7.78 (1H, dt), 8.26 (1H, dd)
HPLC (220 nm) purity 92% (retention time: 2.25 minutes)
MS (ESI+, m/e) 456 (M+1)

### Examples 10(1) to 10 (3)

In the same manner as in Example 10, 4-chloro-3-(2,4-dioxo-1,4-dihydroquinazolin-3(2H)-yl)-N-methyl-N-phenylbenzenesulfonamide was alkylated with benzyl bromide, or 3-(2-chloro-5-(3,4-dihydroquinolin-1(2H)-ylsulfonyl)phenyl)quinazoline-2,4(1H,3H)-dione was alkylated with methyl iodide or benzyl bromide to obtain the following compounds.

### Example 10 (1)

### 3-(1-Benzyl-2,4-dioxo-1,4-dihydro-3(2H)-quinazolinyl)-4-chloro-N-methyl-N-phenylbenzenesulfonamide

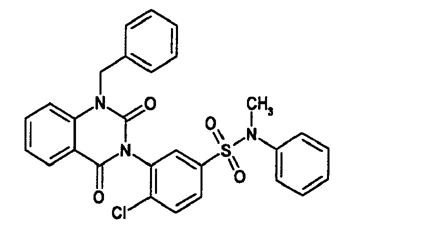

¹H-NMR (CDCl₃) δ 3.18 (3H, s), 5.35 (1H, d), 5.49 (1H, d), 7.10-7.42 (12H, m), 7.51-7.73 (4H, m), 8.25 (1H, dd)
HPLC (220 nm) purity 96% (retention time: 2.50 minutes)
MS (ESI+, m/e) 532 (M+1)

### Example 10(2)

### 3-(2-Chloro-5-(3,4-dihydro-1(2H)-quinolinylsulfonyl)phenyl)-1-methyl-2,4(1H,3H)-quinazolinedione

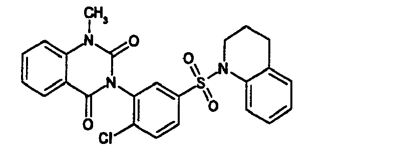

¹H-NMR (CDCl₃) δ 1.61-1.75 (2H, m), 2.51 (2H, t), 3.64 (3H, s), 3.72-3.83 (2H, m), 7.01-7.13 (2H, m), 7.16-7.23 (1H, m), 7.24-7.37 (2H, m), 7.44 (1H, dd), 7.53 (1H, d), 7.61 (d), 7.72-7.82 (2H, m), 8.24 (1H, dd)
HPLC (220 nm) purity 95% (retention time: 2.35 minutes)
MS (ESI+, m/e) 482 (M+1)

### Example 10(3)

### 1-Benzyl-3-(2-chloro-5-(3,4-dihydro-1(2H)-quinolinylsulfonyl)phenyl)-2,4(1H,3H)-quinazolinedione

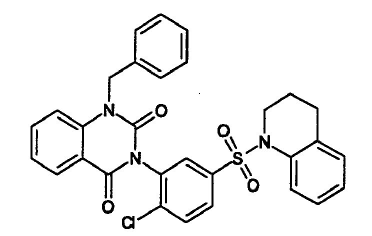

¹H-NMR (CDCl₃) δ 1.60-1.73 (2H, m), 2.39-2.59 (2H, m), 3.67-3.89 (2H, m), 5.31 (1H, d), 5.48 (1H, d), 6.95-7.12 (2H, m), 7.15-7.41 (8H, m), 7.45-7.65 (4H, m), 7.78 (1H, d), 8.23 (1H, d)
HPLC (220 nm) purity 93% (retention time: 2.59 minutes)
MS (ESI+, m/e) 558 (M+1)

### Example 11

### 3-(3-Chloro-5-(3,4-dihydro-1(2H)-quinolinylcarbonyl)phenyl)-2,4(1H,3H)-quinazolinedione

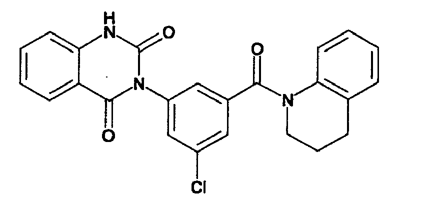

To 3-chloro-5-(3,4-dihydroquinolin-1(2H)-ylcarbonyl)benzoic acid (0.1 M THF solution, 2.0 ml) were added diphenylphosphoryl azide (43.1 µl) and triethylamine (27.9 µl), and the mixture was refluxed for 1 hour. Methyl anthranilate (77.7 µl) and DMAP (29.3 mg) were then added thereto, and the reaction mixture was refluxed overnight. Ethyl acetate was added to the reaction mixture, and the organic layer sequentially washed with 1 N hydrochloric acid, water, a sodium hydrogen carbonate solution and saturated brine, and then dried over anhydrous sodium sulfate. The solvent was distilled off under reduced pressure, and the residue was washed with diethyl ether and then subjected to reversed-phase preparative HPLC (Gilson Inc., UniPoint System, YMC ODS column 30 × 75 mm). The fraction eluted with 0.1% TFA-containing acetonitrile/water (10 : 90 to 100 : 0) was concentrated under reduced pressure to give the desired product (38.0 mg).

¹H-NMR (CDCl₃) δ 2.05 (2H, quintet), 2.84 (2H, t), 3.91 (2H, t), 6.85-7.44 (9H, m), 7.61 (1H, t), 8.09 (1H, d)
HPLC (220 nm) purity 85% (retention time: 2.12 minutes)
MS (ESI+, m/e) 432 (M+1)

### Example 12

### 3-(2-Chloro-5-(3,4-dihydro-1(2H)-quinolinylcarbonyl)phenyl)-2,4(1H,3H)-quinazolinedione

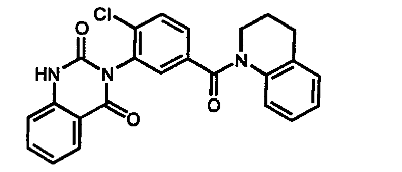

To methyl 2-((((2-chloro-5-(3,4-dihydro-1(2H)-quinolinylcarbonyl)phenyl)amino)carbonyl)amino)benzoate (118 mg), a 1 N solution of potassium hydroxide in ethanol (5.1 ml) was added, and the mixture was stirred for 45 minutes at room temperature. Then, the reaction mixture was neutralized with 1 N hydrochloric acid, and extracted with dichloromethane. The extract was sequentially washed with a 5% sodium bicarbonate solution, water and saturated brine, and dried over anhydrous magnesium sulfate. Then, the solvent was distilled off under reduced pressure, and the crystals, which were crystallized from acetonitrile/methanol/diisopropyl ether, were collected by filtration to give the desired product (91 mg).

¹H-NMR (CDCl₃) δ 1.98-2.16 (2H, m), 2.85 (2H, t), 3.79-4.10 (2H, m), 6.90 (1H, br s), 6.98-7.27 (5H, m), 7.32 (1H, dd), 7.43 (1H, d), 7.55-7.58 (1H, m), 7.60 (1H, d), 8.10 (1H, d), 10.10 (1H, br s)
HPLC (220 nm) purity 98% (retention time: 1.96 minutes)
MS (ESI+, m/e) 432 (M+1)

### Example 13

### 3-(2-Chloro-5-(3,4-dihydro-1(2H)-quinolinylcarbonyl)phenyl)-6-methoxy-2,4(1H,3H)-quinazolinedione

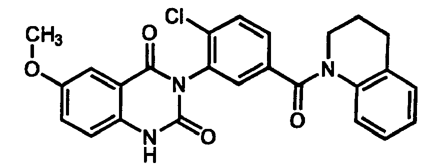

To a solution of 2-amino-N-(2-chloro-5-(3,4-dihydro-1(2H)-quinolinylcarbonyl)phenyl)-5-methoxybenzamide (51 mg) in toluene (2.3 ml), a 0.20 N solution of N,N'-carbonyldiimidazole in toluene (1.2 ml) was added, and the mixture was heated with stirring for 1 hour at 95°C. The reaction mixture was poured into 1 N hydrochloric acid, and extracted with ethyl acetate. The extract was washed with saturated brine and dried over anhydrous magnesium sulfate, and then the solvent was distilled off under reduced pressure. The residue was subjected to silica gel column chromatography, and the fraction eluted with ethyl acetate was concentrated under reduced pressure. Thus obtained crystals were collected by filtration to give the desired product (42 mg).

¹H-NMR (CDCl₃) δ 1. 97-2. 16 (2H, m), 2.84 (2H, t), 3.85 (3H, s), 3.79-4.16 (2H, m), 6.87-7.28 (6H, m), 7.32 (1H, dd), 7.43 (1H, d), 7.50 (1H, d), 7.62 (1H, d), 10.35 (1H, br s)
HPLC (220 nm) purity 99% (retention time: 1.96 minutes)
MS (ESI+, m/e) 462 (M+1)

### Example 14

### 4-Chloro-3-(2,4-dioxo-1,4-dihydroquinazolin-3(2H)-yl)-N-methyl-N-phenylbenzamide

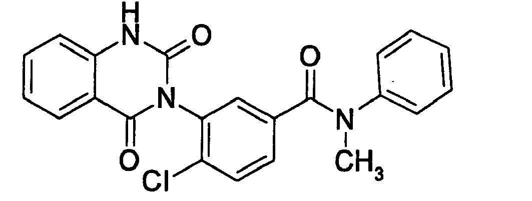

In 48-well FlexChem reactor (Robbins Scientific Corp.), N-methylaniline (26.0 µl) was added to a solution of 4-chloro-3-(2,4-dioxo-1,4-dihydroquinazolin-3(2H)-yl)benzoic acid (63.3 mg), 1-ethyl-3-(3-dimethylaminopropyl)carbodiimide hydrochloride (46.0 mg) and N-methylimidazole (19.1 µl) in dimethylformamide (500 µl) and dichloromethane (500 µl), and the mixture was shaken for 7 hours at room temperature. Thereafter, dichloromethane (2.0 ml) was added to the reaction mixture, and the organic layer was sequentially washed with 1 N hydrochloric acid, a saturated sodium bicarbonate solution, saturated brine and concentrated under reduced pressure using a GeneVac's centrifugal concentrator. The residue was subjected to reversed-phase preparative HPLC (Gilson Inc., UniPoint System, YMC ODS column 30 × 75 mm), and the fraction eluted with 0.1% TFA-containing acetonitrile/water (10 : 90 to 100 : 0) was concentrated under reduced pressure to give the desired product (16.5 mg).

¹H-NMR (CDCl₃-DMSO-d₆) δ 3.48 (3H, s), 7.06-7.09 (2H, m), 7.16-7.36 (8H, m), 7.58 (1H, dt), 8.05 (1H, dd), 11.17 (1H, s) HPLC (220 nm) purity 97% (retention time: 1.83 minutes)
MS (ESI+, m/e) 406 (M+1)

### Examples 14(1) to 14(26)

In the same manner as in Example 14, 4-chloro-3-(2,4-dioxo-1,4-dihydroquinazolin-3(2H)-yl)benzoic acid was reacted with the corresponding amines (commercially available or conventional) to obtain the following compounds.

### Example 14 (1)

### 4-Chloro-3-(2,4-dioxo-1,4-dihydroquinazolin-3(2H)-yl)-N-ethyl-N-phenylbenzamide

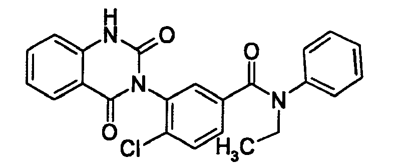

HPLC (220 nm) purity 100% (retention time: 1.91 minutes)
MS (ESI+, m/e) 420 (M+1)

### Example 14(2)

### 4-Chloro-3-(2,4-dioxo-1,4-dihydroquinazolin-3(2H)-yl)-N-isopropyl-N-phenylbenzamide

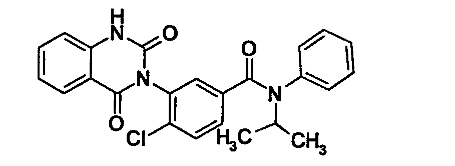

HPLC (220 nm) purity 98% (retention time: 1.97 minutes)
MS (ESI+, m/e) 434 (M+1)

### Example 14(3)

### N-Butyl-4-chloro-3-(2,4-dioxo-1,4-dihydroquinazolin-3(2H)-yl)-N-phenylbenzamide

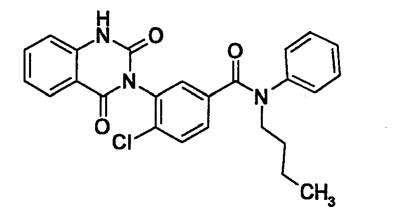

HPLC (220 nm) purity 100% (retention time: 2.08 minutes)
MS (ESI+, m/e) 448 (M+1)

### Example 14 (4)

### N-1,3-Benzodioxol-5-yl-4-chloro-3-(2,4-dioxo-1,4-dihydroquinazolin-3(2H)-yl)-N-ethylbenzamide

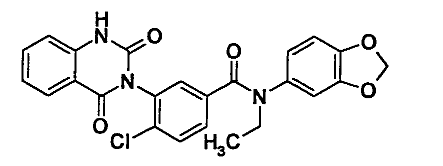

¹H-NMR (CDCl₃) δ 1.22 (3H, t), 3.72-4.08 (2H, m), 5.96 (1H, d), 5.98 (1H, d), 6.50 (1H, d), 6.60 (1H, d), 6.66 (1H, d), 7.01 (1H, d), 7.20 (1H, dt), 7.28-7.42 (2H, m), 7.48-7.61 (2H, m), 8.06 (1H, dd)
HPLC (220 nm) purity 98% (retention time: 1.89 minutes)
MS (ESI+, m/e) 464 (M+1)

### Example 14(5)

### 4-Chloro-N-(4-chlorophenyl)-3-(2,4-dioxo-1,4-dihydroquinazolin-3(2H)-yl)-N-ethylbenzamide

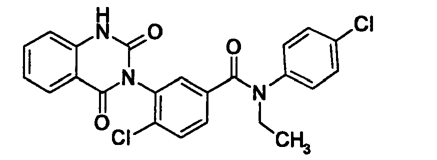

HPLC (220 nm) purity 99% (retention time: 2.02 minutes)
MS (ESI+, m/e) 454 (M+1)

### Example 14(6)

### 4-Chloro-N-(2-cyanoethyl)-3-(2,4-dioxo-1,4-dihydroquinazolin-3(2H)-yl)-N-phenylbenzamide

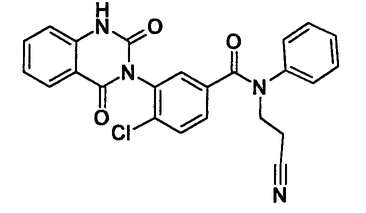

HPLC (220 nm) purity 99% (retention time: 1.81 minutes)
MS (ESI+, m/e) 445 (M+1)

### Example 14(7)

### 3-(2-Chloro-5-(2,3-dihydro-1H-indol-1-ylcarbonyl)phenyl)quinazoline-2,4(1H,3H)-dione

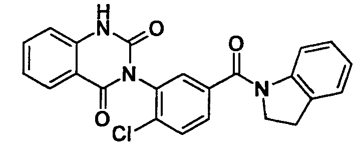

HPLC (220 nm) purity 99% (retention time: 1.91 minutes)
MS (ESI+, m/e) 418 (M+1)

### Example 14(8)

### 3-(2-Chloro-5-(3,4-dihydroisoquinolin-2(1H)-ylcarbonyl)phenyl)quinazoline-2,4(1H,3H)-dione

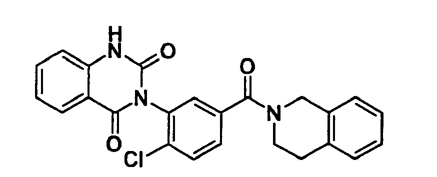

HPLC (220 nm) purity 100% (retention time: 1.90 minutes)
MS (ESI+, m/e) 432 (M+1)

### Example 14 (9)

### 3-(5-((4-Benzylpiperidin-1-yl)carbonyl)-2-chlorophenyl)quinazoline-2,4(1H,3H)-dione

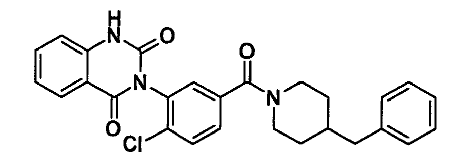

HPLC (220 nm) purity 99% (retention time: 2.08 minutes)
MS (ESI+, m/e) 474 (M+1)

### Example 14(10)

### 3-(2-chloro-5-((4-ethylpiperazin-1-yl)carbonyl)phenyl)quinazoline-2,4(1H,3H)-dione trifluoroacetate

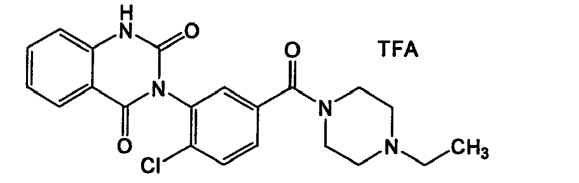

HPLC (220 nm) purity 99% (retention time: 1.29 minutes)
MS (ESI+, m/e) 413 (M+1)

### Example 14 (11) 4-Chloro-3-(2,4-dioxo-1,4-dihydroquinazolin-3(2H)-yl)-N-(1,2,3,4-tetrahydronaphthalen-1-yl)benzamide

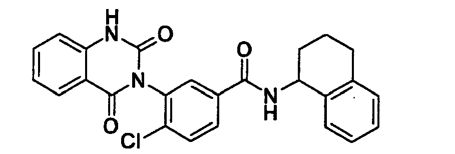

HPLC (220 nm) purity 100% (retention time: 1.98 minutes)
MS (ESI+, m/e) 446 (M+1)

### Example 14(12)

### 4-Chloro-3-(2,4-dioxo-1,4-dihydroquinazolin-3(2H)-yl)-N,N-diethylbenzamide

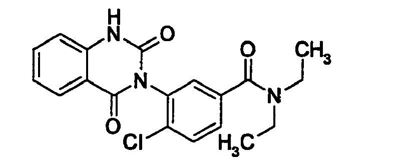

HPLC (220 nm) purity 98% (retention time: 1.71 minutes)
MS (ESI+, m/e) 372 (M+1)

### Example 14(13)

### 4-Chloro-N-cyclohexyl-3-(2,4-dioxo-1,4-dihydroquinazolin-3(2H)-yl)-N-methylbenzamide

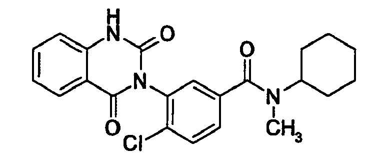

HPLC (220 nm) purity 100% (retention time: 1.92 minutes)
MS (ESI+, m/e) 412 (M+1)

### Example 14 (14)

### N-Benzyl-4-chloro-3-(2,4-dioxo-1,4-dihydroquinazolin-3(2H)-yl)-N-ethylbenzamide

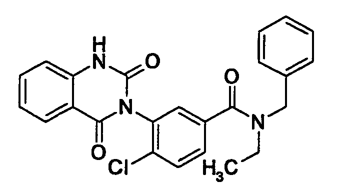

HPLC (220 nm) purity 97% (retention time: 1.95 minutes)
MS (ESI+, m/e) 434 (M+1)

### Example 14(15)

### 4-Chloro-3-(2,4-dioxo-1,4-dihydroquinazolin-3(2H)-yl)-N-methyl-N-pyridin-2-ylbenzamide trifluoroacetate

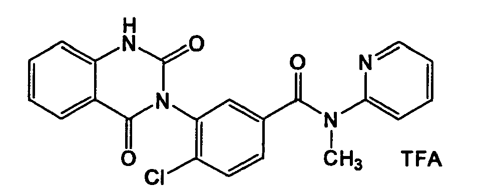

HPLC (220 nm) purity 98% (retention time: 1.62 minutes)
MS (ESI+, m/e) 407 (M+1)

### Example 14(16)

### 3-(2-Chloro-5-((6-methyl-3,4-dihydroquinolin-1(2H)-yl)carbonyl)phenyl)quinazoline-2,4(1H,3H)-dione

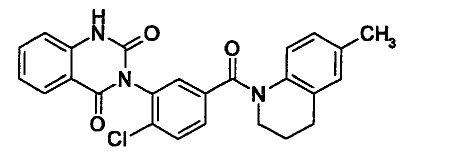

HPLC (220 nm) purity 100% (retention time: 2.02 minutes)
MS (ESI+, m/e) 446 (M+1)

### Example 14(17)

### 3-(2-Chloro-5-((2-methyl-3,4-dihydroquinolin-1(2H)-yl) carbonyl) phenyl) quinazoline-2,4(1H,3H)-dione

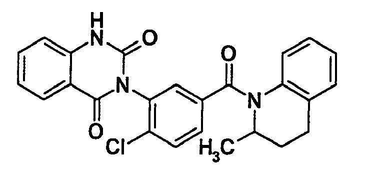

HPLC (220 nm) purity 99% (retention time: 2.01 minutes)
MS (ESI+, m/e) 446 (M+1)

### Example 14(18)

### 4-Chloro-N-(2,3-dihydro-1H-inden-1-yl)-3-(2,4-dioxo-1,4-dihydroquinazolin-3(2H)-yl)benzamide

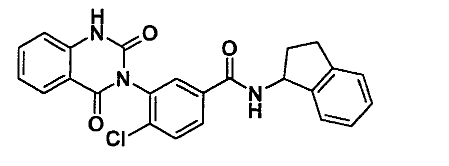

HPLC (220 nm) purity 100% (retention time: 1.92 minutes)
MS (ESI+, m/e) 432 (M+1)

### Example 14(19)

### 3-(2-Chloro-5-((5-methyl-2,3-dihydro-1H-indol-1-yl)carbonyl)phenyl)quinazoline-2,4(1H,3H)-dione

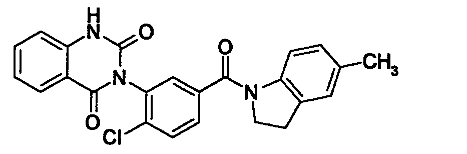

HPLC (220 nm) purity 100% (retention time: 1.99 minutes)
MS (ESI+, m/e) 432 (M+1)

### Example 14(20)

### 3-(2-Chloro-5-(2,3-dihydro-4H-1,4-benzoxazin-4-ylcarbonyl)phenyl)quinazoline-2,4(1H,3H)-dione

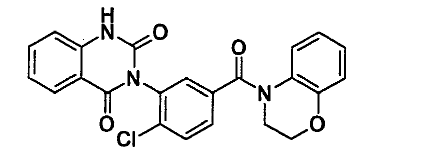

HPLC (220 nm) purity 100% (retention time: 1.89 minutes)
MS (ESI+, m/e) 434 (M+1)

### Example 14(21)

### 4-Chloro-3-(2,4-dioxo-1,4-dihydroquinazolin-3(2H)-yl)-N-methyl-N-pyridin-4-ylbenzamide

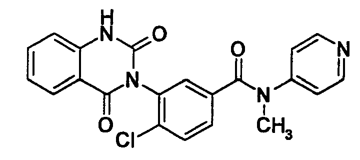

HPLC (220 nm) purity 87% (retention time: 1.35 minutes)
MS (ESI+, m/e) 407 (M+1)

### Example 14(22)

### 3-(2-chloro-5-(morpholin-4-ylcarbonyl)phenyl)quinazoline-2,4 (1H,3H)-dione

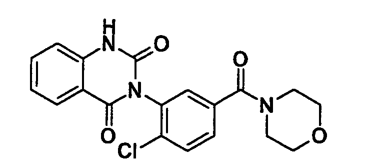

HPLC (220 nm) purity 90% (retention time: 1.53 minutes)
MS (ESI+, m/e) 386 (M+1)

### Example 14(23)

### 3-(5-(Azepan-1-ylcarbonyl)-2-chlorophenyl)quinazoline-2,4 (1H,3H)-dione

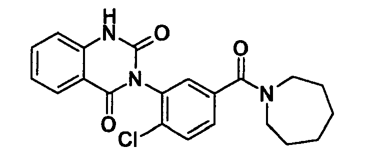

¹H-NMR (CDCl₃) δ 1.49-1.94 (8H, m), 3.36-3.54 (2H, m), 3.63-3.81 (2H, m), 7.07-7.22 (2H, m), 7.41 (1H, dd), 7.46-7.62 (2H, m), 7.70 (1H, d), 8.04 (1H, dd)
HPLC (220 nm) purity 99% (retention time: 1.80 minutes)
MS (ESI+, m/e) 398 (M+1)

### Example 14(24)

### 3-(2-Chloro-5-(octahydroisoquinolin-2(1H)-ylcarbonyl)phenyl)quinazoline-2,4(1H,3H)-dione

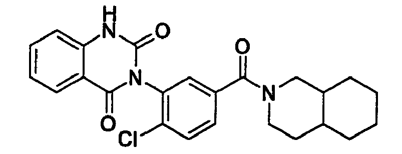

HPLC (220 nm) purity 95% (retention time: 2.05 minutes)
MS (ESI+, m/e) 438 (M+1)

### Example 14(25)

### 3-(2-Chloro-5-(octahydroquinolin-1(2H)-ylcarbonyl)phenyl)quinazoline-2,4(1H,3H)-dione

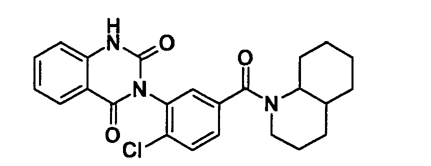

HPLC (220 nm) purity 100% (retention time: 2.02 minutes)
MS (ESI+, m/e) 438 (M+1)

### Example 14(26)

### 4-Chloro-3-(2,4-dioxo-1,4-dihydroquinazolin-3(2H)-yl)-N-1-naphthylbenzamide

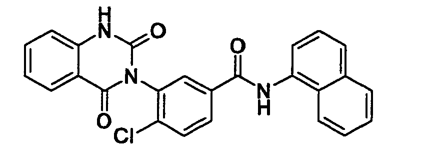

HPLC (220 nm) purity 97% (retention time: 2.06 minutes)
MS (ESI+, m/e) 442 (M+1)

### Example 15

Methyl 4-(3,4-dihydroquinolin-1(2H)-ylcarbonyl)-2-(2,4-dioxo-1,4-dihydroquinazolin-3(2H)-yl)benzoate

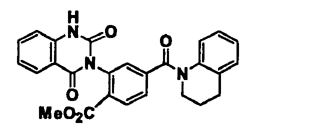

To a solution of methyl 2-[(2-aminobenzoyl)amino]-4-(3,4-dihydroquinolin-1(2H)-ylcarbonyl)benzoate (0.32 g) and N,N-diisopropylethylamine (0.52 ml) in acetonitrile (10 ml) and THF (5 ml), triphosgene (0.22 g) was added at room temperature, and the mixture was stirred overnight as it was. The reaction solution was poured into an aqueous sodium hydrogen carbonate solution, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (2 : 1 to 1 : 4)), and crystallized from ethyl acetate/diethyl ether to give the desired product (0.15 g) as crystals.

¹H-NMR (CDCl₃-DMSO-d₆) δ 2.00-2.10 (2H, m), 2.85 (2H, t), 3.72 (3H, s), 3.82-3.97 (2H, m), 6.88 (1H, br s), 6.96-7.05 (3H, m), 7.13-7.24 (3H, m), 7.39 (1H, s), 7.46 (1H, d), 7.59 (1H, dt), 8.07 (1H, dd), 10.97 (1H, s)

### Examples 15(1) to 15(3)

In the same manner as in Example 15, the following compounds were obtained from the compounds disclosed in Reference Example 21(1), 23 and 24.

### Example 15(1)

### 5-Chloro-3-[2-chloro-5-(3,4-dihydroquinolin-1(2H)-ylsulfonyl)phenyl]quinazoline-2,4(1H,3H)-dione

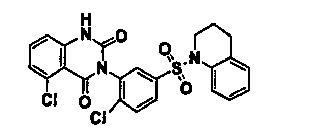

¹H-NMR (CDCl₃-DMSO-d₆) δ 1.63-1.71 (2H, m), 2.52 (2H, t), 3.78 (2H, t), 7.03-7.12 (2H, m), 7.16-7.25 (3H, m), 7.44-7.57 (4H, m), 7.74 (1H, d), 11.48 (1H, br s)

### Example 15(2)

### 5-Chloro-3-[3-(3,4-dihydroquinolin-1(2H)-ylsulfonyl)phenyl]quinazoline-2,4(1H,3H)-dione

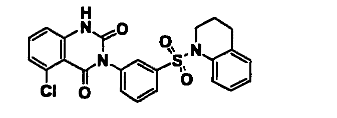

¹H-NMR (CDCl₃) δ 1.63-1.71 (2H, m), 2.50 (2H, t), 3.80 (2H, t), 6.87 (1H, dd), 7.02 (1H, dd), 7.07 (1H, dt), 7.17 (1H, dt), 7.25 (1H, dd), 7.41-7.59 (5H, m), 7.75 (1H, dd), 9.93 (1H, br s)

### Example 15 (3)

### 4-(3,4-Dihydroquinolin-1(2H)-ylcarbonyl)-2-(2,4-dioxo-1,4-dihydroquinazolin-3(2H)-yl)benzonitrile

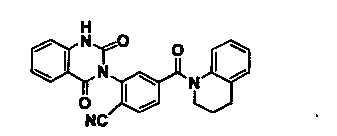

¹H-NMR (CDCl₃) δ 2.02-2.14 (2H, m), 2.85 (2H, t), 3.81-3.90 (1H, m), 3.97-4.05 (1H, m), 6.77 (1H, br s), 6.97-7.07 (3H, m), 7.15 (1H, dd), 7.25 (1H, dt), 7.45 (1H, dd), 7.56 (1H, d), 7.63 (1H, dt), 7.68 (1H, d), 8.11 (1H, dd), 9.11 (1H, br s)

### Example 16

### 3-(3,4-Dihydroquinolin-1(2H)-ylcarbonyl)-5-(2,4-dioxo-1,4-dihydroquinazolin-3(2H)-yl)benzoic acid

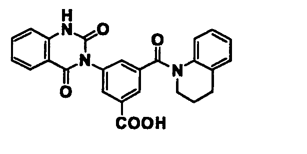

A mixture of methyl 3-(3,4-dihydroquinolin-1(2H)-ylcarbonyl)-5-(2,4-dioxo-1,4-dihydroquinazolin-3(2H)-yl)benzoate (2.65 g), lithium hydroxide monohydrate (0.73 g), THF (60 ml) and water (10 ml) was stirred for 1 hour at room temperature. The reaction solution was acidified with 1 N hydrochloric acid, and then extracted twice with ethyl acetate. The collected organic layers were dried over anhydrous magnesium sulfate, and the solvent was distilled off under reduced pressure. The resulting residue was crystallized from diethyl ether to give the desired product (2.05 g) as a powder.

¹H-NMR (DMSO-d₆) δ 1.91-2.00 (2H, m), 2.82 (2H, t), 3.76 (2H, t), 6.99-7.06 (3H, m), 7.19-7.26 (3H, m), 7.69-7.73 (2H, m), 7.88-7.96 (3H, m), 11.58 (1H, s)

### Example 17

### 3-(3,4-Dihydroquinolin-1(2H)-ylcarbonyl)-5-(2,4-dioxo-1,4-dihydroquinazolin-3(2H)-yl)benzamide

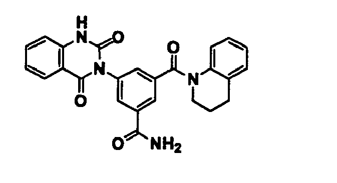

3-(3,4-Dihydroquinolin-1(2H)-ylcarbonyl)-5-(2,4-dioxo-1,4-dihydroquinazolin-3(2H)-yl)benzoic acid (0.50 g), 1-hydroxybenzotriazole (0.18 g), 1-ethyl-3-(3-dimethylaminopropyl)carbodiimide hydrochloride (0.26 g) was stirred in DMF (10 ml) for 0.5 hour at room temperature. 25% Ammonia water (0.39 g) was added to this mixture at room temperature, and the mixture was stirred for 2 hours as it was. The reaction solution was poured into water, and extracted twice with ethyl acetate. The collected organic layers were dried over anhydrous magnesium sulfate, and the solvent was distilled off under reduced pressure. The resulting residue was powdered with ethyl acetate/diethyl ether to give the desired product (0.28 g) as a powder.

¹H-NMR (CDCl₃-DMSO-d₆) δ 1.99-2.08 (2H, m), 2.85 (2H, t), 3. 89 (2H, t), 6.19 (1H, br s), 6.98-7.02 (3H, m), 7.12-7.24 (3H, m), 7.32 (1H, br s), 7.43 (1H, t), 7. 59 (1H, dt), 7.84 (1H, t), 7.98 (1H, s), 8.04 (1H, dd), 11.30 (1H, s)

### Examples 17(1) to 17(4)

In the same manner as in Example 17, 3-(3,4-dihydroquinolin-1(2H)-ylcarbonyl)-5-(2,4-dioxo-1,4-dihydroquinazolin-3(2H)-yl)benzoic acid was reacted with the corresponding commercially available amines to obtain the following compounds.

### Example 17(1)

### 3-(3,4-Dihydroquinolin-1(2H)-ylcarbonyl)-5-(2,4-dioxo-1,4-dihydroquinazolin-3(2H)-yl)-N,N-dimethylbenzamide

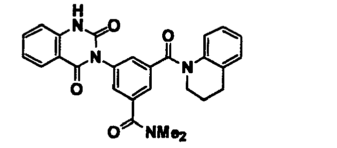

¹H-NMR (CDCl₃-DMSO-d₆) δ 2.00-2.09 (2H, m), 2. 80 (3H, s), 2.84 (2H, t), 3.03 (3H, s), 3.90 (2H, t), 6.90-7.01 (3H, m), 7.12-7.21 (3H, m), 7.36 (1H, s), 7.39 (1H, t), 7.51 (1H, t), 7.58 (1H, dt), 8.06 (1H, dd), 10.95 (1H, s)

### Example 17(2)

### 3-(3,4-Dihydroquinolin-1(2H)-ylcarbonyl)-N-[2-(dimethylamino)ethyl]-5-(2,4-dioxo-1,4-dihydroquinazolin-3(2H)-yl)benzamide

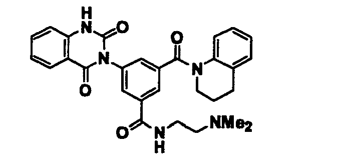

¹H-NMR (CDCl₃) δ 1.99-2.08 (2H, m), 2.24 (6H, s), 2.48 (2H, t), 2.83 (2H, t), 3.48 (2H, q), 3.90 (2H, br t), 6.87-7.04 (6H, m), 7.11-7.18 (2H, m), 7.49 (1H, t), 7.54 (1H, dt), 7.79 (1H, t), 7. 87 (1H, s), 8.00 (1H, dd)

### Example 17(3)

### 3-(3,4-Dihydroquinolin-1(2H)-ylcarbonyl)-5-(2,4-dioxo-1,4-dihydroquinazolin-3(2H)-yl)-N-(2-hydroxyethyl)benzamide

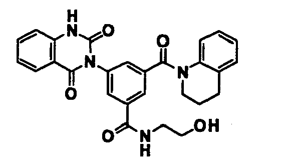

¹H-NMR (CDCl₃) δ 1.66 (1H, br s), 1.93-2.01 (2H, m), 2.79 (2H, t), 3.35 (2H, br q), 3.54 (2H, t), 3.83 (2H, t), 6.90-7.11 (6H, m), 7.41-7.46 (3H, m), 7.81 (1H, s), 7.91 (1H, d), 7.96 (1H, s)

### Example 17(4)

### N-Benzyl-3-(3,4-dihydroquinolin-1(2H)-ylcarbonyl)-5-(2,4-dioxo-1,4-dihydroquinazolin-3(2H)-yl)benzamide

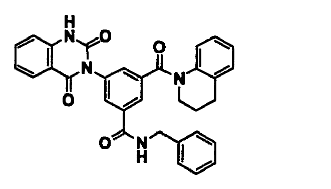

¹H-NMR (CDCl₃) δ 1.98-2.07 (2H, m), 2.82 (2H, t), 3.89 (2H, br t), 4.54 (2H, d), 6.48 (1H, t), 6.85-7.01 (4H, m), 7. 09 (1H, d, J = 5.7 Hz), 7.16 (1H, dt), 7.23-7.37 (6H, m), 7.49-7.55 (2H, m), 7.81-7.84 (2H, m), 8.03 (1H, dd)

### Example 18

### 3-(3,4-Dihydroquinolin-1(2H)-ylcarbonyl)-5-(2,4-dioxo-1,4-dihydroquinazolin-3(2H)-yl)benzonitrile

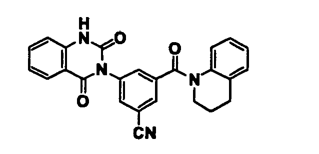

A mixture of 3-(3,4-dihydroquinolin-1(2H)-ylcarbonyl)-5-(2,4-dioxo-1,4-dihydroquinazolin-3(2H)-yl)benzamide (0.17 g), DMF (1 ml), thionyl chloride (55 µl), THF (5 ml) and toluene (20 ml) was stirred for 10 minutes at 80°C. The reaction solution was poured into an aqueous sodium hydrogen carbonate solution, and extracted twice with ethyl acetate. The collected organic layers were dried over anhydrous magnesium sulfate, and the solvent was distilled off under reduced pressure. The resulting residue was crystallized from ethyl acetate/diethyl ether to give the desired product (0.11 g) as crystals.

¹H-NMR (CDCl₃-DMSO-d₆) δ 2. 02-2. 11 (2H, m), 2.86 (2H, t), 3.91 (2H, t), 6. 80 (1H, br d), 6. 97-7.08 (2H, m), 7. 16-7.25 (3H, m), 7.59-7.65 (4H, m), 8.07 (1H, dd), 11.32 (1H, br s)

### Example 19

### 3- [3- (3, 4-Dihydroquinolin-1 (2H) -ylcarbonyl) -5-(hydroxymethyl)phenyl]quinazoline-2,4(1H,3H)-dione

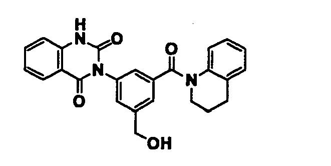

To a solution of 3-(3,4-dihydroquinolin-1(2H)-ylcarbonyl)-5-(2,4-dioxo-1,4-dihydroquinazolin-3(2H)-yl)benzoic acid (0.33 g), triethylamine (0.12 ml) in THF (30 ml), ethyl chlorocarbonate (78 µl) was added with ice cooling, and the mixture was stirred for 0.5 hour as it was. Sodium borohydride (70 mg) and methanol (10 ml) were added to this mixture with ice cooling, and the reaction mixture was stirred for 2 hours at 0°C. The reaction solution was poured into water, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (1 : 1) to ethyl acetate), and crystallized from ethyl acetate/diethyl ether to give the desired product (0.16 g) as crystals.

¹H-NMR (CDCl₃-DMSO-d₆) δ 1.98-2.07 (2H, m), 2.84 (2H, t), 3.89 (2H, t), 4.01 (1H, t), 4.65 (2H, d), 6.98-7.01 (3H, m), 7.11-23 (4H, m), 7.34 (1H, s), 7.49 (1H, s), 7. 59 (1H, dt), 8.06 (1H, dd, J = 1.2 Hz), 11.11 (1H, s)

### Example 20

### 3-[5-(3,4-Dihydroquinolin-1(2H)-ylcarbonyl)-2-furyl]quinazoline-2,4(1H,3H)-dione

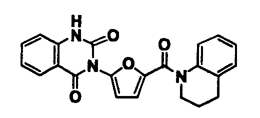

While stirring 5-(2,4-dioxo-1,4-dihydroquinazolin-3(2H)-yl)-2-furoic acid (0.31 g), 1,2,3,4-tetrahydroquinoline (0.22 g) and N-methylimidazole (0.13 ml) in THF (10 ml) and DMF (4 ml), 1-ethyl-3-(3-dimethylaminopropyl)carbodiimide hydrochloride (0.32 g) was added thereto at room temperature, and the mixture was stirred for 0.5 hour as it was. The reaction solution was poured into diluted hydrochloric acid, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (1 : 1) to ethyl acetate), and crystallized from ethyl acetate/diethyl ether to give the desired product (71 mg) as crystals.

¹H-NMR (CDCl₃) δ 1.98-2.07 (2H, m), 2.78 (2H, t), 3.93 (2H, t), 6.44 (1H, d), 6.82 (1H, d), 7.01-7.26 (6H, m), 7.63 (1H, dt), 8.09 (1H, dd), 8.65 (1H, br s)

### Example 21

### 5-(2,4-Dioxo-1,4-dihydroquinazolin-3(2H)-yl)-N-ethyl-N-phenylthiophene-3-carboxamide

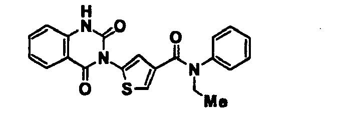

While stirring 5-(2,4-dioxo-1,4-dihydroquinazolin-3(2H)-yl)thiophene-3-carboxylic acid (0.12 g), N-ethylaniline (73 mg) and N-methylimidazole (50 mg) in DMF (15 ml), 1-ethyl-3-(3-dimethylaminopropyl)carbodiimide hydrochloride (0.12 g) was added thereto at room temperature, and the mixture was stirred overnight as it was. The reaction solution was poured into diluted hydrochloric acid, and extracted twice with ethyl acetate. The collected organic layers were dried over anhydrous magnesium sulfate, and the solvent was distilled off under reduced pressure. The resulting residue was passed through silica gel column chromatography, and then crystallized from ethyl acetate/diethyl ether/diisopropyl ether to give the desired product (65 mg) as crystals.

¹H-NMR (CDCl₃-DMSO-d₆) δ 1.16 (3H, t), 3.87 (2H, q), 6.79 (1H, d), 7.04 (1H, d), 7.08-7.16 (4H, m), 7.22-7.35 (3H, m), 7.51 (1H, ddd, J = 1.5 Hz), 7.98 (1H, dd), 11.09 (1H, s)

### Examples 21(1) to 21(13)

In the same manner as in Example 21, the corresponding carboxylic acids (Reference Examples 30(1), 81(1), 81(3), 102, 105, 108(2) and 108(3)) were reacted with the corresponding commercially available amines to obtain the following compounds.

### Example 21(1)

### 5-(2,4-Dioxo-1,4-dihydroquinazolin-3(2H)-yl)-N-phenylthiophene-3-carboxamide

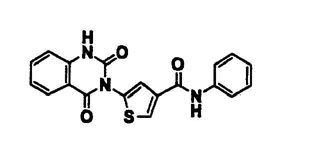

¹H-NMR (CDCl₃-DMSO-d₆) δ 7.11 (1H, t), 7.19-7.36 (4H, m), 7.59-7.64 (2H, m), 7.72 (2H, d), 8.10 (1H, dd), 8.23 (1H, d), 9.19 (1H, s), 11.46 (1H, s)

### Example 21(2)

### 5-(2,4-Dioxo-1,4-dihydroquinazolin-3(2H)-yl)-N-methyl-N-phenylthiophene-3-carboxamide

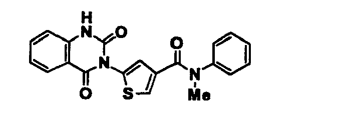

¹H-NMR (CDCl₃-DMSO-d₆) δ 3.45 (3H, s), 6.83 (1H, d), 7.15-7.31 (6H, m), 7.35-7.40 (2H, m), 7.58 (1H, dt), 8.04 (1H, d), 11.34 (1H, s)

### Example 21(3)

### N-Benzyl-5-(2,4-dioxo-1,4-dihydroquinazolin-3(2H)-yl)-N-phenylthiophene-3-carboxamide

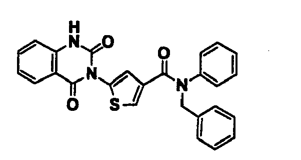

¹H-NMR (CDCl₃-DMSO-d₆) δ 5.97 (2H, s), 6.92 (1H, d), 7.04-7.09 (3H, m), 7.15-7.34 (10H, m), 7.57 (1H, dt), 8.03 (1H, dd), 11.37 (1H, s)

### Example 21(4)

### 6-(2,4-Dioxo-1,4-dihydroquinazolin-3(2H)-yl)-N-methyl-N-phenylpyridine-2-carboxamide

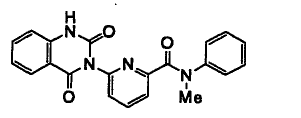

¹H-NMR (DMSO-d₆) δ 3.37 (3H, s), 7.04-7.57 (9H, m), 7.68-7.77 (1H, m), 7.87-7.99 (2H, m), 11.62 (1H, br s)

### Example 21(5)

### 6-(2,4-Dioxo-1,4-dihydroquinazolin-3(2H)-yl)-N-ethyl-N-phenylpyridine-2-carboxamide

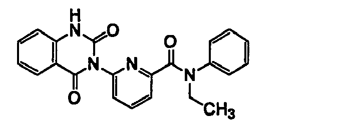

¹H-NMR (DMSO-d₆) δ 0.95-1.20 (3H, m), 3.73-4.01 (2H, m), 7.00-7.56 (9H, m), 7.64-8.02 (3H, m), 11.63 (1H, br s)

### Example 21(6)

### 3-[6-(3,4-Dihydroquinolin-1(2H)-ylcarbonyl)pyridin-2-yl]quinazoline-2,4(1H,3H)-dione

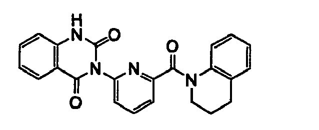

¹H-NMR (DMSO-d₆) δ 1.85-2.00 (2H, m), 2.80 (2H, t), 3.65-3.84 (2H, m), 6.94-7.37 (6H, m), 7.52-7.85 (3H, m), 7.93-8.20 (2H, m), 11.69 (1H, br s)

### Example 21(7)

### 3-[4-(3,4-Dihydroquinolin-1(2H)-ylcarbonyl)pyridin-2-yl]quinazoline-2,4(1H,3H)-dione

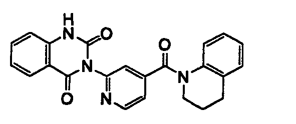

¹H-NMR (DMSO-d₆) δ 1.90-2.03 (2H, m), 2.83 (2H, t), 3.74 (2H, t), 6.95-7.11 (2H, m), 7.18-7.29 (4H, m), 7.40 (1H, d), 7.59 (1H, s), 7.72 (1H, td), 7.94 (1H, dd), 8.60 (1H, d), 11.62 (1H, br s)

### Example 21(8)

### 2-(2,4-Dioxo-1,4-dihydroquinazolin-3(2H)-yl)-N-ethyl-N-phenylisonicotinamide

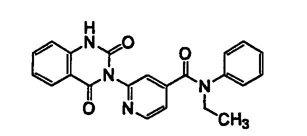

¹H-NMR (DMSO-d₆) δ 1.11 (3H, t), 3.75-3.97 (2H, m), 7.15-7.37 (8H, m), 7.46 (1H, br s), 7.67-7.77 (1H, m), 7.92 (1H, dd), 8.42 (1H, br s), 11.62 (1H, br s)

### Example 21 (9)

### 2-(2,4-Dioxo-1,4-dihydroquinazolin-3(2H)-yl)-N-methyl-N-phenylisonicotinamide

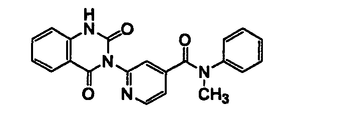

¹H-NMR (DMSO-d₆) δ 3.38 (3H, s), 7.18-7.36 (8H, m), 7.47 (1H, s), 7.67-7.77 (1H, m), 7.92 (1H, dd), 8.44 (1H, s), 11.62 (1H, s)

### Example 21(10)

### 5-(2,4-Dioxo-1,4-dihydroquinazolin-3(2H)-yl)-N-methyl-N-phenylnicotinamide

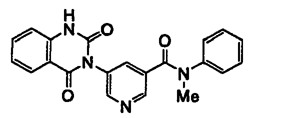

¹H-NMR (DMSO-d₆) δ 3.40 (3H, s), 7.18-7.37 (7H, m), 7.67-7.76 (1H, m), 7.83 (1H, t), 7.93 (1H, dd), 8.34 (1H, br s), 8.43 (1H, d), 11.62 (1H, br s)

### Example 21(11)

### 5-[5-(Hydroxymethyl)-2,4-dioxo-1,4-dihydroquinazolin-3(2H)-yl]-N-methyl-N-phenylnicotinamide

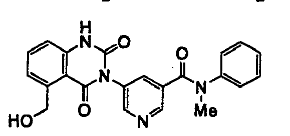

¹H-NMR (DMSO-d₆) δ 3.40 (3H, s), 4.92 (2H, d), 5.24 (1H, t), 7.12 (1H, d), 7.19-7.28 (3H, m), 7.29-7.37 (2H, m), 7.48 (1H, d), 7.67 (1H, t), 7.75-7.81 (1H, m), 8.30-8.36 (1H, m), 8.41 (1H, d), 11.56 (1H, br s)

### Example 21(12)

### 5-Chloro-4-[5-(hydroxymethyl)-2,4-dioxo-1,4-dihydroquinazolin-3(2H)-yl]-N-methyl-N-phenylthiophene-2-carboxamide

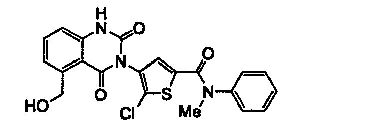

¹H-NMR (DMSO-d₆) δ 3.34 (3H, s), 4.89 (2H, d), 5.25 (1H, t), 6.86 (1H, s), 7.09 (1H, d), 7.41-7.56 (6H, m), 7.67 (1H, t), 11.59 (1H, br s)

### Example 21(13)

### 5-(2,4-Dioxo-1,4-dihydroquinazolin-3(2H)-yl)-N,4-dimethyl-N-phenylthiophene-3-carboxamide

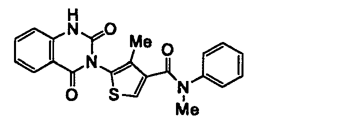

¹H-NMR (DMSO-d₆) δ 1.91 (3H, s), 3.36 (3H, s), 7.18-7.28 (6H, m), 7.30-7.39 (2H, m), 7.67-7.75 (1H, m), 7.92 (1H, dd), 11.61 (1H, br s)

### Example 22

### 5-(2,4-Dioxo-1,4-dihydroquinazolin-3(2H)-yl)-N-methyl-N-(pyridin-2-yl)thiophene-3-carboxamide

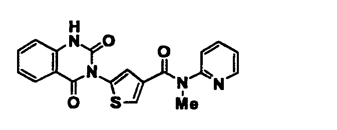

To a solution of 5-(2,4-dioxo-1,4-dihydroquinazolin-3(2H)-yl)thiophene-3-carboxylic acid (0.15 g) and DMF (1 ml) in THF (3 ml), thionyl chloride (76 µl) was added at room temperature, and the mixture was stirred for 3 hours as it was. The solvent of the reaction solution was distilled off under reduced pressure to obtain an oily matter.

To a solution of the above-obtained oily matter in THF (5 ml), 2-(methylamino)pyridine (84 mg) and triethylamine (0.15 ml) were added at room temperature, and the reaction mixture was stirred overnight at room temperature. The reaction solution was poured into diluted hydrochloric acid, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (1 : 1) to ethyl acetate), and crystallized from ethyl acetate/diisopropyl ether to give the desired product (15 mg) as a powder.

¹H-NMR (CDCl₃-DMSO-d₆) δ 3.55 (3H, s), 6.78 (1H, d), 7.02 (1H, d), 7.10-7.19 (3H, m), 7.43 (1H, d), 7.54-7.65 (2H, m), 8.04 (1H, d), 8.48 (1H, dd), 11. 16 (1H, s)

### Example 22 (1)

In the same manner as in Example 22, 5-(2,4-dioxo-1,4-dihydroquinazolin-3(2H)-yl)thiophene-3-carboxylic acid was reacted with anilinoacetonitrile to obtain the following compound.

### N-(Cyanomethyl)-5-(2,4-dioxo-1,4-dihydroquinazolin-3(2H)-yl)-N-phenylthiophene-3-carboxamide

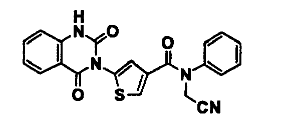

¹H-NMR (CDCl₃-DMSO-d₆) δ 4.72 (2H, s), 6.93 (1H, d), 7.15-7.20 (3H, m), 7.30-7.34 (2H, m), 7.41-7.50 (3H, m), 7.57 (1H, dt), 8.03 (1H, dd), 11.33 (1H, s)

### Example 23

### 5-(2,4-Dioxo-1,4-dihydroquinazolin-3(2H)-yl)-N-phenyl-N-(2,2,2-trifluoroethyl)thiophene-3-carboxamide

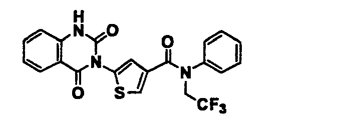

To a mixture of 5-nitro-N-phenyl-N-(2,2,2-trifluoroethyl)thiophene-3-carboxamide (0.28 g), ammonium chloride (0.23 g) and methanol (30 ml), zinc (0.84 g) was added at 60°C, and the reaction mixture was stirred for 10 minutes at 60°C. The insolubles were removed by subjecting the reaction solution to filtration, and the filtrate was distilled off under reduced pressure. The resulting residue was passed through silica gel column chromatography to obtain an oily matter.

To a solution of the above-obtained oily matter and DMAP (0.12 g) in THF (20 ml), methyl 2-isocyanatobenzoate (0.14 g) was added at room temperature, and the mixture was stirred for 1 day as it was. A 28% solution of sodium methoxide in methanol (0.12 g) was added to the reaction solution at room temperature, and the reaction mixture was stirred for 10 minutes as it was. The reaction solution was poured into diluted hydrochloric acid, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (2 : 1 to 1 : 1)), and crystallized from ethyl acetate/diethyl ether to give the desired product (90 mg) as a powder.

¹H-NMR (CDCl₃-DMSO-d₆) δ 4.52 (2H, q), 6.91 (1H, d), 7.04 (1H, d), 7.14-7.20 (2H, m), 7.27-7.30 (2H, m), 7.33-7.45 (3H, m), 7.57 (1H, ddd), 8.03 (1H, dd), 11.33 (1H, s)

### Example 24

### 5-[5-(Hydroxymethyl)-2,4-dioxo-1,4-dihydroquinazolin-3(2H)-yl]-N-methyl-N-phenylthiophene-3-carboxamide

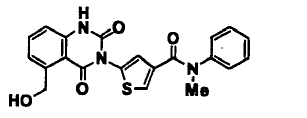

While stirring 5-[5-(hydroxymethyl)-2,4-dioxo-1,4-dihydroquinazolin-3(2H)-yl]thiophene-3-carboxylic acid (0.10 g), N-methylaniline (50 mg) and N-methylimidazole (38 µl) in DMF (10 ml), 1-ethyl-3-(3-dimethylaminopropyl)carbodiimide hydrochloride (90 mg) was added thereto at room temperature, and the mixture was stirred overnight as it was. The reaction solution was poured into diluted hydrochloric acid, and extracted twice with ethyl acetate. The collected organic layers were dried over anhydrous magnesium sulfate and passed through silica gel. Then, the solvent was distilled off under reduced pressure. The resulting residue was crystallized from ethyl acetate/diethyl ether to give the desired product (32 mg) as crystals.

¹H-NMR (CDCl₃-DMSO-d₆) δ 3.46 (3H, s), 4.21 (1H, t), 4.87 (2H, d), 6.72 (1H, d), 7.17-23 (4H, m), 7.26-7.41 (4H, m), 7.53 (1H, t), 11.41 (1H, s)

### Example 25

### 5-[5-(Hydroxymethyl)-2,4-dioxo-1,4-dihydroquinazolin-3(2H)-yl]-N-phenyl-N-(2,2,2-trifluoroethyl)thiophene-3-carboxamide

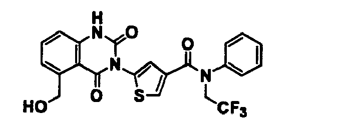

1) To a mixture of 5-nitro-N-phenyl-N-(2,2,2-trifluoroethyl)thiophene-3-carboxamide (1.22 g), ammonium chloride (0.99 g) and methanol (100 ml), zinc (3.63 g) was gradually added at 60°C, and the reaction mixture was stirred for 10 minutes at 60°C. The insolubles were removed by subjecting the reaction solution to filtration, and the filtrate was distilled off under reduced pressure. The resulting residue was passed through silica gel column chromatography (hexane/ethyl acetate (3 : 1 to 1 : 1)) to obtain 5-amino-N-phenyl-N-(2,2,2-trifluoroethyl)thiophene-3-carboxamide (0.53 g) as an oily matter.
2) To a solution of 7-amino-2-benzofuran-1(3H)-one (0.11 g) and triethylamine (0.13 ml) in THF (5 ml), a solution of triphosgene (70 mg) in THF (2 ml) was added dropwise at -20°C, and the mixture was stirred for 1 hour at room temperature. The reaction solution was diluted with THF, and the precipitates were separated by filtration. A solution of 5-amino-N-phenyl-N-(2,2,2-trifluoroethyl)thiophene-3-carboxamide (0.14 g) and DMAP (0.11 g) in THF (5 ml) was added to the filtrate at room temperature, and the reaction solution was stirred overnight as it was. The reaction solution was poured into diluted hydrochloric acid, and extracted twice with ethyl acetate. The collected organic layers were dried over anhydrous magnesium sulfate and passed through silica gel, and the solvent was distilled off under reduced pressure to obtain an oily matter.

To a solution of the above-obtained oily matter in methanol (5 ml) and THF (5 ml), a 28% solution of sodium methoxide in methanol (91 mg) was added at room temperature, and the mixture was stirred overnight at room temperature and further stirred for 1 hour at 50°C. The reaction solution was poured into diluted hydrochloric acid, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (2 : 1 to 1 : 4)), and crystallized from ethyl acetate/diethyl ether/diisopropyl ether to give the desired product (28 mg) as a powder.

¹H-NMR (CDCl₃-DMSO-d₆) δ 4.29 (1H, t), 4.53 (2H, q), 4.88 (2H, d), 6.82 (1H, d), 7.13 (1H, d), 7.17 (1H, d), 7.23-7.30 (3H, m), 7.34-7.46 (3H, m), 7.53 (1H, t), 11.50 (1H, s)

### Example 26

### 3-[2-({[tert-Butyl(dimethyl)silyl]oxy}methyl)-5-(3,4-dihydroquinolin-1(2H)-ylcarbonyl)phenyl]quinazoline-2,4(1H,3H)-dione

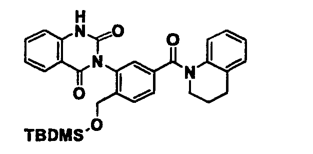

To a mixture of 1-[4-({[tert-butyl(dimethyl)silyl]oxy}methyl)-3-nitrobenzoyl]-1,2,3,4-tetrahydroquinoline (0.64 g), nickel bromide (II) (16 mg), methanol (10 ml) and THF (10 ml), sodium borohydride (0.17 g) was gradually added at room temperature, and the mixture was stirred for 10 minutes as it was. The reaction solution was poured into water, and extracted twice with ethyl acetate. The collected organic layers were dried over anhydrous magnesium sulfate, and the solvent was distilled off under reduced pressure to obtain 2-({[tert-butyl(dimethyl)silyl]oxy}methyl)-5-(3,4-dihydroquinolin-1(2H)-ylcarbonyl)aniline (0.43 g) as a solid.

To a solution of the above-obtained solid and DMAP (0.37 g) in THF (40 ml), methyl 2-isocyanatobenzoate (0.40 g) was added at room temperature, and the mixture was stirred overnight as it was. The solvent of the reaction solution was distilled off under reduced pressure. After stirring the residue for 2 hours at 80°C, it was diluted with ethyl acetate and washed with water. The resulting ethyl acetate solution was dried over anhydrous magnesium sulfate, and the solvent was distilled off under reduced pressure. The residue was purified with silica gel column chromatography (hexane/ethyl acetate (2 : 1 to 1 : 2)) to give the desired product (0.57 g) as foam.

¹H-NMR (CDCl₃) δ -0.06 (3H, s), -0.04 (3H, s), 0.83 (9H, s), 1.96-2.13 (2H, m), 2.84 (2H, t), 3.77-3.86 (1H, m), 3.99-4.07 (1H, m), 4.56 (1H, d), 4.61 (1H, d), 6.95-7.04 (4H, m), 7.11-7.14 (1H, m), 7.20 (1H, t), 7.38-7.44 (2H, m), 7.52-7.60 (2H, m), 8.08 (1H, dd), 9.73 (1H, br s)

### Example 27

### 3- [5- (3, 4-Dihydroquinolin-1 (2H) -ylcarbonyl) -2-(hydroxymethyl)phenyl]quinazoline-2,4(1H,3H)-dione

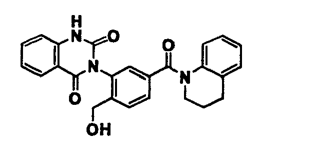

To a solution of 3-[2-({[tert-butyl(dimethyl)silyl]oxy}methyl)-5-(3,4-dihydroquinolin-1(2H)-ylcarbonyl)phenyl]quinazoline-2,4(1H,3H)-dione (0.22 g) in THF (15 ml), a 1 N solution of tetrabutylammonium fluoride in THF (0.60 ml) was added at room temperature, and the mixture was stirred for 3 hours as it was. The solvent of the reaction solution was distilled off under reduced pressure. The residue was purified with silica gel column chromatography (ethyl acetate), and powdered with ethyl acetate/diethyl ether to give the desired product (68 mg) as a powder.

¹H-NMR (CDCl₃-DMSO-d₆) δ 1.99-2.09 (2H, m), 2.84 (2H, t), 3.28 (1H, t), 3. 80-3.97 (2H, m), 4.47 (2H, d), 6.93 (1H, br s), 6.96-7.03 (2H, m), 7. 12-7.29 (4H, m), 7.44 (1H, dd), 7. 56 (1H, d), 7.62 (1H, t), 8.08 (1H, dd), 11.10 (1H, s)

### Example 28

### 3-[5-(3,4-Dihydroquinolin-1(2H)-ylsulfonyl)-2-furyl]quinazoline-2,4(1H,3H)-dione

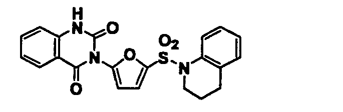

A solution of diphenylphosphoryl azide (0.29 ml) and N,N-diisopropylethylamine (0.30 ml) in toluene (15 ml) was stirred in 5-(3,4-dihydroquinolin-1(2H)-ylsulfonyl)-2-furoic acid (0.35 g) for 3 hours at 65°C. The solvent of the reaction solution was distilled off under reduced pressure to obtain an oily matter.

To a solution of methyl anthranilate (0.34 g) and DMAP (0.28 g) in THF (10 ml), a solution of the above-obtained oily matter in THF (10 ml) was added, and the reaction solution was stirred overnight at 65°C. The reaction solution was poured into diluted hydrochloric acid, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (3 : 1 to 2 : 3)), and crystallized from ethyl acetate/diethyl ether to give the desired product (33 mg) as a powder.

¹H-NMR (CDCl₃) δ 1.82-1.91 (2H, m), 2.70 (2H, t), 3.86 (2H, t), 6.47 (1H, d), 6.92 (1H, d), 7.01-7.29 (5H, m), 7.64-7.71 (2H, m), 8.12 (1H, d), 8.38 (1H, s)

### Examples 28 (1) to 28(4)

In the same manner as in Example 28, the following compounds were obtained from the compounds disclosed in Reference Examples 35 (1) to 35(4).

### Example 28 (1)

### 3-[5-(3,4-Dihydroquinolin-1(2H)-ylsulfonyl)-2-methyl-3-furyl]quinazoline-2,4(1H,3H)-dione

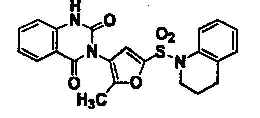

¹H-NMR (CDCl₃-DMSO-d₆) δ 1.85-1.93 (2H, m) , 2.20 (3H, s), 2.72 (2H, t), 3.83 (2H, t), 6.93 (1H, s), 7.07-7.10 (2H, m), 7.15-7.24 (3H, m), 7.60 (1H, dt), 7.67 (1H, d), 8.07 (1H, dd), 11.34 (1H, s)

### Example 28 (2)

### 3-[4-(3,4-Dihydroquinolin-1(2H)-ylsulfonyl)-3-methoxy-2-thienyl]quinazoline-2,4(1H,3H)-dione

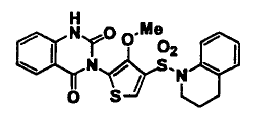

¹H-NMR (CDCl₃-DMSO-d₆) δ 1.79-2.05 (2H, m), 2.67 (2H, t), 3.67 (3H, s), 3.88 (2H, dt), 7.03-7.26 (5H, m), 7.44 (1H, s), 7.60 (1H, dt), 7.69 (1H, d), 8.06 (1H, dd), 11.36 (1H, s)

### Example 28 (3)

### 3-[5-(3,4-Dihydroquinolin-1(2H)-ylsulfonyl)-2-methyl-3-thienyl]quinazoline-2,4(1H,3H)-dione

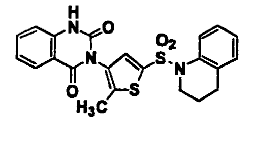

¹H-NMR (CDCl₃-DMSO-d₆) δ 1.75-1. 83 (2H, m), 2.25 (3H, s), 2.59-2.64 (2H, m), 3.74-3.88 (2H, m), 7.05-7.24 (6H, m), 7.59 (1H, dt), 7.72 (1H, d), 8.04 (1H, dd), 11.36 (1H, s)

### Example 28(4)

### 3-[4-(3,4-Dihydroquinolin-1(2H)-ylsulfonyl)-3-methyl-2-thienyl]quinazoline-2,4(1H,3H)-dione

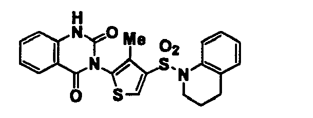

¹H-NMR (CDCl₃-DMSO-d₆) δ 1. 68-1. 81 (2H, m), 2.55 (3H, s), 2.66 (2H, t), 3.68-3. 84 (2H, m), 7.05-7.23 (5H, m), 7.56-7.63 (2H, m), 7.96 (1H, s), 8.04 (1H, d), 11.42 (1H, s)

### Example 29

### 3-[5-(3,4-Dihydroquinolin-1(2H)-ylsulfonyl)-4-methyl-1,3-thiazol-2-yl]quinazoline-2,4(1H,3H)-dione

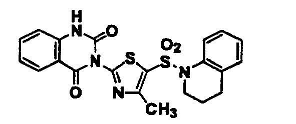

N-[5-(3,4-Dihydroquinolin-1(2H)-ylsulfonyl)-4-methyl-1,3-thiazol-2-yl]acetamide (0.25 g) and concentrated hydrochloric acid (0.5 ml) were stirred in 2-propanol (10 ml) for 5 hours at 70°C. The reaction solution was poured into an aqueous sodium hydrogen carbonate solution, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (3 : 1 to 1 : 1)) to obtain an oily matter.

To a solution of the above-obtained oily matter and DMAP (0.17 g) in acetonitrile (10 ml), methyl 2-isocyanatobenzoate (0.19 g) was added at room temperature, and the mixture was stirred for 2 days at 70°C. The solvent of the reaction solution was distilled off under reduced pressure. The resulting residue was purified with silica gel column chromatography (hexane/ethyl acetate (2 : 1) to ethyl acetate), and crystallized from ethyl acetate/diisopropyl ether to give the desired product (49 mg) as crystals.

¹H-NMR (CDCl₃-DMSO-d₆) δ 1. 81-1. 89 (2H, m), 2.28 (3H, s), 2.63 (2H, t), 3.88 (2H, t), 7.09-7.26 (5H, m), 7.62 (1H, dt), 7.71 (1H, dd), 8.06 (1H, dd), 11.63 (1H, br s)

### Example 30

### Methyl 3-[2-chloro-5-(3,4-dihydroquinolin-1(2H)-ylsulfonyl)phenyl]-2,4-dioxo-1,2,3,4-tetrahydroquinazoline-5-carboxylate

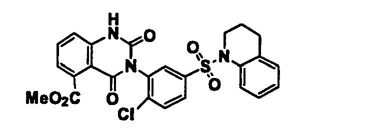

To a solution of dimethyl 3-aminophthalate (1.32 g) and triethylamine (0.88 ml) in THF (30 ml), a solution of triphosgene (0.63 g) in THF (5 ml) was added dropwise with ice cooling. The reaction solution was stirred for 0.5 hour at 60°C, and then diluted with ethyl acetate. The precipitates were separated by filtration, and the filtrate was concentrated to obtain an oily matter.

To a solution of 2-chloro-5-(3,4-dihydro-1(2H)-quinolinylsulfonyl)aniline (1.02 g) and DMAP (0.77 g) in THF (30 ml), a solution of the above-obtained oily matter in THF (30 ml) was added at room temperature. The reaction solution was stirred for 2 days at 60°C, poured into diluted hydrochloric acid, and extracted twice with ethyl acetate. The collected organic layers were dried over anhydrous magnesium sulfate, and the solvent was distilled off under reduced pressure. The resulting residue was purified with silica gel column chromatography (hexane-THF (2 : 1 to 1 : 1)), and crystallized from diethyl ether/diisopropyl ether to give the desired product (1.08 g) as crystals.

¹H-NMR (CDCl₃-DMSO-d₆) δ 1.63-1.71 (2H, m), 2.51 (2H, t), 3.78 (2H, t), 3.90 (3H, s), 7.03-7.21 (4H, m), 7.36 (1H, dd), 7.49 (1H, dd), 7.54-7.57 (2H, m), 7.64 (1H, t), 7.71 (1H, dd), 11.74 (1H, br s)

### Example 30(1)

In the same manner as in Example 30, the following compound was obtained from 3-(3,4-dihydro-1(2H)-quinolinylsulfonyl)aniline.

### Methyl 3-[3-(3,4-dihydroquinolin-1(2H)-ylsulfonyl)phenyl]-2,4-dioxo-1,2,3,4-tetrahydroquinazoline-5-carboxylate

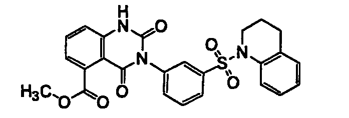

¹H-NMR (DMSO-d₆) δ 1.60-1.69 (2H, m), 2.45-2.52 (2H, m), 3.72-3.80 (5H, m), 7.06-7.10 (2H, m), 7.14-7.20 (2H, m), 7.31 (1H, d), 7.53-7.66 (4H, m), 7.73 (1H, d), 7.76-7.79 (1H, m), 11.75 (1H, s)

### Example 31

### 3-[2-Chloro-5-(3,4-dihydroquinolin-1(2H)-ylsulfonyl)phenyl]-2,4-dioxo-1,2,3,4-tetrahydroquinazoline-5-carboxylic acid

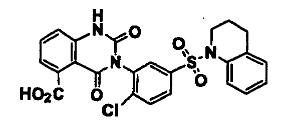

A mixture of methyl 3-[2-chloro-5-(3,4-dihydroquinolin-1(2H)-ylsulfonyl)phenyl]-2,4-dioxo-1,2,3,4-tetrahydroquinazoline-5-carboxylate (0.54 g), lithium hydroxide monohydrate (0.39 g), THF (10 ml), water (5 ml) and methanol (2 ml) was stirred for 1.5 days at 65°C. The reaction solution was poured into water, and the reaction mixture was acidified with 1 N hydrochloric acid, and then extracted twice with ethyl acetate. The collected organic layers were dried over anhydrous magnesium sulfate, and the solvent was distilled off under reduced pressure. The resulting residue was crystallized from ethyl acetate/diethyl ether/diisopropyl ether to give the desired product (0.13 g) as crystals.

¹H-NMR (DMSO-d₆) δ 1. 61-1. 70 (2H, m), 2.51 (2H, t), 3.76 (2H, t), 7.05-7.20 (4H, m), 7.28 (1H, dd), 7.53-7.57 (2H, m), 7.74 (1H, dd), 7.80 (1H, d), 8.05 (1H, d), 11.84 (1H, s), 13.09 (1H, s)

### Example 31(1)

In the same manner as in Example 31, the following compound was obtained from methyl 3-[3-(3,4-dihydroquinolin-1(2H)-ylsulfonyl)phenyl]-2,4-dioxo-1,2,3,4-tetrahydroquinazoline-5-carboxylate.

### 3-[3-(3,4-Dihydroquinolin-1(2H)-ylsulfonyl)phenyl]-2,4-dioxo-1,2,3,4-tetrahydroquinazoline-5-carboxylic acid

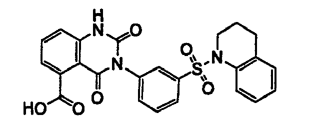

¹H-NMR (DMSO-d₆) δ 1. 58-1. 69 (2H, m), 2. 45-2. 50 (2H, m), 3.72-3.79 (2H, m), 7.06-7.20 (4H, m), 7.26 (1H, d), 7.52-7.65 (4H, m), 7.70 (1H, t), 7.74-7.77 (1H, m), 11.70 (1H, s)

### Example 32

### Sodium 3-[2-chloro-5-(3,4-dihydroquinolin-1(2H)-ylsulfonyl)phenyl]-2,4-dioxo-1,2,3,4-tetrahydroquinazoline-5-carboxylate

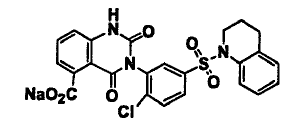

A mixture of 3-[2-chloro-5-(3,4-dihydroquinolin-1(2H)-ylsulfonyl)phenyl]-2,4-dioxo-1,2,3,4-tetrahydroquinazoline-5-carboxylic acid (0.19 g), 1 N aqueous sodium hydroxide solution (0.357 ml), THF (3 ml) and methanol (2 ml) was stirred for 10 minutes at room temperature. The solvent of the mixture was distilled off under reduced pressure. The resulting residue was crystallized from methanol/diethyl ether to give the desired product (0.19 g) as crystals.

¹H-NMR (CD30D) δ 1.63-1.71 (2H, m), 2.45-2.50 (2H, m), 3.70-3.86 (2H, m), 7.05-7.18 (5H, m), 7.44 (1H, dd), 7.59 (1H, d), 7.61 (1H, d), 7.66 (1H, d), 7.70 (1H, d).

### Example 33

2-(Trimethylsilyl)ethyl 3-[2-chloro-5-(2,3,4,5-tetrahydro-1H-1-benzazepin-1-ylsulfonyl)-3-thienyl]-2,4-dioxo-1,2,3,4-tetrahydroquinazoline-5-carboxylate

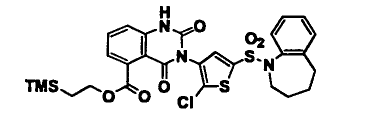

To a solution of 1-[2-(trimethylsilyl)ethyl]2-methyl 3-aminophthalate (1.38 g) and triethylamine (0.87 ml) in THF (30 ml), a solution of triphosgene (0.46 g) in THF (10 ml) was added dropwise at -20°C. The reaction solution was stirred for 0.5 hour at 60°C, and then diluted with ethyl acetate. The precipitates were separated by filtration, and the filtrate was concentrated to obtain an oily matter.

To a solution of 2-chloro-5-(2,3,4,5-tetrahydro-1H-1-benzazepin-1-ylsulfonyl)thiophen-3-amine (1.07 g) and DMAP (0.76 g) in THF (20 ml), a solution of the above-obtained oily matter in THF (30 ml) was added at room temperature. The reaction solution was stirred overnight at 60°C, poured into diluted hydrochloric acid, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (2 : 1 to 1 : 1)) to give the desired product (1.57 g) as foam.

¹H-NMR (CDCl₃) δ 0.05 (9H, s), 1.04-1.14 (2H, m), 1. 64 (2H, br s), 1.92 (2H, br s), 2.63 (2H, t), 3.68 (2H, br s), 4.41-4. 50 (2H, m), 7. 11 (1H, dd), 7. 14-7.21 (5H, m), 7.33 (1H, s), 7.65 (1H, t), 9.65 (1H, br s)

### Example 33(1)

In the same manner as in Example 33, the following compound was obtained from 4-(3,4-dihydroquinolin-1(2H)-ylsulfonyl)thiophen-2-amine.

### 2-(Trimethylsilyl)ethyl 3-[4-(3,4-dihydroquinolin-1(2H)-ylsulfonyl)-2-thienyl]-2,4-dioxo-1,2,3,4-tetrahydroquinazoline-5-carboxylate

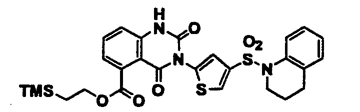

¹H-NMR (CDCl₃) δ 0.05 (9H, s), 1.08-1.13 (2H, m), 1.69-1.78 (2H, m), 2.58 (2H, t), 3.81 (2H, t), 4.42-4.48 (2H, m), 6.89 (1H, d), 7.02-7.11 (3H, m), 7.14-7.20 (2H, m), 7.63 (1H, t), 7.74 (1H, d), 7.74 (1H, d), 9.59 (1H, br s)

### Example 34

### 3-[2-Chloro-5-(2,3,4,5-tetrahydro-1H-1-benzazepin-1-ylsulfonyl)-3-thienyl]-2,4-dioxo-1,2,3,4-tetrahydroquinazoline-5-carboxylic acid

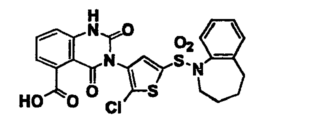

To a solution of 2-(trimethylsilyl)ethyl 3-[2-chloro-5-(2,3,4,5-tetrahydro-1H-1-benzazepin-1-ylsulfonyl)-3-thienyl]-2,4-dioxo-1,2,3,4-tetrahydroquinazoline-5-carboxylate (1.41 g) in THF (50 ml), a 1 N solution of tetrabutylammonium fluoride in THF (2.68 ml) was added at room temperature. The reaction mixture was stirred overnight at room temperature, and further stirred for 1 day at 50°C. The reaction solution was poured into diluted hydrochloric acid, and extracted twice with ethyl acetate. The collected organic layers were dried over anhydrous magnesium sulfate, and the solvent was distilled off under reduced pressure. The resulting residue was purified with silica gel column chromatography (ethyl acetate to ethyl acetate/methanol (3 : 1)), and powdered with methanol/ethyl acetate/diethyl ether to give the desired product (0.26 g) as a powder.

¹H-NMR (DMSO-d₆) δ 1.57 (2H, br s), 1.83 (2H, br s), 2.61 (2H, t), 3.62 (2H, br s), 6. 86 (1H, d), 7.03 (1H, d), 7.20-7.30 (4H, m), 7.53 (1H, t), 7.80 (1H, s), 11.60 (1H, br s)

### Example 34(1)

In the same manner as in Example 34, the following compound was obtained from 2-(trimethylsilyl)ethyl 3-[4-(3,4-dihydroquinolin-1(2H)-ylsulfonyl)-2-thienyl]-2,4-dioxo-1,2,3,4-tetrahydroquinazoline-5-carboxylate.

### 3-[4-(3,4-Dihydroquinolin-1(2H)-ylsulfonyl)-2-thienyl]-2,4-dioxo-1,2,3,4-tetrahydroquinazoline-5-carboxylic acid

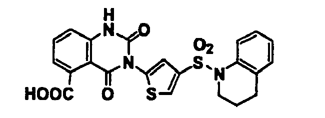

¹H-NMR (DMSO-d₆) δ 1.65-1.74 (2H, m), 2.58 (2H, t), 3.75 (2H, t), 6.94-7.20 (6H, m), 7.56-7.63 (2H, m), 8.13 (1H, d), 11.63 (1H, br s)

### Example 35

### 5-({[tert-Butyl(dimethyl)silyl]oxy}methyl)-3-[2-chloro-5-(3,4-dihydroquinolin-1(2H)-ylsulfonyl)phenyl]quinazoline-2,4(1H,3H)-dione

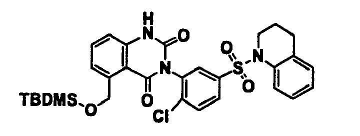

A solution of 2-({[tert-butyl(dimethyl)silyl]oxy}methyl)-N-[2-chloro-5-(3,4-dihydroquinolin-1(2H)-ylsulfonyl)phenyl]-6-nitrobenzamide (0.12 g) in THF (10 ml) was hydrogenated overnight in the presence of platinum dioxide (3 mg) at room temperature, at normal pressure. N,N-Diisopropylethylamine (0.17 ml) was added to the reaction solution, and then triphosgene (59 mg) was further added thereto with ice cooling. The mixture was stirred for 2 hours at room temperature. The reaction solution was poured into an aqueous sodium hydrogen carbonate solution, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (3 : 1 to 1 : 1)) to give the desired product (79 mg) as an oily matter.

¹H-NMR (CDCl₃) δ 0.13 (3H, s), 0.14 (3H, s), 0.98 (9H, s), 1.65-1.73 (2H, m), 2.53 (2H, t), 3.72-3.89 (2H, m), 5.22 (2H, s), 6.89 (1H, dd), 7.02 (1H, dd), 7.08 (1H, dt), 7.18 (1H, dt), 7.52-7.56 (3H, m), 7. 61 (1H, t), 7.68 (1H, dd), 7.76 (1H, dd), 9.60 (1H, s)

### Example 36

### 3-[2-Chloro-5-(3,4-dihydroquinolin-1(2H)-ylsulfonyl)phenyl]-5-(hydroxymethyl)quinazoline-2,4(1H,3H)-dione

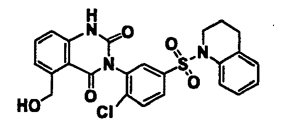

To a solution of 5-({[tert-butyl(dimethyl)silyl]oxy}methyl)-3-[2-chloro-5-(3,4-dihydroquinolin-1(2H)-ylsulfonyl)phenyl]quinazoline-2,4(1H,3H)-dione (79 mg) in THF (10 ml), a 1 N solution of tetrabutylammonium fluoride in THF (0.26 ml) was added at room temperature, and the reaction mixture was stirred for 1 day as it was. The solvent of the reaction solution was distilled off under reduced pressure. The residue was purified with silica gel column chromatography (hexane/ethyl acetate (2 : 1 to 1 : 1)) to give the desired product (33 mg) as a powder.

¹H-NMR (CDCl₃-DMSO-d₆) δ 1.63-1.72 (2H, m), 2.52 (2H, t), 3.73-3.85 (2H, m), 4.39 (1H, t), 4.85-4.98 (2H, m), 7.03 (1H, dd), 7.08 (1H, dt), 7.17 (1H, dt), 7.23 (1H, d), 7.29 (1H, d), 7.50-7.59 (4H, m), 7.72 (1H, d), 11.57 (1H, s)

### Example 37

### 3-[6-Chloro-3-(3,4-dihydroquinolin-1(2H)-ylsulfonyl)-2-methylphenyl]quinazoline-2,4(1H,3H)-dione

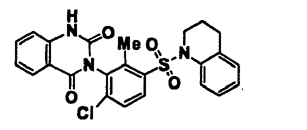

To a solution 6-chloro-3-(3,4-dihydroquinolin-1(2H)-ylsulfonyl)-2-methylaniline (0.35 g) and DMAP (0.19 g) in THF (20 ml), methyl 2-isocyanatobenzoate (0.28 g) was added at room temperature, and the mixture was stirred overnight at 65°C. A 28% solution of sodium methoxide in methanol (0.20 g) was added to the reaction solution at room temperature, and the reaction mixture was stirred for 10 minutes as it was. The reaction solution was diluted with ethyl acetate, sequentially washed with diluted hydrochloric acid and an aqueous sodium hydrogen carbonate solution, and dried over anhydrous magnesium sulfate. The solvent was distilled off under reduced pressure. The resulting residue was purified with silica gel column chromatography (hexane/ethyl acetate (2 : 1 to 1 : 1)), and crystallized from ethyl acetate/diethyl ether/diisopropyl ether to give the desired product (0.21 g) as crystals.

¹H-NMR (CDCl₃-DMSO-d₆) δ 1.74-1.83 (2H, m), 2.14 (3H, s), 2.71 (2H, t), 3.66-3.82 (2H, m), 7.01-7.14 (3H, m), 7.20 (1H, dt), 7.26 (1H, d), 7.44 (1H, d), 7.50 (1H, d), 7.61 (1H, dt), 7.95 (1H, d), 8.07 (1H, dd), 11.42 (1H, s)

### Example 38

### 3-[5-(3,4-Dihydroquinolin-1(2H)-ylsulfonyl)-2-thienyl]quinazoline-2,4(1H,3H)-dione

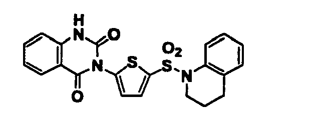

To a solution of 5-(3,4-dihydroquinolin-1(2H)-ylsulfonyl)thiophen-2-amine (80 mg) and DMAP (66 mg) in THF (10 ml), methyl 2-isocyanatobenzoate (72 mg) was added at room temperature, and the mixture was stirred overnight at room temperature. A 28% solution of sodium methoxide in methanol (52 mg) was added to the reaction solution at room temperature, and the reaction mixture was stirred for 10 minutes as it was. The reaction solution was poured into diluted hydrochloric acid, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (3 : 1 to 1 : 1)), and crystallized from ethyl acetate/diethyl ether to give the desired product (42 mg) as crystals.

¹H-NMR (CDCl₃-DMSO-d₆) δ 1.74-1.83 (2H, m), 2.58 (2H, t), 3. 84 (2H, t), 7.04-7.12 (2H, m), 7.06 (1H, d), 7.16-7.22 (3H, m), 7.26 (1H, d), 7.59 (1H, ddd), 7.76 (1H, dd), 8.07 (1H, dd), 11.33 (1H, s)

### Examples 38(1) to 38(3)

In the same manner as in Example 38, the following compounds were obtained from the corresponding amines (compounds disclosed in Reference Examples 61, 86 and 86(1)).

### Example 38 (1)

### 3-(3-{[3-{[tert-Butyl(dimethyl)silyl]oxy}-3,4-dihydro-1,5-benzoxazepin-5(2H)-yl]sulfonyl}phenyl)quinazoline-2,4(1H,3H)-dione

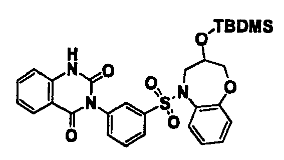

¹H-NMR (CDCl₃) δ 0.12 (6H, s), 0.96 (9H, s), 3.03 (1H, dd), 3.32 (1H, dd), 4. 11-4.23 (2H, m), 4.38 (1H, ddd), 7.00-7.09 (3H, m), 7.20 (1H, dt), 7.26 (1H, t), 7.43 (1H, dd),7.47-7.65 (4H, m), 7.74 (1H, s), 8.12 (1H, dd), 9.16 (1H, s)

### Example 38(2)

### 3-[3-(6,7,8,9-Tetrahydro-5H-benzo[7]annulen-5-ylthio)phenyl]quinazoline-2,4(1H,3H)-dione

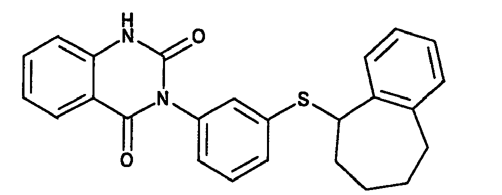

¹H-NMR (DMSO-d₆) δ1.27-1.47 (1H, m), 1.70-1.94 (3H, m), 1.97-2.17 (2H, m), 2.69-2.86 (1H, m), 3.14-3.28 (1H, m), 4.82-4.87 (1H, m), 7.01-7.28 (7H, m), 7.33-7.42 (2H, m), 7.47 (1H, s), 7.66-7.75 (1H, m), 7.95 (1H) , 11.56 (1H, s)

### Example 38(3)

### 3-{3-[(1-Phenylethyl)thio]phenyl}quinazoline-2,4(1H,3H)-dione

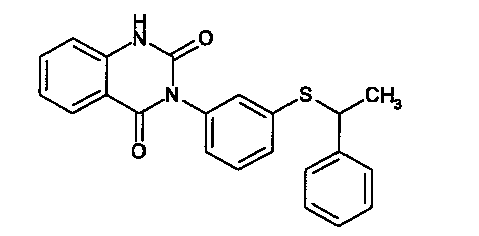

¹H-NMR (DMSO-d₆) δ 1.57 (3H, d), 4.67 (1H, q), 7.14-7.45 (11H, m), 7.66-7.75 (1H, m), 7.94 (1H, dd), 11.56 (1H, s)

### Example 39

### 3-(3-{[3-({[tert-Butyl(dimethyl)silyl]oxy}methyl)-2,3-dihydro-4H-1,4-benzoxazin-4-yl]sulfonyl}phenyl)quinazoline-2,4(1H,3H)-dione

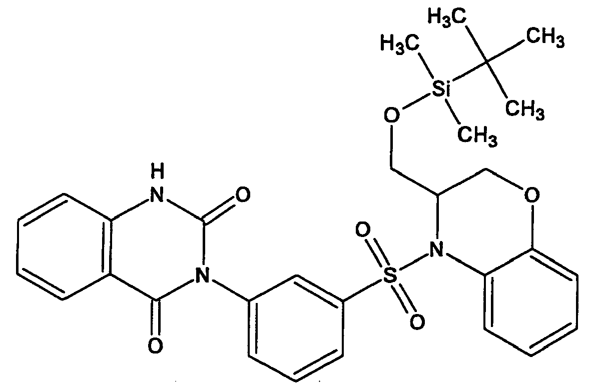

1) A solution of 3-({[tert-butyl(dimethyl)silyl]oxy}methyl)-4-[(3-nitrophenyl)sulfonyl]-3,4-dihydro-2H-1,4-benzoxazine (0.27 g) in THF (2 ml) and methanol (2 ml) was hydrogenated overnight in the presence of 10% Palladium carbon (50% water content, 0.1 g) at room temperature, at normal pressure. The catalyst was separated by filtration, and then the filtrate was concentrated. The resulting residue was purified with silica gel column chromatography (hexane/ethyl acetate (3 : 1)) to obtain 3-{[3-({[tert-butyl(dimethyl)silyl]oxy}methyl)-2,3-dihydro-4H-1,4-benzoxazin-4-yl]sulfonyl}aniline (0.26 g) as an oily matter. 2) To a solution of the above-obtained oily matter (0.26 g) and DMAP (0.15 g) in THF (15 ml), methyl 2-isocyanatobenzoate (0.16 g) was added at room temperature, and the mixture was stirred overnight at room temperature. A 28% solution of sodium methoxide in methanol (0.12 g) was added to the reaction solution at room temperature, and the reaction mixture was stirred for 10 minutes as it was. The reaction solution was diluted with ethyl acetate, sequentially washed with diluted hydrochloric acid and an aqueous sodium hydrogen carbonate solution, and then dried over anhydrous magnesium sulfate. The solvent was distilled off under reduced pressure. The resulting residue was purified with silica gel column chromatography (hexane/ethyl acetate (3 : 1 to 1 : 1)) to give the desired product (0.11 g) as foam.

¹H-NMR (CDCl₃) δ -0.02 (3H, s), 0.01 (3H, s), 0.84 (9H, s), 3.32 (1H, dd), 3.45 (1H, t), 3.70 (1H, dd), 4.26 (1H, dd), 4.31-4.37 (1H, m), 6.81 (1H, dd), 6.93 (1H, ddd), 6.99 (1H, d), 7.05 (1H, ddd), 7.25 (1H, dt), 7.50-7.57 (3H, m), 7.62 (1H, ddd), 7.69 (1H, t), 7.91 (1H, dd), 8.11 (1H, dd), 10.15 (1H, s)

### Example 39(1)

In the same manner as in Example 39, the following compound was obtained from 1-[(4-nitro-2-thienyl)sulfonyl]-2,3,4,5-tetrahydro-1H-1-benzazepine.

### 3-[5-(2,3,4,5-Tetrahydro-1H-1-benzazepin-1-ylsulfonyl)-3-thienyl]quinazoline-2,4(1H,3H)-dione

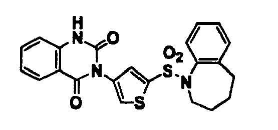

¹H-NMR (CDCl₃-DMSO-d₆) δ 1.64 (2H, br s), 1.87-1.96 (2H, m), 2.58-2.62 (2H, m), 3.72 (2H, br s), 7.15-7.25 (5H, m), 7.35-7.41 (1H, m), 7.52 (1H, d), 7.57-7.63 (2H, m), 8.10 (1H, d), 11.19 (1H, s)

### Example 40

### 3-{3-[(3-Methoxy-3,4-dihydro-1,5-benzoxazepin-5(2H)-yl)sulfonyl]phenyl}quinazoline-2,4(1H,3H)-dione

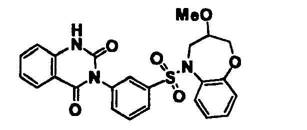

To a mixture of 3-methoxy-5-[(3-nitrophenyl)sulfonyl]-2,3,4,5-tetrahydro-1,5-benzoxazepine (0.12 g), nickel bromide (II) (3.7 mg), methanol (5 ml) and THF (5 ml), sodium borohydride (38 mg) was added at room temperature, and the mixture was stirred for 1 hour as it was. The reaction solution was poured into water, and extracted twice with ethyl acetate. The collected organic layers were dried over anhydrous magnesium sulfate, and the solvent was distilled off under reduced pressure to obtain an oily matter.

To a solution of the above-obtained oily matter and DMAP (82 mg) in THF (15 ml), methyl 2-isocyanatobenzoate (89 mg) was added at room temperature, and the mixture was stirred overnight at room temperature. A 28% solution of sodium methoxide in methanol (65 mg) was added to the reaction solution at room temperature, and the reaction mixture was stirred for 10 minutes as it was. The reaction solution was poured into diluted hydrochloric acid, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (3 : 1 to 2 : 3)), and crystallized from diethyl ether/diisopropyl ether to give the desired product (0.13 g) as crystals.

¹H-NMR (CDCl₃) δ 3.32-3.46 (1H, m), 3.43 (3H, s), 3.68-3.76 (2H, m), 4.05-4.13 (1H, m), 4.30-4.38 (1H, m), 6.98-7.06 (3H, m), 7.15-7.29 (2H, m), 7.47-7.66 (5H, m), 7.81 (1H, s), 8.13 (1H, d), 8.78 (1H, s)

### Examples 40(1) to 40(3)

In the same manner as in Example 40, the following compounds were obtained from the corresponding nitro compounds (compounds disclosed in Reference Examples 64, 67 and 68).

### Example 40(1)

### 3-{3-[(3-Fluoro-3,4-dihydro-1,5-benzoxazepin-5(2H)-yl)sulfonyl]phenyl}quinazoline-2,4(1H,3H)-dione

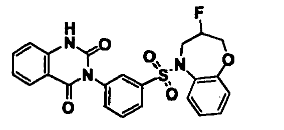

¹H-NMR (CDCl₃) δ 3.94 (1H, br t), 4. 03-4. 15 (3H, m), 4.89 (1H, br d), 7.00-7.10 (3H, m), 7.18-7.28 (.2H, m), 7.49-7.66 (5H, m), 7.73 (1H, s), 8.14 (1H, d), 9.01 (1H, s)

### Example 40(2)

### 2-[3-(2,4-Dioxo-1,4-dihydroquinazolin-3(2H)-yl)phenyl]-N-methyl-N-phenylacetamide

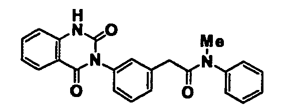

¹H-NMR (CDCl₃) δ 3.28 (3H, s), 3.53 (2H, s), 7.01-7.04 (2H, m), 7.12-7.17 (4H, m), 7.20 (1H, dt), 7.28-7.40 (4H, m), 7.57 (1H, ddd), 8.11 (1H, dd), 9.40 (1H, s)

### Example 40 (3)

### 2-[3-(2,4-Dioxo-1,4-dihydroquinazolin-3(2H)-yl)phenyl]-N-methyl-N-phenylpropanamide

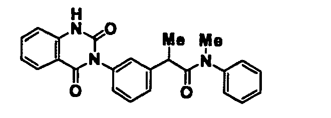

¹H-NMR (CDCl₃) δ 1.44 (3H, d), 3.25 (3H, s), 3.68 (1H, q), 6.97-7.16 (6H, m), 7.21 (1H, dt), 7.28-7.39 (4H, m), 7.55 (1H, dt), 8.12 (1H, d), 9.79 (1H, s)

### Example 41

### 3-(3-{[3-(Hydroxymethyl)-2,3-dihydro-4H-1,4-benzoxazin-4-yl]sulfonyl}phenyl)quinazoline-2,4(1H,3H)-dione

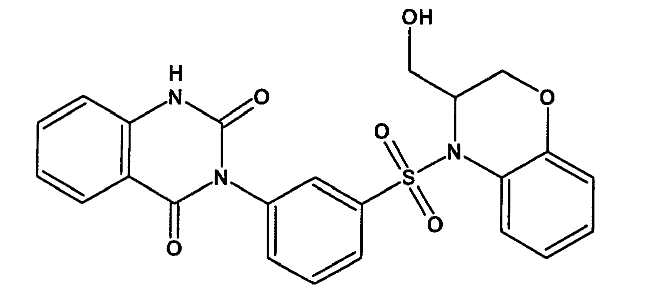

To a solution of 3-(3-{[3-({[tert-butyl(dimethyl)silyl]oxy}methyl)-2,3-dihydro-4H-1,4-benzoxazin-4-yl]sulfonyl}phenyl)quinazoline-2,4(1H,3H)-dione (97 mg) in THF (5 ml), a 1 N solution of tetrabutylammonium fluoride in THF (0.19 ml) was added at room temperature, and the mixture was stirred for 2 hours as it was. The solvent of the reaction solution was distilled off under reduced pressure. The residue was purified with silica gel column chromatography (hexane/ethyl acetate (2: 1 to 1: 2)), and powdered with diethyl ether to give the desired product (53 mg) as a powder.

¹H-NMR (CDCl₃) δ 2.50 (1H, br s), 3.38 (1H, dd), 3.51 (1H, dd), 3.61 (1H, dd), 4.14 (1H, d), 4.38 (1H, br t), 6.79 (1H, dd), 6.93 (1H, dt), 6.98 (1H, d), 7.07 (1H, dt), 7.23 (1H, t), 7.53 (3H, s), 7.58 (1H, dt), 7.82 (1H, s), 7.85 (1H, dd), 8.09 (1H, dd)

### Example 41(1)

In the same manner as in Example 41, the following compound was obtained from 3-(3-{[3-{[tert-butyl(dimethyl)silyl]oxy}-3,4-dihydro-1,5-benzoxazepin-5(2H)-yl]sulfonyl}phenyl)quinazoline-2,4(1H,3H)-dione.

### 3-{3-[(3-Hydroxy-3,4-dihydro-1,5-benzoxazepin-5(2H)-yl)sulfonyl]phenyl}quinazoline-2,4(1H,3H)-dione

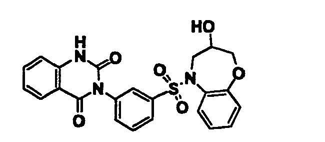

¹H-NMR (CDCl₃-DMSO-d₆) δ 3.63-4.00 (5H, m), 7.02 (1H, dd), 7.07 (1H, dt), 7.18-7.26 (3H, m), 7.46 (1H, dd), 7.50-7.55 (3H, m), 7.61 (1H, dt), 7.86 (1H, s), 8.09 (1H, dd), 11.31 (1H, s)

### Example 42

### 3-(2,4-Dioxo-1,4-dihydroquinazolin-3(2H)-yl)-5-(2,3,4,5-tetrahydro-1H-1-benzazepin-1-ylsulfonyl)thiophene-2-carbonitrile

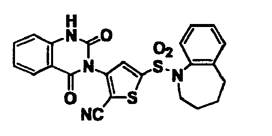

A mixture of 3-(2,4-dioxo-1,4-dihydroquinazolin-3(2H)-yl)-5-(2,3,4,5-tetrahydro-1H-1-benzazepin-1-ylsulfonyl)thiophene-2-carboxamide (45 mg), DMF (0.5 ml), thionyl chloride (20 µl) and THF (5 ml) was stirred at room temperature for 2 hours. The reaction solution was poured into an aqueous sodium hydrogen carbonate solution, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (4 : 1 to 1 : 1)) and crystallized from diisopropyl ether/hexane to give the desired product (13 mg) as a powder.

¹H-NMR (CDCl₃-DMSO-d₆) δ 1.66 (2H, br s), 1.92 (2H, t), 2.55-2.59 (2H, m), 3.74 (2H, br s), 7.17-7.37 (6H, m), 7.46 (1H, s), 7.62 (1H, dt), 8.10 (1H, dd), 11.46 (1H, s)

### Example 43

### 2-(2,4-Dioxo-1,4-dihydroquinazolin-3(2H)-yl)-5-(2,3,4,5-tetrahydro-1H-1-benzazepin-1-ylsulfonyl)thiophene-3-carbonitrile

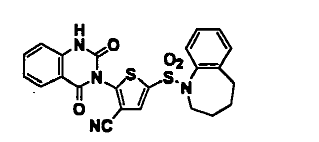

A solution of N-[3-cyano-5-(2,3,4,5-tetrahydro-1H-1-benzazepin-1-ylsulfonyl)-2-thienyl]-2-nitrobenzamide (0.11 g) in THF (5 ml) and methanol (5 ml) was hydrogenated for 1 hour in the presence of 10% palladium carbon (50% water content, 0.1 g) at room temperature at normal pressure. The catalyst was separated by filtration, and then the filtrate was concentrated to obtain a solid (0.14 g).

To a solution of the above-obtained solid and N,N-diisopropylethylamine (0.20 ml) in THF (10 ml) was added triphosgene (68 mg) at -30°C, and the mixture was stirred at room temperature for 1 hour. The reaction solution was poured into an aqueous sodium hydrogen carbonate solution, and extracted twice with ethyl acetate. The collected organic layers were dried over anhydrous magnesium sulfate, and the solvent was distilled off under reduced pressure. The resulting residue was purified with silica gel column chromatography (hexane/ethyl acetate (2 : 1 to 1 : 1)), and crystallized from ethyl acetate/diethyl ether to give the desired product (58 mg) as crystals.

¹H-NMR (CDCl₃-DMSO-d₆) δ 1.66 (2H, br s), 1.90-1.97 (2H, m), 2.61 (2H, t), 3.73 (2H, br s), 7.19-7.34 (6H, m), 7.58 (1H, s), 7.65 (1H, dt), 8.12 (1H, dd), 11.66 (1H, s)

### Example 44

### 4-Chloro-3-(2,4-dioxo-1,4-dihydroquinazolin-3(2H)-yl)-N-methyl-N-pyridin-2-ylbenzamide

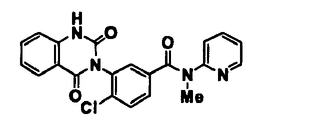

To a solution of 3-amino-4-chloro-N-methyl-N-pyridin-2-ylbenzamide (0.61 g) and DMAP (0.43 g) in THF (30 ml) was added methyl 2-isocyanatobenzoate (0.50 g) at room temperature, and the mixture was stirred at 65°C overnight. The solvent of the reaction solution was distilled off under reduced pressure, and the residue was stirred at 80°C for 1 hour. To the reaction solution was added diluted hydrochloric acid, and extracted twice with ethyl acetate. The collected organic layers were dried over anhydrous magnesium sulfate and passed through silica gel. The solvent was distilled off under reduced pressure. The resulting residue was crystallized from ethyl acetate/diethyl ether to give the desired product (0.37 g) as crystals.

¹H-NMR (CDCl₃-DMSO-d₆) δ 3.56 (3H, s), 6.91 (1H, d), 7.08 (1H, ddd), 7.19 (1H, dt), 7.24 (1H, d), 7.31-7.42 (3H, m), 7.54-7.62 (2H, m), 8.03 (1H, dd), 8.42 (1H, ddd), 11.30 (1H, s)

### Example 45

### 3-[5-({[tert-Butyl(dimethyl)silyl]oxy}methyl)-2,4-dioxo-1,4-dihydroquinazolin-3(2H)-yl]-4-chloro-N-methyl-N-phenylbenzamide

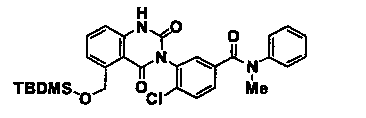

To a solution of methyl 2-amino-6-({[tert-butyl(dimethyl)silyl]oxy}methyl)benzoate (2.61 g) and triethylamine (1.64 ml) in THF (30 ml), was added dropwise a solution of triphosgene (0.87 g) in THF (10 ml) at -20°C, and the mixture was stirred at room temperature for 0.5 hour. The reaction solution was diluted with ethyl acetate, the precipitates were separated by filtration, and the filtrate was concentrated to obtain an oily matter.

To a solution of 3-amino-4-chloro-N-methyl-N-phenylbenzamide (1.54 g) and DMAP (1.44 g) in THF (40 ml) was added a solution of the above-obtained oily matter in THF (20 ml) at room temperature, and the mixture was stirred at room temperature for 3 days. The reaction solution was poured into an aqueous ammonium chloride solution, and extracted twice with ethyl acetate. The collected organic layers were dried over anhydrous magnesium sulfate, and the solvent was distilled off under reduced pressure. The resulting residue was crystallized from ethyl acetate/diethyl ether/diisopropyl ether to give the desired product (2.99 g) as crystals.

¹H-NMR (CDCl₃) δ 0.13 (3H, s), 0.14 (3H, s), 0.98 (9H, s), 3.51 (3H, s), 5.20 (2H, s), 6.90 (1H, d), 7.07-7.35 (8H, m), 7.56-7.66 (2H, m), 9.35 (1H, br s)

### Example 45 (1)

In the same manner as in Example 45, the following compound was obtained from methyl 2-amino-6-({[tert-butyl(dimethyl)silyl]oxy}methyl)benzoate and 3-amino-4-chloro-N-methyl-N-pyridin-2-ylbenzamide.

### 3-[5-({[tert-Butyl(dimethyl)silyl]oxy}methyl)-2,4-dioxo-1,4-dihydroquinazolin-3(2H)-yl]-4-chloro-N-methyl-N-pyridin-2-ylbenzamide

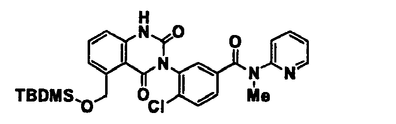

¹H-NMR (CDCl₃) δ 0.13 (6H, s), 0.98 (9H, s), 3.59 (3H, s), 5.19 (2H, s), 6.90 (2H, d), 7.07 (1H, t), 7.33 (1H, s), 7.42 (2H, s), 7.54-7.66 (3H, m), 8.43 (1H, d), 9.46 (1H, s)

### Example 46

### 4-Chloro-3-[5-(hydroxymethyl)-2,4-dioxo-1,4-dihydroquinazolin-3(2H)-yl]-N-methyl-N-phenylbenzamide

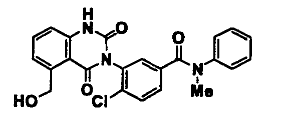

To a solution of 3-[5-({[tert-butyl(dimethyl)silyl]oxy}methyl)-2,4-dioxo-1,4-dihydroquinazolin-3(2H)-yl]-4-chloro-N-methyl-N-phenylbenzamide (2.89 g) in THF (50 ml), was added a 1 N solution of tetrabutylammonium fluoride in THF (10.5 ml) at room temperature, and the mixture was stirred overnight as it was. The reaction solution was poured into diluted hydrochloric acid, and extracted twice with ethyl acetate. The collected organic layers were dried over anhydrous magnesium sulfate and passed through silica gel. The solvent was distilled off under reduced pressure. The resulting residue was crystallized from ethyl acetate/diethyl ether to give the desired product (2.02 g) as crystals.

¹H-NMR (CDCl₃-DMSO-d₆) δ 3.49 (3H, s) , 4.32 (1H, t), 4.79 (1H, dd), 4.92 (1H, dd), 7.08 (2H, d), 7. 18-7.23 (4H, m), 7.28-7.33 (2H, m), 7.38-7.46 (2H, m), 7.55 (1H, t), 11.44 (1H, s)

### Example 46 (1)

In the same manner as in Example 46, the following compound was obtained from 3-[5-({[tert-butyl(dimethyl)silyl]oxy}methyl)-2,4-dioxo-1,4-dihydroquinazolin-3(2H)-yl]-4-chloro-N-methyl-N-pyridin-2-ylbenzamide.

### 4-Chloro-3-[5-(hydroxymethyl)-2,4-dioxo-1,4-dihydroquinazolin-3(2H)-yl]-N-methyl-N-pyridin-2-ylbenzamide

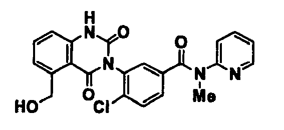

¹H-NMR (CDCl₃-DMSO-d₆) δ 3.57 (3H, s), 4.39 (1H, t), 4.83 (1H, dd), 4.92 (1H, dd), 6.91 (1H, d), 7.11 (1H, dd), 7.19-7.27 (3H, m), 7.47 (2H, s), 7.53-7.63 (2H, m), 8.42-8.43 (1H, m), 11.49 (1H, s)

### Example 47

### 3-[2-Chloro-5-(2,3,4,5-tetrahydro-1H-1-benzazepin-1-ylsulfonyl)-3-thienyl]-5-(hydroxymethyl)quinazoline-2,4(1H,3H)-dione

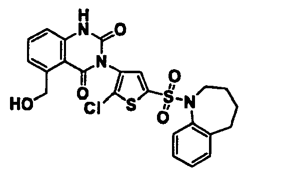

1) To a mixture of 7-amino-2-benzofuran-1(3H)-one (500 mg), triethylamine (0.98 ml) and THF (10 ml), was added a solution of triphosgene (0.35 g) in THF (1 ml) at -40°C, and the mixture was stirred at -30 to 0°C for 1 hour. After elevating the temperature to room temperature (5 minutes), the insolubles were filtered off and washed with THF. The mother liquor and a washing liquor were combined and concentrated. To a mixture of the resulting residue, 2-chloro-5-(2,3,4,5-tetrahydro-1H-1-benzazepin-1-ylsulfonyl)thiophen-3-amine (1.20 g) and THF (16 ml), was added DMAP (0.61 g) at room temperature, and the mixture was stirred for 20 hours. Insolubles were filtered off and washed with THF. The mother liquor and a washing liquor were combined and partitioned into ethyl acetate and brine. The organic layer was washed with 1 N hydrochloric acid and brine, and then purified with silica gel column chromatography (ethyl acetate/hexane) and crystallized from ethyl acetate to obtain N-[2-chloro-5-(2,3,4,5-tetrahydro-1H-1-benzazepin-1-ylsulfonyl)-3-thienyl]-N'-(3-oxo-1,3-dihydro-2-benzofuran-4-yl)urea (1.00 g) as crystals.
2) A mixture of N-[2-chloro-5-(2,3,4,5-tetrahydro-1H-1-benzazepin-1-ylsulfonyl)-3-thienyl]-N'-(3-oxo-1,3-dihydro-2-benzofuran-4-yl)urea (0.95 g), sodium methoxide (0.15 g) and methanol (15 ml) was stirred at 70°C for 1 hour. After cooling to room temperature, the solvent of the reaction solution was distilled off under reduced pressure. To the residue was added water, and the mixture was acidified with 1 N hydrochloric acid and extracted with ethyl acetate. The extract was purified with silica gel column chromatography (ethyl acetate) and crystallized from ethyl acetate to give the desired product (836 mg) as crystals.

¹H-NMR (CDCl₃) δ 1.60-1.70 (2H, m), 1.88-1.96 (2H, m), 2.55-2.70 (2H, m), 3.5-3.9 (2H, br s), 3.91 (1H, t), 4.89 (1H, dd), 4.95 (1H, dd), 7.04-7.07. (1H, m), 7.17-7.30 (5H, m), 7.35-7.39 (1H, m), 7.62 (1H, t), 9.63 (1H, br s)

### Examples 47 (1) to 47(2)

In the same manner as in Example 47, the following compounds were obtained from 7-amino-2-benzofuran-1(3H)-one and 3-(3,4-dihydro-1(2H)-quinolinylsulfonyl)aniline or 5-amino-N-ethyl-N-phenylthiophene-3-carboxamide.

### Example 47(1)

### 3-[3-(3,4-Dihydroquinolin-1(2H)-ylsulfonyl)phenyl]-5-(hydroxymethyl)quinazoline-2,4(1H,3H)-dione

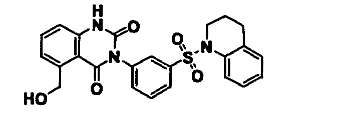

¹H-NMR (DMSO-d₆) δ 1. 60-1. 68 (2H, m), 2.45-2.50 (2H, m), 3.74-3.78 (2H, m), 4.93 (2H, d), 5.24 (1H, t), 7.06-7.19 (4H, m), 7.46-7.69 (7H, m), 11.51 (1H, br s)

### Example 47(2)

### N-Ethyl-5-[5-(hydroxymethyl)-2,4-dioxo-1,4-dihydroquinazolin-3(2H)-yl]-N-phenylthiophene-3-carboxamide

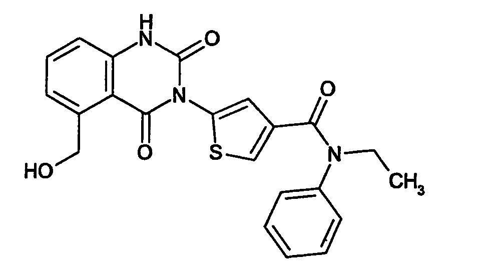

¹H-NMR (DMSO-d₆) δ 1.10 (3H, t), 3.83 (2H, q), 4.91 (2H, d), 5.25 (1H, t), 6.85 (1H, d), 7.05-7.15 (2H, m), 7.23-7.37 (3H, m), 7.38-7.50 (3H, m), 7.65 (1H, t), 11.52 (1H, s)

### Example 48

### 3-[3-(3,4-Dihydroquinolin-1(2H)-ylsulfonyl)phenyl]-2,4-dioxo-1,2,3,4-tetrahydroquinazoline-5-carbaldehyde

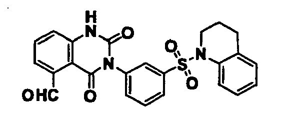

A mixture of 3-[3-(3,4-dihydroquinolin-1(2H)-ylsulfonyl)phenyl]-5-(hydroxymethyl)quinazoline-2,4(1H,3H)-dione (300 mg), manganese dioxide (2.10 g) and DMF (6 ml) was stirred at room temperature for 20 hours. The reactant was diluted with ethyl acetate, and then insolubles were filtered off through Celite and washed with DMF. The mother liquor and a washing liquor were combined and concentrated. The residue was crystallized from ethyl acetate to give the desired product (267 mg) as crystals.

¹H-NMR (DMSO-d₆) δ 1.60-1.68 (2H, m), 2.46-2.51 (2H, m), 3.74-3.78 (2H, m), 7.06-7.08 (2H, m), 7.12-7.19 (1H, m), 7.39 (1H, d), 7.46 (1H, d), 7.52-7.69 (4H, m), 7.78-7.83 (2H, m), 10.75 (1H, s), 11.85 (1H, br s)

### Example 49

### Ethyl 3-{3-[3-(3,4-dihydroquinolin-1(2H)-ylsulfonyl)phenyl]-2,4-dioxo-1,2,3,4-tetrahydroquinazolin-5-yl}acrylate

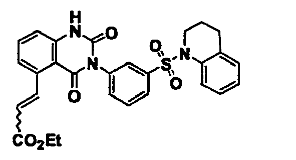

To a mixture of ethyl (diethoxyphosphoryl)acetate (365 mg) and DMF (4 ml) was added sodium hydride (60% in oil, 65 mg) at room temperature, and the mixture was stirred for 20 minutes. 3-[3-(3,4-Dihydroquinolin-1(2H)-ylsulfonyl)phenyl]-2,4-dioxo-1,2,3,4-tetrahydroquinazoline-5-carbaldehyde (250 mg) was added thereto, and the mixture was stirred for 40 minutes. The reactant was poured into water, and the mixture was acidified with 1 N hydrochloric acid and extracted with ethyl acetate. The extract was washed with water, dried and concentrated. The resulting residue was purified with silica gel column chromatography (ethyl acetate/hexane) to give the desired product (256 mg) as a solid (mixture having about 7 : 1 of E-isomer and Z-isomer).

¹H-NMR (CDCl₃) δ 1.16 (3HX1/8, t), 1.32 (3HX7/8, t), 1.62-1. 71 (2H, m), 2.48-2.54 (2H, m), 3.79-3.83 (2H, m), 4.07 (2HX1/8, q), 4.26 (2HX7/8, q), 6.03 (1HX1/8, d), 6.24 (1HX7/8, d), 6.96-7.79 (11H+1HX1/8, m), 8.71 (1HX7/8, d), 9.41 (1HX1/8, br s), 9.67 (1HX7/8, br s)

### Example 50

### Ethyl 3-{3-[3-(3,4-dihydroquinolin-1(2H)-ylsulfonyl)phenyl]-2,4-dioxo-1,2,3,4-tetrahydroquinazolin-5-yl}propanoate

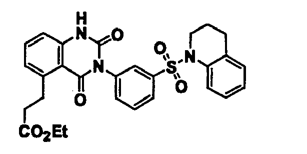

A mixture of ethyl 3-{3-[3-(3,4-dihydroquinolin-1(2H)-ylsulfonyl)phenyl]-2,4-dioxo-1,2,3,4-tetrahydroquinazolin-5-yl}acrylate (100 mg), 10% palladium carbon (50% water content, 8 mg), ethanol (7 ml) and DMF (3 ml) was stirred under hydrogen atmosphere at room temperature for 36 hours. The catalyst was filtered off through Celite and washed with ethanol. The mother liquor and a washing liquor were combined and concentrated. The resulting residue was purified with silica gel column chromatography (ethyl acetate/hexane) and crystallized from diethyl ether to give the desired product (81 mg) as crystals.

¹H-NMR (CDCl₃) δ 1.22 (3H, t), 1.63-1.72 (2H, m), 2.50 (2H, t), 2.64 (2H, t), 3.43 (2H, t), 3.79-3.83 (2H, m), 4.10 (2H, q), 6.85-6.88 (1H, m), 7.02-7.11 (3H, m), 7.16-7.21 (1H, m), 7.45-7.57 (4H, m), 7.61-7.64 (1H, m), 7.77-7.80 (1H, m), 9.33 (1H, s)

### Example 51

### 3-{3-[3-(3,4-Dihydroquinolin-1(2H)-ylsulfonyl)phenyl]-2,4-dioxo-1,2,3,4-tetrahydroquinazolin-5-yl}propanoic acid

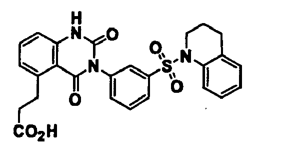

A mixture of ethyl 3-{3-[3-(3,4-dihydroquinolin-1(2H)-ylsulfonyl)phenyl]-2,4-dioxo-1,2,3,4-tetrahydroquinazolin-5-yl}propanoate (45 mg), 1 N sodium hydroxide (0.5 ml) and methanol (1 ml) was stirred at room temperature for 3 hours. The reactant was acidified with 1 N hydrochloric acid, and then extracted with ethyl acetate. The extract was purified with silica gel column chromatography (ethyl acetate/hexane) to give the desired product (33 mg) as crystals.

¹H-NMR (DMSO-d₆) δ 1.59-1.68 (2H, m), 2.45-2.50 (4H, m), 3.24-3.29 (2H, m), 3.74-3.78 (2H, m), 7.03-7.20 (5H, m), 7.53-7.68 (6H, m), 11.51 (1H, s), 12.09 (1H, br s)

### Example 51(1)

In the same manner as in Example 51, the following compound was obtained from ethyl 3-[2-chloro-5-(3,4-dihydroquinolin-1(2H)-ylsulfonyl)phenyl]-2-oxo-1,2,3,4-tetrahydroquinazoline-5-carboxylate.

### 3-[2-Chloro-5-(3,4-dihydroquinolin-1(2H)-ylsulfonyl)phenyl]-2-oxo-1,2,3,4-tetrahydroquinazoline-5-carboxylic acid

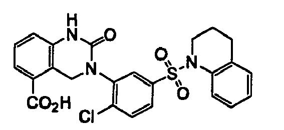

¹H-NMR (DMSO-d₆) δ 1.59-1.71 (2H, m), 2.41-2.49 (2H, m), 3.75-3.86 (2H, m), 4.97 (2H, s), 7.03-7.24 (4H, m), 7.33 (1H, t), 7.49-7.63 (3H, m), 7.76 (1H, d), 7.81 (1H, d), 9.86 (1H, brs)

### Example 52

### 3-[3-(3,4-Dihydroquinolin-1(2H)-ylsulfonyl)phenyl]-5-[(dimethylamino)methyl]quinazoline-2,4(1H,3H)-dione

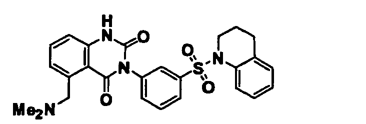

To a mixture of 3-[3-(3,4-dihydroquinolin-1(2H)-ylsulfonyl)phenyl]-5-(hydroxymethyl)quinazoline-2,4(1H,3H)-dione (4.0 g), triethylamine (3.6 ml) and DMF (50 ml), was added methanesulfonyl chloride (1.3 ml) at 0°C, and the reaction mixture was stirred at room temperature for 2 days. Then the reactant was partitioned into ethyl acetate and water. The organic layer was washed with water, and then purified with silica gel column chromatography (ethyl acetate/hexane) to obtain a solid (4.04 g).

To a mixture of the above-obtained solid (97 mg) and DMF (1.0 ml) was added a 2 N solution of dimethylamine in THF (0.5 ml) at room temperature, and the mixture was stirred for 24 hours. The reactant was partitioned into ethyl acetate and water. The organic layer was washed with water, and then purified with basic silica gel column chromatography (ethyl acetate/hexane) to give the desired product (71 mg) as crystals.

¹H-NMR (CDCl₃) δ 1.65-1.73 (2H, m), 2.33 (6H, s), 2.51 (2H, t), 3.80-3.84 (2H, m), 3.97 (2H, s), 6.88-6.91 (1H, m), 7.01-7.10 (2H, m), 7.16-7.22 (1H, m), 7.42-7.61 (6H, m), 7.77-7.80 (1H, m), 9.2-9.5 (1H, br s)

### Example 53

### (3-[3-(3,4-Dihydroquinolin-1(2H)-ylsulfonyl)phenyl]-2,4-dioxo-1,2,3,4-tetrahydroquinazolin-5-yl)acetonitrile

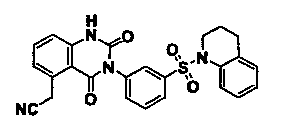

To a mixture of 3-[3-(3,4-dihydroquinolin-1(2H) - ylsulfonyl)phenyl]-5-(hydroxymethyl)quinazoline-2,4(1H,3H)-dione (4.0 g), triethylamine (3.6 ml) and DMF (50 ml), was added methanesulfonyl chloride (1.3 ml) at 0°C, and the mixture was stirred at room temperature for 2 days. Then, the reactant was partitioned into ethyl acetate and water. The organic layer was washed with water, and then purified with silica gel column chromatography (ethyl acetate/hexane) to obtain a solid (4.04 g).

To a mixture of the above-obtained solid (84 mg) and dimethyl sulfoxide (1.2 ml) was added potassium cyanide (24 mg) at room temperature, and the mixture was stirred for 18 hours. Then, the reactant was partitioned into ethyl acetate and water. The organic layer was washed with water, and then purified with silica gel column chromatography (ethyl acetate/hexane) to give the desired product (53 mg) as crystals.

¹H-NMR (DMSO-d₆) δ 1.60-1.69 (2H, m), 2.47-2.51 (2H, m), 3.74-3.78 (2H, m), 4.37 (2H, s), 7.07-7.29 (5H, m), 7.16-7.22 (1H, m), 7.56-7.74 (6H, m), 11.69 (1H, s)

### Example 54

### 3-[3-(3,4-Dihydroquinolin-1(2H)-ylsulfonyl)phenyl]-5-[(methylthio)methyl]quinazoline-2,4(1H,3H)-dione

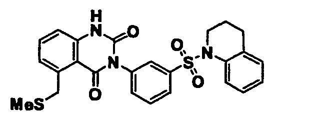

To a mixture of 3-[3-(3,4-dihydroquinolin-1(2H)-ylsulfonyl)phenyl]-5-(hydroxymethyl)quinazoline-2,4(1H,3H)-dione (4.0 g), triethylamine (3.6 ml) and DMF (50 ml), was added methanesulfonyl chloride (1.3 ml) at 0°C, and the reaction mixture was stirred at room temperature for 2 days. Then, the reactant was partitioned into ethyl acetate and water. The organic layer was washed with water, and then purified with silica gel column chromatography (ethyl acetate/hexane) to obtain a solid (4.04 g).

To a mixture of the above-obtained solid (200 mg) and DMF (2.0 ml) was added sodium thiomethylate (35 mg) at room temperature, and the mixture was stirred for 18 hours. The reactant was partitioned into ethyl acetate and water. The organic layer was washed with water, and then purified with silica gel column chromatography (ethyl acetate/hexane) to give the desired product (154 mg) as crystals.

¹H-NMR (DMSO-d₆) δ 1.60-1.69 (2H, m), 1.95 (3H, s), 2.47-2.51 (2H, m), 3.74-3.78 (2H, m), 4.15 (2H, s), 7.05-7.09 (3H, m), 7.15-7.20 (2H, m), 7.55-7.69 (6H, m), 11.58 (1H, s)

### Example 55

### 3-[3-(3,4-Dihydroquinolin-1(2H)-ylsulfonyl)phenyl]-5-[(methylsulfonyl)methyl]quinazoline-2,4(1H,3H)-dione

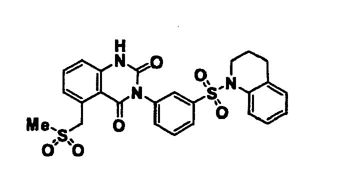

A mixture of 3- [3- (3, 4-dihydroquinolin-1 (2H) - ylsulfonyl)phenyl]-5-[(methylthio)methyl]quinazoline-2,4(1H,3H)-dione (65 mg), 3-chloroperbenzoic acid (70%, 75 mg) and DMF (1.0 ml) was stirred at room temperature for 60 hours. The reaction was stopped by adding a sodium sulfite solution, and the resultant was extracted with ethyl acetate. The organic layer was sequentially washed with sodium carbonate and water, dried, concentrated and purified with silica gel column chromatography (ethyl acetate/hexane) to give the desired product (48 mg) as crystals.

¹H-NMR (DMSO-d₆) δ 1.60-1.69 (2H, m), 2.46-2.51 (2H, m), 2.80 (3H, s), 3.74-3.78 (2H, m), 5.18 (2H, s), 7.08-7.31 (2H, m), 7.55-7.74 (6H, m), 11.74 (1H, s)

### Example 56

### 3-[3-(3,4-Dihydroquinolin-1(2H)-ylsulfonyl)phenyl]-2,4-dioxo-1,2,3,4-tetrahydroquinazoline-5-carbaldehyde oxime

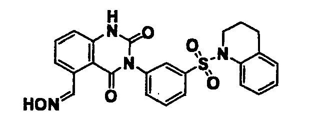

A mixture of 3-[3-(3,4-dihydroquinolin-1(2H)-ylsulfonyl)phenyl]-2,4-dioxo-1,2,3,4-tetrahydroquinazoline-5-carbaldehyde (50 mg), hydroxylamine hydrochloride (15 mg) and N-methylpyrrolidone (2 ml) was stirred at 80°C for 16 hours. The reactant was cooled to room temperature, and then partitioned into ethyl acetate and water. The organic layer was washed with water, and then purified with silica gel column chromatography (ethyl acetate/hexane) to give the desired product (32 mg) as crystals.

¹H-NMR (DMSO-d₆) δ 1.58-1.68 (2H, m), 2.47-2.50 (2H, m), 3.73-3.77 (2H, m), 7.07-7.27 (4H, m), 7.49-7.73 (7H, m), 9.06 (1H, s), 11.38 (1H, s), 11.64 (1H, br s)

### Example 57

### 3-[3-(3,4-Dihydroquinolin-1(2H)-ylsulfonyl)phenyl]-5-(1H-tetrazol-5-ylmethyl)quinazoline-2,4(1H,3H)-dione

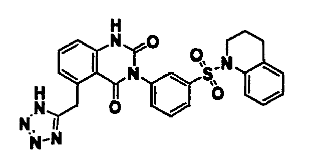

A mixture of {3-[3-(3,4-dihydroquinolin-1(2H)-ylsulfonyl)phenyl]-2,4-dioxo-1,2,3,4-tetrahydroquinazolin-5-yl}acetonitrile (120 mg), sodium azide (83 mg), ammonium chloride (68 mg) and DMF (3.0 ml) was stirred at 90°C for 2 days. Sodium azide (165 mg) and ammonium chloride (136 mg) were added thereto, and the mixture was stirred at 110°C for 24 hours. The reactant was cooled to room temperature, and then partitioned into ethyl acetate and water. The organic layer was washed with water, and then purified with silica gel column chromatography (ethyl acetate/methanol) to give the desired product (20 mg) as crystals.

¹H-NMR (DMSO-d₆) δ 1.54-1.62 (2H, m), 2.41-2.45 (2H, m), 3.70-3.74 (2H, m), 4.69 (2H, s), 7.05-7.25 (5H, m), 7.51-7.69 (6H, m), 11.66 (1H, s)

### Example 58

### 3-(3-{[2-(Hydroxymethyl)-2,3-dihydro-4H-1,4-benzoxazin-4-yl]sulfonyl}phenyl)quinazoline-2,4(1H,3H)-dione

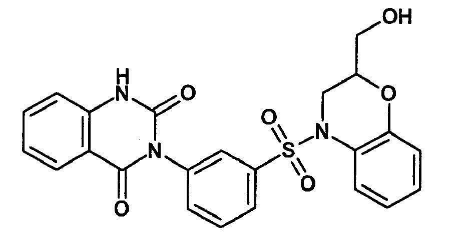

A solution of {4-[(3-aminophenyl)sulfonyl]-3,4-dihydro-2H-1,4-benzoxazin-2-yl}methanol (481 mg), methyl 2-isocyanatobenzoate (399 mg), DMAP (275 mg) and THF (9 ml) was stirred at room temperature for 2 hours. After shaking, a trisamine resin (Argonaut Technologies, Inc., 4.36 mmol/g, 516 mg) was added thereto, and the mixture was further shaken at room temperature for 15 hours. The resin was filtered off, and then washed with acetonitrile. The filtrate and a washing liquor were combined and concentrated under reduced pressure. The residue was suspended in methanol (10 ml), sodium methoxide (28% methanol solution) (347 mg) was added thereto, and the mixture was heated at 75°C for 15 minutes. The reaction mixture was poured into 1 N hydrochloric acid, and extracted with ethyl acetate. The organic layer was sequentially washed with a saturated sodium bicarbonate solution, water and saturated brine, and dried over anhydrous magnesium sulfate. Then, the solvent was distilled off under reduced pressure, and the residue was subjected to silica gel column chromatography. The fraction eluted with ethyl acetate/hexane (1 : 2 to 2 : 1) was concentrated under reduced pressure. The obtained crystals were collected by filtration to give the desired product (370 mg) .

¹H-NMR (DMSO-d₆) δ 3.13-3.39 (1H, m), 3.51-3.60 (3H, m), 4.30 (1H, d), 4.86 (1H, t), 6.86 (1H, d), 6.93 (1H, t), 7.09 (1H, t), 7.24 (1H, d), 7.25 (1H, t), 7.56 (1H, d), 7. 63-7. 75 (4H, m), 7.95 (1H, d), 7.98 (1H, s), 11.66 (1H, s)

### Examples 58 (1) to 58(2)

In the same manner as in Example 58, 4-[(3-aminophenyl)sulfonyl]-1-methyl-3,4-dihydroquinoxalin-2(1H)-one or 5-[(3-aminophenyl)sulfonyl]-1-methyl-1,3,4,5-tetrahydro-2H-1,5-benzodiazepin-2-one was reacted with methyl 2-isocyanatobenzoate to obtain the following compounds.

### Example 58(1)

### 3-{3-[(4-Methyl-3-oxo-3,4-dihydroquinoxalin-1(2H)-yl)sulfonyl]phenyl}quinazoline-2,4(1H,3H)-dione

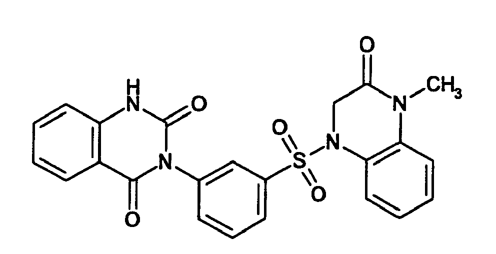

¹H-NMR (DMSO-d₆) δ 2.81 (3H, s), 4.40 (2H, s), 7.04 (1H, d), 7.10-7.26 (4H, m), 7.32 (1H, t), 7.51-7.53 (2H, m), 7.61-7.77 (3H, m), 7.90-7.98 (1H, m), 11.58 (1H, s)

### Example 58(2)

### 3-{3-[(5-Methyl-4-oxo-2,3,4,5-tetrahydro-1H-1,5-benzodiazepin-1-yl)sulfonyl]phenyl}quinazoline-2,4(1H,3H)-dione

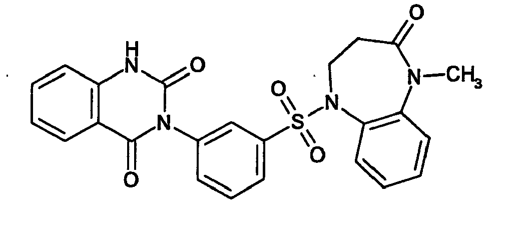

¹H-NMR (DMSO-d₆) δ 2.35-2.39 (2H, m), 2.75 (3H, s), 3.88-4.50 (2H, m), 7.19-7.38 (5H, m), 7.41-7.47 (1H, m), 7.56 (1H, s), 7.64-7.74 (4H, m), 7.92 (1H, d), 11.55 (1H, s)

### Example 59

### 3-{3-[(3-Methyl-2-oxo-2,3-dihydro-4H-1,4-benzoxazin-4-yl)sulfonyl]phenyl}quinazoline-2,4(1H,3H)-dione

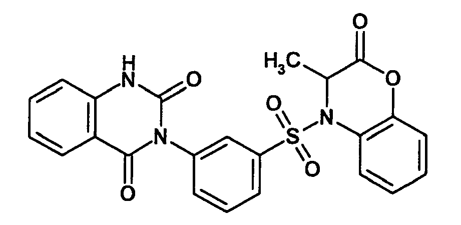

A solution of 4-[(3-aminophenyl)sulfonyl]-3-methyl-3,4-dihydro-2H-1,4-benzoxazin-2-one (285 mg), methyl 2-isocyanatobenzoate (238 mg), DMAP (164 mg) and THF (5.5 ml) was stirred at room temperature for 2 hours. After shaking, a trisamine resin (Argonaut Technologies, Inc., 4.36 mmol/g, 308 mg) was added thereto, the mixture was further shaken at room temperature for 15 hours. The resin was filtered off, and then washed with acetonitrile. The filtrate and a washing liquor were combined and concentrated under reduced pressure. The residue was suspended in methanol (6 ml), sodium methoxide (28% methanol solution) (207 mg) was added thereto, and the mixture was heated at 75°C for 10 minutes. The reaction mixture was poured into water, acidified with 2 N hydrochloric acid to be weakly acidic (pH 3) and extracted with ethyl acetate. The organic layer was sequentially washed with water and saturated brine, and dried over anhydrous magnesium sulfate. Then, the solvent was distilled off under reduced pressure. The residue was dissolved in toluene (20 ml), p-toluenesulfonic acid monohydrate (171 mg) was added thereto, and the reaction mixture was heated at 90°C for 1 hour. The reaction mixture was poured into a saturated sodium bicarbonate solution, and extracted with ethyl acetate. The organic layer was sequentially washed with water and saturated brine, and dried over anhydrous magnesium sulfate. Then, the solvent was distilled off under reduced pressure and the resulting crystals were collected by filtration to give the desired product (42 mg).

¹H-NMR (DMSO-d₆) δ 1.25 (3H, d), 5.04 (1H, q), 7.15-7.26 (3H, m), 7.30 (1H, t), 7.39 (1H, t), 7.58 (1H, s), 7.63 (1H, d), 7.68-7.74 (4H, m), 7.92 (1H, d), 11.59 (1H, s)

### Example 60

### 3-{3-[(1,1-Dioxide-2,3-dihydro-4H-1,4-benzothiazin-4-yl)sulfonyl]phenyl}quinazoline-2,4(1H,3H)-dione

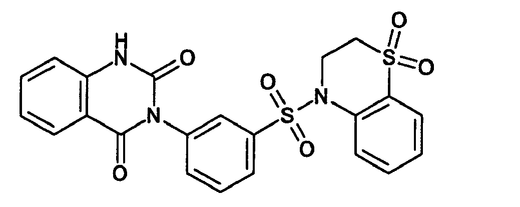

A mixture of 3-[3-(2,3-dihydro-4H-1,4-benzothiazin-4-ylsulfonyl)phenyl]quinazoline-2,4(1H,3H)-dione (300 mg), 3-chloroperbenzoic acid (252 mg) and dichloromethane (10 ml) was agitated at room temperature for 15 hours, and the reaction mixture was poured into a saturated sodium bicarbonate solution and extracted with ethyl acetate/THF (3 : 1). The organic layer was sequentially washed with water and saturated brine, and dried over anhydrous magnesium sulfate. Then, the solvent was distilled off under reduced pressure, and the residue was subjected to silica gel column chromatography. The fraction eluted with ethyl acetate was concentrated under reduced pressure, and the obtained crystals were collected by filtration to give the desired product (156 mg).

¹H-NMR (DMSO-d₆) δ 3.66 (2H, t), 4.47 (2H, t), 7.21-7.26 (2H, m), 7.42 (1H, ddd), 7.57-7.83 (7H, m), 7.94 (1H, dd), 8.13 (1H, s), 11.63 (1H, s)

### Example 60(1)

In the same manner as in Example 60, the following compound was obtained from 3-[2-chloro-5-(2,3-dihydro-4, 1-benzothiazepin-1(5H)-ylsulfonyl)phenyl]quinazoline-2,4(1H,3H)-dione.

### 3-{2-Chloro-5-[(4, 4-dioxide-2,3-dihydro-4, 1-benzothiazepin-1(5H)-yl)sulfonyl]phenyl}quinazoline-2,4(1H,3H)-dione

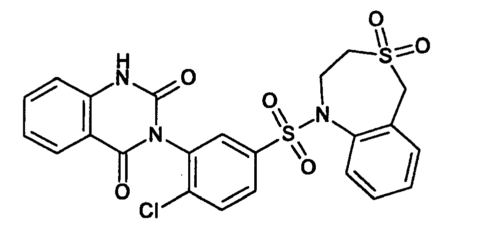

¹H-NMR (CDCl₃) δ 3.47-4.14 (6H, m), 7.03 (1H, d), 7.08-7.13 (1H, m), 7.26-7.38 (4H, m), 7.64 (1H, dt), 7.75 (1H, d), 7.79 (1H, d), 7.85 (1H, dd), 8.15 (1H, d), 9.35 (1H, s)

### Example 61

### 1-{[3-(2,4-Dioxo-1,4-dihydroquinazolin-3(2H)-yl)phenyl]sulfonyl}-1,2,3,4-tetrahydroquinoline-6-carboxylic acid

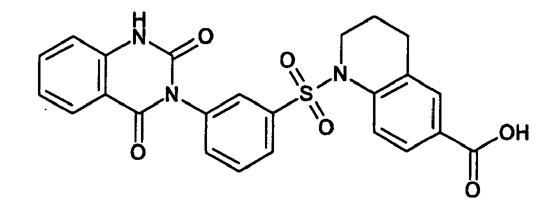

Methyl 1-{[3-(2,4-dioxo-1,4-dihydroquinazolin-3(2H)-yl)phenyl]sulfonyl}-1,2,3,4-tetrahydroquinoline-6-carboxylate (386 mg) was suspended in a mixed solution of methanol/THF (3 ml/2 ml). A 2 N aqueous sodium hydroxide solution (1.2 ml) was added thereto, and the mixture was agitated at 50°C for 50 minutes. The reaction solution was poured into water, and washed with diethyl ether. The aqueous layer was acidified with 2 N hydrochloric acid to be weakly acidic (pH 3), and then extracted with ethyl acetate. The ethyl acetate layer was sequentially washed with water and saturated brine, and dried over anhydrous magnesium sulfate. Then, the solvent was distilled off under reduced pressure, and the crystals were collected by filtration to give the desired product (288 mg).

¹H-NMR (DMSO-d₆) δ 1.71 (2H, quintet), 2.63 (2H, t), 3.83 (2H, t), 7.21-7.26 (2H, m), 7.66-7.74 (7H, m), 7.90 (1H, s), 7.94 (1H, d), 11.61 (1H, s), 12.84 (1H, s)

### Example 62

### 3-[3-(3,4-Dihydroquinolin-1(2H)-ylcarbonyl)phenyl]quinazoline-2,4(1H,3H)-dione

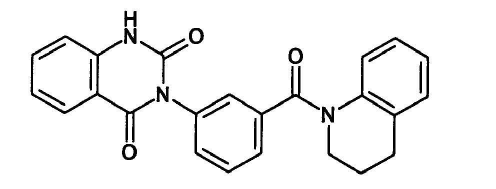

3-(2,4-Dioxo-1,4-dihydroquinazolin-3(2H)-yl)benzoic acid (100 mg) was suspended in THF (3 ml). Oxalyl chloride (67 mg) and DMF (6 µl) were added thereto, and the mixture was agitated at room temperature for 2 hours. The reaction solution was concentrated under reduced pressure, and the residue was suspended in THF (3 ml) and added to a solution of 1,2,3,4-tetrahydroquinoline (57 mg) and pyridine (34 mg) in THF (3 ml). After agitating at room temperature for 1 hour, the reaction solution was poured into water, and extracted with ethyl acetate. The organic layer was sequentially washed with 1 N hydrochloric acid, a saturated sodium bicarbonate solution, water and saturated brine, and dried over anhydrous magnesium sulfate. Then, the solvent was distilled off under reduced pressure, and the crystals were collected by filtration to give the desired product (97 mg).

¹H-NMR (DMSO-d₆) δ 1.95 (2H, quintet), 2.82 (2H, t), 3.75 (2H, t), 6.96-7.04 (3H, m), 7. 16-7.24 (3H, m), 7.32-7.46 (4H, m), 7.68 (1H, t), 7.93 (1H, dd), 11.54 (1H, s)

### Example 63

### Ethyl {3-[3-(3,4-dihydroquinolin-1(2H)-ylsulfonyl)phenyl]-2,4-dioxo-1,2,3,4-tetrahydroquinazolin-5-yl}acetate

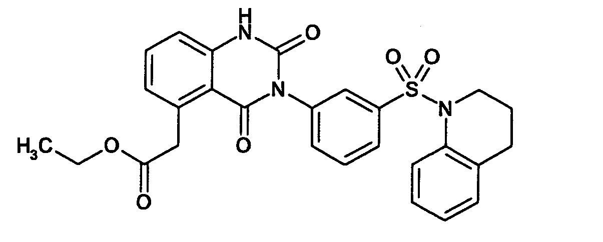

To a solution of methyl 2-amino-6-(2-ethoxy-2-oxoethyl)benzoate (1.1 g) and triethylamine (2.37 ml) in dichloromethane (15 ml), a solution of triphosgene (504 mg) in THF (5 ml) was added portionwise at 0°C, and then the mixture was stirred under nitrogen atmosphere for 1 hour. Insolubles were filtered off, and then the solvent was distilled off under reduced pressure. The resulting residue was dissolved in THF (15 ml), and then 3-(3,4-dihydro-1(2H)-quinolinylsulfonyl)aniline (1.34 g) and DMAP (1.59 g) were added thereto, and the mixture was stirred under nitrogen atmosphere at room temperature overnight. After adding ethyl acetate, the reaction mixture was washed with 1 N hydrochloric acid and an aqueous saturated sodium hydrogen carbonate solution, and dried over magnesium sulfate. The solvent was distilled off under reduced pressure. The obtained solid was washed with ethyl acetate to give the desired product (1.93 g) as crystals.

¹H-NMR (DMSO-d₆) δ 1.10 (3H, t), 1.54-1.69 (2H, m), 2.43-2.52 (2H, m), 3.71-3.78 (2H, m), 3.94-4.06 (4H, m), 7.04-7.10 (3H, m), 7.14-7.20 (2H, m), 7.50-7.66 (6H, m), 11.61 (1H, s)

### Examples 63(1) to 63(2)

In the same manner as in Example 63, the following compounds were obtained from reacting methyl 2-amino-6-(2-ethoxy-1-methyl-2-oxoethyl)benzoate with 3-(3,4-dihydro-1(2H)-quinolinylsulfonyl)aniline, or reacting methyl 2-amino-6-(2-ethoxy-2-oxoethyl)benzoate with 2-chloro-5-(2,3,4,5-tetrahydro-1H-1-benzazepin-1-ylsulfonyl)thiophen-3-amine.

### Example 63(1)

### Ethyl 2-{3-[3-(3,4-dihydroquinolin-1(2H)-ylsulfonyl)phenyl]-2,4-dioxo-1,2,3,4-tetrahydroquinazolin-5-yl}propanoate

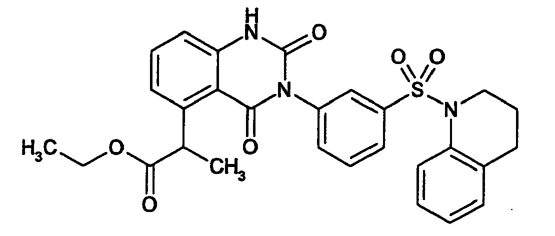

¹H-NMR (DMSO-d₆) δ 1.06 (3H, t), 1.39 (3H, d), 1.57-1.69 (2H, m), 2.45-2.53 (2H, m), 3.72-3.78 (2H, m), 3.99 (2H, q), 4.69-4.91 (1H, m), 7.02-7.11 (3H, m), 7.13-7.21 (2H, m), 7.51-7.69 (6H, m), 11.60 (1H, s)

### Example 63(2)

### Ethyl {3-[2-chloro-5-(2,3,4,5-tetrahydro-1H-1-benzazepin-1-ylsulfonyl)-3-thienyl]-2,4-dioxo-1,2,3,4-tetrahydroquinazolin-5-yl}acetate

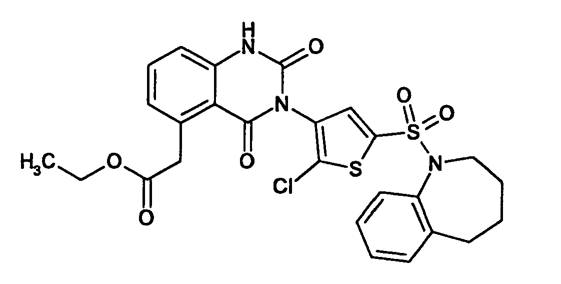

¹H-NMR (DMSO-d₆) δ 1. 12 (3H, t), 1.48-1.63 (2H, m), 1.77-1.88 (2H, m), 2.53-2.61 (2H, m), 3.53-3.73 (2H, m), 3.93-4.15 (4H, m), 7.12 (1H, d), 7.18-7.31 (5H, m), 7.65 (1H, t), 7.77 (1H, s), 11.76 (1H, s)

### Example 64

### {3-[3-(3,4-Dihydroquinolin-1(2H)-ylsulfonyl)phenyl]-2,4-dioxo-1,2,3,4-tetrahydroquinazolin-5-yl}acetic acid

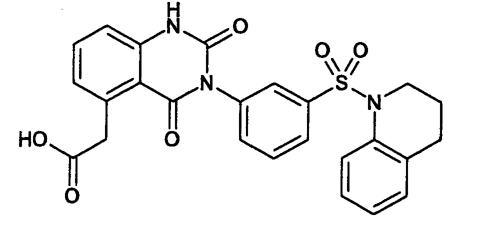

To a solution of ethyl {3-[3-(3,4-dihydroquinolin-1(2H)-ylsulfonyl)phenyl]-2,4-dioxo-1,2,3,4-tetrahydroquinazolin-5-yl}acetate (1.0 g) in ethanol (50 ml) and THF (50 ml), was added a 1 N aqueous sodium hydroxide solution (5.78 ml), and the mixture was stirred at 60°C for 4 hours. The solvent was distilled off under reduced pressure, and then the residue was diluted with water at 0°C. 1 N Hydrochloric acid (8 ml) was added thereto, and the reaction mixture was acidified, and extracted with ethyl acetate. The organic layer was washed with saturated brine and dried over magnesium sulfate. The solvent was distilled off under reduced pressure, and the resulting residue was crystallized from diisopropyl ether to give the desired product (898 mg) as crystals.

¹H-NMR (DMSO-d₆) δ 1.56-1.68 (2H, m), 2.43-2.53 (2H, m), 3.72-3.80 (2H, m), 3.97 (2H, s), 7.04-7.11 (3H, m), 7.12-7.22 (2H, m), 7.53-7.66 (6H, m), 11.58 (1H, s), 12.03 (1H, s)

### Examples 64(1) to 64(2)

In the same manner as in Example 64, the following compounds were obtained from ethyl 2-{3-[3-(3,4-dihydroquinolin-1(2H)-ylsulfonyl)phenyl]-2,4-dioxo-1,2,3,4-tetrahydroquinazolin-5-yl}propanoate or ethyl {3-[2-chloro-5-(2,3,4,5-tetrahydro-1H-1-benzazepin-1-ylsulfonyl)-3-thienyl]-2,4-dioxo-1,2,3,4-tetrahydroquinazolin-5-yl}acetate.

### Example 64(1)

### 2-{3-[3-(3,4-Dihydroquinolin-1(2H)-ylsulfonyl)phenyl]-2,4-dioxo-1,2,3,4-tetrahydroquinazolin-5-yl}propanoic acid

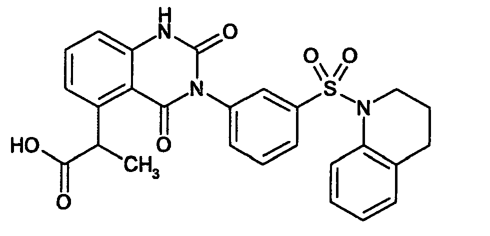

¹H-NMR (DMSO-d₆) δ 1.38 (3H, d), 1.57-1.69 (2H, m), 2.44-2.54 (2H, m), 3.72-3.80 (2H, m), 4.71-5.01 (1H, m), 7.02-7.21 (5H, m), 7.54-7.67 (6H, m), 11.57 (1H, s), 12.09 (1H, br s)

### Example 64(2)

### {3-[2-Chloro-5-(2,3,4,5-tetrahydro-1H-1-benzazepin-1-ylsulfonyl)-3-thienyl]-2,4-dioxo-1,2,3,4-tetrahydroquinazolin-5-yl}acetic acid

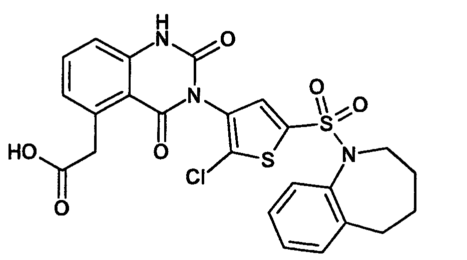

¹H-NMR (DMSO-d₆) δ 1.51-1.64 (2H, m), 1.76-1.88 (2H, m), 2.54-2.66 (2H, m), 3.54-3.71 (2H, m), 3.93 (1H, d), 4.07 (1H, d), 7.10 (1H, d), 7. 16-7.32 (5H, m), 7.64 (1H, t), 7. 76 (1H, s), 11.74 (1H, s), 12.10 (1H, br s)

### Example 65

### 2-{3-[3-(3,4-Dihydroquinolin-1(2H)-ylsulfonyl)phenyl]-2,4-dioxo-1,2,3,4-tetrahydroquinazolin-5-yl}acetamide

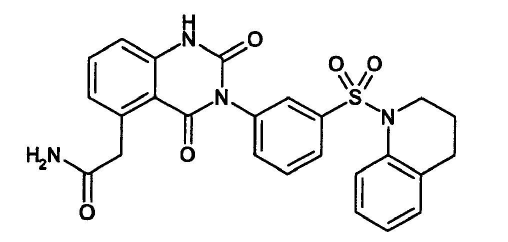

To a solution of {3-[3-(3,4-dihydroquinolin-1(2H)-ylsulfonyl)phenyl]-2,4-dioxo-1,2,3,4-tetrahydroquinazolin-5-yl}acetic acid (100 mg), triethylamine (0.071 ml), 1-hydroxybenzotriazole (34.3 mg) and ammonium chloride (13.6 mg) in DMF (5 ml), 1-ethyl-3-(3-dimethylaminopropyl)carbodiimide hydrochloride (48.7 mg) and DMAP (2 mg) were added, and the mixture was stirred at room temperature overnight. Water was added thereto, and the reaction mixture was extracted twice with ethyl acetate. Then, the organic layer was washed with saturated brine, and dried over magnesium sulfate. The resulting residue was recrystallized from diisopropyl ether/ethyl acetate to give the desired product (41.3 mg) as crystals.

¹H-NMR (DMSO-d₆) δ 1.58-1.68 (2H, m), 2.43-2.55 (2H, m), 3.72-3.79 (2H, m), 3.92 (2H, s), 6.73 (1H, s), 6.98-7.22 (6H, m), 7.52-7.66 (6H, m), 11.54 (1H, s)

### Examples 65(1) to 65(2)

In the same manner as in Example 65, the following compounds were obtained from 2-{3-[3-(3,4-dihydroquinolin-1(2H)-ylsulfonyl)phenyl]-2,4-dioxo-1,2,3,4-tetrahydroquinazolin-5-yl}propanoic acid or {3-[2-chloro-5-(2,3,4,5-tetrahydro-1H-1-benzazepin-1-ylsulfonyl)-3-thienyl]-2,4-dioxo-1,2,3,4-tetrahydroquinazolin-5-yl}acetic acid.

### Example 65(1)

### 2-{3-[2-Chloro-5-(2,3,4,5-tetrahydro-1H-1-benzazepin-1-ylsulfonyl)-3-thienyl]-2,4-dioxo-1,2,3,4-tetrahydroquinazolin-5-yl}acetamide

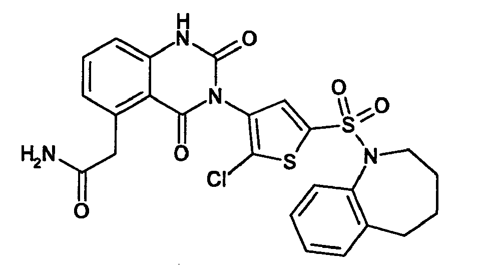

¹H-NMR (DMSO-d₆) δ 1. 51-1. 65 (2H, m), 1. 78-1. 90 (2H, m), 2.56-2.65 (2H, m), 3.54-3.73 (2H, m), 3.89 (1H, d), 3.99 (1H, d), 6.78 (1H, s), 7.05 (1H, d), 7.13-7.33 (6H, m), 7.61 (1H, t), 7.76 (1H, s), 11.69 (1H, s)

### Example 65(2)

### 2-{3-[3-(3,4-Dihydroquinolin-1(2H)-ylsulfonyl)phenyl]-2,4-dioxo-1,2,3,4-tetrahydroquinazolin-5-yl}propanamide

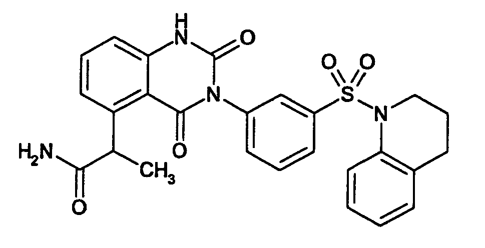

¹H-NMR (DMSO-d₆) δ 1.29 (3H, d), 1.59-1.69 (2H, m), 2.45-2.53 (2H, m), 3.72-3.80 (2H, m), 4.97-5.09 (1H, m), 6.81 (1H, s), 6.98 (1H, s), 7.04-7.22 (5H, m), 7.53-7.71 (6H, m), 11.56 (1H, s)

### Example 66

### 3-[3-(3,4-Dihydroquinolin-1(2H)-ylsulfonyl)phenyl]-5-(2-hydroxyethyl)quinazoline-2,4(1H,3H)-dione

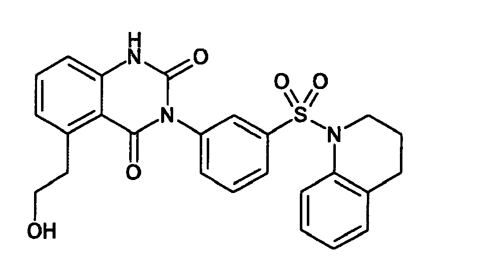

To a suspension of ethyl {3-[3-(3,4-dihydroquinolin-1(2H)-ylsulfonyl)phenyl]-2,4-dioxo-1,2,3,4-tetrahydroquinazolin-5-yl}acetate (150 mg) in THF (15 ml), was added a solution of lithium borohydride (37.8 mg) in THF (4 ml), and the mixture was stirred under nitrogen atmosphere at 50°C for 6 hours. After adding water and saturated brine, the mixture was extracted with ethyl acetate. The organic layer was washed with saturated brine and dried over magnesium sulfate. The solvent was distilled off under reduced pressure, and the resulting residue was purified with a preparative HPLC. The obtained solid was washed with diisopropyl ether to give the desired product (5.7 mg) as crystals.

¹H-NMR (DMSO-d₆) δ 1.58-1.69 (2H, m), 2.41-2.53 (2H, m), 3.20-3.29 (2H, m), 3.52-3.61 (2H, m), 3. 73-3.79 (2H, m), 4.53 (1H, t), 6.99-7.22 (5H, m), 7.51-7.69 (6H, m), 11.47 (1H, s)

### Example 67

### 3- [3- (3,4-Dihydroquinolin-1 (2H) - ylsulfonyl)phenyl]pyrido[3,2-d]pyrimidine-2,4(1H,3H)-dione

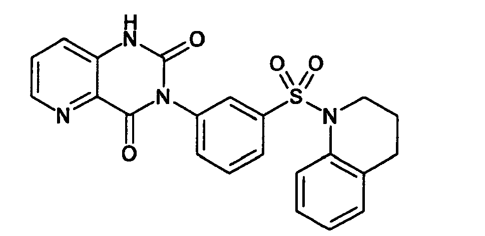

To a solution of 2-(ethoxycarbonyl)nicotinic acid (1.4 g) and triethylamine (1.45 ml) in THF (40 ml), diphenylphosphoryl azide (2.01 ml) was added, and then the mixture was stirred under nitrogen atmosphere at room temperature for 1 hour. The mixture was further stirred at 85°C for 3 hours, and then cooled to 0°C. 3-(3,4-Dihydro-1(2H)-quinolinylsulfonyl)aniline (2.28 g) and DMAP (2.54 g) were added thereto, and the mixture was stirred under nitrogen atmosphere at room temperature overnight. Water was added thereto, and the mixture was extracted with ethyl acetate, washed with an aqueous saturated sodium hydrogen carbonate solution and saturated brine, and dried over magnesium sulfate. The solvent was distilled off under reduced pressure, and then the obtained solid was washed with ethyl acetate to give the desired product (1.98 g) as crystals.

¹H-NMR (DMSO-d₆) δ 1.61-1.72 (2H, m), 2.45-2.53 (2H, m), 3.73-3.81 (2H, m), 7.06-7.12 (2H, m), 7.14-7.22 (1H, m), 7.49-7.73 (6H, m), 7.81-7.86 (1H, m), 8.51 (1H, dd), 11.67 (1H, s)

### Example 68

### 3-[3-(3,4-Dihydroquinolin-1(2H)-ylsulfonyl)phenyl]pyrido[3,2-d]pyrimidine-2,4(1H,3H)-dione 5-oxide

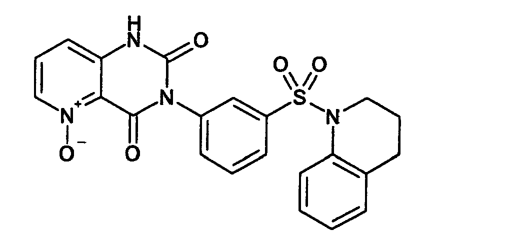

To a solution of 3-[3-(3,4-dihydroquinolin-1(2H)-ylsulfonyl) phenyl] pyrido [3,2-d] pyrimidine-2,4 (1H,3H)-dione (200 mg) in DMF (10 ml), 3-chloroperbenzoic acid (70%, 1.17 g) was added, and then the mixture was stirred at room temperature for 1 week. An aqueous sodium thiosulfate solution was added thereto at 0°C, and then extracted with ethyl acetate. The organic layer was washed with an aqueous saturated sodium hydrogen carbonate solution and saturated brine, and dried over magnesium sulfate. The solvent was distilled off under reduced pressure, and then the residue was crystallized from ethyl acetate/diisopropyl ether to give the desired product (103 mg) as crystals.

¹H-NMR (DMSO-d₆) δ 1. 59-1. 71 (2H, m), 2.44-2.53 (2H, m), 3.71-3.80 (2H, m), 7.03 (1H, dd), 7.06-7.11 (2H, m), 7.14-7.22 (1H, m), 7.44-7.67 (5H, m), 7.78-7. 82 (1H, m), 8.06 (1H, dd), 11.81 (1H, s)

### Example 69

### 3-[3-(6,7,8,9-Tetrahydro-5H-benzo[7]annulen-5-ylsulfinyl)phenyl]quinazoline-2,4(1H,3H)-dione

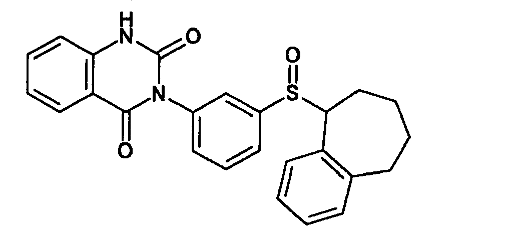

To a solution of 3-[3-(6,7,8,9-tetrahydro-5H-benzo[7]annulen-5-ylthio)phenyl]quinazoline-2,4(1H,3H)-dione (75 mg) in DMF (10 ml), 3-chloroperbenzoic acid (70%, 59 mg) was added, and then the mixture was stirred at room temperature overnight. An aqueous sodium thiosulfate solution was added thereto at 0°C, and then the mixture was extracted with ethyl acetate. The organic layer was washed with an aqueous saturated sodium hydrogen carbonate solution and saturated brine, and dried over magnesium sulfate. The solvent was distilled off under reduced pressure. Then, the resulting residue was purified with silica gel column chromatography (hexane : ethyl acetate = 1 : 1 to ethyl acetate), and crystallized from diisopropyl ether to give the desired product (27 mg) as crystals.
MS (ESI+, m/e) 431 (M+1)

### Example 69 (1)

In the same manner as in Example 69, the following compound was obtained from 3-{3-[(1-phenylethyl)thio]phenyl}quinazoline-2,4(1H,3H)-dione.

### 3-{3-[(1-Phenylethyl)sulfinyl]phenyl}quinazoline-2,4(1H,3H)-dione

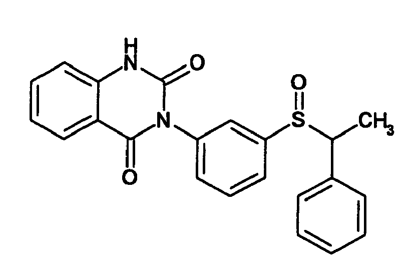

MS (ESI+, m/e) 391 (M+1)

### Example 70

### 3-[3-(6,7,8,9-Tetrahydro-5H-benzo[7]annulen-5-ylsulfonyl)phenyl]quinazoline-2,4(1H,3H)-dione

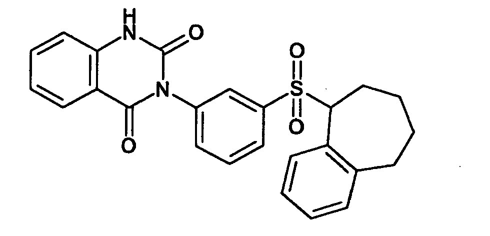

To a solution of 3-[3-(6,7,8,9-tetrahydro-5H-benzo[7]annulen-5-ylthio)phenyl]quinazoline-2,4(1H,3H)-dione (75 mg) in DMF (10 ml), 3-chloroperbenzoic acid (70%, 178 mg) was added, and then the mixture was stirred at room temperature overnight. An aqueous sodium thiosulfate solution was added thereto at 0°C, and then the mixture was extracted with ethyl acetate. The organic layer was washed with an aqueous saturated sodium hydrogen carbonate solution and saturated brine, and dried over magnesium sulfate. The solvent was distilled off under reduced pressure, and then the resulting residue was purified with silica gel column chromatography (hexane : ethyl acetate = 7 : 3 to hexane : ethyl acetate = 1 : 1), and crystallized from diisopropyl ether to give the desired product (43 mg) as crystals.

¹H-NMR (DMSO-d₆) δ 1.32-1.65 (2H, m), 1.75-2.10 (3H, m), 2.14-2.32 (1H, m), 2.51-2.66 (1H, m), 3.15-3.39 (1H, m), 4.85 (1H, t), 6.97-7.08 (2H, m), 7.12-7.30 (4H, m), 7.64-7.76 (4H, m), 7.89-7.92 (1H, m), 7.97 (1H, dd), 11.63 (1H, s)

### Example 70(1)

In the same manner as in Example 70, the following compound was obtained from 3-{3-[(1-phenylethyl)thio]phenyl}quinazoline-2,4(1H,3H)-dione.

### 3-{3-[(1-Phenylethyl)sulfonyl]phenyl}quinazoline-2, 4 (1H, 3H) -dione

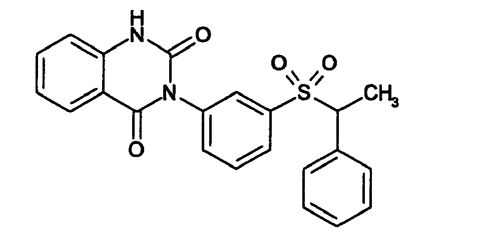

¹H-NMR (DMSO-d₆) δ 1.58 (3H, d), 4.72 (1H, q), 7.21-7.35 (7H, m), 7.57-7.75 (4H, m), 7.82-7.86 (1H, m), 7.97 (1H, dd), 11.63 (1H, s)

### Example 71

### N-Ethyl-3-[5-(hydroxymethyl)-2,4-dioxo-1,4-dihydroquinazolin-3(2H)-yl]-N-phenylbenzamide

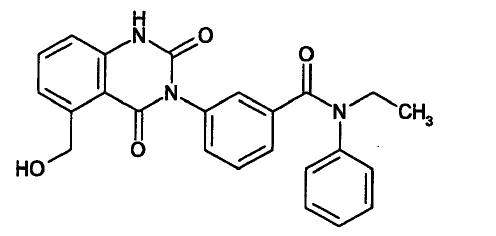

To a solution of 3-[5-(hydroxymethyl)-2,4-dioxo-1,4-dihydroquinazolin-3(2H)-yl]benzoic acid (150 mg), N-ethylaniline (87.2 mg) and N-methylimidazole (59.1 mg) in DMF (10 ml), was added 1-ethyl-3-(3-dimethylaminopropyl)carbodiimide hydrochloride (138 mg), and the mixture was stirred under nitrogen atmosphere at room temperature overnight. Water was added thereto, and the mixture was extracted with ethyl acetate. The organic layer was washed with saturated brine and dried over magnesium sulfate. The solvent was distilled off under reduced pressure, and then the resulting residue was purified with silica gel column chromatography (hexane : ethyl acetate = 1 : 1 to ethyl acetate), and crystallized from diisopropyl ether to give the desired product (66 mg) as crystals.

¹H-NMR (DMSO-d₆) δ 1.09 (3H, t), 3.85 (2H, q), 4.91 (2H, d), 5.22 (1H, t), 7.08-7.35 (10H, m), 7.46 (1H, d), 7.65 (1H, t), 11.46 (1H, s)

### Examples 71 (1) to 71(2)

In the same manner as in Example 71, the following compounds were obtained from reacting 3-[5-(hydroxymethyl)-2,4-dioxo-1,4-dihydroquinazolin-3(2H)-yl]benzoic acid with N-(2-methoxyethyl)aniline, or reacting 4-[5-({[tert-butyl(dimethyl)silyl]oxy}methyl)-2,4-dioxo-1,4-dihydroquinazolin-3(2H)-yl]-5-chlorothiophene-2-carboxylic acid with 2-(methylamino)-1-phenylethanol.

### Example 71 (1)

### 3-[5-(Hydroxymethyl)-2,4-dioxo-1,4-dihydroquinazolin-3(2H)-yl]-N-(2-methoxyethyl)-N-phenylbenzamide

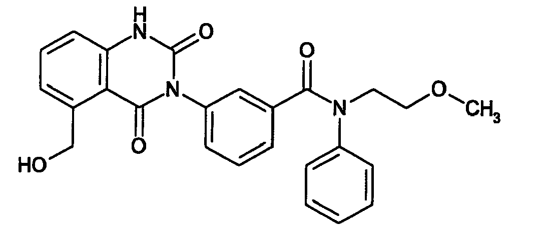

¹H-NMR (DMSO-d₆) δ 3.22 (3H, s), 3.48 (2H, t), 3.98 (2H, t), 4.91 (2H, d), 5.22 (1H, t), 7.08-7.35 (10H, m), 7.46 (1H, d), 7.65 (1H, t), 11.46 (1H, s)

### Example 71 (2)

### 4-[5-({[tert-Butyl(dimethyl)silyl]oxy}methyl)-2,4-dioxo-1,4-dihydroquinazolin-3(2H)-yl]-5-chloro-N-(2-hydroxy-2-phenylethyl)-N-methylthiophene-2-carboxamide

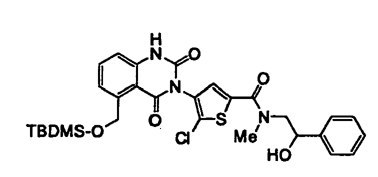

¹H-NMR (CDCl₃) δ 0.12 (6H, s), 0.97 (9H, s), 3.21 (3H, s), 3.62-3.93 (2H, m), 5.08 (1H, br s), 5.26 (2H, d), 6.98 (1H, d), 7.24-7.46 (6H, m), 7.60-7.73 (2H, m), 9.88 (1H, br s)

### Example 72

### 5-Chloro-N-cyclohexyl-4-(5-formyl-2,4-dioxo-1,4-dihydroquinazolin-3(2H)-yl)-N-methylthiophene-2-carboxamide

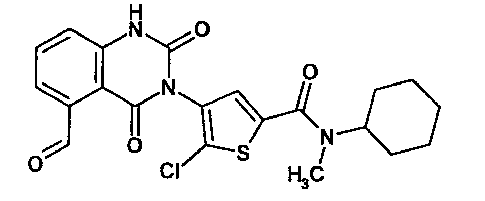

To a solution of 5-chloro-4-(5-formyl-2,4-dioxo-1,4-dihydroquinazolin-3(2H)-yl)thiophene-2-carboxylic acid (200 mg) in THF (10 ml) was added one drop of DMF, and then oxalyl chloride (0.06 ml) was added thereto. The mixture was stirred at room temperature for 30 minutes, and then the mixture was added portionwise to a solution of N-methylcyclohexanamine (84 mg) and pyridine (0.23 ml) in THF (10 ml) at 0°C, and the mixture was then stirred under nitrogen atmosphere at room temperature overnight. Then, water was added thereto, and the mixture was extracted with ethyl acetate. The organic layer was washed with saturated brine, and dried over magnesium sulfate. The solvent was filtered off under reduced pressure, and then the resulting residue was purified with silica gel column chromatography (hexane : ethyl acetate = 2 : 1 to 1 : 4) and recrystallized from THF/diisopropyl ether to give the desired product (57 mg) as crystals.

¹H-NMR (DMSO-d₆) δ 1.06-1.19 (1H, m), 1.21-1.39 (2H, m), 1.47-1.84 (7H, m), 2.99 (3H, br s), 4.03-4.26 (1H, m), 7.44 (1H, dd), 7.50 (1H, dd), 7.54 (1H, s), 7.86 (1H, t), 10.78 (1H, s), 12.04 (1H, s)

### Examples 72(1) to 72(12)

In the same manner as in Example 72, the following compounds were obtained from 5-chloro-4-(5-formyl-2,4-dioxo-1,4-dihydroquinazolin-3(2H)-yl)thiophene-2-carboxylic acid and the corresponding commercially available amine.

### Example 72 (1)

### 5-Chloro-N-(2-chlorophenyl)-4-(5-formyl-2,4-dioxo-1,4-dihydroquinazolin-3(2H)-yl)-N-methylthiophene-2-carboxamide

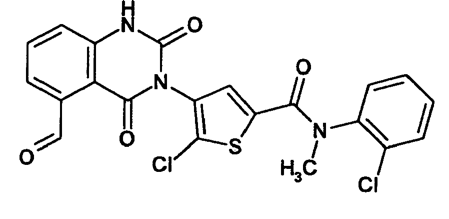

¹H-NMR (DMSO-d₆) δ 3.28 (3H, s), 7.08 (1H, s), 7.41 (1H, d), 7.45 (1H, d), 7.48-7.60 (2H, m), 7.64-7.75 (2H, m), 7.82 (1H, t), 10.72 (1H, s), 11.96 (1H, s)

### Example 72(2)

### 5-Chloro-N-(3-chlorophenyl)-4-(5-formyl-2,4-dioxo-1,4-dihydroquinazolin-3(2H)-yl)-N-methylthiophene-2-carboxamide

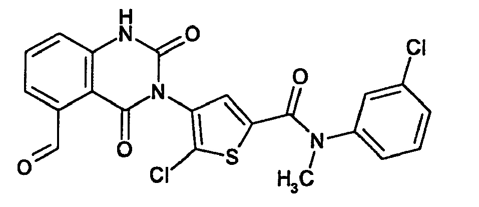

¹H-NMR (DMSO-d₆) δ 3.35 (3H, s), 6.94 (1H, s), 7.37-7.55 (5H, m), 7.64-7.68 (1H, m), 7.82 (1H, t), 10.73 (1H, s), 11.96 (1H, s)

### Example 72(3)

### 5-Chloro-N-(4-chlorophenyl)-4-(5-formyl-2,4-dioxo-1,4-dihydroquinazolin-3(2H)-yl)-N-methylthiophene-2-carboxamide

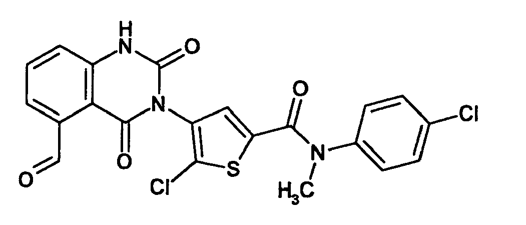

¹H-NMR (DMSO-d₆) δ 3.33 (3H, s), 6.95 (1H, s), 7.38-7.59 (6H, m), 7.82 (1H, t), 10.73 (1H, s), 11.96 (1H, s)

### Example 72(4)

### 5-Chloro-4-(5-formyl-2,4-dioxo-1,4-dihydroquinazolin-3(2H)-yl)-N-methyl-N-(2-methylphenyl)thiophene-2-carboxamide

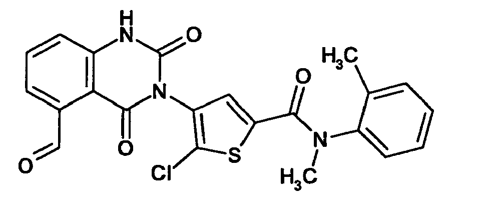

¹H-NMR (DMSO-d₆) δ 2.20 (3H, d), 3.27 (3H, s), 7. 15 (1H, d), 7.32-7.48 (6H, m), 7.82 (1H, t), 10.72 (1H, s), 11.96 (1H, s)

### Example 72(5)

### 5-Chloro-4-(5-formyl-2,4-dioxo-1,4-dihydroquinazolin-3(2H)-yl)-N-methyl-N-(3-methylphenyl)thiophene-2-carboxamide

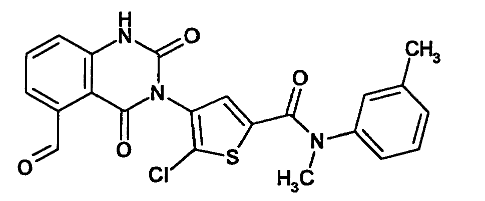

¹H-NMR (DMSO-d₆) δ 2.34 (3H, s), 3.33 (3H, s), 6.93 (1H, s), 7.20-7.31 (3H, m), 7.35-7.47 (3H, m), 7.82 (1H, t), 10.72 (1H, s), 11.96 (1H, s)

### Example 72(6)

### 5-Chloro-4-(5-formyl-2,4-dioxo-1,4-dihydroquinazolin-3(2H)-yl)-N-methyl-N-(4-methylphenyl)thiophene-2-carboxamide

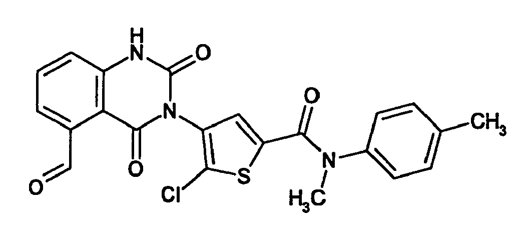

¹H-NMR (DMSO-d₆) δ 2.36 (3H, s), 3.31 (3H, s), 7.08 (1H, s), 7.28-7.36 (4H, m), 7.41 (1H, dd), 7.45 (1H, dd), 7. 82 (1H, t), 10.72 (1H, s), 11.95 (1H, s)

### Example 72(7)

### 5-Chloro-4-(5-formyl-2,4-dioxo-1,4-dihydroquinazolin-3(2H)-yl)-N-methyl-N-pyridin-2-ylthiophene-2-carboxamide

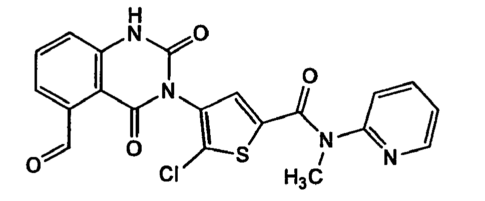

¹H-NMR (DMSO-d₆) δ 3.41 (3H, s), 6.83 (1H, s), 7.36-7.54 (4H, m), 7.83 (1H, t, J=7.8 Hz), 7.91 (1H, td, J=7.8, 2.0 Hz), 8.51 (1H, dd, J=4.9, 1.1 Hz), 10.72 (1H, s), 11.94 (1H, s)

### Example 72(8)

### 5-Chloro-N-(2-fluorophenyl)-4-(5-formyl-2,4-dioxo-1,4-dihydroquinazolin-3(2H)-yl)-N-methylthiophene-2-carboxamide

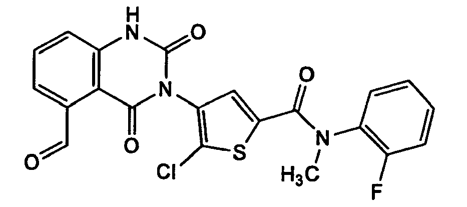

¹H-NMR (DMSO-d₆) δ 3.32 (3H, s), 7.07 (1H, s), 7.31-7.48 (4H, m), 7.49-7.58 (1H, m), 7.60-7.68 (1H, m), 7.82 (1H, t), 10.72 (1H, s), 11.96 (1H, s)

### Example 72(9)

### 5-Chloro-4-(5-formyl-2,4-dioxo-1,4-dihydroquinazolin-3(2H)-yl)-N-methyl-N-[2-(trifluoromethyl)phenyl]thiophene-2-

carboxamide

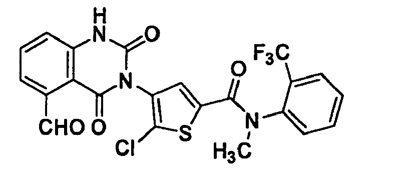

¹H-NMR (DMSO-d₆) δ 3.30 (3H, s), 6.84 (1H, d), 7.35-7.46 (2H, m), 7.73-7.98 (5H, m), 10.71 (1H, s), 11.95 (1H, br s)

### Example 72 (10)

### 5-Chloro-4-(5-formyl-2,4-dioxo-1,4-dihydroquinazolin-3(2H)-yl)-N-methyl-N-[2-(trifluoromethoxy)phenyl]thiophene-2-carboxamide

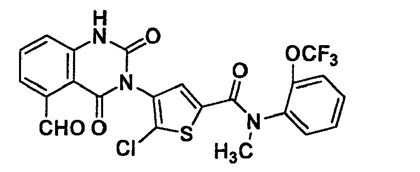

¹H-NMR (DMSO-d₆) δ 3.31 (3H, s), 6.95 (1H, s), 7.38-7.47 (2H, m), 7.50-7.65 (3H, m), 7.71-7.87 (2H, m), 10.72 (1H, s), 11.95 (1H, s)

### Example 72(11)

### 5-Chloro-4-(5-formyl-2,4-dioxo-1,4-dihydroquinazolin-3(2H)-yl)-N-(2-methoxyphenyl)-N-methylthiophene-2-carboxamide

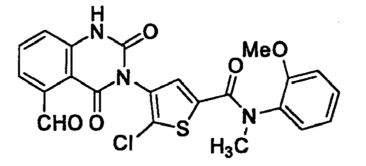

¹H-NMR (DMSO-d₆) δ 3.23 (3H, s), 3.78 (3H, s), 7.05-7.25 (3H, m), 7.38-7.53 (4H, m), 7.82 (1H, t), 10.72 (1H, s), 11.96 (1H, br s)

### Example 72(12)

### 5-Chloro-N-(3-fluorophenyl)-4-(5-formyl-2,4-dioxo-1,4-dihydroquinazolin-3(2H)-yl)-N-methylthiophene-2-carboxamide

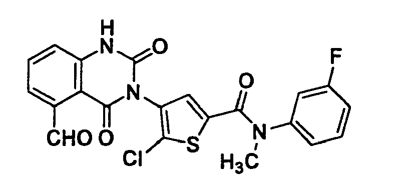

¹H-NMR (DMSO-d₆) δ 3.35 (3H, s), 6.89 (1H, s), 7.25-7.33 (2H, m), 7.37-7.56 (4H, m), 7.82 (1H, t), 10.72 (1H, s), 11.95 (1H, br s)

### Example 73

### 5-Chloro-N-cyclohexyl-4-[5-(hydroxymethyl)-2,4-dioxo-1,4-dihydroquinazolin-3(2H)-yl]-N-methylthiophene-2-carboxamide

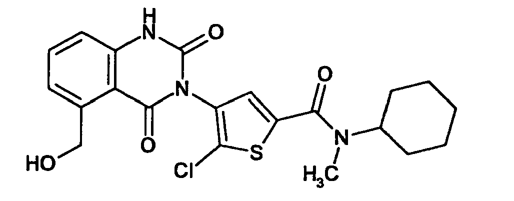

To a solution of 5-chloro-N-cyclohexyl-4-(5-formyl-2,4-dioxo-1,4-dihydroquinazolin-3(2H)-yl)-*N*-methylthiophene-2-carboxamide (45 mg) in THF (5 ml), sodium borohydride (6.2 mg) was added at 0°C, and then the mixture was stirred under nitrogen atmosphere at 0°C for 30 minutes. Water was added thereto, and the mixture was extracted with ethyl acetate. The organic layer was washed with saturated brine and dried over magnesium sulfate. The solvent was filtered off under reduced pressure, and then the resulting residue was purified with silica gel column chromatography (hexane : ethyl acetate = 1 : 1 to ethyl acetate), and recrystallized from THF/diisopropyl ether to give the desired product (32 mg) as crystals.

¹H-NMR (DMSO-d₆) δ 1.06-1.41 (3H, m), 1.45-1.84 (7H, m), 2.99 (3H, s), 4.00-4.26 (1H, m), 4.95 (2H, d), 5.29 (1H, t), 7.14 (1H, d), 7.44-7.60 (2H, m), 7.70 (1H, t), 11.68 (1H, s)

### Examples 73 (1) to 73(12)

In the same manner as in Example 73, the following compounds were obtained from the compounds disclosed in Examples 72 (1) to 72 (12) .

### Example 73 (1)

### 5-Chloro-N-(2-chlorophenyl)-4-[5-(hydroxymethyl)-2,4-dioxo-1,4-dihydroquinazolin-3(2A)-yl]-N-methylthiophene-2-carboxamide

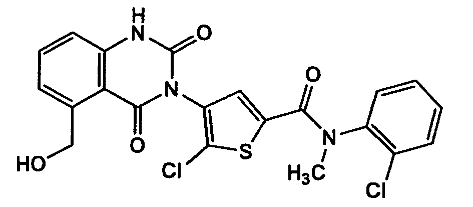

¹H-NMR (DMSO-d₆) δ 3.28 (3H, s), 4.89 (2H, d), 5.26 (1H, t), 7.02 (1H, s), 7.09 (1H, d), 7.45-7.59 (3H, m), 7.63-7.73 (3H, m), 11.61 (1H, s)

### Example 73(2)

### 5-Chloro-N-(3-chlorophenyl)-4-[5-(hydroxymethyl)-2,4-dioxo-1,4-dihydroquinazolin-3(2H)-yl]-N-methylthiophene-2-carboxamide

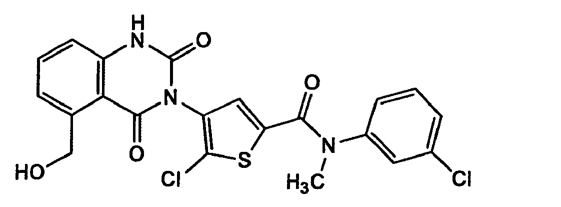

¹H-NMR (DMSO-d₆) δ 3.35 (3H, s), 4.89 (2H, d), 5.26 (1H, t), 6.87 (1H, s), 7.09 (1H, d), 7.39-7.53 (4H, m), 7.61-7.71 (2H, m), 11.61 (1H, s)

### Example 73(3)

### 5-Chloro-N-(4-chlorophenyl)-4-[5-(hydroxymethyl)-2,4-dioxo-1,4-dihydroquinazolin-3(2H)-yl]-N-methylthiophene-2-carboxamide

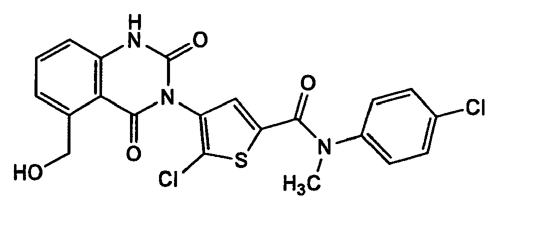

¹H-NMR (DMSO-d₆) δ 3.33 (3H, s), 4.90 (2H, d), 5.26 (1H, t), 6.90 (1H, s), 7.09 (1H, d), 7.44-7.58 (5H, m), 7.67 (1H, t), 11.61 (1H, s)

### Example 73 (4)

### 5-Chloro-4-[5-(hydroxymethyl)-2,4-dioxo-1,4-dihydroquinazolin-3(2H)-yl]-N-methyl-N-(2-methylphenyl)thiophene-2-carboxamide

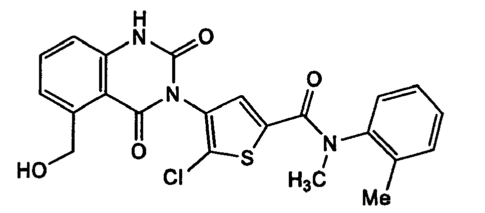

¹H-NMR (DMSO-d₆) δ 2.20 (3H, s), 3.27 (3H, s), 4.90 (2H, d), 5.26 (1H, t), 7.03-7.13 (2H, m), 7.33-7.51 (5H, m), 7.67 (1H, t), 11.61 (1H, s)

### Example 73(5)

### 5-Chloro-4-[5-(hydroxymethyl)-2,4-dioxo-1,4-dihydroquinazolin-3(2H)-yl]-N-methyl-N-(3-methylphenyl)thiophene-2-carboxamide

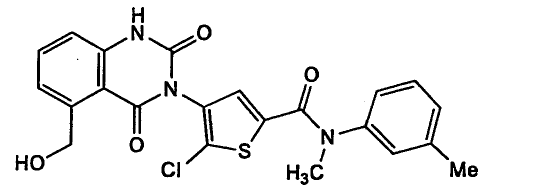

¹H-NMR (DMSO-d₆) δ 2.34 (3H, s), 3.33 (3H, s), 4.89 (2H, d), 5.26 (1H, t), 6.82 (1H, s), 7.09 (1H, d), 7.22-7.31 (3H, m), 7.34-7.51 (2H, m), 7.67 (1H, t), 11.61 (1H, s)

### Example 73(6)

### 5-Chloro-4-[5-(hydroxymethyl)-2,4-dioxo-1,4-dihydroquinazolin-3(2H)-yl]-N-methyl-N-(4-methylphenyl)thiophene-2-carboxamide

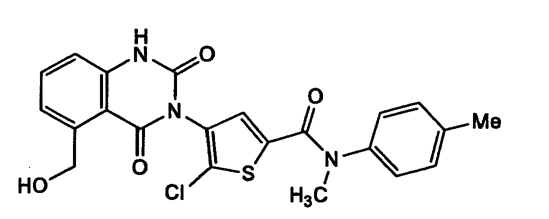

¹H-NMR (DMSO-d₆) δ 2.36 (3H, s), 3.31 (3H, s), 4.90 (2H, d), 5.26 (1H, t), 7.00 (1H, s), 7.10 (1H, d), 7.30-7.34 (4H, m), 7.48 (1H, d), 7.67 (1H, t), 11.60 (1H, s)

### Example 73 (7)

### 5-Chloro-4-[5-(hydroxymethyl)-2,4-dioxo-1,4-dihydroquinazolin-3(2H)-yl]-N-methyl-N-pyridin-2-ylthiophene-2-carboxamide

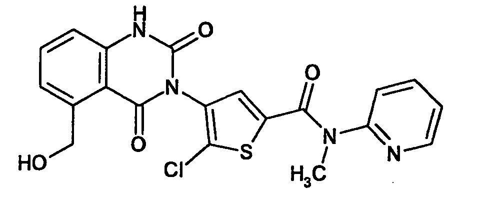

¹H-NMR (DMSO-d₆) δ 3.41 (3H, s), 4.90 (2H, d), 5.26 (1H, t), 6.80 (1H, s), 7.09 (1H, d), 7.36-7.42 (1H, m), 7.45-7.54 (2H, m), 7.67 (1H, t), 7.91 (1H, td), 8.49-8.53 (1H, m), 11.59 (1H, s)

### Example 73 (8)

### 5-Chloro-N-(2-fluorophenyl)-4-[5-(hydroxymethyl)-2,4-dioxo-1,4-dihydroquinazolin-3(2H)-yl]-N-methylthiophene-2-carboxamide

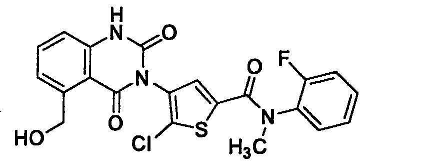

¹H-NMR (DMSO-d₆) δ 3.32 (3H, s), 4.90 (2H, d), 5.27 (1H, t), 7.02 (1H, s), 7.10 (1H, d), 7.32-7.59 (4H, m), 7.60-7.71 (2H, m), 11.61 (1H, s)

### Example 73(9)

### 5-Chloro-4-[5-(hydroxymethyl)-2,4-dioxo-1,4-dihydroquinazolin-3(2H)-yl]-N-methyl-N-[2-(trifluoromethyl)phenyl]thiophene-2-carboxamide

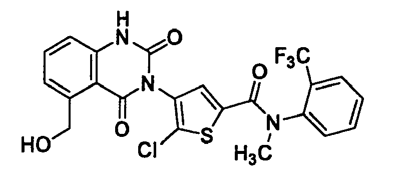

¹H-NMR (DMSO-d₆) δ 3.30 (3H, s), 4.88 (2H, d), 5.26 (1H, t), 6.79 (1H, s), 7.08 (1H, d), 7.46 (1H, d), 7.66 (1H, t), 7.72-7.98 (4H, m), 11.59 (1H, s)

### Example 73(10)

### 5-Chloro-4-[5-(hydroxymethyl)-2,4-dioxo-1,4-dihydroquinazolin-3(2H)-yl]-N-methyl-N-[2-(trifluoromethoxy)phenyl]thiophene-2-carboxamide

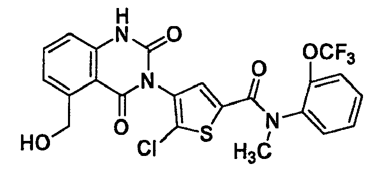

¹H-NMR (DMSO-d₆) δ 3.31 (3H, s), 4.89 (2H, d), 5.26 (1H, t), 6.87 (1H, br s), 7.09 (1H, d), 7.44-7.80 (6H, m), 11.60 (1H, s)

### Example 73(11)

### 5-Chloro-4-[5-(hydroxymethyl)-2,4-dioxo-1,4-dihydroquinazolin-3(2H)-yl]-N-(2-methoxyphenyl)-N-methylthiophene-2-carboxamide

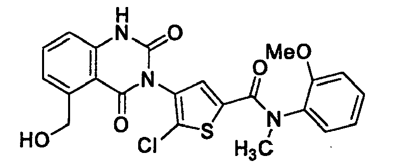

¹H-NMR (DMSO-d₆) δ 3.23 (3H, s), 3.79 (3H, s), 4.90 (2H, d), 5.27 (1H, t), 7.03-7.13 (3H, m), 7.21 (1H, d), 7.41 (1H, dd), 7.45-7.54 (2H, m), 7.67 (1H, t), 11.62 (1H, s)

### Example 73(12)

### 5-Chloro-N-(3-fluorophenyl)-4-[5-(hydroxymethyl)-2,4-dioxo-1,4-dihydroquinazolin-3(2H)-yl]-N-methylthiophene-2-carboxamide

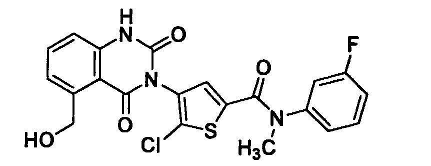

¹H-NMR (DMSO-d₆) δ 3.35 (3H, s), 4.89 (2H, d), 5.26 (1H, t), 6.83 (1H, s), 7.06-7.11 (1H, m), 7.24-7.33 (2H, m), 7.41-7.57 (3H, m), 7.66 (1H, t), 11.60 (1H, s)

### Example 74

### 4-[5-({[tert-Butyl(dimethyl)silyl]oxy}methyl)-2,4-dioxo-1,4-dihydroquinazolin-3(2H)-yl]-5-chloro-N-isobutyl-N-methylthiophene-2-carboxamide

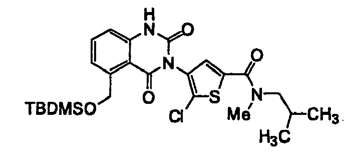

To a solution of 4-[5-({[tert-butyl(dimethyl)silyl]oxy}methyl)-2,4-dioxo-1,4-dihydroquinazolin-3(2H)-yl]-5-chlorothiophene-2-carboxylic acid (100 mg), N-isobutylmethylamine (56 mg) and N,N-diisopropylethylamine (0.056 ml) in DMF (3 ml), was added a 50% solution of propanephosphonic acid anhydride in ethyl acetate (250 mg), and the mixture was stirred at room temperature for 1 hour. The reaction solution was diluted with ethyl acetate and an aqueous sodium hydrogen carbonate solution, and then extracted with ethyl acetate. The organic layer was dried over anhydrous magnesium sulfate, and then concentrated. The resulting residue was purified with silica gel column chromatography to give the desired product (51 mg) as amorphous.

¹H-NMR (DMSO-d₆) δ 0.10 (6H, s), 0.87 (6H, d), 0.94 (9H, s), 1.91-2.07 (1H, m), 3.25-3.40 (2H, m), 3.05-3.27 (3H, m), 5.16 (2H, s), 7.17 (1H, d), 7.49 (1H, d), 7.60 (1H, br s), 7.74 (1H, t), 11.72 (1H, br s)

### Example 74 (1)

In the same manner as in Example 74, 4-[5-({[tert-butyl(dimethyl)silyl]oxy}methyl)-2,4-dioxo-1,4-dihydroquinazolin-3(2H)-yl]-5-chlorothiophene-2-carboxylic acid was reacted with N-methylisopropylamine to obtain the following compound.

### 4-[5-({[tert-Butyl(dimethyl)silyl]oxy}methyl)-2,4-dioxo-1,4-dihydroquinazolin-3(2H)-yl]-5-chloro-N-isopropyl-N-methylthiophene-2-carboxamide

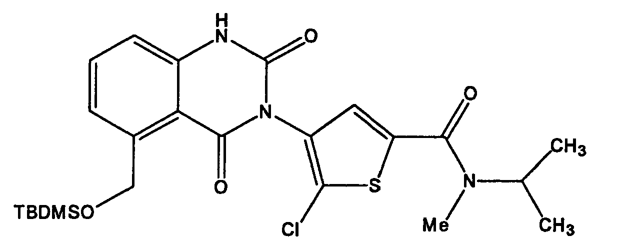

¹H-NMR (DMSO-d₆) δ 0.04-0.14 (6H, m), 0.91-0.98 (9H, m), 1.17 (6H, d), 2.97 (3H, s), 4.56 (1H, br s), 5.16 (2H, s), 7.17 (1H, d), 7.46-7.58 (2H, m), 7.74 (1H, t), 11.70 (1H, br s)

### Example 75

### 3-[5-(3,4-Dihydroquinolin-1(2H)-ylcarbonyl)-2-(trifluoromethyl)phenyl]quinazoline-2,4(1H,3H)-dione

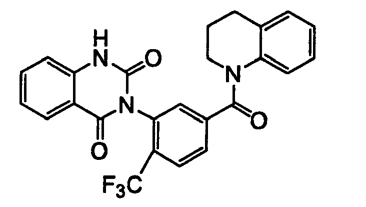

A solution of 2-amino-N-[5-(3,4-dihydroquinolin-1(2H)-ylcarbonyl)-2-(trifluoromethyl)phenyl]benzamide (24 mg) in THF (10 ml) was cooled to 5°C. Then, triphosgene (23 mg) was added thereto, and the mixture was stirred at room temperature for 2 hours. The reaction solution was diluted with ethyl acetate and an aqueous sodium hydrogen carbonate solution. The organic layer was washed with saturated brine, and dried over anhydrous magnesium sulfate. Then, the solvent was distilled off under reduced pressure to give the desired product (18 mg) as amorphous.

¹H-NMR (CDCl₃) δ 1.96-2.18 (2H, m), 2.85 (2H, t), 3.78-3.91 (1H, m), 3.96-4.08 (1H, m), 6.78-7.09 (4H, m), 7.12-7.25 (2H, m), 7.49-7.56 (2H, m), 7.56-7.65 (1H, m), 7.72 (1H, d), 8.09 (1H, dd), 9.62 (1H, s)

### Example 76

### 3-[2-Chloro-5-(3,4-dihydroquinolin-1(2H)-ylsulfonyl)phenyl]-3,4-dihydroquinazolin-2(1H)-one

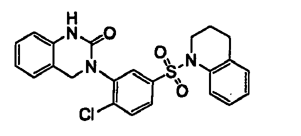

A solution of 2-chloro-5-(3,4-dihydroquinolin-1(2H)-ylsulfonyl)-N-(2-nitrobenzyl)aniline (247 mg) and nickel bromide (II) (4 mg) in THF/methanol (10 ml/10 ml) was cooled to 5°C, sodium tetrahydroborate (120 mg) was slowly added thereto, and the mixture was stirred for 20 minutes as it was. The reaction solution was diluted with ethyl acetate and an aqueous sodium hydrogen carbonate solution. Then, the organic layer was washed with an aqueous saturated sodium chloride solution and dried. After filtering the drying agent, the solvent was distilled off under reduced pressure. The resulting residue was dissolved in THF (10 ml) and the reaction solution was cooled to 5°C. Then, triphosgene (240 mg) was added thereto, and the mixture was stirred for 1 hour as it was. The reaction solution was diluted with ethyl acetate and an aqueous sodium hydrogen carbonate solution. The organic layer was separated and dried over anhydrous magnesium sulfate. After filtering the drying agent, the solvent was distilled off under reduced pressure. The resulting residue was purified with silica gel column chromatography, and recrystallized from ethyl acetate to give the desired product (122 mg) as crystals.

¹H-NMR (CDCl₃) δ 1.61-1.78 (2H, m) , 2.30-2.68 (2H, m), 3.60-4.02 (2H, m), 4.49-4.75 (2H, m), 6.73 (1H, d), 6.96-7.15 (5H, m), 7.16-7.25 (2H, m), 7.39-7.46 (1H, m), 7.47-7.54 (1H, m), 7.65 (1H, d), 7.78 (1H, dd)

### Example 77

### 3- [2-Chloro-5- (3 , 4-dihydroquinolin-1 (2H) - ylsulfonyl)phenyl]-6-fluoro-3,4-dihydroquinazolin-2(1H)-one

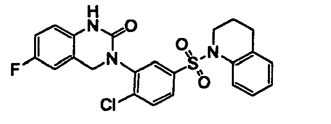

To a solution of 2-chloro-5-(3,4-dihydroquinolin-1(2H)-ylsulfonyl)-N-(5-fluoro-2-nitrobenzyl)aniline (97 mg) and reduced iron (350 mg) in ethanol (10 ml), was added concentrated hydrochloric acid (10 ml), and the mixture was stirred at 60°C for 1 hour. The reaction solution was diluted with an aqueous sodium hydrogen carbonate solution, and then extracted with ethyl acetate. The organic layer was dried over anhydrous magnesium sulfate, the solvent was distilled off under reduced pressure, and the resulting residue was dissolved in THF (5 ml). To the reaction solution was added triphosgene (103 mg), and the mixture was stirred for 30 minutes as it was. The reaction solution was diluted with ethyl acetate and an aqueous sodium hydrogen carbonate solution. The organic layer was separated, and dried over anhydrous magnesium sulfate. After filtering the drying agent, the solvent was distilled off under reduced pressure. The resulting residue was purified with silica gel column chromatography, and recrystallized from hexane/ethyl acetate to give the desired product (66 mg) as crystals.

¹H-NMR (CDCl₃) δ 1. 63-1. 81 (2H, m), 2.30-2.68 (2H, m), 3.58-4.06 (2H, m), 4.46-4.71 (2H, m), 6.68 (1H, dd), 6.77 (1H, dd), 6.87-6.97 (1H, m), 7.01-7.07 (1H, m), 7.07-7.15 (1H, m), 7. 17-7.25 (1H, m), 7.44 (1H, d), 7.51 (1H, d), 7.65 (1H, d), 7.70 (1H, s), 7.78 (1H, d)

### Examples 77 (1) to 77(4)

In the same manner as in Example 77, the following compounds were obtained from the compounds of Reference Examples 94(2) to 94(5).

### Example 77 (1)

### 3-[2-Chloro-5-(3,4-dihydroquinolin-1(2H)-ylsulfonyl)phenyl]-6-methoxy-3,4-dihydroquinazolin-2(1H)-one

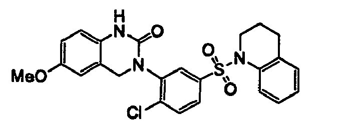

¹H-NMR (CDCl₃) δ 1.62-1.79 (2H, m), 2.32-2.68 (2H, m), 3.62-3.76 (1H, m), 3.78 (3H, s), 3.85-4.01 (1H, m), 4.48-4.70 (2H, m), 6.61 (1H, d), 6.66 (1H, d), 6.79 (1H, dd), 6.86 (1H, s), 7.01-7.07 (1H, m), 7.07-7.15 (1H, m), 7.16-7.25 (1H, m), 7.42 (1H, dd), 7.50 (1H, d), 7.65 (1H, d), 7.78 (1H, d)

### Example 77(2)

### 6-Bromo-3-[2-chloro-5-(3,4-dihydroquinolin-1(2H)-ylsulfonyl)phenyl]-3,4-dihydroquinazolin-2(1H)-one

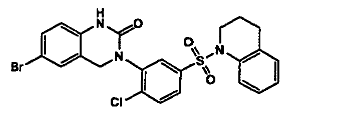

¹H-NMR (CDCl₃) δ 1.63-1.81 (2H, m), 2.30-2.67 (2H, m), 3.62-4.05 (2H, m), 4.44-4.69 (2H, m), 6.62 (1H, d), 7.00-7.08 (1H, m), 7.07-7.15 (1H, m), 7.16-7.25 (2H, m), 7.31 (1H, dd), 7.45 (1H, dd), 7.52 (1H, d), 7.64 (1H, d), 7.70-7.83 (2H, m)

### Example 77 (3)

### 3-[2-Chloro-5-(3,4-dihydroquinolin-1(2H)-ylsulfonyl)phenyl]-7-fluoro-3,4-dihydroquinazolin-2(1H)-one

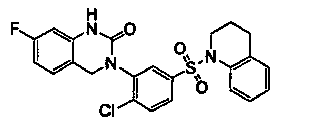

¹H-NMR (CDCl₃) δ 1.63-1. 80 (2H, m), 2.32-2. 66 (2H, m), 3.61-4.04 (2H, m), 4.48-4.71 (2H, m), 6.46 (1H, dd), 6.65-6.76 (1H, m), 6.94-7.25 (4H, m), 7.42 (1H, dd), 7.47-7.55 (1H, m), 7.66 (1H, d), 7.72 (1H, s), 7.79 (1H, d)

### Example 77(4)

### 7-Bromo-3-[2-chloro-5-(3,4-dihydroquinolin-1(2H)-ylsulfonyl)phenyl]-3,4-dihydroquinazolin-2(1H)-one

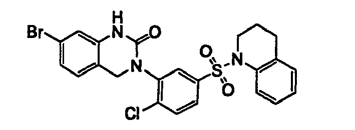

¹H-NMR (DMSO-d₆) δ 1.55-1.75 (2H, m), 2.37-2.68 (2H, m), 3.64-3.92 (2 H, m), 4.46 (1H, d), 4.72 (1H, d), 7.03 (1H, s), 7. 08-7.14 (4H, m), 7.14-7.23 (1H, m), 7.56 (1H, dd), 7.60 (1H, d), 7.75 (1H, d), 7.82 (1H, d), 9.86 (1 H, s)

### Example 78

### 3-[2-Chloro-5-(3,4-dihydroquinolin-1(2H)-ylsulfonyl)phenyl]-2-oxo-1,2,3,4-tetrahydroquinazoline-6-carbonitrile

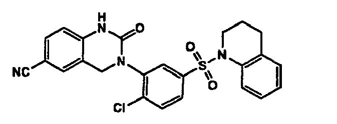

Under nitrogen atmosphere, a solution of 6-bromo-3-[2-chloro-5-(3,4-dihydroquinolin-1(2H)-ylsulfonyl)phenyl]-3,4-dihydroquinazolin-2(1H)-one (140 mg), zinc cyanide (20 mg) and tetrakis(triphenylphosphine)palladium (0) (40 mg) in DMF (3 ml) was stirred at 100°C for 6 hours. The reaction solution was cooled to room temperature, and then diluted with ethyl acetate and an aqueous sodium hydrogen carbonate solution. The organic layer was washed with an aqueous sodium hydrogen carbonate solution and saturated brine, and then dried over anhydrous magnesium sulfate. After filtering the drying agent, the solvent was distilled off under reduced pressure. The resulting residue was purified with NH silica gel column chromatography, and recrystallized from ethyl acetate to give the desired product (34 mg) as crystals.

¹H-NMR (DMSO-d₆) δ 1.55-1. 74 (2H, m), 2.41-2.52 (2H, m), 3.69-3.90 (2H, m), 4.47-4.61 (1H, m), 4.74-4.88 (1H, m), 6.99 (1H, d), 7.07-7.26 (3H, m), 7.53 (1H, dd), 7.59 (1H, d), 7.63-7.71 (2H, m), 7.76 (1H, d), 7.87 (1H, d), 10.23 (1H, s)

### Example 78(1)

In the same manner as in Example 78, the following compound was obtained from 7-bromo-3-[2-chloro-5-(3,4-dihydroquinolin-1(2H)-ylsulfonyl)phenyl]-3,4-dihydroquinazolin-2(1H)-one.

### 3-[2-chloro-5-(3,4-dihydroquinolin-1(2H)-ylsulfonyl)phenyl]-2-oxo-1,2,3,4-tetrahydroquinazoline-7-carbonitrile

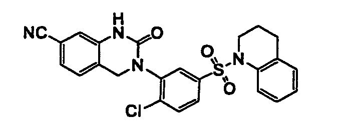

¹H-NMR (DMSO-d₆) δ 1.58-1.72 (2H, m), 2.39-2.49 (2H, m), 3.70-3.91 (2H, m), 4.59 (1H, d), 4.86 (1H, d), 7.07-7.25 (4H, m), 7.36 (1H, d), 7.43 (1H, d), 7.52 (1H, dd), 7.59 (1H, d), 7.75 (1H, d), 7.87 (1H, d), 10.05 (1H, s)

### Example 78(1)-1

### 3-[2-Cyano-5-(3,4-dihydroquinolin-1(2H)-ylsulfonyl)phenyl]-2-oxo-1,2,3,4-tetrahydroquinazoline-7-carbonitrile

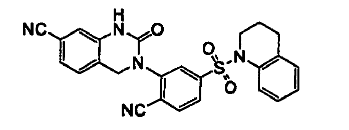

When the above-mentioned compound of Example 78 (1) was synthesized, the compound was isolated as a by-product.

¹H-NMR (DMSO-d₆) δ 1.60-1.72 (2H, m), 2.44-2.49 (2H, m), 3.78-3.87 (2H, m), 4.85 (2H, s), 7.11-7.27 (4H, m), 7.38 (1H, d), 7.46 (1H, dd), 7.55-7.65 (2H, m), 7.89 (1H, d), 8.06 (1H, d), 10.32 (1H, br s)

### Example 79

Ethyl 3-[2-chloro-5-(3,4-dihydroquinolin-1(2H)-ylsulfonyl)phenyl]-2-oxo-1,2,3,4-tetrahydroquinazoline-6-carboxylate

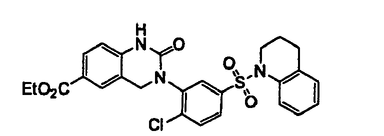

Under carbon monoxide atmosphere, a solution of 6-bromo-3-[2-chloro-5-(3,4-dihydroquinolin-1(2H)-ylsulfonyl)phenyl]-3,4-dihydroquinazolin-2(1H)-one (273 mg), palladium acetate (II) (98 mg), 1,1'-bis(diphenylphosphino)ferrocene (200 mg) and triethylamine (0.3 ml) in ethanol (25 ml) was stirred at 70°C for 16 hours. The catalyst was filtered, and then the filtrate was diluted with ethyl acetate and an aqueous sodium hydrogen carbonate solution. The organic layer was separated, and then dried over anhydrous magnesium sulfate, and the solvent was distilled off under reduced pressure. The resulting residue was purified with silica gel column chromatography, and recrystallized from ethyl acetate to give the desired product (150 mg) .

¹H-NMR (DMSO-d₆) δ 1.30 (3H, t), 1.60-1.74 (2H, m), 2.42-2.55 (2H, m), 3.74-3.88 (2H, m), 4.28 (2H, q), 4.56-4.68 (1H, m), 4.73-4.88 (1H, m), 6.95 (1H, d), 7.06-7.27 (3H, m), 7.52 (1H, dd), 7.60 (1H, d), 7.72-7.79 (2H, m), 7.82 (1H, dd), 7.86 (1H, d), 10.13 (1H, s)

### Example 79(1)

In the same manner as in Example 79, the following compound was obtained from 5-bromo-3-[2-chloro-5-(3,4-dihydroquinolin-1(2H)-ylsulfonyl)phenyl]-3,4-dihydroquinazolin-2(1H)-one.

### Ethyl 3-[2-chloro-5-(3,4-dihydroquinolin-1(2H)-ylsulfonyl)phenyl]-2-oxo-1,2,3,4-tetrahydroquinazoline-5-carboxylate

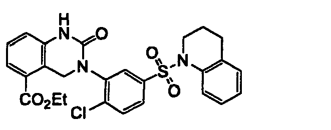

¹H-NMR (DMSO-d₆) δ 1.27 (3H, t), 1.59-1.73 (2H, m), 2.43-2.50 (2H, m), 3.77-3.85 (2H, m), 4.26 (2H, q), 4.98 (2H, s), 7.05-7.24 (4H, m), 7.35 (1H, t), 7.49-7.63 (3H, m), 7.76 (1H, d), 7.88 (1H, d), 9.90 (1H, br s)

### Example 80

### 3-[2-Chloro-5-(3,4-dihydroquinolin-1(2H)-ylsulfonyl)phenyl]-6-(1-hydroxy-1-methylethyl)-3,4-dihydroquinazolin-2(1H)-one

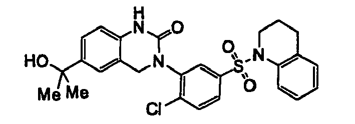

A solution of ethyl 3-[2-chloro-5-(3,4-dihydroquinolin-1(2H)-ylsulfonyl)phenyl]-2-oxo-1,2,3,4-tetrahydroquinazoline-6-carboxylate (88 mg) in THF (10 ml) was cooled to 5°C. A 3.0 N solution of methyl magnesium bromide in diethyl ether (1.6 ml) was slowly added thereto, and the temperature was elevated to room temperature. The reaction solution was stirred at room temperature for 4 hours, and then diluted with an aqueous ammonium chloride solution and ethyl acetate. The organic layer was separated, and then dried over anhydrous magnesium sulfate, and the solvent was distilled off under reduced pressure. The resulting residue was recrystallized from hexane/ethyl acetate to give the desired product (52 mg) as crystals.

¹H-NMR (DMSO-d₆) δ 1.40 (6H, s), 1.56-1.74 (2H, m), 2.34-2.60 (2H, m), 3.69-3.93 (2H, m), 4.36-4.83 (2H, m), 4.94 (1H, s), 6.78 (1H, d), 7.05-7.32 (5H, m), 7.51 (1H, dd), 7.60 (1H, d), 7.70-7.82 (2H, m), 9.64 (1H, s)

### Example 80(1)

In the same manner as in Example 80, the following compound was obtained from ethyl 3-[2-chloro-5-(3,4-dihydroquinolin-1(2H)-ylsulfonyl)phenyl]-2-oxo-1,2,3,4-tetrahydroquinazoline-5-carboxylate.

### 3-[2-Chloro-5-(3,4-dihydroquinolin-1(2H)-ylsulfonyl)phenyl]-5-(1-hydroxy-1-methylethyl)-3,4-dihydroquinazolin-2(1H)-one

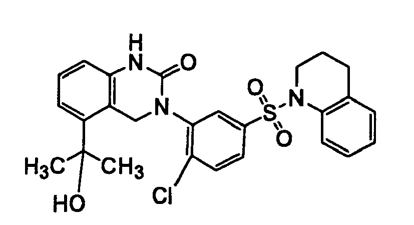

¹H-NMR (DMSO-d₆) δ 1.39-1.48 (6H, m), 1.59-1.71 (2H, m), 2.42-2.49 (2H, m), 3.77-3.84 (2H, m), 4.78-4.88 (1H, m), 5.02-5.12 (2H, m), 6.81 (1H, d), 6.94 (1H, d), 7.07-7.25 (4H, m), 7.52 (1H, dd), 7.60 (1H, d), 7. 75 (1H, d), 7.79 (1H, d), 9.66 (1H, s)

### Example 81

### 3-[2-Chloro-5-(3,4-dihydroquinolin-1(2H)-ylsulfonyl)phenyl]-2-oxo-1,2,3,4-tetrahydroquinazoline-6-carboxylic acid

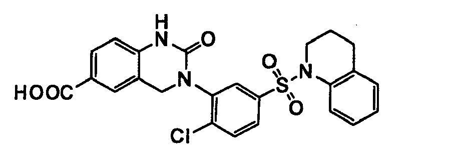

To a solution of ethyl 3-[2-chloro-5-(3,4-dihydroquinolin-1(2H)-ylsulfonyl)phenyl]-2-oxo-1,2,3,4-tetrahydroquinazoline-6-carboxylate (250 mg) in ethanol/THF (15 ml/5 ml), was added 1 N sodium hydroxide (5 ml), and the mixture was stirred at 60°C for 2 hours. The reaction solution was diluted with 1 N hydrochloric acid, and then the solvent was distilled off under reduced pressure. The obtained solid was separated by filtration, washed with water, and then crystallized from THF/methanol/ethyl acetate to give the desired product (210 mg) as crystals.

¹H-NMR (DMSO-d₆) δ 1.57-1.74 (2H, m), 2.39-2.59 (2H, m), 3.69-3.92 (2H, m), 4.51-4.89 (2H, m), 6.92 (1H, d), 7.05-7.26 (3H, m), 7.52 (1H, dd), 7.60 (1H, d), 7.71-7.83 (3H, m), 7.85 (1H, d), 10.09 (1H, s), 12.71 (1H, br s)

### Example 82

### 3-[2-Chloro-5-(3,4-dihydroquinolin-1(2H)-ylsulfonyl)phenyl]-N-methyl-2-oxo-1,2,3,4-tetrahydroquinazoline-6-carboxamide

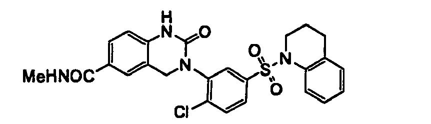

A solution of 3-[2-chloro-5-(3,4-dihydroquinolin-1(2H)-ylsulfonyl)phenyl]-2-oxo-1,2,3,4-tetrahydroquinazoline-6-carboxylic acid (60 mg), 1-[3-(dimethylamino)propyl]-3-ethylcarbodiimide hydrochloride (35 mg), 1-hydroxybenzotriazole (28 mg), N,N-diisopropylethylamine (0.1 ml) and 2 M methylamine in THF (0.5 ml) was dissolved in DMF (3 ml), and the mixture was stirred at room temperature for 16 hours. The reaction solution was diluted with water, and then the resulting solid was separated by filtration and air-dried. The obtained solid was purified with silica gel column chromatography, and recrystallized from ethyl acetate to give the desired product (23 mg) as crystals.

¹H-NMR (DMSO-d₆) δ 1.56-1.72 (2H, m), 2.40-2.54 (2H, m), 2.76 (3H, d), 3.71-3.90 (2H, m), 4.46-4.60 (1H, m), 4.73-4.87 (1H, m), 6.88 (1H, d), 7.06-7.27 (3H, m), 7.52 (1H, dd), 7.57-7.71 (3H, m), 7.75 (1H, d), 7.84 (1H, d), 8.21-8.34 (1H, m), 9.97 (1H, s)

### Examples 82 (1) to 82(10)

In the same manner as in Example 82, the corresponding carboxylic acids (Examples 81, 31, Reference Example 81(1), Example 64 or Example 93) were reacted with the corresponding commercially available amine to obtain the following compounds.

### Example 82(1)

### 3-[2-Chloro-5-(3,4-dihydroquinolin-1(2H)-ylsulfonyl)phenyl]-N-(2-methoxyethyl)-2-oxo-1,2,3,4-tetrahydroquinazoline-6-carboxamide

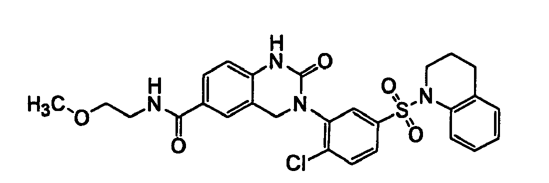

¹H-NMR (DMSO-d₆) δ 1.58-1.74 (2H, m), 2.40-2.57 (2H, m), 3.26 (3H, s), 3.35-3.49 (4H, m), 3.73-3.87 (2H, m), 4.48-4.60 (1H, m), 4.74-4.86 (1H, m), 6.88 (1H, d), 7.07-7.25 (3H, m), 7.52 (1H, dd), 7.60 (1H, d), 7.66 (1H, s), 7.69-7.79 (2H, m), 7.85 (1H, d), 8.32-8.40 (1H, m), 9.98 (1H, s)

### Example 82(2)

### 3-[2-Chloro-5-(3,4-dihydroquinolin-1(2H)-ylsulfonyl)phenyl]-N-methyl-2,4-dioxo-1,2,3,4-tetrahydroquinazoline-5-carboxamide

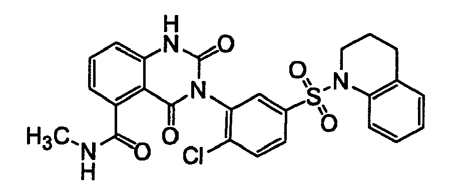

¹H-NMR (DMSO-d₆) δ 1.60-1.73 (2H, m), 2.45-2.54 (2H, m), 2.67 (3H, d), 3.71-3.82 (2H, m), 6.99-7.23 (4H, m), 7.27 (1H, dd), 7.52-7.63 (2H, m), 7.71 (1H, dd), 7.81 (1H, d), 7.93 (1H, d), 7.94-8.03 (1H, m), 11.81 (1H, br s)

### Example 82(3)

### 3-[2-Chloro-5-(3,4-dihydroquinolin-1(2H)-ylsulfonyl)phenyl]-N-(2-methoxyethyl)-2,4-dioxo-1,2,3,4-tetrahydroquinazoline-5-carboxamide

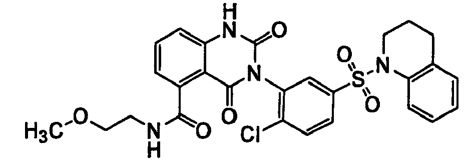

¹H-NMR (DMSO-d₆) δ 1.58-1.72 (2H, m), 2.45-2.50 (2H, m), 3.21 (3H, s), 3.24-3.32 (2H, m), 3.36-3.44 (2H, m), 3.72-3.82 (2H, m), 7.04 (1H, d), 7.08-7.13 (2H, m), 7.14-7.22 (1H, m), 7.27 (1H, d), 7.50-7.60 (2H, m), 7. 71 (1H, t), 7. 81 (1H, d), 7.93 (1H, d), 8.13 (1H, t), 11.81 (1H, br s)

### Example 82(4)

### 3-[2-Chloro-5-(3,4-dihydroquinolin-1(2H)-ylsulfonyl)phenyl]-5-(pyrrolidin-1-ylcarbonyl)quinazoline-2,4(1H,3H)-dione

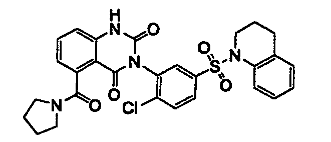

¹H-NMR (DMSO-d₆) δ 1.56-1.89 (6H, m), 2.40-2.60 (2H, m), 2.82-3.13 (2H, m), 3.35-3.45 (2H, m), 3.65-3.91 (2H, m), 6.99-7.23 (4H, m), 7.27 (1H, d), 7.50-7.63 (2H, m), 7.70-7.85 (2H, m), 8.03-8.18 (1H, m), 11.85 (1H, s)

### Example 82(5)

### N-Benzyl-3-[2-chloro-5-(3,4-dihydroquinolin-1(2H)-ylsulfonyl)phenyl]-2,4-dioxo-1,2,3,4-tetrahydroquinazoline-5-carboxamide

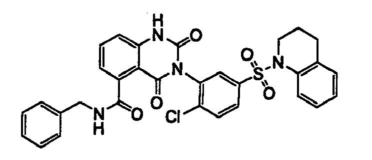

¹H-NMR (DMSO-d₆) δ 1.59-1.72 (2H, m), 2.44-2.54 (2H, m), 3.71-3.82 (2H, m), 4.39 (2H, d), 7.07-7.35 (10H, m), 7.56-7.65 (2H, m), 7.73 (1H, d), 7.83 (1H, d), 7.97 (1H, d), 8.61 (1H, t), 11.83 (1H, br s)

### Example 82(6)

### 3-[2-Chloro-5-(3,4-dihydroquinolin-1(2H)-ylsulfonyl)phenyl]-N,N-dimethyl-2,4-dioxo-1,2,3,4-tetrahydroquinazoline-5-carboxamide

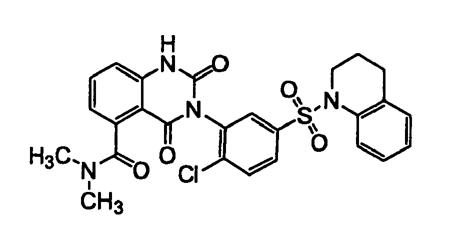

¹H-NMR (DMSO-d₆) δ 1.58-1.76 (2H, m), 2.43-2. 56 (2H, m), 2.61 (3H, d), 2.91 (3H, s), 3.67-3.88 (2H, m), 6.94-7.04 (1H, m), 7.05-7.23 (3H, m), 7.27 (1H, d), 7.49-7.65 (2H, m), 7.69-5 7.86 (2H, m), 8.06 (1H, dd), 11.85 (1H, br s)

### Example 82(7)

### N-Benzyl-6-(2,4-dioxo-1,4-dihydroquinazolin-3(2H)-yl)-N-methylpyridine-2-carboxamide

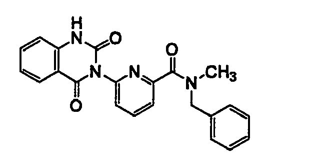

¹H-NMR (DMSO-d₆) δ 2.84-2.96 (3H, m), 4. 54-4. 74 (2H, m), 7.16-7.43 (7H, m), 7.65 (1H, dd), 7.69-7.80 (2H, m), 7.89-7.99 (1H, m), 8.10-8.20 (1H, m), 11.68 (1H, br s)

### Example 82(8)

### N-({3-[3-(3,4-Dihydroquinolin-1(2H)-ylsulfonyl)phenyl]-2,4-dioxo-1,2,3,4-tetrahydroquinazolin-5-yl}acetyl)glycine tert-butyl ester

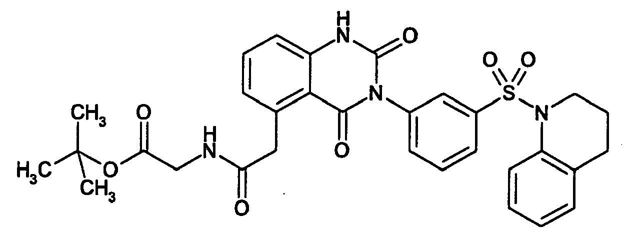

¹H-NMR (DMSO-d₆) δ 1.36 (9H, s), 1.58-1.69 (2H, m), 2.44-2.54 (2H, m), 3.66 (2H, d), 3.72-3.80 (2H, m), 4.02 (2H, s), 7.02-7.11 (3H, m), 7.12-7.21 (2H, m), 7.50-7.64 (5H, m), 7.66 (1H, s), 8.00 (1H, t), 11.56 (1H, s)

### Example 82(9)

### 3-[3-(3,4-Dihydroquinolin-1(2H)-ylsulfonyl)phenyl]-N,N-dimethyl-2,4-dioxo-1,2,3,4-tetrahydroquinazoline-7-carboxamide

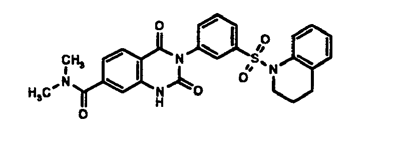

¹H-NMR (DMSO-d₆) δ 1.65 (2H, ddd), 2.48 (2H, t), 2.89 (3H, s), 3.01 (3H, s), 3.76 (2H, t), 7.08-7.09 (2H, m), 7. 15-7.23 (3H, m), 7.57-7.68 (3H, m), 7. 80 (1H, d), 7.98 (1H, d), 11.72 (1H, br s)

### Example 82(10)

### N-Benzyl-3-[3-(3,4-dihydroquinolin-1(2H)-ylsulfonyl)phenyl]-2,4-dioxo-1,2,3,4-tetrahydroquinazoline-7-carboxamide

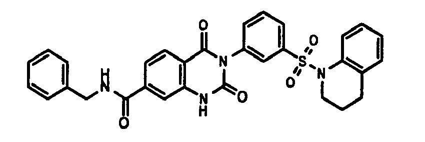

¹H-NMR (DMSO-d₆) δ 1.66 (2H, ddd), 2.48 (2H, t), 3.77 (2H, t), 4.50 (2H, d), 7.08 (2H, d), 7.18 (1H, ddd), 7.23-7.30 (1H, m), 7.34-7.36 (4H, m), 7.51 (1H, dt), 7.57-7.70 (5H, m), 7.82-7.83 (1H, m), 8.02 (1H, d), 9.33 (1H, t), 11.74 (1H, br s)

### Example 83

### N-({3-[3-(3,4-Dihydroquinolin-1(2H)-ylsulfonyl)phenyl]-2,4-dioxo-1,2,3,4-tetrahydroquinazolin-5-yl}acetyl)glycine

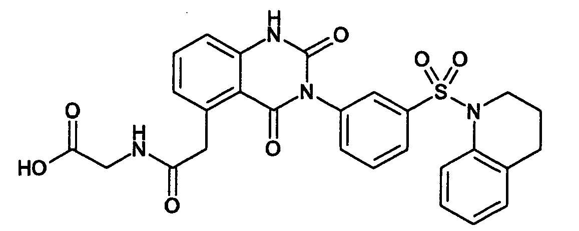

N-({3-[3-(3,4-Dihydroquinolin-1(2H)-ylsulfonyl)phenyl]-2,4-dioxo-1,2,3,4-tetrahydroquinazolin-5-yl}acetyl)glycine tert-butyl ester (120 mg) was dissolved in dichloromethane (5 ml) and trifluoroacetic acid (5 ml), and the mixture was stirred at room temperature overnight. The solvent was distilled off under reduced pressure, and then the resulting residue was crystallized from hexane to give the desired product (98 mg) as crystals.

¹H-NMR (DMSO-d₆) δ 1.58-1.69 (2H, m), 2.44-2.54 (2H, m), 3.71 (2H, d), 3.73-3.79 (2H, m), 4.03 (2H, s), 7.04-7.11 (3H, m), 7.13-7.22 (2H, m), 7.50-7.63 (5H, m), 7.66-7.69 (1H, m), 7.98 (1H, t), 11.55 (1H, s), 12.45 (1H, s)

### Example 84

### 3-[2-Chloro-5-(3,4-dihydroquinolin-1(2H)-ylsulfonyl)phenyl]-6-(hydroxymethyl)-3,4-dihydroquinazolin-2(1H)-one

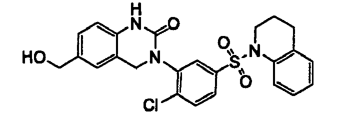

A solution of 3-[2-chloro-5-(3,4-dihydroquinolin-1(2H)-ylsulfonyl)phenyl]-2-oxo-1,2,3,4-tetrahydroquinazoline-6-carboxylic acid (85 mg) in THF (10 ml) was cooled to 5°C, and then a 1.1 mol/l solution of borane in THF (5 ml) was added thereto. The reaction solution was stirred at room temperature for 16 hours, and then diluted with an aqueous sodium hydrogen carbonate solution. The reaction solution was extracted with ethyl acetate, and then the organic layer was dried over anhydrous magnesium sulfate, and the solvent was distilled off under reduced pressure. The resulting residue was washed with hexane and ethyl acetate, and then recrystallized from hexane/ethyl acetate to give the desired product (32 mg) as crystals.

¹H-NMR (CDCl₃) δ 1.64-1.82 (2H, m), 2.29-2.69 (2H, m), 3.51-4.07 (2H, m), 4.48-4.75 (4H, m), 6.71 (1H, d), 7.00-7.15 (3H, m), 7.16-7.25 (2H, m), 7.33-7.58 (3H, m), 7.65 (1H, d), 7.78 (1H, d)

### Example 85

### 3-[2-Chloro-5-(3,4-dihydroquinolin-1(2H)-ylsulfonyl)phenyl]-2,4-dioxo-1,2,3,4-tetrahydroquinazoline-5-carboxamide

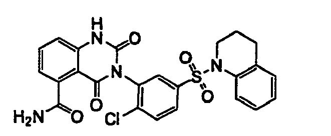

3-[2-chloro-5-(3,4-dihydroquinolin-1(2H)-ylsulfonyl)phenyl]-2,4-dioxo-1,2,3,4-tetrahydroquinazoline-5-carboxylic acid (80 mg), 1-[3-(dimethylamino)propyl]-3-ethylcarbodiimide hydrochloride (45 mg), 1-hydroxybenzotriazole (36 mg), N,N-diisopropylethylamine (0.1 ml) and ammonium carbonate (150 mg) were dissolved in DMF (3 ml), and the mixture was stirred at room temperature for 16 hours. The reaction solution was diluted with water, and then the resulting solid was separated by filtration and air-dried. The obtained solid was recrystallized from DMF/methanol to give the desired product (40 mg) as crystals.

¹H-NMR (DMSO-d₆) δ 1.60-1.70 (2H, m), 2.47-2.50 (2H, m), 3.72-3.80 (2H, m), 7.06 (1H, d), 7.08-7.13 (2H, m), 7.14-7.22 (1H, m), 7.24 (1H, d), 7.30 (1H, s), 7.51-7.59 (4H, m), 7.70 (1H, t), 7.81 (1H, d), 7.92 (1H, d)

### Example 86

### 5-Bromo-3-[2-chloro-5-(3,4-dihydroquinolin-1(2H)-ylsulfonyl)phenyl]-3,4-dihydroquinazolin-2(1H)-one

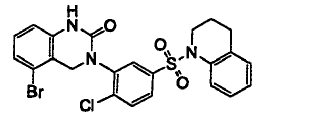

To a solution of ethyl (2-bromo-6-nitrobenzyl)[2-chloro-5-(3,4-dihydroquinolin-1(2H)-ylsulfonyl)phenyl]carbamate (4.67 g) and reduced iron (5.0 g) in ethanol (80 ml), concentrated hydrochloric acid (15 ml) was slowly added, and the mixture was stirred at 60°C for 1 hour. The reaction solution was diluted with an aqueous sodium hydrogen carbonate solution, and then extracted with ethyl acetate. The organic layer was dried over anhydrous magnesium sulfate, and then the solvent was distilled off under reduced pressure. The resulting residue was dissolved in ethanol (60 ml). To the reaction solution, potassium hydroxide (2.40 g) was added, and the mixture was stirred at 80°C for 5 hours. The reaction solution was diluted with ethyl acetate and 1 N hydrochloric acid, and then the organic layer was separated. The aqueous layer was extracted with ethyl acetate. The collected organic layer was dried over anhydrous magnesium sulfate, and then the solvent was distilled off under reduced pressure. The resulting residue was recrystallized from ethyl acetate to give the desired product (1.40 g) as crystals.

¹H-NMR (DMSO-d₆) δ 1.57-1.74 (2H, m), 2.36-2.52 (2H, m), 3.68-3.92 (2H, m), 4.38-4.53 (1H, m), 4.63-4.78 (1H, m), 6.86 (1H, dd), 7.04-7.25 (5H, m), 7.55-7.64 (2H, m), 7.77 (1H, d), 7.87 (1H, d), 9.93 (1H, s)

### Example 87

### 6-(2,4-Dioxo-1,4-dihydroquinazolin-3(2H)-yl)-N-methyl-N-pyridin-2-ylpyridine-2-carboxamide

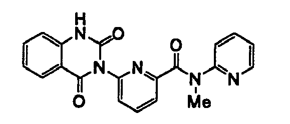

A solution of 6-(2,4-dioxo-1,4-dihydroquinazolin-3(2H)-yl)pyridine-2-carboxylic acid (171 mg) and thionyl chloride (0.2 ml) in DMF (2 ml) was stirred at 100°C for 3 hours, and then the reaction solution was concentrated under reduced pressure. The resulting residue was dissolved in THF (10 ml), and then cooled to 5°C. Triethylamine (0.7 ml) and 2-(methylamino)pyridine (0.7 ml) were added thereto, and the mixture was stirred at room temperature for 2 hours. Then, the reaction solution was diluted with ethyl acetate and an aqueous sodium hydrogen carbonate solution. The organic layer was separated, washed with saturated brine, and then dried over anhydrous magnesium sulfate. The solvent was distilled off under reduced pressure. The resulting residue was washed with ethyl acetate, purified with silica gel column chromatography, and recrystallized from ethyl acetate/methanol to give the desired product (95 mg) as crystals.

¹H-NMR (DMSO-d₆) δ 3.43 (3H, s), 7.04-7.11 (1H, m), 7.15-7.27 (3H, m), 7.51 (1H, dd), 7.57-7.78 (3H, m), 7.85-7.92 (1H, m), 8.06 (1H, t), 8.18-8.25 (1H, m), 11.57 (1H, br s)

### Example 87(1)

In the same manner as in Example 87, 5-chloro-4-(2,4-dioxo-1,4-dihydroquinazolin-3(2H)-yl)thiophene-2-carboxylic acid was reacted with N-methylaniline to obtain the following compound.

### 5-Chloro-4-(2,4-dioxo-1,4-dihydroquinazolin-3(2H)-yl)-N-methyl-N-phenylthiophene-2-carboxamide

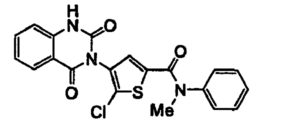

¹H-NMR (DMSO-d₆) δ 3.34 (3H, s), 6.87 (1H, s), 7.18-7.27 (2H, m), 7.42-7.55 (5H, m), 7.67-7.75 (1H, m), 7.90 (1H, dd), 11.67 (1H, s)

### Example 88

### 3-[5-(3,4-Dihydroquinolin-1(2H)-ylsulfonyl)pyridin-3-yl]quinazoline-2,4(1H,3H)-dione

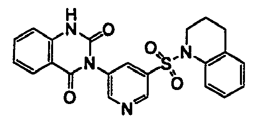

In the same manner as in Reference Example 99, the desired product was synthesized from 5-(3,4-dihydroquinolin-1(2H)-ylsulfonyl)pyridin-3-amine.

¹H-NMR (DMSO-d₆) δ 1.65-1.76 (2H, m), 2.46-2.50 (2H, m), 3.78-3.87 (2H, m), 7.09-7.17 (2H, m), 7.17-7.30 (3H, m), 7.60 (1H, d), 7. 69-7. 77 (1H, m), 7.96 (1H, dd), 8.34 (1H, t), 8.64 (1H, d), 8.84 (1H, d), 11.70 (1H, br s)

### Example 89

### 5-Chloro-4-[5-(hydroxymethyl)-2,4-dioxo-1,4-dihydroquinazolin-3(2H)-yl]-N-isobutyl-N-methylthiophene-2-carboxamide

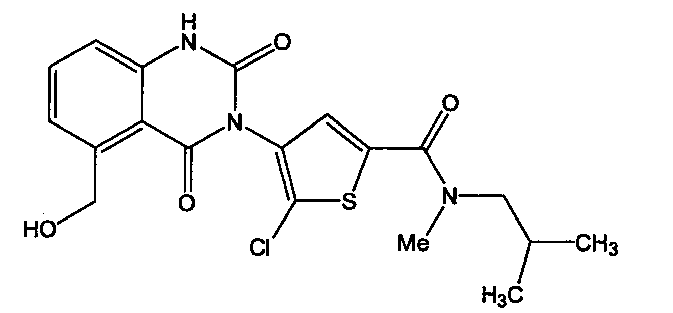

To a solution of 4-[5-({[tert-butyl(dimethyl)silyl]oxy}methyl)-2,4-dioxo-1,4-dihydroquinazolin-3(2H)-yl]-5-chloro-N-isobutyl-N-methylthiophene-2-carboxamide (75 mg) in THF (5 ml), was added a 1 N solution of tetrabutylammonium fluoride in THF (0.40 ml), and the mixture was stirred for 24 hours. The reaction solution was diluted with an aqueous ammonium chloride solution, and then extracted with ethyl acetate. The organic layer was dried over anhydrous magnesium sulfate, and then concentrated. The resulting residue was purified with silica gel column chromatography to give the desired product (43 mg) as amorphous.

¹H-NMR (DMSO-d₆) δ 0.86 (6H, d), 1.90-2.09 (1H, m), 3.17 (3H, br s), 3.25-3.40 (2H, m), 4.95 (2H, d), 5.30 (1H, t), 7.15 (1H, d), 7.48-7.55 (1H, d), 7.61 (1H, br s), 7.71 (1H, t), 11.62 (1H, br s)

### Examples 89 (1) to 89(2)

In the same manner as in Example 89, the following compounds were obtained from 4-[5-({[tert-butyl(dimethyl)silyl]oxy}methyl)-2,4-dioxo-1,4-dihydroquinazolin-3(2H)-yl]-5-chloro-N-isopropyl-N-methylthiophene-2-carboxamide or 4-[5-({[tert-butyl(dimethyl)silyl]oxy}methyl)-2,4-dioxo-1,4-dihydroquinazolin-3(2H)-yl]-5-chloro-N-(2-hydroxy-2-phenylethyl)-N-methylthiophene-2-carboxamide

### Example 89(1)

### 5-Chloro-4-[5-(hydroxymethyl)-2,4-dioxo-1,4-dihydroquinazolin-3(2H)-yl]-N-isopropyl-N-methylthiophene-2-carboxamide

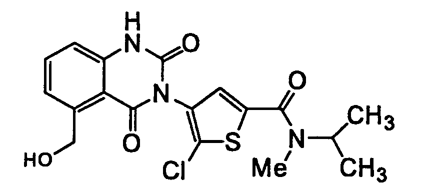

¹H-NMR (DMSO-d₆) δ 1.17 (6H, d), 2.97 (3H, br s), 4.57 (1H, br s), 4.95 (2H, d), 5.30 (1H, t), 7.15 (1H, d), 7.45-7.59 (2H, m), 7.71 (1H, t), 11.67 (1H, br s)

### Example 89(2)

### 5-Chloro-4-[5-(hydroxymethyl)-2,4-dioxo-1,4-dihydroquinazolin-3(2H)-yl]-N-(2-hydroxy-2-phenylethyl)-N-methylthiophene-2-carboxamide

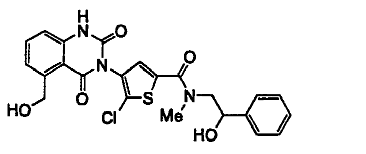

¹H-NMR (DMSO-d₆) δ 2.99-3.29 (3H, m), 3.39-3.89 (2H, m), 4.83-4.93 (1H, m), 4.93-5.05 (2H, m), 5.26-5.38 (1H, m), 5.50-5.94 (1H, m), 7.15 (1H, d), 7.21-7.44 (5H, m), 7.48-7.68 (2H, m), 7.71 (1H, t), 11.69 (1H, s)

### Example 90

### 3-[2-Chloro-5-(3,4-dihydroquinolin-1(2H)-ylsulfonyl)phenyl]-7-fluoroquinazoline-2,4(1H,3H)-dione

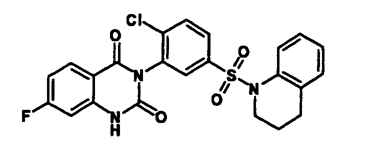

To a solution of 2-amino-N-[2-chloro-5-(3,4-dihydroquinolin-1(2H)-ylsulfonyl)phenyl]-4-fluorobenzamide (78 mg) and dichloromethane (5.1 ml), was added triphosgene (79 mg), and the mixture was heated under reflux for 80 minutes. The reaction mixture was poured into water, and extracted with dichloromethane. The organic layer was diluted with a saturated sodium bicarbonate solution and dried over anhydrous magnesium sulfate, and then the solvent was distilled off under reduced pressure. The residue was subjected to reversed-phase preparative HPLC (Gilson Inc., UniPoint System, YMC ODS column 30 × 75 mm). The fraction eluted with 0.1% TFA-containing acetonitrile/water (5 : 95 to 100 : 0) was concentrated under reduced pressure, and crystallized from ethyl acetate/diisopropyl ether. The crystals were collected by filtration to give the desired product (52 mg).

¹H-NMR (CDCl₃) δ 1.65-1.74 (2H, m), 2.51 (2H, t), 3.81 (2H, t), 6.73 (1H, dd), 6.97 (1H, dd), 7.01-7.05 (1H, m), 7.10 (1H, td), 7.17-7.23 (1H, m), 7.49 (1H, dd), 7.57 (1H, d), 7.65 (1H, d), 7.78 (1H, dd), 8.15 (1H, dd), 9.37 (1H, br s)

### Examples 90(1) to 90(10)

In the same manner as in Example 90, the following compounds were obtained from compounds disclosed in Reference Examples 5(91) to 5(94), 5(97), 5(98), 5(105) to 5(107) and 111.

### Example 90(1)

### 8-Chloro-3-(2-chloro-5-(3,4-dihydro-1(2H)-quinolinylsulfonyl)phenyl)-2,4(1H,3H)-quinazolinedione

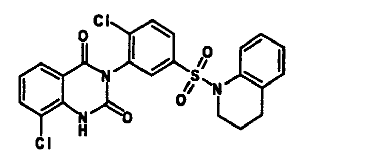

¹H-NMR (CDCl₃) δ 1.63-1.71 (2H, m), 2.49 (2H, t), 3.77-3.81 (2H, m), 7.03 (1H, d), 7.09 (1H, td), 7.17 (1H, d), 7.23 (1H, d), 7.49 (1H, dd), 7.55 (1H, d), 7.59 (1H, d), 7.72-7.78 (2H, m), 8.08 (1H, dd), 8.25 (1H, br s)

### Example 90(2)

### 3-(2-Chloro-5-(3,4-dihydro-1(2H)-quinolinylsulfonyl)phenyl)-8-methyl-2,4(1H,3H)-quinazolinedione

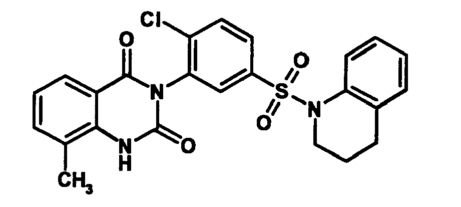

¹H-NMR (CDCl₃) δ 1.64-1.72 (2H, m), 2.50 (2H, td), 3.71-3.86 (2H, m), 7.03 (1H, d), 7.08 (1H, td), 7.16-7.21 (2H, m), 7.47 (1H), 7.45-7.46 (1H, m), 7.54 (1H, d), 7.61 (1H, d), 7.76 (1H, d), 8.01 (1H, d), 8.48 (1H, br s)

### Example 90(3)

### 6-Chloro-3- (2-chloro-5- (3, 4-dihydro-1 (2H) - quinolinylsulfonyl)phenyl)-2,4(1H,3H)-quinazolinedione

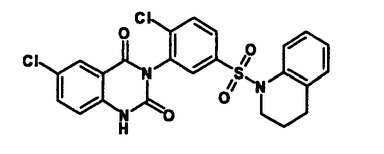

¹H-NMR (CDCl₃) δ 1.64-1.72 (2H, m), 2.43-2.56 (2H, m), 3.72-3.87 (2H, m), 6.99-7.04 (2H, m), 7.09 (1H, t), 7.19 (1H, t), 7.49 (1H, dd), 7.55 (1H, d), 7.54-7.62 (2H, m), 7.76 (1H, d), 8.08 (1H, d), 9.24 (1H, br s)

### Example 90(4)

### 3-(2-Chloro-5-(3,4-dihydro-1(2H)-quinolinylsulfonyl)phenyl)-6-methoxy-2,4(1H,3H)-quinazolinedione

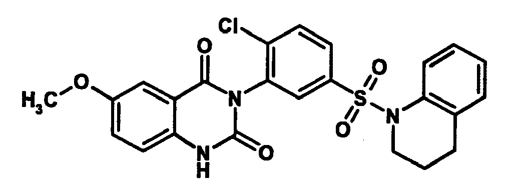

MS (ESI+, m/e) 498 (M+1)

### Example 90(5)

### 7-Chloro-3-[2-chloro-5-(3,4-dihydroquinolin-1(2H)-ylsulfonyl)phenyl]quinazoline-2,4(1H,3H)-dione

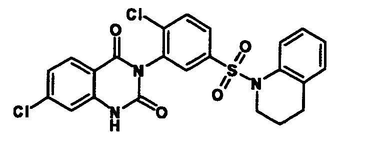

¹H-NMR (CDCl₃) δ 1.65-1.73 (2H, m), 2.51 (2H, t), 3.78-3.82 (2H, m), 7.02-7.12 (2H, m), 7.05 (1H, d), 7.16-7.20 (1H, m), 7.24 (1H, dd), 7.49 (1H, dd), 7.57 (1H, d), 7.64 (1H, d), 7.77 (1H, d), 8.06 (1H, d), 9.35 (1H, br s)

### Example 90(6)

### 3-[2-Chloro-5-(3,4-dihydroquinolin-1(2H)-ylsulfonyl)phenyl]-7-(trifluoromethyl)quinazoline-2,4(1H,3H)-dione

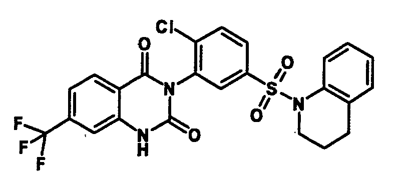

¹H-NMR (CDCl₃) δ 1.64-1.73 (2H, m), 2.50 (2H, t), 3.80 (2H, t), 7.03 (1H, dd), 7.09 (1H, td), 7.19 (1H, td), 7.31 (1H, s), 7.49-7.53 (2H, m), 7.57 (1H, d), 7.65 (1H, d), 7.76 (1H, dd), 8.27 (1H, dd), 9.30 (1H, br s)

### Example 90 (7)

### N-{3-[2-Chloro-5-(3,4-dihydroquinolin-1(2H)-ylsulfonyl)phenyl]-2,4-dioxo-1,2,3,4-tetrahydroquinazolin-6-yl}acetamide

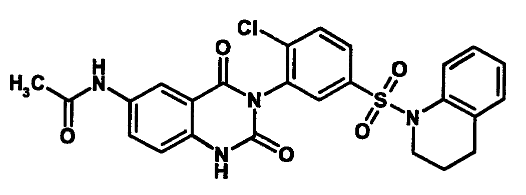

¹H-NMR (CDCl₃) δ 1.59-1.68 (2H, m), 2.11 (3H, s) 2.44 (2H, t), 3.75 (2H, dd), 6.93-7.00 (2H, m), 7.05 (1H, td), 7.14 (1H, t), 7.39 (1H, dd), 7.49 (1H, d), 7.68 (1H, d), 7.73 (1H, d), 7.88 (1H, dd), 8.03 (1H, s), 8.13 (1H, s), 10.05 (1H, br s)

### Example 90(8)

### 3-[3-(3,4-Dihydroquinolin-1(2H)-ylsulfonyl)phenyl]-6-fluoroquinazoline-2,4(1H,3H)-dione

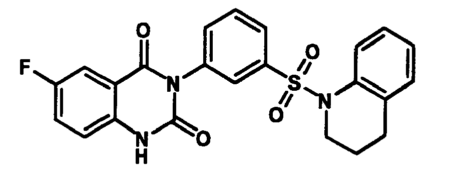

¹H-NMR (CDCl₃) δ 1. 65 (2H, ddd), 2.48 (2H, t), 3.76 (2H, t), 7.08 (2H, d), 7.17 (1H, td), 7.28 (1H, dd), 7.51 (1H, dt), 7.56-7.68 (5H, m), 7.80 (1H, d), 11.66 (1H, br)

### Example 90 (9)

### 3-[3-(3,4-Dihydroquinolin-1 (2H)-ylsulfonyl)phenyl]-8-methoxyquinazoline-2,4(1H,3H)-dione

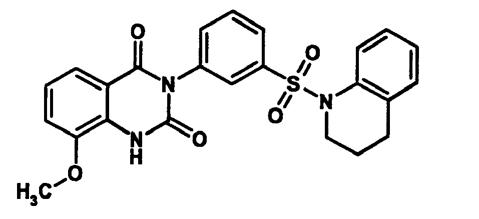

¹H-NMR (CDCl₃) δ 1.65 (2H, ddd), 2.47 (2H, t), 3.76 (2H, t), 3.91 (3H, s), 7.08 (2H, d), 7.15-7.21 (2H, m), 7.36 (1H, dd), 7.48-7.53 (2H, m), 7.57-7.64 (3H, m), 7.78-7.79 (1H, m), 10.99 (1H, br s)

### Example 90 (10)

### 3-[2-Chloro-5-(3,4-dihydroquinolin-1(2H)-ylsulfonyl)phenyl]-8-methoxyquinazoline-2,4(1H,3H)-dione

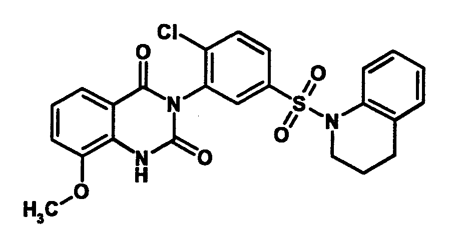

¹H-NMR (CDCl₃) δ 1.68 (2H, dt), 2.50 (2H, t), 3.78 (2H, t), 3.92 (3H, s), 7.10 (2H, d), 7.16-7.20 (1H, m), 7.22 (1H, t), 7.39 (1H, dd), 7.52-7.58 (3H, m), 7.80 (1H, d), 8.08 (1H, d), 11.18 (1H, br s)

### Example 91

### 3-(4-(3,4-Dihydro-1(2H)-quinolinylcarbonyl)-2-thienyl)-2,4(1H,3H)-quinazolinedione

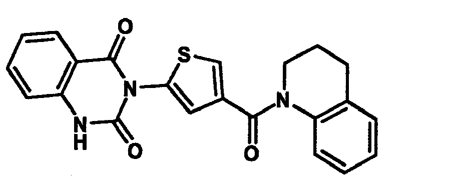

4-(3,4-Dihydro-1(2H)-quinolinylcarbonyl)thiophen-2-amine (88 mg) was dissolved in THF (3.4 ml). To a 0.15 N solution of DMAP in THF (3.4 ml), a 0.15 N solution of methyl 2-isocyanatobenzoate in THF (3.7 ml) was added at room temperature, and the mixture was stirred for 5 hours. A trisamine resin (Argonaut Technologies, Inc., 4.36 mol/g, 123 mg) was added thereto, and the mixture was further stirred at room temperature for 2 hours. The resin was filtered off, and then washed with THF. The filtrate and a washing liquor were combined, and concentrated under reduced pressure. To the residue was added a 1 N solution of potassium hydroxide in ethanol (6.8 ml), and the mixture was stirred at room temperature for 30 minutes. Then, the reaction mixture was neutralized with 0.5 N hydrochloric acid, and extracted with ethyl acetate. The reaction mixture was washed with a saturated sodium bicarbonate solution and saturated brine, and dried over anhydrous magnesium sulfate, and then the solvent was distilled off under reduced pressure. The residue was crystallized from ethyl acetate, and the crystals were collected by filtration and dried under reduced pressure to give the desired product (64 mg).

¹H-NMR (DMSO-d₆) δ 1.90-1.99 (2H, m), 2.80 (2H, t), 3.80 (2H, t), 7.01-7.12 (4H, m), 7.18-7.26 (3H, m), 7.67-7.33 (1H, m), 7.76 (1H, t), 7.93 (1H, dd), 11.63 (1H, br s)

### Examples 91(1) to 91(2)

In the same manner as in Example 91, 5-(3,4-dihydroquinolin-1(2H)-ylcarbonyl)thiophen-2-amine or 3-(3,4-dihydro-1(2H)-quinolinylcarbonyl)-1H-pyrazol-5-amine was reacted with methyl 2-isocyanatobenzoate to obtain the following compounds.

### Example 91(1)

### 3-[5-(3,4-Dihydroquinolin-1(2H)-ylcarbonyl)-2-thienyl]quinazoline-2,4(1H,3H)-dione

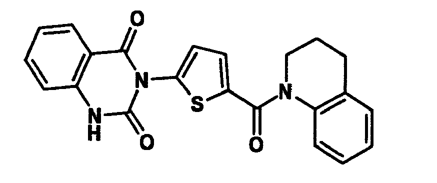

¹H-NMR (DMSO-d₆) δ 1.96 (2H, dt), 2.79 (2H, t), 3.86 (2H, t), 6.97 (1H, d), 7.03 (1H, d), 7.08-7.14 (2H, m), 7.15-7.26 (4H, m), 7.69 (1H, t), 7.92 (1H, d), 11.66 (1H, br s)

### Example 91(2)

### 3-(3-(3,4-Dihydro-1(2H)-quinolinylcarbonyl)-1H-pyrazol-5-yl)-2,4(1H,3H)-quinazolinedione

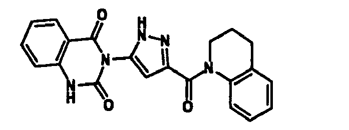

¹H-NMR (DMSO-d₆) δ 1.89 (2H, dt), 2.68 (2H, t), 3.83 (2H, t), 5.73 (1H, br s), 6.92-6.97 (3H, m), 7.02-7.06 (2H, m), 7.21-7.24 (1H, m), 7.46 (1H, t), 7.88 (1H, dd), 10.17 (1H, br s), 12.95 (1H, br s)

### Example 92

### 3-[2-Chloro-5-(2,3,4,5-tetrahydro-1H-1-benzazepin-1-ylsulfonyl)-3-thienyl]quinazoline-2,4(1H,3H)-dione

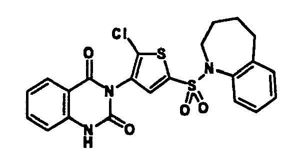

2-Chloro-5-(2,3,4,5-tetrahydro-1H-1-benzazepin-1-ylsulfonyl)thiophen-3-amine (57 mg) was dissolved in THF (1.7 ml). A 0.15 N solution of DMAP in THF (1.7 ml) and a 0.16 N solution of methyl 2-isocyanatobenzoate in THF (1.7 ml) were added thereto, and the mixture was stirred at room temperature for 7.5 hours. Then, a trisamine resin (Argonaut Technologies, Inc., 4.36 mmol/g, 58 mg) was added thereto, and the mixture was further stirred at room temperature for 2 hours. The resin was filtered off, and then washed with THF. The filtrate and a washing liquor were combined and concentrated under reduced pressure. The residue was diluted with acetonitrile, and heated at 75°C for 30 minutes, and then concentrated under reduced pressure.

The residue was purified with silica gel column chromatography (20 to 60% ethyl acetate/hexane), and the solvent was distilled off. Then, the residue was crystallized from diisopropyl ether to give the desired product (64 mg).

¹H-NMR (DMSO-d₆) δ 1.66 (2H, br s), 1.93 (2H, t), 2.65 (2H, t), 3.70 (2H, br s), 7.07 (1H, d), 7.16-7.23 (3H, m), 7.28 (1H, t), 7.34-7.40 (1H, m), 7.40 (1H, s), 7.63-7.69 (1H, m), 8.14 (1H, dd), 9.95 (1H, br s)

### Example 93

### 3-[3-(3,4-Dihydroquinolin-1(2H)-ylsulfonyl)phenyl]-2,4-dioxo-1,2,3,4-tetrahydroquinazoline-7-carboxylic acid

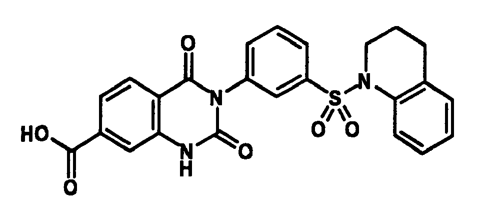

Methyl 3-[3-(3,4-dihydroquinolin-1(2H)-ylsulfonyl)phenyl]-2,4-dioxo-1,2,3,4-tetrahydroquinazoline-7-carboxylate (140 mg) was dissolved in THF (1.4 ml). A 1 N aqueous sodium hydroxide solution (5.7 ml) was added thereto, and the mixture was stirred at room temperature for 70 minutes. The reaction mixture was washed with dichloromethane (2.9 ml). The aqueous layer was neutralized with concentrated hydrochloric acid, and then extracted with dichloromethane containing a small amount of THF. The organic layer was washed with saturated brine, and dried over anhydrous magnesium sulfate, and then the solvent was distilled off under reduced pressure. The crystals, which were crystallized from ethyl acetate, were collected by filtration and dried under reduced pressure to give the desired product (80 mg).

¹H-NMR (DMSO-d₆) δ 1.66 (2H, ddd), 2.48 (2H, t), 3.77 (2H, t), 7.09 (2H, d), 7.17 (1H, td), 7.52 (1H, dt), 7.57-7.66 (3H, m), 7.72 (1H), 7.80 (1H, d), 7.83 (1H, t), 8.04 (1H, d), 11.77 (1H, s)

### Example 94

### 3-[3-(3,4-Dihydroquinolin-1(2H)-ylsulfonyl)phenyl]pyrido[2,3-d]pyrimidine-2,4(1H,3H)-dione

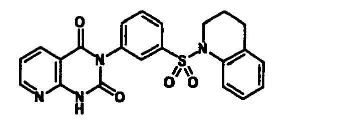

To a 0.5 N solution of methyl 2-aminonicotinate in dichloromethane (4.4 ml), was added a 1.5 N solution of triethylamine in dichloromethane (4.4 ml). Under nitrogen atmosphere, a 0.17 N solution of triphosgene in dichloromethane (4.4 ml) was added dropwise thereto at -78°C while stirring, and the mixture was further stirred at the same temperature for 10 minutes. The cooling bath was taken out, and then the reaction mixture was further stirred for 1 hour. The reaction solution was concentrated under reduced pressure, and the residue was diluted with THF (11 ml). A solution of 3-(3,4-dihydro-1(2H)-quinolinylsulfonyl)aniline (0.25 N) and DMAP (0.375 N) in THF (2.2 ml) was added dropwise thereto, and the mixture was stirred at room temperature for 25 hours. The insoluble triethylamine hydrochloride was filtered, and then the solvent was distilled off under reduced pressure. The residue was subjected to reversed-phase preparative HPLC (Gilson Inc., UniPoint System, YMC ODS column 30 × 75 mm). The fraction eluted with 0.1% TFA-containing acetonitrile/water (2 : 98 to 100 : 0) was concentrated under reduced pressure. The crystals, which were crystallized from methanol, were collected by filtration to give the desired product (37 mg).

¹H-NMR (DMSO-d₆) δ 1.66 (2H, dt), 2.47 (2H, t), 3.76 (2H, t), 7.00-7.09 (2H, m), 7. 18 (1H, dt), 7.31 (1H, dd), 7. 50 (1H, dt), 7.57-7.68 (3H, m), 7.85 (1H, t), 8.32 (1H, dd), 8.67 (1H, dd), 12.11 (1H, br s)

### Examples 94 (1) to 94(4)

In the same manner as in Example 94, the following compounds were respectively obtained from the reaction of dimethyl 2-aminoterephthalate and 3-(3,4-dihydro-1(2H)-quinolinylsulfonyl)aniline; methyl 2-amino-4, 5-dimethoxybenzoate and 3-(3,4-dihydro-1(2H)-quinolinylsulfonyl)aniline; dimethyl 3-aminophthalate and 2-chloro-5-(5,6,7,8-tetrahydro-4H-thieno[3,2-b]azepin-4-ylsulfonyl)thiophen-3-amine; or methyl 3-aminoisonicotinate and 3-(3,4-dihydro-1(2H)-quinolinylsulfonyl)aniline.

### Example 94(1)

### Methyl 3-[3-(3,4-dihydroquinolin-1(2H)-ylsulfonyl)phenyl]-2,4-dioxo-1,2,3,4-tetrahydroquinazoline-7-carboxylate

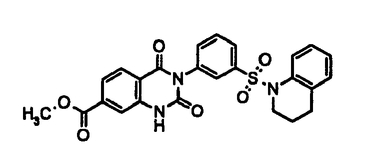

¹H-NMR (DMSO-d₆) δ 1.66 (2H, ddd), 2.48 (2H, t), 3.76 (2H, t), 3.92 (3H, s), 7.08-7.09 (2H, m), 7. 18 (1H, ddd), 7.52 (1H, dt), 7.57-7.69 (3H, m), 7.74 (1H, dd), 7.81-7.84 (2H, m), 8.07 (1H, d), 11.78 (1H, br s)

### Example 94(2)

### 3-[3-(3,4-Dihydroquinolin-1(2H)-ylsulfonyl)phenyl]-6, 7-dimethoxyquinazoline-2,4(1H,3H)-dione

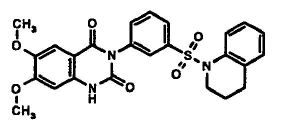

¹H-NMR (DMSO-d₆) δ 1.65 (2H, ddd), 2.47 (2H, t), 3.76 (2H, t), 3.80 (3H, s), 3.86 (3H, s), 6.73 (1H, s), 7.08-7.09 (2H, m), 7.18 (1H, dt), 7.29 (1H, s), 7.48 (1H, td), 7.57-7.62 (3H, m), 7.73 (1H, m), 11.41 (1H, br s)

### Example 94(3)

### Methyl 3-[2-chloro-5-(5,6,7,8-tetrahydro-4H-thieno[3,2-b]azepin-4-ylsulfonyl)-3-thienyl]-2,4-dioxo-1,2,3,4-tetrahydroquinazoline-5-carboxylate

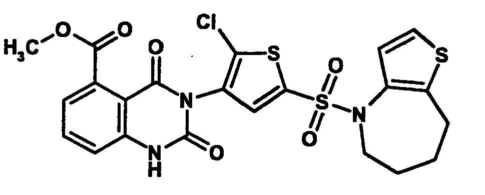

MS (ESI+, m/e) 552 (M+1)

### Example 94(4)

### 3-[3-(3,4-Dihydroquinolin-1(2H)-ylsulfonyl)phenyl]pyrido[3,4-d]pyrimidine-2,4(1H,3H)-dione

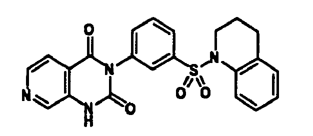

¹H-NMR (DMSO-d₆) δ 1.65 (2H, dt), 2.48 (2H, t), 3.76 (2H, t), 7.08-7.09 (2H, m), 7.17 (1H, td), 7.52 (1H, dt), 7.55-7.68 (3H, m), 7.81 (1H, dd), 7.83-7.84 (1H, m), 8.44 (1H, d), 8.63 (1H, s), 11.86 (1H, br s)

### Example 95

### 3-[3-(3,4-Dihydroquinolin-1(2H)-ylsulfonyl)phenyl]pteridine-2,4(1H,3H)-dione

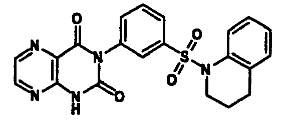

To a solution of 3-amino-N-[3-(3,4-dihydroquinolin-1(2H)-ylsulfonyl)phenyl]pyrazine-2-carboxamide (73 mg) and dichloroethane (3.6 ml), was added triphosgene (267 mg), and the mixture was heated with stirring at 75°C for 38 hours. A trisamine resin (Argonaut Technologies, Inc., 4.36 mol/g, 82 mg) was added thereto, and the mixture was further stirred at room temperature for 1.5 hours. The resin was filtered off, and then washed with THF. The filtrate and a washing liquor were combined, and concentrated under reduced pressure. The residue was subjected to reversed-phase preparative HPLC (Gilson Inc., UniPoint System, YMC ODS column 30 × 75 mm). The fraction eluted with 0.1% TFA-containing acetonitrile/water (2 : 98 to 100 : 0) was concentrated under reduced pressure. The crystals, which were crystallized from methanol/diisopropyl ether, were collected by filtration to give the desired product (18 mg) .

¹H-NMR (CDCl₃) δ 1.66 (2H, ddd), 2.48 (2H, t), 3.76 (2H, t, 7.08-7.10 (2H, m), 7.17 (1H, td), 7.51-7.54 (1H, m), 7.57-7.61 (1H, m), 7.64-7.66 (2H, m), 7.84-7.85 (1H, m), 8.59 (1H, d), 8.72 (1H, d), 12.37 (1H, br s)

### Example 96

### 3- [3- (3, 4-Dihydroquinolin-1 (2H) -ylsulfonyl) phenyl]-8-nitroquinazoline-2,4(1H,3H)-dione

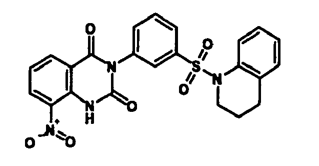

Methyl 3-(3,4-dihydro-1(2H)-quinolinylsulfonyl)aniline (163 mg), 2-amino-3-nitro-benzoic acid (197 mg) and potassium t-butoxide (117 mg) were mixed. The reaction mixture was subjected to microwave irradiation for 10 minutes at 140°C in a closed vessel using a Smith Synthesizer, a microwave reactor, available from Personal Chemistry. The reaction mixture was partitioned into water and ethyl acetate, and extracted with ethyl acetate. The organic layer was washed with saturated brine, and dried over anhydrous magnesium sulfate, and then the solvent was distilled off under reduced pressure. The residue was purified with silica gel column chromatography (20 to 55% ethyl acetate/hexane) to give the crude amide product (96 mg). The above-obtained product was dissolved in dichloroethane (4.2 ml). Triphosgene (268 mg) was added thereto, and the mixture was heated with stirring at 75°C for 38 hours. A trisamine resin (Argonaut Technologies, Inc., 4.36 mol/g, 97 mg) was added thereto, and the mixture was further stirred at room temperature for 1.5 hours. The resin was filtered off, and then washed with THF. The filtrate and a washing liquor were combined and concentrated under reduced pressure. The residue was subjected to reversed-phase preparative HPLC (Gilson Inc., UniPoint System, YMC ODS column 30 × 75 mm). The fraction eluted with 0.1% TFA-containing acetonitrile/water (2 : 98 to 100 : 0) was concentrated under reduced pressure. The crystals, which were crystallized from methanol/diisopropyl ether, were collected by filtration to give the desired product (20 mg).

¹H-NMR (CDCl₃) δ 1.66 (2H, ddd), 2.48 (2H, t), 3.77 (2H, t), 7.08-7.10 (2H, m), 7.18 (1H, td), 7.43 (1H, t), 7.51-7.71 (4H, m), 7.89 (1H, t), 8.38 (1H, dd), 8.51 (1H, dd), 10.88 (1H, br s)

### Example 97

### 3-[3-(1,2,3,4-Tetrahydronaphthalen-1-ylthio)phenyl]quinazoline-2,4(1H,3H)-dione

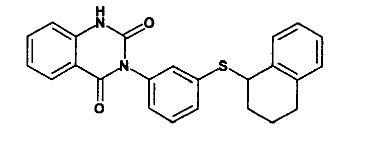

3-(1,2,3,4-Tetrahydronaphthalen-1-ylthio)aniline (0.87 g) and methyl 2-isocyanatobenzoate (0.75 g) were dissolved in pyridine (10 ml), and the mixture was stirred at 60°C for 18 hours. The reaction solution was concentrated under reduced pressure, and the obtained solid was washed with methanol to give the desired product (0.80 g) as a powder.

¹H-NMR (CDCl₃) δ 1.72-1.82 (1H, m), 1.92-2.06 (1H, m), 2.08-2.28 (2H, m), 2.68-2.90 (2H, m), 4.61 (1H, t), 7.06-7.26 (6H, m), 7.37-7.51 (4H, m), 7.60 (1H, t), 8.09 (1H, d), 9.84 (1H, s)

### Examples 97 (1) to 97(3)

In the same manner as in Example 97, 2-chloro-5-[(6-methoxy-2,3,4,5-tetrahydro-1H-1-benzazepin-1-yl)sulfonyl]aniline, 2-chloro-5-[(8-methoxy-2,3,4,5-tetrahydro-1H-1-benzazepin-1-yl)sulfonyl]aniline or 1-[(3-amino-4-chlorophenyl)sulfonyl]-1,2,3,4-tetrahydro-5H-1,4-benzodiazepin-5-one was reacted with methyl 2-isocyanatobenzoate to obtain the following compounds.

### Example 97(1)

### 3-{2-Chloro-5-[(6-methoxy-2,3,4,5-tetrahydro-1H-1-benzazepin-1-yl)sulfonyl]phenyl}quinazoline-2,4(1H,3H)-dione

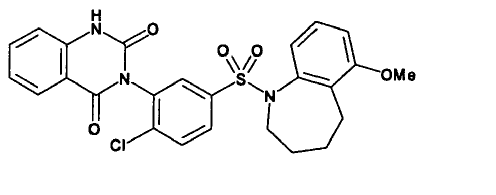

¹H-NMR (CDCl₃) δ 1.45-1.60 (2H, m), 1.79-1.93 (2H, m), 2.50-2.64 (2H, m), 3.50-3.90 (2H, br), 3.80 (3H, s), 6.90 (1H, d), 6.95-7.03 (2H, m), 7.08-7.27 (3H, m), 7.52 (1H, s), 7.55-7.63 (2H, m), 8.15 (1H, d), 9.86 (1H, s)

### Example 97(2)

### 3-{2-Chloro-5-[(8-methoxy-2,3,4,5-tetrahydro-1H-1-benzazepin-1-yl)sulfonyl]phenyl}quinazoline-2,4(1H,3H)-dione

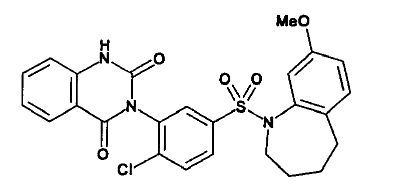

¹H-NMR (CDCl₃) δ 1.50-1.65 (2H, m), 1.75-1.87 (2H, m), 2.28-2.34 (2H, m), 3.65-3.90 (2H, br), 3.77 (3H, s), 6.92 (1H, d), 6.97-7.06 (2H, m), 7.10-7.31 (3H, m), 7.50 (1H, s), 7.54-7.61 (2H, m), 8.11 (1H, d), 9.94 (1H, s)

### Example 97(3)

### 3-{2-Chloro-5-[(5-oxo-2,3,4,5-tetrahydro-1H-1,4-benzodiazepin-1-yl)sulfonyl]phenyl}quinazoline-2,4(1H,3H)-dione

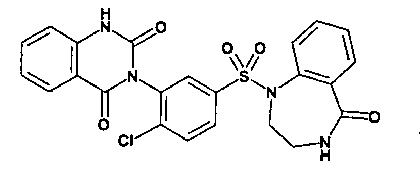

¹H-NMR (CDCl₃-DMSO-d₆) δ 3.00-3.28 (4H, m), 6.55 (1H, br), 7.26-7.34 (2H, m), 7.45-7.78 (7H, m), 7.90 (1H, s), 8.15 (1H, d), 10.56 (1H, s)

### Example 98

### 3-[3-(1,2,3,4-Tetrahydronaphthalen-1-ylsulfinyl)phenyl]quinazoline-2,4(1H,3H)-dione

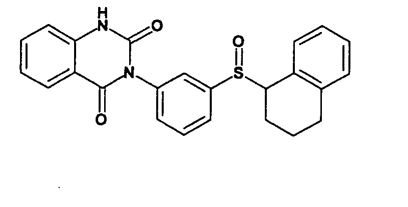

3-[3-(1,2,3,4-Tetrahydronaphthalen-1-ylthio)phenyl]quinazoline-2,4(1H,3H)-dione (0.20 g) was dissolved in DMF (2 ml). With ice cooling, a solution of 3-chloroperbenzoic acid (0.09 g) in DMF (1 ml) was added thereto, and the mixture was stirred for 1 hour with ice cooling. To the reaction solution was added water, and the mixture was extracted with ethyl acetate, dried over magnesium sulfate, and then concentrated under reduced pressure. The residue was purified with silica gel column chromatography (ethyl acetate/methanol), and recrystallized from diethyl ether to give the desired product (0.12 g) as a powder.

¹H-NMR (CDCl₃) δ 1.60-1.78 (1H, m), 2.02-2.17 (2H, m), 2.42-2.55 (1H, m), 2.64-2.72 (2H, m), 4.42-4.48 (1H, m), 7.02-7.32 (6H, m), 7.56-7.65 (4H, m), 7.72 (1H, s), 8.12 (1H, d), 9.00 (1H, s)

### Example 99

### 3-[3-(1,2,3,4-Tetrahydronaphthalen-1-ylsulfonyl)phenyl]quinazoline-2,4(1H,3H)-dione

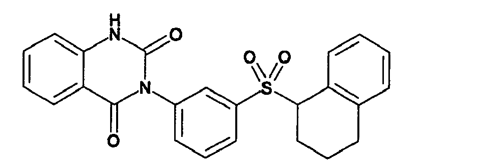

3-[3-(1,2,3,4-Tetrahydronaphthalen-1-ylthio)phenyl]quinazoline-2,4(1H,3H)-dione (0.20 g) was dissolved in DMF (2 ml). With ice cooling, a solution of 3-chloroperbenzoic acid (0.18 g) in DMF (1 ml) was added thereto, and the mixture was stirred for 1 hour with ice cooling. To the reaction solution was added water, and the mixture was extracted with ethyl acetate, dried over magnesium sulfate, and then concentrated under reduced pressure. The residue was purified with silica gel column chromatography (ethyl acetate/methanol), and recrystallized from diethyl ether to give the desired product (0.14 g) as a powder.

¹H-NMR (CDCl₃) δ 1.58-1.72 (1H, m) , 2.02-2.20 (2H, m), 2.40-2.56 (1H, m), 2.62-2.72 (2H, m), 4.44-4.48 (1H, m), 7.02 (1H, d), 7.11 (1H, t), 7.15-7.32 (4H, m), 7.57-7.65 (4H, m), 7.74 (1H, s), 8.13 (1H, d), 9.34 (1H, s)

### Example 100

### 3-[3-(3,4-Dihydroquinolin-1(2H)-ylsulfonyl)phenyl]-5-(1-hydroxy-1-methylethyl)quinazoline-2,4(1H,3H)-dione

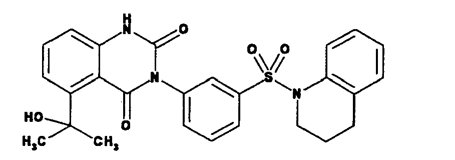

Methyl 3-[3-(3,4-dihydroquinolin-1(2H)-ylsulfonyl)phenyl]-2,4-dioxo-1,2,3,4-tetrahydroquinazoline-5-carboxylate (0.25 g) was dissolved in THF (20 ml). With ice cooling, a 3 N solution of methyl magnesium chloride in THF (1 ml) was added dropwise thereto, and the mixture was stirred at room temperature for 1 hour. Then, 0.1 N hydrochloric acid was added thereto, and the mixture was extracted with ethyl acetate, dried over magnesium sulfate, and then concentrated under reduced pressure. The residue was purified with silica gel column chromatography (hexane/ethyl acetate), and crystallized from diethyl ether to give the desired product (0.06 g) as a powder.

¹H-NMR (CDCl₃) δ 1.65 (6H, s), 1.67 (2H, m), 2.48 (2H, t), 3. 81 (2H, t), 6.73 (1H, s), 6.93 (1H, d), 7.00 (1H, d), 7.06 (1H, t), 7.17 (1H, t), 7.37 (1H, d), 7.44 (1H, d), 7.51-7.58 (3H, m), 7.63 (1H, d), 7.77 (1H, d), 9.89 (1H, s).

### Example 100(1)

In the same manner as in Example 100, the following compound was obtained from methyl 3-[2-chloro-5-(3,4-dihydroquinolin-1(2H)-ylsulfonyl)phenyl]-2,4-dioxo-1,2,3,4-tetrahydroquinazoline-5-carboxylate.

### 3-[2-Chloro-5-(3,4-dihydroquinolin-1(2H)-ylsulfonyl)phenyl]-5-(1-hydroxy-1-methylethyl)quinazoline-2,4(1H,3H)-dione

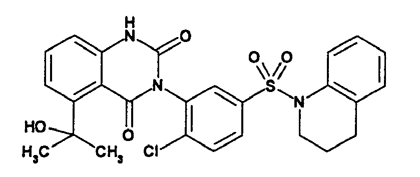

¹H-NMR (CDCl₃) δ 1.65 (6H, s), 1.66 (2H, m), 2.50 (2H, t), 3.81 (2H, t), 6.68 (1H, s), 6.98 (1H, d), 7.02 (1H, d), 7.09 (1H, t), 7.19 (1H, t), 7.41 (1H, d), 7.56-7.59 (4H, m), 7.78 (1H, t), 9.82 (1H, s).

### Example 101

### 3-[3-(3,4-Dihydroquinolin-1(2H)-ylsulfonyl)phenyl]-5-(hydroxymethyl)-3,4-dihydroquinazolin-2(1H)-one

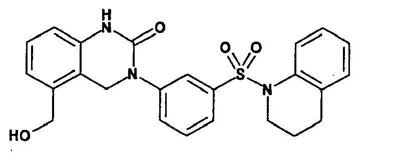

Methyl 3-[3-(3,4-dihydroquinolin-1(2H)-ylsulfonyl)phenyl]-2,4-dioxo-1,2,3,4-tetrahydroquinazoline-5-carboxylate (0.25 g) was dissolved in THF (5 ml). With ice cooling, lithium borohydride (0.15 g) was added thereto, and the mixture was stirred at 60°C for 2 hours. To the reaction solution was added 0.1 N hydrochloric acid, and the mixture was extracted with ethyl acetate, dried over magnesium sulfate, and then concentrated under reduced pressure. The residue was separated using silica gel column chromatography (hexane/ethyl acetate), and crystallized from diethyl ether to give the desired product (0.03 g) as a powder.

¹H-NMR (CDCl₃) δ 1.71 (2H, m), 2.50 (2H, t), 3.82 (2H, t), 4.55 (2H, br s), 4.81 (2H, s), 5.62 (1H, br s), 6.82 (1H, d), 6.98-7.22 (4H, m), 7.37-7.42 (3H, m), 7.60-7.68 (2H, m), 7.77 (1H, d), 8.70 (1H, br s)

### Example 102

### 3-[3-(3,4-Dihydroquinolin-1(2H)-ylsulfonyl)phenyl]-2,4-dioxo-1,2,3,4-tetrahydroquinazoline-5-carboxamide

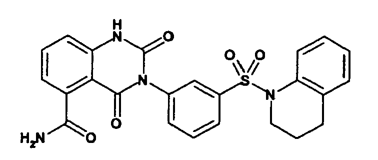

3-[3-(3,4-Dihydroquinolin-1(2H)-ylsulfonyl)phenyl]-2,4-dioxo-1,2,3,4-tetrahydroquinazoline-5-carboxylic acid (1.45 g), 1-hydroxybenzotriazole (0.40 g), N,N-diisopropylethylamine (1.29 g) and 1-ethyl-3-(3-dimethylaminopropyl)carbodiimide hydrochloride (0.86 g) were dissolved in DMF (20 ml). With ice cooling, a solution of ammonium carbonate (2.34 g) in water (10 ml) was added dropwise thereto, and the mixture was stirred at room temperature for 18 hours. Then, water was added thereto, and the mixture was extracted with ethyl acetate, dried over magnesium sulfate, and then concentrated under reduced pressure. The residue was crystallized from diethyl ether to give the desired product (0.76 g) as a powder.

¹H-NMR (CDCl₃-DMSO-d₆) δ 1.67 (2H, m), 2.50 (2H, t), 3.77 (2H, t), 6.33 (1H, br s), 6.62 (1H, br s), 7.02-7.11 (2H, m), 7.14-7.20 (2H, m), 7.30 (1H, d), 7.47-7.52 (3H, m), 7.57-7.63 (2H, m), 7.71 (1H, d), 11.59 (1H, s)

### Example 103

### 3-[3-(3,4-Dihydroquinolin-1(2H)-ylsulfonyl)phenyl]-2,4-dioxo-1,2,3,4-tetrahydroquinazoline-5-carbonitrile

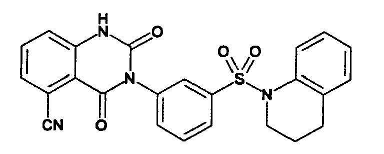

3-[3-(3,4-Dihydroquinolin-1(2H)-ylsulfonyl)phenyl]-2,4-dioxo-1,2,3,4-tetrahydroquinazoline-5-carboxamide (0.10 g) was dissolved in THF (5 ml). Triphosgene (0.15 g) was added thereto, and the mixture was stirred at 60°C for 18 hours. To the reaction solution was added water, and the mixture was extracted with ethyl acetate, dried over magnesium sulfate, and then concentrated under reduced pressure. The residue was purified with silica gel column chromatography (ethyl acetate/methanol), and recrystallized from ethyl acetate to give the desired product (0.05 g) as a powder.

¹H-NMR (CDCl₃-DMSO-d₆) δ 1.65 (2H, t), 2. 50 (2H, t), 3. 78 (2H, t), 7.07 (2H, d), 7.08-7.17 (2H, m), 7.46-7.63 (6H, m), 7.70 (1H, d), 10.34 (1H, s)

### Example 104

### N-(2-Aminoethyl)-3-[3-(3,4-dihydroquinolin-1(2H)-ylsulfonyl)phenyl]-2,4-dioxo-1,2,3,4-tetrahydroquinazoline-5-carboxamide

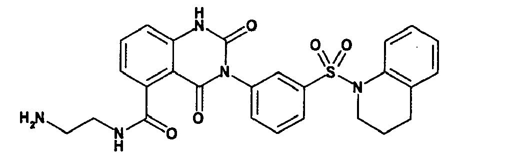

3-[3-(3,4-Dihydroquinolin-1(2H)-ylsulfonyl)phenyl]-2,4-dioxo-1,2,3,4-tetrahydroquinazoline-5-carboxylic acid (0.24 g), ethylenediamine (0.30 g), 1-hydroxybenzotriazole (0.14 g) and N,N-diisopropylethylamine (0.26 g) were dissolved in THF (5 ml). With ice cooling, 1-ethyl-3-(3-dimethylaminopropyl)carbodiimide hydrochloride (0.19 g) was added thereto, and the mixture was stirred at room temperature for 18 hours. Then, water was added thereto, and the mixture was extracted with ethyl acetate, dried over magnesium sulfate, and then concentrated under reduced pressure. The residue was purified with NH silica gel column chromatography (hexane/ethyl acetate), and crystallized from diethyl ether to give the desired product (0.10 g) as a powder.

¹H-NMR (CDCl₃) δ 1.70 (2H, m), 2.49 (2H, t), 2.85 (2H, br), 3.01 (2H, br), 3.75 (2H, br), 3.82 (2H, t), 7.00-7.10 (4H, m), 7.28-7.48 (2H, m), 7.62 (1H, t), 7.71-7.77 (2H, m), 7.80-8.02 (1H, m), 8.68 (1H, d), 9.06 (1H, br), 9.96 (1H, br)

### Example 105

### 3-[3-(3,4-Dihydroquinolin-1(2H)-ylsulfonyl)phenyl]-N-(2-hydroxyethyl)-2,4-dioxo-1,2,3,4-tetrahydroquinazoline-5-carboxamide

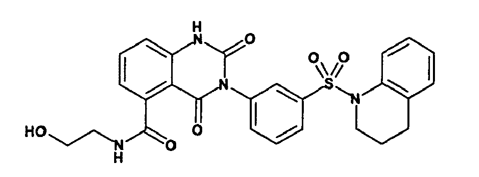

3-[3-(3,4-Dihydroquinolin-1(2H)-ylsulfonyl)phenyl]-2,4-dioxo-1,2,3,4-tetrahydroquinazoline-5-carboxylic acid (0.48 g), 2-aminoethanol (0.12 g), 1-hydroxybenzotriazole (0.21 g) and N,N-diisopropylethylamine (0.26 g) were dissolved in a mixed solution of THF/DMF (2 ml/2 ml). With ice cooling, 1-ethyl-3-(3-dimethylaminopropyl)carbodiimide hydrochloride (0.19 g) was added thereto, and the mixture was stirred at room temperature for 18 hours. Then, water was added thereto, and the mixture was extracted with ethyl acetate, dried over magnesium sulfate, and then concentrated under reduced pressure. The residue was purified with silica gel column chromatography (hexane/ethyl acetate), and recrystallized from ethyl acetate to give the desired product (0.38 g) as a powder.

¹H-NMR (CDCl₃) δ 1.70(2H, m), 2.49(2H, t), 3.01 (2H, t), 3.85 (4H, m), 7.00-7.10 (2H, m), 7.14-7.21 (2H, m), 7.33 (1H, t), 7.43 (1H, d), 7.63 (1H, t), 7.71 (1H, t), 7.75 (1H, d), 7.98 (1H, d), 8.02 (1H, s), 8.69 (1H, d), 9.07 (1H, s), 9.76 (1H, s)

### Example 106

### 3-[3-(3,4-Dihydroquinolin-1(2H)-ylsulfonyl)phenyl]-5-nitroquinazoline-2,4(1H,3H)-dione

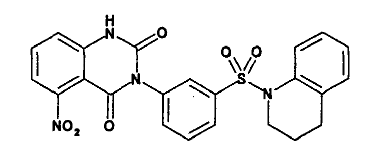

Methyl 2-[({[3-(3,4-dihydroquinolin-1(2H)-ylsulfonyl)phenyl]amino}carbonyl)amino]-6-nitrobenzoate (1.53 g) was dissolved in methanol (10 ml). Sodium methoxide (0.54 g) was added thereto, and the mixture was stirred at 60°C for 2 hours. Then, water was added thereto, and the mixture was extracted with ethyl acetate, dried over magnesium sulfate, and then concentrated under reduced pressure. The residue was crystallized from ethyl acetate to give the desired product (1.42 g) as a powder.

¹H-NMR (CDCl₃-DMSO-d₆) δ 1.61 (2H, m), 2.46 (2H, t), 3. 77 (2H, t), 7.05-7.20 (4H, m), 7.41-7.47 (3H, m), 7.52 (1H, t), 7.59 (1H, d), 7.68 (1H, t), 7.73 (1H, d), 12.01 (1H, s)

### Example 107

### 5-Amino-3-[3-(3,4-dihydroquinolin-1(2H)-ylsulfonyl)phenyl]quinazoline-2,4(1H,3H)-dione

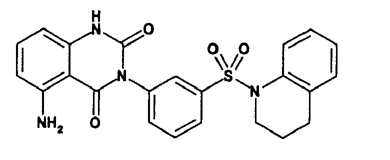

3-[3-(3,4-Dihydroquinolin-1(2H)-ylsulfonyl)phenyl]-5-nitroquinazoline-2,4(1H,3H)-dione (1.34 g) was dissolved in methanol (20 ml). 10% Palladium carbon (50% water content, 1.0 g) was added thereto, and the mixture was stirred under hydrogen atmosphere at room temperature for 2 hours. Palladium carbon was filtered off, and then the filtrate was concentrated under reduced pressure, and crystallized from ethyl acetate to give the desired product (1.19 g) as a powder.

¹H-NMR (CDCl₃-DMSO-d₆) δ 1.67 (2H, m), 2.48 (2H, t), 3.79 (2H, t), 6.24 (2H, br s), 6.34 (2H, dd), 6.99 (1H, d), 7.06 (1H, t), 7.17 (1H, t), 7.22 (1H, t), 7.44-7.51 (3H, m), 7.57 (1H, m), 7.75 (1H, d), 10.68 (1H, s)

### Example 108

### N-{3-[3-(3,4-Dihydroquinolin-1(2H)-ylsulfonyl)phenyl]-2,4-dioxo-1,2,3,4-tetrahydroquinazolin-5-yl}acetamide

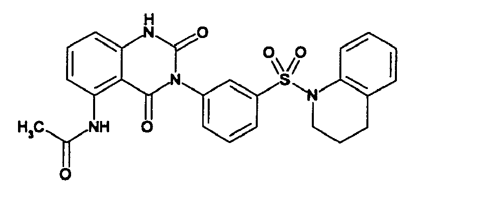

5-Amino-3-[3-(3,4-dihydroquinolin-1(2H)-ylsulfonyl)phenyl]quinazoline-2,4(1H,3H)-dione (0.22 g) was dissolved in pyridine (5 ml). With ice cooling, a solution of acetyl chloride (0.08 g) in THF (1 ml) was added dropwise thereto, and the mixture was stirred at room temperature for 16 hours. Then, water was added thereto, and the precipitated solid was collected by filtration and washed with ethyl acetate to give the desired product (0.19 g) as a powder.

¹H-NMR (CDCl₃-DMSO-d₆) δ 1.69 (2H, m), 2.19 (3H, s), 2. 51 (2H, t), 3. 81 (2H, t), 6.90 (1H, d), 7.02 (1H, t), 7.07 (1H, t), 7.18 (1H, t), 7.44-7. 59 (5H, m), 7.75 (1H, d), 8.43 (1H, d), 11.48 (1H, s), 11.64 (1H, s)

### Example 109

### 3-[3-(3,4-Dihydroquinolin-1(2H)-ylsulfonyl)phenyl]-5-(1H-tetrazol-1-yl)quinazoline-2,4(1H,3H)-dione

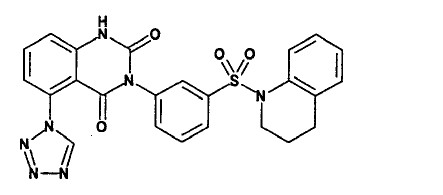

5-Amino-3-[3-(3,4-dihydroquinolin-1(2H)-ylsulfonyl) phenyl] quinazoline-2,4 (1H, 3H) -dione (0.22 g) was dissolved in acetic acid (5 ml). Sodium azide (0.13 g) was added thereto, and the mixture was stirred at 60°C for 18 hours. To the reaction solution was added water, and the mixture was extracted with ethyl acetate, dried over magnesium sulfate, and then concentrated under reduced pressure. The residue was crystallized by adding water, and the crystals were collected by filtration and washed with ethyl acetate to give the desired product (0.21 g) as a powder.

¹H-NMR (CDCl₃-DMSQ-d₆) δ 1.54 (2H, m), 2.37 (2H, t), 3.73 (2H, t), 6.98 (1H, d), 7.05 (1H, t), 7. 14 (1H, t), 7.22 (1H, d), 7.36-7.39 (2H, m), 7.48 (1H, t), 7.51-7.60 (2H, m), 7.69-7.76 (2H, m), 8.84 (1H, s), 11.96 (1H, s)

### Example 110

### 5-Acetyl-3-[3-(3,4-dihydroquinolin-1(2H)-ylsulfonyl)phenyl]quinazoline-2,4(1H,3H)-dione

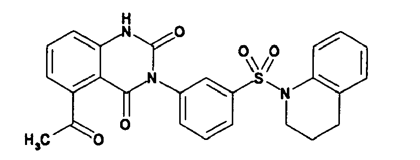

3-[3-(3,4-Dihydroquinolin-1(2H)-ylsulfonyl)phenyl]-2,4-dioxo-1,2,3,4-tetrahydroquinazoline-5-carbonitrile (0.05 g) was suspended in THF (2 ml). With ice cooling, a 3 N solution of methyl magnesium chloride in THF (0.1 ml) was added thereto, and the mixture was stirred at room temperature for 10 minutes. Then, 0.1 N hydrochloric acid was added to the reaction solution, and the mixture was extracted with ethyl acetate, dried over magnesium sulfate, and then concentrated under reduced pressure. The residue was crystallized from ethyl acetate/diethyl ether to give the desired product (0.015 g) as a powder.

¹H-NMR (CDCl₃-DMSO-d₆) δ 1. 66 (2H, m), 2.49 (2H, t), 2.33 (3H, s), 3.77 (2H, t), 6.94-7.02 (2H, m), 7.05 (1H, t), 7.16 (1H, t), 7.24 (1H, d), 7.39-7.60 (5H, m), 7.73 (1H, d), 11.53 (1H, s)

### Example 111

### 3-[3-(3,4-Dihydroquinolin-1(2H)-ylsulfonyl)phenyl]-5-(1H-tetrazol-5-yl)quinazoline-2,4(1H,3H)-dione

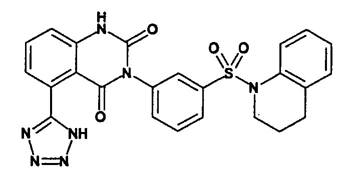

3-(3,4-Dihydro-1(2H)-quinolinylsulfonyl)aniline (2.88 g) and N,N-diisopropylethylamine (1.29 g) were dissolved in THF (20 ml). With ice cooling, triphosgene (1.04 g) was added thereto, and the mixture was stirred for 0.5 hour with ice cooling. Insolubles were filtered off, and the filtrate was concentrated under reduced pressure. The resulting residue was dissolved in pyridine (20 ml), methyl 2-amino-6-(1H-tetrazol-5-yl)benzoate (1.10 g) was added thereto, and then the mixture was stirred at 60°C for 18 hours. To the reaction solution was added water, and the mixture was extracted with ethyl acetate, dried over magnesium sulfate, and then concentrated under reduced pressure. The residue was purified with silica gel column chromatography (hexane/ethyl acetate), and recrystallized from ethyl acetate to give the desired product (1.10 g) as a powder.

¹H-NMR (CDCl₃-DMSO-d₆) δ 1.65 (2H, m), 2.44 (2H, t), 3.78 (2H, t), 6.99 (1H, d), 7.07 (1H, t), 7.16 (1H, t), 7.43-7.59 (6H, m), 7.67-7.73 (2H, m), 11.79 (1H, s)

### Preparation Example 1

According to a conventional method, tablets were prepared using the compound of Example 6(4) (100 mg), lactose (165 mg), corn starch (25 mg), polyvinyl alcohol (4 mg) and magnesium stearate (1 mg).

### Preparation Example 2

The compound of Example 6(4) (5 g) was dissolved in distilled water for injection, to make 100 ml of a total volume. The solution was aseptically filtered using a 0.22 µm membrane filter (manufactured by Sumitomo Electric Industries, Ltd. or Sartorius Co., Ltd.). The washing sterilizer was poured into each vial in an amount of 2 ml, and the reaction mixture was freeze-dried according to a conventional method to prepare 100 mg/vial of freeze-dried injection preparations.

### Preparation Example 3

According to a conventional method, tablets were prepared using the compound of Example 8(1) (100 mg), lactose (165 mg), corn starch (25 mg), polyvinyl alcohol (4 mg) and magnesium stearate (1 mg).

### Preparation Example 4

The compound of Example 8(1) (5 g) was dissolved in distilled water for injection, to make 100 ml of a total volume. The solution was aseptically filtered using a 0.22 µm membrane filter (manufactured by Sumitomo Electric Industries, Ltd. or Sartorius Co., Ltd.). The washing sterilizer was poured into each vial in an amount of 2 ml, and the reaction mixture was freeze-dried according to a conventional method to prepare 100 mg/vial of freeze-dried injection preparations.

### Experimental Example

### (1) Production of ¹²⁵ I-Leuprorelin

10 µL of a 3 × 10⁻⁴ M aqueous solution of leuprorelin and 10 µL of 0.01 mg/mL lactoperoxidase were placed in a tube, and 10 µL (37 MBq) of a Na¹²⁵I solution was added thereto. After stirring, 10 µL of 0.001% H₂O₂ was added to allow the mixture to undergo a reaction at room temperature for 20 minutes. 700 µL of a 0.05% TFA solution was added to stop the reaction, and purification by reversed phase HPLC was carried out. The conditions for HPLC are described below. ¹²⁵ I-leuprorelin was eluted at a retention time of 26 to 27 minutes.
Column: TSKgel ODS-80™ (TM refers to a registered trademark. The same as above)
CTR (4.6 mm × 10 cm) eluent:
Solvent A (0.05% TFA)
Solvent B (40% CH₃CN-0.05% TFA)
0^{th} minute (100% Solvent A) - 3^{rd} minute (100% Solvent A) - 7^{th} minute (50% Solvent A + 50% Solvent B) - 40^{th} minute (100% Solvent B)
Elution temperature: Room temperature
Elution rate: 1 mL/min

### (2) Production of CHO (Chinese Hamster Ovary) cell membrane fraction containing human GnRH receptor

Human GnRH receptor-expressing CHO cells were cultured until the cells were reached a 90% confluent state in a T-150 flask. The cells were separated using 0.02% EDTA-PBS and were centrifuged at 500 × g for 5 minutes. A homogenate buffer for cells (10 mM NaHCO₃, 1 mM EDTA, 1 mM PMSF, 0.5 µg/mL Pepstatin, 2 µg/mL Leupeptin, pH 7.4) was added to the cell pellet, and the mixture was homogenized with a Polytron homogenizer. After centrifugation of the homogenate at 4°C and at 1000 × g for 10 minutes, the supernatant was placed in an ultracentrifuge tube and was centrifuged at 4°C and at 49,300 × g for 1 hour to obtain a precipitate of a membrane fraction. This precipitate was suspended in an assay buffer (50 mM Tris-HCl, 150 mM NaCl, 5 mM EDTA, pH 7.4) and then dispensed and stored at -80°C, so that the suspension can be thawed every time it is taken for use.

### (3) Measurement of the binding inhibitory rate of ¹²⁵I-Leuprorelin

A ¹²⁵ I-leuprorelin binding assay was carried out using 200 µL of an assay buffer containing 10 µg of human GnRH receptor membrane fraction, 800 pM of ¹²⁵I-leuprorelin and 10 nM of the testing compound.

The non-specific binding radioactivity was measured in the presence of 1 µM of non-labeled leuprorelin. After leaving the mixture to stand at room temperature for 1 hour, ¹²⁵I-leuprorelin was captured on a GF/C filter (PerkinElmer, Inc.), and the filter was washed with ice-cold 50 mM Tris-HCl (pH 7.4) using a cell harvester. After drying the filter, a liquid scintillation cocktail for microplate was added, and the membrane-bound radioactivity was measured using a TopCount.

The value of (TB-SB)/(TB-NSB)x100 (SB: radioactivity upon addition of the compound, TB: maximum binding radioactivity, NSB: non-specific binding radioactivity) was calculated to determine the binding inhibitory rate in the presence of 10 nM of each testing material. The results are presented in the following.

**[Table 1]**

| Example No. | Binding inhibitory ratio (%) |
|---|---|
| 6(4) | 88.5 |
| 6(16) | 81.8 |
| 7(5) | 95.6 |
| 7(8) | 87.6 |
| 7(22) | 75.5 |
| 7(70) | 94.5 |
| 8 | 84.7 |
| 8 (1) | 91.7 |
| 14 | 88.9 |
| 21(12) | 84.2 |
| 30 | 98.0 |
| 31 | 98.2 |
| 36 | 96.7 |
| 42 | 77.4 |
| 46(1) | 98.2 |
| 47 | 94.8 |
| 73 | 79.0 |
| 73 (1) | 91.5 |
| 73(2) | 93.8 |
| 73(4) | 82.2 |
| 73(5) | 95.6 |
| 85 | 94.0 |
| 89 | 76.2 |
| 100(1) | 99.9 |

### Industrial Applicability

The compound of the invention has excellent antagonistic activity against gonadotropin releasing hormone. Moreover, the compound shows good oral absorbability and is excellent in the aspects of stability and pharmacokinetics. The compound also has low toxicity and is excellent in the aspect of safety. Therefore, it can be used, for example, as a prophylactic or therapeutic agent for hormone-dependent diseases. Specifically, for example, the compound is effective as a medicine such as a prophylactic or therapeutic agent for sex hormone-dependent cancers (e.g., prostate cancer, uterine cancer, breast cancer, pituitary cancer, etc.), prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease and the like, as an agent for birth control (e.g., contraceptive, etc.), a therapeutic agent for infertility, a menstrual regulator, a prophylactic and/or therapeutic agent for irritable bowel syndrome, or a preventive agent for post-operative recurrence of sex hormone-dependent cancers; and is also effective for regulation of animal's estrus, improvement in table meat quality and animal growth regulation in the field of stockbreeding, and also as a promoting agent for fish spawning in the field of fishery.

The invention of the present application is based on Japanese Patent Application No. 2003-298637, the disclosure of which is incorporated herein in its entirety by reference.

## Claims

1. A gonadotropin releasing hormone antagonist which comprises a compound represented by the formula: 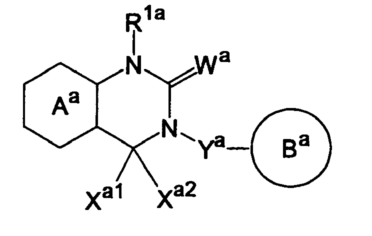 wherein R^{1a} is a hydrocarbon group which may be substituted or a hydrogen atom,
ring A^{a} is a 6-membered aromatic ring which may be further substituted,
ring B^{a} is a homocyclic or heterocyclic ring which may be further substituted,
W^{a} is an oxygen atom or a sulfur atom,
X^{a1} and X^{a2}, which may be identical or different, are each a hydrogen atom, a hydrocarbon group which may be substituted or a heterocyclic group which may be substituted, or X^{a1} and X^{a2} together may form an oxygen atom, a sulfur atom, or NR^{3a} (wherein R^{3a} is a hydrocarbon group which may be substituted or a hydrogen atom), and
Y^{a} is C₁₋₆ alkylene which may be substituted or a bond, or a salt or prodrug thereof.

2. The gonadotropin releasing hormone antagonist according to claim 1, wherein X^{a1} and X^{a2} together form an oxygen atom, a sulfur atom, or NR^{3a} (wherein R^{3a} is a hydrocarbon group which may be substituted or a hydrogen atom).

3. The gonadotropin releasing hormone antagonist according to claim 1 or 2, which is a prophylactic and/or therapeutic agent for prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease, infertility, irritable bowel syndrome, prostate cancer, uterine cancer, breast cancer or pituitary tumor, a reproduction regulator, a contraceptive, an ovulation inducing agent, or a preventive agent for post-operative recurrence of sex hormone-dependent cancers.

4. A method for prevention and/or treatment of prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease, infertility or irritable bowel syndrome, a method for reproduction regulation, a method for contraception, or a method for ovulation induction, which comprises administering an effective amount of a compound represented by the formula: 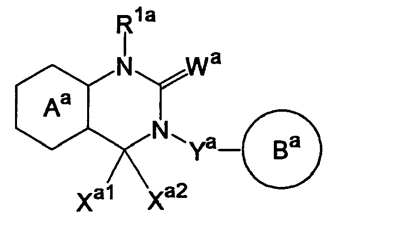 wherein R^{1a} is a hydrocarbon group which may be substituted or a hydrogen atom,
ring A^{a} is a 6-membered aromatic ring which may be further substituted,
ring B^{a} is a homocyclic or heterocyclic ring which may be further substituted,
W^{a} is an oxygen atom or a sulfur atom,
X^{a1} and X^{a2}, which may be identical or different, are each a hydrogen atom, a hydrocarbon group which may be substituted or a heterocyclic group which may be substituted, or X^{a1} and X^{a2} together may form an oxygen atom, a sulfur atom, or NR^{3a} (wherein R^{3a} is a hydrocarbon group which may be substituted or a hydrogen atom), and
Y^{a} is C₁₋₆ alkylene which may be substituted or a bond, or a salt or prodrug thereof to a mammal.

5. The method according to claim 4, wherein X^{a1} and X^{a2} together form an oxygen atom, a sulfur atom, or NR^{3a} (wherein R^{3a} is a hydrocarbon group which may be substituted or a hydrogen atom).

6. Use of a compound represented by the formula: 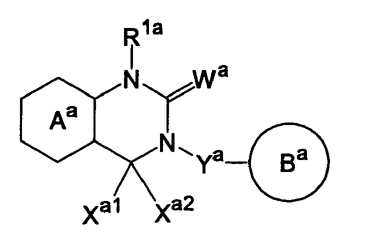 wherein R^{1a} is a hydrocarbon group which may be substituted or a hydrogen atom,
ring A^{a} is a 6-membered aromatic ring which may be further substituted,
ring B^{a} is a homocyclic or heterocyclic ring which may be further substituted,
W^{a} is an oxygen atom or a sulfur atom,
X^{a1} and X^{a2}, which may be identical or different, are each a hydrogen atom, a hydrocarbon group which may be substituted or a heterocyclic group which may be substituted, or X^{a1} and X^{a2} together may form an oxygen atom, a sulfur atom, or NR^{3a} (wherein R^{3a} is a hydrocarbon group which may be substituted or a hydrogen atom), and
Y^{a} is C₁₋₆ alkylene which may be substituted or a bond, or a salt or prodrug thereof for the manufacture of a prophylactic and/or therapeutic agent for prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease, infertility or irritable bowel syndrome, a reproduction regulating agent, a contraceptive, or an ovulation inducing agent.

7. The use according to claim 6, wherein X^{a1} and X^{a2} together form an oxygen atom, a sulfur atom, or NR^{3a} (wherein R^{3a} is a hydrocarbon group which may be substituted or a hydrogen atom).

8. A compound represented by Formula (I-a) : 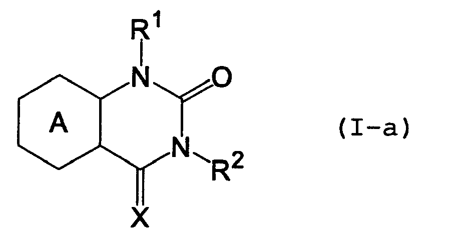 wherein ring A is a 6-membered aromatic ring which may be further substituted,
X is an oxygen atom, a sulfur atom or NR³ (wherein R³ is a hydrocarbon group which may be substituted or a hydrogen atom),
R¹ is a hydrocarbon group which may be substituted or a hydrogen atom, and
R² is
(1) a group represented by the formula: 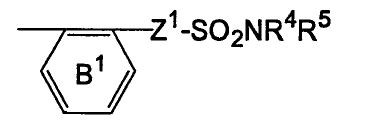 [wherein ring B¹ is a benzene ring which may be further substituted; Z¹ is C₁₋₆ alkylene which may be substituted or a bond; R⁴ is (a) a hydrogen atom, (b) a hydrocarbon group which may be substituted, or (c) a heterocyclic group which may be substituted; and R⁵ is (a) a hydrocarbon group which may be substituted, or (b) a heterocyclic group which may be substituted; or R⁴ and R⁵ may form a ring together with the adjacent nitrogen atom],
(2) a group represented by the formula: 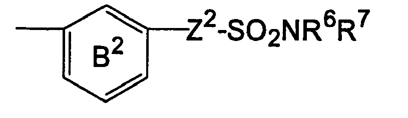 [wherein ring B² is a benzene ring which may be further substituted; Z² is C₁₋₆ alkylene which may be substituted or a bond; R⁶ is (a) a hydrogen atom, (b) a hydrocarbon group which may be substituted, or (c) a heterocyclic group which may be substituted; and R⁷ is (a) a hydrocarbon group which may be substituted, or (b) a heterocyclic group which may be substituted; or R⁶ and R⁷ may form a ring together with the adjacent nitrogen atom],
(3) a group represented by the formula: 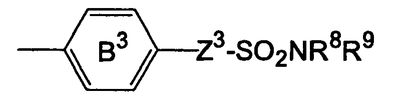 [wherein ring B³ is a benzene ring which may be further substituted; Z³ is C₁₋₆ alkylene which may be substituted or a bond; R⁸ is (a) a hydrogen atom, (b) a hydrocarbon group which may be substituted, or (c) a heterocyclic group which may be substituted; and R⁹ is (a) a hydrocarbon group which may be substituted, or (b) a heterocyclic group which may be substituted; or R⁸ and R⁹ may form a ring together with the adjacent nitrogen atom, provided that when R⁸ is a hydrogen atom, R⁹ is (a) an aryl group which may be substituted, (b) an aralkyl group which may be substituted, or (c) an alkyl group substituted with a heterocyclic group which may be substituted],
(4) a group represented by the formula: 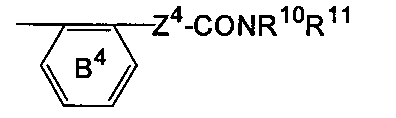 [wherein ring B⁴ is a benzene ring which may be further substituted; Z⁴ is C₁₋₆ alkylene which may be substituted or a bond; R¹⁰ is (a) a hydrogen atom, (b) a hydrocarbon group which may be substituted, or (c) a heterocyclic group which may be substituted; and R¹¹ is (a) an aromatic group which may be substituted, (b) an aralkyl group which may be substituted, or (c) an alkyl group substituted with a heterocyclic group which may be substituted; or R¹⁰ and R¹¹ may form, together with the adjacent nitrogen atom, a heterocyclic ring comprising carbon and nitrogen atoms as ring-constituting moieties],
(5) a group represented by the formula: 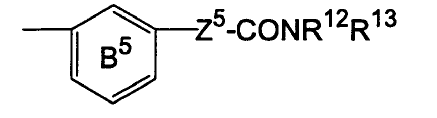 [wherein ring B⁵ is a benzene ring which may be further substituted; Z⁵ is C₁₋₆ alkylene which may be substituted or a bond; and R¹² and R¹³, which may be identical or different, are each (a) a hydrogen atom, (b) a hydrocarbon group which may be substituted, or (c) a heterocyclic group which may be substituted; or R¹² and R¹³ may form a ring together with the adjacent nitrogen atom],
(6) a group represented by the formula: 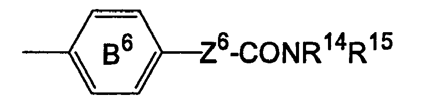 [wherein ring B⁶ is a benzene ring which may be further substituted; Z⁶ is C₁₋₆ alkylene which may be substituted or a bond; and R¹⁴ and R¹⁵, which may be identical or different, are each (a) a hydrocarbon group which may be substituted, or (b) a heterocyclic group which may be substituted; or R¹⁴ and R¹⁵ may form a ring together with the adjacent nitrogen atom],
(7) a group represented by the formula: 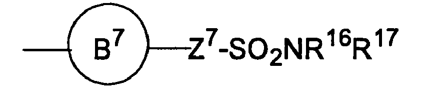 [wherein ring B⁷ is a C₁₀₋₁₄ aryl ring which may be further substituted, or an aromatic heterocycle which may be further substituted; Z⁷ is C₁₋₆ alkylene which may be substituted or a bond; R¹⁶ is (a) a hydrogen atom, (b) a hydrocarbon group which may be substituted, or (c) a heterocyclic group which may be substituted; and R¹⁷ is (a) a hydrocarbon group which may be substituted, or (b) a heterocyclic group which may be substituted; or R¹⁶ and R¹⁷ may form a ring together with the adjacent nitrogen atom],
(8) a group represented by the formula: 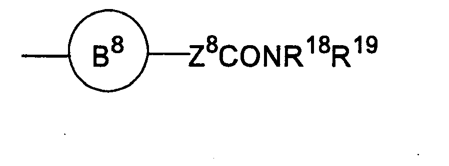 [wherein ring B⁸ is a C₁₀₋₁₄ aryl ring which may be further substituted, or an aromatic heterocycle which may be further substituted; Z⁸ is C₁₋₆ alkylene which may be substituted or a bond; R¹⁸ is (a) a hydrogen atom, (b) a hydrocarbon group which may be substituted, or (c) a heterocyclic group which may be substituted; and R¹⁹ is (a) a hydrocarbon group which may be substituted, or (b) a heterocyclic group which may be substituted; or R¹⁸ and R¹⁹ may form a ring together with the adjacent nitrogen atom], or
(9) a group represented by the formula: 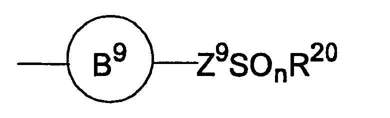 [wherein ring B⁹ is a C₆₋₁₄ aryl ring which may be further substituted, or an aromatic heterocycle which may be further substituted, provided that when X is an oxygen atom, ring B⁹ is not an indole ring; Z⁹ is C₁₋₆ alkylene which may be substituted or a bond; R²⁰ is a hydrocarbon group which may be substituted, or a heterocyclic group which may be substituted; and n is an integer from 0 to 2]; or
Formula (1-b): 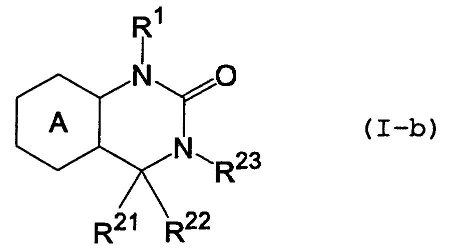
wherein ring A is a 6-membered aromatic ring which may be further substituted,
R¹ is a hydrocarbon group which may be substituted or a hydrogen atom,
R²¹ and R²², which may be identical or different, are each a hydrocarbon group which may be substituted or a hydrogen atom, and
R²³ is
(1) a group represented by the formula: 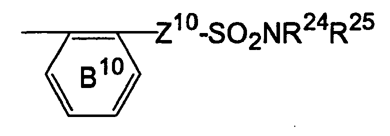 [wherein ring B¹⁰ is a benzene ring which may be further substituted; Z¹⁰ is C₁₋₆ alkylene which may be substituted or a bond; and R²⁴ and R²⁵, which may be identical or different, are each (a) a hydrogen atom, (b) a hydrocarbon group which may be substituted, or (c) a heterocyclic group which may be substituted; or R²⁴ and R²⁵ may form a ring together with the adjacent nitrogen atom],
(2) a group represented by the formula: 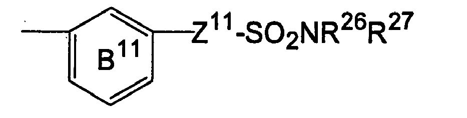 [wherein ring B¹¹ is a benzene ring which may be further substituted; Z¹¹ is C₁₋₆ alkylene which may be substituted or a bond; and R²⁶ and R²⁷, which may be identical or different, are each (a) a hydrogen atom, (b) a hydrocarbon group which may be substituted, or (c) a heterocyclic group which may be substituted; or R²⁶ and R²⁷ may form a ring together with the adjacent nitrogen atom],
(3) a group represented by the formula: 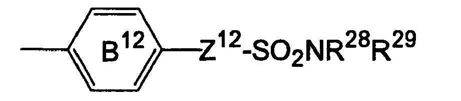 [wherein ring B¹² is a benzene ring which may be further substituted; Z¹² is C₁₋₆ alkylene which may be substituted or a bond; and R²⁸ and R²⁹, which may be identical or different, are each (a) a hydrogen atom, (b) a hydrocarbon group which may be substituted, or (c) a heterocyclic group which may be substituted; or R²⁸ and R²⁹ may form a ring together with the adjacent nitrogen atom],
(4) a group represented by the formula: 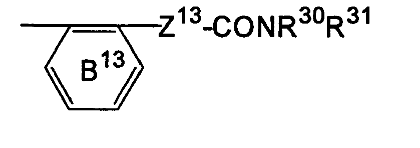 [wherein ring B¹³ is a benzene ring which may be further substituted; Z¹³ is C₁₋₆ alkylene which may be substituted or a bond; and R³⁰ and R³¹, which may be identical or different, are each (a) a hydrogen atom, (b) a hydrocarbon group which may be substituted, or (c) a heterocyclic group which may be substituted; or R³⁰ and R³¹ may form a ring together with the adjacent nitrogen atom],
(5) a group represented by the formula: 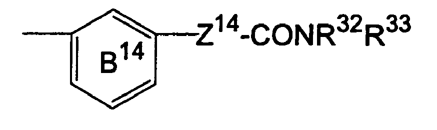 [wherein ring B¹⁴ is a benzene ring which may be further substituted; Z¹⁴ is C₁₋₆ alkylene which may be substituted or a bond; and R³² and R³³, which may be identical or different, are each (a) a hydrogen atom, (b) a hydrocarbon group which may be substituted, or (c) a heterocyclic group which may be substituted; or R³² and R³³ may form a ring together with the adjacent nitrogen atom],
(6) a group represented by the formula: 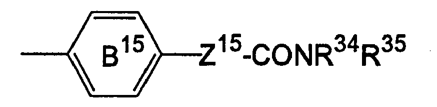 [wherein ring B¹⁵ is a benzene ring which may be further substituted; Z¹⁵ is C₁₋₆ alkylene which may be substituted or a bond; and R³⁴ and R³⁵, which may be identical or different, are each (a) a hydrogen atom, (b) a hydrocarbon group which may be substituted, or (c) a heterocyclic group which may be substituted; or R³⁴ and R³⁵ may form a ring together with the adjacent nitrogen atom],
(7) a group represented by the formula: 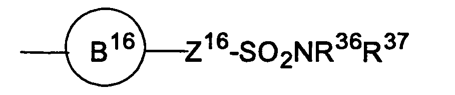 [wherein ring B¹⁶ is a C₁₀₋₁₄ aryl ring which may be further substituted, or an aromatic heterocycle which may be further substituted; Z¹⁶ is C₁₋₆ alkylene which may be substituted or a bond; and R³⁶ and R³⁷, which may be identical or different, are each (a) a hydrogen atom, (b) a hydrocarbon group which may be substituted, or (c) a heterocyclic group which may be substituted; or R³⁶ and R³⁷ may form a ring together with the adjacent nitrogen atom],
(8) a group represented by the formula: 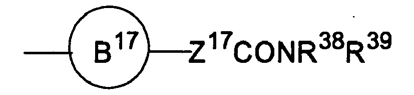 [wherein ring B¹⁷ is a C₁₀₋₁₄ aryl ring which may be further substituted, or an aromatic heterocycle which may be further substituted; Z¹⁷ is C₁₋₆ alkylene which may be substituted or a bond; and R³⁸ and R³⁹, which may be identical or different, are each (a) a hydrogen atom, (b) a hydrocarbon group which may be substituted, or (c) a heterocyclic group which may be substituted; or R³⁸ and R³⁹ may form a ring together with the adjacent nitrogen atom], or
(9) a group represented by the formula: 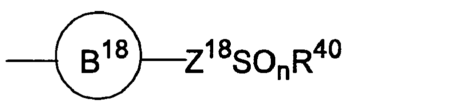 [wherein ring B¹⁸ is a C₆₋₁₄ aryl ring which may be further substituted, or an aromatic heterocycle which may be further substituted; Z¹⁸ is C₁₋₆ alkylene which may be substituted or a bond; R⁴⁰ is a hydrocarbon group which may be substituted, or a heterocyclic group which may be substituted; and n is an integer from 0 to 2, provided that the case where R²¹ and R²² are each a hydrogen atom, ring B¹⁸ is a thiazole ring, Z¹⁸ is methylene, and n is 2, and the case where R²¹ and R²² are each dimethylaminophenyl, are excluded], or a salt thereof;
provided that the following are excluded:
2-[2-(5-chloro-2,4-dioxo-1,2,3,4-tetrahydro-3-quinazolinyl)phenyl]-N-(1-naphthylmethyl)acetamide,
2-[3-(5-chloro-2,4-dioxo-1,2,3,4-tetrahydro-3-quinazolinyl)phenyl]-N-(1-naphthylmethyl)acetamide,
3-(5-chloro-2,4-dioxo-1,2,3,4-tetrahydro-3-quinazolinyl)-N-(1-naphthylmethyl)benzamide,
7-chloro-1-[4-(1,4-dihydro-2,4-dioxo-3(2H)-quinazolinyl)benzoyl]-2,3,4,5-tetrahydro-1H-1-benzazepine,
N,N-dibutyl-4-(1,4-dihydro-2,4-dioxo-3(2H)-quinazolinyl)benzenesulfonamide,
3,3'-(6,6'-sulfonyldibenzothiazole-2,2'-diyl)bis(2,4-dioxo-1,2,3,4-tetrahydroquinazoline),
N-[4-(benzyloxy)phenyl]-3-methoxy-4-(2-oxo-1,4-dihydroquinazolin-3(2H)-yl)benzamide, and
N-[4-(benzyloxy)phenyl]-4-(6,7-dimethoxy-2-oxo-1,4-dihydroquinazolin-3(2H)-yl)-3-methoxybenzamide.

9. The compound according to claim 8, represented by the formula: 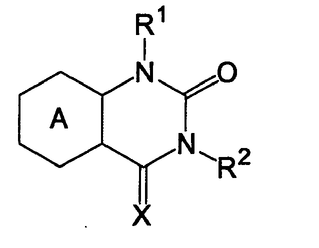 wherein ring A is a 6-membered aromatic ring which may be further substituted,
X is an oxygen atom, a sulfur atom or NR³ (wherein R³ is a hydrocarbon group which may be substituted or a hydrogen atom),
R¹ is a hydrocarbon group which may be substituted or a hydrogen atom, and
R² is
(1) a group represented by the formula: 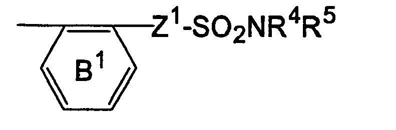 [wherein ring B¹ is a benzene ring which may be further substituted; Z¹ is C₁₋₆ alkylene which may be substituted or a bond; R⁴ is (a) a hydrogen atom, (b) a hydrocarbon group which may be substituted, or (c) a heterocyclic group which may be substituted; and R⁵ is (a) a hydrocarbon group which may be substituted, or (b) a heterocyclic group which may be substituted; or R⁴ and R⁵ may form a ring together with the adjacent nitrogen atom],
(2) a group represented by the formula: 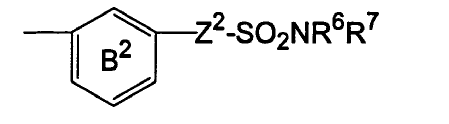 [wherein ring B² is a benzene ring which may be further substituted; Z² is C₁₋₆ alkylene which may be substituted or a bond; R⁶ is (a) a hydrogen atom, (b) a hydrocarbon group which may be substituted, or (c) a heterocyclic group which may be substituted; and R⁷ is (a) a hydrocarbon group which may be substituted, or (b) a heterocyclic group which may be substituted; or R⁶ and R⁷ may form a ring together with the adjacent nitrogen atom],
(3) a group represented by the formula: 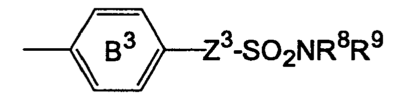 [wherein ring B³ is a benzene ring which may be further substituted; Z³ is C₁₋₆ alkylene which may be substituted or a bond; R⁸ is (a) a hydrogen atom, (b) a hydrocarbon group which may be substituted, or (c) a heterocyclic group which may be substituted; and R⁹ is (a) a hydrocarbon group which may be substituted, or (b) a heterocyclic group which may be substituted; or R⁸ and R⁹ may form a ring together with the adjacent nitrogen atom, provided that when R⁸ is a hydrogen atom, R⁹ is (a) an aryl group which may be substituted, (b) an aralkyl group which may be substituted, or (c) an alkyl group substituted with a heterocyclic group which may be substituted],
(4) a group represented by the formula: 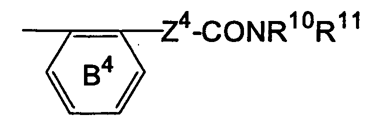 [wherein ring B⁴ is a benzene ring which may be further substituted; Z⁴ is C₁₋₆ alkylene which may be substituted or a bond; R¹⁰ is (a) a hydrogen atom, (b) a hydrocarbon group which may be substituted, or (c) a heterocyclic group which may be substituted; and R¹¹ is (a) an aromatic group which may be substituted, (b) an aralkyl group which may be substituted, or (c) an alkyl group substituted with a heterocyclic group which may be substituted; or R¹⁰ and R¹¹ may form, together with the adjacent nitrogen atom, a heterocyclic ring comprising carbon and nitrogen atoms as ring-constituting moieties],
(5) a group represented by the formula: 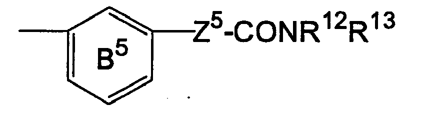 [wherein ring B⁵ is a benzene ring which may be further substituted; Z⁵ is C₁₋₆ alkylene which may be substituted or a bond; and R¹² and R¹³, which may be identical or different, are each (a) a hydrogen atom, (b) a hydrocarbon group which may be substituted, or (c) a heterocyclic group which may be substituted; or R¹² and R¹³ may form a ring together with the adjacent nitrogen atom],
(6) a group represented by the formula: 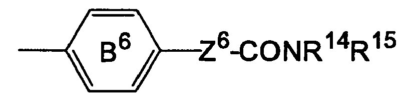 [wherein ring B⁶ is a benzene ring which may be further substituted; Z⁶ is C₁₋₆ alkylene which may be substituted or a bond; and R¹⁴ and R¹⁵, which may be identical or different, are each (a) a hydrocarbon group which may be substituted, or (b) a heterocyclic group which may be substituted; or R¹⁴ and R¹⁵ may form a ring together with the adjacent nitrogen atom],
(7) a group represented by the formula: 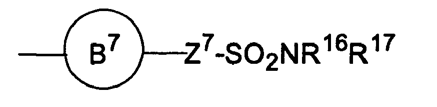 [wherein ring B⁷ is a C₁₀₋₁₄ aryl ring which may be further substituted, or an aromatic heterocycle which may be further substituted; Z⁷ is C₁₋₆ alkylene which may be substituted or a bond; R¹⁶ is (a) a hydrogen atom, (b) a hydrocarbon group which may be substituted, or (c) a heterocyclic group which may be substituted; and R¹⁷ is (a) a hydrocarbon group which may be substituted, or (b) a heterocyclic group which may be substituted; or R¹⁶ and R¹⁷ may form a ring together with the adjacent nitrogen atom], or
(8) a group represented by the formula: 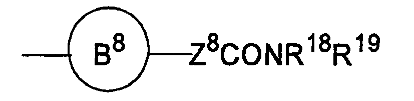 [wherein ring B⁸ is a C₁₀₋₁₄ aryl ring which may be further substituted, or an aromatic heterocycle which may be further substituted; Z⁸ is C₁₋₆ alkylene which may be substituted or a bond; R¹⁸ is (a) a hydrogen atom, (b) a hydrocarbon group which may be substituted, or (c) a heterocyclic group which may be substituted; and R¹⁹ is (a) a hydrocarbon group which may be substituted, or (b) a heterocyclic group which may be substituted; or R¹⁸ and R¹⁹ may form a ring together with the adjacent nitrogen atom], provided that the following are excluded:
7-chloro-1-[4-(1,4-dihydro-2,4-dioxo-3(2H)-quinazolinyl)benzoyl]-2,3,4,5-tetrahydro-1H-1-benzazepine, and
N,N-dibutyl-4-(1,4-dihydro-2,4-dioxo-3(2H)-quinazolinyl)benzenesulfonamide.

10. The compound according to claim 8 or 9, wherein
R¹ is (1) a hydrogen atom, or (2) C₁₋₆ alkyl which may be substituted with 1 to 3 substituents selected from (a) C₆₋₁₄ aryl which may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen, (b) cyano, (c) hydroxy, (d) carboxy, (e) nitro, (f) C₁₋₆ alkyl which may be substituted with di-C₁₋₆ alkylamino, (g) C₁₋₆ alkoxy, (h) C₁₋₆ alkyl-carbonyloxy, (i) C₁₋₆ alkylthio, (j) C₁₋₆ alkylsulfinyl, (k) C₁₋₆ alkylsulfonyl, (1) halogen, (m) amino, (n) mono-C₁₋₆ alkylamino, (o) di-C₁₋₆ alkylamino, (p) a 5-membered ring group containing 1 to 4 heteroatoms selected from oxygen, sulfur and nitrogen atoms and the like in addition to carbon atoms (wherein this 5-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (q) a 6-membered ring group containing 1 to 4 heteroatoms selected from oxygen, sulfur and nitrogen atoms and the like in addition to carbon atoms (wherein this 6-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (r) a bicyclic or tricyclic fused ring group containing 1 to 4 heteroatoms selected from oxygen, sulfur and nitrogen atoms and the like in addition to carbon atoms (wherein this bicyclic or tricyclic fused ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (s) C₁₋₆ alkoxycarbonyl, (t) carbamoyl, (u) N-mono-C₁₋₆ alkylcarbamoyl, (v) N,N-di-C₁₋₆ alkylcarbamoyl, and (w) C₁₋₂ alkylenedioxy.

11. The compound according to claim 8 or 9, wherein X is an oxygen atom, or NR³ (wherein R³ is (1) a hydrogen atom, (2) C₁₋₆ alkyl which may be substituted with 1 to 3 substituents selected from (a) C₆₋₁₄ aryl which may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen, (b) cyano, (c) hydroxy, (d) carboxy, (e) nitro, (f) C₁₋₆ alkyl which may be substituted with di-C₁₋₆ alkylamino, (g) C₁₋₆ alkoxy, (h) C₁₋₆ alkyl-carbonyloxy, (i) C₁₋₆ alkylthio, (j) C₁₋₆ alkylsulfinyl, (k) C₁₋₆ alkylsulfonyl, (1) halogen, (m) amino, (n) mono-C₁₋₆ alkylamino, (o) di-C₁₋₆ alkylamino, (p) a 5-membered ring group containing 1 to 4 heteroatoms selected from oxygen, sulfur and nitrogen atoms and the like in addition to carbon atoms (wherein this 5-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (q) a 6-membered ring group containing 1 to 4 heteroatoms selected from oxygen, sulfur and nitrogen atoms and the like in addition to carbon atoms (wherein this 6-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (r) a bicyclic or tricyclic fused ring group containing 1 to 4 heteroatoms selected from oxygen, sulfur and nitrogen atoms and the like in addition to carbon atoms (wherein this bicyclic or tricyclic fused ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (s) C₁₋₆ alkoxycarbonyl, (t) carbamoyl, (u) N-mono-C₁₋₆ alkylcarbamoyl, (v) N,N-di-C₁₋₆ alkylcarbamoyl, and (w) C₁₋₂ alkylenedioxy (hereinafter (a) to (w) are briefly referred to as Substituent Group A), (3) C₂₋₆ alkenyl which may be substituted with 1 to 3 substituents selected from the Substituent Group A, (4) C₂₋₆ alkynyl which may be substituted with 1 to 3 substituents selected from the Substituent Group A, (5) C₃₋₈ cycloalkyl which may be substituted with 1 to 3 substituents selected from the Substituent Group A, (6) C₆₋₁₄ aryl which may be substituted with 1 to 3 substituents selected from the Substituent Group A, or (7) C₇₋₁₄ aralkyl which may be substituted with 1 to 3 substituents selected from the Substituent Group A).

12. The compound according to claim 8, wherein ring A is a benzene ring substituted with one substituent selected from (a) hydroxy, (b) cyano, (c) carboxyl, (d) mono- or dialkylcarbamoyl, (e) acylamino, (f) alkylamino, and (g) alkoxycarbonyl, or a benzene ring substituted with a hydrocarbon group substituted with a substituent selected from the above substituent groups.

13. The compound according to claim 8, wherein ring B², ring B⁵, ring B¹¹ and ring B¹⁴, which may be identical or different, are each a benzene ring which may be further substituted with 1 to 3 substituents selected from (a) C₆₋₁₄ aryl which may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen, (b) cyano, (c) hydroxy, (d) carboxy, (e) nitro, (f) C₁₋₆ alkyl which may be substituted with di-C₁₋₆ alkylamino, (g) C₁₋₆ alkoxy, (h) C₁₋₆ alkylcarbonyloxy, (i) C₁₋₆ alkylthio, (j) C₁₋₆ alkylsulfinyl, (k) C₁₋₆ alkylsulfonyl, (1) halogen, (m) amino, (n) mono-C₁₋₆ alkylamino, (o) di-C₁₋₆ alkylamino, (p) a 5-membered ring group containing 1 to 4 heteroatoms selected from oxygen, sulfur and nitrogen atoms and the like in addition to carbon atoms (wherein this 5-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (q) a 6-membered ring group containing 1 to 4 heteroatoms selected from oxygen, sulfur and nitrogen atoms and the like in addition to carbon atoms (wherein this 6-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (r) a bicyclic or tricyclic fused ring group containing 1 to 4 heteroatoms selected from oxygen, sulfur and nitrogen atoms and the like in addition to carbon atoms (wherein this bicyclic or tricyclic fused ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (s) C₁₋₆ alkoxycarbonyl, (t) carbamoyl, (u) N-mono-C₁₋₆ alkylcarbamoyl, (v) N,N-di-C₁₋₆ alkylcarbamoyl, and (w) C₁₋₂ alkylenedioxy (hereinafter (a) - (w) are briefly referred to as Substituent Group A) ;
ring B⁷, ring B⁸, ring B¹⁶ and ring B¹⁷, which may be identical or different, are each an aromatic heterocycle which may be further substituted with 1 to 3 substituents selected from the Substituent Group A;
Z², Z⁵, Z⁷, Z⁸, Z¹¹, Z¹⁴, Z¹⁶ and Z¹⁷, which may be identical or different, are each C₁₋₆ alkylene which may be substituted or a bond;
R⁶ , R¹² , R¹⁶ , R¹⁸ , R²⁶, R³², R³⁶ and R³⁸ , which may be identical or different, are each (a) a hydrogen atom, (b) C₁₋₆ alkyl which may be substituted with 1 to 3 substituents selected from the Substituent Group A, (c) C₂₋₆ alkenyl which may be substituted with 1 to 3 substituents selected from the Substituent Group A, (d) C₂₋₆ alkynyl which may be substituted with 1 to 3 substituents selected from the Substituent Group A, (e) C₃₋₈ cycloalkyl which may be substituted with 1 to 3 substituents selected from the Substituent Group A, (f) C₆₋₁₄ aryl which may be substituted with 1 to 3 substituents selected from the Substituent Group A, (g) C₇₋₁₄ aralkyl which may be substituted with 1 to 3 substituents selected from the Substituent Group A, or (h) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A), provided that R¹² is not a hydrogen atom; and
R⁷, R¹³, R¹⁷, R¹⁹, R²⁷, R³³ R³⁷ and R³⁹, which may be identical or different, are each (a) C₁₋₁₀ alkyl which may be substituted with 1 to 3 substituents selected from the Substituent Group A, (b) C₆₋₁₄ aryl which may be substituted with 1 to 3 substituents selected from the Substituent Group A, or (c) a 5- or 6-membered aromatic ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or
R⁶ and R⁷, R¹² and R¹³, R¹⁶ and R¹⁷, R¹⁸ and R¹⁹, R²⁶ and R²⁷, R³² and R³³, R³⁶ and R³⁷, and R³⁸ and R³⁹ may respectively form a ring together with the adjacent nitrogen atom.

14. The compound according to claim 8, wherein ring A is a benzene ring, X is an oxygen atom or NH, R¹ is a hydrogen atom or C₁₋₆ alkyl;
ring B², ring B⁵, ring B¹¹ and ring B¹⁴, which may be identical or different, are each a benzene ring which may be further substituted with one substituent selected from halogen, lower alkyl and cyano;
ring B⁷, ring B⁸, ring B¹⁶ and ring B¹⁷, which may be identical or different, are each a thiophene ring or furan ring which may be further substituted;
Z², Z⁵ Z⁷ , Z⁸ Z¹¹, Z¹⁴ Z¹⁶ and Z¹⁷ , which may be identical or different, are each C₁₋₆ alkylene which may be substituted or a bond;
R⁶ , R¹² , R¹⁶ , R¹⁸ , R²⁶ , R³², R³⁶ and R³⁸ , which may be identical or different, are each C₁₋₆ alkyl which may be substituted with cyano; and
R⁷, R¹³, R¹⁷, R¹⁹, R²⁷, R³³, R³⁷ and R³⁹, which may be identical or different, are each (a) C₁₋₁₀ alkyl which may be substituted with 1 to 3 substituents selected from the Substituent Group A, (b) C₆₋₁₄ aryl which may be substituted with halogen, or (c) pyridyl; or
R⁶ and R⁷, R¹² and R¹³, R¹⁶ and R¹⁷, R¹⁸ and R¹⁹, R²⁶ and R²⁷, R³² and R³³, R³⁶ and R³⁷, and R³⁸ and R³⁹ may respectively form, together with the adjacent nitrogen atom, (1) 1,2,3,4-tetrahydroquinolin-1-yl which may be substituted with C₁₋₆ alkyl, halogen or C₁₋₆ alkoxy, (2) 2,3-dihydro-1H-indol-1-yl which may be substituted with C₁₋₆ alkyl, (3) 2,3,4,5-tetrahydro-1H-1-benzazepin-1-yl which may be substituted with C₁₋₆ alkyl, or (4) 3,4-dihydro-2H-1,4-benzoxazin-1-yl.

15. The compound according to claim 13 or 14, wherein Z², Z⁵, Z⁷, Z⁸, Z¹¹, Z¹⁴, Z¹⁶ and Z¹⁷ are each a bond.

16. The compound according to claim 9, wherein R² is
(1) a group represented by the formula: 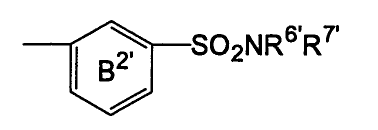 [wherein ring B^{2'} is a benzene ring which may be further substituted with 1 to 3 substituents selected from (a) C₆₋₁₄ aryl which may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen, (b) cyano, (c) hydroxy, (d) carboxy, (e) nitro, (f) C₁₋₆ alkyl which may be substituted with di-C₁₋₆ alkylamino, (g) C₁₋₆ alkoxy, (h) C₁₋₆ alkyl-carbonyloxy, (i) C₁₋₆ alkylthio, (j) C₁₋₆ alkylsulfinyl, (k) C₁₋₆ alkylsulfonyl, (1) halogen, (m) amino, (n) mono-C₁₋₆ alkylamino, (o) di-C₁₋₆ alkylamino, (p) a 5-membered ring group containing 1 to 4 heteroatoms selected from oxygen, sulfur and nitrogen atoms and the like in addition to carbon atoms (wherein this 5-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (q) a 6-membered ring group containing 1 to 4 heteroatoms selected from oxygen, sulfur and nitrogen atoms and the like in addition to carbon atoms (wherein this 6-membered ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (r) a bicyclic or tricyclic fused ring group containing 1 to 4 heteroatoms selected from oxygen, sulfur and nitrogen atoms and the like in addition to carbon atoms (wherein this bicyclic or tricyclic fused ring group may be substituted with 1 to 3 substituents selected from (i) hydroxy, (ii) amino, (iii) mono-C₁₋₆ alkylamino, (iv) di-C₁₋₆ alkylamino, (v) C₁₋₆ alkoxy and (vi) halogen), (s) C₁₋₆ alkoxycarbonyl, (t) carbamoyl, (u) N-mono-C₁₋₆ alkylcarbamoyl, (v) N,N-di-C₁₋₆ alkylcarbamoyl, and (w) C₁₋₂ alkylenedioxy (hereinafter (a) - (w) are briefly referred to as Substituent Group A);
R^{6'} is (a) a hydrogen atom, (b) C₁₋₆ alkyl which may be substituted with 1 to 3 substituents selected from the Substituent Group A, (c) C₂₋₆ alkenyl which may be substituted with 1 to 3 substituents selected from the Substituent Group A, (d) C₂₋₆ alkynyl which may be substituted with 1 to 3 substituents selected from the Substituent Group A, (e) C₃₋₈ cycloalkyl which may be substituted with 1 to 3 substituents selected from the Substituent Group A, (f) C₆₋₁₄ aryl which may be substituted with 1 to 3 substituents selected from the Substituent Group A, (g) C₇₋₁₄ aralkyl which may be substituted with 1 to 3 substituents selected from the Substituent Group A, or (h) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); and
R^{7'} is (a) C₆₋₁₄ aryl which may be substituted with 1 to 3 substituents selected from the Substituent Group A, or (b) a 5-or 6-membered aromatic ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or
R^{6'} and R^{7'} may form a ring together with the adjacent nitrogen atom],
(2) a group represented by the formula: 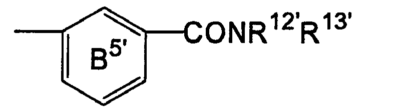 [wherein ring B^{5'} is a benzene ring which may be further substituted with 1 to 3 substituents selected from the Substituent Group A;
R¹²' is (a) C₁₋₆ alkyl which may be substituted with 1 to 3 substituents selected from the Substituent Group A, (b) C₂₋₆ alkenyl which may be substituted with 1 to 3 substituents selected from the Substituent Group A, (c) C₂₋₆ alkynyl which may be substituted with 1 to 3 substituents selected from the Substituent Group A, (d) C₃₋₈ cycloalkyl which may be substituted with 1 to 3 substituents selected from the Substituent Group A, (e) C₆₋₁₄ aryl which may be substituted with 1 to 3 substituents selected from the Substituent Group A, (f) C₇₋₁₄ aralkyl which may be substituted with 1 to 3 substituents selected from the Substituent Group A, or (g) a 5-or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); and
R¹³' is (a) C₆₋₁₄ aryl which may be substituted with 1 to 3 substituents selected from the Substituent Group A, or (b) a 5-or 6-membered aromatic ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or
R^{12'} and R¹³' may form a ring together with the adjacent nitrogen atom], or
(3) a group represented by the formula: 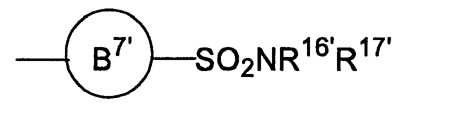 [wherein ring B^{7'} is an aromatic heterocycle which may be further substituted with 1 to 3 substituents selected from the Substituent Group A;
R¹⁶' is (a) a hydrogen atom, (b) C₁₋₆ alkyl which may be substituted with 1 to 3 substituents selected from the Substituent Group A, (c) C₂₋₆ alkenyl which may be substituted with 1 to 3 substituents selected from the Substituent Group A, (d) C₂₋₆ alkynyl which may be substituted with 1 to 3 substituents selected from the Substituent Group A, (e) C₃₋₈ cycloalkyl which may be substituted with 1 to 3 substituents selected from the Substituent Group A, (f) C₆₋₁₄ aryl which may be substituted with 1 to 3 substituents selected from the Substituent Group A, (g) C₇₋₁₄ aralkyl which may be substituted with 1 to 3 substituents selected from the Substituent Group A, or (h) a 5- or 6-membered ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); and
R^{17'} is (a) C₆₋₁₄ aryl which may be substituted with 1 to 3 substituents selected from the Substituent Group A, or (b) a 5-or 6-membered aromatic ring group containing 1 to 4 heteroatoms selected from oxygen, sulfur and nitrogen atoms and the like in addition to carbon atoms (wherein this 5- or 6-membered ring may be substituted with a substituent selected from the Substituent Group A); or
R^{16'} and R^{17'} may form a ring together with the adjacent nitrogen atom].

17. The compound according to claim 9, wherein ring A is a benzene ring, X is an oxygen atom or NH, R¹ is a hydrogen atom or C₁₋₆ alkyl, and R² is
(1) a group represented by the formula: 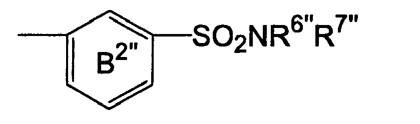 [wherein ring B^{2"} is a benzene ring which may be further substituted with halogen, R^{6"} is C₁₋₆ alkyl which may be substituted with cyano, and R^{7"} is C₆₋₁₄ aryl or pyridyl which may be substituted with halogen, or R^{6"} and R^{7"} form, together with the adjacent nitrogen atom, (1) 1,2,3,4-tetrahydroquinolin-1-yl which may be substituted with C₁₋₆ alkyl, halogen or C₁₋₆ alkoxy, (2) 2,3-dihydro-1H-indol-1-yl which may be substituted with C₁₋₆ alkyl, (3) 2,3,4,5-tetrahydro-1H-1-benzazepin-1-yl which may be substituted with C₁₋₆ alkyl, or (4) 3,4-dihydro-2H-1,4-benzoxazin-1-yl],
(2) a group represented by the formula: 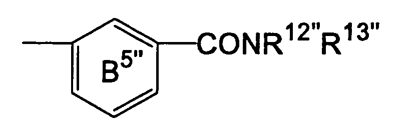 [wherein ring B^{5"} is a benzene ring which may be further substituted with halogen, R^{12"} is C₁₋₆ alkyl which may be substituted with cyano, and R^{13"}is C₆₋₁₄ aryl or pyridyl which may be substituted with halogen, or R^{12"} and R^{13"} form, together with the adjacent nitrogen atom, (1) 1,2,3,4-tetrahydroquinolin-1-yl which may be substituted with C₁₋₆ alkyl, halogen or C₁₋₆ alkoxy, (2) 2,3-dihydro-1H-indol-1-yl which may be substituted with C₁₋₆ alkyl, (3) 2,3,4,5-tetrahydro-1H-1-benzazepin-1-yl which may be substituted with C₁₋₆ alkyl, or (4) 3,4-dihydro-2H-1,4-benzoxazin-1-yl], or
(3) a group represented by the formula: 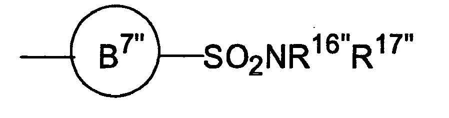 [wherein ring B^{7"} is a thiophene ring which may be further substituted with halogen, R^{16"} is C₁₋₆ alkyl which may be substituted with cyano, and R^{17"} is C₆₋₁₄ aryl or pyridyl which may be substituted with halogen, or R^{16"} and R^{17"} form, together with the adjacent nitrogen atom, (1) 1,2,3,4-tetrahydroquinolin-1-yl which may be substituted with C₁₋₆ alkyl, halogen or C₁₋₆ alkoxy, (2) 2,3-dihydro-1H-indol-1-yl which may be substituted with C₁₋₆ alkyl, (3) 2,3,4,5-tetrahydro-1H-1-benzazepin-1-yl which may be substituted with C₁₋₆ alkyl, or (4) 3,4-dihydro-2H-1,4-benzoxazin-1-yl].

18. 3- (2-chloro-5-(3,4-dihydroquinolin-1(2H)- ylsulfonyl)phenyl)quinazoline-2,4(1H,3H)-dione,
4-chloro-3-(2,4-dioxo-1,4-dihydro-3(2H)-quinazolinyl)-N-methyl-N-2-pyridinylbenzenesulfonamide trifluoroacetate,
3-(2-chloro-5-(2,3,4,5-tetrahydro-1H-1-benzazepin-1-ylsulfonyl)phenyl)-2,4(1H,3H)-quinazolinedione,
3-(2-chloro-5-(3,4-dihydro-1,5-benzoxazepin-5(2H)-ylsulfonyl)phenyl)-2,4(1H,3H)-quinazolinedione,
4-chloro-3-(4-imino-2-oxo-1,4-dihydro-3(2H)-quinazolinyl)-N-methyl-N-phenylbenzenesulfonamide trifluoroacetate, or
3-(2-chloro-5-(3,4-dihydro-1(2H)-quinolinylsulfonyl)phenyl)-4-imino-3,4-dihydro-2(1H)-quinazolinone trifluoroacetate.

19. 3-[5-(3,4-dihydroquinolin-1(2H)-ylsulfonyl)-2-fluorophenyl]quinazoline-2,4(1H,3H)-dione,
3-[2-chloro-5-(5,6,7,8-tetrahydro-4H-thieno[3,2-b]azepin-4-ylsulfonyl)phenyl]quinazoline-2,4(1H,3H)-dione,
4-chloro-3-(2,4-dioxo-1,4-dihydroquinazolin-3(2H)-yl)-N-methyl-N-phenylbenzamide,
5-chloro-4-[5-(hydroxymethyl)-2,4-dioxo-1,4-dihydroquinazolin-3(2H)-yl]-N-methyl-*N*-phenylthiophene-2-carboxamide,
Methyl 3-[2-chloro-5-(3,4-dihydroquinolin-1(2H)-ylsulfonyl)phenyl]-2,4-dioxo-1,2,3,4-tetrahydroquinazoline-5-carboxylate,
3-[2-chloro-5-(3,4-dihydroquinolin-1(2H)-ylsulfonyl)phenyl]-2,4-dioxo-1,2,3,4-tetrahydroquinazoline-5-carboxylic acid,
3-[2-chloro-5-(3,4-dihydroquinolin-1(2H)-ylsulfonyl)phenyl]-5-(hydroxymethyl)quinazoline-2,4(1H,3H)-dione,
3-(2,4-dioxo-1,4-dihydroquinazolin-3(2H)-yl)-5-(2,3,4,5-tetrahydro-1H-1-benzazepin-1-ylsulfonyl)thiophene-2-carbonitrile,
4-chloro-3-[5-(hydroxymethyl)-2,4-dioxo-1,4-dihydroquinazolin-3(2H)-yl]-N-methyl-N-phenylbenzamide,
4-chloro-3-[5-(hydroxymethyl)-2,4-dioxo-1,4-dihydroquinazolin-3(2H)-yl]-N-methyl-N-pyridin-2-ylbenzamide,
3-[2-chloro-5-(2,3,4,5-tetrahydro-1H-1-benzazepin-1-ylsulfonyl)-3-thienyl]-5-(hydroxymethyl)quinazoline-2,4(1H,3H)-dione,
5-chloro-N-cyclohexyl-4-[5-(hydroxymethyl)-2,4-dioxo-1,4-dihydroquinazolin-3(2H)-yl]-N-methylthiophene-2-carboxamide,
5-chloro-N-(2-chlorophenyl)-4-[5-(hydroxymethyl)-2,4-dioxo-1,4-dihydroquinazolin-3(2H)-yl]-N-methylthiophene-2-carboxamide,
5-chloro-N-(3-chlorophenyl)-4-[5-(hydroxymethyl)-2,4-dioxo-1,4-dihydroquinazolin-3(2H)-yl]-N-methylthiophene-2-carboxamide,
5-chloro-N-(4-chlorophenyl)-4-[5-(hydroxymethyl)-2,4-dioxo-1,4-dihydroquinazolin-3(2H)-yl]-N-methylthiophene-2-carboxamide,
5-chloro-4-[5-(hydroxymethyl)-2,4-dioxo-1,4-dihydroquinazolin-3(2H)-yl]-N-methyl-N-(2-methylphenyl)thiophene-2-carboxamide,
5-chloro-4-[5-(hydroxymethyl)-2,4-dioxo-1,4-dihydroquinazolin-3(2H)-yl]-N-methyl-N-(3-methylphenyl)thiophene-2-carboxamide,
3-[2-chloro-5-(3,4-dihydroquinolin-1(2H)-ylsulfonyl)phenyl]-2,4-dioxo-1,2,3,4-tetrahydroquinazoline-5-carboxamide,
3-[2-chloro-5-(3,4-dihydroquinolin-1(2H)-ylsulfonyl)phenyl]-3,4-dihydroquinazolin-2(1H)-one,
3-[2-chloro-5-(3,4-dihydroquinolin-1(2H)-ylsulfonyl)phenyl]-2-oxo-1,2,3,4-tetrahydroquinazoline-5-carboxylic acid,
5-chloro-4-[5-(hydroxymethyl)-2,4-dioxo-1,4-dihydroquinazolin-3(2H)-yl]-N-isobutyl-N-methylthiophene-2-carboxamide, or
3-[2-chloro-5-(3,4-dihydroquinolin-1(2H)-ylsulfonyl)phenyl]-5-(1-hydroxy-1-methylethyl)quinazoline-2,4(1H,3H)-dione.

20. A prodrug of the compound according to claim 8 or 9.

21. A medicine which comprises the compound according to claim 8 or 9, or a prodrug thereof.

22. A prophylactic and/or therapeutic agent for sex hormone-dependent diseases, containing the compound according to claim 8 or a prodrug thereof.

23. A prophylactic and/or therapeutic agent for prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease, infertility, irritable bowel syndrome, prostate cancer, uterine cancer, breast cancer or pituitary tumor, a reproduction regulator, a contraceptive, an ovulation inducing agent, or a preventive agent for post-operative recurrence of sex hormone-dependent cancers, containing the compound according to claim 8 or a prodrug thereof.

24. A prophylactic and/or therapeutic agent for prostate cancer, uterine cancer, breast cancer or pituitary tumor, or a preventive agent for post-operative recurrence of sex hormone-dependent cancers, containing the compound according to claim 8 or 9, or a prodrug thereof.

25. A method for prevention and/or treatment of prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease, infertility, irritable bowel syndrome, prostate cancer, uterine cancer, breast cancer or pituitary tumor, a method for reproduction regulation, a method for contraception, a method for ovulation induction, or a method for prevention of post-operative recurrence of sex hormone-dependent cancers, which comprises administering an effective amount of the compound according to claim 8 or a prodrug thereof to a mammal.

26. A method for prevention and/or treatment of prostate cancer, uterine cancer, breast cancer or pituitary tumor, or a method for prevention of post-operative recurrence of sex hormone-dependent cancers, which comprises administering an effective amount of the compound according to claim 8 or 9, or a prodrug thereof to a mammal.

27. Use of the compound according to claim 8 or a prodrug thereof for the manufacture of a prophylactic and/or therapeutic agent for prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease, infertility, irritable bowel syndrome, prostate cancer, uterine cancer, breast cancer or pituitary tumor, a reproduction regulator, a contraceptive, an ovulation inducing agent, or a preventive agent for post-operative recurrence of sex hormone-dependent cancers.

28. Use of the compound according to claim 8 or 9, or a prodrug thereof for the manufacture of a prophylactic and/or therapeutic agent for prostate cancer, uterine cancer, breast cancer or pituitary tumor, or a preventive agent for post-operative recurrence of sex hormone-dependent cancers.
